# EUROPEAN PATENT SPECIFICATION

(11) **EP 4 563 199 B1**
(45) Date of publication and mention of the grant of the patent: **16.09.2026**
(21) Application number: 25162106.6
(22) Date of filing: 27.01.2022
(51) Int. Cl.: C07D 405/14, C07D 413/14, A61K 31/437, A61K 31/4184, A61K 31/439, A61K 31/444, A61K 31/496, A61P 3/00

(54) **GPCR RECEPTOR AGONISTS, PHARMACEUTICAL COMPOSITIONS COMPRISING THE SAME, AND METHODS FOR THEIR USE**
GPCR-REZEPTORAGONISTEN, PHARMAZEUTISCHE ZUSAMMENSETZUNGEN DAMIT UND VERFAHREN ZU IHRER VERWENDUNG
AGONISTES DU RÉCEPTEUR GPCR, COMPOSITIONS PHARMACEUTIQUES LES COMPRENANT ET PROCÉDÉS POUR LEUR UTILISATION

(30) Priority: 28.01.2021 US 202163143025 P; 03.05.2021 US 202163183612 P; 02.11.2021 US 202163274893 P
(43) Date of publication of application: 04.06.2025
(62) Document number(s) of the earlier application(s) in accordance with Art. 76 EPC: 22704219.9 / 4 284 795
(73) Proprietor: Carmot Therapeutics Inc., South San Francisco, California 94080 (US)
(72) Inventor: DU, Xiaohui, South San Francisco, CA 94080 (US)
(74) Representative: Mathys & Squire

(56) References cited:
- WO-A1-2020/103815
- US-A1- 2018 170 908
- US-A1- 2019 382 384

## Description

Provided herein are GLP-1 receptor modulator compounds; pharmaceutical compositions comprising the compounds; and the compounds, and compositions for use in therapy, including the treatment of type 2 diabetes. The compounds, and compositions are useful, for instance, in methods of treatment, and prevention of metabolic diseases or conditions, methods of detection of metabolic diseases or conditions, and methods of diagnosis of metabolic diseases or conditions.

### BACKGROUND

Diabetes is a serious chronic disease that occurs when the pancreas does not produce enough insulin, or when the body cannot effectively use the insulin it produces. Complications of diabetes include, damage to the heart, blood vessels, eyes, kidneys, and nerves. Diabetes can increase risk of heart disease, and stroke. The results include serious effects on quality of life, health, and mortality. WHO Global Report on Diabetes, 2016, World Health Organization. As of 2017, approximately 462 million individuals worldwide, about 6.28% of the population was affected by type 2 diabetes, and this prevalence was increasing measurably. Khan et al., 2020, J. Epidemiol. Glob. Health 10(1):107-111. The global economic burden of diabetes in 2015 was estimated to be $1.3T, and estimated to increase to $2.1T by 2030. Bommer et al., 2018, Diabetes Care 41(5):963-970. Approximately 90-95% of all diabetes cases are type 2 diabetes. Tripathi & Srivastava, 2016, Med. Sci. Monit. 12(7):RA130-147.

The glucagon-like peptide-1 receptor (GLP-1 receptor, or GLP1R) has emerged as a potential target for treating type 2 diabetes. Its ligand, glucagon-like peptide-1 (GLP-1) enhances glucose-induced insulin secretion, and increases insulin synthesis among many other effects. Doyle and Egan, 2007, Pharmacol. Ther. 113(3):546-593. GLP-1 is known to delay gastric emptying, suppress food intake, increase satiety, and reduce weight in humans. Shah and Vella, 2014 Rev Endocr Metab Disord. 15(3): 181-187. Activating the GLP-1 receptor has been shown to have beneficial effects on insulin secretion and the maintenance of beta cell glucose sensing, transcription, synthesis, proliferation, and survival. Doyle and Egan, 2007, *supra.* While the GLP-1 receptor is a promising therapeutic target, only a handful of GLP-1 receptor drugs have been approved to date, and most, or all of these are peptide, or polypeptide drugs.

US 2018/0170908 A1 discloses 6-carboxylic acids of benzimidazoles and 4-aza-, 5-aza-, 7-aza- and 4,7-diaza-benzimidazoles as GLP-1R agonists, processes to make said compounds, and methods comprising administering said compounds to a mammal in need thereof.

WO 2020/103815 A1 discloses compounds and pharmaceutical compositions thereof which are said to be useful in e.g. treating type 2 diabetes mellitus, pre-diabetes, obesity, non-alcoholic fatty liver disease, non-alcoholic steatohepatitis, and cardiovascular disease.

US 2019/0382384 A1 discloses 6-carboxylic acids of benzimidazoles and 4-aza-, 5-aza-, and 7-aza-benzimidazoles as GLP-1R agonists, processes to make said compounds, and methods comprising administering said compounds to a mammal in need thereof.

There is a need for additional therapies for treating metabolic diseases, and conditions, like type 2 diabetes. Small molecules targeting GLP-1 receptor should provide safe, stable, and easy to administer therapeutics for metabolic diseases, and conditions such as type 2 diabetes.

### SUMMARY OF THE INVENTION

In one aspect, the claimed invention provides a compound which is 2-{[(2S,4S)-4-({2-[(2,4-difluorophenoxy)methyl]pyrimidin-4-yl}oxy)-2-methylpiperidin-1-yl]methyl}-1-{[(2S)-oxetan-2-yl]methyl}-1H-1,3-benzodiazole-6-carboxylic acid: 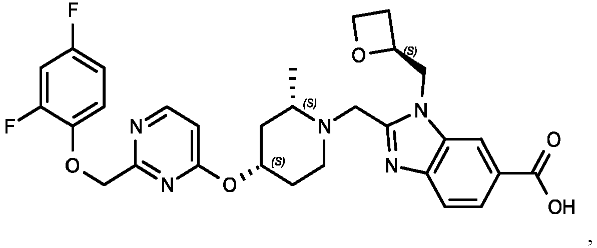 or a pharmaceutically acceptable salt or solvate thereof.
In a further aspect, the invention provides a pharmaceutical composition comprising the compound of the invention and one or more pharmaceutically acceptable carriers, excipients, or diluents.
In a further aspect, the invention provides the compound or the composition of the invention for use in therapy.
In a further aspect, the invention provides the compound or the composition of the invention for use in the treatment of type 2 diabetes.

### SUMMARY OF THE DISCLOSURE

Provided herein are GLP-1 receptor modulator compounds of Formulas (**I**)-(**XVIII**), (**XXV**) - (**XXVII**), (**XXX**), (**XXXI**), (**XXXIX**), and (**XL**-**XLVII**), and sub-formulas thereof, compositions comprising the compounds, methods of producing the compounds, and methods of using the compounds, and compositions in treatment, and diagnosis. The compounds of Formula (**I**)-(**XVIII**), (**XXV**) - (**XXVII**), (**XXX**), (**XXXI**), (**XXXIX**), and (**XL-XLVII**), and sub-formulas and instances thereof, are useful for modulating the activity of GLP-1 receptor. In certain instances, the compounds can be used to agonize the activity of GLP-1 receptor. In certain instances, the compounds can be used for treating diseases, or conditions modulated by GLP-1 receptor.

In one aspect, provided is a compound of Formula (**I**), or a pharmaceutically acceptable salt, or stereoisomer thereof: 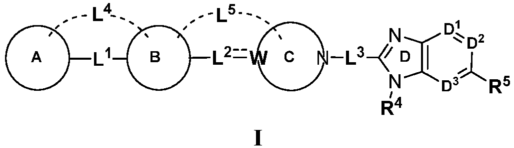
wherein, **A** is substituted or unsubstituted aryl, substituted or unsubstituted heteroaryl, substituted or unsubstituted cycloalkyl, -NMe₂, or -CF₃;
Ring **B** is substituted or unsubstituted aryl, substituted or unsubstituted heteroaryl, or substituted or unsubsuted bicyclic;
Ring **C** is substituted or unsubstituted heterocycloalkyl, or substituted or unsubstituted heterocycloalkenyl, each comprising at least one N, linked to **L³** as depicted;
zero, one, or two of **D¹**, **D²**, and **D³** are N, and the rest are CH, or C**R⁶**;
**W** is N, C**R¹⁴**, or C; when **W** is C**R¹⁴**, the adjacent dashed line indicates a single bond; when **W** is C, the adjacent dashed line indicates a double bond or **L²** is -C(H)=;
**L¹** is selected from the group consisting of a bond, -O-, -CH₂-, -NH-, -N(Me)-, -N(Et)-, -OCH₂- , -OC(H,Me)-, -CH₂O-, -NHCH₂-, -N(Me)CH₂-, and -SO₂NH-;
**L²** is selected from the group consisting of a bond, -C(O)-, -CH₂-, -C(H)=, -SO₂-, -O-, -CF₂-. -CHF-, -CH(OH)-, -NH-, -N(Me)-, and 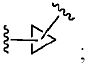
**L³** is -CH₂-, or -C(O)-;
**L⁴** is absent, or **L⁴** together with **L¹** and Rings **A** and **B** form a fused tricycle, and **L⁵** is absent;
**L⁵** is absent, or **L⁵** together with **L²** and Rings **B** and **C** form a fused tricycle, and **L⁴** is absent;
**R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 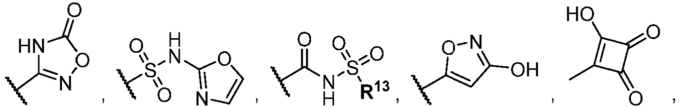 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen, or alkyl;
**R¹²** is hydrogen, or alkyl;
**R¹³** is hydrogen, or alkyl;
**R¹⁴** is hydrogen, cyano, halo, hydroxyl, alkyl, haloalklyl, methyl, CH₂F, or CH₂OH;
wherein when **L¹** is a -CH₂O-, **L⁴** is absent, and **L⁵** is absent, then either **L²** is -C(O)-, -CH₂-, - C(H)=, -SO₂-, -O-, -NH-, -N(Me)-, or 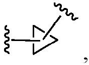 or **L³** is -C(O)-, or both.

In one aspect, provided is a compound of Formula (Ia), or a pharmaceutically acceptable salt, or stereoisomer thereof: 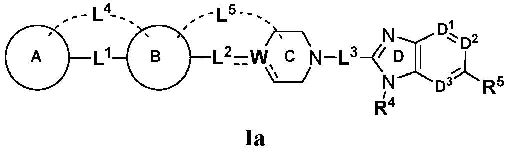
wherein, **A** is substituted or unsubstituted aryl, substituted or unsubstituted heteroaryl, substituted or unsubstituted cycloalkyl, -NMe₂, or -CF₃;
Ring **B** is substituted or unsubstituted aryl, or substituted or unsubstituted heteroaryl;
Ring **C** is further unsubstituted or further substituted, and/or bridged;
zero, one, or two of **D¹**, **D²**, and **D³** are N, and the rest are CH or C**R⁶**;
**W** is N, CH, or C; when **W** is CH the adjacent dashed lines indicate single bonds;
   when **W** is C, one adjacent dashed line indicates a double bond, for instance **L²** is - C(H)=;
**L¹** is selected from the group consisting of -O-, -CH₂-, -NH-, -N(Me)-, -N(Et)-, -OCH₂-, - OC(H,Me)-, -CH₂O-, -NHCH₂-, -N(Me)CH₂-, and -SO₂NH-;
**L²** is selected from the group consisting of a bond, -C(O)-, -CH₂-, -C(H)=, -CF₂-, -SO₂-, -O-, -NH-, -N(Me)-, and 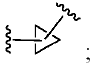
**L³** is -CH₂-, or -C(O)-;
**L⁴** is absent, or **L⁴** together with **L¹** and Rings **A** and **B** form a fused tricycle, and **L⁵** is absent;
**L⁵** is absent, or **L⁵** together with **L²** and Rings **B** and **C** form a fused tricycle, and **L⁴** is absent;
**R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 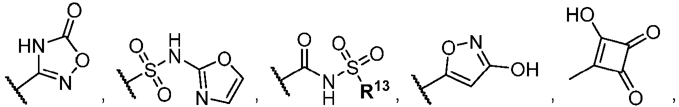 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen or alkyl;
**R¹²** is hydrogen or alkyl; and
**R¹³** is hydrogen or alkyl;
wherein, when **L¹** is -CH₂O-, **L⁴** is absent, and **L⁵** is absent, then either **L²** is -C(O)-, -CH₂-, - C(H)=, -SO₂-, -O-, -NH-, -N(Me)-, or 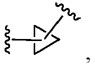 or **L³** is -C(O)-, or both.

In one aspect, provided is a compound of Formula **(XXX),** or a pharmaceutically acceptable salt, or stereoisomer thereof: 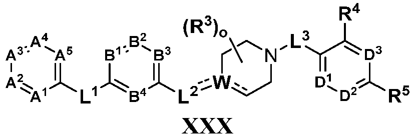
Wherein, **W** is N, CH, or C; when **W** is CH, the adjacent dashed lines indicate single bonds;
when **W** is C, one adjacent dashed line indicates a double bond, for instance **L²** is -C(H)=;
zero, one, or two of **A¹**, **A²**, **A³**, **A⁴**, and **A⁵** are N, and the rest are CH or C**R¹**;
zero, one, or two of **B¹**, **B²**, **B³**, and **B⁴** are N, and the rest are CH or C**R²**;
zero, one, or two of **D¹**, **D²**, and **D³** are N, and the rest are CH or C**R⁶**;
**L¹** is selected from the group consisting of -O-, -CH₂-, -NH-, -N(Me)-, -N(Et)-, -OCH₂-, - CH₂O-, -NHCH₂-, -N(Me)CH₂-, and -SO₂NH-;
**L²** is selected from the group consisting of a bond, -C(O)-, -CH₂-. -C(H)=, -SO₂-, -O-, -CF₂-, -CHF-, -CH(OH)-, -NH-, -N(Me)-, and 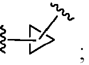
**L³** is -CH₂-, or -C(O)-;
each **R¹** is independently selected from the group consisting of alkyl, substituted alkyl, halo, cyano, azido, alkoxy, haloalkoxy, and **R¹¹R¹²**NCO-;
each **R²** is independently selected from the group consisting of alkyl, substituted alkyl, halo, cyano, azido, alkoxy, haloalkoxy, and **R¹¹R¹²**NCO-;
each **R³** is independently selected from the group consisting of alkyl, substituted alkyl, halo, cyano, azido, alkoxy, haloalkoxy, and **R¹¹R¹²**NCO-;
**R⁴** is unsubstituted alkyl, substituted alkyl, unsubstituted heteroalkyl, substituted heteroalkyl, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 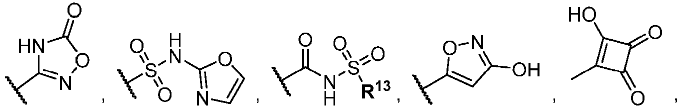 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen, or alkyl;
**R¹²** is hydrogen, or alkyl;
**R¹³** is hydrogen, or alkyl; and
o is an integer from 0 to 4.

In one aspect provided herein is a compound of Formula **XXXI,** or a pharmaceutically acceptable salt, or stereoisomer thereof: 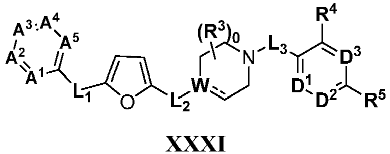
wherein **W** is N, CH, or C; when **W** is CH, the adjacent dashed lines indicate single; zero, one, or two of **A¹**, **A²**, **A³**, **A⁴**, and **A⁵** are N, and the rest are CH, or C**R¹**;
zero, one, or two of **D¹**, **D²**, and **D³** are N, and the rest are CH, or C**R⁶**;
each **R¹** is independently selected from the group consisting of alkyl, substituted alkyl, halo, cyano, azido, alkoxy, haloalkoxy, and **R¹¹R¹²**NCO-;
**L¹** is selected from the group consisting of -O-, -CH₂-, -NH-, -N(Me)-, -N(Et)-, -OCH₂-, - CH₂O-, -NHCH₂-, -N(Me)CH₂-, and -SO₂NH-;
**L²** is selected from the group consisting of a bond, -C(O)-, -CH₂-. -C(H)=, -SO₂-, -O-, -CF₂-, -CHF-, -CH(OH)-, -NH-, -N(Me)-, and 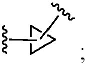
**L³** is -CH₂-, or -C(O)-;
each **R³** is independently selected from the group consisting of alkyl, substituted alkyl, halo, cyano, azido, alkoxy, haloalkoxy, and **R¹¹R¹²**NCO-;
**R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 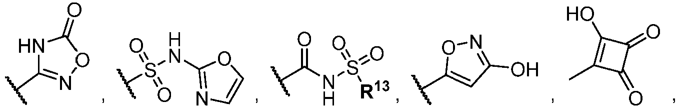 and tetrazolyl;
each **R⁶** is independently selected from the group consisting of F, and methyl;
**R¹¹** is hydrogen, or alkyl;
**R¹²** is hydrogen, or alkyl;
**R¹³** is hydrogen, or alkyl; and
**o** is 1, 2, 3, or 4.

In certain aspects, the compounds are useful in methods of treatment and prevention of metabolic diseases, and conditions, methods of detection of metabolic diseases, and conditions, and methods of diagnosis of metabolic diseases, and conditions. Throughout this disclosure, references to methods of treatment by therapy and methods of diagnosis are to be interpreted as references to compounds and pharmaceutical compositions of the present disclosure for use in those methods of treatment and methods of diagnosis.

In another aspect, provided are compositions comprising a compound of Formula (**I**), (**Ia**), (**XXX**), or (**XXXI**). In some instances, the compositions are pharmaceutical compositions. Any suitable pharmaceutical composition may be used. In a further aspect, provided herein is a kit comprising the compound of Formula (**I**), (**Ia**), (**XXX**), or (**XXXI**), or instances thereof, or a pharmaceutical composition thereof.

In another aspect, provided herein are methods of using the compounds or the compositions described herein. In some instances, the methods are for treatment. In some instances, the methods are diagnostic methods. In some instances, the methods are analytical methods. In some instances, the compounds, or compositions described herein are used to treat a disease, or condition. In some aspects, the disease, or condition is selected from metabolic diseases, or conditions. In certain instances, the disease is type 2 diabetes.

Also provided herein is the use of compounds described herein, and compositions thereof, for the treatment of a metabolic disease, or condition. Also provided herein is the use of compounds described herein, and compositions thereof, for the treatment of type 2 diabetes.

### BRIEF DESCRIPTION OF THE FIGURES

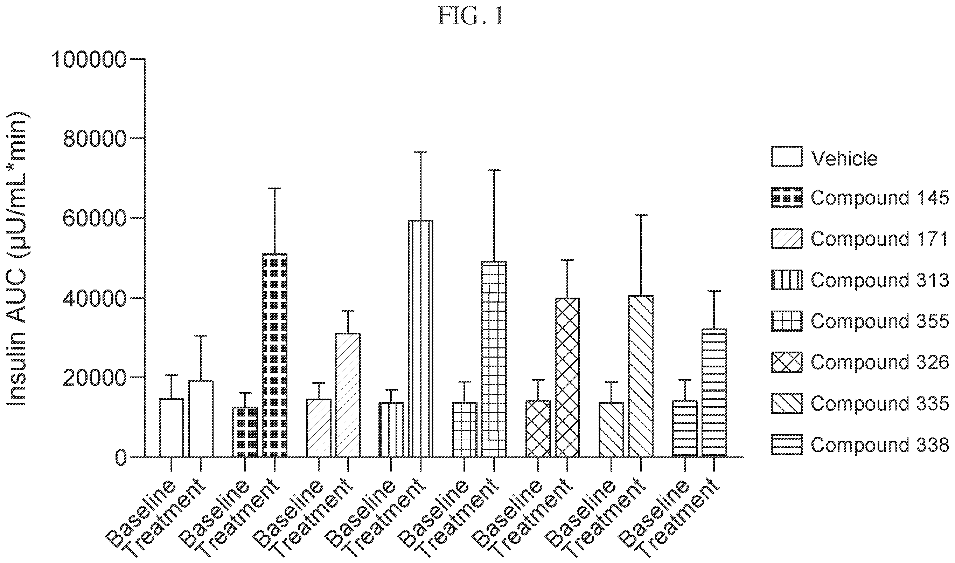

FIG. 1 provides intravenous glucose tolerance test (IVGTT) in cynomolgus monkeys.

### DESCRIPTION OF EXEMPLARY INSTANCES

Described herein are GLP-1 receptor compounds useful for treating metabolic diseases or conditions, such as type 2 diabetes.

### Definitions

Unless otherwise defined, all terms of art, notations and other scientific terminology used herein are intended to have the meanings commonly understood by those of skill in the art to which this disclosure pertains. In some cases, terms with commonly understood meanings are defined herein for clarity, and/or ready reference. The techniques and procedures described or referenced herein are generally well understood, and are commonly employed using conventional methodologies by those skilled in the art. As appropriate, procedures involving the use of commercially available kits, and reagents are generally carried out in accordance with manufacturer-defined protocols, and conditions unless otherwise noted.

As used herein, the singular forms "a," "an," and "the" include the plural referents unless the context clearly indicates otherwise.

The term "about" indicates and encompasses an indicated value, and a range above and below that value. In certain instances, the term "about" indicates the designated value ± 10%, ± 5%, or ± 1%. In certain instances, the term "about" indicates the designated value ± one standard deviation of that value. In certain instances, for example, logarithmic scales (e.g., pH), the term "about" indicates the designated value ± 0.3, ± 0.2, or ± 0.1.

When referring to the compounds provided herein, the following terms have the following meanings unless indicated otherwise. Unless defined otherwise, all technical and scientific terms used herein have the same meaning as is commonly understood by one of ordinary skill in the art. In the event that there is a plurality of definitions for a term herein, those in this section prevail unless stated otherwise.

"Alkoxy" and "alkoxyl," refer to the group -OR" where **R"** is alkyl or cycloalkyl. Alkoxy groups include, in certain instances, methoxy, ethoxy, n-propoxy, isopropoxy, n-butoxy, tert-butoxy, sec-butoxy, n-pentoxy, n-hexoxy, 1,2-dimethylbutoxy, and the like.

The term "alkoxyamine," as used herein, refers to the group -alkylene-O-NH₂, wherein alkylene is as defined herein. In some instances, alkoxyamine groups can react with aldehydes to form oxime residues. Examples of alkoxyamine groups include -CH₂CH₂-O-NH₂ and -CH₂-O-NH₂.

The term "alkyl," as used herein, unless otherwise specified, refers to a saturated straight, or branched hydrocarbon. In certain instances, the alkyl group is a primary, secondary, or tertiary hydrocarbon. In certain instances, the alkyl group includes one to ten carbon atoms (i.e., C₁ to C₁₀ alkyl). In certain instances, the alkyl is a lower alkyl, for example, C₁₋₆ alkyl, and the like. In certain instances, the alkyl group is selected from the group consisting of methyl, ethyl, propyl, isopropyl, butyl, isobutyl, secbutyl, t-butyl, pentyl, isopentyl, neopentyl, hexyl, isohexyl, 3-methylpentyl, 2,2-dimethylbutyl, and 2,3-dimethylbutyl. In certain instances, "substituted alkyl" refers to an alkyl substituted with one, two, or three groups independently selected from a halogen (e.g., fluoro (F), chloro (Cl), bromo (Br), or iodo (I)), alkyl, haloalkyl, hydroxyl, amino, alkylamino, alkoxy, aryl, heteroaryl, cycloalkyl, cyano, oxo, alkyne, and heterocycloalkylalkylene. In some instances, alkyl is unsubstituted.

The term "alkylene," as used herein, unless otherwise specified, refers to a divalent alkyl group, as defined herein. "Substituted alkylene" refers to an alkylene group substituted as described herein for alkyl. In some instances, alkylene is unsubstituted.

"Alkenyl" refers to an olefinically unsaturated hydrocarbon group, in certain instances, having up to about eleven carbon atoms, or from two to six carbon atoms (e.g., "lower alkenyl"), which can be straight-chained or branched, and having at least one, or from one to two sites of olefinic unsaturation. "Substituted alkenyl" refers to an alkenyl group substituted as described herein for alkyl.

"Alkenylene" refers to a divalent alkenyl as defined herein. Lower alkenylene is, for example, C₂-C₆-alkenylene.

"Alkynyl" refers to acetylenically unsaturated hydrocarbon groups, in certain instances, having up to about eleven carbon atoms, or from two to six carbon atoms (e.g., "lower alkynyl"), which can be straight-chained or branched, and having at least one, or from one to two sites of acetylenic unsaturation. Non-limiting examples of alkynyl groups include acetylene (-C≡CH), propargyl (-CH₂C≡CH), and the like. "Substituted alkynyl" refers to an alkynyl group substituted as described herein for alkyl.

"Alkynylene" refers to a divalent alkynyl as defined herein. Lower alkynylene is, for example, C₂-C₆-alkynylene.

"Amino" refers to -NH₂.

The term "alkylamino," as used herein, and unless otherwise specified, refers to the group -NH**R"** where **R"** is, for example, C₁₋₁₀alkyl, as defined herein. In certain instances, alkylamino is C₁₋₆alkylamino.

The term "dialkylamino," as used herein, and unless otherwise specified, refers to the group -N**R"R"** where, each **R"** is independently C₁₋₁₀alkyl, as defined herein. In certain instances, dialkylamino is di-C₁₋₆alkylamino.

The term "aryl," as used herein, and unless otherwise specified, refers to phenyl, biphenyl, or naphthyl. The term includes both substituted and unsubstituted moieties. An aryl group can be substituted with any described moiety including, but not limited to, one or more moieties (e.g., in some instances one, two, or three moieties) selected from the group consisting of halogen (e.g., fluoro (F), chloro (Cl), bromo (Br), or iodo (I)), alkyl, haloalkyl, hydroxyl, amino, alkylamino, arylamino, alkoxy, aryloxy, nitro, cyano, sulfonic acid, sulfate, phosphonic acid, phosphate, and phosphonate, wherein each moiety is independently either unprotected, or protected as necessary, as would be appreciated by those skilled in the art (e.g., Greene, et al., Protective Groups in Organic Synthesis, John Wiley and Sons, Second Edition, 1991); and wherein the aryl in the arylamino and aryloxy substituents are not further substituted.

The term "arylamino," as used herein, and unless otherwise specified, refers to an -N**R'R"** group where **R' is** hydrogen, or C₁-C₆-alkyl; and **R"** is aryl, as defined herein.

The term "arylene," as used herein, and unless otherwise specified, refers to a divalent aryl group, as defined herein.

The term "aryloxy," as used herein, and unless otherwise specified, refers to an -O**R** group where **R** is aryl, as defined herein.

"Alkarylene" refers to an arylene group, as defined herein, wherein the aryl ring is substituted with one or two alkyl groups. "Substituted alkarylene" refers to an alkarylene, as defined herein, where the arylene group is further substituted, as defined herein for aryl.

"Aralkylene" refers to an -alkyl-arylene- or arylene-alkyl, for instance, a -C₁-C₂ alkyl-arylene-, -arylene-C₁-C₂ alkyl-, or - C₁-C₂ alkyl-arylene-C₁-C₂ alkyl- group, where arylene is as defined herein. "Substituted aralkylene" refers to an aralkylene, as defined herein, where the aralkylene group is substituted, as defined herein for aryl. "Substituted C₁-C₂ alkyl" refers to a C₁-C₂ alkyl group that is substituted as defined herein for an alkyl.

"Arylalkylene" refers to -alkyl-arylene- or arylene-alkyl, for instance, a -C₁-C₂ alkyl-arylene-, -arylene-C₁-C₂ alkyl -, or - C₁-C₂ alkyl-arylene-C₁-C₂ alkyl- group, where arylene is as defined herein. "Substituted arylalkylene" refers to an arylalkylene, as defined herein, where the arylalkylene group is substituted, as defined herein for aryl. "Substituted C₁-C₂ alkyl" refers to a C₁-C₂ alkyl group that is substituted as defined herein for an alkyl.

"Carboxyl" or "carboxy" refers to -C(O)OH or -COOH.

The term "cycloalkyl," as used herein, unless otherwise specified, refers to a saturated cyclic hydrocarbon. In certain instances, the cycloalkyl group may be a saturated, and/or bridged, and/or non-bridged, and/or a fused bicyclic group. In certain instances, the cycloalkyl group includes three to ten carbon atoms (i.e., C₃ to C₁₀ cycloalkyl). In some instances, the cycloalkyl has from three to fifteen carbons (C₃₋₁₅), from three to ten carbons (C₃₋₁₀), from three to seven carbons (C₃₋₇), or from three to six carbons (C₃-C₆) (i.e., "lower cycloalkyl"). In certain instances, the cycloalkyl group is cyclopropyl, cyclobutyl, cyclopentyl, cyclohexyl, cyclohexylmethyl, cycloheptyl, bicyclo[2.1.1]hexyl, bicyclo[2.2.1]heptyl, decalinyl, or adamantyl.

The term "cycloalkylene," as used herein refers to a divalent cycloalkyl group, as defined herein. In certain instances, the cycloalkylene group is cyclopropylene 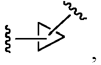 cyclobutylene 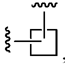 , cyclopentylene 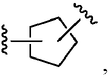 cyclohexylene 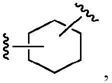 cycloheptylene 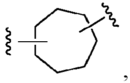 and the like. Lower cycloalkylene refers to a C₃-C₆-cycloalkylene.

The term "cycloalkylalkyl," as used herein, unless otherwise specified, refers to an alkyl group, as defined herein, substituted with one or two cycloalkyl, as defined herein.

The term "ester," as used herein, refers to -C(O)O**R**, or -COO**R**, where **R** is alkyl, as defined herein.

The term "haloalkyl" refers to an alkyl group, as defined herein, substituted with one, or more halogen atoms (e.g., in some instances one, two, three, four, or five) which are independently selected.

The term "heteroalkyl" refers to an alkyl, as defined herein, in which one or more carbon atoms are replaced by heteroatoms. As used herein, "heteroalkenyl" refers to an alkenyl, as defined herein, in which one, or more carbon atoms are replaced by heteroatoms. As used herein, "heteroalkynyl" refers to an alkynyl, as defined herein, in which one, or more carbon atoms are replaced by heteroatoms. Suitable heteroatoms include, but are not limited to, nitrogen (N), oxygen (O), and sulfur (S) atoms. Heteroalkyl, heteroalkenyl, and heteroalkynyl are optionally substituted. Examples of heteroalkyl moieties include, but are not limited to, aminoalkyl, sulfonylalkyl, and sulfinylalkyl. Examples of heteroalkyl moieties also include, but are not limited to, methylamino, methylsulfonyl, and methylsulfinyl. "Substituted heteroalkyl" refers to heteroalkyl substituted with one, two, or three groups independently selected from halogen (e.g., fluoro (F), chloro (Cl), bromo (Br), or iodo (I)), alkyl, haloalkyl, hydroxyl, amino, alkylamino, and alkoxy. In some instances, a heteroalkyl group may comprise one, two, three, or four heteroatoms. Those of skill in the art will recognize that a 4-membered heteroalkyl may generally comprise one, or two heteroatoms, a 5- or 6-membered heteroalkyl may generally comprise one, two, or three heteroatoms, and a 7- to 10-membered heteroalkyl may generally comprise one, two, three, or four heteroatoms.

The term "heteroalkylene," as used herein, refers to a divalent heteroalkyl, as defined herein. "Substituted heteroalkylene" refers to a divalent heteroalkyl, as defined herein, substituted as described for heteroalkyl.

The term "heterocycloalkyl" refers to a monovalent, monocyclic, or multicyclic non-aromatic ring system, wherein one, or more of the ring atoms are heteroatoms independently selected from oxygen (O), sulfur (S), and nitrogen (N) (e.g., where the nitrogen, or sulfur atoms may be optionally oxidized, and the nitrogen atoms may be optionally quaternized) and the remaining ring atoms of the non-aromatic ring are carbon atoms. In certain instances, heterocycloalkyl is a monovalent, monocyclic, or multicyclic fully-saturated ring system. In certain instances, the heterocycloalkyl group has from three to twenty, from three to fifteen, from three to ten, from three to eight, from four to seven, from four to eleven, or from five to six ring atoms. The heterocycloalkyl may be attached to a core structure at any heteroatom or carbon atom which results in the creation of a stable compound. In certain instances, the heterocycloalkyl is a monocyclic, bicyclic, tricyclic, or tetracyclic ring system, which may include a fused or bridged ring system and in which the nitrogen or sulfur atoms may be optionally oxidized, and/or the nitrogen atoms may be optionally quaternized. In some instances, heterocycloalkyl radicals include, but are not limited to, 2,5-diazabicyclo[2.2.2]octanyl, decahydroisoquinolinyl, dihydrobenzisoxazinyl, dihydrofuryl, dihydroisoindolyl, dihydropyranyl, dihydropyrazolyl, dihydropyrazinyl, dihydropyridinyl, dihydropyrimidinyl, dihydropyrrolyl, dioxolanyl, 1,4-dithianyl, furanonyl, imidazolidinyl, imidazolinyl, indolinyl, isothiazolidinyl, isoxazolidinyl, morpholinyl, octahydroindolyl, octahydroisoindolyl, oxazolidinonyl, oxazolidinyl, oxiranyl, piperazinyl, piperidinyl, 4-piperidonyl, pyrazolidinyl, pyrazolinyl, pyrrolidinyl, pyrrolinyl, quinuclidinyl, tetrahydrofuryl, tetrahydroisoquinolinyl, tetrahydropyranyl, tetrahydrothienyl, thiamorpholinyl, thiazolidinyl, tetrahydroquinolinyl, and 1,3,5-trithianyl. In certain instances, heterocycloalkyl may also be optionally substituted as described herein. In certain instances, heterocycloalkyl is substituted with one, two, or three groups independently selected from halogen (e.g., fluoro (F), chloro (Cl), bromo (Br), or iodo (I)), alkyl, haloalkyl, hydroxyl, amino, alkylamino, oxo, cyano, and alkoxy. In some instances, a heterocycloalkyl group may comprise one, two, three, or four heteroatoms. Those of skill in the art will recognize that a 4-membered heterocycloalkyl may generally comprise one or two heteroatoms, a 5 or 6-membered heterocycloalkyl may generally comprise one, two, or three heteroatoms, and a 7-to 10-membered heterocycloalkyl may generally comprise one, two, three, or four heteroatoms.

"Heterocycloalkylene" refers to a divalent heterocycloalkyl as defined herein.

The term "heteroaryl" refers to a monovalent monocyclic aromatic group, and/or multicyclic aromatic group, wherein at least one aromatic ring contains one, or more heteroatoms independently selected from oxygen, sulfur, and nitrogen in the ring. Each ring of a heteroaryl group can contain one, or two oxygen atoms, one, or two sulfur atoms, and/or one to four nitrogen atoms, provided that the total number of heteroatoms in each ring is four, or less and each ring contains at least one carbon atom. In certain instances, the heteroaryl has from five to twenty, from five to fifteen, or from five to ten ring atoms. A heteroaryl may be attached to the rest of the molecule via a nitrogen or a carbon atom. In some instances, monocyclic heteroaryl groups include, but are not limited to, furanyl, imidazolyl, isothiazolyl, isoxazolyl, oxadiazolyl, oxazolyl, pyrazinyl, pyrazolyl, pyridazinyl, pyridyl, pyrimidinyl, pyrrolyl, triazolyl, thiadiazolyl, thiazolyl, thienyl, tetrazolyl, and triazinyl. Examples of bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyridyl, imidazopyridinyl, imidazothiazolyl, indolizinyl, indolyl, indazolyl, isobenzofuranyl, isobenzothienyl, isoindolyl, isoquinolinyl, naphthyridinyl, oxazolopyridinyl, phthalazinyl, pteridinyl, purinyl, pyridopyridyl, pyrrolopyridyl, quinolinyl, quinoxalinyl, quinazolinyl, thiadiazolopyrimidyl, and thienopyridyl. Examples of tricyclic heteroaryl groups include, but are not limited to, acridinyl, benzindolyl, carbazolyl, dibenzofuranyl, perimidinyl, phenanthrolinyl, phenanthridinyl, phenarsazinyl, phenazinyl, phenothiazinyl, phenoxazinyl, and xanthenyl. In certain instances, heteroaryl may also be optionally substituted as described herein. "Substituted heteroaryl" is a heteroaryl substituted as defined for aryl.

The term "heteroarylene" refers to a divalent heteroaryl group, as defined herein. "Substituted heteroarylene" is a heteroarylene substituted as defined for aryl.

The term "heteroarylalkylene" refers to -alkyl-heteroarylene- or -heteroarylene-alkyl, for instance, a a -C₁-C₂ alkyl-heteroarylene-, -heteroarylene-C₁-C₂ alkyl-, or -C₁-C₂ alkyl-heteroarylene-C₁-C₂ alkyl- group, where heteroarylene is as defined herein. "Substituted heteroarylalkylene" refers to a heteroarylalkylene, as defined herein, where the heteroarylalkylene group is substituted, as defined herein for aryl. "Substituted C₁-C₂ alkyl" refers to a C₁-C₂ alkyl group that is substituted as defined herein for an alkyl.

As used herein, an "oxo" refers to =O.

The term "protecting group," as used herein, and unless otherwise specified, refers to a group that is added to an oxygen, nitrogen, or phosphorus atom to prevent its further reaction, or for other purposes. A wide variety of oxygen, and nitrogen protecting groups are known to those skilled in the art of organic synthesis. (See, e.g., Greene, et al., Protective Groups in Organic Synthesis, John Wiley and Sons, Fourth Edition, 2006).

"Pharmaceutically acceptable salt" refers to any salt of a compound provided herein which retains its biological properties, and which is not toxic or otherwise undesirable for pharmaceutical use. Such salts may be derived from a variety of organic, and inorganic counter-ions well known in the art. Such salts include, but are not limited to (1) acid addition salts formed with organic, or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, tert-butylacetic, lauryl sulfuric, gluconic, glutamic, hydroxynaphthoic, salicylic, stearic, cyclohexylsulfamic, quinic, muconic acid, and the like; or (2) salts formed when an acidic proton present in the parent compound either (a) is replaced by a metal ion, for example, an alkali metal ion, an alkaline earth ion, or an aluminum ion, or alkali metal, or alkaline earth metal hydroxides, such as sodium, potassium, calcium, magnesium, aluminum, lithium, zinc, and barium hydroxide, or ammonia; or (b) coordinates with an organic base, such as aliphatic, alicyclic, or aromatic organic amines, including, without limitation, ammonia, methylamine, dimethylamine, diethylamine, picoline, ethanolamine, diethanolamine, triethanolamine, ethylenediamine, lysine, arginine, ornithine, choline, *N*,*N'*-dibenzylethylene-diamine, chloroprocaine, procaine, N-benzylphenethylamine, N-methylglucamine piperazine, tris(hydroxymethyl)-aminomethane, tetramethylammonium hydroxide, and the like.

Pharmaceutically acceptable salts further include, by way of example and without limitation, sodium, potassium, calcium, magnesium, ammonium, and tetraalkylammonium salts, and the like, and when the compound contains a basic functionality, salts of non-toxic organic, or inorganic acids, such as hydrohalides, for example, hydrochloride and hydrobromide, sulfate, phosphate, sulfamate, nitrate, acetate, trifluoroacetate, trichloroacetate, propionate, hexanoate, cyclopentylpropionate, glycolate, glutarate, pyruvate, lactate, malonate, succinate, sorbate, ascorbate, malate, maleate, fumarate, tartarate, citrate, benzoate, 3-(4-hydroxybenzoyl)benzoate, picrate, cinnamate, mandelate, phthalate, laurate, methanesulfonate (mesylate), ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, tert-butylacetate, lauryl sulfate, gluconate, glutamate, hydroxynaphthoate, salicylate, stearate, cyclohexylsulfamate, quinate, muconate, and the like.

The term "substantially free of" or "substantially in the absence of" with respect to a composition refers to a composition that includes at least 85%, or 90% by weight, in certain instances 95%, 98 %, 99%, or 100% by weight; or in certain instances, 95%, 98%, 99%, or 100% of the designated enantiomer or diastereomer of a compound. In certain instances, in the methods and compounds provided herein, the compounds are substantially free of one of two enantiomers. In certain instances, in the methods and compounds provided herein, the compounds are substantially free of one of two diastereomers. In certain instances, in the methods, and compounds provided herein, the compounds are substantially free of enantiomers (i.e., a racemic, or 50:50 mixture of compounds).

Similarly, the term "isolated" with respect to a composition refers to a composition that includes at least 85%, 90%, 95%, 98%, or 99% to 100% by weight, of the compound, the remainder comprising other chemical species, enantiomers or diastereomers.

"Solvate" refers to a compound provided herein, or a salt thereof, that further includes a stoichiometric, or non-stoichiometric amount of solvent bound by non-covalent intermolecular forces. Where the solvent is water, the solvate is a hydrate.

"Isotopic composition" refers to the amount of each isotope present for a given atom, and "natural isotopic composition" refers to the naturally occurring isotopic composition, or abundance for a given atom. Atoms containing their natural isotopic composition may also be referred to herein as "non-enriched" atoms. Unless otherwise designated, the atoms of the compounds recited herein are meant to represent any stable isotope of that atom. For example, unless otherwise stated, when a position is designated specifically as hydrogen (H), the position is understood to have hydrogen at its natural isotopic composition.

"Isotopic enrichment" refers to the percentage of incorporation of an amount of a specific isotope at a given atom in a molecule in the place of that atom's natural isotopic abundance. For example, deuterium (D) enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%. The isotopic enrichment of the compounds provided herein can be determined using conventional analytical methods known to one of ordinary skill in the art, including mass spectrometry and nuclear magnetic resonance spectroscopy.

"Isotopically enriched" refers to an atom having an isotopic composition other than the natural isotopic composition of that atom. "Isotopically enriched" may also refer to a compound containing at least one atom having an isotopic composition other than the natural isotopic composition of that atom.

As used herein, "alkyl," "alkylene," "alkylamino," "dialkylamino," "cycloalkyl," "aryl," "arylene," "alkoxy," "amino," "carboxyl," "heterocycloalkyl," "heteroaryl," "heteroarylene," "carboxyl," and "amino acid" groups optionally comprise deuterium (D) at one, or more positions where hydrogen (H) atoms are present, and wherein the deuterium composition of the atom or atoms is other than the natural isotopic composition.

Also as used herein, "alkyl," "alkylene," "alkylamino," "dialkylamino," "cycloalkyl," "aryl," "arylene," "alkoxy," "amino," "carboxyl," "heterocycloalkyl," "heteroaryl," "heteroarylene," "carboxyl," and "amino acid" groups optionally comprise carbon-13 (¹³C) at an amount other than the natural isotopic composition.

As used herein, term "EC₅₀" refers to a dosage, concentration, or amount of a particular test compound that elicits a dose-dependent response at 50% of maximal expression of a particular response that is induced, provoked, or potentiated by the particular test compound.

As used herein, and unless otherwise specified, the term "IC₅₀" refers to an amount, concentration, or dosage of a particular test compound that achieves a 50% inhibition of a maximal response in an assay that measures such response.

As used herein, the terms "subject", and "patient" are used interchangeably. The terms "subject", and "subjects" refer to an animal, such as a mammal including a non-primate (e.g., a cow, pig, horse, cat, dog, rat, and mouse), and a primate (e.g., a monkey, such as a cynomolgous monkey, a chimpanzee, and a human), and in certain instances, a human. In certain instances, the subject is a farm animal (e.g., a horse, a cow, a pig, etc.), or a pet (e.g., a dog or a cat). In certain instances, the subject is a human.

As used herein, the terms "therapeutic agent", and "therapeutic agents" refer to any agent(s) which can be used in the treatment, or prevention of a disorder, or one or more symptoms thereof. In certain instances, the term "therapeutic agent" includes a compound provided herein. In certain instances, a therapeutic agent is an agent which is known to be useful for, or has been, or is currently being used for the treatment, or prevention of a disorder, or one, or more symptoms thereof.

"Therapeutically effective amount" refers to an amount of a compound, or composition that, when administered to a subject for treating a condition, is sufficient to effect such treatment for the condition. A "therapeutically effective amount" can vary depending on, *inter alia,* the compound, the disease or disorder, and its severity, and the age, weight, *etc.,* of the subject to be treated.

"Treating" or "treatment" of any disease, or disorder refers, in certain instances, to ameliorating a disease, or disorder that exists in a subject. In another instance, "treating", or "treatment" includes ameliorating at least one physical parameter, which may be indiscernible by the subject. In yet another instance, "treating", or "treatment" includes modulating the disease, or disorder, either physically (e.g., stabilization of a discernible symptom), or physiologically (e.g., stabilization of a physical parameter), or both. In yet another instance, "treating", or "treatment" includes delaying, or preventing the onset of the disease, or disorder, or delaying, or preventing recurrence of the disease, or disorder. In yet another instance, "treating", or "treatment" includes the reduction or elimination of either the disease, or disorder, or retarding the progression of the disease, or disorder, or of one, or more symptoms of the disease, or disorder, or reducing the severity of the disease, or disorder, or of one, or more symptoms of the disease, or disorder.

As used herein, the terms "prophylactic agent", and "prophylactic agents" as used refer to any agent(s) which can be used in the prevention of a disorder, or one or more symptoms thereof. In certain instances, the term "prophylactic agent" includes a compound provided herein. In certain other instances, the term "prophylactic agent" does not refer a compound provided herein. For example, a prophylactic agent is an agent which is known to be useful for, or has been or is currently being used to prevent, or impede the onset, development, progression, and/or severity of a disorder.

As used herein, the phrase "prophylactically effective amount" refers to the amount of a therapy (e.g., prophylactic agent) which is sufficient to result in the prevention, or reduction of the development, recurrence, or onset of one or more symptoms associated with a disorder, or to enhance or improve the prophylactic effect(s) of another therapy (e.g., another prophylactic agent).

### Compounds of Formulae (I)-(XVIII), (XXV) - (XXVII), (XXX), (XXXI), (XXXIX), and (XL-XLVII)

Provided herein are GLP-1 receptor compounds useful for modulating one, or more properties of GLP-1 receptor. The compounds can be prepared as described herein and used for therapy, or diagnosis. In certain instances, the therapy is the treatment of a metabolic disease or condition. In certain instances, the therapy is the treatment of type 2 diabetes.

The instances described herein include the recited compounds as well as pharmaceutically acceptable salts, hydrates, solvates, stereoisomers, tautomers, and/or mixtures thereof.

In certain instances, provided is a compound of Formula (**I**), or a pharmaceutically acceptable salt, tautomer, stereoisomer, and/or mixture of stereoisomers thereof, wherein **A** is substituted or unsubstituted aryl, substituted or unsubstituted heteroaryl, substituted or unsubstituted cycloalkyl, -NMe₂, or -CF₃; Ring **B** is substituted or unsubstituted aryl, or substituted or unsubstituted heteroaryl; Ring **C** is further unsubstituted or further substituted, and/or bridged; zero, one, or two of **D¹, D²,** and **D³** are N, and the rest are CH, or C**R⁶**; **L¹** is selected from the group consisting of a bond, -O-, -CH₂-, -NH-, -N(Me)-, -N(Et)-, -OCH₂-, - OC(H,Me)-, -CH₂O-, -NHCH₂-, -N(Me)CH₂-, and -SO₂NH-; **L²** is selected from the group consisting of a bond, -C(O)-, -CH₂-, -C(H)=, -CF₂-, -SO₂-, -O-, -CF₂-. -CHF-, -CH(OH)-, - NH-, -N(Me)-, and 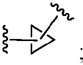 **L³** is -CH₂- or -C(O)-; **L⁴** is absent, or **L⁴** together with **L¹** and Rings **A** and **B** form a fused tricycle, and **L⁵** is absent; **L⁵** is absent, or **L⁵** together with **L²** and Rings **B** and **C** form a fused tricycle, and **L⁴** is absent; **R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 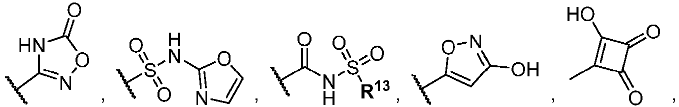 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; **R¹¹** is hydrogen, or alkyl; **R¹²** is hydrogen, or alkyl; and **R¹³** is hydrogen, or alkyl. In certain instances, **L²** is selected from the group consisting of a bond, -C(O)-, -CH₂-, -C(H)=, -CF₂-, -SO₂-, -O-, -CF₂-. -CHF-, -CH(OH)-, and 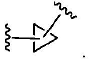 In certain instances, **L²** is selected from the group consisting of -NH- and - N(Me)-.

In certain instances of Formula (**I**), when **L¹** is -CH₂O-, **L⁴** is absent, and **L⁵** is absent, then either **L²** is -C(O)-, -CH₂-, -C(H)=, -SO₂-, -O-, -CF₂-, CHF-, -CHOH-, -NH-, - N(Me)-, or 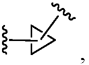 or **L³** is -C(O)-, or both. In certain instances, when **L¹** is -CH₂O-, **L⁴** is absent, and **L⁵** is absent, then **L²** is -C(O)-, -CH₂-, -C(H)=, -SO₂-, -O-, -NH-, -N(Me)-, or 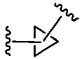 . In certain instances, when **L¹** is -CH₂O-, **L⁴** is absent, and **L⁵** is absent, then **L³** is - C(O)-. In certain instances, when **L¹** is -CH₂O-, **L⁴** together with **L¹** and Rings **A** and **B** form a fused tricycle. In certain instances, when **L¹** is -CH₂O-, **L⁵** together with **L²** and Rings **B** and **C** form a fused tricycle. In certain instances, when **L¹** is -CH₂O-, Ring C is substituted or unsubstituted azetidinyl, substituted or unsubstituted pyrrolidinyl, or substituted or unsubstituted piperidinyl.

In certain instances, **A** is a ring selected from phenyl and cyclohexyl. In certain instances, Ring **C** is selected from piperidine and piperazine. In certain instances, Ring **B** is selected from phenyl, furan, pyridine, pyrimidine, thiophene, oxazole, and benzofuran. In certain instances, **A** is phenyl and Ring **C** is piperazine. In certain instances, **A** is phenyl, and Ring **C** is piperidine. In certain instances, **A** is phenyl, and Ring **C** is piperazine, and Ring **B** is phenyl. In certain instances, **A** is phenyl, and Ring **C** is piperazine, and Ring **B** is furan. In certain instances, **A** is phenyl, and Ring **C** is piperidine, and Ring **B** is phenyl. In certain instances, **A** is phenyl, and Ring **C** is piperidine, and Ring **B** is furan.

In certain instances, **A** is a ring selected from phenyl and cyclohexyl. In certain instances, Ring **C** is selected from substituted piperidine, azetidine, and pyrrolidine. In certain instances, Ring **B** is selected from phenyl, furan, pyridine, pyrimidine, thiophene, oxazole, and benzofuran. In certain instances, **A** is phenyl and Ring **C** is piperazine. In certain instances, **A** is phenyl, and Ring **C** is piperidine. In certain instances, **A** is phenyl, and Ring **C** is substituted piperidine. In certain instances, **A** is phenyl, and Ring **C** is azetidine. In certain instances, **A** is phenyl, and Ring **C** is pyrrolidine. In certain instances, **A** is phenyl, Ring **C** is piperazine, and Ring **B** is phenyl. In certain instances, **A** is phenyl, Ring **C** is piperazine, and Ring **B** is furan. In certain instances, **A** is phenyl, Ring **C** is piperidine, and Ring **B** is phenyl. In certain instances, **A** is phenyl, Ring **C** is piperidine, and Ring **B** is furan. In certain instances, **A** is phenyl, Ring **C** is substituted piperidine, and Ring **B** is phenyl. In certain instances, **A** is phenyl, Ring **C** is substituted piperidine, and Ring **B** is furan. In certain instances, **A** is phenyl, Ring **C** is azetidine, and Ring **B** is phenyl. In certain instances, **A** is phenyl, Ring **C** is azetidine, and Ring **B** is furan. In certain instances, **A** is phenyl, Ring **C** is pyrrolidine, and Ring **B** is phenyl. In certain instances, **A** is phenyl, Ring **C** is pyrrolidine, and Ring **B** is furan.

In certain instances, provided is a compound of Formula **(Ia),** or a pharmaceutically acceptable salt, tautomer, stereoisomer, and/or mixture of stereoisomers thereof, wherein **A** is substituted or unsubstituted aryl, substituted or unsubstituted heteroaryl, substituted or unsubstituted cycloalkyl, -NMe₂, or -CF₃; Ring **B** is substituted or unsubstituted aryl, or substituted or unsubstituted heteroaryl; Ring **C** is further unsubstituted or further substituted, and/or bridged; zero, one, or two of **D¹**, **D²**, and **D³** are N, and the rest are CH, or C**R⁶**; **W** is N, CH, or C; when **W** is CH, the adjacent dashed lines indicate single bonds; when **W** is C, one adjacent dashed line indicates a double bond, for instance **L²** is -C(H)=; **L¹** is selected from the group consisting of -O-, -CH₂-, -NH-, -N(Me)-, -N(Et)-, -OCH₂-, -OC(H,Me)-, -CH₂O-, - NHCH₂-, -N(Me)CH₂-, and -SO₂NH-; **L²** is selected from the group consisting of a bond, - C(O)-, -CH₂-, -C(H)=, -SO₂-, -O-, -NH-, -N(Me)-, and 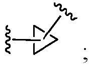 **L³** is -CH₂- or -C(O)-; **L⁴** is absent, or **L⁴** together with **L¹** and Rings **A** and **B** form a fused tricycle, and **L⁵** is absent; **L⁵** is absent, or **L⁵** together with **L²** and Rings **B** and **C** form a fused tricycle, and **L⁴** is absent; **R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 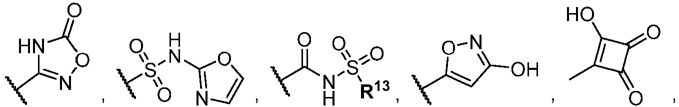 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; **R¹¹** is hydrogen, or alkyl; **R¹²** is hydrogen, or alkyl; and **R¹³** is hydrogen, or alkyl. In certain instances, **L²** is selected from the group consisting of a bond, -C(O)-, -CH₂-, -C(H)=, -CF₂-, -SO₂-, -O-, -CF₂-. -CHF-, -CH(OH)-, and 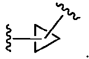 In certain instances, **L²** is selected from the group consisting of -NH- and -N(Me)-.

In certain instances of Formula (**Ia**), when **L¹** is -CH₂O-, **L⁴** is absent, and **L⁵** is absent, then either **L²** is -C(O)-, -CH₂-, -C(H)=, -SO₂-, -O-, -NH-, -N(Me)-, or 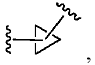 or**L³** is -C(O)-, or both. In certain instances, when **L¹** is -CH₂O-, **L⁴** is absent, and **L⁵** is absent, then **L²** is -C(O)-, -CH₂-, -C(H)=, -SO₂-, -O-, -NH-, -N(Me)-, or 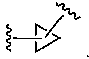 In certain instances, when **L¹** is -CH₂O-, **L⁴** is absent, and **L⁵** is absent, then **L²** is -C(O)-, -CH₂-, -C(H)=, -SO₂-, -O-, or 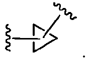 In certain instances, when **L¹** is -CH₂O-, **L⁴** is absent, and **L⁵** is absent, then **L³** is - C(O)-. In certain instances, when **L¹** is -CH₂O-, **L⁴** together with **L¹** and Rings **A** and **B** form a fused tricycle. In certain instances, when **L¹** is -CH₂O-, **L⁵** together with **L²** and Rings **B** and **C** form a fused tricycle.

In certain instances, **A** is a ring selected from phenyl, and cyclohexyl. In certain instances, Ring **C** is selected from piperidine, and piperazine. In certain instances, Ring **B** is selected from phenyl, furan, pyridine, pyrimidine, thiophene, oxazole, and benzofuran. In certain instances, **A** is phenyl and Ring **C** is piperazine. In certain instances, **A** is phenyl, and Ring **C** is piperidine. In certain instances, **A** is phenyl, and Ring **C** is piperazine, and Ring **B** is phenyl. In certain instances, **A** is phenyl, and Ring **C** is piperazine, and Ring **B** is furan. In certain instances, **A** is phenyl, and Ring **C** is piperidine, and Ring **B** is phenyl. In certain instances, **A** is phenyl, and Ring **C** is piperidine, and Ring **B** is furan.

In certain instances, provided is a compound of Formula **(XXX),** or a pharmaceutically salt, tautomer, stereoisomer, and/or mixture of stereoisomers thereof; wherein **W** is N, CH, or C; when **W** is CH, the adjacent dashed lines indicate single bonds; when **W** is C, one adjacent dashed line indicates a double bond, for instance **L²** is -C(H)=; zero, one, or two of **A¹**, **A²**, **A³**, **A⁴**, and **A⁵** are N, and the rest are CH, or C**R¹**; zero, one, or two of **B¹**, **B²**, **B³**, and **B⁴** are N, and the rest are CH, or C**R²**; zero, one, or two of **D¹**, **D²**, and **D³** are N, and the rest are CH, or C**R⁶**; **L¹** is selected from the group consisting of -O-, -CH₂-, -NH-, -N(Me)-, -N(Et)-, -OCH₂-, -CH₂O-, -NHCH₂-, -N(Me)CH₂-, and -SO₂NH-;
**L²** is selected from the group consisting of a bond, -C(O)-, -CH₂-. -C(H)=, -SO₂-, -O-, -NH-, - N(Me)-, and 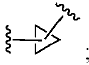 **; L³** is -CH₂- or -C(O)-; each **R¹** is independently selected from the group consisting of alkyl, substituted alkyl, halo, cyano, azido, alkoxy, haloalkoxy, and **R¹¹R¹²**NCO- ;each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is unsubstituted alkyl, substituted alkyl, unsubstituted heteroalkyl, substituted heteroalkyl, unsubstituted heterocycloalkylalkylene, or substituted heterocycloalkylalkylene; **R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 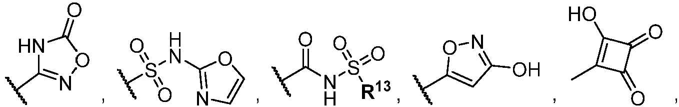 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; **R¹¹** is hydrogen or alkyl; **R¹²** is hydrogen, or alkyl; **R¹³** is hydrogen, or alkyl; and **o** is an integer from 0 to 4. In certain instances, the compound of Formula **(XXX)** is selected from compounds 82, 83, 91, 92, and 110, in Table 1.

In certain instances, provided is a compound of Formula **(XXXI),** or a pharmaceutically salt, tautomer, stereoisomer, and/or mixture of stereoisomers thereof; wherein **W** is N, CH, or C; when **W** is CH, the adjacent dashed lines indicate single; zero, one, or two of **A¹**, **A²**, **A³**, **A⁴**, and **A⁵** are N, and the rest are CH, or C**R¹**;
zero, one, or two of **D¹**, **D²**, and **D³** are N, and the rest are CH, or C**R⁶**;
each **R¹** is independently selected from the group consisting of alkyl, substituted alkyl, halo, cyano, azido, alkoxy, haloalkoxy, and **R¹¹R¹²**NCO-;
**L¹** is selected from the group consisting of -O-, -CH₂-, -NH-, -N(Me)-, -N(Et)-, -OCH₂-, - CH₂O-, -NHCH₂-, -N(Me)CH₂-, and -SO₂NH-;
**L²** is selected from the group consisting of a bond, -C(O)-, -CH₂-. -C(H)=, -SO₂-, -O-, -NH-, - N(Me)-, and 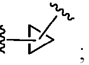
**L³** is -CH₂-, or -C(O)-;
each **R³** is independently selected from the group consisting of alkyl, substituted alkyl, halo, cyano, azido, alkoxy, haloalkoxy, and **R¹¹R¹²**NCO-;
**R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 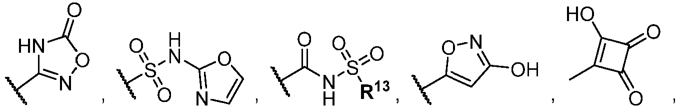 and tetrazolyl;
each **R⁶** is independently selected from the group consisting of F, and methyl;
**R¹¹** is hydrogen, or alkyl;
**R¹²** is hydrogen, or alkyl;
**R¹³** is hydrogen, or alkyl; and
**o** is 1, 2, 3, or 4. In certain instances, the compound of Formula **(XXXI)** is selected from compounds 97 and 107 in Table 1.

In certain instances, the compound of Formula **(I)** is according to Formula **(IIa),** or a pharmaceutically acceptable salt, tautomer, stereoisomer, and/or mixture of stereoisomers thereof: 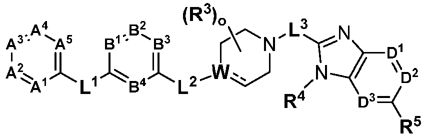 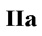 wherein zero, one, or two of **A¹**, **A²**, **A³**, **A⁴**, and **A⁵** are N, and the rest are CH, or C**R¹**; zero, one, or two of **B¹**, **B²**, **B³**, and **B⁴** are N, and the rest are CH, or C**R²**, wherein, in Formula **IIa B⁴** is absent; zero, one, or two of **D¹**, **D²**, and **D³** are N, and the rest are CH, or C**R⁶**; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 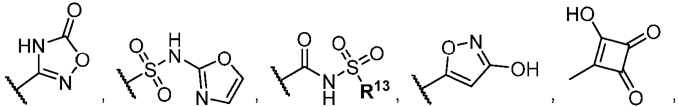 and tetrazolyl; each **R⁶** is independently selected from the group consisting of F, and methyl; each **R¹¹** is hydrogen, or alkyl; each **R¹²** is hydrogen or alkyl; each **R¹³** is hydrogen or alkyl; and **o** is 1, 2, 3, or 4.

In certain instances, the compound of Formula **(I)** is according to Formula **(IIb),** or a pharmaceutically acceptable salt, tautomer, stereoisomer, and/or mixture of stereoisomers thereof: 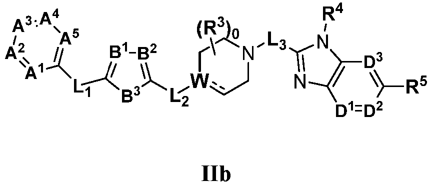 wherein zero, one, or two of **A¹**, **A²**, **A³**, **A⁴**, and **A⁵** are N, and the rest are CH, or C**R¹**; zero, one, or two of **B¹**, **B²**, **B³**, and **B⁴** are N, and the rest are CH or C**R²**, wherein, in Formula **IIb, B⁴** is absent; zero, one, or two of **D¹**, **D²**, and **D³** are N, and the rest are CH, or C**R⁶**; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 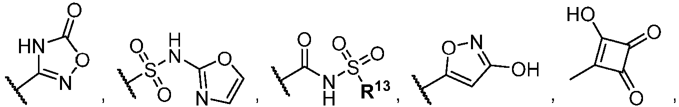 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl; and o is 1, 2, 3, or 4.

In certain instances, the compound of Formula **(I)** is according to Formula **(IIc),** or a pharmaceutically acceptable salt, tautomer, stereoisomer, and/or mixture of stereoisomers thereof: 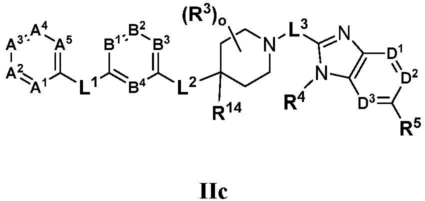 wherein zero, one, or two of **A¹**, **A²**, **A³**, **A⁴**, and **A⁵** are N, and the rest are CH, or C**R¹**; zero, one, or two of **B¹**, **B²**, **B³**, and **B⁴** are N, and the rest are CH or C**R²**, wherein, in Formula **IIc B⁴** is absent; zero, one, or two of **D¹**, **D²**, and **D³** are N, and the rest are CH, or C**R⁶**; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 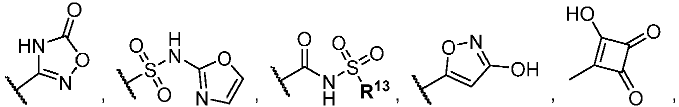 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl; **R¹⁴** is hydrogen, cyano, halo, hydroxyl, or methyl; and **o** is 1, 2, 3, or 4.

In certain instances, the compound of Formula (I) is according to Formula (**IId**), or a pharmaceutically acceptable salt, tautomer, stereoisomer, and/or mixture of stereoisomers thereof: 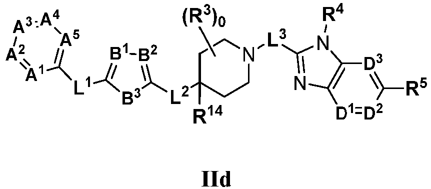 wherein zero, one, or two of **A¹**, **A²**, **A³**, **A⁴**, and **A⁵** are N, and the rest are CH, or C**R¹**; zero, one, or two of **B¹**, **B²**, **B³**, and **B⁴** are N, and the rest are CH or C**R²**, wherein, in Formula **IId B⁴** is absent; zero, one, or two of **D¹**, **D²**, and **D³** are N, and the rest are CH, or C**R⁶**; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 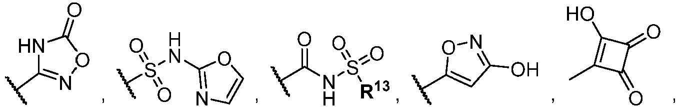 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl; **R¹⁴** is hydrogen, cyano, halo, hydroxyl, or methyl; and **o** is 1, 2, 3, or 4.

In certain instances, the compound of Formula **(I)** is according to Formula **(IIg),** or a pharmaceutically acceptable salt, tautomer, stereoisomer, and/or mixture of stereoisomers thereof: 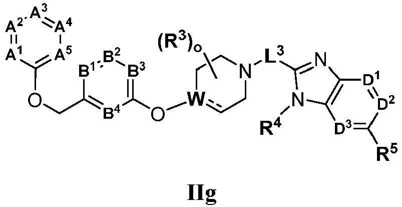 wherein zero, one, or two of **A¹**, **A²**, **A³**, **A⁴**, and **A⁵** are N, and the rest are CH, or C**R¹**; zero, one, or two of **B¹**, **B²**, **B³**, and **B⁴** are N, and the rest are CH or C**R²**, wherein, in Formula **IIg**, **B⁴** is absent; zero, one, or two of **D¹**, **D²**, and **D³** are N, and the rest are CH, or C**R⁶**; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 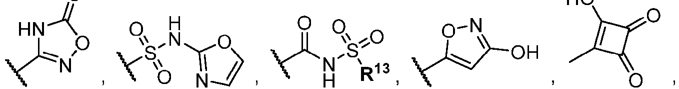 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl.

In certain instances, the compound of Formula **(I)** is according to Formula **(IIh),** or a pharmaceutically acceptable salt, tautomer, stereoisomer, and/or mixture of stereoisomers thereof: 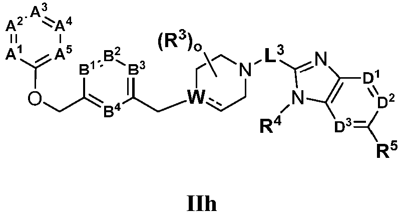 wherein zero, one, or two of **A¹**, **A²**, **A³**, **A⁴**, and **A⁵** are N, and the rest are CH, or C**R¹**; zero, one, or two of **B¹**, **B²**, **B³**, and **B⁴** are N, and the rest are CH or C**R²**, wherein, in Formula **IIg**, **B⁴** is absent; zero, one, or two of **D¹**, **D²**, and **D³** are N, and the rest are CH, or C**R⁶**; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 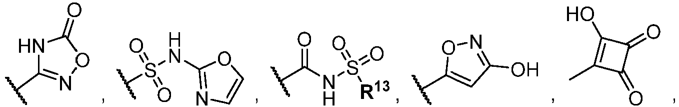 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl.

In certain instances, provided is a compound of Formula **(III),** or a pharmaceutically salt, tautomer, stereoisomer, and/or mixture of stereoisomers thereof: 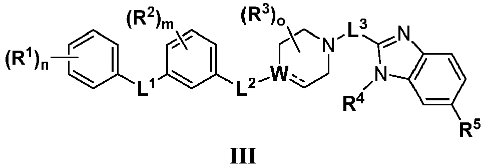 wherein **W** is N, C**R¹⁴**, or C; when **W** is C**R¹⁴**, the adjacent dashed line indicates a single bond; when **W** is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -CF₂-, -SO₂-, -O-, -NH-, -N(Me)-, and 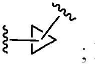 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 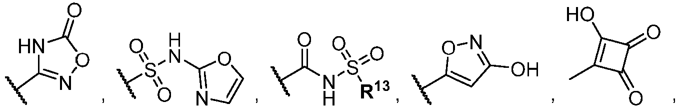 and tetrazolyl; each **R¹¹** is hydrogen, or alkyl; each **R¹²** is hydrogen, or alkyl; each **R¹³** is hydrogen, or alkyl; n is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 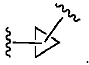 In certain instances, **L²** is selected from the group consisting of -NH- and -N(Me)-. In certain instances, **L2** is O. In certain instances, the compound of Formula **(III)** is selected from compounds 2, 3, 5-7, 10-18, 21-23, 25, 29, 32, 33, 35, 45, 46, 48, 50-52, 54, 56-58, 64-70, 75-80, 86, 89, 93, 95, 98, 100, 103-105, 111, 112, 115, 116, 122, 126, 130-133, 135, 137, 148, 149, 151, 157-159, 161, 162, 165, 166, 174, 187, 188, 202-208, 215-217, 221, 231, 232, 242, 243, 254, 255, 260, 263, 288, 294, 301, 318, 319, and 321 in Table 1.

In certain instances, provided is a compound of Formula **(IIIa),** or a pharmaceutically salt, tautomer, stereoisomer, and/or mixture of stereoisomers thereof: 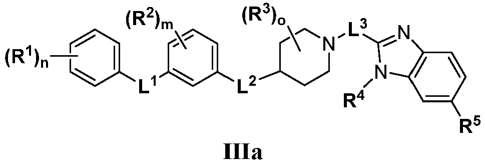 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -CF₂-. -CHF-, or -CH(OH)-, - NH-, -N(Me)-, and 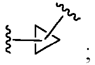 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of - COOH, -COCF₃, -C(OH)CF₃, -CONHCN, -CONHOH, CONHOMe, -CONHSO₂N(Me)₂, - PO₃H₂, -PO(Me)(OH), -SO₃H, -SO₂NH₂, -SO₂NHMe, -B(OH)₂, 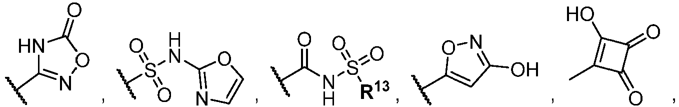 and tetrazolyl; each **R¹¹** is hydrogen or alkyl; each **R¹²** is hydrogen or alkyl; each **R¹³** is hydrogen or alkyl; n is an integer from 0 to 5; **m** is an integer from 0 to 4; **o** is an integer from 0 to 4. In certain instances, **L²** is selected from the group consisting of -NH- and -N(Me)-. In certain instances, **L2** is O. In certain instances, the compound of Formula **(IIIa)** is selected from compounds 103, 104, 111, 116, 126, 130-133, 135, 137, 148, 149, 151, 157-159, 161, 162, 165, 166, 174, 187, 188, 202-208, 215-217, 221, 231, 232, 242, 243, 254, 255, 260, 263, 288, 294, 301, 318, 319, and 321 in Table 1.

In certain instances, provided is a compound of Formula **(IV),** or a pharmaceutically salt, tautomer, stereoisomer, and/or mixture of stereoisomers thereof: 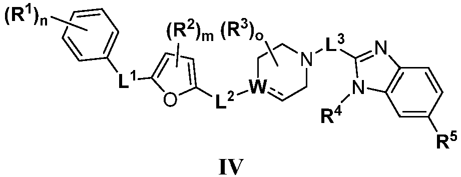 wherein **W** is N, C**R¹⁴**, or C; when **W** is C**R¹⁴**, the adjacent dashed line indicates a single bond; when **W** is **C,** the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 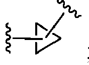 ; **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 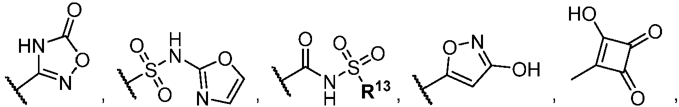 and tetrazolyl; each **R¹¹** is hydrogen or alkyl; each **R¹²** is hydrogen or alkyl; each **R¹³** is hydrogen or alkyl; n is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 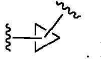 In certain instances, **L²** is selected from the group consisting of -NH- and -N(Me)-. In certain instances, the compound of Formula **(IV)** is selected from compounds 8, 9, 19, 20, 24, 26, 30, 31, 36-43, 47, 49, 55, 59, 63, 72-74, 81, 84, 94, 96, 108, and 109 in Table 1.

In certain instances, provided is a compound of Formula **(V),** or a pharmaceutically salt, tautomer, stereoisomer, and/or mixture of stereoisomers thereof: 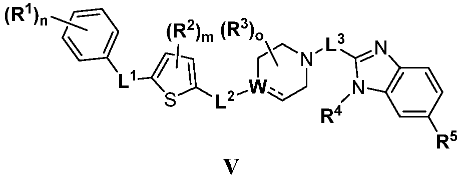 wherein **W** is N, C**R¹⁴**, or C; when **W** is C**R¹⁴**, the adjacent dashed line indicates a single bond; when **W** is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 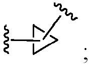 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 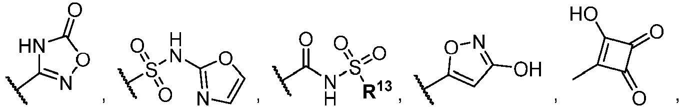 and tetrazolyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 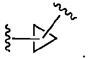 In certain instances, **L²** is selected from the group consisting of -NH- and -N(Me)-. In certain instances, the compound of Formula (V) is selected from compound 71 in Table 1.

In certain instances, provided is a compound of Formula (VI) or a pharmaceutically salt, tautomer, stereoisomer, and/or mixture of stereoisomers thereof: 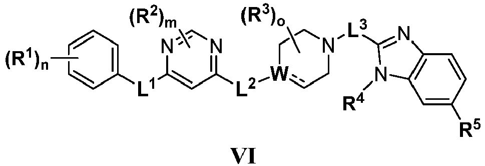 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 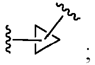 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 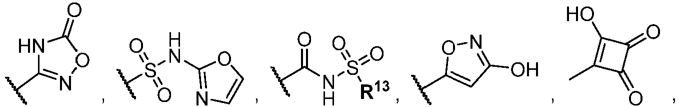 and tetrazolyl; each **R¹¹** is hydrogen, or alkyl; each **R¹²** is hydrogen, or alkyl; each **R¹³** is hydrogen, or alkyl; n is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 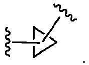 In certain instances, **L²** is selected from the group consisting of -NH- and -N(Me)-. In certain instances, the compound of Formula **(VI)** is selected from compounds **34, 62,** and **331** in Table 1.

In certain instances, provided is a compound of Formula **(VII),** or a pharmaceutically salt, tautomer, stereoisomer, and/or mixture of stereoisomers thereof: 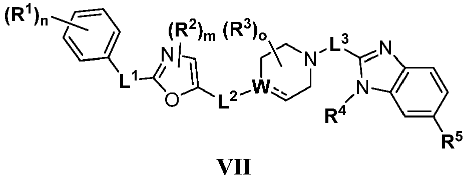 wherein **W** is N, C**R¹⁴**, or C; when **W** is C**R¹⁴**, the adjacent dashed line indicates a single bond; when **W** is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O- -NH-, -N(Me)-, and 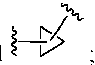 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 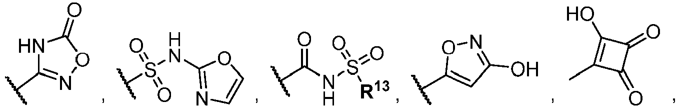 and tetrazolyl; each **R¹¹** is hydrogen, or alkyl; each **R¹²** is hydrogen, or alkyl; each **R¹³** is hydrogen or alkyl; n is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 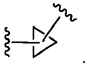 In certain instances, **L²** is selected from the group consisting of -NH- and -N(Me)-. In certain instances, the compound of Formula **(VII)** is selected from compounds 60, 102, 113, 124, 136, 138, 141, 144, 154, 156, 160, 186, 214, 256, 278, and 279 in Table 1.

In certain instances, provided is a compound of Formula **(VIII),** or a pharmaceutically salt, tautomer, stereoisomer, and/or mixture of stereoisomers thereof: 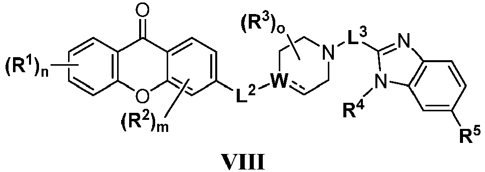 wherein **W** is N, C**R¹⁴**, or C; when **W** is C**R¹⁴**, the adjacent dashed line indicates a single bond; when W is C, the adjacent dashed line indicates a double bond or **L²** is -C(H)=; **L²** is selected from the group consisting of a bond, -C(O)-, -CH₂-, -C(H)=, -SO₂-, -O-, -NH-, -N(Me)-, and 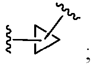 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 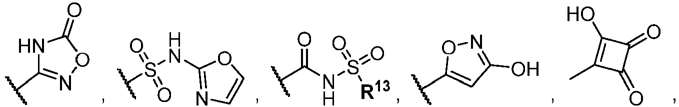 and tetrazolyl; each **R¹¹** is hydrogen or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 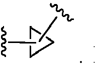 In certain instances, **L²** is selected from the group consisting of -NH- and -N(Me)-. In certain instances, the compound of Formula **(VIII)** is selected from compound **44** in Table 1.

In certain instances, provided is a compound of Formula **(IX),** or a pharmaceutically salt, tautomer, stereoisomer, and/or mixture of stereoisomers thereof: 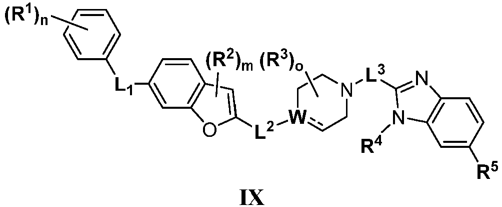 wherein **W** is N, C**R¹⁴**, or C; when **W** is C**R¹⁴**, the adjacent dashed line indicates a single bond; when W is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 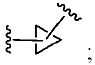 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 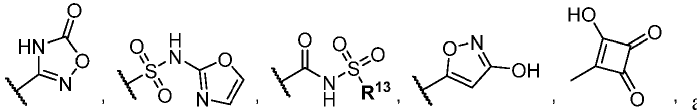 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 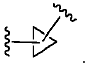 In certain instances, **L²** is selected from the group consisting of -NH- and -N(Me)-. In certain instances, the compound of Formula **(IX)** is selected from compounds **27** and **28** in Table 1.

In certain instances, provided is a compound of Formula **(X),** or a pharmaceutically salt, tautomer, stereoisomer, and/or mixture of stereoisomers thereof: 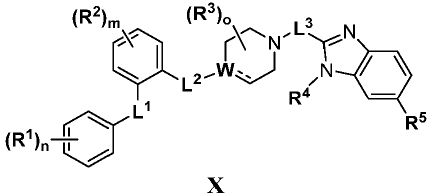 wherein **W** is N, C**R¹⁴**, or C; when **W** is C**R¹⁴**, the adjacent dashed line indicates a single bond; when **W** is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 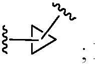 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 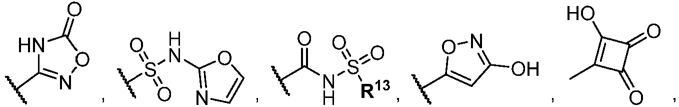 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 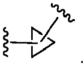 In certain instances, **L²** is selected from the group consisting of -NH- and -N(Me)-. In certain instances, the compound of Formula **(X)** is selected from compound 4, 272, and 284 in Table 1.

In certain instances, provided is a compound of Formula **(XI),** or a pharmaceutically salt, tautomer, stereoisomer, and/or mixture of stereoisomers thereof: 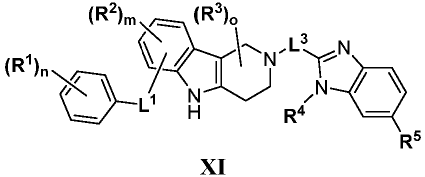 wherein **W** is N, C**R¹⁴**, or C; when **W** is C**R¹⁴**, the adjacent dashed line indicates a single bond; when W is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, - COCF₃, -C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 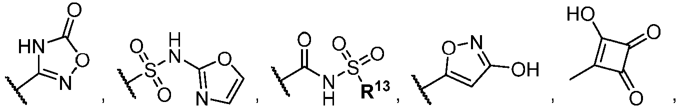 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 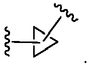 In certain instances, **L²** is selected from the group consisting of -NH- and -N(Me)-. In certain instances, the compound of Formula **(XI)** is selected from compounds 53, 90, 121, 142, and 143 in Table 1.

In certain instances, provided is a compound of Formula **(XII),** or a pharmaceutically salt, tautomer, stereoisomer, and/or mixture of stereoisomers thereof: 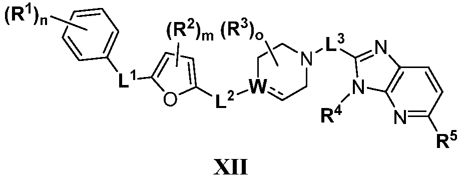 wherein **W** is N, C**R¹⁴**, or C; when **W** is C**R¹⁴**, the adjacent dashed line indicates a single bond; when W is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 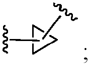 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 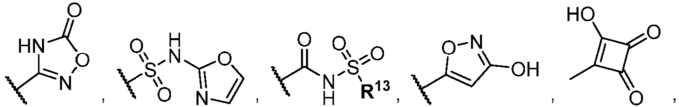 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 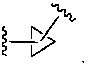 In certain instances, **L²** is selected from the group consisting of -NH- and -N(Me)-. In certain instances, the compound of Formula **(XII)** is compound 108 in Table 1.

In certain instances, provided is a compound of Formula **(XIII),** or a pharmaceutically salt, tautomer, stereoisomer, and/or mixture of stereoisomers thereof: 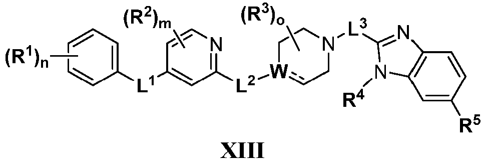 wherein **W** is N, C**R¹⁴**, or C; when **W** is C**R¹⁴**, the adjacent dashed line indicates a single bond; when **W** is **C,** the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 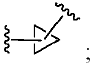 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 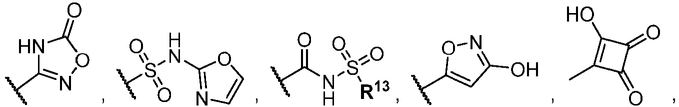 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 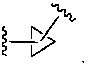 In certain instances, **L²** is selected from the group consisting of -NH- and -N(Me)-. In certain instances, the compound of Formula **(XIII)** is selected from compounds 88, 178, and 179 in Table 1.

In certain instances, provided is a compound of Formula **(XIV),** or a pharmaceutically salt, tautomer, stereoisomer, and/or mixture of stereoisomers thereof: 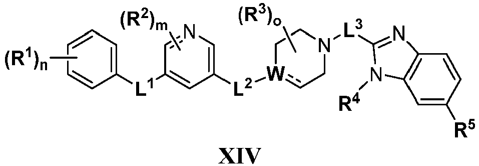 wherein **W** is N, C**R¹⁴**, or C; when **W** is C**R¹⁴**, the adjacent dashed line indicates a single bond; when W is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 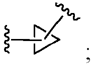 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 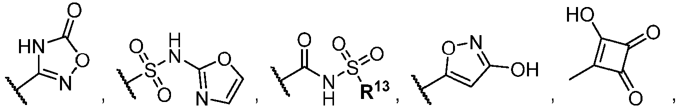 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 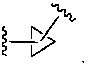 In certain instances, **L²** is selected from the group consisting of -NH- and -N(Me)-. In certain instances, the compound of Formula **(XIV)** is selected from compounds 85, 139, and 220 in Table 1.

In certain instances, provided is a compound of Formula **(XV),** or a pharmaceutically salt, tautomer, stereoisomer, and/or mixture of stereoisomers thereof: 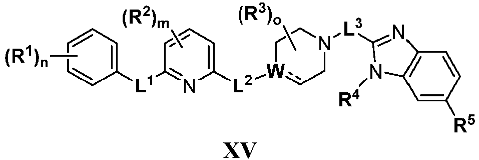 wherein **W** is N, C**R¹⁴**, or C; when **W** is C**R¹⁴**, the adjacent dashed line indicates a single bond; when **W** is **C,** the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 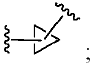 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 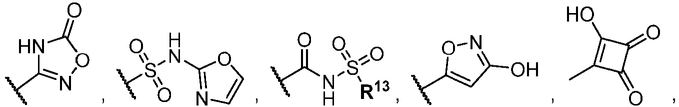 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 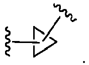 In certain instances, **L²** is selected from the group consisting of -NH- and -N(Me)-. In certain instances, **L2** is O. In certain instances, the compound of Formula **(XV)** is selected from compounds 101, 114, 118, 120, 123, 140, 146, 152, 153, 198-200, 246-249, 251-253, 264, 268-271, 285, 290, 291, 330, and 347 in Table 1.

In certain instances, provided is a compound of Formula **(XVI),** or a pharmaceutically salt, tautomer, stereoisomer, and/or mixture of stereoisomers thereof: 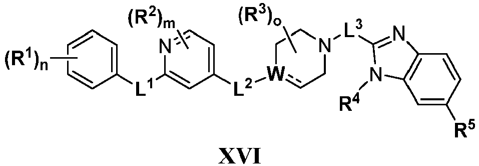 wherein **W** is N, C**R¹⁴**, or C; when **W** is C**R¹⁴**, the adjacent dashed line indicates a single bond; when W is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 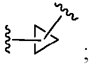 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 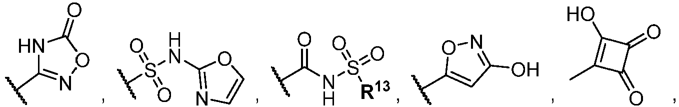 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 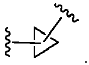 In certain instances, **L²** is selected from the group consisting of -NH- and -N(Me)-. In certain instances, **L2** is O. In certain instances R¹⁴ is CH₂F or CH₂OH. In certain instances, the compound of Formula **(XVI)** is selected from compounds 99, 128, 145, 150, 155, 163, 164, 168-171, 182, 183, 189, 227, 230, 239-241, 250, 257-259, 265-267, 274-277, 280, 283, 287, 292, 295, 297, 299, 308, 316, 321, 322, 333, 335, 337, 345, 346, 352, 354, 356-358, 362-364, 366-368, 378, and 380 in Table 1.

In certain instances, provided is a compound of Formula **(XVIa),** or a pharmaceutically salt, tautomer, stereoisomer, and/or mixture of stereoisomers thereof: 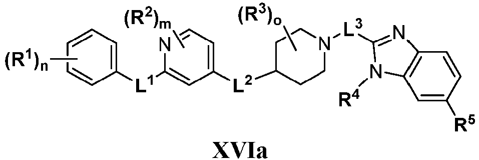 wherein **L¹** is selected from the group consisting of -O-, -CH₂-, -NH-, -N(Me)-, - N(Et)-, -OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -CF₂-. -CHF-, or - CH(OH)-, -NH-, -N(Me)-, and 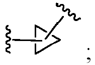 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of - COOH, -COCF₃, -C(OH)CF₃, -CONHCN, -CONHOH, CONHOMe, -CONHSO₂N(Me)₂, - PO₃H₂, -PO(Me)(OH), -SO₃H, -SO₂NH₂, -SO₂NHMe, -B(OH)₂, 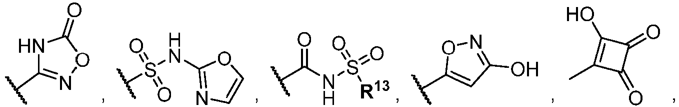 and tetrazolyl; each **R¹¹** is hydrogen or alkyl; each **R¹²** is hydrogen or alkyl; each **R¹³** is hydrogen or alkyl; **R¹⁴** is hydrogen, cyano, halo, hydroxyl, or methyl; **n** is an integer from 0 to 5; **m** is an integer from 0 to 4. In certain instances, **L²** is selected from the group consisting of a bond, -C(O)-, -CH₂-, - C(H)=, -CF₂-, -SO₂-, -O-, -CF₂-. -CHF-, -CH(OH)-, and 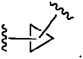 In certain instances, **L²** is selected from the group consisting of -NH- and -N(Me)-. In certain instances, **L2** is **O.** In certain instances, the compound of Formula **(XVI)** is selected from compounds 128, 145, 168-171, 182, 183, 227, 230, 239-241, 250, 257-259, 265-267, 274-277, 280, 283, 287, 292, 295, 297, 299, 302, 308, 316, 321, 322, 333, 335-337, 344-346, 352, 354, 356-359, 362-368, 378, and 380 in Table 1.

In certain instances, provided is a compound of Formula **(XVII),** or a pharmaceutically salt, tautomer, stereoisomer, and/or mixture of stereoisomers thereof: 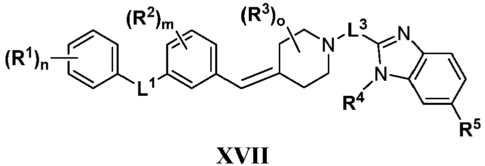 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 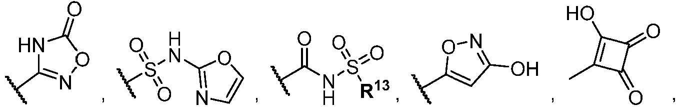 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 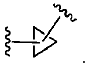 In certain instances, **L²** is selected from the group consisting of -NH- and -N(Me)-. In certain instances, the compound of Formula **(XVII)** is selected from compound 106 in Table 1.

In certain instances, provided is a compound of Formula **(XVIII),** or a pharmaceutically salt, tautomer, stereoisomer, and/or mixture of stereoisomers thereof: 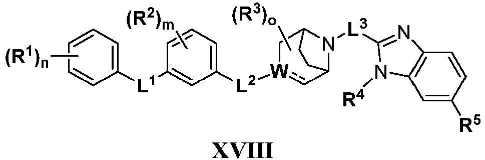 wherein **W** is N, C**R¹⁴**, or C; when **W** is C**R¹⁴**, the adjacent dashed line indicates a single bond; when **W** is C, the adjacent dashed line indicates a double bond or **L²** is -C(H)=; **L¹** is selected from the group consisting of -O-, -CH₂-, -NH-, -N(Me)-, -N(Et)-, -OCH₂-, - OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 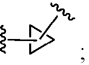 **L³** is - CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 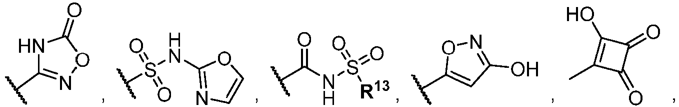 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 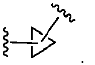 In certain instances, **L²** is selected from the group consisting of -NH- and -N(Me)-. In certain instances, the compound of Formula **(XVIII)** is compound **87** in Table 1.

In certain instances, provided is a compound of Formula **(XXV),** or a pharmaceutically salt, tautomer, stereoisomer, and/or mixture of stereoisomers thereof: 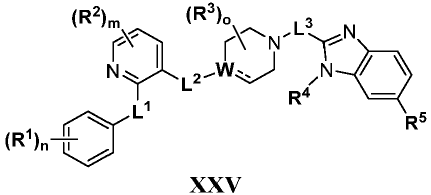 wherein **W** is N, C**R¹⁴**, or C; when **W** is C**R¹⁴**, the adjacent dashed line indicates a single bond; when **W** is C, the adjacent dashed line indicates a double bond or **L²** is -C(H)=; **L¹** is selected from the group consisting of -O-, -CH₂-, -NH-, -N(Me)-, -N(Et)-, -OCH₂-, -OC(H,Me)-, - CH₂O-, -NHCH₂-, -N(Me)CH₂-, and -SO₂NH-; **L²** is selected from the group consisting of a bond, -C(O)-, -CH₂-, -C(H)=, -SO₂-, -O-, -CF₂-. -CHF-, or -CH(OH)-, -NH-, -N(Me)-, and 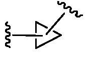 **; L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 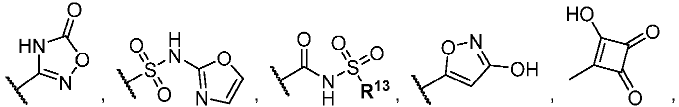 and tetrazolyl; each **R¹¹** is hydrogen or alkyl; each **R¹²** is hydrogen or alkyl; each **R¹³** is hydrogen or alkyl; **R¹⁴** is hydrogen, cyano, halo, hydroxyl, or methyl; **n** is an integer from 0 to 5; **m** is an integer from 0 to 4; **o** is an integer from 0 to 4; **p** is an integer from 0 or 1; **q** is an integer from 0 or 1. In certain instances, **p** is 0; and **q** is 0. In certain instances, **p** is 1; and **q** is 0. In certain instances, **p** is 0; and **q** is 1. In certain instances, **p** is 1; and **q** is 1. In certain instances, **L²** is selected from the group consisting of a bond, -C(O)-, -CH₂-, -C(H)=, -CF₂-, -SO₂-, -O-, -CF₂-. -CHF-, -CH(OH)-, and 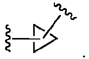 In certain instances, **L²** is selected from the group consisting of - NH- and -N(Me)-. In certain instances, the compound of Formula **(XXV)** is compound 286 in Table 1.

In certain instances, provided is a compound of Formula **(XXVI),** or a pharmaceutically salt, tautomer, stereoisomer, and/or mixture of stereoisomers thereof: 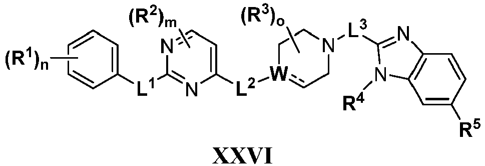 wherein **W** is N, C**R¹⁴**, or C; when **W** is C**R¹⁴**, the adjacent dashed line indicates a single bond; when **W** is C, the adjacent dashed line indicates a double bond or **L²** is -C(H)=; **L¹** is selected from the group consisting of -O-, -CH₂-, -NH-, -N(Me)-, -N(Et)-, -OCH₂-, - OC(H,Me)-, -CHaO-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 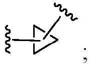 **L³** is - CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 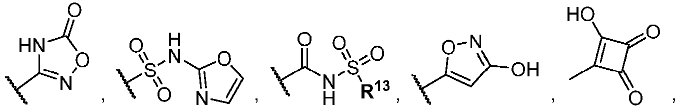 and tetrazolyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, - C(H)=, -SO₂-, -O-, -CF₂-. -CHF-, or -CH(OH)-, and 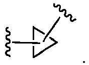 In certain instances, **L²** is selected from the group consisting of -NH- and -N(Me)-. In certain instances, **L2** is O. In certain instances, the compound of Formula **(XXVI)** is selected from compounds 261, 262, 296, 304, 306, 307, 311, 313, 315, 325, 326, 331, 338, 340, 342, 343, 348, 353, 354, 355, 369, 374-, 376, 475-481, 483, 485, 489, 490, 492, 493, , 495, 498, 504, 510, 512, 514, and 515 in Table 1. The compound recited in appended claim 1 is compound 485 or a pharmaceutically acceptable salt or solvate thereof.

In certain instances, provided is a compound of Formula **(XXVII),** or a pharmaceutically salt, tautomer, stereoisomer, and/or mixture of stereoisomers thereof: 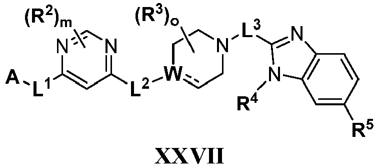 wherein **A** is -NMe₂ or -N(CH₃)cylcohexane; **L¹** is absent; **W** is N, C**R¹⁴**, or C; when **W** is C**R¹⁴**, the adjacent dashed line indicates a single bond; when **W** is C, the adjacent dashed line indicates a double bond or **L²** is -C(H)=; **L²** is selected from the group consisting of a bond, - C(O)-, -CH₂-, -C(H)=, -SO₂-, -O-, -NH-, -N(Me)-, and 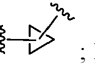 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of - COOH, -COCF₃, -C(OH)CF₃, -CONHCN, -CONHOH, CONHOMe, -CONHSO₂N(Me)₂, - PO₃H₂, -PO(Me)(OH), -SO₃H, -SO₂NH₂, -SO₂NHMe, -B(OH)₂, 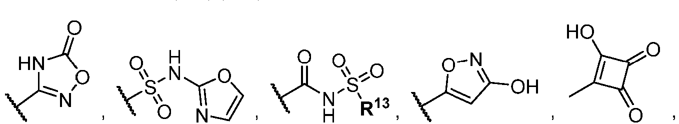 and tetrazolyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, -C(H)=, -SO₂-, - O-, -CF₂-. -CHF-, or -CH(OH)-, and 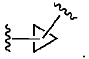 In certain instances, the compound of Formula **(XXVII)** is selected from compounds 1 and 62 in Table 1.

In certain instances, provided is a compound of Formula **(XXXIX),** or a pharmaceutically salt, tautomer, stereoisomer, and/or mixture of stereoisomers thereof: 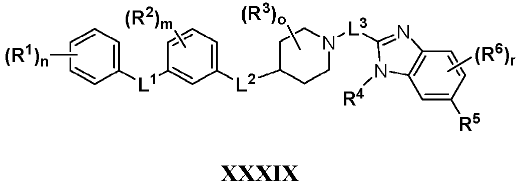 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 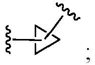 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 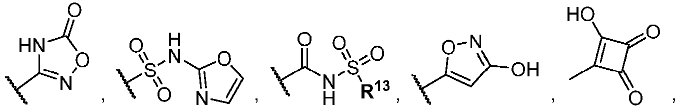 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; **o** is an integer from 0 to 4; and **r** is an integer from 0 to 3. In certain instances, **L²** is selected from the group consisting of a bond, -C(O)-, -CH₂-, -C(H)=, -SO₂-, - O-, -CF₂-. -CHF-, or -CH(OH)-, and 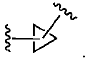 In certain instances, each **R⁶** is independently selected from the group consisting of F, and methyl. In certain instances, each **R⁶** is F. In certain instances, the compound of Formula **(XXXIX)** is selected from compounds **224, 300, 303, 309, 310, 317,** and **320** in Table 1.

In certain instances, provided is a compound of Formula **(XL),** or a pharmaceutically salt, tautomer, stereoisomer, and/or mixture of stereoisomers thereof: 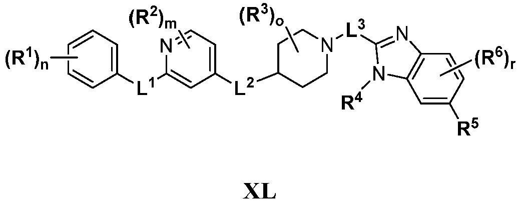 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 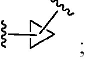 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 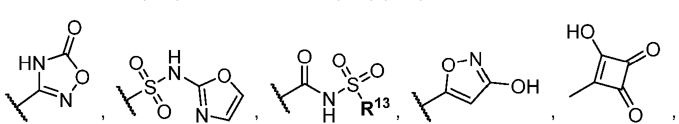 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; **o** is an integer from 0 to 4; and **r** is an integer from 0 to 3. In certain instances, **L²** is selected from the group consisting of a bond, -C(O)-, -CH₂-, -C(H)=, -SO₂-, - O-, -CF₂-. -CHF-, or -CH(OH)-, and 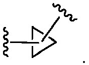 In certain instances, each **R⁶** is independently selected from the group consisting of F, and methyl. In certain instances, each **R⁶** is F. In certain instances, the compound of Formula **(XL)** is selected from compounds **302, 325, 336, 344, 359,** and **365** in Table 1.

In certain instances, provided is a compound of Formula **(XLI),** or a pharmaceutically salt, tautomer, stereoisomer, and/or mixture of stereoisomers thereof: 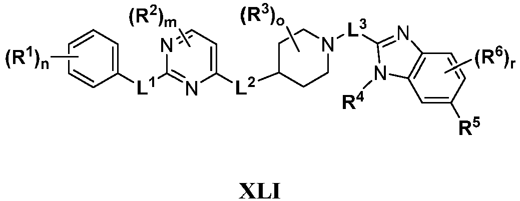 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 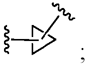 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 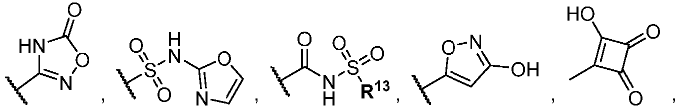 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; **o** is an integer from 0 to 4; and **r** is an integer from 0 to 3. In certain instances, **L²** is selected from the group consisting of a bond, -C(O)-, -CH₂-, -C(H)=, -SO₂-, - O-, -CF₂-. -CHF-, or -CH(OH)-, and 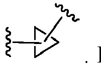 In certain instances, each **R⁶** is independently selected from the group consisting of F, and methyl. In certain instances, each **R⁶** is F. In certain instances, each **R⁶** is methyl. In certain instances, the compound of Formula **(XLI)** is selected from compounds **302, 325, 336, 344, 359, 365, 478, 482, 484, 494, 496, 500-503, 505, 508, 509,** and **511** in Table 1.

In certain instances, provided is a compound of Formula **(XLII),** or a pharmaceutically salt, tautomer, stereoisomer, and/or mixture of stereoisomers thereof: 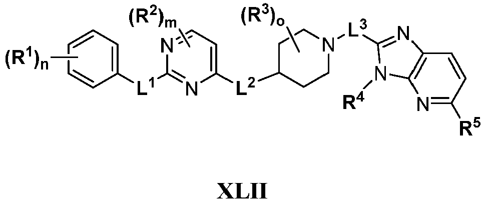 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 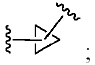 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 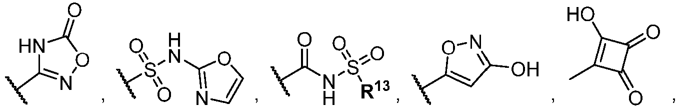 and tetrazolyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, - C(H)=, -SO₂-, -O-, -CF₂-. -CHF-, or -CH(OH)-, and 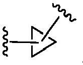 . In certain instances, **L²** is -O-. In certain instances, the compound of Formula **(XLII)** is selected from compounds **370, 371, 373, 375, 377, 378, 486, and 513** in Table 1.

In certain instances, provided is a compound of Formula **(XLIII),** or a pharmaceutically salt, tautomer, stereoisomer, and/or mixture of stereoisomers thereof: 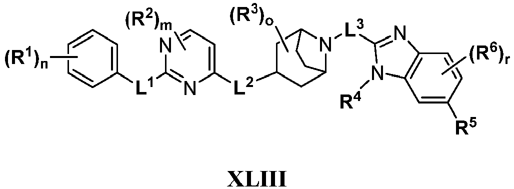 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 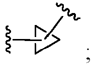 **L³** is -CH₂-, or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 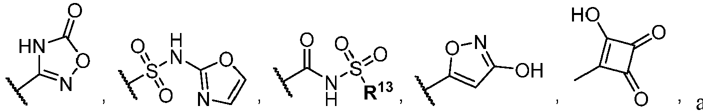 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4; **r** is an integer from 0 to 3. In certain instances, **L²** is selected from the group consisting of a bond, -C(O)-, -CH₂-, -C(H)=, -SO₂-, -O-, -CF₂-. -CHF-, or -CH(OH)-, and 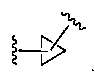 In certain instances, **L²** is -O-. In certain instances, each **R⁶** is independently selected from the group consisting of F, and methyl. In certain instances, each **R⁶** is F. In certain instances, each **R⁶** is methyl. In certain instances, the compound of Formula **(XLIII)** is c selected from compounds 487 and **488** in Table 1.

In certain instances, provided is a compound of Formula **(XLII),** or a pharmaceutically salt, tautomer, stereoisomer, and/or mixture of stereoisomers thereof: 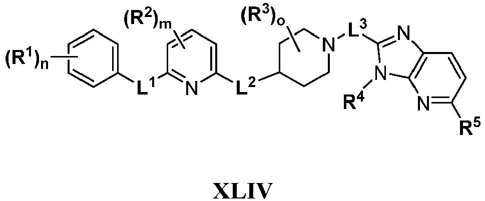 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 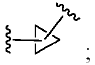 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 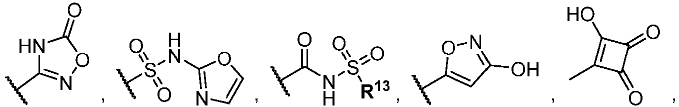 and tetrazolyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, - C(H)=, -SO₂-, -O-, -CF₂-. -CHF-, or -CH(OH)-, and 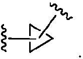 In certain instances, **L²** is -O-. In certain instances, the compound of Formula **(XLIV)** is compound 372 in Table 1.

In certain instances, provided is a compound of Formula **(XLV),** or a pharmaceutically salt, tautomer, stereoisomer, and/or mixture of stereoisomers thereof: 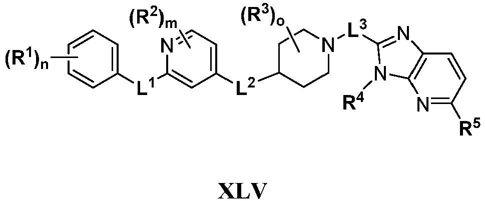 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 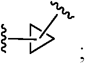 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 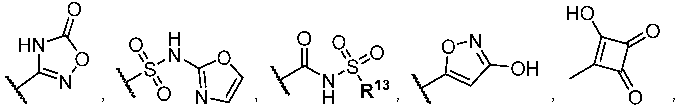 and tetrazolyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, - C(H)=, -SO₂-, -O-, -CF₂-. -CHF-, or -CH(OH)-, and 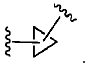 In certain instances, **L²** is -O-. In certain instances, the compound of Formula **(XLV)** is compound 379 in Table 1.

In certain instances, provided is a compound of Formula **(XLVI),** or a pharmaceutically salt, tautomer, stereoisomer, and/or mixture of stereoisomers thereof: 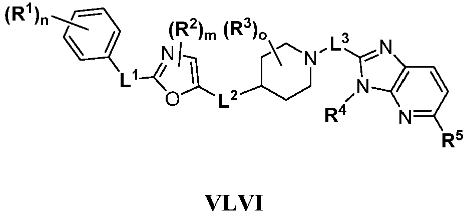 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 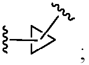 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 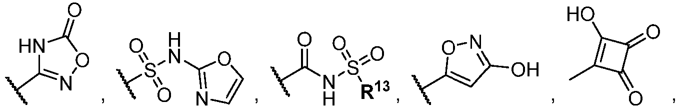 and tetrazolyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4. In certain instances, **L²** is selected from the group consisting of a bond, -C(O)-, -CH₂-, - C(H)=, -SO₂-, -O-, -CF₂-. -CHF-, or -CH(OH)-, and 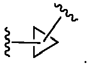 In certain instances, **L²** is -O-. In certain instances, the compound of Formula **(XLVI)** is selected from compounds**125** and **129** in Table 1.

In certain instances, provided is a compound of Formula **(XLI),** or a pharmaceutically salt, tautomer, stereoisomer, and/or mixture of stereoisomers thereof: 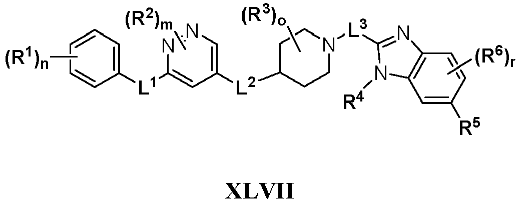 wherein **L¹** is selected from the group consisting of -O-, -CH₂-, -NH-, -N(Me)-, -N(Et)-, - OCH₂-, -OC(H,Me)-, -CH₂O-, -NHCH₂-, -N(Me)CH₂-, and -SO₂NH-; **L²** is selected from the group consisting of a bond, -C(O)-, -CH₂-, -C(H)=, -SO₂-, -O-, -NH-, -N(Me)-, and 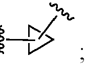 **L³** is -CH₂- or -C(O)-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 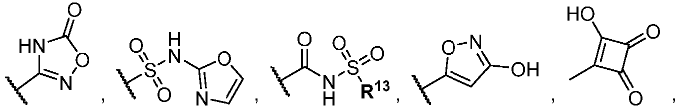 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; **o** is an integer from **o** to 4; and **r** is an integer from 0 to 3. In certain instances, **L²** is selected from the group consisting of a bond, -C(O)-, -CH₂-, -C(H)=, -SO₂-, - O-, -CF₂-. -CHF-, or -CH(OH)-, and 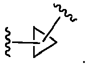 In certain instances, each **R⁶** is independently selected from the group consisting of F, and methyl. In certain instances, each **R⁶** is F. In certain instances, each **R⁶** is methyl. In certain instances, the compound of Formula **(XLVII)** is compound **491** in Table 1.

In certain instances, provided is a compound of any of Formulas **(I)-(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein each **R²** and **R³** is H, or F.

In certain instances, provided is a compound of any of Formulas **(I)-(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **m** is 0; and **o** is 0.

In certain instances, provided is a compound of any of Formulas **(I)-(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **W** is N, or CH.

In certain instances, provided is a compound of any of Formulas **(I)-(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **W** is C.

In certain instances, provided is a compound of any of Formulas **(I)-(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **W** is CH.

In certain instances, provided is a compound of any of Formulas **(I)-(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **W** is N.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **W** is C**R¹⁴**.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **R⁴** is selected from the group consisting of oxetan-2-yl-methyl, (1-ethyl-1H-imidazol-5-yl)-methyl, oxelan-3-yl-methyl and 1,3-oxazol-2-yl-methyl.

In certain instances, provided is a compound of any of Formulas **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **R⁴** is selected from the group consisting of oxetan-2-yl-methyl, and (1-ethyl-1H-imidazol-5-yl)-methyl.

In certain instances, provided is a compound of any of Formulas **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **R⁴** is oxetan-2-yl-methyl.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **R⁴** is (2*S*)-oxetan-2-yl-methyl.

In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **R⁴** is (1-ethyl-1H-imidazol-5-yl)-methyl.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **R⁴** is selected from the group consisting of (S)-buta-4-yl-1,3-diol, butan-1-yl-one, 1-(cyanomethyl)-cycloprop-1-yl-methyl, 1-(Fluoromethyl)-cycloprop-1-yl-methyl, 1-hydroxyl-cycloprop-1-yl-methyl, isoxazol-5-yl-methyl, (1-methyl-1H-imidazol-5-yl)-methyl, 1-methoxy-cycloprop-lyl-methyl, methoxyethan-2-yl, 1-(2-methoxyethyl)-cycloprop-1-yl-methyl, and 2-oxabicyclo[2.1.1]hexa-1-yl-methyl.

In certain instances, provided is a compound of Formula **(XXX),** or a pharmaceutically salt, tautomer, stereoisomer, and/or mixture of stereoisomers thereof, wherein **R⁴** is H.

In certain instances, provided is a compound of Formula **(XXX),** or a pharmaceutically salt, tautomer, stereoisomer, and/or mixture of stereoisomers thereof, wherein **R⁴** is selected from the group consisting of oxelan-3-yl-methyl and 1,3-oxazol-2-yl-methyl.

In certain instances, provided is a compound of Formula **(XXX)** and **(XXXI),** or a pharmaceutically salt, tautomer, stereoisomer, and/or mixture of stereoisomers thereof, wherein **R⁴** is (3*R*)-oxelan-3-yl-methyl.

In certain instances, provided is a compound of Formula **(XXX)** and **(XXXI),** or a pharmaceutically salt, tautomer, stereoisomer, and/or mixture of stereoisomers thereof, wherein **R⁴** is 1,3-oxazol-2-yl-methyl.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **R⁵** is -COOH, or - COOMe.

In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **R⁵** is -COOH

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **R⁵** is tetrazolyl.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **R⁵** is 1H-1,2,3,4-tetrazol-5-yl.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -CH₂-, -C(O)-, or -SO₂-.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is a bond, -C(O)-, -CH₂-, -C(H)=, -SO₂-, -O-, -CF₂-. -CHF-, or -CH(OH)-.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -CH= or -O-.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -CF₂-, -CHF-, or -CH(OH)-.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH- or - N(Me)-.

In certain instances, provided is a compound of Formula **(XXX),** or a pharmaceutically salt, tautomer, stereoisomer, and/or mixture of stereoisomers thereof, wherein **L²** is a bond.

In certain instances, provided is a compound of Formula **(XXXI),** or a pharmaceutically salt, tautomer, stereoisomer, and/or mixture of stereoisomers thereof, wherein **L²** is -CH₂-.

In certain instances, provided is a compound of any of Formulas (I) - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L³** is -CH₂-.

In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L³** is -C(O)-.

In certain instances, provided is a compound of Formulas **(XXX),** or a pharmaceutically salt, tautomer, stereoisomer, and/or mixture of stereoisomers thereof, wherein **L¹** is -CH₂O-, or -OCH₂-.

In certain instances, provided is a compound of Formula **(XXXI),** or a pharmaceutically salt, tautomer, stereoisomer, and/or mixture of stereoisomers thereof, wherein **L¹** is -CH₂O-, or -OCH₂.

In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L¹** is -CH₂O-; and **L²** is -CH₂- or -CO -.

In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L¹** is -CH₂O-; and **L³** is -C(O)-.

In certain instances, provided is a compound of any of Formulas **(I) - (XVIII), (XXV) - (XXVII), (XXX),** and **(XXXI),** or a pharmaceutically salt, tautomer, stereoisomer, and/or mixture of stereoisomers thereof, wherein **L¹** is -O-.

In certain instances, provided is a compound of any of Formulas **(I) - (XVIII), (XXV) - (XXVII), (XXX), (XXXI), (XXXIX), and (XL-XLVII),** or a pharmaceutically salt, tautomer, stereoisomer, and/or mixture of stereoisomers thereof, wherein **L¹** is -CH₂-.

In certain instances, provided is a compound of any of Formulas **(I) - (XVIII), (XXV) - (XXVII), (XXX), (XXXI), (XXXIX), and (XL-XLVII),** or a pharmaceutically salt, tautomer, stereoisomer, and/or mixture of stereoisomers thereof, wherein **L¹** is -NH-.

In certain instances, provided is a compound of any of Formulas **(I) - (XVIII), (XXV) - (XXVII), (XXX), (XXXI), (XXXIX), and (XL-XLVII),** or a pharmaceutically salt, tautomer, stereoisomer, and/or mixture of stereoisomers thereof, wherein **L¹** is -N(Me)-.

In certain instances, provided is a compound of any of Formulas **(I) - (XVIII), (XXV) - (XXVII), (XXX), (XXXI), (XXXIX), and (XL-XLVII),** or a pharmaceutically salt, tautomer, stereoisomer, and/or mixture of stereoisomers thereof, wherein **L¹** is -N(Et)-.

In certain instances, provided is a compound of any of Formulas **(I) - (XVIII), (XXV) - (XXVII), (XXX), (XXXI), (XXXIX), and (XL-XLVII),** or a pharmaceutically salt, tautomer, stereoisomer, and/or mixture of stereoisomers thereof, wherein **L¹** is -OCH₂-.

In certain instances, provided is a compound of any of Formulas **(I) - (XVIII), (XXV) - (XXVII), (XXX),** and **(XXXI),** or a pharmaceutically salt, tautomer, stereoisomer, and/or mixture of stereoisomers thereof, wherein **L¹** is -OC(H,Me)-.

In certain instances, provided is a compound of any of Formulas **(I) - (XVIII), (XXV) - (XXVII), (XXX), (XXXI), (XXXIX), and (XL-XLVII),** or a pharmaceutically salt, tautomer, stereoisomer, and/or mixture of stereoisomers thereof, wherein **L¹** is -CH₂O-.

In certain instances, provided is a compound of any of Formulas **(I) - (XVIII), (XXV) - (XXVII), (XXX), (XXXI), (XXXIX), and (XL-XLVII),** or a pharmaceutically salt, tautomer, stereoisomer, and/or mixture of stereoisomers thereof, wherein **L¹** is -NHCH₂-.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L¹** is -N(Me)CH₂-.

In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L¹** is -SO₂NH-.

In certain instances of any of Formulas **(I)-(XVIII), (XXV)** - **(XXVII), (XXX),** a **(XXXI), (XXXIX),** and **(XL-XLVII), L¹** is -O-; and **L²** is -C(O)-. In certain instances of any of Formulas **(I)-(XVIII), (XXV)** - **(XXVII), (XXX), (XXXI), (XXXIX),** and **(XL-XLVII), L¹** is -O-; and **L²** is -CH₂-. In certain instances of any of Formulas **(I)-(XVIII), (XXV)** - **(XXVII), (XXX), (XXXI), (XXXIX),** and **(XL-XLVII), L¹** is -O-; and **L²** is -C(H)=. In certain instances of any of Formulas **(I)-(XVIII), (XXV)** - **(XXVII), (XXX), (XXXI), (XXXIX),** and **(XL-XLVII), L¹** is -O-; and **L²** is -SO₂-. In certain instances of any of Formulas **(I)-(XVIII), (XXV) - (XXVII), (XXX),** (**XXXI**), (**XXXIX**), and **(XL-XLVII), L¹** is -O-; and **L²** is -NH-. In certain instances of any of Formulas **(I)-(XVIII), (XXV)** - **(XXVII), (XXX), (XXXI), (XXXIX),** and **(XL-XLVII), L¹** is -O-; and **L²** is -N(Me)-. In certain instances of any of Formulas **(I)-(XVIII), (XXV)** - **(XXVII),** (**XXX**), **(XXXI), (XXXIX),** and **(XL-XLVII), L¹** is -O-; **L²** is -C(O)-; and **L³** is -CH₂-. In certain instances of any of Formulas **(I)-(XVIII), (XXV) - (XXVII), (XXX), (XXXI), (XXXIX),** and **(XL-XLVII), L¹** is -O-; **L²** is -CH₂-; and **L³** is -CH₂-. In certain instances of any of Formulas **(I)-(XVIII), (XXV)** - **(XXVII), (XXX), (XXXI), (XXXIX),** and **(XL-XLVII), L¹** is -O-; **L²** is -C(H)=; and **L³** is -CH₂-. In certain instances of any of Formulas **(I)-(XVIII), (XXV)** - **(XXVII), (XXX), (XXXI), (XXXIX),** and **(XL-XLVII), L¹** is -O-; **L²** is -SO₂-; and **L³** is -CH₂-. In certain instances of any of Formulas **(I)-(XVIII), (XXV) - (XXVII), (XXX), (XXXI), (XXXIX),** and **(XL-XLVII), L¹** is -O-; **L²** is -NH-; and **L³** is -CH₂-. In certain instances of any of Formulas **(I)-(XVIII), (XXV)** - **(XXVII), (XXX), (XXXI), (XXXIX),** and **(XL-XLICI), L¹** is -O-; **L²** is -N(Me)-; and **L³** is - CH₂-. In certain instances of any of Formulas **(I)-(XVIII), (XXV)** - **(XXVII), (XXX), (XXXI), (XXXIX),** and **(XL-XLVII), L¹** is -O-; **L²** is -C(O)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -CH₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -C(H)=; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -SO₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -C(O)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -CH₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII),** (**XXX**), **(XXXI), (XXXIX),** and **(XL-XLVII), L¹** is -O-; **L²** is -C(H)=; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -SO₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII),** (**XXX**), **(XXXI), (XXXIX),** and **(XL-XLVII), L¹** is -O-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -C(O)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -O-; **L²** is -CH₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -O-; **L²** is -C(H)=; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -O-; **L²** is -SO₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -O-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -O-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -CH₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is - C(H)=; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is - NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and R³ are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is - C(O)-; **L³** is -CH₂-; **R²** and **R³** are each H; **R⁴** is (1-ethyl-1H-imidazol-5-yl)-methyl; ; and **R³** is -COOH. In certain instances of any of Formulas **(I)-(XVIII), (XXV)** - **(XXVII), (XXX),** and **(XXXI), L¹** is -O-; **L²** is -CH₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -C(H)=; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -SO₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -C(O)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -CH₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is - SO₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -C(O)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -CH₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -O-; **L²** is -C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -SO₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLIIV), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII),** (**XXX**), **(XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV)** - **(XXVII), (XXX),** and **(XXXI) R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX), and (XL-XLVII), L¹** is -CH₂O-; and **L²** is -C(O)-. In certain instances of any of Formulas **(I)-(XVIII), (XXV) - (XXVII), (XXX), (XXXI), (XXXIX), and (XL-XLVII), L¹** is -CH₂O-; and **L²** is -CH₂-. In certain instances of any of Formulas **(I)-(XVIII), (XXV) - (XXVII), (XXX), (XXXI), (XXXIX), and (XL-XLVII), L¹** is -CH₂O-; and **L²** is - C(H)=. In certain instances of any of Formulas **(I)-(XVIII), (XXV) - (XXVII), (XXX), (XXXI), (XXXIX), and (XL-XLVII), L¹** is -CH₂O-; and **L²** is -SO₂-. In certain instances of any of Formulas **(I)-(XVIII), (XXV) - (XXVII), (XXX), (XXXI), (XXXIX), and (XL-XLVII), L¹** is -CH₂O-; and **L²** is -NH-. In certain instances of any of Formulas **(I)-(XVIII), (XXV) - (XXVII), (XXX), (XXXI), (XXXIX), and (XL-XLVII), L¹** is -CH₂O-; and **L²** is - N(Me)-. In certain instances of any of Formulas **(I)-(XVIII), (XXV) - (XXVII), (XXX), (XXXI), (XXXIX), and (XL-XLVII), L¹** is -CH₂O-; **L²** is -C(O)-; and **L³** is -CH₂-. In certain instances of any of Formulas **(I)-(XVIII), (XXV) - (XXVII), (XXX),** and **(XXXI), L¹** is - CH₂O-; **L²** is -CH₂-; and **L³** is -CH₂-. In certain instances of any of Formulas **(I)-(XVIII), (XXV) - (XXVII), (XXX), (XXXI), (XXXIX),** and **(XL-XLVII), L¹** is -CH₂O-; **L²** is -C(H)=; and **L³** is -CH₂-. In certain instances of any of Formulas **(I)-(XVIII), (XXV) - (XXVII), (XXX), (XXXI), (XXXIX), and (XL-XLVII), L¹** is -CH₂O-; **L²** is -SO₂-; and **L³** is -CH₂-. In certain instances of any of Formulas **(I)-(XVIII), (XXV) - (XXVII), (XXX), (XXXI), (XXXIX), and (XL-XLVII), L¹** is -CH₂O-; **L²** is -NH--; and **L³** is -CH₂-. In certain instances of any of Formulas **(I)-(XVIII), (XXV) - (XXVII), (XXX), (XXXI), (XXXIX), and (XL-XLVII), L¹** is -CH₂O-; **L²** is -N(Me)- -; and **L³** is -CH₂-. In certain instances of any of Formulas **(I)-(XVIII), (XXV) - (XXVII), (XXX), (XXXI), (XXXIX), and (XL-XLIII), L¹** is -CH₂O-; **L²** is -C(O)-; **L³** is -CH₂-; and **R²** and **R³** are each H. In certain instances of any of Formulas **(I)-(XVIII), (XXV) - (XXVII), (XXX), (XXXI), (XXXIX), and (XL-XLVII), L¹** is -CH₂O-; **L²** is -CH₂-; **L³** is -CH₂-; and **R²** and **R³** are each H. In certain instances of any of Formulas **(I)-(XVIII), (XXV) - (XXVII), (XXX), (XXXI), (XXXIX), and (XL-XLVII), L¹** is -CH₂O-; **L²** is -C(H)=; **L³** is -CH₂-; and **R²** and **R³** are each H. In certain instances of any of Formulas **(I)-(XVIII), (XXV) - (XXVII), (XXX), (XXXI), (XXXIX), and (XL-XLVII), L¹** is -CH₂O-; **L²** is -SO₂-; **L³** is -CH₂-; and **R²** and **R³** are each H. In certain instances of any of Formulas **(I)-(XVIII), (XXV) - (XXVII), (XXX), (XXXI), (XXXIX), and (XL-XLVII), L¹** is -CH₂O-; **L²** is -NH-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -CH₂O-; **L²** is -C(O)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -C(H)=; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -SO₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -C(O)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -CH₂O-; **L²** is -C(H)=; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -SO₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -CH₂O-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -CH₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -C(O)-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -CH₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -C(H)=; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -SO₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -C(O)-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH₂-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -C(H)=; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -SO₂-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is - CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -CH₂O-; **L²** is -C(O)-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH₂-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is - CH₂-; and **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is -CH₂-; and **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -C(H)=; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -SO₂-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3R)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (*S*)-buta-4-yl-1,3-diol. In certain instances of any of Formulas **(I)-(XVIII), (XXV)** - **(XXVII), (XXX),** and **(XXXI) R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX),** and **(XXXI) R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX),** and **(XXXI) R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX),** and **(XL-XLVII), L¹** is -OCH₂-; and **L²** is -C(O)-. In certain instances of any of Formulas **(I)-(XVIII), (XXV)** - **(XXVII), (XXX), (XXXI), (XXXIX),** and **(XL-XLVII), L¹** is -OCH₂-; and **L²** is -CH₂-. In certain instances of any of Formulas **(I)-(XVIII), (XXV) - (XXVII), (XXX), (XXXI), (XXXIX),** and **(XL-XLVII), L¹** is -OCH₂-; and **L²** is - C(H)=. In certain instances of any of Formulas **(I)-(XVIII), (XXV) - (XXVII), (XXX), (XXXI), (XXXIX),** and **(XL-XLVII), L¹** is -OCH₂-; and **L²** is -SO₂-. In certain instances of any of Formulas **(I)-(XVIII), (XXV) - (XXVII), (XXX),** and **(XXXI), L¹** is -OCH₂-; and **L²** is -NH-. In certain instances of any of Formulas **(I)-(XVIII), (XXV) - (XXVII), (XXX), (XXXI), (XXXIX),** and **(XL-XLVII), L¹** is -OCH₂-; and **L²** is -N(Me)-. In certain instances of any of Formulas **(I)-(XVIII), (XXV) - (XXVII), (XXX), (XXXI), (XXXIX),** and **(XL-XLVII), L¹** is -OCH₂-; **L²** is -C(O)-; and **L³** is -CH₂-. In certain instances of any of Formulas **(I)-(XVIII), (XXV) - (XXVII), (XXX), (XXXI), (XXXIX),** and **(XL-XLVII), L¹** is -OCH₂-; **L²** is -CH₂-; and **L³** is -CH₂-. In certain instances of any of Formulas **(I)-(XVIII), (XXV)** - **(XXVII), (XXX), (XXXI), (XXXIX),** and **(XL-XLVII), L¹** is -OCH₂-; **L²** is -C(H)=; and **L³** is -CH₂-. In certain instances of any of Formulas **(I)-(XVIII), (XXV) - (XXVII), (XXX), (XXXI), (XXXIX),** and **(XL-XLVII), L¹** is -OCH₂-; **L²** is -SO₂-; and **L³** is -CH₂-. In certain instances of any of Formulas **(I)-(XVIII), (XXV) - (XXVII), (XXX), (XXXI), (XXXIX),** and **(XL-XLVII), L¹** is -OCH₂-; **L²** is -NH-; and **L³** is -CH₂-. In certain instances of any of Formulas **(I)-(XVIII), (XXV) - (XXVII), (XXX), (XXXI), (XXXIX),** and **(XL-XLVII), L¹** is -OCH₂-; **L²** is -N(Me)- ; and **L³** is -CH₂-. In certain instances of any of Formulas **(I)-(XVIII), (XXV) - (XXVII), (XXX), (XXXI), (XXXIX),** and **(XL-XLVII), L¹** is -OCH₂-; **L²** is -C(O)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -OCH₂-; **L²** is -CH₂-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -OCH₂-; **L²** is -C(H)=; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -OCH₂-; **L²** is -SO₂-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLIVII), L¹** is -OCH₂-; **L²** is -NH-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLIII), L¹** is -OCH₂-; **L²** is -N(Me)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -OCH₂-; **L²** is -C(O)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -CH₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -C(H)=; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -SO₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -C(O)-; **L³** is -CH₂-; **R²** and **R³** are each H; and **R⁴** is (1-ethyl-1H-imidazol-5-yl)-methyl. In certain instances of any of Formulas **(I)-(XVIII),** (XXV) - **(XXVII), (XXX), (XXXI), (XXXIX),** and **(XL-XLVII), L¹** is -OCH₂-; **L²** is -CH₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -OCH₂-; **L²** is -C(H)=; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -SO₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -OCH₂-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -OCH₂-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -CH₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLIII), L¹** is -OCH₂-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -C(O)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -CH₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -C(H)=; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -SO₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLIVII), L¹** is -OCH₂-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -C(O)-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -CH₂-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -C(H)=; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -SO₂-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -N(Me)-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLIII), L¹** is -OCH₂-; **L²** is -C(O)-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -CH₂-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -OCH₂-; **L²** is -C(H)=; **L³** - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -SO₂-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX),** and **(XXXI) R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLIII), R⁴** is 1-(Fluoromethyl)-cycloprop-1-yl-methyl. In certain instances of any of Formulas **(I)-(XVIII), (XXV) - (XXVII), (XXX),** and **(XXXI) R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX),** and **(XXXI) R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -NH- and **L²** is -C(O)-. In certain instances of any of Formulas **(I)-(XVIII), (XXV) - (XXVII), (XXX), (XXXI), (XXXIX),** and **(XL-XLVII), L¹** is -NH- and **L²** is -CH₂-. In certain instances of any of Formulas **(I)-(XVIII), (XXV)** - **(XXVII), (XXX),** and **(XXXI), L¹** is -NH-; and **L²** is -C(H)=. In certain instances of any of Formulas **(I)-(XVIII), (XXV)** - **(XXVII),** (**XXX**), **(XXXI), (XXXIX),** and **(XL-XLVII), L¹** is -NH-; and **L²** is -SO₂-. In certain instances of any of Formulas **(I)-(XVIII), (XXV)** - **(XXVII), (XXX), (XXXI), (XXXIX),** and **(XL-XLVII), L¹** is -NH-; and **L²** is -NH-. In certain instances of any of Formulas **(I)-(XVIII), (XXV)** - **(XXVII), (XXX), (XXXI), (XXXIX),** and **(XL-XLVII), L¹** is -NH-; and **L²** is -N(Me)-. In certain instances of any of Formulas **(I)-(XVIII), (XXV) - (XXVII), (XXX), (XXXI), (XXXIX),** and **(XL-XLIII), L¹** is -NH-; **L²** is - C(O)-; and **L³** is -CH₂-. In certain instances of any of Formulas **(I)-(XVIII), (XXV)** - **(XXVII), (XXX), (XXXI), (XXXIX),** and **(XL-XLVII), L¹** is -NH-; **L²** is -CH₂-; and **L³** is -CH₂-. In certain instances of any of Formulas **(I)-(XVIII), (XXV)** - **(XXVII), (XXX), (XXXI), (XXXIX),** and **(XL-XLVII), L¹** is -NH-; **L²** is -C(H)=; and **L³** is -CH₂-. In certain instances of any of Formulas **(I)-(XVIII), (XXV) - (XXVII), (XXX), (XXXI), (XXXIX),** and **(XL-XLVII), L¹** is -NH-; **L²** is -SO₂-; and **L³** is -CH₂-. In certain instances of any of Formulas **(I)-(XVIII), (XXV) - (XXVII), (XXX), (XXXI), (XXXIX),** and **(XL-XLVII), L¹** is -NH-; **L²** is -NH-; and **L³** is -CH₂-. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; and **L³** is -CH₂-. In certain instances of any of Formulas **(I)-(XVIII), (XXV)** - **(XXVII), (XXX), (XXXI), (XXXIX),** and **(XL-XLVII), L¹** is -NH-; **L²** is -C(O)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NH-; **L²** is -CH₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NH-; **L²** is -C(H)=; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NH-; **L²** is -SO₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NH-; **L²** is -C(O)-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CH₂-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -C(H)=; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI),** (**XXXIX**), and (**XL-XLVII**), **L¹** is -NH-; **L²** is -SO₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI),** (**XXXIX**), and (**XL-XLVII**), **L¹** is -NH-; **L²** is -C(O)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NH-; **L²** is -CH₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -NH-; **L²** is -C(H)=; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -NH-; **L²** is -SO₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NH-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NH-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -CH₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; **R⁴** is (2*S*)-oxetan-2-yl-methyl; and **R³** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -C(O)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -CH₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -C(H)=; L³ is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLIII), L¹** is -NH-; **L²** is -SO₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -NH-; **L²** is -C(O)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -CH₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -SO₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; **R²** and **R³** are each H; **R⁴ is** (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -C(O)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -CH₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -NH-; **L²** is -C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -SO₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴ is** butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII),** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII),** (**XXX**)**, (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX),** and **(XL-XLVII), L¹** is -N(Me)- and **L²** is -C(O)-. In certain instances of any of Formulas **(I)-(XVIII), (XXV) - (XXVII), (XXX), (XXXI), (XXXIX),** and **(XL-XLVII), L¹** is -N(Me)- and **L²** is -CH₂-. In certain instances of any of Formulas **(I)-(XVIII), (XXV) - (XXVII), (XXX), (XXXI), (XXXIX),** and **(XL-XLVII), L¹** is -N(Me)- and **L²** is - SO₂-. In certain instances of any of Formulas **(I)-(XVIII), (XXV) - (XXVII), (XXX), (XXXI), (XXXIX),** and **(XL-XLVII), L¹** is -N(Me)-; and **L²** is -NH-. In certain instances of any of Formulas **(I)-(XVIII), (XXV) - (XXVII), (XXX), (XXXI), (XXXIX),** and **(XL-XLVII), L¹** is -N(Me)-; and **L²** is -N(Me)-. In certain instances of any of Formulas **(I)-(XVIII), (XXV) - (XXVII), (XXX), (XXXI), (XXXIX),** and **(XL-XLVII), L¹** is -N(Me)-; **L²** is -C(O)-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; L**²** is -CH₂-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; L² is -C(H)=; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -SO₂-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -NH-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -C(O)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Me)-; **L²** is -CH₂-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Me)-; **L²** is -C(H)=; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Me)-; **L²** is -SO₂-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLIII), L¹** is -N(Me)-; **L²** is -NH-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Me)-; **L²** is -C(O)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is **-CH₂-**; **L³** is -CH₂**-; R²** and **R³** are each H; and **R⁴** is (*2S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -C(H)=; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII),** (XXX), **(XXXI), (XXXIX),** and **(XL-XLVII), L¹** is -N(Me)-; **L²** is -SO₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII),** (XXX), **(XXXI), (XXXIX),** and **(XL-XLVII), L¹** is -N(Me)-; **L²** is -C(O)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -CH₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLIII), L¹** is -N(Me)-; **L²** is -C(H)=; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -SO₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -CH₂-; L³ is -CH₂-; **R²** and **R³** are each H; **R⁴** is (2*S*)-oxetan-2-yl-methyl; and **R³** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas (**I**)-**(XVIII), (XXV) - (XXVII), (XXX), (XXXI), (XXXIX),** and **(XL-XLVII), L¹** is -N(Me)-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XVII), L¹** is -N(Me)-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is (2*S*)-oxetan-2-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -C(O)-; **L³** is -CH₂-; **R²** and **R³** are each H; **R⁴ is** (1-ethyl-1H-imidazol-5-yl)-methyl; ; and **R³** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -CH₂-; **L³** is -CH₂-; **R²** and **R³** are each H; **R⁴** is (1-ethyl-1H-imidazol-5-yl)-methyl; and **R³** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -C(H)=; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -SO₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is (1-ethyl-1H-imidazol-5-yl)-methyl; and **R³** is -COOH. In certain instances of any of Formulas **(I)-(XVIII), (XXV)** - **(XXVII), (XXX), (XXXI), (XXXIX),** and **(XL-XLVII), L¹** is -N(Me)-; L² is -C(O)-; L³ is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX),** and **(XXXI), L¹** is -N(Me)-; **L²** is -CH₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -SO₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -C(O)-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -CH₂-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -N(Me)-; **L²** is -C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -SO₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is (1-ethyl-1H-imidazol-5-yl)-methyl; and **R³** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R**³ are each H; **R⁴** is (1-ethyl-1H-imidazol-5-yl)-methyl; and **R³** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴ is** (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴ is** butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴** is 1-methoxy-cycloproplyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴**is{[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLIII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -N(Et)-; and **L²** is -C(O)-. In certain instances of any of Formulas **(I)-(XVIII), (XXV)** - **(XXVII), (XXX), (XXXI), (XXXIX),** and **(XL-XLVII), L¹** is -N(Et)-; and **L²** is -CH₂-. In certain instances of any of Formulas **(I)-(XVIII), (XXV)** - **(XXVII), (XXX), (XXXI), (XXXIX),** and **(XL-XLVII), L¹** is -N(Et)-; and **L²** is - C(H)=. In certain instances of any of Formulas **(I)-(XVIII), (XXV)** - **(XXVII), (XXX), (XXXI), (XXXIX),** and **(XL-XLVII), L¹** is -N(Et)-; and **L²** is -SO₂-. In certain instances of any of Formulas **(I)-(XVIII), (XXV) - (XXVII), (XXX), (XXXI), (XXXIX),** and **(XL-XLVII), L¹** is -N(Et)-; and **L²** is -NH-. In certain instances of any of Formulas **(I)-(XVIII), (XXV) - (XXVII), (XXX), (XXXI), (XXXIX),** and **(XL-XLVII), L¹** is -N(Et)-; and **L²** is - N(Me)-. In certain instances of any of Formulas **(I)-(XVIII), (XXV)** - **(XXVII), (XXX), (XXXI), (XXXIX),** and **(XL-XLVII), L¹** is -N(Et)-; **L²** is -C(O)-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Et)-; **L²** is -CH₂-; and **L³** is -CH₂-. In certain instances of any of Formulas **(I)-(XVIII), (XXV) - (XXVII), (XXX), (XXXI), (XXXIX),** and **(XL-XLVII), L¹** is -N(Et)-; **L²** is -C(H)=; and **L³** is -CH₂-. In certain instances of any of Formulas **(I)-(XVIII), (XXV) - (XXVII), (XXX), (XXXI), (XXXIX),** and **(XL-XLVII), L¹** is -N(Et)-; **L²** is -SO₂-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; and **L³** is -CH₂-. In certain instances of any of Formulas **(I)-(XVIII), (XXV) - (XXVII), (XXX), (XXXI), (XXXIX),** and **(XL-XLVII), L¹** is -N(Et)-; **L²** is -C(O)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -CH₂-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -C(H)=; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -SO₂-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Et)-; **L²** is -C(O)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -CH₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX), and (XL-XLVII), L¹** is -N(Et)-; **L²** is -C(H)=; **L³** is -CH₂-; **R²** and **R³** are each **H;** and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLIVI), L¹** is -N(Et)-; **L²** is -SO₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is -CH₂-; **R²** and **R³** are each H; and **R⁴** is (2*S*)-oxetan-2-yl-mehyl. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -C(O)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CH₂-; **L³** is - CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -C(H)=; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -SO₂-; **L³** is - CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLIII), L¹** is -N(Et)-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -CH₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -N(Et)-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII),** (**XXX**), **(XXXI),** (XXXIX), and **(XL-XLVII), L¹** is -N(Et)-; **L²** is -C(O)-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -CH₂-; **L³** is - CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLIII), L¹** is -N(Et)-; **L²** is -C(H)=; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -N(Et)-; **L²** is -SO₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹ is** -N(Et)-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -C(O)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -CH₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -SO₂-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -C(O)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -CH₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -N(Et)-; **L²** is -C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -SO₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX),** and **(XXXI) R⁴** is butan-1-yl-one. In certain instances of any of Formulas **(I)-(XVIII), (XXV) - (XXVII),** (**XXX**), **(XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX),** and **(XXXI) R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX),** and **(XXXI) R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX),** and **(XXXI) R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII),** (**XXX**), **(XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV)** - **(XXVII),** (**XXX**), **(XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -NHCH₂-; and **L²** is -C(O)-. In certain instances of any of Formulas **(I)-(XVIII), (XXV) - (XXVII), (XXX), (XXXI), (XXXIX),** and **(XL-XLVII), L¹ is** -NHCH₂-; and **L²** is -CH₂-. In certain instances of any of Formulas **(I)-(XVIII), (XXV) - (XXVII), (XXX), (XXXI), (XXXIX),** and **(XL-XLVII), L¹ is** -NHCH₂-; and **L²** is - C(H)=. In certain instances of any of Formulas **(I)-(XVIII), (XXV)** - **(XXVII), (XXX), (XXXI), (XXXIX),** and **(XL-XLVII), L¹ is** -NHCH₂-; and **L²** is -SO₂-. In certain instances of any of Formulas **(I)-(XVIII), (XXV) - (XXVII), (XXX), (XXXI), (XXXIX),** and **(XL-XLVII), L¹** is -NHCH₂-; and **L²** is -NH-. In certain instances of any of Formulas **(I)-(XVIII), (XXV) - (XXVII), (XXX), (XXXI), (XXXIX),** and **(XL-XLVII), L¹ is** -NHCH₂-; and **L²** is - N(Me)-. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; and **L³** is -CH₂-. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH₂-; and **L³** is -CH₂-. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -C(H)=; and **L³** is -CH₂-. In certain instances of any of Formulas **(I)-(XVIII), (XXV) - (XXVII), (XXX), (XXXI), (XXXIX),** and **(XL-XLVII), L¹** is -NHCH₂-; **L²** is -SO₂-; and **L³** is -CH₂-. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; and **L³** is -CH₂-. In certain instances of any of Formulas **(I)-(XVIII), (XXV)** - **(XXVII), (XXX), (XXXI), (XXXIX),** and **(XL-XLVII), L¹** is -NHCH₂-; **L²** is -N(Me)-; and **L³** is -CH₂-. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(H)=; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NHCH₂-; **L²** is -SO₂-; **L³** is -CH₂-; and **R**² and **R³** are each H. In certain instances of any of Formulas **(I)-(XVIII), (XXV)** - **(XXVII), (XXX),** and **(XXXI), L¹** is - NHCH₂-; **L²** is -NH-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NHCH₂-; **L²** is -N(Me)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -NHCH₂-; **L²** is -C(O)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is - C(H)=; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -SO₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -NHCH₂-; **L² is** -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -CH₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(H)=; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -SO₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NHCH₂-; **L² is** -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -CH₂-; **L³** is -CH₂-; **R²** and **R³** are each H; **R⁴** is (2*S*)-oxetan-2-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -NHCH₂-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹ is** -NHCH₂-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; **L³** is - CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -C(H)=; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -SO₂-; **L³** is - CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH₂-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹ is** -NHCH₂-; **L²** is -SO₂-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹ is** -NHCH₂-; **L²** is -C(O)-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII),** (XXX), **(XXXI), (XXXIX),** and **(XL-XLVII), L¹** is -NHCH₂-; **L²** is -CH₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -NHCH₂-; **L²** is - C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -SO₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L² is** -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L² is** -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R'** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII),** (**XXX**), **(XXXI), (XXXIX),** and **(XL-XLVII), R⁴** is (3*S*)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (S)-buta-4-yl-1,3-diol. In certain instances of any of Formulas (**I**)-(**XVIII**), (**XXV**) - (**XXVII**), (**XXX**), (**XXXI**), (**XXXIX**), and (**XL-XLVII**), **R⁴** is butan-1-yl-one. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(cyanomethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(Fluoromethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-hydroxyl-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is isoxazol-5-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (1-methyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-methoxy-cycloprop-lyl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is methoxyethan-2-yl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(2-methoxyethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is {[1-(1,2-oxazole-3-carbonyl)azetidin-2-yl]methyl}. In certain instances of any of Formulas (**I**)-(**XVIII**), (**XXV**) - (**XXVII**), (**XXX**), (**XXXI**), (**XXXIX**), and (**XL-XLVII**), **R⁴** is {[(2R)-1-(1,2-oxazole-3-carbonyl)azetidin-2-yl]methyl}. In certain instances of any of Formulas (**I**)-(**XVIII**), (**XXV**) - (**XXVII**), (**XXX**), (**XXXI**), (**XXXIX**), and (**XL-XLVII**), **R⁴**is{[(2S)-1-(1,2-oxazole-3-carbonyl)azetidin-2-yl]methyl. In certain instances of any of Formulas (**I**)-(**XVIII**), (**XXV**) - (**XXVII**), (**XXX**), (**XXXI**), (**XXXIX**), and (**XL-XLIII**), **R⁴** is 2-oxabicyclo[2.1.1]hexa-1-yl-methyl. In certain instances, provided is a compound of any of Formulas (**I**) - (**XVIII**), (**XXV**) - (**XXVII**), (**XXX**), (**XXXI**), (**XXXIX**), and (**XL-XLII**), or a pharmaceutically salt, tautomer, stereoisomer, and/or mixture of stereoisomers thereof, wherein **L²** is -NH-. In certain instances, provided is a compound of any of Formulas (**I**) - (**XVIII**), (**XXV**) - (**XXVII**), (**XXX**), (**XXXI**), (**XXXIX**), and (**XL-XLVII**), or a pharmaceutically salt, tautomer, stereoisomer, and/or mixture of stereoisomers thereof, wherein **L²** is -N(Me)-.

In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; and **L²** is -C(O)-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; and **L²** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; and **L²** is -C(H)=. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; and **L²** is -SO₂-. In certain instances of any of Formulas (**I**)-(**XVIII**), (**XXV**) - (**XXVII**), (**XXX**), and (**XXXI**), **L¹** is - N(Me)CH₂-; and **L²** is -NH-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; and **L²** is -N(Me)-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(O)-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -CH₂-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(H)=; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -SO₂-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -NH-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -N(Me)-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(O)-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -CH₂-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(H)=; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -SO₂-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -NH-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -N(Me)-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is - C(O)-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -CH₂-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(H)=; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -SO₂-; **L**³ is -CH₂-; **R²** and **R³** are each H; and R**⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLIII**), **L¹** is -N(Me)CH₂-; **L²** is -NH-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -N(Me)-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(O)-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -CH₂-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(H)=; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -SO₂-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -NH-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -N(Me)-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -CH₂-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is - C(O)-; **L³** is -CH₂-; **R²** and **R³** are each H; **R⁴** is (1-ethyl-1H-imidazol-5-yl)-methyl; ; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -CH₂-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(H)=; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -SO₂-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -N(Me)-; **L³** is -CH₂-; **R²** and **R³** are each H; **R**⁴ is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(O)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -CH₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -SO₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -N(Me)-; **L³** is **-**CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(O)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -CH₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -SO₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -N(Me)CH₂-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas (**I**)-(**XVIII**), (**XXV**) - (**XXVII**), (**XXX**), (**XXXI**), (**XXXIX**), and (**XL-XLVII**), **R⁴** is azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (3*R*)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (3*S*)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (S)-buta-4-yl-1,3-diol. In certain instances of any of Formulas (**I**)-(**XVIII**), (**XXV**) - (**XXVII**), (**XXX**), (**XXXI**), (**XXXIX**), and (**XL-XLVII**), **R⁴** is butan-1-yl-one. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(cyanomethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(Fluoromethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-hydroxyl-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is isoxazol-5-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (1-methyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-methoxy-cycloprop-lyl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is methoxyethan-2-yl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(2-methoxyethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is {[1-(1,2-oxazole-3-carbonyl)azetidin-2-yl]methyl}. In certain instances of any of Formulas (**I**)-(**XVIII**), (**XXV**) - (**XXVII**), (**XXX**), (**XXXI**), (**XXXIX**), and (**XL-XLVII**), **R⁴** is {[(2R)-1-(1,2-oxazole-3-carbonyl)azetidin-2-yl]methyl}. In certain instances of any of Formulas (**I**)-(**XVIII**), (**XXV**) - (**XXVII**), (**XXX**), (**XXXI**), (**XXXIX**), and (**XL-XLVII**), **R⁴** is{[(2S)-1-(1,2-oxazole-3-carbonyl)azetidin-2-yl]methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 2-oxabicyclo[2.1.1]hexa-1-yl-methyl. In certain instances, provided is a compound of any of Formulas (**I**) - (**XVIII**), (**XXV**) - (**XXVII**), (**XXX**), (**XXXI**), (**XXXIX**), and (**XL-XLVII**), or a pharmaceutically salt, tautomer, stereoisomer, and/or mixture of stereoisomers thereof, wherein **L²** is -NH-. In certain instances, provided is a compound of any of Formulas (**I**) - (**XVIII**), (**XXV**) - (**XXVII**), (**XXX**), (**XXXI**), (**XXXIX**), and (**XL-XLVII**), or a pharmaceutically salt, tautomer, stereoisomer, and/or mixture of stereoisomers thereof, wherein **L²** is -N(Me)-.

In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; and **L²** is -C(O)-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; and **L²** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; and **L²** is -C(H)=. In certain instances of any of Formulas (**I**)-(**XVIII**), (**XXV**) - (**XXVII**), (**XXX**), (**XXXI**), (**XXXIX**), and **(**X**L-XLVII**), **L¹** is -OC(H,Me)- and **L²** is -SO₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; and **L²** is -NH-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; and **L²** is -N(Me)-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(O)-; and **L³** is - CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -CH₂-; and **L³** is -CH₂-. In certain instances of any of Formulas (**I**)-(**XVIII**), (**XXV**) - (**XXVII**), (**XXX**), a (**XXXI**), (**XXXIX**), and (**XL-XLVII**), **L¹** is -OC(H,Me)-; **L²** is -C(H)=; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -SO₂-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -NH-; and **L³** is -CH₂-. In certain instances of any of Formulas (**I**)-(**XVIII**), (**XXV**) - (**XXVII**), (**XXX**), and (**XXXI**), **L¹** is -OC(H,Me)-; **L²** is -N(Me)-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(O)-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -CH₂-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(H)=; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -SO₂-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -NH-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -N(Me)-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is - C(O)-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -CH₂-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(H)=; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -SO₂-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -NH-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -N(Me)-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(O)-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -CH₂-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(H)=; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -SO₂-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -NH-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -N(Me)-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -CH₂-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -SO₂-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is - C(O)-; **L³** is -CH₂-; **R²** and **R³** are each H; **R⁴** is (1-ethyl-1H-imidazol-5-yl)-methyl; ; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -CH₂-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(H)=; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -SO₂-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(O)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -CH₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OC(H,Me)-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OC(H,Me)-; **L²** is -SO₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OC(H,Me)-; **L²** is -C(O)-; **L³** is -CH₂-; R**²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII),** (**XXX**), **(XXXI), (XXXIX),** and **(XL-XLVII), L¹** is -OC(H,Me)-; L² is -CH₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -OC(H,Me)-; **L²** is -C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -SO₂-; **L³** is -CH₂-; **R²** and **R⁵** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is (1-ethyl-1H-imidazol-5-yl)-methyl; and **R³** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (*S*)-buta-4-yl-1,3-diol. In certain instances of any of Formulas **(I)-(XVIII), (XXV)** - **(XXVII), (XXX),** and **(XXXI) R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴**is{[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -SO₂NH-; and **L²** is -C(O)-. In certain instances of any of Formulas **(I)-(XVIII), (XXV) - (XXVII), (XXX), (XXXI), (XXXIX),** and **(XL-XLVII), L¹** is -SO₂NH-; and **L²** is -CH₂-. In certain instances of any of Formulas **(I)-(XVIII), (XXV) - (XXVII), (XXX), (XXXI), (XXXIX),** and **(XL-XLVII), L¹ is** -SO₂NH-; and **L²** is - C(H)=. In certain instances of any of Formulas **(I)-(XVIII), (XXV)** - **(XXVII), (XXX), (XXXI), (XXXIX),** and **(XL-XLVII), L¹** is -SO₂NH-; and **L²** is -SO₂-. In certain instances of any of Formulas **(I)-(XVIII), (XXV) - (XXVII), (XXX), (XXXI), (XXXIX),** and **(XL-XLVII), L¹** is -SO₂NH-; and **L²** is -NH-. In certain instances of any of Formulas **(I)-(XVIII), (XXV) - (XXVII), (XXX), (XXXI), (XXXIX),** and **(XL-XLVII), L¹ is** -SO₂NH-; and **L²** is - N(Me)-. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(O)-; and **L³** is -CH₂-. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; and **L³** is -CH₂-. In certain instances of any of Formulas **(I)-(XVIII), (XXV) - (XXVII), (XXX), (XXXI), (XXXIX),** and **(XL-XVII), L¹** is -SO₂NH-; **L²** is -C(H)=; and **L³** is -CH₂-. In certain instances of any of Formulas **(I)-(XVIII), (XXV) - (XXVII), (XXX), (XXXI), (XXXIX),** and **(XL-XLVII), L¹ is** -SO₂NH-; **L²** is -SO₂-; and **L³** is -CH₂-. In certain instances of any of Formulas **(I)-(XVIII), (XXV)** - **(XXVII),** (XXX), **(XXXI), (XXXIX),** and **(XL-XLVII), L¹** is -SO₂NH-; **L²** is -NH-; and **L³** is -CH₂-. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; and **L³** is -CH₂-. In certain instances of any of Formulas **(I)-(XVIII), (XXV)** - **(XXVII), (XXX), (XXXI), (XXXIX),** and **(XL-XLVII), L¹** is -SO₂NH-; L² is -C(O)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; L² is -C(H)=; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; L² is -SO₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XVII), L¹** is -SO₂NH-; **L²** is -C(O)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -C(H)=; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -SO₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(O)-; **L³** is -CH₂-; **R²** and **R³** are each H; and **R⁴** is (1-ethyl-1H-imidazol-5-yl)-methyl. In certain instances of any of Formulas **(I)-(XVIII),** (XXV) - **(XXVII), (XXX), (XXXI), (XXXIX),** and **(XL-XLVII), L¹** is -SO₂NH-; **L²** is -CH₂-; **L³** is - CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -C(H)=; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -SO₂-; **L³** is - CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII),** (XXX), **(XXXI), (XXXIX),** and **(XL-XLVII), L¹** is -SO₂NH-; L² is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -C(O)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; **L³** is - CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(H)=; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -SO₂-; **L³** is -CH₂-; **R²** and **R³** are each H; **R⁴** is (2*S*)-oxetan-2-yl-methyl; and **R³** is -COOH. In certain instances of any of Formulas **(I)-(XVIII), (XXV)** - **(XXVII), (XXX), (XXXI), (XXXIX),** and **(XL-XLVII), L¹ is** -SO₂NH-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is (2*S*)-oxetan-2-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(O)-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -CH₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX), and (XL-XLVII), L¹** is -SO₂NH-; L² is - C(H)=; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹ is** -SO₂NH-; **L²** is -SO₂-; **L³** is -CH₂-; **R**² and **R³** are each H; **R⁴** is (1-ethyl-1H-imidazol-5-yl)-methyl; and **R³** is -COOH. In certain instances of any of Formulas **(I)-(XVIII), (XXV) - (XXVII), (XXX), (XXXI), (XXXIX),** and **(XL-XLIVII), L¹** is -SO₂NH-; **L²** is -NH-; **L³** is -CH₂-; **R²** and **R³** are each H; **R⁴** is (1-ethyl-1H-imidazol-5-yl)-methyl; and **R**⁵ is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is - N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX),** and **(XXXI), L¹** is -SO₂NH-; **L²** is -C(O)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹ is** -SO₂NH-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -SO₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹is** -SO₂NH-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹ is** -SO₂NH-; **L²** is -C(O)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -SO2NH-; **L²** is -C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -SO₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is -CH₂-; **R²** and **R³** are each H; **R⁴** is (1-ethyl-1H-imidazol-5-yl)-methyl; and **R**⁵ is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R**⁴ is (*S*)-buta-4-yl-1,3-diol. In certain instances of any of Formulas **(I)-(XVIII), (XXV)** - **(XXVII), (XXX), (XXXI), (XXXIX),** and **(XL-XLVII), R⁴ is** butan-1-yl-one. In certain instances of any of Formulas **(I)-(XVIII), (XXV)** - **(XXVII), (XXX),** and **(XXXI) R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX),** and **(XXXI) R⁴** is 1-methoxy-cycloprop-lyl-methyl. In certain instances of any of Formulas **(I)-(XVIII), (XXV) - (XXVII),** (**XXX**), **(XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -O-; **L²** is -C(O)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -CH₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -O-; **L²** is -C(H)=; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -O-; **L²** is -SO₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is - CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -O-; **L²** is -C(O)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -CH₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -C(H)=; **L³** is -CH₂-; **R²** and **R³** are each H; and **R⁴ is** 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -O-; **L²** is -SO₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII),** (XXX), **(XXXI), (XXXIX),** and **(XL-XLVII), L¹** is -O-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -O-; **L²** is -C(O)-; **L³** is -CH₂-; **R²** and **R³** are each H; **R⁴** is oxelan-3-yl-methyl; and **R³** is - COOH. In certain instances of any of Formulas **(I)-(XVIII), (XXV)** - **(XXVII), (XXX), (XXXI), (XXXIX),** and **(XL-XLVII), L¹** is -O-; **L²** is -CH₂-; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -O-; **L²** is - C(H)=; **L³** is -CH₂-; **R²** and **R³** are each H; **R⁴** is oxelan-3-yl-methyl; and **R³** is -COOH. In certain instances of any of Formulas **(I)-(XVIII), (XXV)** - **(XXVII), (XXX), (XXXI), (XXXIX),** and **(XL-XLVII), L¹ is** -O-; **L²** is -SO₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -C(O)-; **L³** is -CH₂-; **R²** and **R³** are each H; **R⁴** is 1,3-oxazol-2-yl-methyl; ; and **R⁵** is -COOH. In certain instances of any of Formulas **(I)-(XVIII), (XXV) - (XXVII),** (**XXX**), **(XXXI), (XXXIX),** and **(XL-XLVII), L¹** is -O-; **L²** is -CH₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLIII), L¹** is -O-; **L²** is -C(H)=; **L³** is -CH₂-; **R²** and **R³** are each H; **R⁴** is 1,3-oxazol-2-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -O-; **L²** is -SO₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -C(O)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is - C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII),** (XXX), **(XXXI), (XXXIX),** and **(XL-XLVII), L¹** is -O-; **L²** is -SO₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; R² and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -CH₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII),** (**XXX**), **(XXXI), (XXXIX),** and **(XL-XLVII), L¹** is -O-; **L²** is -C(H)=; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is - SO₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; **R⁴** is 1,3-oxazol-2-yl-methyl; and **R³** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴ is** azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴is** (*3S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴ is** 1-methoxy-cycloprop-lyl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -CH₂O-; **L²** is -C(O)-; L³ is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -CH₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -C(H)=; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -CH₂0-; **L²** is -SO₂-; **L**³ is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is - CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -C(O)-; L³ is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH₂-; **L³** is - CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -C(H)=; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -SO₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; L³ is - CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -C(O)-; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -CH₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -C(H)=; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -SO₂-; L³ is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; L³ is -CH₂-; **R²** and **R³** are each H; **R⁴** is oxelan-3-yl-methyl; and **R³** is -COOH. In certain instances of any of Formulas **(I)-(XVIII), (XXV)** - **(XXVII), (XXX), (XXXI), (XXXIX),** and **(XL-XLVII), L¹is** -CH₂O-; **L²** is -C(O)-; L³ is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -CH₂-; **L³** is - CH₂-; **R²** and **R³** are each H; **R⁴** is 1,3-oxazol-2-yl-methyl; and **R³** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -C(H)=; L³ is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -SO₂-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹ is** -CH₂O-; **L²** is -NH-; **L³ is** -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -C(O)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹ is** -CH₂O-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -CH₂O-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -CH₂O-; **L²** is -SO₂-; **L³** is -CH₂-; **R²** and **R³** are each H; **R⁴** is oxelan-3-yl-methyl; and **R³** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹ is** -CH₂O-; **L²** is -N(Me)-; **L³** is - CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII),** (XXX), **(XXXI), (XXXIX),** and **(XL-XLVII), L¹** is -CH₂O-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L**² is -CH₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -CH₂O-; **L²** is -C(H)=; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -SO₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹ is** -CH₂O-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹ is** -CH₂O-; **L²** is -N(Me)-; L³ is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl.

In certain instances of any of Formulas **(I)-(XVIII), (XXV)** - **(XXVII), (XXX), (XXXI), (XXXIX),** and **(XL-XLVII), L¹** is -OCH₂-; **L²** is -C(O)-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -CH₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -C(H)=; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -SO₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is - CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -N(Me)-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -C(O)-; L³ is -CH₂-; **R²** and **R³** are each H; and **R⁴** is 1,3-oxazol-2-yl-methyl. In certain instances of any of Formulas **(I)-(XVIII), (XXV)** - **(XXVII),** (XXX), **(XXXI), (XXXIX),** and **(XL-XLVII), L¹** is -OCH₂-; **L²** is -CH₂-; **L³** is - CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -C(H)=; L³ is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -SO₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is - CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -N(Me)-; L³ is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -C(O)-; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -CH₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -C(H)=; L³ is -CH₂-; **R²** and **R³** are each H; **R⁴** is oxelan-3-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -SO₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLIII), L¹** is -OCH₂-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLIII), L¹** is -OCH₂-; **L²** is -C(O)-; **L³ is** -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -CH₂-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -C(H)=; **L³ is** -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -SO₂-; **L³** is - CH₂-; **R²** and **R³** are each H; **R⁴** is 1,3-oxazol-2-yl-methyl; and **R³** is -COOH. In certain instances of any of Formulas **(I)-(XVIII), (XXV)** - **(XXVII), (XXX), (XXXI), (XXXIX),** and **(XL-XLVII), L¹ is** -OCH₂-; **L²** is -NH-; **L³ is** -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -N(Me)-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -C(O)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹ is** -OCH₂-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OCH₂-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OCH₂-; **L²** is -SO₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OCH₂-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII),** (XXX), **(XXXI), (XXXIX),** and **(XL-XLVII), L¹ is** -OCH₂-; **L²** is -N(Me)-; **L³** is - CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -CH₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -OCH₂-; **L²** is -C(H)=; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -SO₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹ is** -OCH₂-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹ is** -OCH₂-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴ is** butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLIII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII),** (**XXX**), **(XXXI), (XXXIX),** and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLIVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII),** (**XXX**), **(XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴i**s{[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -NH-; **L²** is -C(O)-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -NH-; **L²** is -CH₂-; **L³** is -CH₂-; **R²** and **R³** are each H; and **R⁴** is oxelan-3-yl-methyl. In certain instances of any of Formulas **(I)-(XVIII), (XXV) - (XXVII),** (**XXX)**, **(XXXI), (XXXIX),** and **(XL-XLVII), L¹** is -NH-; **L²** is -C(H)=; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -SO₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NH-**; L²** is -NH-; **L³** is -CH₂-; **R²** and **R³** are each H; and R⁴ is oxelan-3-yl-methyl. In certain instances of any of Formulas **(I)-(XVIII), (XXV) - (XXVII), (XXX), (XXXI), (XXXIX),** and **(XL-XLVII), L¹** is -NH-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -C(O)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -CH₂-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -NH-; **L²** is -C(H)=; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -SO₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -NH-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -NH-; **L²** is -C(O)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -CH₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII),** (**XXX)**, **(XXXI), (XXXIX),** and **(XL-XLVII), L¹** is -NH-; **L²** is -C(H)=; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NH-; **L²** is -SO₂-; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII), (XXX), (XXXI), (XXXIX), and (XL-XLVII), L¹** is -NH-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII),** (XXX), **(XXXI), (XXXIX), and (XL-XLVII), L¹** is -NH-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NH-; **L²** is -C(O)-; **L³** is -CH₂-; **R²** and **R**³ are each H; **R⁴** is 1,3-oxazol-2-yl-methyl; ; and **R⁵** is -COOH. In certain instances of any of Formulas **(I)-(XVIII), (XXV) - (XXVII), (XXX), (XXXI), (XXXIX), and (XL-XLVII), L¹** is -NH-; **L²** is -CH₂-; **L³ is** -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NH-; **L²** is -C(H)=; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NH-; **L²** is -SO₂-; **L³ is** -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NH-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NH-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NH-; **L²** is -C(O)-; **L³** is -CH₂-; **R²** and **R³** are each H; **R⁴** is oxelan-3-yl-methyl; and **R³** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX), and (XL-XLVII), L¹** is -NH-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -SO₂-; **L³ is** -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX), and (XL-XLVII), L¹** is -NH-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -CH₂-; **L³** is -CH₂-; **R²** and **R³** are each H; **R⁴** is 1,3-oxazol-2-yl-methyl; and **R³** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -NH-; **L²** is -C(H)=; **L³** is -CH₂-; **R²** and **R³** are each H; **R⁴** is 1,3-oxazol-2-yl-methyl; and **R³** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -SO₂-; **L³** is -CH₂-; **R²** and **R³** are each H; **R⁴** is 1,3-oxazol-2-yl-methyl; and **R³** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹ is** -NH-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, - C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴is** (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX),** and **(XXXI) R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein L² is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -N(Me)-; **L² i**s -C(O)-; **L³ is** -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -CH₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -C(H)=; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -SO₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is - CH₂-; **R²** and **R³** are each H; and **R⁴** is **o**xelan-3-yl-methyl. In certain instances of any of Formulas **(I)-(XVIII), (XXV)** - **(XXVII),** (**XXX)**, **(XXXI), (XXXIX),** and **(XL-XLVII), L¹** is -N(Me)-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -C(O)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -CH₂-; **L³** is - CH₂-; **R²** and **R³** are each H; and **R⁴** is 1,3-oxazol-2-yl-methyl. In certain instances of any of Formulas **(I)-(XVIII), (XXV) - (XXVII),** (**XXX)**, **(XXXI), (XXXIX),** and **(XL-XLVII), L¹** is -N(Me)-; **L²** is -C(H)=; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -SO₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is - CH₂-; **R²** and **R³** are each H; and **R⁴** is 1,3-oxazol-2-yl-methyl. In certain instances of any of Formulas **(I)-(XVIII), (XXV) - (XXVII),** (**XXX)**, **(XXXI), (XXXIX), and (XL-XLVII), L¹** is -N(Me)-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -C(O)-; **L³** is -CH₂-; **R²** and **R³** are each H; **R⁴** is oxelan-3-yl-methyl; and **R**⁵ is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -CH₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -C(H)=; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -SO₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -C(O)-; **L³** is -CH₂-; **R²** and **R³** are each H; **R⁴** is 1,3-oxazol-2-yl-methyl; ; and **R³** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -CH₂-; **L³** is -CH₂-; **R²** and **R³** are each H; **R⁴** is 1,3-oxazol-2-yl-methyl; and **R³** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -C(H)=; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is '-SO₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; L**³ is** -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -C(O)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -SO₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; L³ is -CH₂-; **R²** and **R³** are each H; **R⁴** is oxelan-3-yl-methyl; and **R³** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is - N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and (**XL-XLVII), L¹** is -N(Me)-; **L²** is -C(O)-; **L**³ is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -CH₂-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -N(Me)-; **L²** is -C(H)=; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII),** (XXX), **(XXXI), (XXXIX),** and **(XL-XLVII), L¹** is -N(Me)-; **L²** is -SO₂-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII),** is -N(Me)-; L**²** is -N(Me)-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴**is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴ is** 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴ is** 1-hydroxyl-cycloprop-1-yl-methyl. In certain instances of any of Formulas **(I)-(XVIII), (XXV) - (XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-1yl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -N(Et)-; **L²** is -C(O)-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CH₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX), and (XL-XLVII), L¹** is -N(Et)-; **L²** is -C(H)=; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX), and (XL-XLVII), L¹** is -N(Et)-; **L²** is -SO₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX), and (XL-XLVII), L¹** is -N(Me)-; **L²** is -NH-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX), and (XL-XLVII), L¹** is -N(Me)-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX), and (XL-XLVII), L¹** is -N(Et)-; **L²** is -C(O)-; **L³ is** - CH₂-; **R²** and **R³** are each H; and **R⁴** is 1,3-oxazol-2-yl-methyl. In certain instances of any of Formulas **(I)-(XVIII), (XXV) - (XXVII),** (**XXX)**, **(XXXI), (XXXIX),** and **(XL-XLVII), L¹** is -N(Et)-; **L²** is -CH₂-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX), and (XL-XLVII), L¹** is -N(Et)-; **L²** is -C(H)=; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX), and (XL-XLVII), L¹** is -N(Et)-; **L²** is -SO₂-; **L³ is** - CH₂-; **R²** and **R³** are each H; and **R⁴** is 1,3-oxazol-2-yl-methyl. In certain instances of any of Formulas **(I)-(XVIII), (XXV) - (XXVII),** (**XXX)**, **(XXXI), (XXXIX),** and **(XL-XLVII), L¹** is -N(Et)-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX), and (XL-XLVII), L¹** is -N(Et)-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX), and (XL-XLVII), L¹** is -N(Et)-; **L²** is -C(O)-; **L³ is** - CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -N(Et)-; **L²** is -CH₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -N(Et)-; **L²** is -C(H)=; **L³ is** - CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -N(Et)-; **L²** is -SO₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -C(O)-; **L³** is - CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -CH₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -C(H)=; **L³** is - CH₂-; **R²** and **R³** are each H; **R⁴** is 1,3-oxazol-2-yl-methyl; and **R³** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -SO₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -N(Et)-; **L²** is -NH-; **L³** is -CH₂-; **R²** and **R³** are each H; **R⁴** is 1,3-oxazol-2-yl-methyl; and **R³** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴ is** 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -C(O)-; **L³** is - CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII),** (XXX), **(XXXI), (XXXIX),** and **(XL-XLVII), L¹** is -N(Et)-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -C(H)=; **L³** is -CH₂-; **R²** and **R³** are each H; **R⁴** is oxelan-3-yl-methyl; and **R³** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -SO₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; **R⁴ is** oxelan-3-yl-methyl; and **R³** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -CH₂-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -N(Et)-; **L²** is -C(H)=; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -SO₂-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII),** (XXX), **(XXXI), (XXXIX),** and **(XL-XLVII), L¹** is -N(Et)-; **L²** is -N(Me)-; **L³** is -CH₂-; **R²** and **R³** are each H; **R⁴** is 1,3-oxazol-2-yl-methyl; and **R³** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴ is** (3R)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX),** and **(XXXI) R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV) - (XXVII), (XXX), (XXXI), (XXXIX),** and **(XL-XLIII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-1yl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII),** (**XXX**), **(XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -CH₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -C(H)=; **L³** is -CH₂-; **R²** and **R³** are each H; and **R⁴ is** oxelan-3-yl-methyl. In certain instances of any of Formulas **(I)-(XVIII),** (XXV) - **(XXVII), (XXX), (XXXI), (XXXIX),** and **(XL-XLVII), L¹** is -NHCH₂-; **L²** is -SO₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is - CH₂-; **R²** and **R³** are each H; and **R⁴** is oxelan-3-yl-methyl. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII),** (XXX), **(XXXI), (XXXIX),** and **(XL-XLVII), L¹** is -NHCH₂-; **L²** is -CH₂-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -C(H)=; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -SO₂-; **L³** is -CH₂-; **R²** and **R³** are each H; and **R⁴ is** 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -C(O)-; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -CH₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX), and (XL-XLVII), L¹** is -NHCH₂-; **L²** is -C(H)=; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NHCH₂-; **L²** is -SO₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII),** (XXX), **(XXXI), (XXXIX), and (XL-XLVII), L¹** is -NHCH₂-; **L²** is -NH-; **L³** is -CH₂-; **R²** and **R³** are each H; **R⁴** is oxelan-3-yl-methyl; and **R³** is -COOH. In certain instances of any of Formulas **(I)-(XVIII), (XXV) - (XXVII),** (XXX), **(XXXI), (XXXIX), and (XL-XLVII), L¹** is -NHCH₂-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -NHCH₂-; **L²** is -C(O)-; **L³** is -CH₂-; **R²** and **R³** are each H; **R⁴** is 1,3-oxazol-2-yl-methyl; ; and **R⁵** is -COOH. In certain instances of any of Formulas **(I)-(XVIII), (XXV) - (XXVII), (XXX), (XXXI), (XXXIX), and (XL-XLVII), L¹** is -NHCH₂-; **L²** is -CH₂-; **L³** is -CH₂-; **R²** and **R³** are each H; **R⁴** is 1,3-oxazol-2-yl-methyl; and **R⁵** is - COOH. In certain instances of any of Formulas **(I)-(XVIII), (XXV) - (XXVII),** (XXX), **(XXXI), (XXXIX), and (XL-XLVII), L¹** is -NHCH₂-; **L²** is -C(H)=; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -SO₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII),** (XXX), **(XXXI), (XXXIX), and (XL-XLVII), L¹** is -NHCH₂-; **L²** is -NH-; **L³** is -CH₂-; **R²** and **R³** are each H; **R⁴** is 1,3-oxazol-2-yl-methyl; and **R³** is -COOH. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII),** (XXX), **(XXXI), (XXXIX), and (XL-XLVII), L¹** is -NHCH₂-; **L²** is -C(O)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX), and (XL-XLVII), L¹** is -NHCH₂-; **L²** is -CH₂-; **L³** is -CH₂-; **R²** and **R³** are each H; R⁴ is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX), and (XL-XLVII), L¹** is -NHCH₂-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -SO₂-; **L³** is -CH₂-; **R²** and **R³** are each H; **R⁴** is oxelan-3-yl-methyl; and **R³** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -CH₂-; **L³** is -CH₂-; **R²** and **R³** are each H; **R⁴ is** 1,3-oxazol-2-yl-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -NHCH₂-; **L²** is -C(H)=; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -SO₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX),** and **(XXXI) R⁴** is (1-methyl-1H-imidazol-5-yl)-methyl. In certain instances of any of Formulas **(I)-(XVIII), (XXV) - (XXVII),** (**XXX**), **(XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -C(O)-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CH₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -C(H)=; **L³** is -CH₂-; **R²** and **R³** are each H; and **R⁴** is oxelan-3-yl-methyl. In certain instances of any of Formulas **(I)-(XVIII), (XXV)** - **(XXVII),** (XXX), **(XXXI), (XXXIX),** and **(XL-XLVII), L¹** is -N(Me)CH₂-; **L²** is -SO₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -NH-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -C(O)-; **L³** is -CH₂-; **R²** and **R³** are each H; and R⁴ is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is - N(Me)CH₂-; **L²** is -CH₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -C(H)=; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is - SO₂-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII),** (XXX), **(XXXI), (XXXIX),** and **(XL-XLVII), L¹** is -N(Me)CH₂-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII),** (**XXX**), **(XXXI), (XXXIX),** and **(XL-XLVII), L¹** is -N(Me)CH₂-; **L²** is -C(O)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is - N(Me)CH₂-; **L²** is -C(H)=; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -SO₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is - N(Me)CH₂-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX), and (XL-XLVII), L¹** is -N(Me)CH₂-; **L²** is -C(O)-; **L³** is -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)CH₂-; **L²** is -C(H)=; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -SO₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)CH₂-; **L²** is - N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -C(O)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII),** (**XXV**) - **(XXVII),** (**XXX**), **(XXXI), (XXXIX),** and **(XL-XLVII), L¹** is -N(Me)CH₂-; **L²** is -SO₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl.. In certain instances of any of Formulas **(I)-(XVIII),** (**XXV**) - **(XXVII),** (**XXX**), **(XXXI), (XXXIX),** and **(XL-XLVII), L¹ is** -N(Me)CH₂-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl.. In certain instances of any of Formulas **(I)-(XVIII),** (**XXV**) - **(XXVII),** (**XXX**), **(XXXI), (XXXIX),** and **(XL-XLVII), L¹** is -N(Me)CH₂-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII),** (**XXV**) - **(XXVII),** (**XXX**), **(XXXI), (XXXIX),** and **(XL-XLVII), L¹** is -N(Me)CH₂-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII),** (**XXV**) - **(XXVII),** (XXX), **(XXXI), (XXXIX),** and **(XL-XLVII), L¹** is -N(Me)CH₂-; **L²** is -CH₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII),** (**XXV**) - **(XXVII),** (**XXX**), **(XXXI), (XXXIX),** and **(XL-XLVII), L¹** is -N(Me)CH₂-; **L²** is -C(H)=; **L³ is** -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII),** (**XXV**) - **(XXVII),** (**XXX)**, **(XXXI), (XXXIX),** and **(XL-XLVII), L¹** is -N(Me)CH₂-; **L²** is -SO₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, - OMe, -CN, -C(O)NMe₂, alkyl, propyl, isopropyl, and cyclopropyl. In certain instances of any of Formulas (**I**)-(**XVIII**), **(XXV)** - (**XXVII**), **(XXX),** (**XXXI**), (**XXXIX**), and (**XL-XLVII**), **R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (S)-buta-4-yl-1,3-diol. In certain instances of any of Formulas **(I)-(XVIII), (XXV)** - **(XXVII),** (XXX), **(XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(Fluoromethyl)-cycloprop-1-yl-methyl. In certain instances of any of Formulas **(I)-(XVIII), (XXV)** - (**XXVII**), **(XXX),** (**XXXI**), (**XXXIX**), and (**XL-XLVII**), **R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas (**I**)-(**XVIII**), (**XXV**) - (**XXVII**), (**XXX**), (**XXXI**), (**XXXIX**), and **(XL-XLVII), R⁴** is (1-methyl-1H-imidazol-5-yl)-methyl. In certain instances of any of Formulas (**I**)-(**XVIII**), **(XXV)** - (**XXVII**), **(XXX),** (**XXXI**), (**XXXIX**), and (**XL-XLVII**), **R⁴** is 1-methoxy-cycloprop-lyl-methyl. In certain instances of any of Formulas **(I)-(XVIII), (XXV)** - **(XXVII), (XXX),** and **(XXXI) R⁴** is methoxyethan-2-yl. In certain instances of any of Formulas (**I**)-(**XVIII**), **(XXV)** - (**XXVII**), (**XXX**), (**XXXI**), (**XXXIX**), and (**XL-XLVII**), **R⁴** is 1-(2-methoxyethyl)-cycloprop-1-yl-methyl. In certain instances of any of Formulas **(I)-(XVIII), (XXV)** - (**XXVII**), **(XXX),** (**XXXI**), (**XXXIX**), and (**XL-XLVII**), **R⁴** is {[1-(1,2-oxazole-3-carbonyl)azetidin-2-yl]methyl}. In certain instances of any of Formulas **(I)-**(XVIII), **(XXV)** - (**XXVII**), **(XXX),** (**XXXI**), (**XXXIX**), and (**XL-XLVII**), **R⁴** is {[(2R)-1-(1,2-oxazole-3-carbonyl)azetidin-2-yl]methyl}. In certain instances of any of Formulas **(I)-(XVIII), (XXV)** - (**XXVII**), **(XXX),** (**XXXI**), (**XXXIX**), and (**XL-XLVII**), **R⁴** is {[(2S)-1-(1,2-oxazole-3-carbonyl)azetidin-2-yl]methyl. In certain instances of any of Formulas **(I)-(XVIII), (XXV)** - (**XXVII**), **(XXX),** (**XXXI**), (**XXXIX**), and (**XL-XLVII**), **R⁴** is 2-oxabicyclo[2.1.1]hexa-1-yl-methyl. In certain instances, provided is a compound of any of Formulas **(I)** - **(XVIII), (XXV)** - (**XXVII**), **(XXX),** (**XXXI**), (**XXXIX**), and (**XL-XLVII**), or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX),** (**XXXI**), (**XXXIX**), and (**XL-XLVII**), or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹ is** -OC(H,Me)-; **L² is** -C(O)-; **L³ is** -CH₂-; **R²** and **R³** are each H; and **R⁴** is oxelan-3-yl-methyl. In certain instances of any of Formulas **(I)-(XVIII), (XXV)** - (**XXVII**), **(XXX),** (**XXXI**), (**XXXIX**), and (**XL-XLVII**), **L¹** is -OC(H,Me)-; **L²** is - CH₂-; **L³** is -CH₂-; **R²** and **R³** are each H; and **R⁴** is oxelan-3-yl-methyl. In certain instances of any of Formulas (**I**)-(**XVIII**), **(XXV)** - (**XXVII**), **(XXX),** (**XXXI**), (**XXXIX**), and **(XL-XLVII), L¹ is** -OC(H,Me)-; **L²** is -C(H)=; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -SO₂-; **L³** is -CH₂-; **R²** and **R³** are each H; and **R⁴** is oxelan-3-yl-methyl. In certain instances of any of Formulas **(I)-(XVIII), (XXV)** - (**XXVII**), **(XXX),** (**XXXI**), (**XXXIX**), and (**XL-XLVII**), **L¹** is -OC(H,Me)-; **L²** is - NH-; **L³** is -CH₂-; **R²** and **R³** are each H; and **R⁴** is oxelan-3-yl-methyl. In certain instances of any of Formulas (**I**)-(**XVIII**), **(XXV)** - (**XXVII**), **(XXX),** (**XXXI**), (**XXXIX**), and **(XL-XLVII), L¹** is -OC(H,Me)-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -C(O)-; **L³** is -CH₂-; **R²** and **R³** are each H; and **R⁴** is 1,3-oxazol-2-yl-methyl. In certain instances of any of Formulas (**I**)-(**XVIII**), **(XXV)** - (**XXVII**), **(XXX),** (**XXXI**), (**XXXIX**), and (**XL-XLVII**), **L¹** is - OC(H,Me)-; **L² is** -CH₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -C(H)=; **L³** is -CH₂-; **R²** and **R³** are each H; and **R⁴** is 1,3-oxazol-2-yl-methyl. In certain instances of any of Formulas **(I)-(XVIII), (XXV)** - (**XXVII**), **(XXX),** (**XXXI**), (**XXXIX**), and (**XL-XLVII**), **L¹** is -OC(H,Me)-; **L²** is - SO₂-; **L³** is -CH₂-; **R²** and **R³** are each H; and **R⁴** is 1,3-oxazol-2-yl-methyl. In certain instances of any of Formulas (**I**)-(**XVIII**), **(XXV)** - (**XXVII**), **(XXX),** (**XXXI**), (**XXXIX**), and **(XL-XLVII), L¹** is -OC(H,Me)-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII),** (XXX), **(XXXI), (XXXIX),** and **(XL-XLVII), L¹** is -OC(H,Me)-; **L²** is -N(Me)-; **L³** is -CH₂-; **R²** and **R³** are each H; and **R⁴** is 1,3-oxazol-2-yl-methyl. In certain instances of any of Formulas (**I**)-(**XVIII**), **(XXV)** - (**XXVII**), (**XXX**), (**XXXI**), (**XXXIX**), and (**XL-XLVII**), **L¹** is -OC(H,Me)-; **L²** is -C(O)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH₂-; **L³** is -CH₂-; **R²** and **R³** are each H; **R⁴** is oxelan-3-yl-methyl; and **R⁵** is -COOH. In certain instances of any of Formulas (**I**)-(**XVIII**), **(XXV)** - (**XXVII**), **(XXX),** (**XXXI**), (**XXXIX**), and (**XL-XLVII**), **L¹** is - OC(H,Me)-; **L²** is -C(H)=; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -SO₂-; **L³** is -CH₂-; **R²** and **R³** are each H; **R⁴** is oxelan-3-yl-methyl; and **R⁵** is -COOH. In certain instances of any of Formulas (**I**)-(**XVIII**), **(XXV)** - (**XXVII**), **(XXX),** (**XXXI**), (**XXXIX**), and **(XL-XLIVII), L¹** is - OC(H,Me)-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³ is** -CH₂-; **R²** and **R³** are each H; **R⁴** is oxelan-3-yl-methyl; and **R⁵** is -COOH. In certain instances of any of Formulas (**I**)-(**XVIII**), **(XXV)** - (**XXVII**), (**XXX**), (**XXXI**), (**XXXIX**), and (**XL-XLVII**), **L¹** is -OC(H,Me)-; **L²** is -C(O)-; **L³ is** -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH₂-; **L³** is -CH₂-; **R²** and **R³** are each H; **R⁴** is 1,3-oxazol-2-yl-methyl; and **R⁵** is -COOH. In certain instances of any of Formulas (**I**)-(**XVIII**), **(XXV)** - (**XXVII**), **(XXX),** (**XXXI**), (**XXXIX**), and (**XL-XLVII**), **L¹** is -OC(H,Mₑ)-; **L²** is -C(H)=; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -SO₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII),** (XXV) - **(XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - (**XXVII**), (XXX), (**XXXI**), (**XXXIX**), and (**XL-XLVII**), **L¹** is -OC(H,Me)-; **L²** is - N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -C(O)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -SO₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -OC(H,Me)-; **L²** is -C(H)=; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -SO₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, - OMe, -CN, -C(O)NMe₂, alkyl, propyl, isopropyl, and cyclopropyl. In certain instances of any of Formulas (**I**)-(**XVIII**), (**XXV**) - (**XXVII**), (**XXX**), (**XXXI**), (**XXXIX**), and (**XL-XLVII**), **R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(Fluoromethyl)-cycloprop-1-yl-methyl. In certain instances of any of Formulas **(I)-(XVIII), (XXV)** - (**XXVII**), **(XXX),** (**XXXI**), (**XXXIX**), and (**XL-XLVII**), **R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas (**I**)-(**XVIII**), **(XXV)** - (**XXVII**), **(XXX),** (**XXXI**), (**XXXIX**), and **(XL-XLVII), R⁴** is (1-methyl-1H-imidazol-5-yl)-methyl. In certain instances of any of Formulas (**I**)-(**XVIII**), **(XXV)** - (**XXVII**), **(XXX),** (**XXXI**), (**XXXIX**), and (**XL-XLVII**), **R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII),** (XXV) - **(XXVII), (XXX), (XXXI), (XXXIX),** and **(XL-XLVII), R⁴** is 1-(2-methoxyethyl)-cycloprop-1-yl-methyl. In certain instances of any of Formulas (**I**)-(**XVIII**), **(XXV)** - (**XXVII**), (**XXX**), (**XXXI**), (**XXXIX**), and (**XL-XLVII**), **R⁴** is {[1-(1,2-oxazole-3-carbonyl)azetidin-2-yl]methyl}. In certain instances of any of Formulas (**I**)-(**XVIII**), **(XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII),** (**XXV**) - (**XXVII**), **(XXX),** (**XXXI**), (**XXXIX)**, and (**XL-XLVII**), **R⁴** is 2-oxabicyclo[2.1.1]hexa-1-yl-methyl. In certain instances, provided is a compound of any of Formulas **(I) - (XVIII), (XXV)** - (**XXVII**), **(XXX),** (**XXXI**), (**XXXIX**), and (**XL-XLVII**), or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - (**XXVII**), **(XXX),** (**XXXI**), (**XXXIX**), and **(b),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -SO₂NH-; **L²** is -C(O)-; **L³** is -CH₂-; **R²** and **R³** are each H; and **R⁴** is oxelan-3-yl-methyl. In certain instances of any of Formulas **(I)-(XVIII), (XXV)** - (**XXVII**), **(XXX),** (**XXXI**), (**XXXIX**), and (**XL-XLVII**), **L¹** is -SO₂NH-; **L²** is -CH₂-; **L³** is -CH₂-; **R²** and **R³** are each H; and **R⁴** is oxelan-3-yl-methyl. In certain instances of any of Formulas (**I**)-(**XVIII**), **(XXV)** - (**XXVII**), **(XXX),** (**XXXI**), (**XXXIX**), and (**XL-XLVII**), **L¹** is -SO₂NH-; **L²** is -C(H)=; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -SO₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLIII), L¹** is -SO₂NH-; **L²** is -NH-; **L³** is - CH₂-; **R²** and **R³** are each H; and **R⁴** is oxelan-3-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -SO₂NH-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(O)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; **L³** is - CH₂-; **R²** and **R³** are each H; and **R⁴** is 1,3-oxazol-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -SO₂NH-; **L²** is -C(H)=; **L³ is** -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -SO₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is - CH₂-; **R²** and **R³** are each H; and **R⁴** is 1,3-oxazol-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -SO₂NH-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(O)-; **L³** is -CH₂-; **R²** and **R³** are each H; **R⁴** is oxelan-3-yl-methyl; and **R⁵** is -COOH. In certain instances of any of Formulas (**I**)-(**XVIII**), (**XXV**) - (**XXVII**), (**XXX**), **(XXXI**), (**XXXIX**), and (**XL-XLVII**), **L¹** is -SO₂NH-; **L² is** -CH₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(H)=; **L³** is -CH₂-; **R²** and **R³** are each H; **R⁴** is oxelan-3-yl-methyl; and **R⁵** is -COOH. In certain instances of any of Formulas **(I)-**(**XVIII**), (**XXV)** - (**XXVII**), **(XXX),** (**XXXI**), (**XXXIX**), and (**XL-XLVII**), **L¹** is -SO₂NH-; **L²** is -SO₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹ is** -SO₂NH-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - (**XXVII**), (**XXX**), (**XXXI**), (**XXXIX**), and (**XL-XLVII**), **L¹** is -SO₂NH-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(O)-; L³ is -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - (**XXVII**), **(XXX),** (**XXXI**), (**XXXIX**), and (**XL-XLVII**), **L¹** is -SO₂NH-; **L²** is -CH₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -C(H)=; **L³ is** -CH₂-; **R²** and **R³** are each H; **R⁴** is 1,3-oxazol-2-yl-methyl; and **R⁵** is -COOH. In certain instances of any of Formulas **(I)-(XVIII), (XXV)** - (**XXVII**), **(XXX),** (**XXXI**), (**XXXIX**), and (**XL-XLVII**), **L¹** is -SO₂NH-; **L²** is -SO₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹ is** -SO₂NH-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - (**XXVII**), **(XXX),** (**XXXI**), (**XXXIX**), and (**XL-XLVII**), **L¹** is -SO₂NH-; **L²** is -N(Me)-; **L**³ is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹ is** -SO₂NH-; **L²** is -C(O)-; **L³ is** -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and **(XL-XLVII), L¹ is** -SO₂NH-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -SO₂NH-; **L²** is -SO₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -SO₂NH-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII),** (**XXV**) - (**XXVII**), (**XXX**), (**XXXI**), (**XXXIX**), and (**XL-XLVII**), **L¹** is -SO₂NH-; **L²** is - N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - (**XXVII**), (**XXX**), (**XXXI**), (**XXXIX**), and (**XL-XLVII**), **L¹** is -SO₂NH-; **L²** is -C(O)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH₂-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - (**XXVII**), (**XXX**), (**XXXI**), (**XXXIX**), and (**XL-XLVII**), **L¹** is -SO₂NH-; **L²** is -C(H)=; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -SO₂-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl.In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas (**I**)-(**XVIII**), **(XXV)** - (**XXVII**), (**XXX**), (**XXXI**), (**XXXIX**), and (**XL-XLVII**), **R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-1yl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII),** (XXV) - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -O-; and **L²** is -O-. In certain instances of any of Formulas **(I)-(XVIII), (XXV)** - **(XXVII),** (**XXX**), **(XXXI), (XXXIX),** and **(XL-XLVII), L¹** is -O-; and **L²** is -CF₂-. In certain instances of any of Formulas **(I)-(XVIII), (XXV)** - **(XXVII), (XXX), (XXXI), (XXXIX),** and **(XL-XLVII), L¹** is -O-; and **L²** is -CHF-. In certain instances of any of Formulas **(I)-(XVIII), (XXV)** - **(XXVII), (XXX), (XXXI), (XXXIX),** and **(XL-XLVII), L¹** is -O-; and **L²** is -CH(OH)-. In certain instances of any of Formulas **(I)-(XVIII), (XXV)** - **(XXVII), (XXX), (XXXI), (XXXIX),** and **(XL-XLVII), L¹** is -O-; and **L²** is -NH-. In certain instances of any of Formulas **(I)-(XVIII), (XXV)** - **(XXVII), (XXX), (XXXI), (XXXIX),** and **(XL-XVII), L¹** is -O-; and **L²** is -N(Me)-. In certain instances of any of Formulas **(I)-(XVIII), (XXV) - (XXVII),** (**XXX**), **(XXXI), (XXXIX),** and **(XL-XLVII), L¹** is -O-; **L²** is -O-; and **L³** is -CH₂-. In certain instances of any of Formulas **(I)-(XVIII), (XXV)** - **(XXVII), (XXX), (XXXI), (XXXIX),** and **(XL-XLVII), L¹** is -O-; **L²** is -CF₂-; and **L³** is - CH₂-. In certain instances of any of Formulas **(I)-(XVIII), (XXV)** - **(XXVII), (XXX), (XXXI), (XXXIX),** and **(XL-XLVII), L¹** is -O-; **L²** is -CHF-; and **L³** is -CH₂-. In certain instances of any of Formulas **(I)-(XVIII), (XXV) - (XXVII), (XXX), (XXXI), (XXXIX),** and **(XL-XLVII), L¹** is -O-; **L²** is -CH(OH)-; and **L³** is -CH₂-. In certain instances of any of Formulas **(I)-(XVIII), (XXV)** - **(XXVII), (XXX), (XXXI), (XXXIX),** and **(XL-XLVII), L¹** is -O-; **L²** is -NH-; and **L³** is -CH₂-. In certain instances of any of Formulas **(I)-(XVIII), (XXV)** - **(XXVII),** (XXX), **(XXXI), (XXXIX),** and **(XL-XLIII), L¹** is -O-; **L²** is -N(Me)-; and **L³** is - CH₂-. In certain instances of any of Formulas **(I)-(XVIII), (XXV)** - **(XXVII), (XXX), (XXXI), (XXXIX),** and **(XL-XLVII), L¹** is -O-; **L²** is -O-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -CF₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -CHF-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -CH(OH)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -O-; **L²** is -O-; **L³** is -CH₂-; **R²** and **R³** are each H; and **R⁴** is (2*S*)-oxetan-2-yl-methyl. In certain instances of any of Formulas **(I)-(XVIII),** (XXV) - **(XXVII), (XXX), (XXXI), (XXXIX),** and **(XL-XLVII), L¹** is -O-; **L²** is -CF₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -O-; **L²** is -CHF-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -CH(OH)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII),** (**XXX**), **(XXXI), (XXXIX),** and **(XL-XLVII), L¹** is -O-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -O-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -O-; **L²** is -CF₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -O-; **L²** is -CHF-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -O-; **L²** is -CH(OH)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII),** (**XXX**), **(XXXI), (XXXIX),** and **(XL-XLVII), L¹** is -O-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -O-; **L²** is -O-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is - CHF-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -CH(OH)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is - NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is - O-; **L³** is -CH₂-; **R²** and **R³** are each H; **R⁴** is (1-ethyl-1H-imidazol-5-yl)-methyl; ; and **R⁵** is - COOH. In certain instances of any of Formulas **(I)-(XVIII), (XXV)** - **(XXVII), (XXX), (XXXI), (XXXIX),** and **(XL-XLVII), L¹** is -O-; **L²** is -CF₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -CHF-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -CH(OH)-; **L³** is - CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -O-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI),** v**R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-1yl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX),(XXXI), (XXXIX),** and **(XL-XLVII), L¹** is -CH₂O-; and **L²** is -O-. In certain instances of any of Formulas **(I)-(XVIII), (XXV) - (XXVII), (XXX), (XXXI), (XXXIX),** and **(XL-XLVII), L¹** is -CH₂O-; and **L²** is -CF₂-. In certain instances of any of Formulas **(I)-(XVIII), (XXV) - (XXVII), (XXX), (XXXI), (XXXIX),** and **(XL-XLVII), L¹** is -CH₂O-; and **L²** is - CHF-. In certain instances of any of Formulas **(I)-(XVIII), (XXV)** - **(XXVII), (XXX), (XXXI), (XXXIX),** and **(XL-XLVII), L¹** is -CH₂O-; and **L²** is -CH(OH)-. In certain instances of any of Formulas **(I)-(XVIII), (XXV)** - **(XXVII), (XXX), (XXXI), (XXXIX),** and **(XL-XLVII), L¹** is -CH₂O-; and **L²** is -NH-. In certain instances of any of Formulas **(I)-(XVIII), (XXV)** - **(XXVII), (XXX), (XXXI), (XXXIX),** and **(XL-XLVII), L¹** is -CH₂O-; and **L²** is - N(Me)-. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; and **L³** is -CH₂-. In certain instances of any of Formulas **(I)-(XVIII), (XXV)** - **(XXVII), (XXX), (XXXI), (XXXIX),** and **(XL-XLVII), L¹** is -CH₂O-; **L²** is -CF₂-; and **L³** is -CH₂-. In certain instances of any of Formulas **(I)-(XVIII), (XXV)** - **(XXVII),** (**XXX**), **(XXXI), (XXXIX),** and **(XL-XLVII), L¹** is -CH₂O-; **L²** is -CHF-; and **L³** is -CH₂-. In certain instances of any of Formulas **(I)-(XVIII), (XXV)** - **(XXVII), (XXX),** and **(XXXI), L¹** is -CH₂O-; **L²** is -CH(OH)-; and **L³** is -CH₂-. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -CH₂O-; **L²** is -CF₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -CH₂O-; **L²** is -CHF-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -CH₂O-; **L²** is -CH(OH)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -CF₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -CHF-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH(OH)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; and **R⁴** is (2*S*)-oxetan-2-yl-methyl. In certain instances of any of Formulas **(I)-(XVIII),** (XXV) - **(XXVII), (XXX), (XXXI), (XXXIX),** and **(XL-XLVII), L¹** is -CH₂O-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII),** (**XXX**), **(XXXI), (XXXIX),** and **(XL-XLVII), L¹** is -CH₂O-; **L²** is -CF₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -CHF-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -CH₂O-; **L²** is -CH(OH)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -CHF-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -CH(OH)-; **L³** is -CH₂-; **R²** and **R³** are each H; **R⁴** is (2*S*)-oxetan-2-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII),** (**XXX**), **(XXXI), (XXXIX),** and **(XL-XLVII), L¹** is -CH₂O-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV)** - **(XXVII), (XXX), (XXXI), (XXXIX),** and (**XL-XLVII**), **L¹** is -CH₂O-; **L²** is -CF₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -CHF-; **L³** is -CH₂-; **R²** and **R³** are each H; **R⁴** is (1-ethyl-1H-imidazol-5-yl)-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -CH(OH)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; **R²** and **R³** are each H; **R⁴** is (1-ethyl-1H-imidazol-5-yl)-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI),** (**XXXIX**), and (**XL-XLVII**), **L¹** is -CH₂O-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -CH₂O-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -CH₂O-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -CH₂O-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas (**I**)-(**XVIII**), (**XXV**) - (**XXVII**), (**XXX**), (**XXXI**), (**XXXIX**), and (**XL-XLVII**), **L¹** is -CH₂O-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -CH₂O-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -CH₂O-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -CH₂O-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas (**I**)-(**XVIII**), (**XXV**) - (**XXVII**), (**XXX**), (**XXXI**), (**XXXIX**), and (**XL-XLVII**), **R⁴** is azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (3*R*)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (3S)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (S)-buta-4-yl-1,3-diol. In certain instances of any of Formulas (**I**)-(**XVIII**), (**XXV**) - (**XXVII**), (**XXX**), (**XXXI**), (**XXXIX**), and (**XL-XLVII**), **R⁴** is butan-1-yl-one. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(cyanomethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(Fluoromethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-hydroxyl-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is isoxazol-5-yl-methyl. In certain instances of any of Formulas (**I**)-(**XVIII**), (**XXV**) - (**XXVII**), (**XXX**), and (**XXXI**) **R⁴** is (1-methyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-methoxy-cycloprop-lyl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is methoxyethan-2-yl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(2-methoxyethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is {[1-(1,2-oxazole-3-carbonyl)azetidin-2-yl]methyl}. In certain instances of any of Formulas (**I**)-(**XVIII**), (**XXV**) - (**XXVII**), (**XXX**), (**XXXI**), (**XXXIX**), and (**XL-XLVII**), **R⁴** is {[(2R)-1-(1,2-oxazole-3-carbonyl)azetidin-2-yl]methyl}. In certain instances of any of Formulas (**I**)-(**XVIII**), (**XXV**) - (**XXVII**), (**XXX**), (**XXXI**), (**XXXIX**), and (**XL-XLVII**), **R⁴** is {[(2S)-1-(1,2-oxazole-3-carbonyl)azetidin-2-yl]methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 2-oxabicyclo[2.1.1]hexa-1-yl-methyl. In certain instances, provided is a compound of any of Formulas (**I**) - (**XVIII**), (**XXV**) - (**XXVII**), (**XXX**), (**XXXI**), (**XXXIX**), and (**XL-XLVII**), or a pharmaceutically salt, tautomer, stereoisomer, and/or mixture of stereoisomers thereof, wherein **L²** is -NH-. In certain instances, provided is a compound of any of Formulas (**I**) - (**XVIII**), (**XXV**) - (**XXVII**), (**XXX**), (**XXXI**), (**XXXIX**), and (**XL-XLVII**), or a pharmaceutically salt, tautomer, stereoisomer, and/or mixture of stereoisomers thereof, wherein **L²** is -N(Me)-.

In certain instances of any of Formulas (**I**)-(**XVIII**), (**XXV**) - (**XXVII**), (**XXX**), and (**XXXI**), **L¹** is -OCH₂-; and **L²** is -O-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; and **L²** is - CF₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; and **L²** is -CHF-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; and **L²** is -CH(OH)-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; and **L²** is -NH-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; and **L²** is -N(Me)-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -O-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CF₂-; and **L³** is -CH₂-. In certain instances of any of Formulas (**I**)-(**XVIII**), (**XXV**) - (**XXVII**), (**XXX**), and (**XXXI**), **L¹** is -OCH₂-; **L²** is -CHF-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CH(OH)-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -NH-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -N(Me)-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -O-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CF₂-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CHF-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CH(OH)-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -NH-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -N(Me)-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -O-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CF₂-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CHF-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CH(OH)-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -NH-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -N(Me)-; **L³** is -CH₂-; **R²** and **R³** are each H; and **R⁴** is (2S)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -O-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CF₂-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CHF-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CH(OH)-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -NH-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -N(Me)-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -O-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CF₂-; **L³** is -CH₂-; **R²** and **R³** are each H; **R⁴** is (2*S*)-oxetan-2-yl-methyl; and **R³** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CHF-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CH(OH)-; **L³** is -CH₂-; **R²** and **R³** are each H; **R⁴** is (2*S*)-oxetan-2-yl-methyl; and **R⁵** is - COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -O-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CF₂-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CHF-; **L³** is -CH₂-; **R²** and **R³** are each H; **R⁴** is (1-ethyl-1H-imidazol-5-yl)-methyl; and **R⁵** is - COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CH(OH)-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -N(Me)-; **L³** is -CH₂-; **R²** and **R³** are each H; **R⁴** is (1-ethyl-1H-imidazol-5-yl)-methyl; and **R⁵** is - COOH. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), and (**XXXI**), **L¹** is -OCH₂-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -OCH₂-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas (**I**)-(**XVIII**), (**XXV**) - (**XXVII**), (**XXX**), (**XXXI**), (**XXXIX**), and (**XL-XLVII**), **R⁴** is azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (3*R*)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (3*S*)-azetidine-3-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (S)-buta-4-yl-1,3-diol. In certain instances of any of Formulas (**I**)-(**XVIII**), (**XXV**) - (**XXVII**), (**XXX**), (**XXXI**), (**XXXIX**), and (**XL-XLVII**), **R⁴** is butan-1-yl-one. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(cyanomethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-(Fluoromethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-hydroxyl-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is isoxazol-5-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is (1-methyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 1-methoxy-cycloprop-1yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is methoxyethan-2-yl. In certain instances of any of Formulas (**I**)-(**XVIII**), (**XXV**) - (**XXVII**), (**XXX**), and (**XXXI**) **R⁴** is 1-(2-methoxyethyl)-cycloprop-1-yl-methyl. In certain instances of any of Formulas (**I**)-(**XVIII**), (**XXV**) - (**XXVII**), (**XXX**), (**XXXI**), (**XXXIX**), and (**XL-XLVII**), **R⁴** is {[1-(1,2-oxazole-3-carbonyl)azetidin-2-yl]methyl}. In certain instances of any of Formulas (**I**)-(**XVIII**), (**XXV**) - (**XXVII**), (**XXX**), (**XXXI**), (**XXXIX**), and (**XL-XLVII**), **R⁴** is {[(2R)-1-(1,2-oxazole-3-carbonyl)azetidin-2-yl]methyl}. In certain instances of any of Formulas (**I**)-(**XVIII**), (**XXV**) - (**XXVII**), (**XXX**), (**XXXI**), (**XXXIX**), and (**XL-XLVII**), **R⁴** is{[(2S)-1-(1,2-oxazole-3-carbonyl)azetidin-2-yl]methyl. In certain instances of any of Formulas (**I**)-(**XVIII**), (**XXV**) - (**XXVII**), (**XXX**), (**XXXI**), (**XXXIX**), and (**XL-XLVII**), **R⁴** is 2-oxabicyclo[2.1.1]hexa-1-yl-methyl. In certain instances, provided is a compound of any of Formulas (**I**) - (**XVIII**), (**XXV**) - (**XXVII**), (**XXX**), (**XXXI**), (**XXXIX**), and (**XL-XLVII**), or a pharmaceutically salt, tautomer, stereoisomer, and/or mixture of stereoisomers thereof, wherein **L²** is -NH-. In certain instances, provided is a compound of any of Formulas (**I**) - (**XVIII**), (**XXV**) - (**XXVII**), (**XXX**), (**XXXI**), (**XXXIX**), and (**XL-XLVII**), or a pharmaceutically salt, tautomer, stereoisomer, and/or mixture of stereoisomers thereof, wherein **L²** is -N(Me)-.

In certain instances of any of Formulas (**I**)-(**XVIII**), (**XXV**) - (**XXVII**), (**XXX**), (**XXXI**), (**XXXIX**), and (**XL-XLVII**), **L¹** is -NH- and **L²** is -O-. In certain instances of any of Formulas (**I**)-(**XVIII**), (**XXV**) - (**XXVII**), (**XXX**), (**XXXI**), (**XXXIX**), and (**XL-XLVII**), **L¹** is -NH- and **L²** is -CF₂-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; and **L²** is -CHF-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; and **L²** is -CH(OH)-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; and **L²** is -NH-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; and **L²** is -N(Me)-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -O-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CF₂-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CHF-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CH(OH)-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -NH-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -N(Me)-; and **L³** is -CH₂-. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -O-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CF₂-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CHF-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CH(OH)-; **L³** is - CH₂-; and **R²** and **R³** are each H. In certain instances of any of Formulas (**I**)-(**XVIII**), (**XXV**) - (**XXVII**), (**XXX**), and (**XXXI**), **L¹** is -NH-; **L²** is -NH-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -N(Me)-; **L³** is -CH₂-; and **R²** and **R³** are each H. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -O-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CF₂-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CHF-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CH(OH)-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -NH-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -N(Me)-; **L³** is -CH₂-; **R²** and **R³** are each H; and **R⁴** is (2*S*)-oxetan-2-yl-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -O-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CF₂-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CHF-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CH(OH)-; **L³** is - CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -NH-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -N(Me)-; **L³** is -CH₂-; **R²** and **R³** are each H; and **R⁴** is (1-ethyl-1H-imidazol-5-yl)-methyl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -O-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CHF-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CH(OH)-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -O-; **L³** is -CH₂-; **R²** and **R³** are each H; **R⁴** is (1-ethyl-1H-imidazol-5-yl)-methyl; and **R⁵** is - COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CF₂-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CHF-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CH(OH)-; **L³** is - CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (X**XVII**), (**XXX**), (**XXXI**), (**XXXIX**), and (**XL-XLVII**), **L¹** is -NH-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**), (**XXV**) - (**XXVII**), (**XXX**), (**XXXI**), (**XXXIX**), and (**XL-XLVII**), **L¹** is -NH-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -NH-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX),** and **(XXXI) R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI) (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI),** o(**XXXIX**), and **(XL-XLVII),** r a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII),** (XXX), **(XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX),** and **(XL-XLVII), L¹** is -N(Me)- and **L²** is -O-. In certain instances of any of Formulas **(I)-(XVIII), (XXV) - (XXVII), (XXX),** and **(XXXI), L¹** is -N(Me)- and **L²** is - CF₂-. In certain instances of any of Formulas **(I)-(XVIII), (XXV)** - **(XXVII), (XXX), (XXXI), (XXXIX),** and **(XL-XLVII), L¹** is -N(Me)- and **L²** is -CHF-. In certain instances of any of Formulas **(I)-(XVIII), (XXV) - (XXVII), (XXX), (XXXI), (XXXIX),** and **(XL-XLVII), L¹** is -N(Me)- and **L²** is -CH(OH)-. In certain instances of any of Formulas **(I)-(XVIII), (XXV)** - **(XXVII), (XXX), (XXXI), (XXXIX),** and **(XL-XLVII), L¹** is -N(Me)- and **L²** is -NH-. In certain instances of any of Formulas **(I)-(XVIII), (XXV) - (XXVII), (XXX), (XXXI), (XXXIX),** and **(XL-XLVII), L¹** is -N(Me)- and **L²** is -N(Me)-. In certain instances of any of Formulas **(I)-(XVIII), (XXV) - (XXVII), (XXX), (XXXI), (XXXIX),** and **(XL-XLVII), L¹** is -N(Me)-; **L²** is -O-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -CF₂-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Me)-; **L²** is -CHF-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -CH(OH)-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -NH-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)-; **L²** is -O-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)-; **L²** is -CF₂-; **L³** is - CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)-; **L²** is -CHF-; **L³** is - CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)-; **L²** is -CH(OH)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is - CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)-; **L²** is -O-; **L³** is - CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -CF₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -CHF-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -CH(OH)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -O-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -CF₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -CHF-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -CH(OH)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)-; **L²** is -O-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -CHF-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -CH(OH)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is (2*S*)-oxetan-2-yl-methyl; and **R⁵** is - COOH. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -O-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -CF₂-; **L³** is - CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -CHF-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -CH(OH)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is - CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is - N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -N(Me)-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (*S*)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX),** and **(XXXI) R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX),** and **(XL-XLVII), L¹** is -N(Et)-; and **L²** is -O-. In certain instances of any of Formulas **(I)-(XVIII), (XXV) - (XXVII), (XXX),** and **(XXXI), L¹** is -N(Et)-; and **L²** is - CF₂-. In certain instances of any of Formulas **(I)-(XVIII), (XXV) - (XXVII), (XXX), (XXXI), (XXXIX),** and **(XL-XLVII), L¹** is -N(Et)-; and **L²** is -CHF-. In certain instances of any of Formulas **(I)-(XVIII), (XXV) - (XXVII), (XXX), (XXXI), (XXXIX),** and **(XL-XLVII), L¹** is -N(Et)-; and **L²** is -CH(OH)-. In certain instances of any of Formulas **(I)-(XVIII), (XXV)** - **(XXVII), (XXX), (XXXI), (XXXIX),** and **(XL-XLVII), L¹** is -N(Et)-; and **L²** is -NH-. In certain instances of any of Formulas **(I)-(XVIII), (XXV) - (XXVII), (XXX), (XXXI), (XXXIX),** and **(XL-XLVII), L¹** is -N(Et)-; and **L²** is -N(Me)-. In certain instances of any of Formulas **(I)-(XVIII), (XXV) - (XXVII), (XXX),** and **(XXXI), L¹** is -N(Et)-; **L²** is -O-; and **L³** is -CH₂-. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Et)-; **L²** is -CHF-; and **L³** is -CH₂-. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; and **L³** is -CH₂-. In certain instances of any of Formulas **(I)-(XVIII), (XXV) - (XXVII), (XXX), (XXXI), (XXXIX),** and **(XL-XLVII), L¹** is -N(Et)-; **L²** is -NH-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Et)-; **L²** is -N(ME)-; and **L³** is -CH₂-. In certain instances of any of Formulas **(I)-(XVIII), (XXV) - (XXVII), (XXX), (XXXI), (XXXIX),** and **(XL-XLVII), L¹** is -N(Et)-; **L²** is -O-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Et)-; **L²** is -O-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is -CH₂-; **R²** and **R³** are each H; and **R⁴** is (2S)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -O-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Et)-; **L²** is -O-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; **L³** is -CH₂-; **R²** and **R³** are each H; **R⁴** is (2*S*)-oxetan-2-yl-methyl; and **R⁵** is - COOH. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -O-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is - CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX), and (XL-XLVII), R⁴** is (S)-buta-4-yl-1,3-diol. In certain instances of any of Formulas **(I)-(XVIII), (XXV)** - **(XXVII), (XXX),** and **(XXXI) R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX),** and **(XXXI) R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI), (XXXIX),** and **(XL-XVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX),** and **(XL-XLVII), L¹** is -NHCH₂-; and **L²** is -O-. In certain instances of any of Formulas **(I)-(XVIII), (XXV) - (XXVII), (XXX), (XXXI), (XXXIX),** and **(XL-XLVII), L¹** is -NHCH₂-; and **L²** is -CF₂-. In certain instances of any of Formulas **(I)-(XVIII), (XXV) - (XXVII), (XXX), (XXXI), (XXXIX),** and **(XL-XLVII), L¹** is -NHCH₂-; and **L²** is - CHF-. In certain instances of any of Formulas **(I)-(XVIII), (XXV) - (XXVII), (XXX), (XXXI), (XXXIX),** and **(XL-XLVII), L¹** is -NHCH₂-; and **L²** is -CH(OH)-. In certain instances of any of Formulas **(I)-(XVIII), (XXV)** - **(XXVII), (XXX), (XXXI), (XXXIX),** and **(XL-XLVII), L¹** is -NHCH₂-; and **L²** is -NH-. In certain instances of any of Formulas **(I)-(XVIII), (XXV) - (XXVII), (XXX), (XXXI), (XXXIX),** and **(XL-XLVII), L¹** is -NHCH₂-; and **L²** is -N(Me)-. In certain instances of any of Formulas **(I)-(XVIII), (XXV)** - **(XXVII), (XXX), (XXXI), (XXXIX),** and **(XL-XLVII), L¹** is -NHCH₂-; **L²** is -O-; and **L³** is -CH₂-. In certain instances of any of Formulas **(I)-(XVIII), (XXV) - (XXVII), (XXX), (XXXI), (XXXIX),** and **(XL-XLVII), L¹** is -NHCH₂-; **L²** is -CF₂-; and **L³** is -CH₂-. In certain instances of any of Formulas **(I)-(XVIII), (XXV) - (XXVII), (XXX),** and **(XXXI), L¹** is -NHCH₂-; **L²** is -CHF-; and **L³** is -CH₂-. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH(OH)-; and **L³** is -CH₂-. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; and **L³** is -CH₂-. In certain instances of any of Formulas **(I)-(XVIII), (XXV)** - **(XXVII), (XXX), (XXXI), (XXXIX),** and **(XL-XLVII), L¹** is -NHCH₂-; **L²** is -N(Me)-; and **L³** is -CH₂-. In certain instances of any of Formulas **(I)-(XVIII), (XXV) - (XXVII), (XXX),** and **(XXXI), L¹** is -NHCH₂-; **L²** is -O-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NHCH₂-; **L²** is -CF₂-; **L³** is - CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NHCH₂-; **L²** is -CHF-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH(OH)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -NHCH₂-; **L²** is - N(Me)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -NHCH₂-; **L²** is -O-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CF₂-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CHF-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -CH(OH)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -O-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CF₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -CHF-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -CH(OH)-; **L³** is -CH₂-; **R²** and **R³** are each H; and **R⁴** is (1-ethyl-1H-imidazol-5-yl)-methyl. n certain instances of any of Formulas **(I)-(XVIII), (XXV) - (XXVII), (XXX), (XXXI), (XXXIX),** and **(XL-XLVII), L¹** is -NHCH₂-; **L²** is -NH-; **L³** is -CH₂-; **R²** and **R³** are each H; and **R⁴** is (1-ethyl-1H-imidazol-5-yl)-methyl. n certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -O-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -CHF-; **L³** is -CH₂-; **R²** and **R³** are each H; **R⁴** is (2*S*)-oxetan-2-yl-methyl; and **R⁵** is - COOH. In certain instances of any of Formulas **(I)-(XVIII), (XXV) - (XXVII), (XXX), (XXXI), (XXXIX),** and **(XL-XLVII), L¹** is -NHCH₂-; **L²** is -CH(OH)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -O-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -CF₂-; **L³** is -CH₂-; **R²** and **R³** are each H; **R⁴** is (1-ethyl-1H-imidazol-5-yl)-methyl; and **R⁵** is - COOH. In certain instances of any of Formulas **(I)-(XVIII), (XXV) - (XXVII), (XXX), (XXXI), (XXXIX),** and **(XL-XLVII), L¹** is -NHCH₂-; **L²** is -CHF-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -CH(OH)-; **L³** is -CH₂-; **R²** and **R³** are each H; **R⁴** is (1-ethyl-IH-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is (1-ethyl-1H-imidazol-5-yl)-methyl; and **R³** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -NHCH₂-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI) (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX),** and **(XL-XLVII), L¹** is -N(Me)CH₂-; and **L²** is -O-. In certain instances of any of Formulas **(I)-(XVIII), (XXV) - (XXVII), (XXX), (XXXI), (XXXIX),** and **(XL-XLVII), L¹** is -N(Me)CH₂-; and **L²** is -CF₂-. In certain instances of any of Formulas **(I)-(XVIII), (XXV) - (XXVII), (XXX), (XXXI), (XXXIX),** and **(XL-XLVII), L¹** is -N(Me)CH₂-; and **L²** is -CHF-. In certain instances of any of Formulas **(I)-(XVIII), (XXV)** - **(XXVII), (XXX), (XXXI), (XXXIX),** and **(XL-XLVII), L¹** is -N(Me)CH₂-; and **L²** is -CH(OH)-. In certain instances of any of Formulas **(I)-(XVIII), (XXV) - (XXVII), (XXX), (XXXI), (XXXIX),** and **(XL-XLVII), L¹** is -N(Me)CH₂-; and **L²** is -NH-. In certain instances of any of Formulas **(I)-(XVIII), (XXV) - (XXVII), (XXX), (XXXI), (XXXIX),** and **(XL-XLVII), L¹** is -N(Me)CH₂-; and **L²** is -N(Me)-. In certain instances of any of Formulas **(I)-(XVIII), (XXV) - (XXVII), (XXX), (XXXI), (XXXIX),** and **(XL-XLVII), L¹** is -N(Me)CH₂-; **L²** is -O-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CF₂-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH(OH)-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)CH₂-; **L²** is -NH-; and **L³** is -CH₂-. In certain instances of any of Formulas **(I)-(XVIII), (XXV) - (XXVII), (XXX), (XXXI), (XXXIX),** and **(XL-XLVII), L¹** is -N(Me)CH₂-; **L²** is -N(Me)-; and **L³** is -CH₂-. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -O-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CF₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH(OH)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Me)CH₂-; **L²** is - NH-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -N(Me)CH₂-; **L²** is -N(Me)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is - N(Me)CH₂-; **L²** is -O-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CF₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is - CHF-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII),** (XXX), **(XXXI), (XXXIX),** and **(XL-XLVII), L¹** is -N(Me)CH₂-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -N(Me)-; **L³** is -CH₂-; **R²** and R³ are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -O-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CF₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -O-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)CH₂-; **L²** is -NH-; **L³** is -CH₂-; **R²** and **R³** are each H; **R⁴** is (2*S*)-oxetan-2-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)CH₂-; **L²** is -O-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CF₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)CH₂-; **L²** is-NH-; **L³** is -CH₂-; **R²** and **R³** are each H; **R⁴** is (1-ethyl-1H-imidazol-5-yl)-methyl; and **R⁵** is-COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII)**, **(XXV)** - **(XXVII), (XXX), (XXXI), (XXXIX),** and **(XL-XLVII), L¹** is -N(Me)CH₂-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹ is** -N(Me)CH₂-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX),** and **(XXXI), L¹** is - N(Me)CH₂-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas (**I**)-(**XVIII**)**,** (**XXV**) - (**XXVII**), (**XXX**), (**XXXI**), (**XXXIX**), and (**XL-XLVII**), **L¹** is -N(Me)CH₂-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX),** and **(XXXI), L¹** is -N(Me)CH₂-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -N(Me)CH₂-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3R)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3S)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), R⁴** is 1-(cyanomethyl)-cycloprop-1-yl-methyl. In certain instances of any of Formulas **(I(XXXIX),** and (**XL-XLVII**),)-(**XVIII**), **(XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI),** (**XXXIX**), and (**XL-XLVII**), **R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX),** and **(XL-XLVII), L¹** is -OC(H,Me)-; and **L²** is -O-. In certain instances of any of Formulas **(I)-(XVIII), (XXV) - (XXVII), (XXX), (XXXI), (XXXIX),** and **(XL-XLVII), L¹** is -OC(H,Me)-; and **L²** is -CF₂-. In certain instances of any of Formulas **(I)-(XVIII), (XXV) - (XXVII), (XXX), (XXXI), (XXXIX),** and **(XL-XLVII), L¹** is -OC(H,Me)-; and **L²** is -CHF-. In certain instances of any of Formulas **(I)-(XVIII), (XXV)** - **(XXVII), (XXX), (XXXI), (XXXIX),** and **(XL-XLVII), L¹** is -OC(H,Me)- and **L²** is -CH(OH)-. In certain instances of any of Formulas **(I)-(XVIII), (XXV)** - **(XXVII), (XXX), (XXXI), (XXXIX),** and **(XL-XLVII), L¹** is -OC(H,Me)- and **L²** is -NH-. In certain instances of any of Formulas **(I)-(XVIII)**, **(XXV)** - **(XXVII)**, **(XXX)**, **(XXXI)**, **(XXXIX)**, and **(XL-XLVII)**, **L¹** is -OC(H,Me)- and **L²** is -N(Me)-. In certain instances of any of Formulas **(I)-(XVIII), (XXV) - (XXVII), (XXX), (XXXI), (XXXIX),** and **(XL-XLVII), L¹** is -OC(H,Me)-; **L²** is -O-; and **L³** is -CH₂-. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CF₂-; and **L³** is -CH₂-. In certain instances of any of Formulas **(I)-(XVIII), (XXV) - (XXVII), (XXX), (XXXI), (XXXIX),** and **(XL-XLVII), L¹** is -OC(H,Me)-; **L²** is -CHF-; and **L³** is -CH₂-. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH(OH)-; and **L³** is -CH₂-. In certain instances of any of Formulas **(I)-(XVIII), (XXV) - (XXVII), (XXX), (XXXI), (XXXIX),** and **(XL-XLVII), L¹** is -OC(H,Me)-; **L²** is -NH-; and **L³** is -CH₂-. In certain instances of any of Formulas **(I)-(XVIII), (XXV) - (XXVII), (XXX), (XXXI), (XXXIX),** and **(XL-XLVII), L¹** is -OC(H,Me)-; **L²** is -NH(Me)-; and **L³** is -CH₂-. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -O-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CF₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CHF-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH(OH)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -OC(H,Me)-; **L²** is-NH-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is -OC(H,Me)-; **L²** is -N(Me)-; **L³** is -CH₂-; and **R²** and **R³** are each H. In certain instances of any of Formulas **(I)-(XVIII), (XXV) - (XXVII), (XXX), (XXXI), (XXXIX),** and **(XL-XLVII), L¹** is - OC(H,Me)-; **L²** is -O-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹ is** -OC(H,Me)-; **L²** is -CF₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -OC(H,Me)-; **L²** is - CHF-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; and **R⁴** is (2*S*)-oxetan-2-yl-methyl. In certain instances of any of Formulas **(I)-(XVIII), (XXV)-(XXVII), (XXX), (XXXI), (XXXIX),** and **(XL-XLVII), L¹** is -OC(H,Me)-; **L²** is -NH-; **L³** is -CH₂-; **R²** and **R³** are each H; and **R⁴** is (2*S*)-oxetan-2-yl-methyl. In certain instances of any of Formulas **(I)-(XVIII)**, **(XXV) - (XXVII), (XXX), (XXXI), (XXXIX),** and **(XL-XLVII), L¹** is -OC(H,Me)-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -O-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII),** (**XXX**), **(XXXI), (XXXIX),** and **(XL-XLVII), L¹** is -OC(H,Me)-; **L²** is -CF₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CHF-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII),** (**XXX**), **(XXXI), (XXXIX),** and **(XL-XLVII), L¹** is -OC(H,Me)-; **L²** is -CH(OH)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -NH-; **L³** is -CH₂-; **R²** and **R³** are each H; and **R⁴** is (1-ethyl-1H-imidazol-5-yl)-methyl. In certain instances of any of Formulas **(I)-(XVIII)**, **(XXV)** - **(XXVII), (**XXX**), (XXXI), (XXXIX),** and **(XL-XLVII), L¹** is -OC(H,Me)-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -O-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹ is** -OC(H,Me)-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)-(XXVII), (XXX), (XXXI), (XXXIX),** and **(XL-XLVII), L¹** is -OC(H,Me)-; **L²** is -CHF-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is (2*S*)-oxetan-2-yl-methyl; and **R⁵** is-COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OC(H,Me)-; **L²** is -O-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV)-(XXVII), (XXX),** and **(XXXI), L¹** is -OC(H,Me)-; **L²** is -CF₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OC(H,Me)-; **L²** is -CHF-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)-(XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -O-; **L³** is-CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)-(XXVII), (XXX), (XXXI), (XXXIX),** and **(XL-XLVII), L¹** is -OC(H,Me)-; **L²** is -CF₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)-(XXVII), (XXX), (XXXI), (XXXIX),** and **(XL-XLVII), L¹** is -OC(H,Me)-; **L²** is -CHF-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)-(XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)-(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)-(XXVII), (XXX), (XXXI), (XXXIX),** and **(XL-XLVII), L¹** is -OC(H,Me)-; **L²** is -O-; **L³** is-CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CF₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -OC(H,Me)-; **L²** is-CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is (1-ethyl-IH-imidazol-5-yl)-methyl; and **R⁵** is tetrazolyl, for instance 1H-1,2,3,4-tetrazol-5-yl. In any instance according to this paragraph, **R'** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII),** (**XXX**), **(XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII),** (**XXX**), **(XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴**is{[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas (I) **- (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)-(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV) - (XXVII), (XXX), (XXXI), (XXXIX),** and **(XL-XLVII), L¹** is -SO₂NH-; and **L²** is -O-. In certain instances of any of Formulas **(I)-(XVIII), (XXV) - (XXVII), (XXX), (XXXI), (XXXIX),** and **(XL-XLVII), L¹** is -SO₂NH-; and **L²** is -CF₂-. In certain instances of any of Formulas **(I)-(XVIII), (XXV) - (XXVII), (XXX), (XXXI),** (XXXIX), and **(XL-XLVII), L¹ is** -SO₂NH-; and **L²** is-CHF-. In certain instances of any of Formulas **(I)-(XVIII), (XXV) - (XXVII), (XXX), (XXXI), (XXXIX),** and **(XL-XLVII), L¹** is -SO₂NH-; and L**²** is -CH(OH)-. In certain instances of any of Formulas **(I)-(XVIII), (XXV) - (XXVII), (XXX), (XXXI), (XXXIX),** and **(XL-XLVII), L¹** is -SO₂NH-; and **L²** is -NH-. In certain instances of any of Formulas **(I)-(XVIII), (XXV) - (XXVII), (XXX), (XXXI), (XXXIX),** and **(XL-XLVII), L¹ is** -SO₂NH-; and **L²** is-N(Me)-. In certain instances of any of Formulas **(I)-(XVIII), (XXV) - (XXVII), (XXX), (XXXI), (XXXIX),** and **(XL-XLVII), L¹** is -SO₂NH-; **L²** is -O-; and **L³** is -CH₂-. In certain instances of any of Formulas **(I)-(XVIII), (XXV) - (XXVII), (XXX), (XXXI), (XXXIX),** and **(XL-XLVII), L¹** is -SO₂NH-; **L²** is -CF₂-; and **L³** is -CH₂-. In certain instances of any of Formulas **(I)-(XVIII), (XXV) - (XXVII), (XXX), (XXXI), (XXXIX),** and **(XL-XLVII), L¹** is -SO₂NH-; **L²** is -CHF-; and **L³** is -CH₂-. In certain instances of any of Formulas **(I)-(XVIII), (XXV) - (XXVII), (XXX), (XXXI), (XXXIX),** and **(XL-XLVII), L¹** is -SO₂NH-; **L²** is-CH(OH)-; and **L³** is -CH₂-. In certain instances of any of Formulas **(I)-(XVIII), (XXV)-(XXVII), (XXX), (XXXI), (XXXIX),** and **(XL-XLVII), L¹** is -SO₂NH-; **L²** is -NH-; and **L³** is -CH₂-. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; and **L³** is -CH₂-. In certain instances of any of Formulas **(I)-(XVIII), (XXV)** - **(XXVII), (XXX), (XXXI), (XXXIX),** and **(XL-XLVII), L¹** is -SO₂NH-; **L²** is -O-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -CHF-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH(OH)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; and **R²** and **R³** are each H. In certain instances of any of Formulas **(I)-(XVIII), (XXV)** - **(XXVII), (XXX), (XXXI), (XXXIX),** and **(XL-XLVII), L¹** is -SO₂NH-; **L²** is -O-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -CHF-; **L³** is -CH₂-; **R²** and **R³** are each H; and **R⁴** is (2S)-oxetan-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH(OH)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII),** (**XXX**), **(XXXI), (XXXIX),** and **(XL-XLVII), L¹** is -SO₂NH-; **L²** is -NH-; **L³** is -CH₂-; **R²** and **R³** are each H; and **R⁴** is (2*S*)-oxetan-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; **R²** and **R³** are each H; and **R⁴** is (2*S*)-oxetan-2-yl-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -O-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CHF-; **L³** is -CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -CH(OH)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII),** (XXX), **(XXXI), (XXXIX),** and **(XL-XLVII), L¹** is -SO₂NH-; **L²** is -NH-; **L³** is-CH₂-; **R²** and **R³** are each H; and **R⁴** is (1-ethyl-1H-imidazol-5-yl)-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -N(Me)-; **L³** is -CH₂-; **R²** and **R³** are each H; and **R⁴** is (1-ethyl-1H-imidazol-5-yl)-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -O-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -CF₂-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -CHF-; **L³** is-CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH(OH)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -NH-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹ is** -SO₂NH-; **L²** is -N(Me)-; **L³** is -CH₂-; **R²** and **R³** are each H; **R⁴** is (2*S*)-oxetan-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -O-; **L³** is -CH₂-; **R²** and **R³** are each H; **R⁴** is (1-ethyl-1H-imidazol-5-yl)-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -CHF-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is - CH(OH)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L'is** -SO₂NH-; **L²** is -N(Me)-; **L³** is -CH₂-; **R²** and **R³** are each H; **R⁴** is (1-ethyl-1H-imidazol-5-yl)-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** i**s** -SO₂NH-; **L²** is -CF₂-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CHF-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** i**s** -SO₂NH-; **L²** is -CH(OH)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is - CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is - CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -SO₂NH-; **L²** is -CHF-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -SO₂NH-; **L²** is - CH(OH)-; **L³** is -CH₂-; R² and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -SO₂NH-; **L²** is -NH-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloproplyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴**is{[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** i**s** -O-; **L²** is -O-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -CF₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -O-; **L²** is -CHF-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -O-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is - CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -O-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** i**s** -O-; **L²** is -CF₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -O-; **L²** is -CH(OH)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** i**s** -O-; **L²** is -O-; **L³** is - CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -O-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -CH(OH)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** i**s** -O-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -O-; **L³** is -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -CF₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -CHF-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -CH(OH)-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -O-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -O-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -CF₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX),** and **(XXXI), L¹** is -O-; **L²** is -CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is - N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -CF₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -O-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -O-; **L²** is -CH(OH)-; **L³** is -CH₂-; **R²** and R³ are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -O-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII),** (**XXX**), **(XXXI),** (**XXXIX**), and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII), (XXX), (XXXI), (XXXIX),** and **(XL-XLVII), R⁴** is (1-methyl-1H-imidazol-5-yl)-methyl. In certain instances of any of Formulas **(I)-(XVIII),** (XXV) - **(XXVII),** (XXX), **(XXXI), (XXXIX),** and **(XL-XLVII), R⁴** is 1-methoxy-cycloproplyl-methyl. In certain instances of any of Formulas **(I)-(XVIII),** (XXV) - **(XXVII),** (XXX), **(XXXI), (XXXIX),** and **(XL-XLVII), R⁴** is methoxyethan-2-yl. In certain instances of any of Formulas (I)-(XVIII), (XXV) - (XXVII), (**XXX**), (XXXI), (**XXXIX**), and (XL-XLVII), **R⁴** is 1-(2-methoxyethyl)-cycloprop-1-yl-methyl. In certain instances of any of Formulas **(I)-**(XVIII), (XXV) - (XXVII), (XXX), (XXXI), (XXXIX), and (XL-XLVII), **R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; **R²** and **R³** are each H; and **R⁴** is oxelan-3-yl-methyl. In certain instances of any of Formulas **(I)-(XVIII),** (XXV) - **(XXVII), (XXX), (XXXI), (XXXIX),** and **(XL-XLVII), L¹** is -CH₂O-; **L²** is -CF₂-; **L³** is - CH₂-; **R²** and **R³** are each H; and **R⁴** is oxelan-3-yl-methyl. In certain instances of any of Formulas Formulas **(I)-(XVIII), (XXV) - (XXVII), (XXX), (XXXI), (XXXIX),** and **(XL-XLVII), L¹** is -CH₂O-; **L²** is -CHF-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** i**s** -CH₂O-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -CF₂-; **L³** is - CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH(OH)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** i**s** -CH₂O-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -CH(OH)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -O-; **L³** is -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -CF₂-; **L³** is - CH₂-; **R²** and **R³** are each H; **R⁴** is 1,3-oxazol-2-yl-methyl; and **R⁵** is -COOH. In certain instances of any of Formulas **(I)-(XVIII),** (XXV) - **(XXVII), (XXX), (XXXI), (XXXIX),** and **(XL-XLVII), L¹** is -CH₂O-; **L²** is -CHF-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX),** and **(XXXI), L¹** is -CH₂O-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -CH₂O-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -CF₂-; **L³** is - CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -CHF-; **L³** is -CH₂-; **R²** and **R³ are** each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -CH₂O-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -CH₂O-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -CH₂O-; **L²** is -CF₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -CH₂O-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -CH₂O-; **L²** is -CH(OH)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -CH₂O-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -CH₂O-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -OCH₂-; **L²** is -O-; **L³** is -CH₂-; **R²** and **R³** are each H; and **R⁴** is oxelan-3-yl-methyl. In certain instances of any of Formulas **(I)-(XVIII),** (XXV) - **(XXVII), (XXX), (XXXI), (XXXIX),** and **(XL-XLVII), L¹** is -OCH₂-; **L²** is -CF₂-; **L³** is - CH₂-; **R²** and **R³** are each H; and **R⁴** is oxelan-3-yl-methyl. In certain instances of any of Formulas **(I)-(XVIII), (XXV) - (XXVII),** (**XXX**), **(XXXI), (XXXIX),** and **(XL-XLVII), L¹** is -OCH₂-; **L²** is -CHF-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is - CH₂-; **R²** and **R³** are each H; and **R⁴** is oxelan-3-yl-methyl. In certain instances of any of Formulas **(I)-(XVIII), (XXV) - (XXVII),** (**XXX**), **(XXXI), (XXXIX),** and **(XL-XLVII), L¹** is -OCH₂-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -CF₂-; **L³** is - CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OCH₂-; **L²** is -CH(OH)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -OCH₂-; **L²** is -O-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -CH(OH)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -OCH₂-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -O-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -CF₂-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -CHF-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -N(Me)-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OCH₂-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OCH₂-; **L²** is -CF₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OCH₂-; **L²** is -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OCH₂-; **L²** is - CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -OCH₂-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -N(Me)-; **L³** is - CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -CF₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -OCH₂-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -CH(OH)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OCH₂-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and (**XL-XLVII**), **L¹** is -OCH₂-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, - C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII),** (**XXX**), **(XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII),** (**XXX**), **(XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -NH-; **L²** is -O-; **L³** is -CH₂-; **R²** and **R³** are each H; and **R⁴** is oxelan-3-yl-methyl. In certain instances of any of Formulas **(I)-(XVIII), (XXV)** - **(XXVII),** (XXX), **(XXXI), (XXXIX),** and **(XL-XLVII), L¹** is -NH-; **L²** is -CF₂-; L³ is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -NH-; **L²** is -CHF-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -NH-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is - CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -NH-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -CF₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -NH-; **L²** is -CH(OH)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is - CH₂-; **R²** and **R³** are each H; and **R⁴** is 1,3-oxazol-2-yl-methyl. In certain instances of any of Formulas **(I)-(XVIII), (XXV) - (XXVII),** (**XXX**), **(XXXI), (XXXIX),** and **(XL-XLVII), L¹** is -NH-; **L²** is -O-; **L³** is -CH₂-; **R²** and **R³** are each H; **R⁴ is** oxelan-3-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -NH-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -NH-; **L²** is -CH(OH)-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -O-; **L³** is -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -CF₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -CHF-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -NH-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NH-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -CF₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII),** (**XXX**), **(XXXI), (XXXIX),** and **(XL-XLVII), L¹** is -NH-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII),** (**XXX**), **(XXXI), (XXXIX),** and **(XL-XLVII), L¹** is -NH-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NH-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -CF₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -NH-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -CH(OH)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NH-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, - C(O)NMe₂, alkyl, propyl, isopropyl, and cyclopropyl. In certain instances of any of Formulas **(I)-(XVIII), (XXV) - (XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV) - (XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV) - (XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII),** (**XXX**), **(XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII),** (**XXX**), **(XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -N(Me)-; **L²** is -O-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -CF₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -CHF-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is - CH₂-; **R²** and **R³** are each H; and **R⁴** is oxelan-3-yl-methyl. In certain instances of any of Formulas **(I)-(XVIII), (XXV)** - **(XXVII),** (**XXX**), **(XXXI), (XXXIX),** and **(XL-XLVII), L¹** is -N(Me)-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -CF₂-; **L³** is - CH₂-; **R²** and **R³** are each H; and **R⁴** is 1,3-oxazol-2-yl-methyl. In certain instances of any of Formulas **(I)-(XVIII), (XXV) - (XXVII),** (**XXX**), **(XXXI), (XXXIX),** and **(XL-XLVII), L¹** is -N(Me)-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -CH(OH)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)-; **L²** is -O-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -N(Me)-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L² is** -CH(OH)-; **L³** is -CH₂-; **R²** and **R³** are each H; **R⁴** is oxelan-3-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is oxelan-3-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -O-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII),** (**XXX**), **(XXXI), (XXXIX),** and **(XL-XLVII), L¹** is -N(Me)-; **L²** is -CF₂-; L³ is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -CHF-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -N(Me)-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; L³ is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)-; **L²** is -O-; L³ is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -CF₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII),** (XXV) - **(XXVII), (XXX), (XXXI), (XXXIX),** and **(XL-XLVII), L¹** is -N(Me)-; **L²** is -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII),** (**XXX**), **(XXXI), (XXXIX),** and **(XL-XLVII), L¹** is -N(Me)-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -CF₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV) - (XXVII), (XXX), (XXXI), (XXXIX),** and **(XL-XLVII), L¹** is -N(Me)-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)-; **L²** is -CH(OH)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -NH-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV) - (XXVII),** (**XXX**), **(XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII),** (**XXX**), **(XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX),** and **(XXXI) R⁴** is (1-methyl-1H-imidazol-5-yl)-methyl. In certain instances of any of Formulas **(I)-(XVIII), (XXV) - (XXVII),** (**XXX**), **(XXXI), (XXXIX),** and **(XL-XLVII), R⁴** is 1-methoxy-cycloprop-lyl-methyl. In certain instances of any of Formulas **(I)-(XVIII), (XXV) - (XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I) - (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -N(Et)-; **L²** is -O-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is - CH₂-; **R²** and **R³** are each H; and **R⁴** is oxelan-3-yl-methyl. In certain instances of any of Formulas **(I)-(XVIII), (XXV) - (XXVII),** (**XXX**), **(XXXI), (XXXIX),** and **(XL-XLVII), L¹** is -N(Et)-; **L²** is -CHF-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is - CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -O-; L³ is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -CF₂-; **L³** is - CH₂-; **R²** and **R³** are each H; and **R⁴** is 1,3-oxazol-2-yl-methyl. In certain instances of any of Formulas **(I)-(XVIII), (XXV) - (XXVII),** (**XXX**), **(XXXI), (XXXIX),** and **(XL-XLVII), L¹** is -N(Et)-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -CH(OH)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Et)-; **L²** is -O-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³ is** -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -O-; **L³** is -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -CF₂-; **L³** is - CH₂-; **R²** and **R³** are each H; **R⁴** is 1,3-oxazol-2-yl-methyl; and **R⁵** is -COOH. In certain instances of any of Formulas **(I)-(XVIII),** (XXV) - **(XXVII), (XXX), (XXXI), (XXXIX),** and **(XL-XLVII), L¹** is -N(Et)-; **L²** is -CHF-; **L³** is -CH₂-; **R²** and R**³ are** each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Et)-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -CF₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is - CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -N(Me)-; **L³** is - CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Et)-; **L²** is -CF₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -N(Et)-; **L²** is -CHF-; **L³** is -CH₂-; **R²** and R**³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -CH(OH)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Et)-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII),** (**XXX**), **(XXXI),** (**XXXIX**), and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV) - (XXVII),** (**XXX**), **(XXXI),** (**XXXIX**), and **(XL-XLVII), R⁴** is (1-methyl-1H-imidazol-5-yl)-methyl. In certain instances of any of Formulas **(I)-(XVIII), (XXV) - (XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV)** - **(XXVII),** (**XXX**), **(XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV) - (XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas (I) **- (XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV) - (XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -NHCH₂-; **L²** is -O-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -CF₂-; **L³** is -CH₂-; **R²** and **R³** are each H; and **R⁴** is oxelan-3-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -CHF-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is - CH₂-; **R²** and **R³** are each H; and **R⁴** is oxelan-3-yl-methyl. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CF₂-; L³ is - CH₂-; **R²** and **R³** are each H; and **R⁴** is 1,3-oxazol-2-yl-methyl. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH(OH)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -NHCH₂-; **L²** is -O-; L³ is -CH₂-; **R²** and **R³** are each H; **R⁴** is oxelan-3-yl-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -NHCH₂-; **L²** is -CF₂-; L³ is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; L² is -CH(OH)-; **L³** is -CH₂-; **R²** and **R³** are each H; **R⁴** is oxelan-3-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; L³ is -CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is oxelan-3-yl-methyl; and **R⁵** is - COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -O-; L³ is -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII),** (**XXX**), **(XXXI),** (**XXXIX**), and **(XL-XLVII), L¹** is -NHCH₂-; **L²** is -CF₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -CHF-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -NHCH₂-; **L²** is -O-; **L³** is - CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CF₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -NHCH₂-; **L²** is -CF₂-; **L³ is** -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -NHCH₂-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -CH(OH)-; **L³ is** -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -NHCH₂-; **L²** is -NH-; **L³ is** -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX), and (XL-XLVII), L¹** is -NHCH₂-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.
In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -O-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CF₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is - NH-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is - N(Me)CH₂-; **L²** is -CF₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is - CH(OH)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -NH-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII),** (**XXX**), **(XXXI), (XXXIX),** and **(XL-XLVII), L¹** is -N(Me)CH₂-; **L²** is -O-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is - N(Me)CH₂-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; **R⁴** is oxelan-3-yl-methyl; and **R⁵** is -COOH. In certain instances of any of Formulas **(I)-(XVIII), (XXV) - (XXVII),** (**XXX**), **(XXXI), (XXXIX),** and **(XL-XLVII), L¹** is -N(Me)CH₂-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII),** (**XXX**), **(XXXI), (XXXIX),** and **(XL-XLVII), L¹** is -N(Me)CH₂-; **L²** is -O-; **L³ is** -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CF₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is - N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CF₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -N(Me)CH₂-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII),** (**XXX**), **(XXXI), (XXXIX),** and **(XL-XLVII), L¹** is -N(Me)CH₂-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII),** (**XXX**), **(XXXI), (XXXIX),** and **(XL-XLVII), L¹** is -N(Me)CH₂-; **L²** is -CF₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII),** (**XXX**), **(XXXI), (XXXIX),** and **(XL-XLVII), L¹** is -N(Me)CH₂-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -CH(OH)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -N(Me)CH₂-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*R*)-azetidine-3-yl-methyl. In certain instances of any of Formulas **(I)-(XVIII), (XXV)** - **(XXVII),** (**XXX**), **(XXXI), (XXXIX),** and **(XL-XLVII), R⁴** is (3S)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV)** - **(XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII),** (**XXX**), **(XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV)** - **(XXVII),** (**XXX**), **(XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV) - (XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R**⁴is{[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CF₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is - CHF-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -NH-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CF₂-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is - OC(H,Me)-; **L²** is -CHF-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is - NH-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -O-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII),** (**XXX**), **(XXXI), (XXXIX),** and **(XL-XLVII), L¹** is -OC(H,Me)-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CHF-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is - OC(H,Me)-; **L²** is -CH(OH)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -OC(H,Me)-; **L²** is -NH-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII),** (**XXX**), **(XXXI), (XXXIX),** and **(XL-XLVII), L¹** is -OC(H,Me)-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -O-; **L³** is -CH₂-; **R²** and **R³** are each H; **R⁴** is 1,3-oxazol-2-yl-methyl; ; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CF₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CHF-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is - NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII),** (**XXX**), **(XXXI), (XXXIX),** and **(XL-XLVII), L¹** is -OC(H,Me)-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is - OC(H,Me)-; **L²** is -CF₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L² is** -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is - CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is - NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -O-; **L³ is** - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CF₂-; **L³ is** -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -CH(OH)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -OC(H,Me)-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII),** (XXV) - **(XXVII), (XXX), (XXXI), (XXXIX),** and **(XL-XLVII), R⁴** is azetidine-3-yl-methyl. In certain instances of any of Formulas **(I)-(XVIII), (XXV)** - **(XXVII),** (**XXX**), **(XXXI), (XXXIX),** and **(XL-XLVII), R⁴** is (3*R*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV)** - **(XXVII),** (**XXX**), **(XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of Formulas **(I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV)** - **(XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances of any of Formulas **(I)-(XVIII), (XXV)** - **(XXVII), (XXX), (XXXI), (XXXIX),** and **(XL-XLVII), L¹** is -SO₂NH-; **L²** is -O-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CHF-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH(OH)-; **L³** is -CH₂-; **R²** and **R³** are each H; and **R⁴** is oxelan-3-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is - CH₂-; **R²** and **R³** are each H; and **R⁴** is oxelan-3-yl-methyl. In certain instances of any of Formulas **(I)-(XVIII), (XXV)** - **(XXVII),** (**XXX**), **(XXXI), (XXXIX),** and **(XL-XLVII), L¹** is -SO₂NH-; **L²** is -O-; **L³** is -CH₂-; **R²** and **R³** are each H; and **R⁴** is 1,3-oxazol-2-yl-methyl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is -CH₂-; **R²** and **R³** are each H; and **R⁴** is 1,3-oxazol-2-yl-methyl. In certain instances of any of Formulas Formulas **(I)-(XVIII), (XXV)** - **(XXVII), (XXX), (XXXI), (XXXIX),** and **(XL-XLVII), L¹** is -SO₂NH-; **L²** is -CHF-; **L³** is -CH₂-; **R²** and **R³** are each H; and **R⁴** is 1,3-oxazol-2-yl-methyl. In certain instances of any of Formulas Formulas **(I)-(XVIII), (XXV)** - **(XXVII), (XXX), (XXXI), (XXXIX),** and **(XL-XLVII), L¹** is -SO₂NH-; **L²** is -CH(OH)-; **L³** is -CH₂-; **R²** and **R³** are each H; and **R⁴** is 1,3-oxazol-2-yl-methyl. In certain instances of any of Formulas Formulas **(I)-(XVIII), (XXV)** - **(XXVII), (XXX), (XXXI), (XXXIX),** and **(XL-XLVII), L¹** is -SO₂NH-; **L²** is -NH-; **L³** is -CH₂-; **R²** and **R³** are each H; and **R⁴** is 1,3-oxazol-2-yl-methyl. In certain instances of any of Formulas Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; **R²** and **R³** are each H; and **R⁴** is 1,3-oxazol-2-yl-methyl. In certain instances of any of Formulas **(I)-(XVIII), (XXV) - (XXVII), (XXX), (XXXI), (XXXIX),** and **(XL-XLVII), L¹** is -SO₂NH-; **L²** is -O-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -SO₂NH-; **L²** is -CHF-; **L³** is - CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -CH(OH)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is - CH₂-; **R²** and **R³** are each H; **R⁴** is oxelan-3-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is oxelan-3-yl-methyl; and **R⁵** is -COOH. In certain instances of any of Formulas **(I)-(XVIII), (XXV)** - **(XXVII),** (XXX), **(XXXI), (XXXIX),** and **(XL-XLVII), L¹** is -SO₂NH-; **L²** is -O-; **L³** is -CH₂-; **R² and R³** are each H; **R⁴** is 1,3-oxazol-2-yl-methyl; ; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -CF₂-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -CHF-; **L³** is - CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH(OH)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -NH-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is 1,3-oxazol-2-yl-methyl; and **R⁵** is -COOH. In certain instances of any of Formulas **(I)-(XVIII), (XXV) - (XXVII), (XXX), (XXXI), (XXXIX),** and **(XL-XLVII), L¹** is -SO₂NH-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CF₂-; **L³** is - CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CHF-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -CH(OH)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -NH-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV)** - **(XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -SO₂NH-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -SO₂NH-; **L²** is -CF₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl In certain instances of any of Formulas **(I)-(XVIII), (XXV) - (XXVII),** (**XXX**), **(XXXI), (XXXIX),** and **(XL-XLVII), L¹** is -SO₂NH-; **L²** is -CHF-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII),** (**XXX**), **(XXXI), (XXXIX),** and **(XL-XLVII), L¹** is -SO₂NH-; **L²** is -CH(OH)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII),** (**XXX**), **(XXXI), (XXXIX),** and **(XL-XLVII), L¹** is -SO₂NH-; **L²** is -NH-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain instances of any of Formulas **(I)-(XVIII), (XXV) - (XXVII), (XXX), (XXXI), (XXXIX),** and **(XL-XLVII), L¹** is -SO₂NH-; **L²** is -N(Me)-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any instance according to this paragraph, **R¹** can be selected from H, Cl, F, Me, -CF₃, -OMe, -CN, -C(O)NMe₂, alkyl, propyl, isopropyl, and cyclopropyl. In certain instances of any of Formulas **(I)-(XVIII), (XXV)** - **(XXVII), (XXX), (XXXI), (XXXIX),** and **(XL-XLVII), R⁴is** azetidine-3-yl-methyl. In certain instances of any of Formulas **(I)-(XVIII), (XXV) - (XXVII), (XXX), (XXXI), (XXXIX),** and **(XL-XLVII), R⁴** is (3*R*)-azetidine-3-yl-methyl. In certain instances of any of Formulas **(I)-(XVIII), (XXV) - (XXVII), (XXX), (XXXI), (XXXIX),** and **(XL-XLVII), R⁴** is (3*S*)-azetidine-3-yl-methyl. In certain instances of any of Formulas **(I)-(XVIII), (XXV)** - **(XXVII), (XXX), (XXXI), (XXXIX),** and **(XL-XLVII), R⁴** is (S)-buta-4-yl-1,3-diol. In certain instances of any of Formulas **(I)-(XVIII), (XXV) - (XXVII), (XXX), (XXXI), (XXXIX),** and **(XL-XLVII), R⁴** is butan-1-yl-one. In certain instances of any of Formulas **(I)-(XVIII), (XXV)** - **(XXVII), (XXX), (XXXI), (XXXIX),** and **(XL-XLVII), R⁴** is 1-(cyanomethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-(Fluoromethyl)-cycloprop-1-yl-methyl. In certain instances of any of Formulas **(I)-(XVIII), (XXV)** - **(XXVII), (XXX), (XXXI), (XXXIX),** and **(XL-XLVII), R⁴** is 1-hydroxyl-cycloprop-1-yl-methyl. In certain instances of any of Formulas **(I)-(XVIII), (XXV) - (XXVII), (XXX), (XXXI), (XXXIX),** and **(XL-XLVII), R⁴** is isoxazol-5-yl-methyl. In certain instances of any of Formulas **(I)-(XVIII), (XXV)** - **(XXVII), (XXX), (XXXI), (XXXIX),** and **(XL-XLVII), R⁴** is (1-methyl-1H-imidazol-5-yl)-methyl. In certain instances of any of **Formulas(XXXIX),** and **(XL-XLVII), (I)-(XVIII), (XXV)** - **(XXVII), (XXX), (XXXI), (XXXIX),** and **(XL-XLVII), R⁴** is 1-methoxy-cycloprop-lyl-methyl. In certain instances of any of Formulas **(I)-(XVIII), (XXV)** - **(XXVII), (XXX), (XXXI), (XXXIX),** and **(XL-XLVII), R⁴** is methoxyethan-2-yl. In certain instances of any of Formulas **(I)-(XVIII), (XXV) - (XXVII), (XXX), (XXXI), (XXXIX),** and **(XL-XLVII), R⁴** is 1-(2-methoxyethyl)-cycloprop-1-yl-methyl. In certain instances of any of Formulas **(I)-(XVIII), (XXV) - (XXVII), (XXX), (XXXI), (XXXIX),** and **(XL-XLVII), R⁴** is {[1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2R)-1-(1,2-oxazole-3-carbonyl)azetidin-2-yl]methyl}. In certain instances of any of Formulas **(I)-(XVIII), (XXV)** - **(XXVII), (XXX), (XXXI), (XXXIX),** and **(XL-XLVII), R⁴** is {[(2S)-1-(1,2-oxazole-3-carbonyl)azetidin-2-yl]methyl. In certain instances of any of Formulas **(I)-(XVIII), (XXV)** - **(XXVII), (XXX), (XXXI), (XXXIX),** and **(XL-XLVII), R⁴** is 2-oxabicyclo[2.1.1]hexa-1-yl-methyl. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -NH-. In certain instances, provided is a compound of any of Formulas **(I)** - **(XVIII), (XXV)** - **(XXVII), (XXX), (XXXI), (XXXIX),** and **(XL-XLVII),** or a pharmaceutically salt, tautomer, stereoisomer, and/or mixture of stereoisomers thereof, wherein **L²** is -N(Me)-.

In certain instances, provided is a compound of Table 1 below, or a pharmaceutically salt, tautomer, stereoisomer, and/or mixture of stereoisomers thereof. The compound recited in appended claim 1 is Compound 485 or a pharmaceutically acceptable salt or solvate thereof.

| **Compound** | **Structure** | **IUPAC** |
|---|---|---|
| 1 | 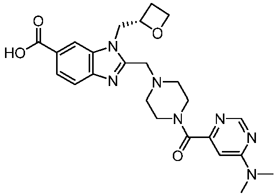 | 2-({4-[6-(dimethylamino)pyrimidine-4-carbonyl]piperazin-1-yl}methyl)-1-{[(2S)-oxetan-2-yl]methyl}-1H-1,3-benzodiazole-6-carboxylic acid |
| 2 | 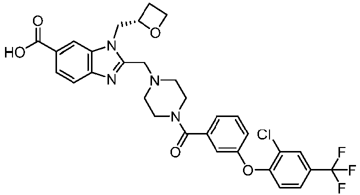 | 2-[(4-{3-[2-chloro-4-(trifluoromethyl)phenoxy]benzoyl}piperazin -1-yl)methyl]-1-{[(2S)-oxetan-2-yl]methyl}-1H-1,3-benzodiazole-6-carboxylic acid |
| 3 | 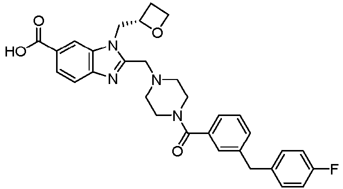 | 2-[(4-{3-[(4-fluorophenyl)methyl]benzoyl}piperazin-1-yl)methyl]-1-{[(2S)-oxetan-2-yl]methyl}-1H-1,3-benzodiazole-6-carboxylic acid |
| 4 | 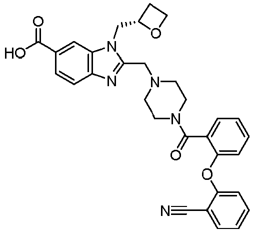 | 2-({4-[2-(2-cyanophenoxy)benzoyl]piperazin-1-yl}methyl)-1-{[(2S)-oxetan-2-yl]methyl}-1H-1,3-benzodiazole-6-carboxylic acid |
| 5 | 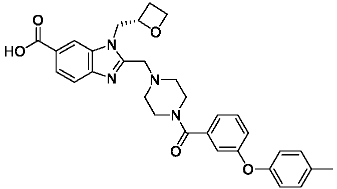 | 2-({4-[3-(4-methylphenoxy)benzoyl]piperazin-1-yl}methyl)-1-{[(2S)-oxetan-2-yl]methyl}-1H-1,3-benzodiazole-6-carboxylic acid |
| 6 | 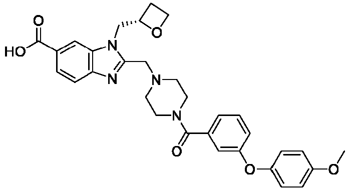 | 2-({4-[3-(4-methoxyphenoxy)benzoyl]piperazin-1-yl}methyl)-1-{[(2S)-oxetan-2-yl]methyl}-1H-1,3-benzodiazole-6-carboxylic acid |
| 7 | 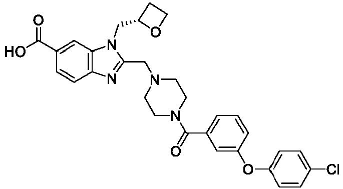 | 2-({4-[3-(4-chlorophenoxy)benzoyl]piperazin-1-yl}methyl)-1-{[(2S)-oxetan-2-yl]methyl}-1H-1,3-benzodiazole-6-carboxylic acid |
| 8 | 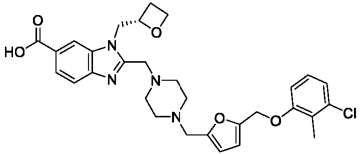 | 2-{[4-({5-[(3-chloro-2-methylphenoxy)methyl]furan-2-yl}methyl)piperazin-1-yl]methyl}-1-{[(2S)-oxetan-2-yl]methyl}-1H-1,3-benzodiazole-6-carboxylic acid |
| 9 | 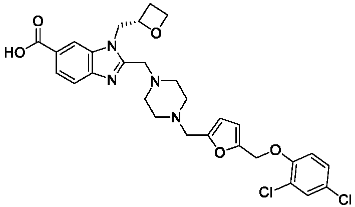 | 2-{[4-({5-[(2,4-dichlorophenoxy)methyl]furan-2-yl}methyl)piperazin-1-yl]methyl}-1-{[(2S)-oxetan-2-yl]methyl}-1H-1,3-benzodiazole-6-carboxylic acid |
| 10 | 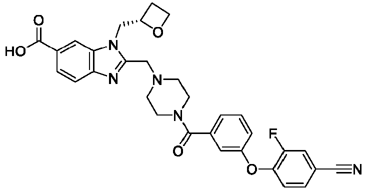 | 2-({4-[3-(4-cyano-2-fluorophenoxy)benzoyl]piperazin-1-yl}methyl)-1-{[(2S)-oxetan-2-yl]methyl}-1H-1,3-benzodiazole-6-carboxylic acid |
| 11 | 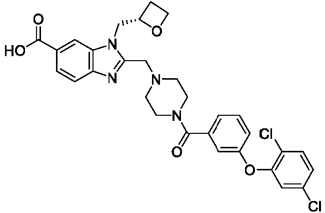 | 2-({4-[3-(2,5-dichlorophenoxy)benzoyl]piperazin-1-yl}methyl)-1-{[(2S)-oxetan-2-yl]methyl}-1H-1,3-benzodiazole-6-carboxylic acid |
| 12 | 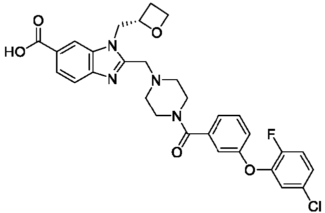 | 2-({4-[3-(5-chloro-2-fluorophenoxy)benzoyl]piperazin-1-yl}methyl)-1-{[(2S)-oxetan-2-yl]methyl}-1H-1,3-benzodiazole-6-carboxylic acid |
| 13 | 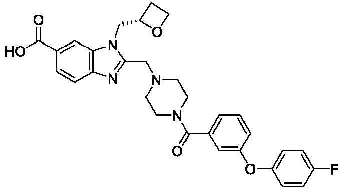 | 2-({4-[3-(4-fluorophenoxy)benzoyl]piperazin-1-yl}methyl)-1-{[(2S)-oxetan-2-yl]methyl}-1H-1,3-benzodiazole-6-carboxylic acid |
| 14 | 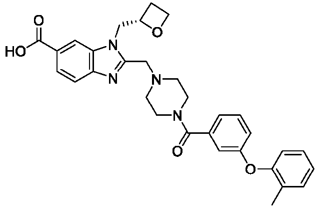 | 2-({4-[3-(2-methylphenoxy)benzoyl]piperazin-1-yl}methyl)-1-{[(2S)-oxetan-2-yl]methyl}-1H-1,3-benzodiazole-6-carboxylic acid |
| 15 | 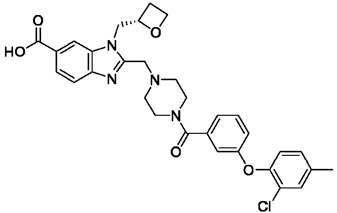 | 2-({4-[3-(2-chloro-4-methylphenoxy)benzoyl]piperazin-1-yl}methyl)-1-{[(2S)-oxetan-2-yl]methyl}-1H-1,3-benzodiazole-6-carboxylic acid |
| 16 | 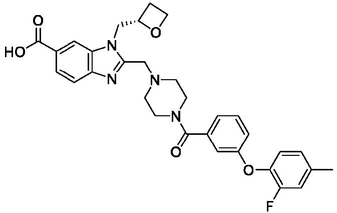 | 2-({4-[3-(2-fluoro-4-methylphenoxy)benzoyl]piperazin-1-yl}methyl)-1-{[(2S)-oxetan-2-yl]methyl}-1H-1,3-benzodiazole-6-carboxylic acid |
| 17 | 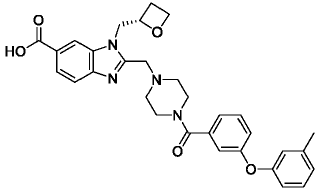 | 2-({4-[3-(3-methylphenoxy)benzoyl]piperazin-1-yl}methyl)-1-{[(2S)-oxetan-2-yl]methyl}-1H-1,3-benzodiazole-6-carboxylic acid |
| 18 | 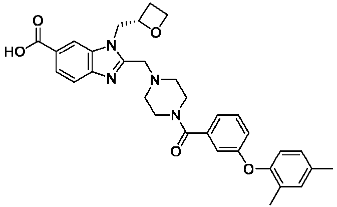 | 2-({4-[3-(2,4-dimethylphenoxy)benzoyl]piperazin-1-yl}methyl)-1-{[(2S)-oxetan-2-yl]methyl}-1H-1,3-benzodiazole-6-carboxylic acid |
| 19 | 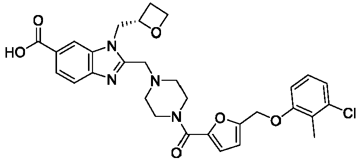 | 2-[(4-{5-[(3-chloro-2-methylphenoxy)methyl]furan-2-carbonyl} piperazin-1-yl)ymethyl]-1-{[(2S)-oxetan-2-yl]methyl}-1H-1,3-benzodiazole-6-carboxylic acid |
| 20 | 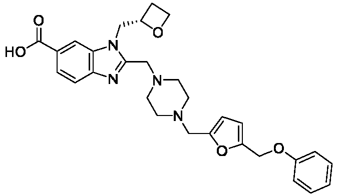 | 1-{[(2S)-oxetan-2-yl]methyl}-2-[(4-{[5-(phenoxymethyl)furan-2-yl]methyl}piperazin-1-yl)methyl]-1H-1,3-benzodiazole-6-carboxylic acid |
| 21 | 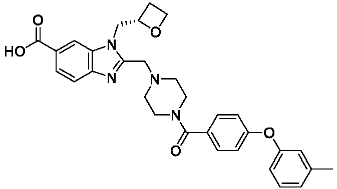 | 2-({4-[4-(3-methylphenoxy)benzoyl]piperazin-1-yl}methyl)-1-{[(2S)-oxetan-2-yl]methyl}-1H-1,3-benzodiazole-6-carboxylic acid |
| 22 | 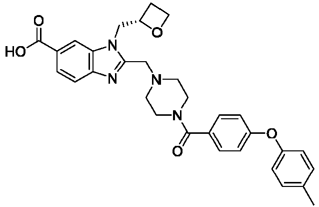 | 2-({4-[4-(4-methylphenoxy)benzoyl]piperazin-1-yl}methyl)-1-{[(2S)-oxetan-2-yl]methyl}-1H-1,3-benzodiazole-6-carboxylic acid |
| 23 | 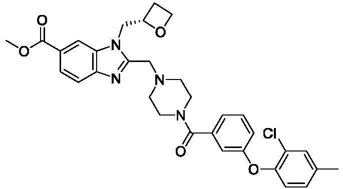 | methyl 2-({4-[3-(2-chloro-4-methylphenoxy)benzoyl]piperazin-1-yl}methyl)-1-{[(2S)-oxetan-2-yl]methyl}-1H-1,3-benzodiazole-6-carboxylate |
| 24 | 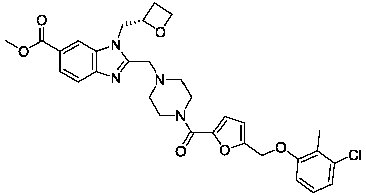 | methyl 2-[(4-{5-[(3-chloro-2-methylphenoxy)methyl]furan-2-carbonyl}piperazin-1-yl)methyl]-1-{[(2S)-oxetan-2-yl]methyl}-1H-1,3-benzodiazole-6-carboxylate |
| 25 | 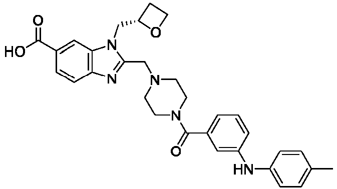 | 2-[(4-{3-[(4-methylphenyl)amino]benzoyl}piperazin-1-yl)methyl]-1-{[(2S)-oxetan-2-yl]methyl}-1H-1,3-benzodiazole-6-carboxylic acid |
| 26 | 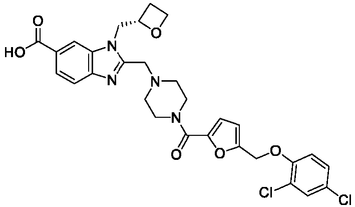 | 2-[(4-{5-[(2,4-dichlorophenoxy)methyl]furan-2-carbonyl}piperazin-1-yl)methyl]-1-{[(2S)-oxetan-2-yl]methyl}-1H-1,3-benzodiazole-6-carboxylic acid |
| 27 | 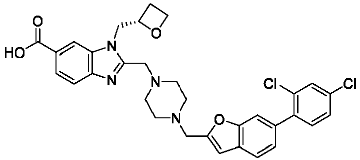 | 2-[(4-{[6-(2,4-dichlorophenyl)-1-benzofuran-2-yl]methyl}piperazin-1-yl)methyl]-1-{[(2S)-oxetan-2-yl]methyl}-1H-1,3-benzodiazole-6-carboxylic acid |
| 28 | 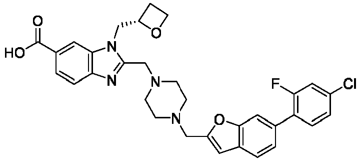 | 2-[(4-{[6-(4-chloro-2-fluorophenyl)-1-benzofuran-2-yl]methyl}piperazin-1-yl)methyl]-1-{[(2S)-oxetan-2-yl]methyl}-1H-1,3-benzodiazole-6-carboxylic acid |
| 29 | 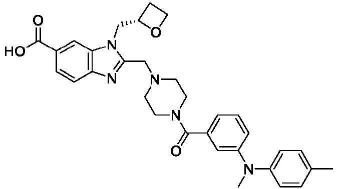 | 2-[(4-{3-[methyl(4-methylphenyl)amino]benzoyl}piperazin-1-yl)methyl]-1-{[(2S)-oxetan-2-yl]methyl}-1H-1,3-benzodiazole-6-carboxylic acid |
| 30 | 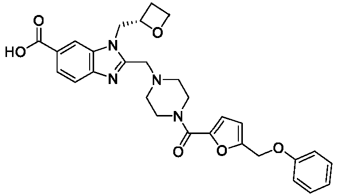 | 1-{[(2S)-oxetan-2-yl]methyl}-2-({4-[5-(phenoxymethyl)furan-2-carbonyl]piperazin-1-yl}methyl)-1H-1,3-benzodiazole-6-carboxylic acid |
| 31 | 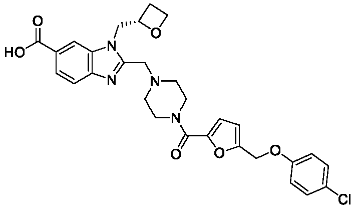 | 2-[(4-{5-[(4-chlorophenoxy)methyl]furan-2-carbonyl}piperazin-1-yl)methyl]-1-{[(2S)-oxetan-2-yl]methyl}-1H-1,3-benzodiazole-6-carboxylic acid |
| 32 | 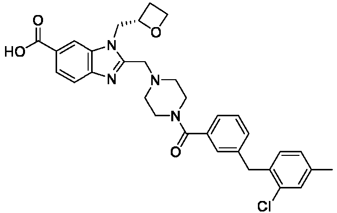 | 2-[(4-{3-[(2-chloro-4-methylphenyl)methyl]benzoyl}piperazin-1-yl)methyl]-1-{[(2S)-oxetan-2-yl]methyl}-1H-1,3-benzodiazole-6-carboxylic acid |
| 33 | 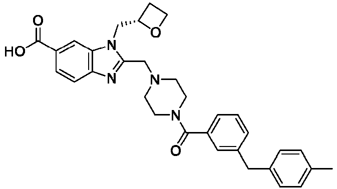 | 2-[(4-{3-[(4-methylphenyl)methyl]benzoyl}piperazin-1-yl)methyl]-1-{[(2S)-oxetan-2-yl]methyl}-1H-1,3-benzodiazole-6-carboxylic acid |
| 34 | 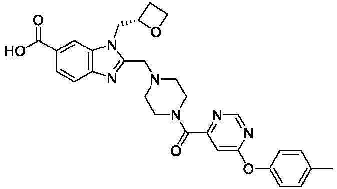 | 2-({4-[6-(4-methylphenoxy)pyrimidine-4-carbonyl]piperazin-1-yl}methyl)-1-{[(2S)-oxetan-2-yl]methyl}-1H-1,3-benzodiazole-6-carboxylic acid |
| 35 | 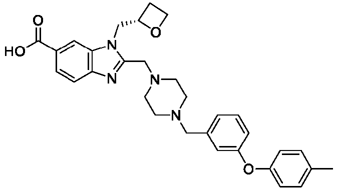 | 2-[(4-{[3-(4-methylphenoxy)phenyl]methyl }piperazin-1-yl)methyl]-1-{[(2S)-oxetan-2-yl]methyl}-1H-1,3-benzodiazole-6-carboxylic acid |
| 36 | 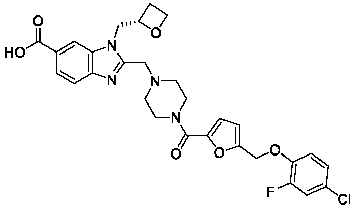 | 2-[(4-{5-[(4-chloro-2-fluorophenoxy)methyl]furan-2-carbonyl}piperazin-1-yl)methyl]-1-{[(2S)-oxetan-2-yl]methyl}-1H-1,3-benzodiazole-6-carboxylic acid |
| 37 | 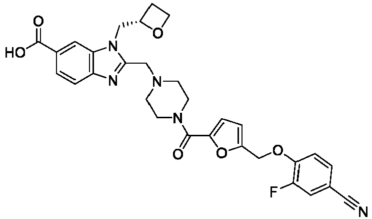 | 2-[(4-{5-[(4-cyano-2-fluorophenoxy)methyl]furan-2-carbonyl}piperazin-1-yl)methyl]-1-{[(2S)-oxetan-2-yl]methyl}-1H-1,3-benzodiazole-6-carboxylic acid |
| 38 | 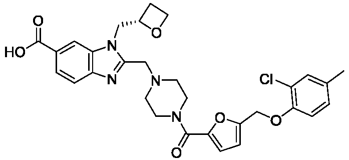 | 2-[(4-{5-[(2-chloro-4-methylphenoxy)methyl]furan-2-carbonyl}piperazin-1-yl)methyl]-1-{[(2S)-oxetan-2-yl]methyl}-1H-1,3-benzodiazole-6-carboxylic acid |
| 39 | 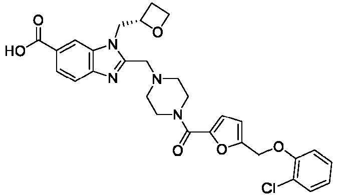 | 2-[(4-{5-[(2-chlorophenoxy)methyl]furan-2-carbonyl}piperazin-1-yl)methyl]-1-{[(2S)-oxetan-2-yl]methyl}-1H-1,3-benzodiazole-6-carboxylic acid |
| 40 | 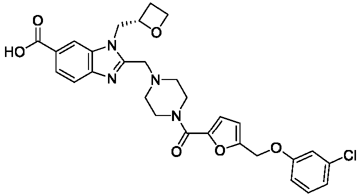 | 2-[(4-{5-[(3-chlorophenoxy)methyl]furan-2-carbonyl}piperazin-1-yl)methyl]-1-{[(2S)-oxetan-2-yl]methyl}-1H-1,3-benzodiazole-6-carboxylic acid |
| 41 | 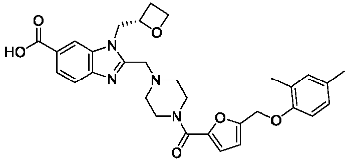 | 2-[(4-{5-[(2,4-dimethylphenoxy)methyl]furan-2-carbonyl}piperazin-1-yl)methyl]-1-{[(2S)-oxetan-2-yl]methyl}-1H-1,3-benzodiazole-6-carboxylic acid |
| 42 | 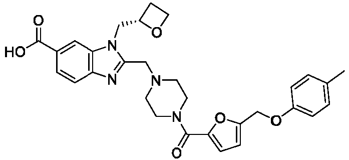 | 2-[(4-{5-[(4-methylphenoxy)methyl]furan-2-carbonyl}piperazin-1-yl)methyl]-1-{[(2S)-oxetan-2-yl]methyl}-1H-1,3-benzodiazole-6-carboxylic acid |
| 43 | 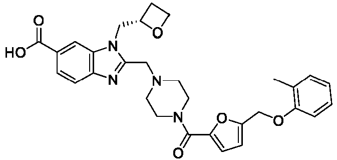 | 2-[(4-{5-[(2-methylphenoxy)methyl]furan-2-carbonyl}piperazin-1-yl)methyl]-1-{[(2S)-oxetan-2-yl]methyl}-1H-1,3-benzodiazole-6-carboxylic acid |
| 44 | 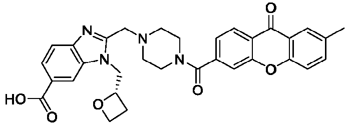 | 2-{[4-(7-methyl-9-oxo-9H-xanthene-3-carbonyl)piperazin-1-yl]methyl}-1-{[(2S)-oxetan-2-yl]methyl}-1H-1,3-benzodiazole-6-carboxylic acid |
| 45 | 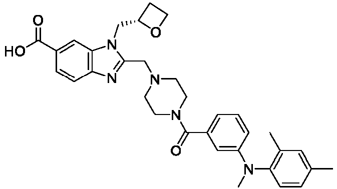 | 2-[(4-{3-[(2,4-dimethylphenyl)(methyl)amino]benzoyl}pi perazin-1-yl)methyl]-1-{[(2S)-oxetan-2-yl]methyl}-1H-1,3-benzodiazole-6-carboxylic acid |
| 46 | 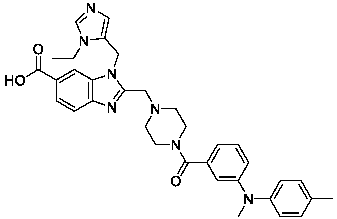 | 1-[(1-ethyl-1H-imidazol-5-yl)methyl]-2-[(4-{3-[methyl(4-methylphenyl)amino]benzoyl}piperazin-1-yl)methyl]-1H-1,3-benzodiazole-6-carboxylic acid |
| 47 | 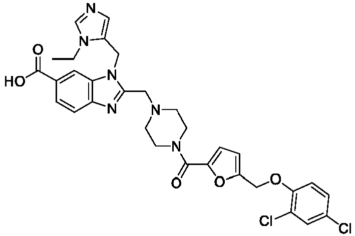 | 2-[(4-{5-[(2,4-dichlorophenoxy)methyl]furan-2-carbonyl}piperazin-1-yl)methyl]-1-[(1-ethyl-1H-imidazol-5-yl)methyl]-1H-1,3-benzodiazole-6-carboxylic acid |
| 48 | 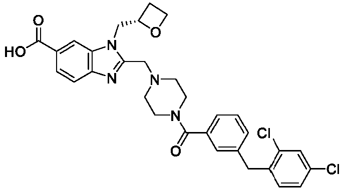 | 2-[(4-{3-[(2,4-dichlorophenyl)methyl]benzoyl}piperazin-1-yl)methyl]-1-{[(2S)-oxetan-2-yl]methyl}-1H-1,3-benzodiazole-6-carboxylic acid |
| 49 | 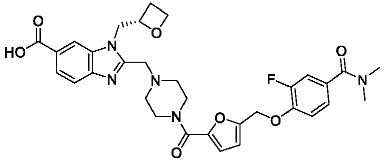 | 2-{[4-(5-{[4-(dimethylcarbamoyl)-2-fluorophenoxy]methyl}furan-2-carbonyl)piperazin-1-yl]methyl}-1-{[(2S)-oxetan-2-yl]methyl}-1H-1,3-benzodiazole-6-carboxylic acid |
| 50 | 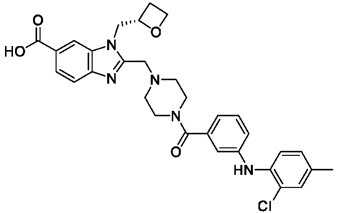 | 2-[(4-{3-[(2-chloro-4-methylphenyl)amino]benzoyl}piperazin-1-yl)methyl]-1-{[(2S)-oxetan-2-yl]methyl}-1H-1,3-benzodiazole-6-carboxylic acid |
| 51 | 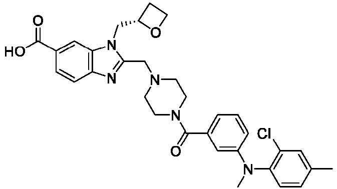 | 2-[(4-{3-[(2-chloro-4-methylphenyl)(methyl)amino]benzoyl}pipe razin-1-yl)methyl]-1-{[(2S)-oxetan-2-yl]methyl}-1H-1,3-benzodiazole-6-carboxylic acid |
| 52 | 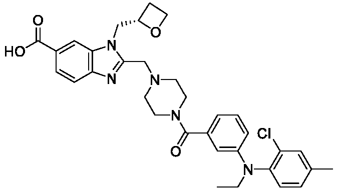 | 2-[(4-{3-[(2-chloro-4-methylphenyl)(ethyl)amino]benzoyl}pipera zin-1-yl)methyl]-1-{[(2S)-oxetan-2-yl]methyl}-1H-1,3-benzodiazole-6-carboxylic acid |
| 53 | 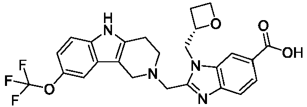 | 1-{[(2S)-oxetan-2-yl]methyl}-2-{[8-(trifluoromethoxy)-1H,2H,3H,4H,5H-pyrido[4,3-b]indol-2-yl]methyl}-1H-1,3-benzodiazole-6-carboxylic acid |
| 54 | 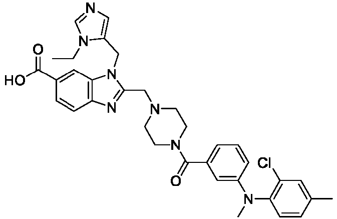 | 2-[(4-{3-[(2-chloro-4-methylphenyl)(methyl)amino]benzoyl}pipe razin-1-yl)methyl]-1-[(1-ethyl-1H-imidazol-5-yl)methyl]-1H-1,3-benzodiazole-6-carboxylic acid |
| 55 | 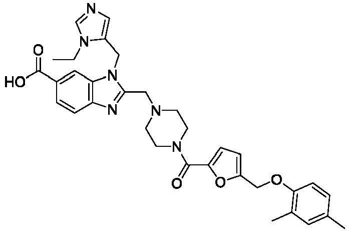 | 2-[(4-{5-[(2,4-dimethylphenoxy)methyl]furan-2-carbonyl}piperazin-1-yl)methyl]-1-[(1-ethyl-1H-imidazol-5-yl)methyl]-1H-1,3-benzodiazole-6-carboxylic acid |
| 56 | 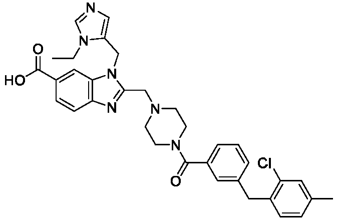 | 2-[(4-{3-[(2-chloro-4-methylphenyl)methyl]benzoyl}piperazin-1-yl)methyl]-1-[(1-ethyl-1H-imidazol-5-yl)methyl]-1H-1,3 -benzodiazole-6-carboxylic acid |
| 57 | 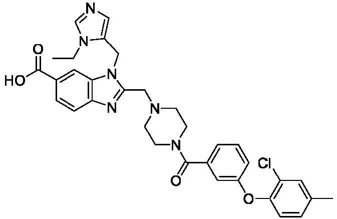 | 2-({4-[3-(2-chloro-4-methylphenoxy)benzoyl]piperazin-1-yl} methyl)-1-[(1-ethyl-1H-imidazol-5-yl)methyl]-1H-1,3 -benzodiazole-6-carboxylic acid |
| 58 | 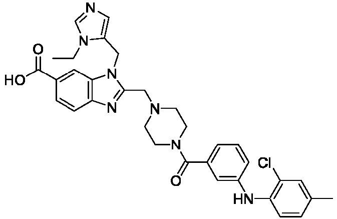 | 2-[(4-{3-[(2-chloro-4-methylphenyl)amino]benzoyl}piperazin-1-yl)methyl]-1-[(1-ethyl-1H-imidazol-5-yl)methyl]-1H-1,3-benzodiazole-6-carboxylic acid |
| 59 | 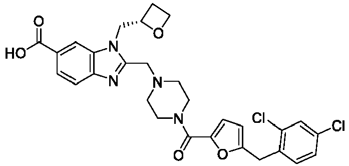 | 2-[(4-{5-[(2,4-dichlorophenyl)methyl]furan-2-carbonyl}piperazin-1-yl)methyl]-1-{[(2S)-oxetan-2-yl]methyl}-1H-1,3-benzodiazole-6-carboxylic acid |
| 60 | 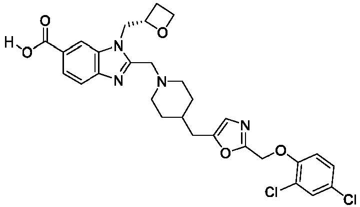 | 2-{[4-({2-[(2,4-dichlorophenoxy)methyl]-1,3-oxazol-5-yl}methyl)piperidin-1-yl]methyl}-1-{[(2S)-oxetan-2-yl]methyl}-1H-1,3-benzodiazole-6-carboxylic acid |
| 61 | 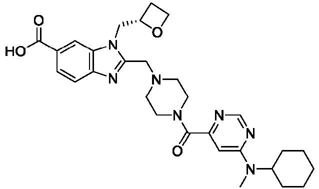 | 2-[(4-{6-[cyclohexyl(methyl)amino]pyrimidine-4-carbonyl}piperazin-1-yl)methyl]-1-{[(2S)-oxetan-2-yl]methyl}-1H-1,3-benzodiazole-6-carboxylic acid |
| 62 | 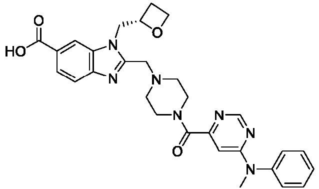 | 2-[(4-{6-[methyl(phenyl)amino]pyrimidine-4-carbonyl}piperazin-1-yl)methyl]-1-{[(2S)-oxetan-2-yl]methyl}-1H-1,3-benzodiazole-6-carboxylic acid |
| 63 | 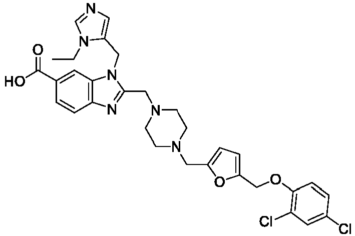 | 2-{[4-({5-[(2,4-dichlorophenoxy)methyl]furan-2-yl}methyl)piperazin-1-yl]methyl}-1-[(1-ethyl-1H-imidazol-5-yl)methyl]-1H-1,3-benzodiazole-6-carboxylic acid |
| 64 | 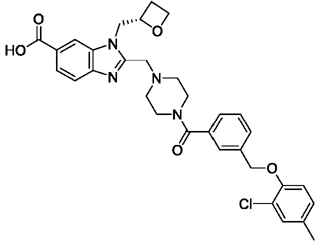 | 2-[(4-{3-[(2-chloro-4-methylphenoxy)methyl]benzoyl}piperazin-1-yl)methyl]-1-{[(2S)-oxetan-2-yl]methyl}-1H-1,3-benzodiazole-6-carboxylic acid |
| 65 | 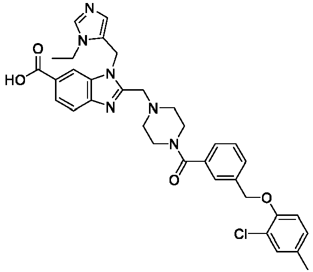 | 2-[(4-{3-[(2-chloro-4-methylphenoxy)methyl]benzoyl}piperazin-1-yl)methyl]-1-[(1-ethyl-1H-imidazol-5-yl)methyl]-1H-1,3 -benzodiazole-6-carboxylic acid |
| 66 | 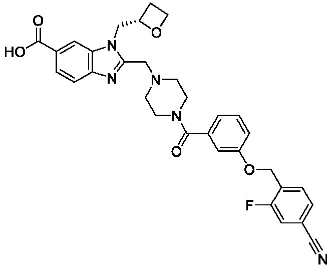 | 2-[(4-{3-[(4-cyano-2-fluorophenyl)methoxy]benzoyl}piperazin-1-yl)methyl]-1-{[(2S)-oxetan-2-yl]methyl}-1H-1,3-benzodiazole-6-carboxylic acid |
| 67 | 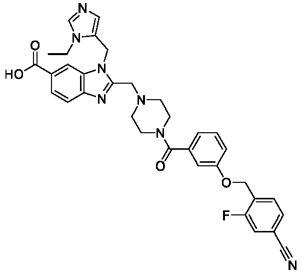 | 2-[(4-{3-[(4-cyano-2-fluorophenyl)methoxy]benzoyl}piperazin-1-yl)methyl]-1-[(1-ethyl-1H-imidazol-5-yl)methyl]-1H-1,3 -benzodiazole-6-carboxylic acid |
| 68 | 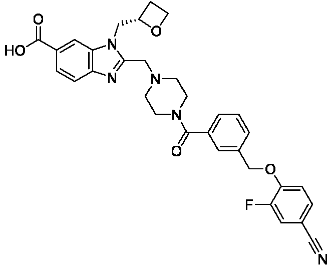 | 2-[(4-{3-[(4-cyano-2-fluorophenoxy)methyl]benzoyl}piperazin-1-yl)methyl]-1-{[(2S)-oxetan-2-yl]methyl}-1H-1,3-benzodiazole-6-carboxylic acid |
| 69 | 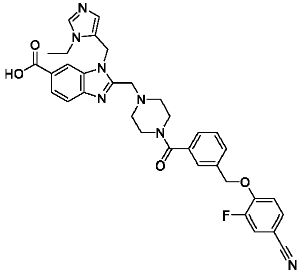 | 2-[(4-{3-[(4-cyano-2-fluorophenoxy)methyl]benzoyl}piperazin-1-yl)methyl]-1-[(1-ethyl-1H-imidazol-5-yl)methyl]-1H-1,3-benzodiazole-6-carboxylic acid |
| 70 | 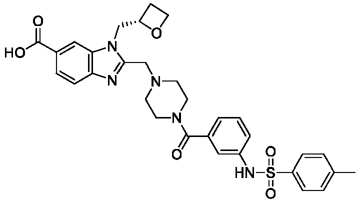 | 2-({4-[3-(4-methylbenzenesulfonamido)benzoyl]pipera zin-1-yl}methyl)-1-{[(2S)-oxetan-2-yl]methyl}-1H-1,3-benzodiazole-6-carboxylic acid |
| 71 | 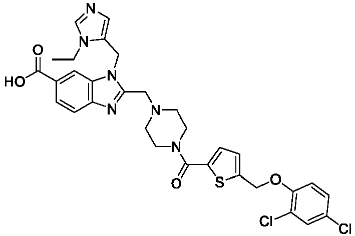 | 2-[(4-{5-[(2,4-dichlorophenoxy)methyl]thiophene-2-carbonyl}piperazin-1-yl)methyl]-1-[(1-ethyl-1H-imidazol-5-yl)methyl]-1H-1,3-benzodiazole-6-carboxylic acid |
| 72 | 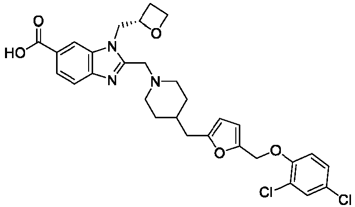 | 2-{[4-({5-[(2,4-dichlorophenoxy)methyl]furan-2-yl}methyl)piperidin-1-yl]methyl}-1-{[(2S)-oxetan-2-yl]methyl}-1H-1,3-benzodiazole-6-carboxylic acid |
| 73 | 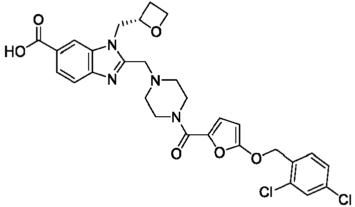 | 2-[(4-{5-[(2,4-dichlorophenyl)methoxy]furan-2-carbonyl}piperazin-1-yl)methyl]-1-{[(2S)-oxetan-2-yl]methyl}-1H-1,3-benzodiazole-6-carboxylic acid |
| 74 | 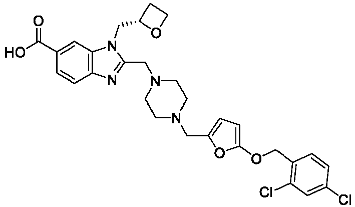 | 2-{[4-({5-[(2,4-dichlorophenyl)methoxy]furan-2-yl}methyl)piperazin-1-yl]methyl}-1-{[(2S)-oxetan-2-yl]methyl}-1H-1,3-benzodiazole-6-carboxylic acid |
| 75 | 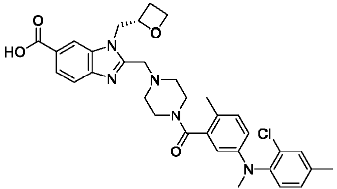 | 2-[(4-{5-[(2-chloro-4-methylphenyl)(methyl)amino]-2-methylbenzoyl}piperazin-1-yl)methyl]-1-{[(2S)-oxetan-2-yl]methyl}-1H-1,3-benzodiazole-6-carboxylic acid |
| 76 | 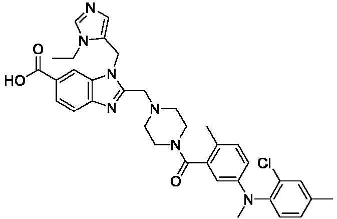 | 2-[(4-{5-[(2-chloro-4-methylphenyl)(methyl)amino]-2-methylbenzoyl}piperazin-1-yl)methyl]-1-[(1-ethyl-1H-imidazol-5-yl)methyl]-1H-1,3-benzodiazole-6-carboxylic acid |
| 77 | 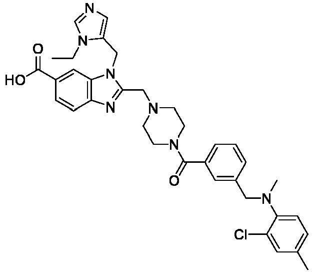 | 2-{[4-(3-{[(2-chloro-4-methylphenyl)(methyl)amino]methyl}benzo yl)piperazin-1-yl]methyl}-1-[(1-ethyl-1H-imidazol-5-yl)methyl]-1H-1,3-benzodiazole-6-carboxylic acid |
| 78 | 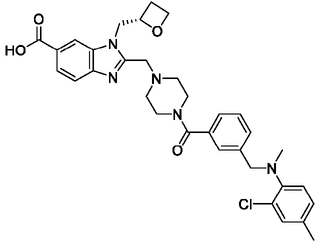 | 2-{[4-(3-{[(2-chloro-4-methylphenyl)(methyl)amino]methyl}benzo yl)piperazin-1-yl]methyl}-1-{ [(2S)-oxetan-2-yl]methyl}-1H-1,3-benzodiazole-6-carboxylic acid |
| 79 | 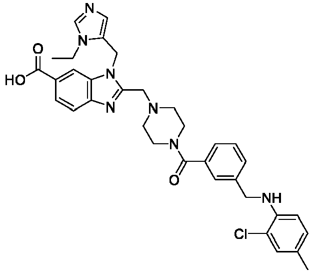 | 2-{[4-(3-{[(2-chloro-4-methylphenyl)amino]methyl}benzoyl)piper azin-1-yl]methyl}-1-[(1-ethyl-1H-imidazol-5-yl)methyl]-1H-1,3-benzodiazole-6-carboxylic acid |
| 80 | 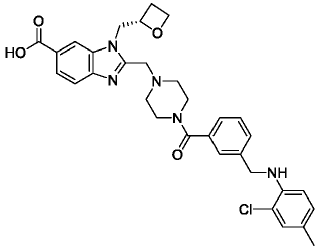 | 2-{[4-(3-{[(2-chloro-4-methylphenyl)amino]methyl}benzoyl)piper azin-1-yl]methyl}-1-{[(2S)-oxetan-2-yl]methyl}-1H-1,3-benzodiazole-6-carboxylic acid |
| 81 | 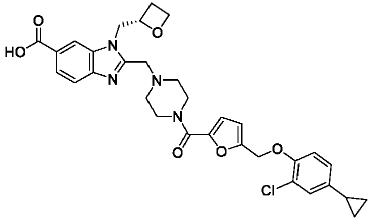 | 2-[(4-{5-[(2-chloro-4-cyclopropylphenoxy)methyl]furan-2-carbonyl}piperazin-1-yl)methyl]-1-{[(2S)-oxetan-2-yl]methyl}-1H-1,3-benzodiazole-6-carboxylic acid |
| 82 | 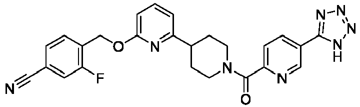 | 3-fluoro-4-{[(6-{1-[5-(1H-1,2,3,4-tetrazol-5-yl)pyridine-2-carbonyl]piperidin-4-yl}pyridin-2-yl)oxy]methyl} benzonitrile |
| 83 | 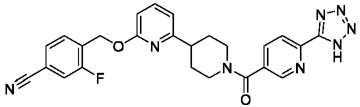 | 3-fluoro-4-{[(6-{1-[6-(1H-1,2,3,4-tetrazol-5-yl)pyridine-3-carbonyl]piperidin-4-yl}pyridin-2-yl)oxy]methyl}benzonitrile |
| 84 | 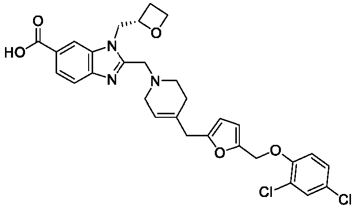 | 2-{[4-({5-[(2,4-dichlorophenoxy)methyl]furan-2-yl}methyl)-1,2,3,6-tetrahydropyridin-1-yl]methyl}-1-{[(2S)-oxetan-2-yl]methyl}-1H-1,3-benzodiazole-6-carboxylic acid |
| 85 | 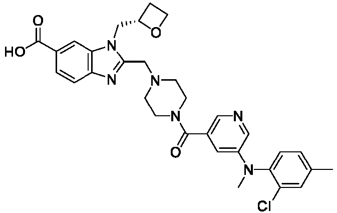 | 2-[(4-{5-[(2-chloro-4-methylphenyl)(methyl)amino]pyridine-3-carbonyl}piperazin-1-yl)methyl]-1-{[(2S)-oxetan-2-yl]methyl}-1H-1,3-benzodiazole-6-carboxylic acid |
| 86 | 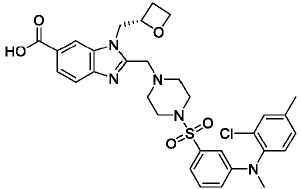 | 2-[(4-{3-[(2-chloro-4-methylphenyl)(methyl)amino]benzenesulfo nyl}piperazin-1-yl)methyl]-1-{[(2S)-oxetan-2-yl]methyl}-1H-1,3-benzodiazole-6-carboxylic acid |
| 87 | 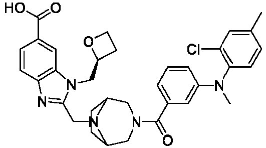 | 2-[(3-{3-[(2-chloro-4-methylphenyl)(methyl)amino]benzoyl}-3,8-diazabicyclo[3.2.1]octan-8-yl)methyl]-1-{[(2S)-oxetan-2-yl]methyl}-1H-1,3-benzodiazole-6-carboxylic acid |
| 88 | 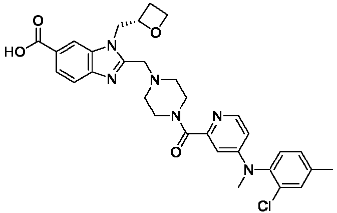 | 2-[(4-{4-[(2-chloro-4-methylphenyl)(methyl)amino]pyridine-2-carbonyl}piperazin-1-yl)methyl]-1-{[(2S)-oxetan-2-yl]methyl}-1H-1,3-benzodiazole-6-carboxylic acid |
| 89 | 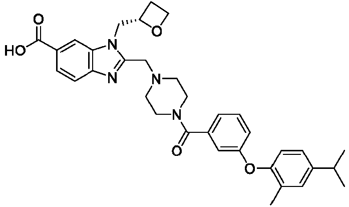 | 2-[(4-{3-[2-methyl-4-(propan-2-yl)phenoxy]benzoyl}piperazin-1-yl)methyl]-1-{[(2S)-oxetan-2-yl]methyl}-1H-1,3-benzodiazole-6-carboxylic acid |
| 90 | 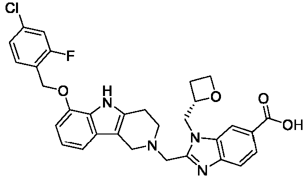 | 2-({6-[(4-chloro-2-fluorophenyl)methoxy]-1H,2H,3H,4H, 5H-pyrido[4,3-b]indol-2-yl}methyl)-1-{[(2S)-oxetan-2-yl]methyl}-1H-1,3-benzodiazole-6-carboxylic acid |
| 91 | 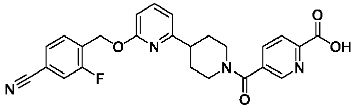 | 5-(4-{6-[(4-cyano-2-fluorophenyl)methoxy]pyridin-2-yl}piperidine-1-carbonyl)pyridine-2-carboxylic acid |
| 92 | 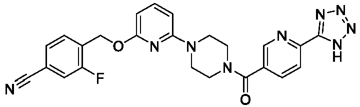 | 3-fluoro-4-{[(6-{4-[6-(1H-1,2,3,4-tetrazol-5-yl)pyridine-3-carbonyl]piperazin-1-yl}pyridin-2-yl)oxy]methyl}benzonitrile |
| 93 | 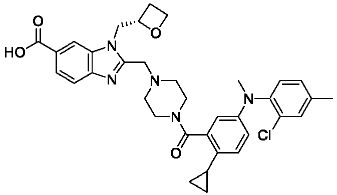 | 2-[(4-{5-[(2-chloro-4-methylphenyl)(methyl)amino]-2-cyclopropylbenzoyl}piperazin-1-yl)methyl]-1-{[(2S)-oxetan-2-yl]methyl}-1H-1,3-benzodiazole-6-carboxylic acid |
| 94 | 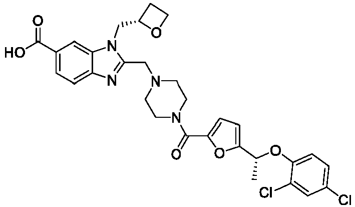 | 2-[(4-{5-[(1R*)-1-(2,4-dichlorophenoxy)ethyl]furan-2-carbonyl}piperazin-1-yl)methyl]-1-{[(2S)-oxetan-2-yl]methyl}-1H-1,3-benzodiazole-6-carboxylic acid |
| 95 | 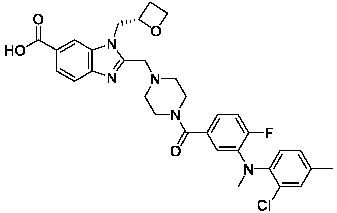 | 2-[(4-{3-[(2-chloro-4-methylphenyl)(methyl)amino]-4-fluorobenzoyl}piperazin-1-yl)methyl]-1-{[(2S)-oxetan-2-yl]methyl}-1H-1,3-benzodiazole-6-carboxylic acid |
| 96 | 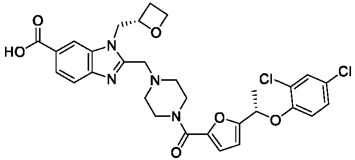 | 2-[(4-{5-[(1S)-1-(2,4-dichlorophenoxy)ethyl]furan-2-carbonyl}piperazin-1-yl)methyl]-1-{[(2S)-oxetan-2-yl]methyl}-1H-1,3-benzodiazole-6-carboxylic acid |
| 97 | 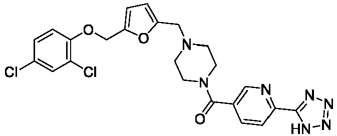 | 1-({5-[(2,4-dichlorophenoxy)methyl]furan-2-yl}methyl)-4-[6-(1H-1,2,3,4-tetrazol-5-yl)pyridine-3-carbonyl]piperazine |
| 98 | 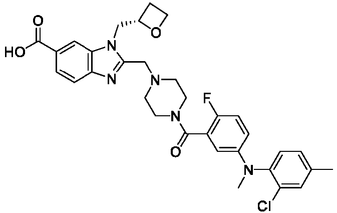 | 2-[(4-{5-[(2-chloro-4-methylphenyl)(methyl)amino]-2-fluorobenzoyl}piperazin-1-yl)methyl]-1-{[(2S)-oxetan-2-yl]methyl}-1H-1,3-benzodiazole-6-carboxylic acid |
| 99 | 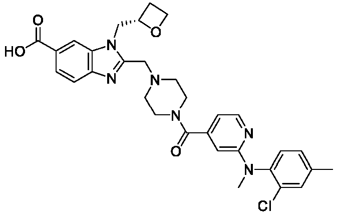 | 2-[(4-{2-[(2-chloro-4-methylphenyl)(methyl)amino]pyridine-4-carbonyl}piperazin-1-yl)methyl]-1-{[(2S)-oxetan-2-yl]methyl}-1H-1,3-benzodiazole-6-carboxylic acid |
| 100 | 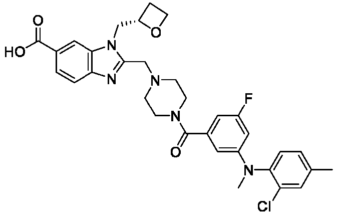 | 2-[(4-{3-[(2-chloro-4-methylphenyl)(methyl)amino]-5-fluorobenzoyl}piperazin-1-yl)methyl]-1-{[(2S)-oxetan-2-yl]methyl}-1H-1,3-benzodiazole-6-carboxylic acid |
| 101 | 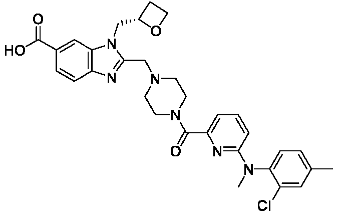 | 2-[(4-{6-[(2-chloro-4-methylphenyl)(methyl)amino]pyridine-2-carbonyl}piperazin-1-yl)methyl]-1-{[(2S)-oxetan-2-yl]methyl}-1H-1,3-benzodiazole-6-carboxylic acid |
| 102 | 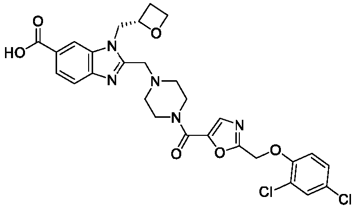 | 2-[(4-{2-[(2,4-dichlorophenoxy)methyl]-1,3-oxazole-5-carbonyl}piperazin-1-yl)methyl]-1-{[(2S)-oxetan-2-yl]methyl}-1H-1,3-benzodiazole-6-carboxylic acid |
| 103 | 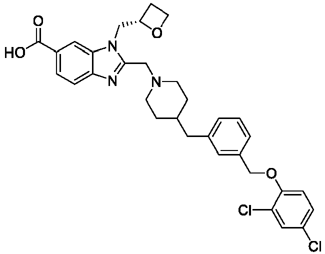 | 2-{[4-({3-[(2,4-dichlorophenoxy)methyl]phenyl} methyl)pi peridin-1-yl]methyl}-1-{[(2S)-oxetan-2-yl]methyl}-1H-1,3-benzodiazole-6-carboxylic acid |
| 104 | 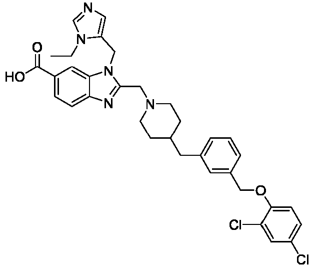 | 2-{[4-({3-[(2,4-dichlorophenoxy)methyl]phenyl}methyl)pi peridin-1-yl]methyl}-1-[(1-ethyl-1H-imidazol-5-yl)methyl]-1H-1,3-benzodiazole-6-carboxylic acid |
| 105 | 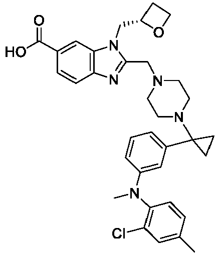 | 2-{[4-(1-{3-[(2-chloro-4-methylphenyl)(methyl)amino]phenyl}cyclo propyl)piperazin-1-yl]methyl}-1-{[(2S)-oxetan-2-yl]methyl}-1H-1,3-benzodiazole-6-carboxylic acid |
| 106 | 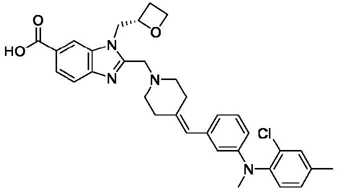 | 2-{[4-({3-[(2-chloro-4-methylphenyl)(methyl)amino]phenyl}meth ylidene)piperidin-1-yl]methyl}-1-{[(2S)-oxetan-2-yl]methyl}-1H-1,3-benzodiazole-6-carboxylic acid |
| 107 | 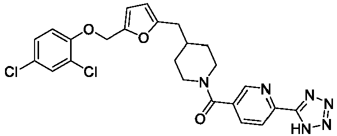 | 5-[4-({5-[(2,4-dichlorophenoxy)methyl]furan-2-yl}methyl)piperidine-1-carbonyl]-2-(1H-1,2,3,4-tetrazol-5-yl)pyridine |
| 108 | 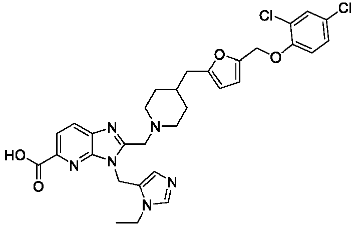 | 2-{[4-({5-[(2,4-dichlorophenoxy)methyl]furan-2-yl}methyl)piperidin-1-yl]methyl}-3-[(1-ethyl-1H-imidazol-5-yl)methyl]-3H-imidazo[4,5-b]pyridine-5-carboxylic acid |
| 109 | 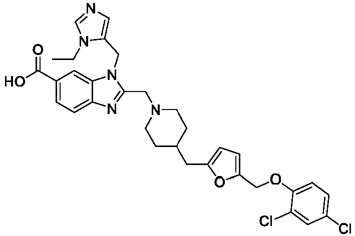 | 2-{[4-({5-[(2,4-dichlorophenoxy)methyl]furan-2-yl}methyl)piperidin-1-yl]methyl}-1-[(1-ethyl-1H-imidazol-5-yl)methyl]-1H-1,3-benzodiazole-6-carboxylic acid |
| 110 | 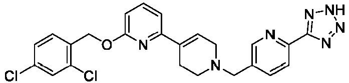 | 6-[(2,4-dichlorophenyl)methoxy]-1'-{[6-(2H-1,2,3,4-tetrazol-5-yl)pyridin-3-yl]methyl}-1',2',3',6'-tetrahydro-2,4'-bipyridine |
| 111 | 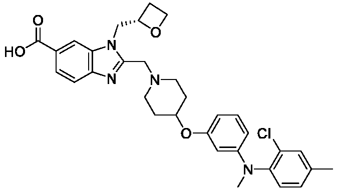 | 2-[(4-{3-[(2-chloro-4-methylphenyl)(methyl)amino]phenoxy}pipe ridin-1-yl)methyl]-1-{[(2S)-oxetan-2-yl]methyl}-1H-1,3-benzodiazole-6-carboxylic acid |
| 112 | 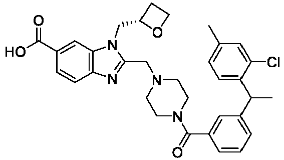 | 2-[(4-{3-[1-(2-chloro-4-methylphenyl)ethyl]benzoyl }piperazin-1-yl)methyl]-1-{[(2S)-oxetan-2-yl]methyl}-1H-1,3-benzodiazole-6-carboxylic acid |
| 113 | 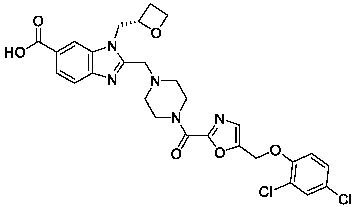 | 2-[(4-{5-[(2,4-dichlorophenoxy)methyl]-1,3-oxazole-2-carbonyl}piperazin-1-yl)methyl]-1-{[(2S)-oxetan-2-yl]methyl}-1H-1,3-benzodiazole-6-carboxylic acid |
| 114 | 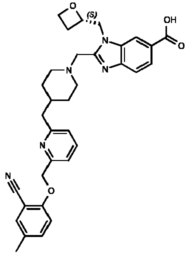 | 2-{[4-({6-[(2-cyano-4-methylphenoxy)methyl]pyridin-2-yl}methyl)piperidin-1-yl]methyl}-1-{[(2S)-oxetan-2-yl]methyl}-1H-1,3-benzodiazole-6-carboxylic acid |
| 115 | 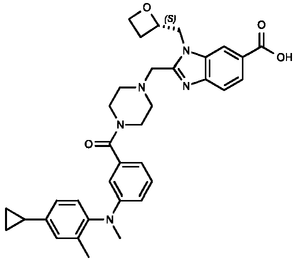 | 2-[(4-{3-[(4-cyclopropyl-2-methylphenyl)(methyl)amino]benzoyl}pipe razin-1-yl)methyl]-1-{[(2S)-oxetan-2-yl]methyl}-1H-1,3-benzodiazole-6-carboxylic acid |
| 116 | 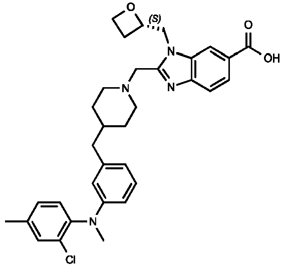 | 2-{[4-({3-[(2-chloro-4-methylphenyl)(methyl)amino]phenyl}meth yl)piperidin-1-yl]methyl}-1-{[(2S)-oxetan-2-yl]methyl}-1H-1,3-benzodiazole-6-carboxylic acid |
| 117 | 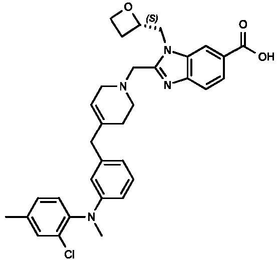 | 2-{[4-({3-[(2-chloro-4-methylphenyl)(methyl)amino]phenyl}meth yl)-1,2,3,6-tetrahydropyridin-1-yl]methyl}-1-{[(2S)-oxetan-2-yl]methyl}-1H-1,3-benzodiazole-6-carboxylic acid |
| 118 | 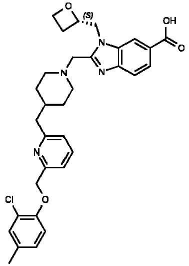 | 2-{[4-({6-[(2-chloro-4-methylphenoxy)methyl]pyridin-2-yl}methyl)piperidin-1-yl]methyl}-1-{[(2S)-oxetan-2-yl]methyl}-1H-1,3-benzodiazole-6-carboxylic acid |
| 120 | 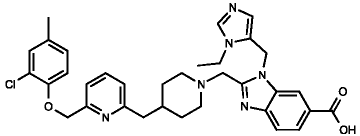 | 2-{[4-({6-[(2-chloro-4-methylphenoxy)methyl]pyridin-2-yl}methyl)piperidin-1-yl]methyl}-1-[(1-ethyl-1H-imidazol-5-yl)methyl]-1H-1,3-benzodiazole-6-carboxylic acid |
| 121 | 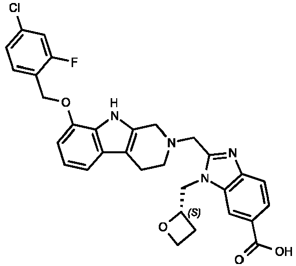 | 2-({8-[(4-chloro-2-fluorophenyl)methoxy]-1H,2H,3H,4H,9H-pyrido[3,4-b]indol-2-yl}methyl)-1-{[(2S)-oxetan-2-yl]methyl}-1H-1,3-benzodiazole-6-carboxylic acid |
| 122 | 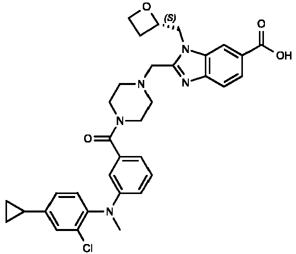 | 2-[(4-{3-[(2-chloro-4-cyclopropylphenyl)(methyl)amino]benzoyl} piperazin-1-yl)methyl]-1-{[(2S)-oxetan-2-yl]methyl}-1H-1,3-benzodiazole-6-carboxylic acid |
| 123 | 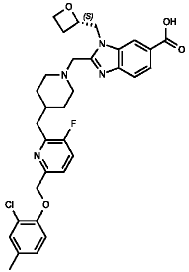 | 2-{[4-({6-[(2-chloro-4-methylphenoxy)methyl]-3-fluoropyridin-2-yl}methyl)piperidin-1-yl]methyl}-1-{[(2S)-oxetan-2-yl]methyl}-1H-1,3-benzodiazole-6-carboxylic acid |
| 124 | 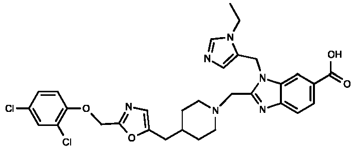 | 2-{[4-({2-[(2,4-dichlorophenoxy)methyl]-1,3-oxazol-5-yl}methyl)piperidin-1-yl]methyl}-1-[(1-ethyl-1H-imidazol-5-yl)methyl]-1H-1,3 -benzodiazole-6-carboxylic acid |
| 125 | 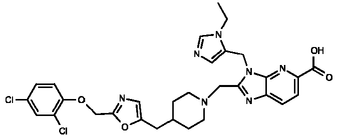 | 2-{[4-({2-[(2,4-dichlorophenoxy)methyl]-1,3-oxazol-5-yl}methyl)piperidin-1-ylmethyl}-3-[(1-ethyl-1H-imidazol-5-yl)methyl]-3H-imidazo[4,5-b]pyridine-5-carboxylic acid |
| 126 | 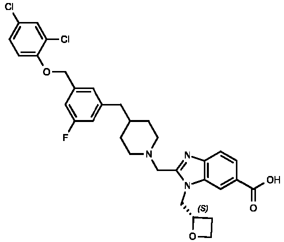 | 2-{[4-({3-[(2,4-dichlorophenoxy)methyl]-5-fluorophenyl}methyl)piperidin-1-yl]methyl }-1-{ [(2S)-oxetan-2-yl]methyl}-1H-1,3-benzodiazole-6-carboxylic acid |
| 128 | 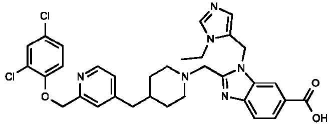 | 2-{[4-({2-[(2,4-dichlorophenoxy)methyl]pyridin-4-yl}methyl)piperidin-1-yl]methyl}-1-[(1-ethyl-1H-imidazol-5-yl)methyl]-1H-1,3-benzodiazole-6-carboxylic acid |
| 129 | 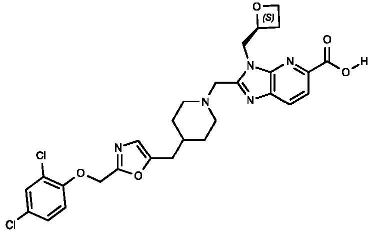 | 2-{[4-({2-[(2,4-dichlorophenoxy)methyl]-1,3-oxazol-5-yl}methyl)piperidin-1-yl]methyl}-3-{[(2S)-oxetan-2-yl]methyl}-3H-imidazo[4,5-b]pyridine-5-carboxylic acid |
| 130 | 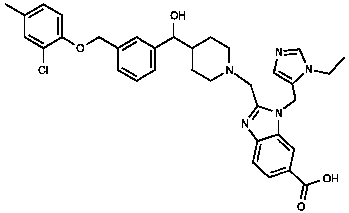 | 2-{[4-({3-[(2-chloro-4-methylphenoxy)methyl]phenyl}(hydroxy)m ethyl)piperidin-1-yl]methyl}-1-[(1-ethyl-1H-imidazol-5-yl)methyl]-1H-1,3-benzodiazole-6-carboxylic acid |
| 131 | 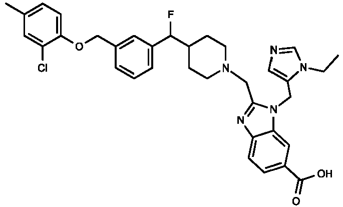 | 2-{[4-({3-[(2-chloro-4-methylphenoxy)methyl]phenyl}(fluoro)met hyl)piperidin-1-yl]methyl}-1-[(1-ethyl-1H-imidazol-5-yl)methyl]-1H-1,3-benzodiazole-6-carboxylic acid |
| 132 | 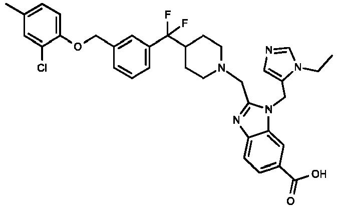 | 2-{[4-({3-[(2-chloro-4-methylphenoxy)methyl]phenyl}difluoromet hyl)piperidin-1-yl]methyl}-1-[(1-ethyl-1H-imidazol-5-yl)methyl]-1H-1,3-benzodiazole-6-carboxylic acid |
| 133 | 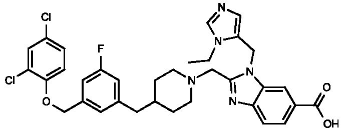 | 2-{[4-({3-[(2,4-dichlorophenoxy)methyl]-5-fluorophenyl}methyl)piperidin-1-yl]methyl}-1-[(1-ethyl-1H-imidazol-5-yl)methyl]-1H-1,3 -benzodiazole-6-carboxylic acid |
| 135 | 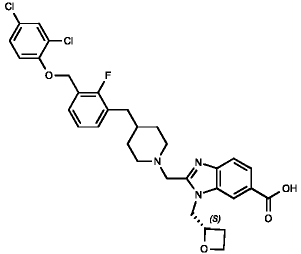 | 2-{[4-({3-[(2,4-dichlorophenoxy)methyl]-2-fluorophenyl}methyl)piperidin-1-yl]methyl}-1-{[(2S)-oxetan-2-yl]methyl}-1H-1,3-benzodiazole-6-carboxylic acid |
| 136 | 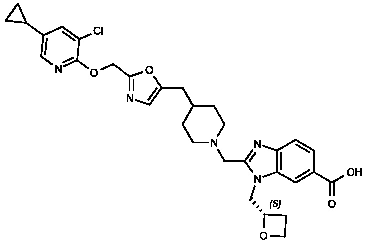 | 2-({4-[(2-{[(3-chloro-5-cyclopropylpyridin-2⁻yl)oxy]methyl}-1,3-oxazol-5-yl)methyl]piperidin-1-yl}methyl)-1-{[(2S)-oxetan-2-yl]methyl}-1H-1,3-benzodiazole-6-carboxylic acid |
| 137 | 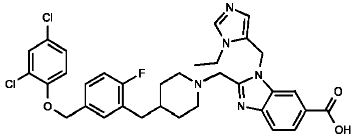 | 2-{[4-({5-[(2,4-dichlorophenoxy)methyl]-2-fluorophenyl}methyl)piperidin-1-yl]methyl}-1-[(1-ethyl-1H-imidazol-5-yl)methyl]-1H-1,3-benzodiazole-6-carboxylic acid |
| 138 | 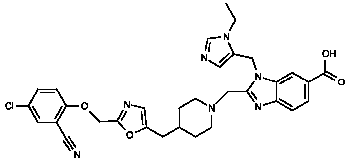 | 2-{[4-({2-[(4-chloro-2-cyanophenoxy)methyl]-1,3-oxazol-5-yl}methyl)piperidin-1-yl]methyl}-1-[(1-ethyl-1H-imidazol-5-yl)methyl]-1H-1,3-benzodiazole-6-carboxylic acid |
| 139 | 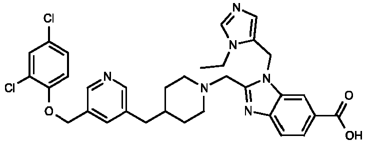 | 2-{[4-({5-[(2,4-dichlorophenoxy)methyl]pyridin-3-yl}methyl)piperidin-1-yl]methyl}-1-[(1-ethyl-1H-imidazol-5-yl)methyl]-1H-1,3-benzodiazole-6-carboxylic acid |
| 140 | 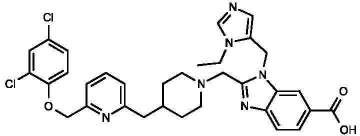 | 2-{[4-({6-[(2,4-dichlorophenoxy)methyl]pyridin-2-yl}methyl)piperidin-1-yl]methyl}-1-[(1-ethyl-1H-imidazol-5-yl)methyl]-1H-1,3-benzodiazole-6-carboxylic acid |
| 141 | 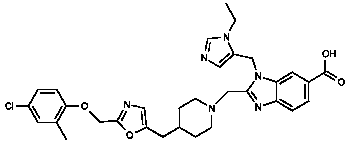 | 2-{[4-({2-[(4-chloro-2-methylphenoxy)methyl]-1,3-oxazol-5-yl}methyl)piperidin-1-yl]methyl}-1-[(1-ethyl-1H-imidazol-5-yl)methyl]-1H-1,3-benzodiazole-6-carboxylic acid |
| 142 | 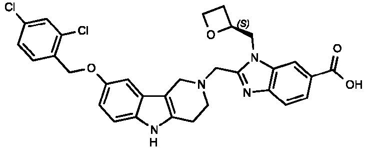 | 2-({8-[(2,4-dichlorophenyl)methoxy]-1H,2H,3H,4H,5H-pyrido[4,3-b]indol-2-yl}methyl)-1-{[(2S)-oxetan-2-yl]methyl}-1H-1,3-benzodiazole-6-carboxylic acid |
| 143 | 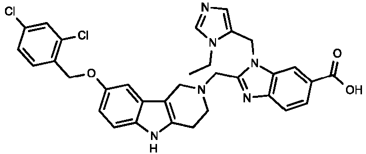 | 2-({8-[(2,4-dichlorophenyl)methoxy]-1H,2H,3H,4H,5H-pyrido[4,3-b]indol-2-yl} methyl)-1-[(1-ethyl-1H-imidazol-5-yl)methyl]-1H-1,3-benzodiazole-6-carboxylic acid |
| 144 | 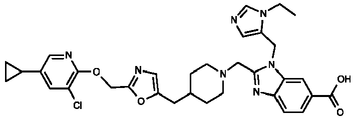 | 2-({4-[(2-{[(3-chloro-5-cyclopropylpyridin-2-yl)oxy]methyl}-1,3-oxazol-5-yl)methyl]piperidin-1-yl}methyl)-1-[(1-ethyl-1H-imidazol-5-yl)methyl]-1H-1,3-benzodiazole-6-carboxylic acid |
| 145 | 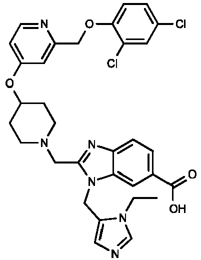 | 2-{[4-({2-[(2,4-dichlorophenoxy)methyl]pyridin-4-yl}oxy)piperidin-1-yl]methyl}-1-[(1-ethyl-1H-imidazol-5-yl)methyl]-1H-1,3-benzodiazole-6-carboxylic acid |
| 146 | 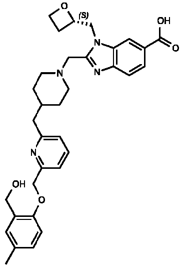 | 2-({4-[(6-{[2-(hydroxymethyl)-4-methylphenoxy]methyl}pyridin-2-yl)methyl]piperidin-1-yl}methyl)-1-{[(2S)-oxetan-2-yl]methyl}-1H-1,3-benzodiazole-6-carboxylic acid |
| 148 | 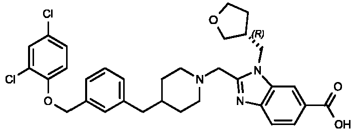 | 2-{[4-({3-[(2,4-dichlorophenoxy)methyl]phenyl}methyl)pi peridin-1-yl]methyl}-1-{[(3R)-oxolan-3-yl]methyl}-1H-1,3-benzodiazole-6-carboxylic acid |
| 149 | 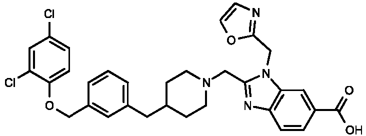 | 2-{[4-({3-[(2,4-dichlorophenoxy)methyl]phenyl}methyl)pi peridin-1-yl]methyl}-1-[(1,3-oxazol-2-yl)methyl]-1H-1,3-benzodiazole-6-carboxylic acid |
| 150 | 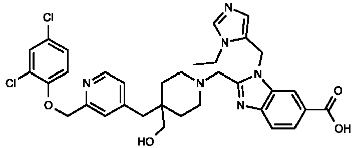 | 2-{[4-({2-[(2,4-dichlorophenoxy)methyl]pyridin-4-yl}methyl)-4-(hydroxymethyl)piperidin-1-yl]methyl}-1-[(1-ethyl-1H-imidazol-5-yl)methyl]-1H-1,3-benzodiazole-6-carboxylic acid |
| 151 | 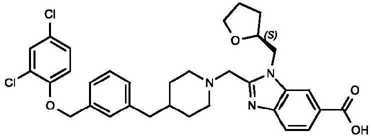 | 2-{[4-({3-[(2,4-dichlorophenoxy)methyl]phenyl}methyl)pi peridin-1-yl]methyl}-1-{[(2S)-oxolan-2-yl]methyl}-1H-1,3-benzodiazole-6-carboxylic acid |
| 152 | 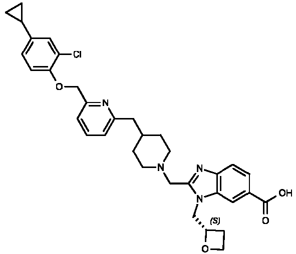 | 2-{[4-({6-[(2-chloro-4-cyclopropylphenoxy)methyl]pyridin-2-yl}methyl)piperidin-1-yl]methyl}-1-{[(2S)-oxetan-2-yl]methyl}-1H-1,3-benzodiazole-6-carboxylic acid |
| 153 | 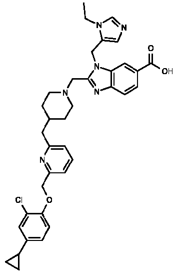 | 2-{[4-({6-[(2-chloro-4-cyclopropylphenoxy)methyl]pyridin-2-yl}methyl)piperidin-1-yl]methyl}-1-[(1-ethyl-1H-imidazol-5-yl)methyl]-1H-1,3-benzodiazole-6-carboxylic acid |
| 154 | 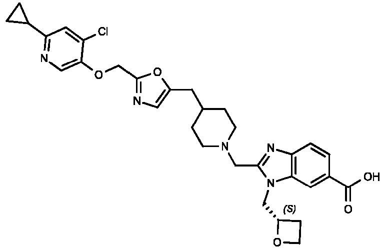 | 2-({4-[(2-{[(4-chloro-6-cyclopropylpyridin-3⁻yl)oxy]methyl}-1,3-oxazol-5-yl)methyl]piperidin-1-yl}methyl)-1-{[(2S)-oxetan-2-yl]methyl}-1H-1,3-benzodiazole-6-carboxylic acid |
| 155 | 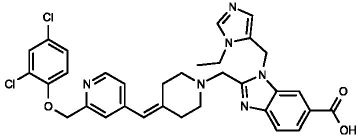 | 2-{[4-({2-[(2,4-dichlorophenoxy)methyl]pyridin-4-yl} methylidene)piperidin-1-yl]methyl }-1-[(1-ethyl-1H-imidazol-5-yl)methyl]-1H-1,3-benzodiazole-6-carboxylic acid |
| 156 | 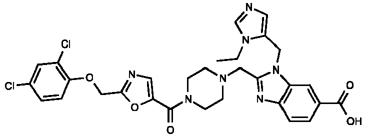 | 2-[(4-{2-[(2,4-dichlorophenoxy)methyl]-1,3-oxazole-5-carbonyl}piperazin-1-yl)methyl]-1-[(1-ethyl-1H-imidazol-5-yl)methyl]-1H-1,3 -benzodiazole-6-carboxylic acid |
| 157 | 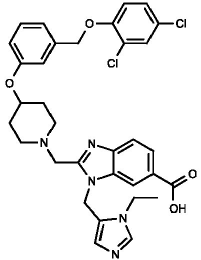 | 2-[(4-{3-[(2,4-dichlorophenoxy)methyl]phenoxy}piperidin -1-yl)methyl]-1-[(1-ethyl-1H-imidazol-5-yl)methyl]-1H-1,3 -benzodiazole-6-carboxylic acid |
| 158 | 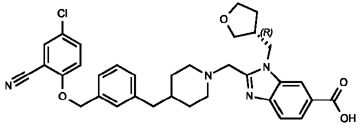 | 2-{[4-({3-[(4-chloro-2-cyanophenoxy)methyl]phenyl}methyl)piper idin-1-yl]methyl}-1-{[(3R)-oxolan-3-yl]methyl}-1H-1,3-benzodiazole-6-carboxylic acid |
| 159 | 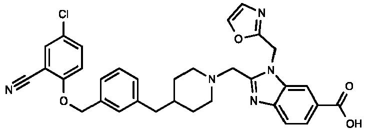 | 2-{[4-({3-[(4-chloro-2-cyanophenoxy)methyl]phenyl}methyl)piper idin-1-yl]methyl}-1-[(1,3-oxazol-2-yl)methyl]-1H-1,3 -benzodiazole-6-carboxylic acid |
| 160 | 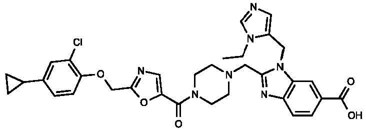 | 2-[(4-{2-[(2-chloro-4-cyclopropylphenoxy)methyl]-1,3-oxazole-5-carbonyl}piperazin-1-yl)methyl]-1-[(1-ethyl-1H-imidazol-5-yl)methyl]-1H-1,3-benzodiazole-6-carboxylic acid |
| 161 | 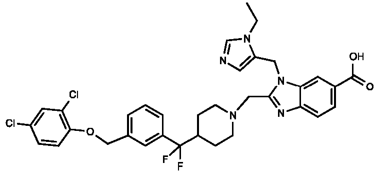 | 2-{[4-({3-[(2,4-dichlorophenoxy)methyl]phenyl}difluorom ethyl)piperidin-1-yl]methyl}-1-[(1-ethyl-1H-imidazol-5-yl)methyl]-1H-1,3-benzodiazole-6-carboxylic acid |
| 162 | 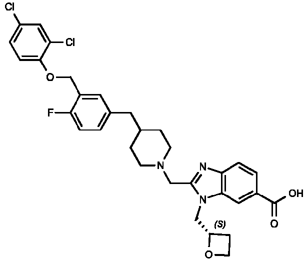 | 2-{[4-({3-[(2,4-dichlorophenoxy)methyl]-4-fluorophenyl}methyl)piperidin-1-yl]methyl }-1-{ [(2S)-oxetan-2-yl]methyl}-1H-1,3-benzodiazole-6-carboxylic acid |
| 163 | 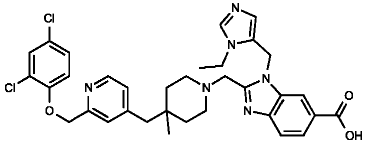 | 2-{[4-({2-[(2,4-dichlorophenoxy)methyl]pyridin-4-yl} methyl)-4-methylpiperidin-1-yl]methyl}-1-[(1-ethyl-1H-imidazol-5-yl)methyl]-1H-1,3-benzodiazole-6-carboxylic acid |
| 164 | 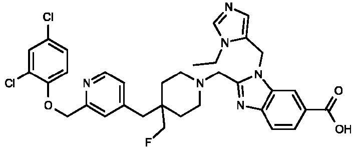 | 2-{[4-({2-[(2,4-dichlorophenoxy)methyl]pyridin-4-yl}methyl)-4-(fluoromethyl)piperidin-1-yl]methyl}-1-[(1-ethyl-1H-imidazol-5-yl)methyl]-1H-1,3 -benzodiazole-6-carboxylic acid |
| 165 | 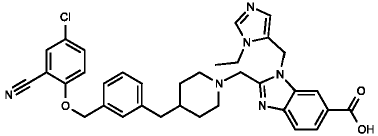 | 2-{[4-({3-[(4-chloro-2-cyanophenoxy)methyl]phenyl}methyl)piper idin-1-yl]methyl}-1-[(1-ethyl-1H-imidazol-5-yl)methyl]-1H-1,3-benzodiazole-6-carboxylic acid |
| 166 | 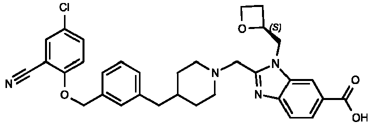 | 2-{[4-({3-[(4-chloro-2-cyanophenoxy)methyl]phenyl}methyl)piper idin-1-yl]methyl}-1-{[(2S)-oxetan-2-yl]methyl}-1H-1,3-benzodiazole-6-carboxylic acid |
| 168 | 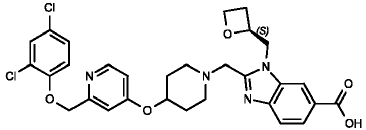 | 2-{[4-({2-[(2,4-dichlorophenoxy)methyl]pyridin-4-yl}oxy)piperidin-1-yl]methyl}-1-{[(2S)-oxetan-2-yl]methyl}-1H-1,3-benzodiazole-6-carboxylic acid |
| 169 | 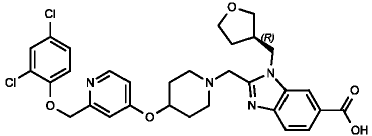 | 2-{[4-({2-[(2,4-dichlorophenoxy)methyl]pyridin-4-yl}oxy)piperidin-1-yl]methyl}-1-{[(3R)-oxolan-3-yl]methyl}-1H-1,3-benzodiazole-6-carboxylic acid |
| 170 | 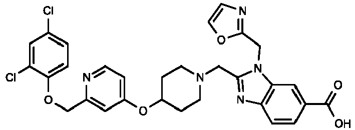 | 2-{[4-({2-[(2,4-dichlorophenoxy)methyl]pyridin-4-yl}oxy)piperidin-1-yl]methyl}-1-[(1,3-oxazol-2-yl)methyl]-1H-1,3-benzodiazole-6-carboxylic acid |
| 171 | 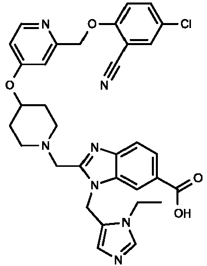 | 2-{[4-({2-[(4-chloro-2-cyanophenoxy)methyl]pyridin-4-yl}oxy)piperidin-1-yl]methyl}-1-[(1-ethyl-1H-imidazol-5-yl)methyl]-1H-1,3-benzodiazole-6-carboxylic acid |
| 174 | 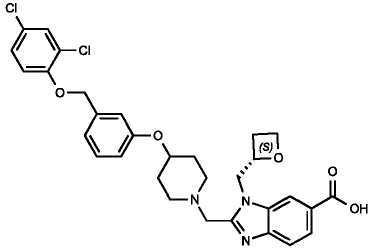 | 2-[(4-{3-[(2,4-dichlorophenoxy)methyl]phenoxy}piperidin -1-yl)methyl]-1-{[(2S)-oxetan-2-yl]methyl}-1H-1,3-benzodiazole-6-carboxylic acid |
| 178 | 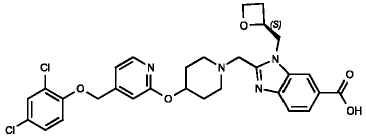 | 2-{[4-({4-[(2,4-dichlorophenoxy)methyl]pyridin-2-yl}oxy)piperidin-1-yl]methyl }-1-{ [(2S)-oxetan-2-yl]methyl}-1H-1,3-benzodiazole-6-carboxylic acid |
| 179 | 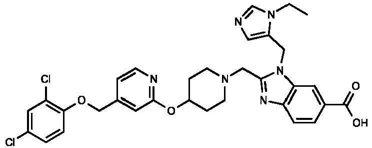 | 2-{[4-({4-[(2,4-dichlorophenoxy)methyl]pyridin-2-yl}oxy)piperidin-1-yl]methyl}-1-[(1-ethyl-1H-imidazol-5-yl)methyl]-1H-1,3-benzodiazole-6-carboxylic acid |
| 182 | 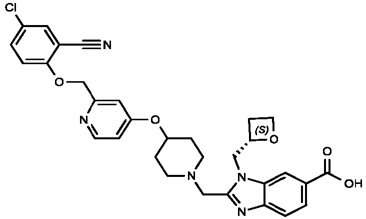 | 2-{[4-({2-[(4-chloro-2-cyanophenoxy)methyl]pyridin-4-yl}oxy)piperidin-1-yl]methyl }-1-{[(2S)-oxetan-2-yl]methyl}-1H-1,3-benzodiazole-6-carboxylic acid |
| 183 | 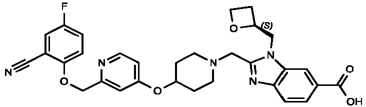 | 2-{[4-({2-[(2-cyano-4-fluorophenoxy)methyl]pyridin-4-yl}oxy)piperidin-1-yl]methyl}-1-{[(2S)-oxetan-2-yl]methyl}-1H-1,3-benzodiazole-6-carboxylic acid |
| 186 | 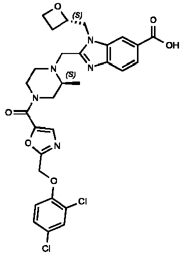 | 2-{[(2S)-4-{2-[(2,4-dichlorophenoxy)methyl]-1,3-oxazole-5-carbonyl }-2-methylpiperazin-1-yl]methyl}-1-{[(2S)-oxetan-2-yl]methyl}-1H-1,3-benzodiazole-6-carboxylic acid |
| 187 | 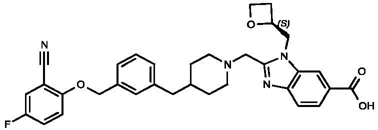 | 2-{[4-({3-[(2-cyano-4-fluorophenoxy)methyl]phenyl}methyl)piper idin-1-yl]methyl}-1-{[(2S)-oxetan-2-yl]methyl}-1H-1,3-benzodiazole-6-carboxylic acid |
| 188 | 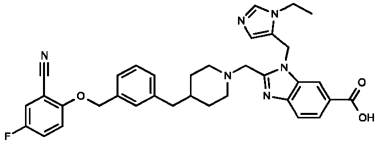 | 2-{[4-({3-[(2-cyano-4-fluorophenoxy)methyl]phenyl}methyl)piper idin-1-yl]methyl}-1-[(1-ethyl-1H-imidazol-5-yl)methyl]-1H-1,3-benzodiazole-6-carboxylic acid |
| 189 | 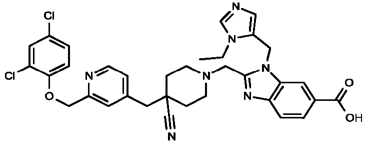 | 2-{[4-cyano-4-({2-[(2,4-dichlorophenoxy)methyl]pyridin-4-yl}methyl)piperidin-1-yl]methyl}-1-[(1-ethyl-1H-imidazol-5-yl)methyl]-1H-1,3-benzodiazole-6-carboxylic acid |
| 198 | 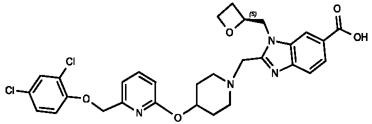 | (S)-2-((4-((6-((2,4-Dichlorophenoxy)methyl)pyridin-2-yl)oxy)piperidin-1-yl)methyl)-1-(oxetan-2-ylmethyl)-1H-benzo[d]imidazole-6-carboxylic acid |
| 199 | 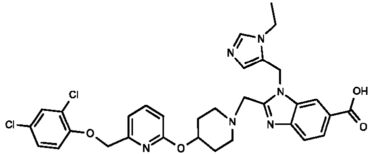 | 2-{[4-({6-[(2,4-dichlorophenoxy)methyl]pyridin-2-yl}oxy)piperidin-1-yl]methyl}-1-[(1-ethyl-1H-imidazol-5-yl)methyl]-1H-1,3-benzodiazole-6-carboxylic acid |
| 200 | 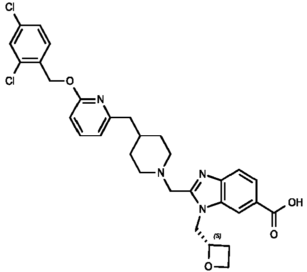 | 2-{[4-({6-[(2,4-dichlorophenyl)methoxy]pyridin-2-yl}methyl)piperidin-1-yl]methyl}-1-{[(2S)-oxetan-2-yl]methyl}-1H-1,3-benzodiazole-6-carboxylic acid |
| 202 | 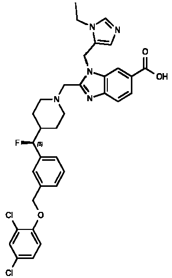 | 2-({4-[(S)-{3-[(2,4-dichlorophenoxy)methyl]phenyl}(fluoro)me thyl]piperidin-1-yl}methyl)-1-[(1-ethyl-1H-imidazol-5-yl)methyl]-1H-1,3-benzodiazole-6-carboxylic acid |
| 203 | 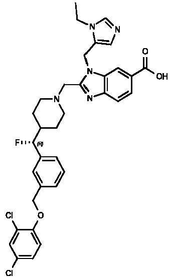 | 2-({4-[(R)-{3-[(2,4-dichlorophenoxy)methyl]phenyl}(fluoro)me thyl]piperidin-1-yl}methyl)-1-[(1-ethyl-1H-imidazol-5-yl)methyl]-1H-1,3-benzodiazole-6-carboxylic acid |
| 204 | 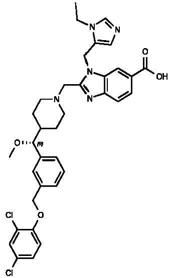 | (R)-2-((4-((3-((2,4-dichlorophenoxy)methyl)phenyl)(methoxy) methyl)piperidin-1-yl)methyl)-1-((1-ethyl-1H-imidazol-5-yl)methyl)-1H-benzo[d]imidazole-6-carboxylic acid |
| 205 | 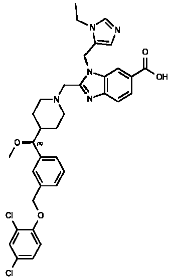 | (S)-2-((4-((3-((2,4-dichlorophenoxy)methyl)phenyl)(methoxy) methyl)piperidin-1-yl)methyl)-1-((1-ethyl-1H-imidazol-5-yl)methyl)-1H-benzo[d]imidazole-6-carboxylic acid |
| 206 | 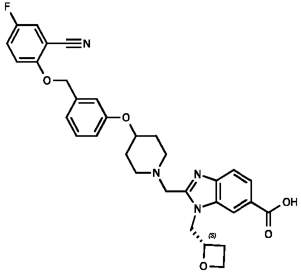 | (S)-2-((4-(3-((2-Cyano-4-fluorophenoxy)methyl)phenoxy)piperidin-1-yl)methyl)-1-(oxetan-2-ylmethyl)-1H-benzo[d]imidazole-6-carboxylic acid |
| 207 | 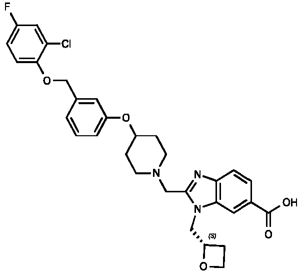 | 2-[(4-{3-[(2,4-dichlorophenoxy)methyl]phenoxy}piperidin -1-yl)methyl]-1-[(1,2-oxazol-5-yl)methyl]-1H-1,3-benzodiazole-6-carboxylic acid |
| 208 | 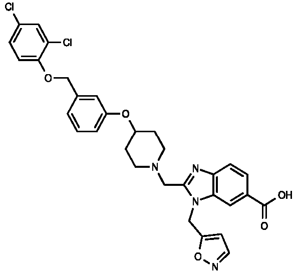 | 2-((4-(3-((2,4-Dichlorophenoxy)methyl)phenoxy)piperidin -1-yl)methyl)-1-(isoxazol-5-ylmethyl)-1H-benzo[d]imidazole-6-carboxylic acid |
| 214 | 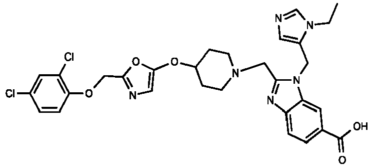 | 2-{[4-({2-[(2,4-dichlorophenoxy)methyl]-1,3-oxazol-5-yl}oxy)piperidin-1-yl]methyl}-1-[(1-ethyl-1H-imidazol-5-yl)methyl]-1H-1,3 -benzodiazole-6-carboxylic acid |
| 215 | 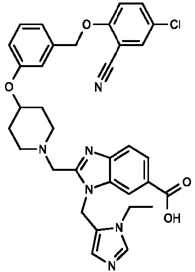 | 2-[(4-{3-[(4-chloro-2-cyanophenoxy)methyl]phenoxy}piperidin-1-yl)methyl]-1-[(1-ethyl-1H-imidazol-5-yl)methyl]-1H-1,3 -benzodiazole-6-carboxylic acid |
| 216 | 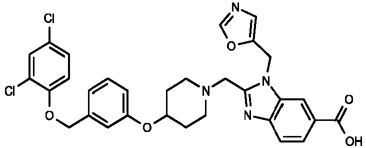 | 2-((4-(3-((2,4-Dichlorophenoxy)methyl)phenoxy)piperidin -1-yl)methyl)-1-(oxazol-5-ylmethyl)-1H-benzo[d]imidazole-6-carboxylic acid |
| 217 | 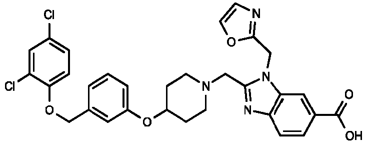 | 2-[(4-{3-[(2,4-dichlorophenoxy)methyl]phenoxy}piperidin -1-yl)methyl]-1-[(1,3-oxazol-2-yl)methyl]-1H-1,3-benzodiazole-6-carboxylic acid |
| 220 | 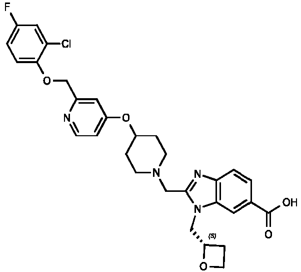 | (S)-2-((4-((2-((2-Chloro-4-fluorophenoxy)methyl)pyridin-4-yl)oxy)piperidin-1-yl)methyl)-1-(oxetan-2-ylmethyl)-1H-benzo[d]imidazole-6-carboxylic acid |
| 221 | 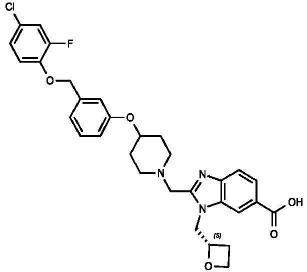 | 2-[(4-{3-[(4-chloro-2-fluorophenoxy)methyl]phenoxy}piperidin-1-yl)methyl]-1-{ [(2S)-oxetan-2-yl]methyl}-1H-1,3-benzodiazole-6-carboxylic acid |
| 224 | 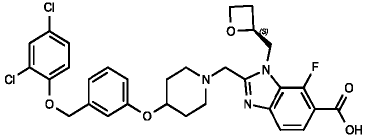 | 2-[(4-{3-[(2,4-dichlorophenoxy)methyl]phenoxy}piperidin -1-yl)methyl]-7-fluoro-1-{[(2S)-oxetan-2-yl]methyl}-1H-1,3-benzodiazole-6-carboxylic acid |
| 227 | 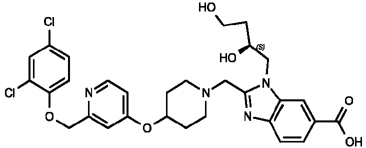 | 2-{[4-({2-[(2,4-dichlorophenoxy)methyl]pyridin-4-yl}oxy)piperidin-1-yl]methyl}-1-[(2S)-2,4-dihydroxybutyl]-1H-1,3-benzodiazole-6-carboxylic acid |
| 230 | 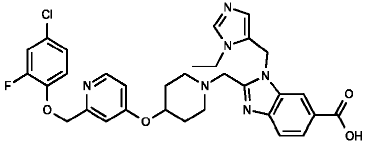 | 2-((4-((2-((4-Chloro-2-fluorophenoxy)methyl)pyridin-4-yl)oxy)piperidin-1-yl)methyl)-1-((1-ethyl-1H-imidazol-5-yl)methyl)-1H-benzo[d]imidazole-6-carboxylic acid |
| 231 | 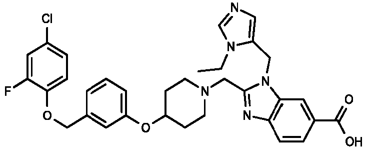 | 2-[(4-{3-[(4-chloro-2-fluorophenoxy)methyl]phenoxy}piperidin-1-yl)methyl]-1-[(1-ethyl-1H-imidazol-5-yl)methyl]-1H-1,3 -benzodiazole-6-carboxylic acid |
| 232 | 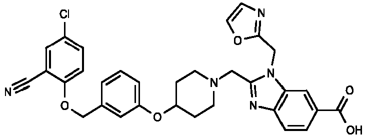 | 2-[(4-{3-[(4-chloro-2-cyanophenoxy)methyl]phenoxy}piperidin-1⁻yl)methyl]-1-[(1,3-oxazol-2-yl)methyl]-1H-1,3-benzodiazole-6-carboxylic acid |
| 239 | 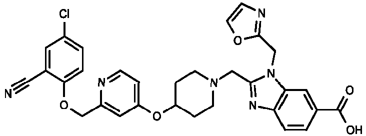 | 2-((4-((2-((4-Chloro-2-isocyanophenoxy)methyl)pyridin-4-yl)oxy)piperidin-1-yl)methyl)-1-(oxazol-2-ylmethyl)-1H-benzo[d]imidazole-6-carboxylic acid |
| 240 | 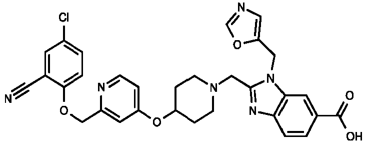 | 2-{[4-({2-[(4-chloro-2-cyanophenoxy)methyl]pyridin-4-yl}oxy)piperidin-1-yl]methyl}-1-[(1,3-oxazol-5-yl)methyl]-1H-1,3-benzodiazole-6-carboxylic acid |
| 241 | 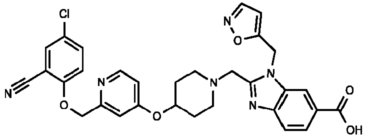 | 2-{[4-({2-[(4-chloro-2-cyanophenoxy)methyl]pyridin-4-yl}oxy)piperidin-1-yl]methyl}-1-[(1,2-oxazol-5-yl)methyl]-1H-1,3-benzodiazole-6-carboxylic acid |
| 242 | 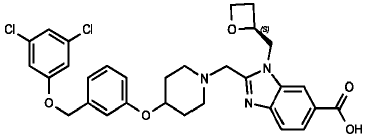 | 2-[(4-{3-[(3,5-dichlorophenoxy)methyl]phenoxy}piperidin -1-yl)methyl]-1-{[(2S)-oxetan-2-yl]methyl}-1H-1,3-benzodiazole-6-carboxylic acid |
| 243 | 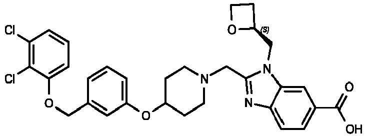 | 2-[(4-{3-[(2,3-dichlorophenoxy)methyl]phenoxy}piperidin -1-yl)methyl]-1-{[(2S)-oxetan-2-yl]methyl}-1H-1,3-benzodiazole-6-carboxylic acid |
| 246 | 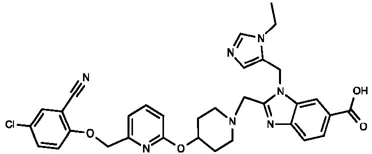 | 2-{[4-({6-[(4-chloro-2-cyanophenoxy)methyl]pyridin-2-yl}oxy)piperidin-1-yl]methyl}-1-[(1-ethyl-1H-imidazol-5-yl)methyl]-1H-1,3-benzodiazole-6-carboxylic acid |
| 247 | 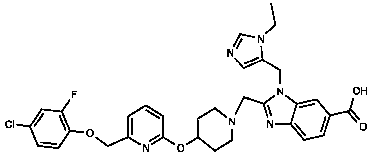 | 2-{[4-({6-[(4-chloro-2-fluorophenoxy)methyl]pyridin-2-yl}oxy)piperidin-1-yl]methyl}-1-[(1-ethyl-1H-imidazol-5-yl)methyl]-1H-1,3-benzodiazole-6-carboxylic acid |
| 248 | 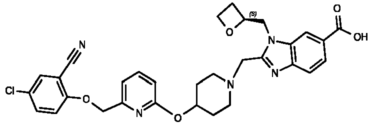 | 2-{[4-({6-[(4-chloro-2-cyanophenoxy)methyl]pyridin-2-yl}oxy)piperidin-1-yl]methyl }-1-{ [(2S)-oxetan-2-yl]methyl}-1H-1,3-benzodiazole-6-carboxylic acid |
| 249 | 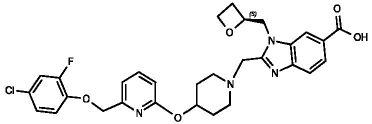 | 2-{[4-({6-[(4-chloro-2-fluorophenoxy)methyl]pyridin-2-yl}oxy)piperidin-1-yl]methyl }-1-{ [(2S)-oxetan-2-yl]methyl}-1H-1,3-benzodiazole-6-carboxylic acid |
| 250 | 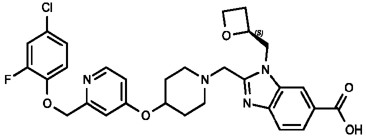 | 2-{[4-({2-[(4-chloro-2-fluorophenoxy)methyl]pyridin-4-yl}oxy)piperidin-1-yl]methyl }-1-{ [(2S)-oxetan-2-yl]methyl}-1H-1,3-benzodiazole-6-carboxylic acid |
| 251 | 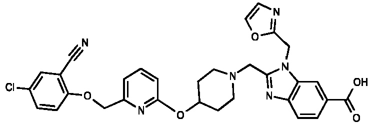 | 2-{[4-({6-[(4-chloro-2-cyanophenoxy)methyl]pyridin-2-yl}oxy)piperidin-1-yl]methyl}-1-[(1,3-oxazol-2-yl)methyl]-1H-1,3-benzodiazole-6-carboxylic acid |
| 252 | 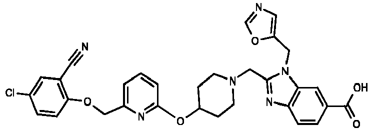 | 2-{[4-({6-[(4-chloro-2-cyanophenoxy)methyl]pyridin-2-yl}oxy)piperidin-1-yl]methyl}-1-[(1,3-oxazol-5-yl)methyl]-1H-1,3-benzodiazole-6-carboxylic acid |
| 253 | 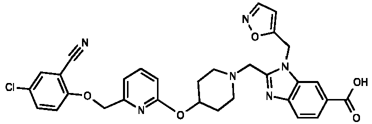 | 2-{[4-({6-[(4-chloro-2-cyanophenoxy)methyl]pyridin-2-yl}oxy)piperidin-1-yl]methyl}-1-[(1,2-oxazol-5-yl)methyl]-1H-1,3-benzodiazole-6-carboxylic acid |
| 254 | 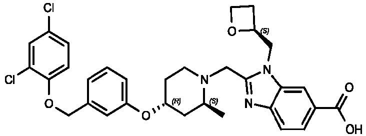 | 2-{[(2S,4R)-4-{3-[(2,4-dichlorophenoxy)methyl]phenoxy}-2-methylpiperidin-1-yl]methyl }-1-{[(2S)-oxetan-2-yl]methyl}-1H-1,3-benzodiazole-6-carboxylic acid |
| 255 | 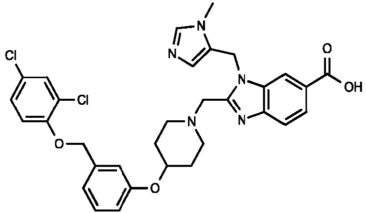 | 2-[(4-{3-[(2,4-dichlorophenoxy)methyl]phenoxy}piperidin -1-yl)methyl]-1-[(1-methyl-1H-imidazol-5-yl)methyl]-1H-1,3 -benzodiazole-6-carboxylic acid |
| 256 | 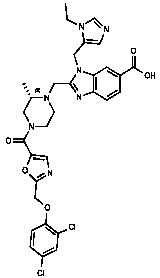 | 2-{[(2S)-4-{2-[(2,4-dichlorophenoxy)methyl]-1,3-oxazole-5-carbonyl}-2-methylpiperazin-1-yl]methyl}-1-[(1-ethyl-1H-imidazol-5-yl)methyl]-1H-1,3-benzodiazole-6-carboxylic acid |
| 257 | 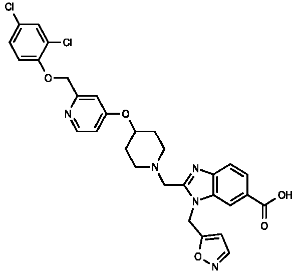 | 2-{[4-({2-[(2,4-dichlorophenoxy)methyl]pyridin-4-yl}oxy)piperidin-1-yl]methyl}-1-[(1,2-oxazol-5-yl)methyl]-1H-1,3-benzodiazole-6-carboxylic acid |
| 258 | 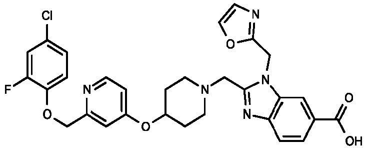 | 2-{[4-({2-[(4-chloro-2-fluorophenoxy)methyl]pyridin-4-yl}oxy)piperidin-1-yl]methyl}-1-[(1,3-oxazol-2-yl)methyl]-1H-1,3-benzodiazole-6-carboxylic acid |
| 259 | 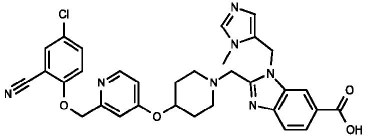 | 2-{[4-({2-[(4-chloro-2-cyanophenoxy)methyl]pyridin-4-yl}oxy)piperidin-1-yl]methyl}-1-[(1-methyl-1H-imidazol-5-yl)methyl]-1H-1,3-benzodiazole-6-carboxylic acid |
| 260 | 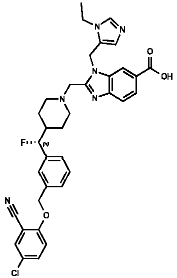 | )-2-((4-((3 -((4-Chloro-2-cyanophenoxy)methyl)phenyl)fluoromethyl )piperidin-1-yl)methyl)-1-((1-ethyl-1H-imidazol-5-yl)methyl)-1H-benzo[d]imidazole-6-carboxylic acid |
| 261 | 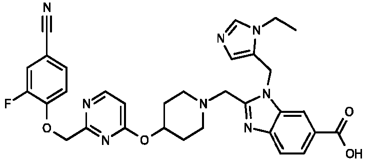 | 2-((4-((2-((4-Cyano-2-fluorophenoxy)methyl)pyrimidin-4-yl)oxy)piperidin-1-yl)methyl)-1-((1-ethyl-1H-imidazol-5-yl)methyl)-1H-benzo[d]imidazole-6-carboxylic acid |
| 262 | 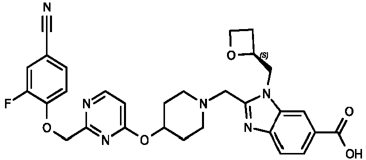 | (S)-2-((4-((2-((4-Cyano-2-fluorophenoxy)methyl)pyrimidin-4-yl)oxy)piperidin-1-yl)methyl)-1-(oxetan-2-ylmethyl)-1H-benzo[d]imidazole-6-carboxylic acid |
| 263 | 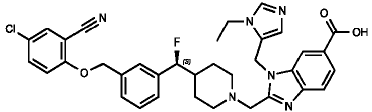 | (S)-2-((4-((3-((4-chloro-2-cyanophenoxy)methyl)phenyl)fluoromethyl )piperidin-1-yl)methyl)-1-((1-ethyl-1H-imidazol-5-yl)methyl)-1H-benzo[d]imidazole-6-carboxylic acid |
| 264 | 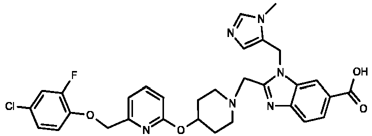 | 2-{[4-({6-[(4-chloro-2-fluorophenoxy)methyl]pyridin-2-yl}oxy)piperidin-1-yl]methyl}-1-[(1-methyl-1H-imidazol-5-yl)methyl]-1H-1,3-benzodiazole-6-carboxylic acid |
| 265 | 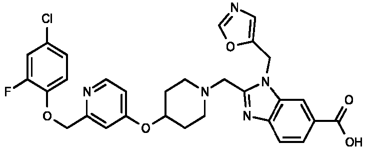 | 2-{[4-({2-[(4-chloro-2-fluorophenoxy)methyl]pyridin-4-yl}oxy)piperidin-1-yl]methyl}-1-[(1,3-oxazol-5-yl)methyl]-1H-1,3-benzodiazole-6-carboxylic acid |
| 266 | 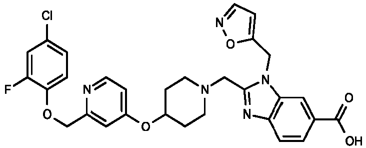 | 2-{[4-({2-[(4-chloro-2-fluorophenoxy)methyl]pyridin-4-yl}oxy)piperidin-1-yl]methyl}-1-[(1,2-oxazol-5-yl)methyl]-1H-1,3-benzodiazole-6-carboxylic acid |
| 267 | 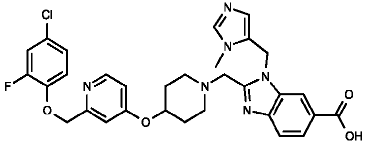 | 2-{[4-({2-[(4-chloro-2-fluorophenoxy)methyl]pyridin-4-yl}oxy)piperidin-1-yl]methyl}-1-[(1-methyl-1H-imidazol-5-yl)methyl]-1H-1,3-benzodiazole-6-carboxylic acid |
| 268 | 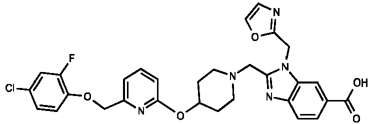 | 2-{[4-({6-[(4-chloro-2-fluorophenoxy)methyl]pyridin-2-yl}oxy)piperidin-1-yl]methyl}-1-[(1,3-oxazol-2-yl)methyl]-1H-1,3-benzodiazole-6-carboxylic acid |
| 269 | 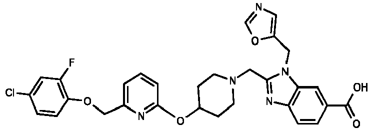 | 2-{[4-({6-[(4-chloro-2-fluorophenoxy)methyl]pyridin-2-yl}oxy)piperidin-1-yl]methyl}-1-[(1,3-oxazol-5-yl)methyl]-1H-1,3-benzodiazole-6-carboxylic acid |
| 270 | 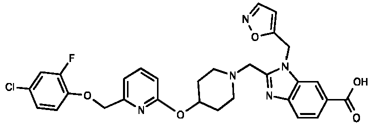 | 2-{[4-({6-[(4-chloro-2-fluorophenoxy)methyl]pyridin-2-yl}oxy)piperidin-1-yl]methyl}-1-[(1,2-oxazol-5-yl)methyl]-1H-1,3-benzodiazole-6-carboxylic acid |
| 271 | 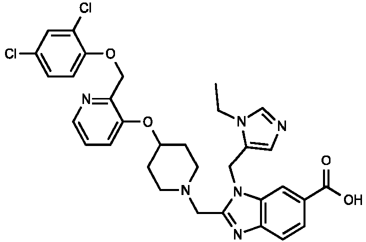 | 2-((4-((2-((2,4-Dichlorophenoxy)methyl)pyridin-3-yl)oxy)piperidin-1-yl)methyl)-1-((1-ethyl-1H-imidazol-5-yl)methyl)-1H-benzo[d]imidazole-6-carboxylic acid |
| 272 | 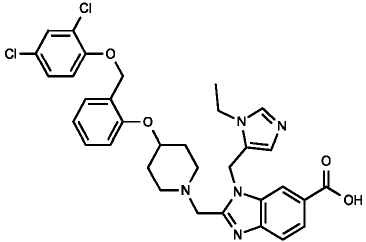 | 2-[(4-{2-[(2,4-dichlorophenoxy)methyl]phenoxy}piperidin -1-yl)methyl]-1-[(1-ethyl-1H-imidazol-5-yl)methyl]-1H-1,3 -benzodiazole-6-carboxylic acid |
| 274 | 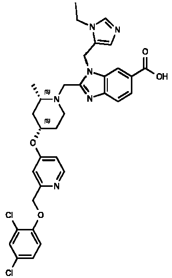 | 2-(((2S,4S)-4-((2-((2,4-Dichlorophenoxy)methyl)pyridin-4-yl)oxy)-2-methylpiperidin-1-yl)methyl)-1-((1-ethyl-1H-imidazol-5-yl)methyl)-1H-benzo[d]imidazole-6-carboxylic acid |
| 275 | 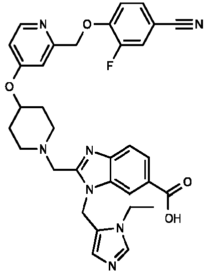 | 2-{[4-({2-[(4-cyano-2-fluorophenoxy)methyl]pyridin-4-yl}oxy)piperidin-1-yl]methyl}-1-[(1-ethyl-1H-imidazol-5-yl)methyl]-1H-1,3-benzodiazole-6-carboxylic acid |
| 276 | 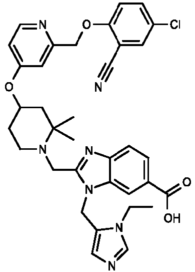 | 2-((4-((2-((4-Chloro-2-cyanophenoxy)methyl)pyridin-4-yl)oxy)-2,2-dimethylpiperidin-1-yl)methyl)-1-((1-ethyl-1H-imidazol-5-yl)methyl)-1H-benzo[d]imidazole-6-carboxylic acid |
| 277 | 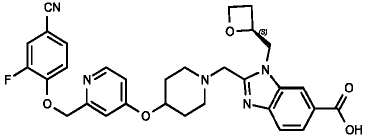 | 2-{[4-({2-[(4-cyano-2-fluorophenoxy)methyl]pyridin-4-yl}oxy)piperidin-1-yl]methyl }-1-{ [(2S)-oxetan-2-yl]methyl}-1H-1,3-benzodiazole-6-carboxylic acid |
| 278 | 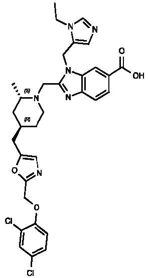 | 2-{[(2S,4R)-4-({2-[(2,4-dichlorophenoxy)methyl]-1,3-oxazol-5-yl} methyl)-2-methylpiperidin-1-yl]methyl}-1-[(1-ethyl-1H-imidazol-5-yl)methyl]-1H-1,3-benzodiazole-6-carboxylic acid |
| 279 | 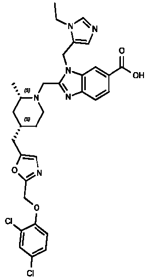 | 2-{[(2S,4S)-4-({2-[(2,4-dichlorophenoxy)methyl]-1,3-oxazol-5-yl}methyl)-2-methylpiperidin-1-yl]methyl}-1-[(1-ethyl-1H-imidazol-5-yl)methyl]-1H-1,3-benzodiazole-6-carboxylic acid |
| 280 | 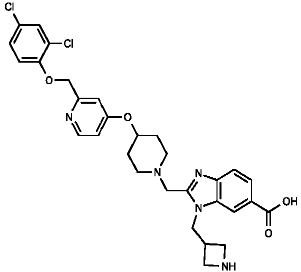 | 1-[(azetidin-3-yl)methyl]-2-{[4-({2-[(2,4-dichlorophenoxy)methyl]pyridin-4-yl}oxy)piperidin-1-yl]methyl}-1H-1,3-benzodiazole-6-carboxylic acid |
| 283 | 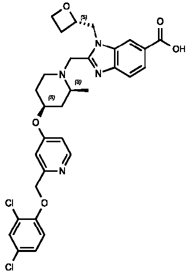 | 2-{[(2S,4S)-4-({2-[(2,4-dichlorophenoxy)methyl]pyridin-4-yl}oxy)-2-methylpiperidin-1-yl]methyl }-1-{[(2S)-oxetan-2-yl]methyl}-1H-1,3-benzodiazole-6-carboxylic acid |
| 284 | 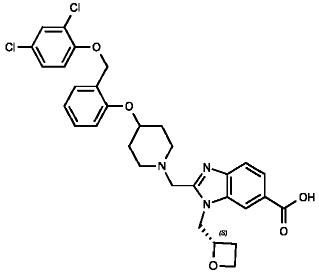 | 2-[(4-{2-[(2,4-dichlorophenoxy)methyl]phenoxy}piperidin -1-yl)methyl]-1-{[(2S)-oxetan-2-yl]methyl}-1H-1,3-benzodiazole-6-carboxylic acid |
| 285 | 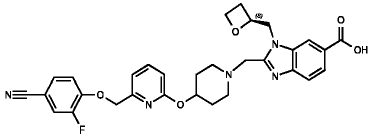 | (S)-2-((4-((6-((4-Cyano-2-fluorophenoxy)methyl)pyridin-2-yl)oxy)piperidin-1-yl)methyl)-1-(oxetan-2-ylmethyl)-1H-benzo[d]imidazole-6-carboxylic acid |
| 286 | 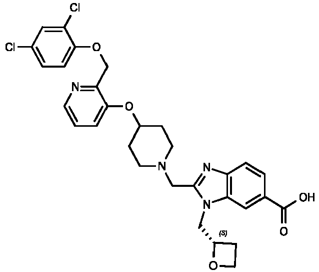 | 2-{[4-({2-[(2,4-dichlorophenoxy)methyl]pyridin-3-yl}oxy)piperidin-1-yl]methyl }-1-{ [(2S)-oxetan-2-yl]methyl}-1H-1,3-benzodiazole-6-carboxylic acid |
| 287 | 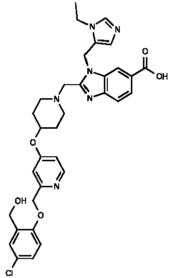 | 2-({4-[(2-{[4-chloro-2-(hydroxymethyl)phenoxy]methyl}pyridin-4-yl)oxy]piperidin-1-yl}methyl)-1-[(1-ethyl-1H-imidazol-5-yl)methyl]-1H-1,3-benzodiazole-6-carboxylic acid |
| 288 | 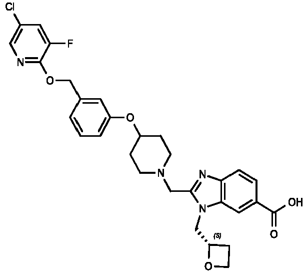 | 2-{[4-(3-{[(5-chloro-3-fluoropyridin-2-yl)oxy]methyl}phenoxy)piperidin-1-yl]methyl }-1-{ [(2S)-oxetan-2-yl]methyl}-1H-1,3-benzodiazole-6-carboxylic acid |
| 290 | 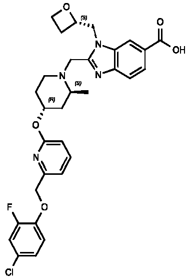 | 2-{[(2S,4R)-4-({6-[(4-chloro-2-fluorophenoxy)methyl]pyridin-2-yl}oxy)-2-methylpiperidin-1-yl]methyl }-1-{ [(2S)-oxetan-2-yl]methyl}-1H-1,3-benzodiazole-6-carboxylic acid |
| 291 | 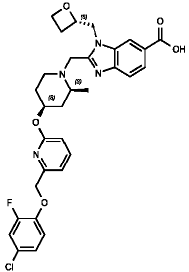 | 2-{[(2S,4S)-4-({6-[(4-chloro-2-fluorophenoxy)methyl]pyridin-2-yl}oxy)-2-methylpiperidin-1-yl]methyl }-1-{ [(2S)-oxetan-2-yl]methyl}-1H-1,3-benzodiazole-6-carboxylic acid |
| 292 | 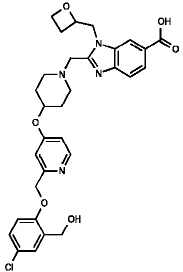 | 2-({4-[(2-{[4-chloro-2-(hydroxymethyl)phenoxy]methyl}pyridin-4-yl)oxy]piperidin-1-yl}methyl)-1-[(oxetan-2-yl)methyl]-1H-1,3-benzodiazole-6-carboxylic acid |
| 294 | 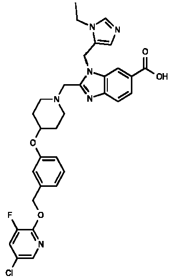 | 2-{[4-(3-{[(5-chloro-3-fluoropyridin-2-yl)oxy]methyl}phenoxy)piperidin-1-yl]methyl}-1-[(1-ethyl-1H-imidazol-5-yl)methyl]-1H-1,3-benzodiazole-6-carboxylic acid |
| 295 | 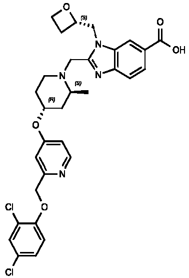 | 2-{[(2S,4R)-4-({2-[(2,4-dichlorophenoxy)methyl]pyridin-4-yl}oxy)-2-methylpiperidin-1-yl]methyl }-1-{ [(2S)-oxetan-2-yl]methyl}-1H-1,3-benzodiazole-6-carboxylic acid |
| 296 | 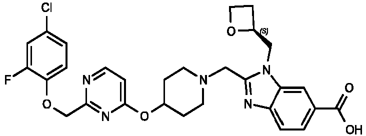 | 2-{[4-({2-[(4-chloro-2-fluorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 297 | 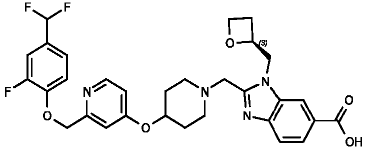 | (S)-2-((4-((2-((4-(Difluoromethyl)-2-fluorophenoxy)methyl)pyridin-4-yl)oxy)piperidin-1-yl)methyl)-1-(oxetan-2-ylmethyl)-1H-benzo[d]imidazole-6-carboxylic acid |
| 299 | 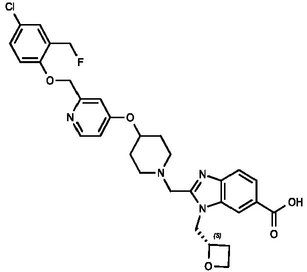 | (S)-2-((4-((2-((4-Chloro-2-(fluoromethyl)phenoxy)methyl)pyridin-4-yl)oxy)piperidin-1-yl)methyl)-1-(oxetan-2-ylmethyl)-1H-benzo[d]imidazole-6-carboxylic acid |
| 300 | 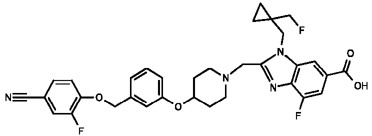 | 2-((4-(3-((4-Cyano-2-fluorophenoxy)methyl)phenoxy)piperidin-1-yl)methyl)-4-fluoro-1-((1-(fluoromethyl)cyclopropyl)methyl)-1H-benzo[d]imidazole-6-carboxylic acid |
| 301 | 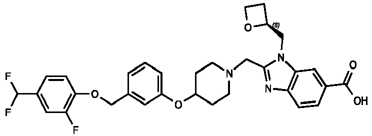 | 2-[(4-{3-[(4-cyano-2-fluorophenoxy)methyl]phenoxy}piperidin-1-yl)methyl]-4-fluoro-1-{[1-(fluoromethyl)cyclopropyl]methyl}-1H-1,3-benzodiazole-6-carboxylic acid |
| 302 | 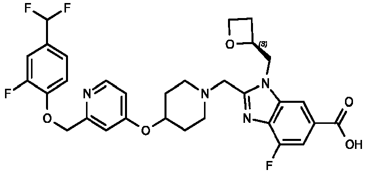 | 2-{[4-(3-{[4-(difluoromethyl)-2-fluorophenoxy]methyl}phenoxy)piperidin-1-yl]methyl}-1-{[(2S)-oxetan-2-yl]methyl}-1H-1,3-benzodiazole-6-carboxylic acid |
| 303 | 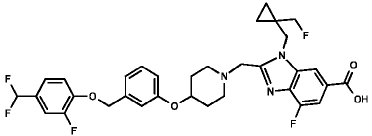 | 2-({4-[(2-{[4-(difluoromethyl)-2-fluorophenoxy]methyl}pyridin-4-yl)oxy]piperidin-1-yl}methyl)-4-fluoro-1-{[(2S)-oxetan-2-yl]methyl}-1H-1,3-benzodiazole-6-carboxylic acid |
| 304 | 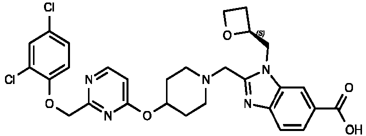 | (S)-2-((4-((2-((2,4-Dichlorophenoxy)methyl)pyrimidin-4-yl)oxy)piperidin-1-yl)methyl)-1-(oxetan-2-ylmethyl)-1H-benzo[d]imidazole-6-carboxylic acid |
| 305 | 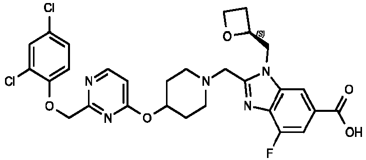 | 2-{[4-({2-[(2,4-dichlorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 306 | 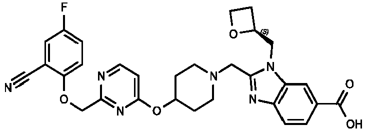 | 2-{[4-({2-[(2,4-dichlorophenoxy)methyl]pyrimidin-4-yl}oxy)piperidin-1-yl]methyl}-4-fluoro-1-{[(2S)-oxetan-2-yl]methyl}-1H-1,3-benzodiazole-6-carboxylic acid |
| 307 | 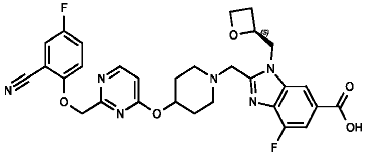 | 2-{[4-({2-[(2-cyano-4-fluorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 308 | 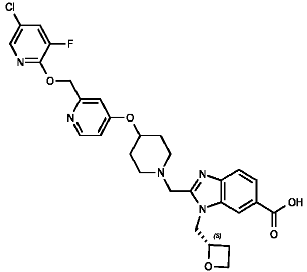 | 2-{[4-({2-[(2-cyano-4-fluorophenoxy)methyl]pyrimidin-4-yl}oxy)piperidin-1-yl]methyl}-4-fluoro-1-{[(2S)-oxetan-2-yl]methyl}-1H-1,3-benzodiazole-6-carboxylic acid |
| 309 | 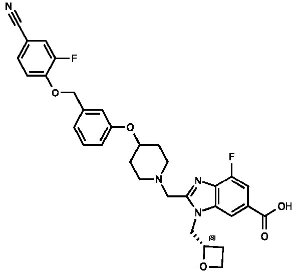 | 2-({4-[(2-{[(5-chloro-3-fluoropyridin-2-yl)oxy]methyl}pyridin-4-yl)oxy]piperidin-1-yl}methyl)-1-{[(2S)-oxetan-2-yl]methyl}-1H-1,3-benzodiazole-6-carboxylic acid |
| 310 | 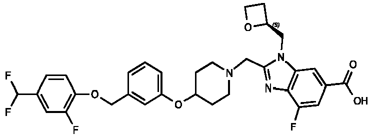 | 2-[(4-{3-[(4-cyano-2-fluorophenoxy)methyl]phenoxy}piperidin-1-yl)methyl]-4-fluoro-1-{[(2S)-oxetan-2-yl]methyl}-1H-1,3-benzodiazole-6-carboxylic acid |
| 311 | 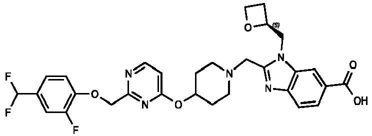 | 2-{[4-(3-{[4-(difluoromethyl)-2-fluorophenoxy] methyl} phenoxy)piperidin-1-yl]methyl}-4-fluoro-1-{[(2S)-oxetan-2-yl]methyl}-1H-1,3-benzodiazole-6-carboxylic acid |
| 312 | 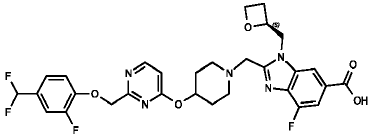 | 2-({4-[(2-{[4-(difluoromethyl)-2-fluorophenoxy] methyl } pyrimidin-4-yl)oxy]piperidin-1-yl}methyl)-1-{[(2S)-oxetan-2-yl]methyl}-1H-1,3-benzodiazole-6-carboxylic acid |
| 313 | 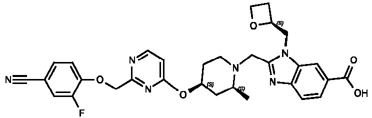 | 2-(((2S,4S)-4-((2-((4-Cyano-2-fluorophenoxy)methyl)pyrimidin-4-yl)oxy)-2-methylpiperidin-1-yl)methyl)-1-(((S)-oxetan-2-yl)methyl)-1H-benzo[d]imidazole-6-carboxylic acid |
| 314 | 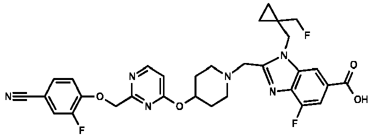 | 2-{[(2S,4S)-4-({2-[(4-cyano-2-fluorophenoxy)methyl]pyrimidin-4-yl}oxy)-2-methylpiperidin-1-yl]methyl}-1-{[(2S)-oxetan-2-yl]methyl}-1H-1,3-benzodiazole-6-carboxylic acid |
| 315 | 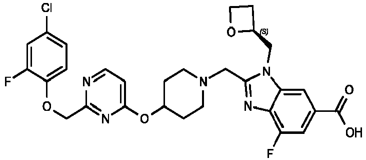 | 2-{[4-({2-[(4-cyano-2-fluorophenoxy)methyl]pyrimidin-4-yl}oxy)piperidin-1-yl]methyl }-4-fluoro-1-{[1-(fluoromethyl)cyclopropyl]methyl}-1H-1,3-benzodiazole-6-carboxylic acid |
| 316 | 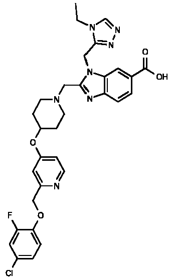 | 2-{[4-({2-[(4-chloro-2-fluorophenoxy)methyl]pyrimidin-4-yl}oxy)piperidin-1-yl]methyl }-4-fluoro-1-{[(2S)-oxetan-2-yl]methyl}-1H-1,3-benzodiazole-6-carboxylic acid |
| 317 | 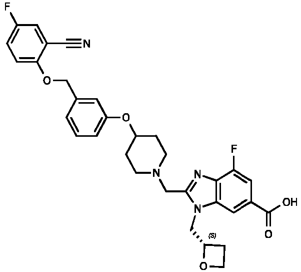 | 2-{[4-({2-[(4-chloro-2-fluorophenoxy)methyl]pyridin-4-yl}oxy)piperidin-1-yl]methyl}-1-[(4-ethyl-4H-1,2,4-triazol-3-yl)methyl]-1H-1,3-benzodiazole-6-carboxylic acid |
| 318 | 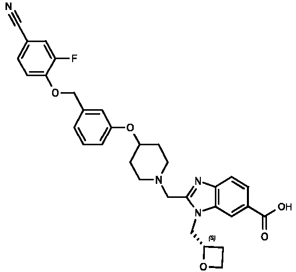 | 2-[(4-{3-[(2-cyano-4-fluorophenoxy)methyl]phenoxy}piperidin-1-yl)methyl]-4-fluoro-1-{[(2S)-oxetan-2-yl]methyl}-1H-1,3-benzodiazole-6-carboxylic acid |
| 319 | 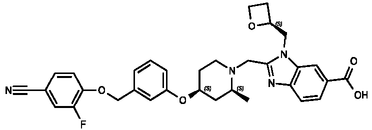 | 2-[(4-{3-[(4-cyano-2-fluorophenoxy)methyl]phenoxy}piperidin-1-yl)methyl]-1-{[(2S)-oxetan-2-yl]methyl}-1H-1,3-benzodiazole-6-carboxylic acid |
| 320 | 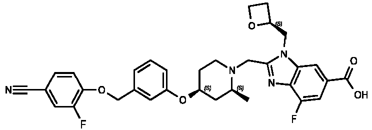 | 2-{[(2S,4S)-4-{3-[(4-cyano-2-fluorophenoxy)methyl]phenoxy}-2-methylpiperidin-1-yl]methyl}-1-{[(2S)-oxetan-2-yl]methyl}-1H-1,3-benzodiazole-6-carboxylic acid |
| 321 | 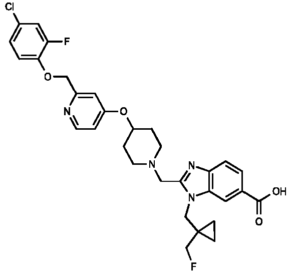 | 2-{[(2S,4S)-4-{3-[(4-cyano-2-fluorophenoxy)methyl]phenoxy}-2-methylpiperidin-1-yl]methyl}-4-fluoro-1-{[(2S)-oxetan-2-yl]methyl}-1H-1,3-benzodiazole-6-carboxylic acid |
| 322 | 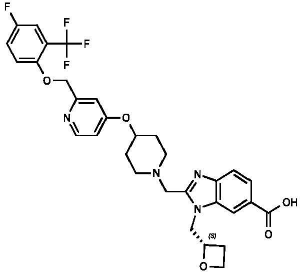 | 2-{[4-({2-[(4-chloro-2-fluorophenoxy)methyl]pyridin-4-yl}oxy)piperidin-1-yl]methyl }-1-{ [1-(fluoromethyl)cyclopropyl]methyl}-1H-1,3-benzodiazole-6-carboxylic acid |
| 323 | 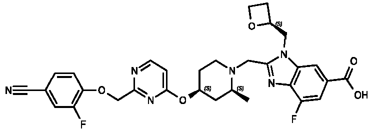 | 2-({4-[(2-{[4-fluoro-2-(trifluoromethyl)phenoxy]methyl}pyridin-4-yl)oxy]piperidin-1-yl}methyl)-1-{[(2S)-oxetan-2-yl]methyl}-1H-1,3-benzodiazole-6-carboxylic acid |
| 324 | 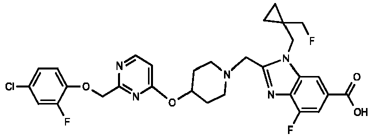 | 2-{[(2S,4S)-4-({2-[(4-cyano-2-fluorophenoxy)methyl]pyrimidin-4-yl}oxy)-2-methylpiperidin-1-yl]methyl }-4-fluoro-1-{[(2S)-oxetan-2-yl]methyl}-1H-1,3-benzodiazole-6-carboxylic acid |
| 325 | 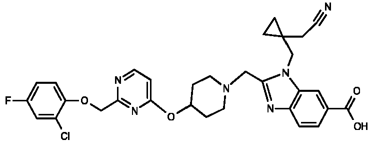 | 2-{[4-({2-[(4-chloro-2-fluorophenoxy)methyl]pyrimidin-4-yl}oxy)piperidin-1-yl]methyl}-4-fluoro-1-{[1-(fluoromethyl)cyclopropyl methyl} -1H-1,3-benzodiazole-6-carboxylic acid |
| 326 | 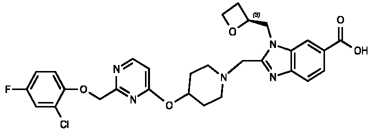 | 2-{[4-({2-[(2-chloro-4-fluorophenoxy)methyl]pyrimidin-4-yl}oxy)piperidin-1-yl]methyl}-1-{[1-(cyanomethyl)cyclopropyl]methyl}-1H-1,3-benzodiazole-6-carboxylic acid |
| 327 | 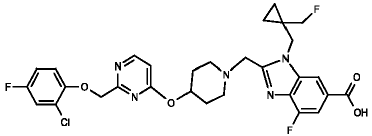 | 2-{[4-({2-[(2-chloro-4-fluorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 328 | 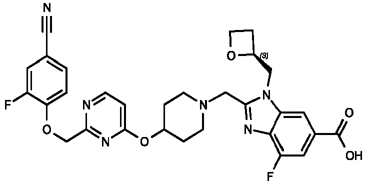 | 2-{[4-({2-[(2-chloro-4-fluorophenoxy)methyl]pyrimidin-4-yl}oxy)piperidin-1-yl]methyl}-4-fluoro-1-{[1-(fluoromethyl)cyclopropyl]methyl}-1H-1,3-benzodiazole-6-carboxylic acid |
| 329 | 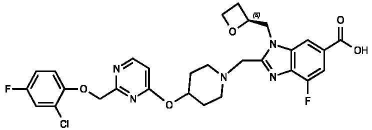 | 2-{[4-({2-[(4-cyano-2-fluorophenoxy)methyl]pyrimidin-4-yl}oxy)piperidin-1-yl]methyl}-4-fluoro-1-{[(2S)-oxetan-2-yl]methyl}-1H-1,3-benzodiazole-6-carboxylic acid |
| 330 | 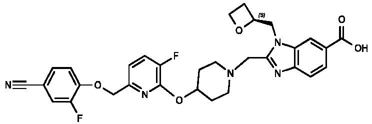 | (S)-2-((4-((6-((4-Cyano-2-fluorophenoxy)methyl)-3-fluoropyridin-2-yl)oxy)piperidin-1-yl)methyl)-1-(oxetan-2-ylmethyl)-1H-benzo[d]imidazole-6-carboxylic acid |
| 331 | 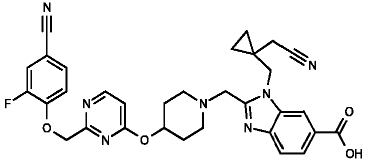 | 2-{[4-({6-[(4-cyano-2-fluorophenoxy)methyl]-3-fluoropyridin-2-yl}oxy)piperidin-1-yl]methyl}-1-{[(2S)-oxetan-2-yl]methyl}-1H-1,3-benzodiazole-6-carboxylic acid |
| 333 | 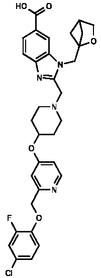 | 2-{[(3S)-3-{3-[(4-cyano-2-fluorophenoxy)methyl]phenoxy}pyrrolidin-1-yl]methyl}-4-fluoro-1-{[(2S)-oxetan-2-yl]methyl}-1H-1,3-benzodiazole-6-carboxylic acid |
| 335 | 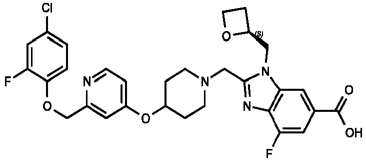 | 2-{[(3S)-3-{3-[(4-chloro-2-fluorophenoxy)methyl]phenoxy}pyrrolidin-1-yl]methyl}-1-{[(2S)-oxetan-2-yl]methyl}-1H-1,3-benzodiazole-6-carboxylic acid |
| 336 | 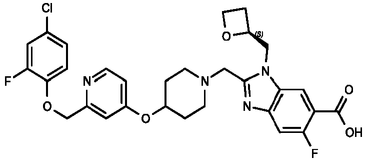 | 2-{[4-({2-[(4-chloro-2-fluorophenoxy)methyl]pyridin-4-yl}oxy)piperidin-1-yl]methyl}-4-fluoro-1-{[(2S)-oxetan-2-yl]methyl}-1H-1,3-benzodiazole-6-carboxylic acid |
| 337 | 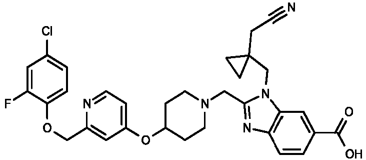 | 2-{[4-({2-[(4-chloro-2-fluorophenoxy)methyl]pyridin-4-yl}oxy)piperidin-1-yl]methyl}-5-fluoro-1-{[(2S)-oxetan-2-yl]methyl}-1H-1,3-benzodiazole-6-carboxylic acid |
| 338 | 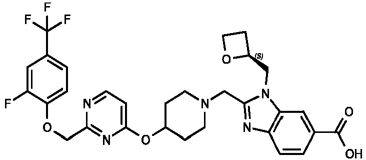 | 2-{[4-({2-[(4-chloro-2-fluorophenoxy)methyl]pyridin-4-yl}oxy)piperidin-1-yl]methyl}-1-{[1-(cyanomethyl)cyclopropyl]methyl}-1H-1,3-benzodiazole-6-carboxylic acid |
| 339 | 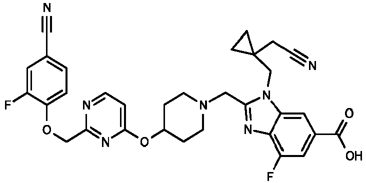 | 2-({4-[(2-{[2-fluoro-4-(trifluoromethyl)phenoxy methyl } pyrimidin -4-yl)oxy]piperidin-1-yl}methyl)-1-{[(2S)-oxetan-2-yl]methyl}-1H-1,3-benzodiazole-6-carboxylic acid |
| 340 | 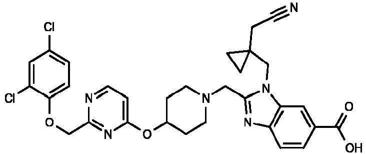 | 2-{[4-({2-[(4-cyano-2-fluorophenoxy)methyl]pyrimidin-4-yl}oxy)piperidin-1-yl]methyl}-1-{[1-(cyanomethyl)cyclopropyl]methyl}-4-fluoro-1H-1,3-benzodiazole-6-carboxylic acid |
| 341 | 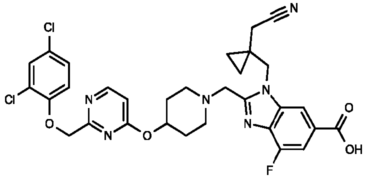 | 1-{[1-(cyanomethyl)cyclopropyl]methyl}-2-{[4-({2-[(2,4-dichlorophenoxy)methyl]pyrimidin-4-yl}oxy)piperidin-1-yl]methyl}-1H-1,3-benzodiazole-6-carboxylic acid |
| 342 | 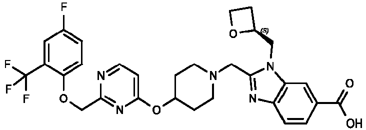 | 1-{[1-(cyanomethyl)cyclopropyl]methyl}-2-{[4-({2-[(2,4-dichlorophenoxy)methyl]pyrimidin-4-yl}oxy)piperidin-1-yl]methyl }-4-fluoro-1H-1,3-benzodiazole-6-carboxylic acid |
| 343 | 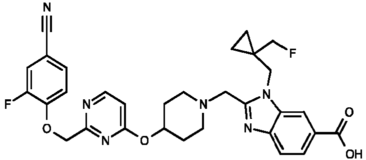 | 2-({4-[(2-{[4-fluoro-2-(trifluoromethyl)phenoxy methyl } pyrimidin -4-yl)oxy]piperidin-1-yl}methyl)-1-{[(2S)-oxetan-2-yl]methyl}-1H-1,3-benzodiazole-6-carboxylic acid |
| 344 | 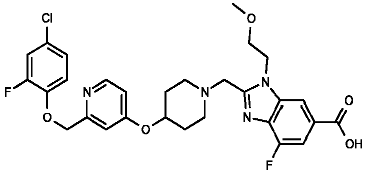 | 2-{[4-({2-[(4-cyano-2-fluorophenoxy)methyl]pyrimidin-4-yl}oxy)piperidin-1-yl]methyl}-1-{[1-(fluoromethyl)cyclopropyl]methyl}-1H-1,3-benzodiazole-6-carboxylic acid |
| 345 | 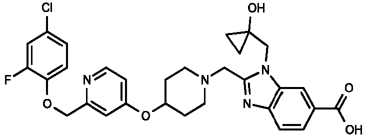 | 2-{[4-({2-[(4-chloro-2-fluorophenoxy)methyl]pyridin-4-yl}oxy)piperidin-1-yl]methyl}-4-fluoro-1-(2-methoxyethyl)-1H-1,3-benzodiazole-6-carboxylic acid |
| 346 | 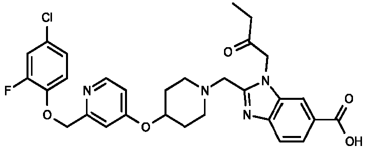 | 2-{[4-({2-[(4-chloro-2-fluorophenoxy)methyl]pyridin-4-yl}oxy)piperidin-1-yl]methyl}-1-[(1-hydroxycyclopropyl)methyl]-1H-1,3-benzodiazole-6-carboxylic acid |
| 347 | 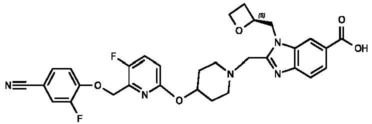 | 2-{[4-({2-[(4-chloro-2-fluorophenoxy)methyl]pyridin-4-yl}oxy)piperidin-1-yl]methyl}-1-(2-oxobutyl)-1H-1,3-benzodiazole-6-carboxylic acid |
| 348 | 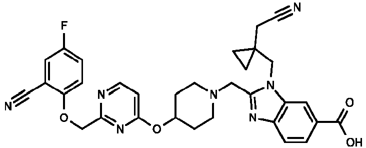 | 2-{[4-({6-[(4-cyano-2-fluorophenoxy)methyl]-5-fluoropyridin-2-yl}oxy)piperidin-1-yl]methyl}-1-{[(2S)-oxetan-2-yl]methyl}-1H-1,3-benzodiazole-6-carboxylic acid |
| 349 | 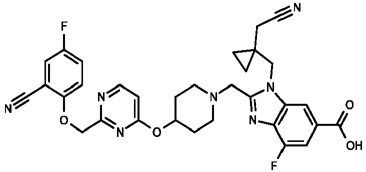 | 2-{[4-({2-[(2-cyano-4-fluorophenoxy)methyl]pyrimidin-4-yl}oxy)piperidin-1-yl]methyl}-1-{[1-(cyanomethyl)cyclopropyl]methyl}-1H-1,3-benzodiazole-6-carboxylic acid |
| 352 | 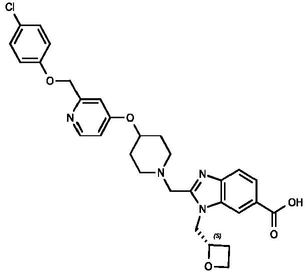 | 2-{[(3S)-3-{3-[(4-chloro-2-fluorophenoxy)methyl]phenoxy}pyrrolidin-1-yl]methyl}-4-fluoro-1-{[(2S)-oxetan-2-yl]methyl}-1H-1,3-benzodiazole-6-carboxylic acid |
| 353 | 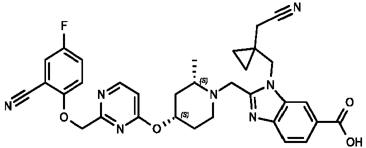 | 2-{[4-({2-[(4-chlorophenoxy)methyl]pyridin-4-yl}oxy)piperidin-1-yl]methyl}-1-{[(2S)-oxetan-2-yl]methyl}-1H-1,3-benzodiazole-6-carboxylic acid |
| 354 | 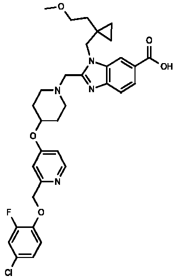 | 2-{[(2S,4S)-4-({2-[(2-cyano-4-fluorophenoxy)methyl]pyrimidin-4-yl}oxy)-2-methylpiperidin-1-yl]methyl }-1-{[1-(cyanomethyl)cyclopropyl]methyl}-1H-1,3-benzodiazole-6-carboxylic acid |
| 355 | 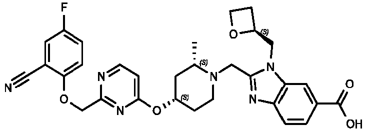 | 2-(((2S,4S)-4-((2-((2-cyano-4-fluorophenoxy)methyl)pyrimidin-4-yl)oxy)-2-methylpiperidin-1-yl)methyl)-1-(((S)-oxetan-2-yl)methyl)-1H-benzo[d]imidazole-6-carboxylic acid |
| 356 | 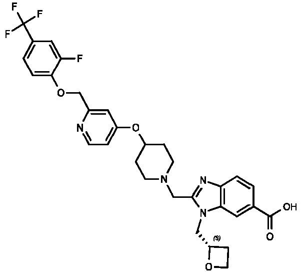 | 2-{[(2S,4S)-4-({2-[(2-cyano-4-fluorophenoxy)methyl]pyrimidin-4-yl}oxy)-2-methylpiperidin-1-yl]methyl}-1-{[(2S)-oxetan-2-yl]methyl}-1H-1,3-benzodiazole-6-carboxylic acid |
| 357 | 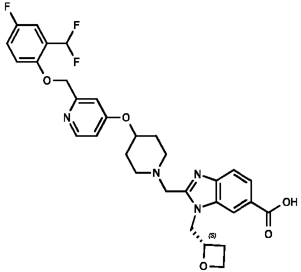 | 2-({4-[(2-{[2-fluoro-4-(trifluoromethyl)phenoxy]methyl}pyridin-4-yl)oxy]piperidin-1-yl}methyl)-1-{[(2S)-oxetan-2-yl]methyl}-1H-1,3-benzodiazole-6-carboxylic acid |
| 358 | 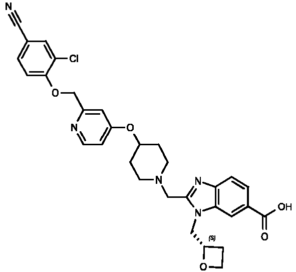 | 2-({4-[(2-{[2-(difluoromethyl)-4-fluorophenoxy]methyl}pyridin-4-yl)oxy]piperidin-1-yl}methyl)-1-{[(2S)-oxetan-2-yl]methyl}-1H-1,3-benzodiazole-6-carboxylic acid |
| 359 | 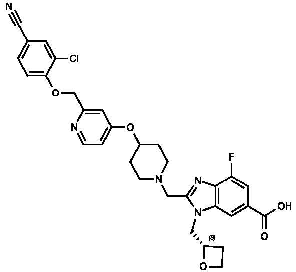 | 2-{[4-({2-[(2-chloro-4-cyanophenoxy)methyl]pyridin-4-yl}oxy)piperidin-1-yl]methyl}-1-{[(2S)-oxetan-2-yl]methyl}-1H-1,3-benzodiazole-6-carboxylic acid |
| 362 | 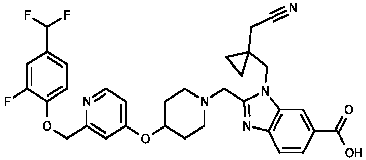 | 2-{[(3S)-3-({2-[(4-chloro-2-fluorophenoxy)methyl]pyrimidin-4-yl}oxy)pyrrolidin-1-yl]methyl}-4-fluoro-1-[(oxetan-2-yl)methyl]-1H-1,3 -benzodiazole-6-carboxylic acid |
| 363 | 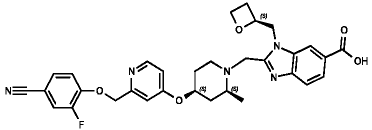 | 1-{[1-(cyanomethyl)cyclopropyl]methyl}-2-({4-[(2-{[4-(difluoromethyl)-2-fluorophenoxy]methyl}pyridin-4-yl)oxy]piperidin-1-yl}methyl)-1H-1,3-benzodiazole-6-carboxylic acid |
| 364 | 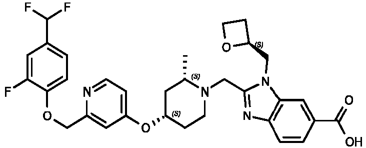 | 2-{[(2S,4S)-4-({2-[(4-cyano-2-fluorophenoxy)methyl]pyridin-4-yl}oxy)-2-methylpiperidin-1-yl]methyl}-1-{[(2S)-oxetan-2-yl]methyl}-1H-1,3-benzodiazole-6-carboxylic acid |
| 365 | 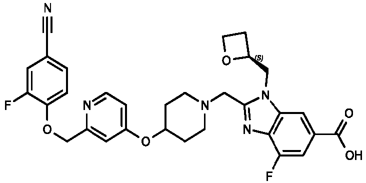 | 2-{[(2S,4S)-4-[(2-{[4-(difluoromethyl)-2-fluorophenoxy]methyl}pyridin-4-yl)oxy]-2-methylpiperidin-1-yl]methyl}-1-{[(2S)-oxetan-2-yl]methyl}-1H-1,3-benzodiazole-6-carboxylic acid |
| 366 | 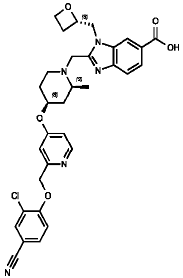 | 2-{[4-({2-[(4-cyano-2-fluorophenoxy)methyl]pyridin-4-yl}oxy)piperidin-1-yl]methyl}-4-fluoro-1-{[(2S)-oxetan-2-yl]methyl}-1H-1,3-benzodiazole-6-carboxylic acid |
| 367 | 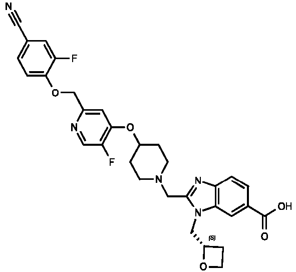 | (S)-2-((4-((2-((4-Cyano-2-fluorophenoxy)methyl)-5-fluoropyridin-4-yl)oxy)piperidin-1-yl)methyl)-1-(oxetan-2-ylmethyl)-1H-benzo[d]imidazole-6-carboxylic acid |
| 368 | 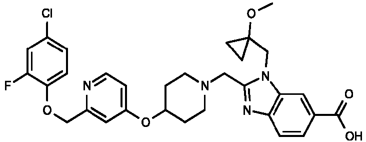 | 2-((4-((2-((4-Chloro-2-fluorophenoxy)methyl)pyridin-4-yl)oxy)piperidin-1-yl)methyl)-1-((1-methoxycyclopropyl)methyl)-1H-benzo[d]imidazole-6-carboxylic acid |
| 369 | 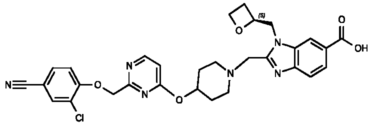 | 2-{[4-({2-[(4-chloro-2-fluorophenoxy)methyl]pyridin-4-yl}oxy)piperidin-1-yl]methyl}-1-[(1-methoxycyclopropyl)methyl]-1H-1,3-benzodiazole-6-carboxylic acid |
| 370 | 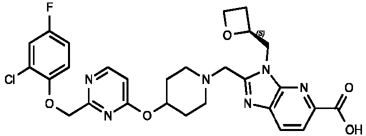 | 2-{[4-({2-[(2-chloro-4-cyanophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 371 | 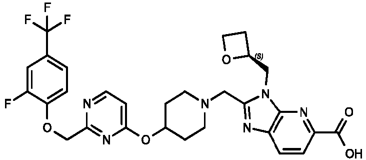 | 2-{[4-({2-[(2-chloro-4-fluorophenoxy)methyl]pyrimidin-4-yl}oxy)piperidin-1-yl]methyl}-3-{[(2S)-oxetan-2-yl]methyl}-3H-imidazo[4,5-b]pyridine-5-carboxylic acid |
| 372 | 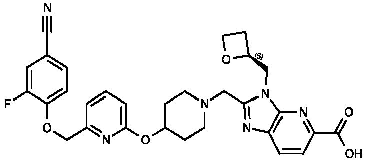 | (S)-2-((4-((6-((4-Cyano-2-fluorophenoxy)methyl)pyridin-2-yl)oxy)piperidin-1-yl)methyl)-3-(oxetan-2-ylmethyl)-3H-imidazo[4,5-b]pyridine-5-carboxylic acid |
| 373 | 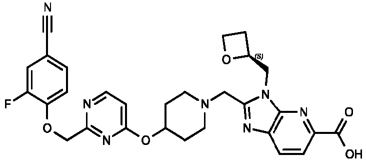 | 2-{[4-({6-[(4-cyano-2-fluorophenoxy)methyl]pyridin-2-yl}oxy)piperidin-1-yl]methyl}-3-{[(2S)-oxetan-2-yl]methyl}-3H-imidazo[4,5-b]pyridine-5-carboxylic acid |
| 374 | 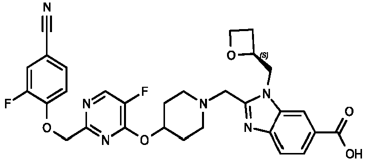 | 2-{[4-({2-[(4-cyano-2-fluorophenoxy)methyl]pyrimidin-4-yl}oxy)piperidin-1-yl]methyl}-3-{[(2S)-oxetan-2-yl]methyl}-3H-imidazo[4,5-b]pyridine-5-carboxylic acid |
| 375 | 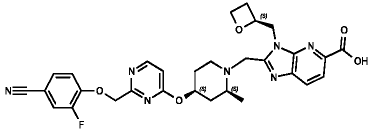 | 2-{[4-({2-[(4-cyano-2-fluorophenoxy)methyl]-5-fluoropyrimidin-4-yl}oxy)piperidin-1-yl]methyl}-1-{[(2S)-oxetan-2-yl]methyl}-1H-1,3-benzodiazole-6-carboxylic acid |
| 376 | 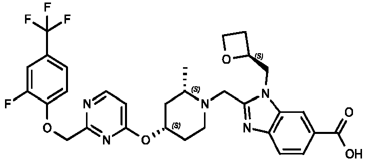 | 2-{[(2S,4S)-4-({2-[(4-cyano-2-fluorophenoxy)methyl]pyrimidin-4-yl}oxy)-2-methylpiperidin-1-yl]methyl}-3-{[(2S)-oxetan-2-yl]methyl}-3H-imidazo[4,5-b]pyridine-5-carboxylic acid |
| 377 | 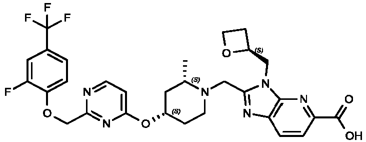 | 2-{[4-({2-[(4-cyano-2-fluorophenoxy)methyl]pyrimidin-4-yl}oxy)-2-fluorophenyl]methyl}-1-[(1-ethyl-1H-imidazol-5-yl)methyl]-1H-1,3-benzodiazole-6-carboxylic acid |
| 378 | 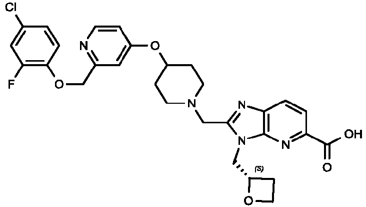 | 2-{[4-({6-[(4-cyano-2-fluorophenoxy)methyl]pyridin-2-yl}oxy)-2-fluorophenyl]methyl}-1-[(1-ethyl-1H-imidazol-5-yl)methyl]-1H-1,3-benzodiazole-6-carboxylic acid |
| 379 | 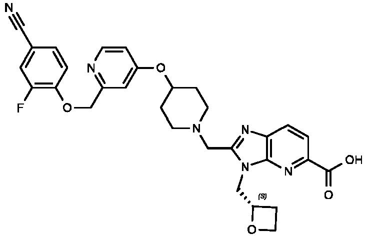 | 2-{[4-({2-[(4-chloro-2-fluorophenoxy)methyl]pyridin-4-yl}oxy)-2-fluorophenyl]methyl}-1-[(1-ethyl-1H-imidazol-5-yl)methyl]-1H-1,3-benzodiazole-6-carboxylic acid |
| 380 | 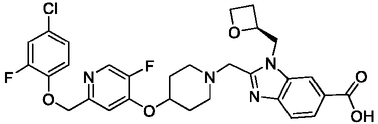 | (S)-2-((4-((2-((4-chloro-2-fluorophenoxy)methyl)-5-fluoropyridin-4-yl)oxy)piperidin-1-yl)methyl)-1-(oxetan-2-ylmethyl)-1H-benzo[d]imidazole-6-carboxylic acid |
| 381 | 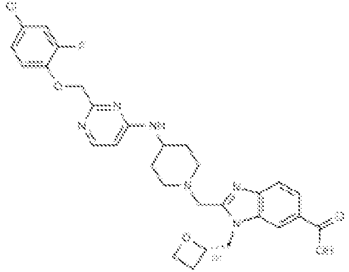 | 2-{[4-({2-[(4-chloro-2-fluorophenoxy)methyl]pyrimidin-4-yl}amino)piperidin-1-yl]methyl}-1-{[(2S)-oxetan-2-yl]methyl}-1H-1,3-benzodiazole-6-carboxylic acid |
| 382 | 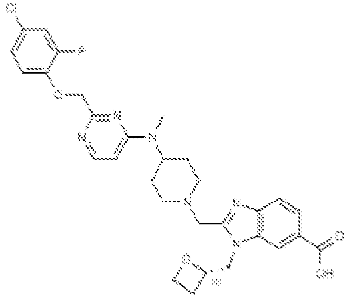 | 2-{[4-({2-[(4-chloro-2-fluorophenoxy)methyl]pyrimidin-4-yl}(methyl)amino)piperidin-1-yl]methyl}-1-{[(2S)-oxetan-2-yl]methyl}-1H-1,3-benzodiazole-6-carboxylic acid |
| 475 | 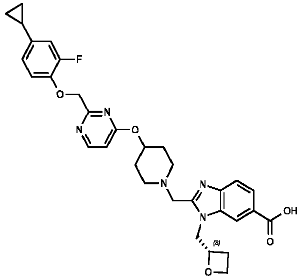 | 2-{[4-({2-[(4-cyclopropyl-2-fluorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 476 | 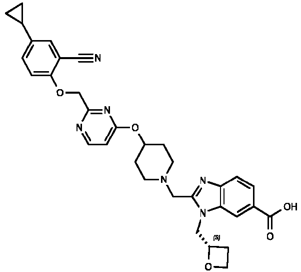 | 2-{[4-({2-[(2-cyano-4-cyclopropylphenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 477 | 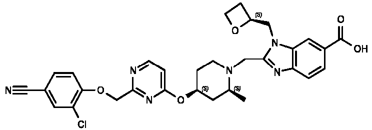 | 2-{[(2S,4S)-4-({2-[(2-chloro-4-cyanophenoxy)methyl]pyrimidin-4-yl}oxy)-2-methylpiperidin-1-yl]methyl}-1-{[(2S)-oxctan-2-yl]methyl}-1H-1,3-benzodiazole-6-carboxylic acid |
| 478 | 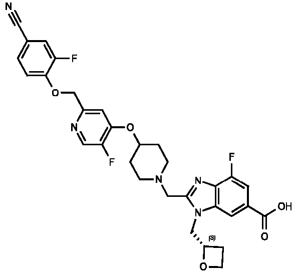 | 2-{[4-({2-[(4-cyano-2-fluorophenoxy)methyl]-5-fluoropyridin-4-yl}oxy)piperidin-1-yl]methyl}-4-fluoro-1-{[(2S)-oxetan-2-yl]methyl}-1H-1,3-benzodiazole-6-carboxylic acid |
| 479 | 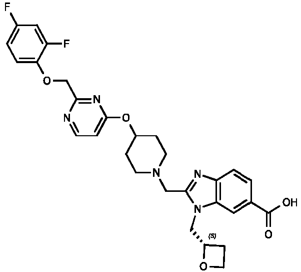 | 2-{[4-({2-[(2,4-difluorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 480 | 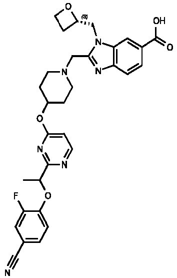 | 2-{[4-({2-[1-(4-cyano-2-fluorophenoxy)ethyl]pyrimidin-4-yl}oxy)piperidin-1-yl]methyl}-1-{[(2S)-oxetan-2-yl]methyl}-1H-1,3-benzodiazole-6-carboxylic acid |
| 481 | 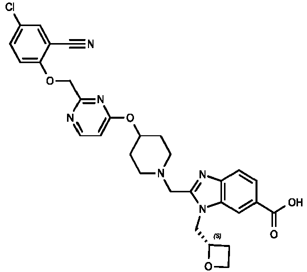 | 2-{[4-({2-[(4-chloro-2-cyanophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 482 | 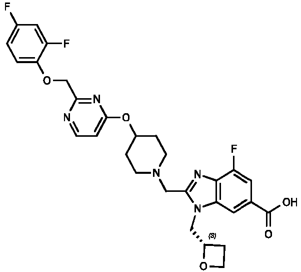 | 2-{[4-({2-[(24-difluorophenoxy)methyl]pyrimidin-4-yl}oxy)piperidin-1-yl]methyl}-4-fluoro-1-{[(2S)-oxetan-2-yl]methyl}-1H-1,3-benzodiazole-6-carboxylic acid |
| 483 | 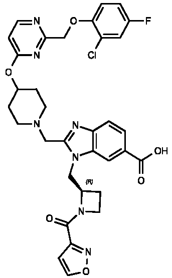 | 2-{[4-({2-[(2-chloro-4-fluorophenoxy)methyl]pyrimidin-4-yl}oxy)piperidin-1-yl]methyl}-1-{[(2R)-1-(1,2-oxazole-3-carbonyl)azetidin-2-yl]methyl}-1H-1,3-benzodiazole-6-carboxylic acid |
| 484 | 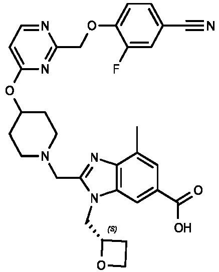 | 2-{[4-({2-[(4-cyano-2-fluorophenoxy)methyl]pyrimidin-4-yl}oxy)piperidin-1-yl]methyl }-4-methyl-1-{[(2S)-oxetan-2-yl]methyl}-1H-1,3-benzodiazole-6-carboxylic acid |
| 485 | 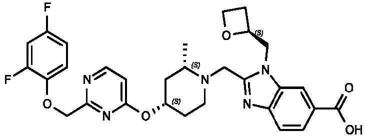 | 2-{[(2S,4S)-4-({2-[(2,4-difluorophenoxy)methyl]pyrimidin-4-yl}oxy)-2-methylpiperidin-1-yl]methyl }-1-{[(2S)-oxetan-2-yl]methyl}-1H-1,3-benzodiazole-6-carboxylic acid |
| 486 | 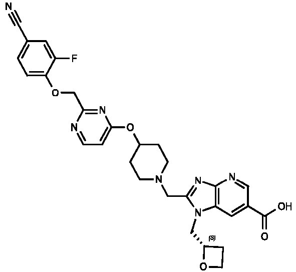 | 2-{[4-({2-[(4-cyano-2-fluorophenoxy)methyl]pyrimidin-4-yl}oxy)piperidin-1-yl]methyl}-1-{[(2S)-oxetan-2-yl]methyl}-1H-imidazo[4,5-b]pyridine-6-carboxylic acid |
| 487 | 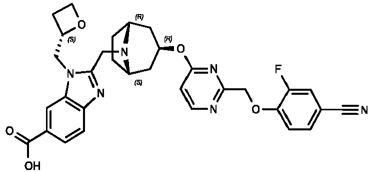 | 2-{[(1R,3S,5S)-3-({2[(4-cyano-2-fluorophenoxy)methyl]pyrimidin-4-yl}oxy)-8-azabicyclo[3.2.1]octan-8-yl]methyl}-1-{[(2S)-oxetan-2-yl]methyl}-1H-1,3-benzodiazole-6-carboxylic acid |
| 488 | 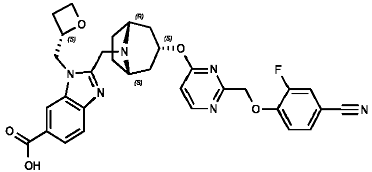 | 2-{[(1R,3R,5S)-3-({2-[(4-cyano-2-fluorophenoxy)methyl]pyrimidin-4-yl}oxy)-8-azabicyclo[3.2.1]octan-8-yl]methyl}-1-{[(2S)-oxetan-2-yl]metlryl}-1H-1,3-benzodiazole-6-carboxylic acid |
| 489 | 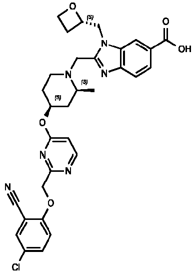 | 2-{[(2S,4S)-4-({2-[(4-chloro-2-cyanophenoxy)methyl]pyrimidin-4-yl}oxy)-2-methylpiperidin-1-yl]methyl}-1-{[(2S)-oxetan-2-yl]methyl}-1H-1,3-benzodiazole-6-carboxylic acid |
| 490 | 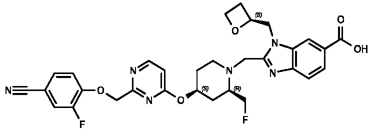 | 2-1[(2R,4S)-4-(12-[(4-cyano-2-fluorophenoxy)methyl]pyrimidin-4-yl}oxy)-2-(fluoromethyl)piperidin-1-yl]methyl}-1-{[(2S)-oxetan-2-yl]methyl}-1H-1,3-benzodiazole-6-carboxylic acid |
| 491 | 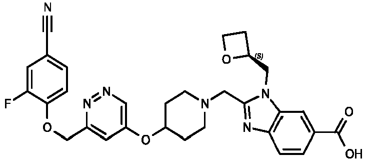 | 2-{[4-({6-[(4-cyano-2-fluorophenoxy)methyl]pyridazin-4-yl}oxy)piperidin-1-yl]methyl}-1-{[(2S)-oxetan-2-yl]methyl}-1H-1,3-benzodiazole-6-carboxylic acid |
| 492 | 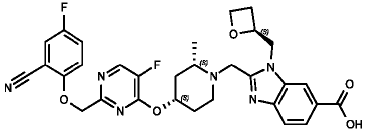 | 2-{[(2S,4S)-4-({2-[(2-cyano-4-fluorophenoxy)methyl]-5-fluoropyrimidin-4-yl}oxy)-2-methylpiperidin-1-yl]methyl}-1-{[(2S)-oxetan-2-yl]methyl}-1H-1,3-benzodiazole-6-carboxylic acid |
| 493 | 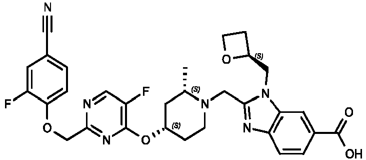 | 2-{[(2S,4S)-4-({2-[(4-cyano-2-fluorophenoxy)methyl]-5-fluoropyrimidin-4-yl}oxy)-2-methylpiperidin-1-yl]methyl}-1-{[(2S)-oxetan-2-yl]methyl}-1H-1,3-benzodiazole-6-carboxylic acid |
| 494 | 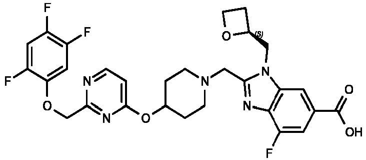 | 4-fluoro-1-{[(2S)-oxetan-2-yl]methyl}-2-{[4-({2-[(2,4,5-trifluorophenoxy)methyl]pyrimidin-4-yl}oxy)piperidin-1-yl]methyl}-1H-1,3-benzodiazole-6-carboxylic acid |
| 495 | 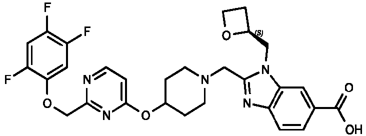 | 1-{[(2S)-oxetan-2-yl]methyl}-2-{[4-({2-[(2,4,5-trifluorophenoxy)methyl]pyrimidin-4⁻yl}oxy)piperidin-1-yl]methyl}-1H-1,3-benzodiazole-6-carboxylic acid |
| 496 | 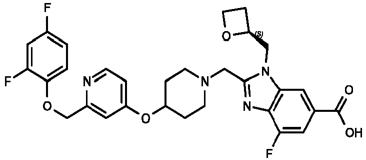 | 2-{[4-({2-[(2,4-difluorophenoxy)methyl]pyridin-4-yl}oxy)piperidin-1-yl]methyl}-4-fluoro-1-{[(2S)-oxetan-2-yl]methyl}-1H-1,3-benzodiazole-6-carboxylic acid |
| 497 | 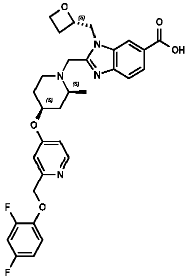 | 2-{[(2S,4S)-4-({2-[(2,4-difluorophenoxy)methyl]pyridin-4-yl}oxy)-2-methylpiperidin-1-yl]methyl}-1-{[(2S)-oxetan-2-yl]methyl}-1H-1,3-benzodiazole-6-carboxylic acid |
| 498 | 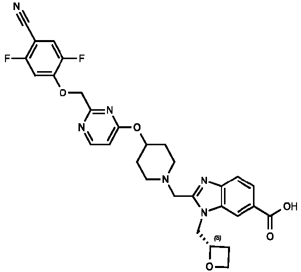 | 2-{[4-({2-[(4-cyano-2,5-difluorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 499 | 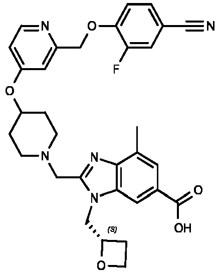 | 2-{[4-({2-[(4-cyano-2-fluorophenoxy)methyl]pyridin-4-yl}oxy)piperidin-1-yl]methyl}-4-methyl-1-{[(2S)-oxetan-2-yl\|methyl}-1H-1,3-benzodiazole-6-carboxylic acid |
| 500 | 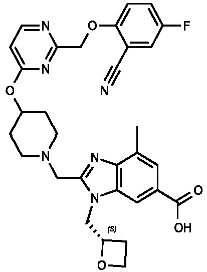 | 2-{[4-({2-[(2-cyano-4-fluorophenoxy)methyl]pyrimidin-4-yl}oxy)piperidin-1-yl]methyl}-4-methyl-1-{[(2S)-oxetan-2-yl]methyl}-1H-1,3-benzodiazole-6-carboxylic acid |
| 501 | 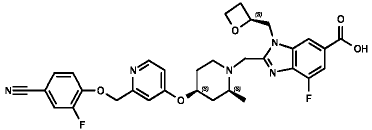 | 2-{[(2S,4S)-4-({2-[(4-cyano-2-fluorophenoxy)methyl]pyridin-4-yl}oxy)-2-methylpiperidin-1-yl]methyl}-4-fluoro-1-{[(2S)-oxetan-2-yl]methyl}-1H-1,3-benzodiazole-6-carboxylic acid |
| 502 | 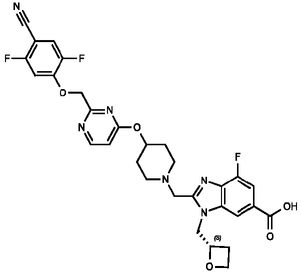 | 2-{[4-({2-[(4-cyano-2,5-difluorophenoxy)methyl]pyrimidin-4-yl}oxy)piperidin-1-yljmethyl}-4-fluoro-1-{[(2S)-oxetan-2-yl]methyl}-1H-1,3-benzodiazole-6-carboxylic acid |
| 503 | 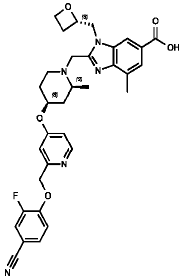 | 2-{[(2S,4S)-4-({2-[(4-cyano-2-fluorophenoxy)methyl]pyridin-4-yl}oxy)-2-methylpiperidin-1-yl]methyl}-4-methyl-1-{[(2S)-oxetan-2-yl]methyl}-1H-1,3-benzodiazole-6-carboxylic acid |
| 504 | 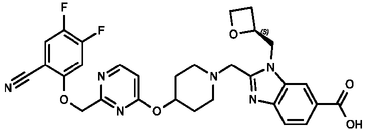 | 2-{[4-({2-[(2-cyano-4,5-difluorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 505 | 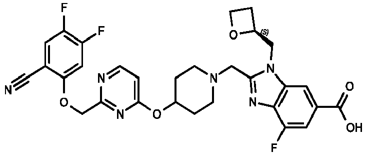 | 2-{[4-({2-[(2-cyano-4,5-difluorophenoxy)methyl]pyrimidin-4-yl}oxy)piperidin-1-yl]methyl}-4-fluoro-1-{[(2S)-oxetan-2-yl]methyl}-1H-1,3-benzodiazole-6-carboxylic acid |
| 506 | 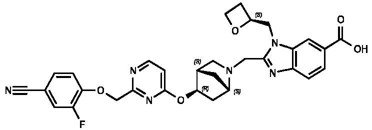 | 2-{[(1S,4S,5R)-5-({2-[(4-cyano-2-fluorophenoxy)methyl]pyrimidin-4-yl}oxy)-2-azabicyclo[2.2.1]heptan-2-yl]methyl}-1-{[(2S)-oxetan-2-yl]methyl}-1H-1,3-benzodiazole-6-carboxylic acid |
| 507 | 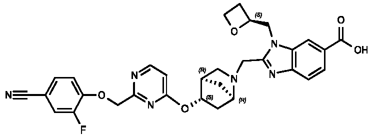 | 2-{[(1R,4R,5S)-5-({2-[(4-cyano-2-fluorophenoxy)methyl]pyrimidin-4-yl}oxy)-2-azabicyclo[2.2.1]heptan-2-yl]methyl}-1-{[(2S)-oxetan-2-yl]methyl}-1H-1,3-benzodiazole-6-carboxylic acid |
| 508 | 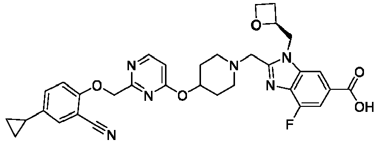 | (S)-2-((4-((2-((2-cyano-4-cyclopropylphenoxy)methyl)pyrimidin-4-yl)oxy)piperidin-1-yl)methyl)-4-fluoro-1-(oxetan-2-ylmethyl)-1H-benzo[d]imidazole-6-carboxylic acid |
| 509 | 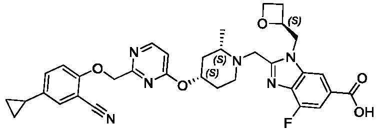 | 2-(((2S,4S)-4-((2-((2-cyano-4-cyclopropylphenoxy)methyl)pyrimidin-4-yl)oxy)-2-methylpiperidin-1-yl)methyl)-4-fluoro-1-(((S)-oxetan-2-yl)methyl)-1H-benzo[d]imidazole-6-carboxylic acid |
| 510 | 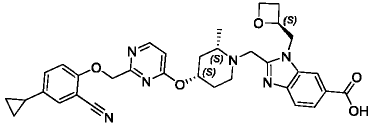 | 2-(((2S,4S)-4-((2-((2-cyano-4-cyclopropylphenoxy)methyl)pyrimidin-4-yl)oxy)-2-methylpiperidin-1-yl)methyl)-1-(((S)-oxetan-2-yl)methyl)-1H-benzo[d]imidazole-6-carboxylic acid |
| 511 | 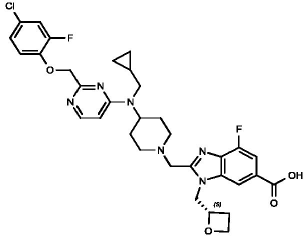 | 2-{[4-({2-[(4-chloro-2-fluorophenoxy)methyl]pyrimidin-4-yl}(cyclopropylmethyl)amino)piperidin-1-yl]methyl}-4-fluoro-1-{[(2S)-oxetan-2-yl]methyl}-1H-1,3-benzodiazole-6-carboxylic acid |
| 512 | 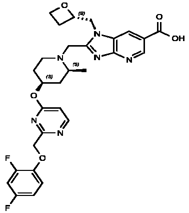 | 2-{[(2S,4S)-4-({2-[(2,4-difluorophenoxy)methyl]pyrimidin-4-yl}oxy)-2-methylpiperidin-1-yl]methyl}-1-{[(2S)-oxetan-2-yl]methyl}-1H-imidazo[4,5-b]pyridine-6-carboxylic acid |
| 513 | 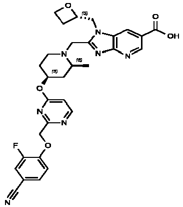 | 2-{[(2S,4S)-4-({2-[(4-cyano-2-fluorophenoxy)methyl]pyrimidin-4-yl}oxy)-2-methylpiperidin-1-yl]methyl }-1-{[(2S)-oxetan-2-yl]methyl}-1H-imidazo[4,5-b]pyridine-6-carboxylic acid |
| 514 | 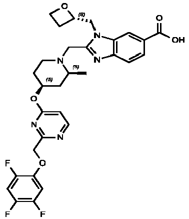 | 2-{[(2S,4S)-2-methyl-4-({2-[(2,4,5-trifluorophenoxy)methyl]pyrimidin-4-yl}oxy)piperidin-1-yl]methyl}-1-{[(2S)-oxetan-2-yl]methyl}-1H-1,3-benzodiazole-6-carboxylic acid |
| 515 | 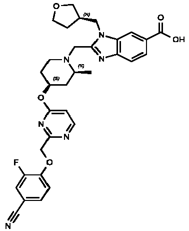 | 2-{[(2S,4S)-4-({2-[(4-cyano-2-fluorophenoxy)methyl]pyrimidin-4-yl}oxy)-2-methylpiperidin-1-yl]methyl}-1-{[(3R)-oxolan-3-yl]methyl}-1H-1,3-benzodiazole-6-carboxylic acid |

### Optically Active Compounds

In certain instances, compounds provided herein may have several chiral centers and may exist in and be isolated in optically active and racemic forms. In certain instances, some compounds may exhibit polymorphism. A person of skill in the art will appreciate that compounds provided herein can exist in any racemic, optically-active, diastereomeric, polymorphic, or stereoisomeric form, and/or mixtures thereof. A person of skill in the art will also appreciate that such compounds described herein that possess the useful properties also described herein is within the scope of this disclosure. A person of skill in the art will further appreciate how to prepare optically active forms of the compounds described herein, for example, by resolution of racemic forms via recrystallization techniques, by synthesis from optically-active starting materials, by chiral synthesis, or by chromatographic separation using a chiral stationary phase. In addition, most amino acids are chiral (i.e., designated as L- or D-, wherein the L- enantiomer is the naturally occurring configuration) and can exist as separate enantiomers.

Examples of methods to obtain optically active materials are known in the art, and include at least the following:
i) physical separation of crystals - a technique whereby macroscopic crystals of the individual enantiomers are manually separated. This technique can be used if crystals of the separate enantiomers exist (i.e., the material is a conglomerate, and the crystals are visually distinct);
ii) simultaneous crystallization - a technique whereby the individual enantiomers are separately crystallized from a solution of the racemate, only if the latter is a conglomerate in the solid state;
iii) enzymatic resolutions - a technique wherein partial, or complete separation of a racemate is accomplished by virtue of different rates of reaction of the enantiomers in the presence of an enzyme;
iv) enzymatic asymmetric synthesis - a synthetic technique wherein at least one step of the synthesis uses an enzymatic reaction to obtain an enantiomerically pure, or enriched synthetic precursor of the desired enantiomer;
v) chemical asymmetric synthesis - a synthetic technique wherein the desired enantiomer is synthesized from an achiral precursor using chiral catalysts, or chiral auxiliaries to produce asymmetry (i.e., chirality) in the product;
vi) diastereomer separations - a technique wherein a racemic compound is treated with an enantiomerically pure reagent (a chiral auxiliary) that converts the individual enantiomers to diastereomers. The resulting diastereomers are then separated by chromatography, or crystallization by virtue of their now more distinct diastereomeric differences, and then the chiral auxiliary is removed to obtain each enantiomer;
vii) first- and second-order asymmetric transformations - a technique wherein diastereomers of the racemate equilibrate in solution to yield a preponderance of a diastereomer of the desired enantiomer, or where kinetic, or thermodynamic crystallization of the diastereomer of the desired enantiomer perturbs the equilibrium such that eventually in principle all the material is converted to the crystalline diastereomer of the desired enantiomer. The desired enantiomer is then derived from the diastereomer;
viii) kinetic resolutions - this technique refers to the achievement of partial or complete resolution of a racemate (or of a further resolution of a partially resolved compound) by virtue of unequal reaction rates of the enantiomers with a chiral, or non-racemic reagent, or catalyst under kinetic conditions;
ix) enantiospecific synthesis from non-racemic precursors - a synthetic technique wherein the desired enantiomer is obtained from chiral starting materials, and where the stereochemical integrity is not or is only minimally compromised over the course of the synthesis;
x) chiral liquid chromatography - a technique wherein the enantiomers of a racemate are separated in a liquid mobile phase by virtue of their different interactions with a stationary phase. The stationary phase can be made of chiral material, or the mobile phase can contain an additional chiral material to provoke the different interactions;
xi) chiral gas chromatography - a technique wherein the racemate is volatilized and enantiomers are separated by virtue of their different interactions in the gaseous mobile phase with a column containing a fixed non-racemic adsorbent phase;
xii) extraction with chiral solvents - a technique wherein the enantiomers are separated by virtue of kinetic or thermodynamic dissolution of one enantiomer into a particular chiral solvent;
xiii) transport across chiral membranes - a technique wherein a racemate is placed in contact with a thin membrane barrier. The barrier typically separates two miscible fluids, one containing the racemate, and a driving force such as a concentration, or pressure differential causes preferential transport across the membrane barrier. Separation occurs as a result of the non-racemic nature of the membrane which allows only one enantiomer of the racemate to pass through.

In some instances, provided herein are compositions of compounds of any of Formulas (**I**)-(**XVIII**), **(XXV) - (XXVII), (XXX), (XXXI), (XXXIX),** and **(XL-XLVII),** that are substantially free of a designated stereoisomer of that compound. In certain instances, in the methods and compounds of this disclosure, the compounds are substantially free of other stereoisomers. In some instances, the composition includes a compound that is at least 85%, 90%, 95%, 98%, or 99% to 100% by weight of the compound, the remainder comprising other chemical species, or enantiomers. In some instances, provided herein are compositions of compounds of any of Formulas **(I)-(XVIII), (XXV) - (XXVII), (XXX), (XXXI), (XXXIX),** and **(XL-XLVII).**

that are substantially free of a designated enantiomer of that compound. In certain instances, in the methods and compounds of this disclosure, the compounds are substantially free of other enantiomers. In some instances, the composition includes a compound that is at least 85%, 90%, 95%, 98%, or 99% to 100% by weight of the compound, the remainder comprising other chemical species or enantiomers.

### Isotopically Enriched Compounds

Also provided herein are isotopically enriched compounds including, but not limited to, isotopically enriched compounds of any of Formulas **(I)-(XVIII), (XXV) - (XXVII), (XXX), (XXXI), (XXXIX),** and **(XL-XLVII).**

Isotopic enrichment (for example, deuteration) of pharmaceuticals to improve pharmacokinetics ("PK"), pharmacodynamics ("PD"), and/or toxicity profiles, has been previously demonstrated within some classes of drugs. See, for example, Lijinsky et al., Food Cosmet. Toxicol., 20: 393 (1982); Lijinsky et al., J. Nat. Cancer Inst., 69: 1127 (1982); Mangold et al., Mutation Res. 308: 33 (1994); Gordon et al., Drug Metab. Dispos., 15: 589 (1987); Zello et al., Metabolism, 43: 487 (1994); Gately et al., J. Nucl. Med., 27: 388 (1986); Wade D, Chem. Biol. Interact. 117: 191 (1999).

Isotopic enrichment of a drug can be used, for example, to (1) reduce or eliminate unwanted metabolites; (2) increase the half-life of the parent drug; (3) decrease the number of doses needed to achieve a desired effect; (4) decrease the amount of a dose necessary to achieve a desired effect; (5) increase the formation of active metabolites if any are formed; and/or (6) decrease the production of deleterious metabolites in specific tissues. Isotopic enrichment of a drug can also be used to create a more effective and/or safer drug for combination therapy, whether the combination therapy is intentional or not.

Replacement of an atom for one of its isotopes often will result in a change in the reaction rate of a chemical reaction. This phenomenon is known as the Kinetic Isotope Effect ("KIE"). For example, if a C-H bond is broken during a rate-determining step in a chemical reaction (i.e., the step with the highest transition state energy), substitution of a (heavier) isotope for that reactive hydrogen will cause a decrease in the reaction rate. The Deuterium Kinetic Isotope Effect ("DKIE") is the most common form of KIE. *(See, e.g.,* Foster et al., Adv. Drug Res., vol. 14, pp. 1-36 (1985); Kushner et al., Can. J. Physiol. Pharmacol., vol. 77, pp. 79-88 (1999)).

The magnitude of the DKIE can be expressed as the ratio between the rates of a given reaction in which a C-H bond is broken, and the same reaction where deuterium is substituted for hydrogen and the C-D bond is broken. The DKIE can range from about one (no isotope effect) to very large numbers, such as 50, or more, meaning that the reaction can be fifty, or more, times slower when deuterium has been substituted for hydrogen.

Substitution of tritium ("T") for hydrogen results in yet a stronger bond than deuterium and gives numerically larger isotope effects. Similarly, substitution of isotopes for other elements including, but not limited to, ¹³C, or ¹⁴C for carbon; ³³S, ³⁴S, or ³⁶S for sulfur; ¹⁵N for nitrogen; and ¹⁷O, or ¹⁸O for oxygen may lead to a similar kinetic isotope effect.

The animal body expresses a variety of enzymes for the purpose of eliminating foreign substances, such as therapeutic agents, from its circulation system. Examples of such enzymes include the cytochrome P450 enzymes ("CYPs"), esterases, proteases, reductases, dehydrogenases, and monoamine oxidases to react with and convert these foreign substances to more polar intermediates, or metabolites for renal excretion. Some of the most common metabolic reactions of pharmaceutical compounds involve the oxidation of a carbon-hydrogen (C-H) bond to either a carbon-oxygen (C-O), or carbon-carbon (C=C) pi-bond. The resultant metabolites may be stable, or unstable under physiological conditions, and can have substantially different PK/PD, and acute, and long-term toxicity profiles relative to the parent compounds. For many drugs, such oxidations are rapid. Therefore, these drugs often require the administration of multiple or high daily doses.

Therefore, isotopic enrichment at certain positions of a compound provided herein will produce a detectable KIE that will affect the pharmacologic, PK, PD, and/or toxicological profiles of a compound provided herein in comparison with a similar compound having a natural isotopic composition.

### Compositions and Uses

### Pharmaceutical Compositions and Methods of Administration

The compounds provided herein can be formulated into pharmaceutical compositions using methods available in the art and those disclosed herein. Any of the compounds provided herein can be provided in the appropriate pharmaceutical composition and be administered by a suitable route of administration. The pharmaceutical composition recited in appended claim 4 comprises 2-{[(2S,4S)-4-({2-[(2,4-difluorophenoxy) methyl]pyrimidin-4-yl}oxy)-2-methylpiperidin-1-yl]methyl}-1- f [(2S)-oxetan-2-yl]methyl}-1H-1,3-benzodiazole-6-carboxylic acid or a pharmaceutically acceptable salt or solvate thereof and one or more pharmaceutically acceptable carriers, excipients, or diluents.

The methods provided herein encompass administering pharmaceutical compositions comprising at least one compound provided herein and one or more compatible and pharmaceutically acceptable carriers. In this context, the term "pharmaceutically acceptable" means approved by a regulatory agency of the Federal, or state government, or listed in the U.S. Pharmacopeia, or other generally recognized pharmacopeia for use in animals, and in certain instances in humans. The term "carrier" includes a diluent, adjuvant (e.g., Freund's adjuvant (complete and incomplete)), excipient, or vehicle with which the therapeutic is administered. Such pharmaceutical carriers can be sterile liquids, such as water and oils including petroleum, animal, vegetable, or oils of synthetic origin, such as peanut oil, soybean oil, mineral oil, sesame oil, and the like. Water can be used as a carrier when the pharmaceutical composition is administered intravenously. Saline solutions, and aqueous dextrose, and glycerol solutions can also be employed as liquid carriers, particularly for injectable solutions. Examples of suitable pharmaceutical carriers are described in Martin, E.W., *Remington's Pharmaceutical Sciences.*

In clinical practice the pharmaceutical compositions, or compounds provided herein may be administered by any route known in the art. Exemplary routes of administration include, but are not limited to, inhalation, intraarterial, intradermal, intramuscular, intraperitoneal, intravenous, nasal, parenteral, pulmonary, and subcutaneous routes. In some instances, a pharmaceutical composition or compound provided herein is administered parenterally.

The compositions for parenteral administration can be emulsions or sterile solutions. Parenteral compositions may include, for example, propylene glycol, polyethylene glycol, vegetable oils, and injectable organic esters (e.g., ethyl oleate). These compositions can also contain wetting, isotonizing, emulsifying, dispersing, and stabilizing agents. Sterilization can be carried out in several ways, for example, using a bacteriological filter, via radiation, or via heating. Parenteral compositions can also be prepared in the form of sterile solid compositions which can be dissolved at the time of use in sterile water, or any other injectable sterile medium.

In some instances, a composition provided herein is a pharmaceutical composition, or a single unit dosage form. Pharmaceutical compositions, and single unit dosage forms provided herein comprise a prophylactically, or therapeutically effective amount of one, or more prophylactic, or therapeutic compounds.

The pharmaceutical composition may comprise one, or more pharmaceutical excipients. Any suitable pharmaceutical excipient may be used, wherein a person of ordinary skill in the art is capable of selecting suitable pharmaceutical excipients. Non-limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like. Whether a particular excipient is suitable for incorporation into a pharmaceutical composition, or dosage form depends on a variety of factors well known in the art including, but not limited to, the way in which the dosage form will be administered to a subject and the specific compound in the dosage form. The composition, or single unit dosage form, if desired, can also contain minor amounts of wetting, or emulsifying agents, or pH buffering agents. Accordingly, the pharmaceutical excipients provided below are intended to be illustrative, and not limiting. Additional pharmaceutical excipients include, for example, those described in the Handbook of Pharmaceutical Excipients, Rowe et al. (Eds.) 6th Ed. (2009).

In some instances, the pharmaceutical composition comprises an anti-foaming agent. Any suitable anti-foaming agent may be used. In some aspects, the anti-foaming agent is selected from an alcohol, an ether, an oil, a wax, a silicone, a surfactant, and combinations thereof. In some aspects, the anti-foaming agent is selected from a mineral oil, a vegetable oil, ethylene bis stearamide, a paraffin wax, an ester wax, a fatty alcohol wax, a long-chain fatty alcohol, a fatty acid soap, a fatty acid ester, a silicon glycol, a fluorosilicone, a polyethylene glycol-polypropylene glycol copolymer, polydimethylsiloxane-silicon dioxide, ether, octyl alcohol, capryl alcohol, sorbitan trioleate, ethyl alcohol, 2-ethyl-hexanol, dimethicone, oleyl alcohol, simethicone, and combinations thereof.

In some instances, the pharmaceutical composition comprises a co-solvent. Illustrative examples of co-solvents include, ethanol, poly(ethylene) glycol, butylene glycol, dimethylacetamide, glycerin, and propylene glycol.

In some instances, the pharmaceutical composition comprises a buffer. Illustrative examples of buffers include, acetate, borate, carbonate, lactate, malate, phosphate, citrate, hydroxide, diethanolamine, monoethanolamine, glycine, methionine, guar gum, and monosodium glutamate.

In some instances, the pharmaceutical composition comprises a carrier, or filler. Illustrative examples of carriers, or fillers include, lactose, maltodextrin, mannitol, sorbitol, chitosan, stearic acid, xanthan gum, and guar gum.

In some instances, the pharmaceutical composition comprises a surfactant. Illustrative examples of surfactants, include d-alpha tocopherol, benzalkonium chloride, benzethonium chloride, cetrimide, cetylpyridinium chloride, docusate sodium, glyceryl behenate, glyceryl monooleate, lauric acid, macrogol 15 hydroxystearate, myristyl alcohol, phospholipids, polyoxyethylene alkyl ethers, polyoxyethylene sorbitan fatty acid esters, polyoxyethylene stearates, polyoxylglycerides, sodium lauryl sulfate, sorbitan esters, and vitamin E polyethylene(glycol) succinate.

In some instances, the pharmaceutical composition comprises an anti-caking agent. Illustrative examples of anti-caking agents include, calcium phosphate (tribasic), hydroxymethyl cellulose, hydroxypropyl cellulose, and magnesium oxide.

Other excipients that may be used with the pharmaceutical compositions include, for example, albumin, antioxidants, antibacterial agents, antifungal agents, bioabsorbable polymers, chelating agents, controlled release agents, diluents, dispersing agents, dissolution enhancers, emulsifying agents, gelling agents, ointment bases, penetration enhancers, preservatives, solubilizing agents, solvents, stabilizing agents, and sugars. Specific examples of each of these agents are described, for example, in the Handbook of Pharmaceutical Excipients, Rowe et al. (Eds.) 6th Ed. (2009), The Pharmaceutical Press.

In some instances, the pharmaceutical composition comprises a solvent. In some aspects, the solvent is saline solution, such as a sterile isotonic saline solution, or dextrose solution. In some aspects, the solvent is water for injection.

In some instances, the pharmaceutical compositions are in a particulate form, such as a microparticle or a nanoparticle. Microparticles, and nanoparticles may be formed from any suitable material, such as a polymer, or a lipid. In some aspects, the microparticles, or nanoparticles are micelles, liposomes, or polymersomes.

Further provided herein are anhydrous pharmaceutical compositions, and dosage forms comprising a compound, since, in some instances, water can facilitate the degradation of some compounds.

Anhydrous pharmaceutical compositions, and dosage forms provided herein can be prepared using anhydrous, or low moisture containing ingredients, and low moisture, or low humidity conditions. Pharmaceutical compositions, and dosage forms that comprise lactose, and at least one active ingredient that comprises a primary, or secondary amine can be anhydrous if substantial contact with moisture, and/or humidity during manufacturing, packaging, and/or storage is expected.

An anhydrous pharmaceutical composition can be prepared and stored such that its anhydrous nature is maintained. Accordingly, anhydrous compositions can be packaged using materials known to prevent exposure to water such that they can be included in suitable formulary kits. Examples of suitable packaging include, but are not limited to, hermetically sealed foils, plastics, unit dose containers (e.g., vials), blister packs, and strip packs.

Lactose-free compositions provided herein can comprise excipients that are well known in the art and are listed, for example, in the U.S. Pharmocopeia (USP) SP (XXI)/NF (XVI). In general, lactose-free compositions comprise an active ingredient, a binder/filler, and a lubricant in pharmaceutically compatible, and pharmaceutically acceptable amounts. Exemplary lactose-free dosage forms comprise an active ingredient, microcrystalline cellulose, pre gelatinized starch, and magnesium stearate.

Also provided are pharmaceutical compositions, and dosage forms that comprise one, or more excipients that reduce the rate by which a compound will decompose. Such excipients, which are referred to herein as "stabilizers," include, but are not limited to, antioxidants such as ascorbic acid, pH buffers, or salt buffers.

### Parenteral Dosage Forms

In certain instances, provided are parenteral dosage forms. Parenteral dosage forms can be administered to subjects by various routes including, but not limited to, subcutaneous, intravenous (including bolus injection), intramuscular, and intraarterial. Because their administration typically bypasses subjects' natural defenses against contaminants, parenteral dosage forms are typically sterile, or capable of being sterilized prior to administration to a subject. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to Water for Injection USP; aqueous vehicles such as, but not limited to, Sodium Chloride Injection, Ringer's Injection, Dextrose Injection, Dextrose, and Sodium Chloride Injection, and Lactated Ringer's Injection; water miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Excipients that increase the solubility of one, or more of the antibodies disclosed herein can also be incorporated into the parenteral dosage forms.

### Dosage and Unit Dosage Forms

In human therapeutics, the doctor will determine the posology which he considers most appropriate according to a preventive, or curative treatment, and according to the age, weight, condition, and other factors specific to the subject to be treated.

In certain instances, a composition provided herein is a pharmaceutical composition, or a single unit dosage form. Pharmaceutical compositions, and single unit dosage forms provided herein comprise a prophylactically, or therapeutically effective amount of one, or more prophylactic, or therapeutic antibodies, or antigen binding fragments thereof.

The amount of the compound, or composition which will be effective in the prevention, or treatment of a disorder, or one, or more symptoms thereof will vary with the nature, and severity of the disease, or condition, and the route by which the compound is administered. The frequency and dosage will also vary according to factors specific for each subject depending on the specific therapy (e.g., therapeutic or prophylactic agents) administered, the severity of the disorder, disease, or condition, the route of administration, as well as age, body, weight, response, and the past medical history of the subject. Effective doses may be extrapolated from dose-response curves derived from *in vitro,* or animal model test systems.

In certain instances, exemplary doses of a composition include milligram, or microgram amounts of the compound per kilogram of subject, or sample weight (e.g., about 10 micrograms per kilogram to about 50 milligrams per kilogram, about 100 micrograms per kilogram to about 25 milligrams per kilogram, or about 100 microgram per kilogram to about 10 milligrams per kilogram). In certain instances, the dosage of the compound provided herein, based on weight of the compound, administered to prevent, treat, manage, or ameliorate a disorder, or one, or more symptoms thereof in a subject is 0.1 mg/kg, 1 mg/kg, 2 mg/kg, 3 mg/kg, 4 mg/kg, 5 mg/kg, 6 mg/kg, 10 mg/kg, or 15 mg/kg or more of a subject's body weight. In another instance, the dosage of the composition, or a composition provided herein administered to prevent, treat, manage, or ameliorate a disorder, or one, or more symptoms thereof in a subject is 0.1 mg to 200 mg, 0.1 mg to 100 mg, 0.1 mg to 50 mg, 0.1 mg to 25 mg, 0.1 mg to 20 mg, 0.1 mg to 15 mg, 0.1 mg to 10 mg, 0.1 mg to 7.5 mg, 0.1 mg to 5 mg, 0.1 to 2.5 mg, 0.25 mg to 20 mg, 0.25 to 15 mg, 0.25 to 12 mg, 0.25 to 10 mg, 0.25 mg to 7.5 mg, 0.25 mg to 5 mg, 0.25 mg to 2.5 mg, 0.5 mg to 20 mg, 0.5 to 15 mg, 0.5 to 12 mg, 0.5 to 10 mg, 0.5 mg to 7.5 mg, 0.5 mg to 5 mg, 0.5 mg to 2.5 mg, 1 mg to 20 mg, 1 mg to 15 mg, 1 mg to 12 mg, 1 mg to 10 mg, 1 mg to 7.5 mg, 1 mg to 5 mg, or 1 mg to 2.5 mg.

The dose can be administered according to a suitable schedule, for example, once, two times, three times, or four times weekly. It may be necessary to use dosages of the compound outside the ranges disclosed herein in some cases, as will be apparent to those of ordinary skill in the art. Furthermore, it is noted that the clinician, or treating physician will know how, and when to interrupt, adjust, or terminate therapy in conjunction with subject response.

Different therapeutically effective amounts may be applicable for different diseases, and conditions, as will be readily known by those of ordinary skill in the art. Similarly, amounts sufficient to prevent, manage, treat, or ameliorate such disorders, but insufficient to cause, or sufficient to reduce, adverse effects associated with the antibodies, or antigen binding fragments thereof provided herein are also encompassed by the described dosage amounts, and dose frequency schedules herein. Further, when a subject is administered multiple dosages of a composition provided herein, not all of the dosages need be the same. For example, the dosage administered to the subject may be increased to improve the prophylactic, or therapeutic effect of the composition, or it may be decreased to reduce one, or more side effects that a particular subject is experiencing.

In certain instances, treatment, or prevention can be initiated with one, or more loading doses of a compound, or composition provided herein followed by one, or more maintenance doses.

In certain instances, a dose of a compound, or composition provided herein can be administered to achieve a steady-state concentration of the compound in blood, or serum of the subject. The steady-state concentration can be determined by measurement according to techniques available to those of skill or can be based on the physical characteristics of the subject such as height, weight, and age.

In certain instances, administration of the same composition may be repeated and the administrations may be separated by at least one day, two days, three days, five days, ten days, fifteen days, thirty days, forty-five days, two months, seventy-five days, three months, or six months. In other instances, administration of the same prophylactic, or therapeutic agent may be repeated, and the administration may be separated by at least one day, two days, three days, five days, ten days, fifteen days, thirty days, forty-five days, two months, seventy-five days, three months, or six months.

### Therapeutic Applications

For therapeutic applications, the compounds are administered to a mammal, in certain instances, a human, in a pharmaceutically acceptable dosage suitable for admisntration form such as those known in the art, and those discussed herein, intravenously as a bolus or by continuous infusion over a period of time, by intramuscular, intraperitoneal, intra-cerebrospinal, subcutaneous, intra-articular, intrasynovial, intrathecal, or intratumoral routes. The compounds also are suitably administered by peritumoral, intralesional, or perilesional routes, to exert local as well as systemic therapeutic effects. In certain instances, the compounds are administered to a mammal, in certain instances, a human, in a pharmaceutically acceptable dosage suitable for oral admisntration form such as those known in the art, and those discussed herein. For example, the compounds of this disclosure may be administered orally to a human as a liquid, or solid form. Solid dosage forms include, capsules, tablets, pills, powders, and granules. In such solid dosage forms, the chemical entity is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

The compounds provided herein may be useful for the treatment of any disease, or condition described herein (e.g., a metabolic disease or condition). In certain instances, the disease, or condition is any disease, or condition that benefits from modulation of GLP-1 receptor activity. In certain instances, the disease, or condition is any disease, or condition that benefits from agonizing GLP-1 receptor activity. In certain instances, the methods reduce blood glucose levels. In certain instances, the methods promote insulin synthesis, stimulate insulin secretion, increase the mass of β-cells, modulate gastric acid secretion, modulate gastric emptying, and/or decrease glucagon production. In certain instances, the disease, or condition is type 2 diabetes.

In certain instances, the disease, or condition is obesity, or one, or more diseases, or conditions associated with obesity. Non-limiting examples of obesity, and obesity related conditions include, symptomatic obesity, simple obesity, childhood obesity, morbid obesity, and abdominal obesity (central obesity characterized by abdominal adiposity). Non-limiting examples of symptomatic obesity include, endocrine obesity (e.g., Cushing syndrome, hypothyroidism, insulinoma, obese type II diabetes, pseudohypoparathyroidism, hypogonadism), hypothalamic obesity, hereditary obesity (e.g., Prader-Willi syndrome, Laurence-Moon-Biedl syndrome), and drug-induced obesity (e.g., steroid, phenothiazine, insulin, sulfonylurea agent, or β-blocker-induced obesity).

Examples of such diseases, and conditions associated with obesity include, without limitation, glucose tolerance disorders, diabetes (e.g., type 2 diabetes, obese diabetes), lipid metabolism abnormality, hyperlipidemia, hypertension, cardiac failure, hyperuricemia, gout, fatty liver (including non-alcoholic steatohepatitis (NASH)), coronary heart disease (e.g., myocardial infarction, angina pectoris), cerebral infarction (e.g., brain thrombosis, transient cerebral ischemic attack), bone, or articular disease (e.g., knee osteoarthritis, hip osteoarthritis, spondylitis deformans, lumbago), sleep apnea syndrome, obesity hypoventilation syndrome (Pickwickian syndrome), menstrual disorder (e.g., abnormal menstrual cycle, abnormality of menstrual flow and cycle, amenorrhea, abnormal catamenial symptom), visceral obesity syndrome, and metabolic syndrome. In certain instances, the compounds described herein can be used to treat subjects exhibiting symptoms of both obesity, and insulin deficiency.

In some instances, the disease, or condition is diabetes. Non-limiting examples of diabetes include, type 1 diabetes, type 2 diabetes (e.g., diet-treated type 2-diabetes, sulfonylurea-treated type 2-diabetes, a far-advanced stage type 2-diabetes, long-term insulin-treated type 2-diabetes), diabetes mellitus (e.g., non-insulin-dependent diabetes mellitus, insulin-dependent diabetes mellitus), gestational diabetes, obese diabetes, autoimmune diabetes, and borderline type diabetes.

In some instances, the disease, or condition is associated with diabetes (e.g., a complication of diabetes). Non-limiting examples of disorders associated with diabetes include, obesity, obesity-related disorders, metabolic syndrome, neuropathy, nephropathy (e.g., diabetic nephropathy), retinopathy, diabetic cardiomyopathy, cataract, macroangiopathy, osteopenia, hyperosmolar diabetic coma, infectious disease (e.g., respiratory infection, urinary tract infection, gastrointestinal infection, dermal soft tissue infections, inferior limb infection), diabetic gangrene, xerostomia, hypacusis, cerebrovascular disorder, diabetic cachexia, delayed wound healing, diabetic dyslipidemia peripheral blood circulation disorder, cardiovascular risk factors, (e.g., coronary artery disease, peripheral artery disease, cerebrovascular disease, hypertension, and risk factors related to unmanaged cholesterol, and/or lipid levels, and/or inflammation), NASH, bone fracture, and cognitive dysfunction

Other non-limiting examples of diseases, or conditions related to diabetes include, pre-diabetes, hyperlipidemia (e.g., hypertriglyceridemia, hypercholesterolemia, high LDL-cholesterolemia, low HDL-cholesterolemia, postprandial hyperlipemia), metabolic syndrome (e.g., metabolic disorder where activation of GLP-1R is beneficial, metabolic syndrome X), hypertension, impaired glucose tolerance (IGT), insulin resistance, and sarcopenia.

In some instances, the disease, or condition is diabetes, and obesity (diabesity). In certain instances, the compounds described herein are useful in improving the therapeutic effectiveness of metformin.

In some instances, the disease or condition is a disorder of a metabolically important tissue.

In some instances, the disease, or condition is a fatty liver disease. Fatty liver diseases include, but are not limited to, non-alcoholic fatty acid liver disease (NAFLD), steatohepatitis, non-alcoholic steatohepatitis (NASH), fatty liver disease resulting from hepatitis, fatty liver disease resulting from obesity, fatty liver disease resulting from diabetes, fatty liver disease resulting from insulin resistance, fatty liver disease resulting from hypertriglyceridemia, Abetalipoproteinemia, glycogen storage diseases, Weber-Christian disease, Wolmans disease, acute fatty liver of pregnancy, and lipodystrophy.

Non-alcoholic fatty liver disease (NAFLD) represents a spectrum of disease occurring in the absence of alcohol abuse, and is typically characterized by the presence of steatosis (fat in the liver). NAFLDis believed to be linked to a variety of conditions, e.g., metabolic syndrome (including obesity, diabetes, and hypertriglyceridemia), and insulin resistance. It can cause liver disease in adults and children, and may ultimately lead to cirrhosis (Skelly et al., J Hepatol 2001; 35: 195-9; Chitturi et al., Hepatology 2002; 35(2):373-9). The severity of NAFLD ranges from the relatively benign isolated predominantly macrovesicular steatosis (i.e., nonalcoholic fatty liver or NAFL) to non-alcoholic steatohepatitis (NASH)

(Angulo et al., J Gastroenterol Hepatol 2002; 17 SuppkS 186-90). In certain instances, the subject is a pediactric subject (e.g., 6-16 years old; or 6-12 years old; or 6-10 years old). In certain instances, the subject is an adult subject.

Other non-limiting examples of diseases, or conditions in metabolically important tissues include, joint disorders (e.g., osteoarthritis, secondary osteoarthritis), steatosis (e.g. in the liver); gall stones; gallbladder disorders; gastroesophageal reflux; sleep apnea; hepatitis; fatty liver; bone disorder characterized by altered bone metabolism, such as osteoporosis, including post-menopausal osteoporosis, poor bone strength, osteopenia, Paget's disease, osteolytic metastasis in cancer patients, osteodistrophy in liver disease, and the altered bone metabolism caused by renal failure, or haemodialysis, bone fracture, bone surgery, aging, pregnancy, protection against bone fractures, and malnutritionpolycystic ovary syndrome; renal disease (e.g., chronic renal failure, glomerulonephritis, glomerulosclerosis, nephrotic syndrome, hypertensive nephrosclerosis, end-stage renal disease); muscular dystrophy, angina pectoris, acute, or chronic diarrhea, testicular dysfunction, respiratory dysfunction, frailty, sexual dysfunction (e.g., erectile dysfunction), and geriatric syndrome. In certain instances, the chemical entities described herein can be used for treating surgical trauma by improving recovery after surgery, and/or by preventing the catabolic reaction caused by surgical trauma.

In some instances, the disease, or condition is a cardiovascular disease. Non-limiting examples of cardiovascular disease include, congestive heart failure, atherosclerosis, arteriosclerosis, coronary heart disease, or peripheral artery disease, stroke, coronary artery disease, congestive heart failure, coronary heart disease, hypertension, cardiac failure, cerebrovascular disorder (e.g., cerebral infarction), vascular dysfunction, myocardial infarction, elevated blood pressure (e.g., 130/85 mm Hg or higher), and prothrombotic state (exemplified by high fibrinogen, or plasminogen activator inhibitor in the blood).

In some instances, the disease, or condition is a neurological disorder (e.g., neurodegenerative disorder), or a psychiatric disorder. Non-limiting examples of neurological disorders include, brain insulin resistance, mild cognitive impairment (MCI), Alzheimer's disease (AD), Parkinson's disease (PD), anxiety, dementia (e.g., senile dementia), traumatic brain injury, Huntington's chores, tardive dyskinesia, hyperkinesia, mania, Morbus Parkinson, steel-Richard syndrome, Down's syndrome, myasthenia gravis, nerve trauma, brain trauma, vascular amyloidosis, cerebral hemorrhage I with amyloidosis, brain inflammation, Friedrich's ataxia, acute confusion disorder, amyotrophic lateral sclerosis (ALS), glaucoma, and apoptosis-mediated degenerative diseases of the central nervous system (e.g., Creutzfeld-Jakob Disease, bovine spongiform encephalopathy (mad cow disease), chronic wasting syndrome). See, e.g., US20060275288A1.

Non-limiting examples of psychiatric disorders include, drug dependence/addiction (narcotics, amphetamines, and attention deficit/hyperactivity disorder (ADHD). The chemical entities described herein can be useful in improving behavioral response to addictive drugs, decreasing drug dependence, prevention drug abuse relapse, and relieving anxiety caused by the absence of a given addictive substance. See, e.g., US20120021979A1.

In certain instances, the chemical entities described herein are useful in improving learning, and memory by enhancing neuronal plasticity, and facilitation of cellular differentiation, and also in preserving dopamine neurons, and motor function in Morbus Parkinson.

In some instances, the disease, or condition is impaired fasting glucose (IFG), impaired fasting glycemia (IFG), hyperglycemia, insulin resistance (impaired glucose homeostasis), hyperinsulinemia, elevated blood levels of fatty acids, or glycerol, a hypoglycemic condition, insulin resistant syndrome, paresthesia caused by hyperinsulinemia, hyperlipidaemia, hypercholesteremia, impaired wound healing, leptin resistance, glucose intolerance, increased fasting glucose, dyslipidemia (e.g., hyperlipidemia, atherogenic dyslipidemia characterized by high triglycerides and low HDL cholesterol), glucagonoma, hyperuricacidemia, hypoglycemia (e.g., nighttime hypoglycemia), and concomitant comatose endpoint associated with insulin.

In certain instances, the compounds described herein can reduce, or slow down the progression of borderline type, impaired fasting glucose, or impaired fasting glycemia into diabetes.

In some instances, the disease, or condition is an autoimmune disorder. Non-limiting examples of autoimmune disorders include, multiple sclerosis, experimental autoimmune encephalomyelitis, autoimmune disorder is associated with immune rejection, graft versus host disease, uveitis, optic neuropathies, optic neuritis, transverse myelitis, inflammatory bowel disease, rheumatoid arthritis, ankylosing spondylitis, systemic lupus erythematosus, myasthenia gravis, and Graves disease. See, e.g., US20120148586A1.

In some instances, the disease, or condition is a stomach, or intestine related disorder. Non-limiting examples of these disorders include, ulcers of any etiology (e.g. peptic ulcers, Zollinger-Ellison syndrome, drug-induced ulcers, ulcers related to infections, or other pathogens), digestion disorders, malabsorption, short bowel syndrome, cul-de-sac syndrome, inflammatory bowel diseases (Crohn's disease, and ulcerative colitis), celiac sprue, hypogammaglobulinemic sprue, chemotherapy, and/or radiation therapy-induced mucositis, and diarrhea, gastrointestinal inflammation, short bowel syndrome, colitis ulcerosa, gastric mucosal injury (e.g., gastric mucosal injury caused by aspirin), small intestinal mucosal injury, and cachexia (e.g., cancerous cachexia, tuberculous cachexia, cachexia associated with blood disease, cachexia associated with endocrine disease, cachexia associated with infectious disease, cachexia caused by acquired immunodeficiency syndrome).

In some instances, the compounds described herein can be used to reduce body weight (e.g., excess body weight), prevent body weight gain, induce weight loss, decrease body fat, or reduce food intake in a subject (e.g., a subject in need thereof). In certain instances, the weight increase in a subject may be attributed to excessive ingestion of food, or unbalanced diets, or may be weight increase derived from a concomitant drug (e.g., insulin sensitizers having a PPARγ agonist-like action, such as troglitazone, rosiglitazone, englitazone, ciglitazone, pioglitazone, and the like). Alternatively, the weight increase may be weight increase before reaching obesity, or may be weight increase in an obese subject. The weight increase may also be medication-induced weight gain, or weight gain subsequent to cessation of smoking.

In some instances, the condition, disease, or disorder is an eating disorder, such as hyperphagia, binge eating, bulimia, or compulsive eating.

In some instances, the disease, or condition is an inflammatory disorder. Non-limiting examples of inflammatory disorders include, chronic rheumatoid arthritis, spondylitis deformans, arthritis deformans, lumbago, gout, post-operational or post-traumatic inflammation, bloating, neuralgia, laryngopharyngitis, cystitis, pneumonia, pancreatitis, enteritis, inflammatory bowel disease (including inflammatory large bowel disease), inflammation in metabolically important tissues including liver, fat, pancreas, kidney, and gut, and a proinflammatory state (e.g., elevated levels of proinflammatory cytokines or, markers of inflammation-like C-reactive protein in the blood).

In some instances, the disease, or condition is cancer. Suitable examples of cancer include, breast cancer (e.g., invasive ductal breast cancer, noninvasive ductal breast cancer, inflammatory breast cancer), prostate cancer (e.g., hormone-dependent prostate cancer, hormone-independent prostate cancer), pancreatic cancer (e.g., ductal pancreatic cancer), gastric cancer (e.g., papillary adenocarcinoma, mucous adenocarcinoma, adenosquamous carcinoma), lung cancer (e.g., non-small cell lung cancer, small-cell lung cancer, malignant mesothelioma), colon cancer (e.g., gastrointestinal stromal tumor), rectal cancer (e.g., gastrointestinal stromal tumor), colorectal cancer (e.g., familial colorectal cancer, hereditary non-polyposis colorectal cancer, gastrointestinal stromal tumor), small intestinal cancer (e.g., non-Hodgkin's lymphoma, gastrointestinal stromal tumor), esophageal cancer, duodenal cancer, tongue cancer, pharyngeal cancer (e.g., nasopharyngeal cancer, oropharynx cancer, hypopharyngeal cancer), salivary gland cancer, brain tumor (e.g., pineal astrocytoma, pilocytic astrocytoma, diffuse astrocytoma, anaplastic astrocytoma), neurilemmoma, liver cancer (e.g., primary liver cancer, extrahepatic bile duct cancer), renal cancer (e.g., renal cell cancer, transitional cell cancer of the renal pelvis and ureter), bile duct cancer, endometrial cancer, uterine cervical cancer, ovarian cancer (e.g., epithelial ovarian cancer, extragonadal germ cell tumor, ovarian germ cell tumor, ovarian tumor of low malignant potential), bladder cancer, urethral cancer, skin cancer (e.g., intraocular (ocular) melanoma, Merkel cell carcinoma), hemangioma, malignant lymphoma, malignant melanoma, thyroid cancer (e.g., medullary thyroid cancer), parathyroid cancer, nasal cavity cancer, sinus cancer, bone tumor (e.g., osteosarcoma, Ewing tumor, uterine sarcoma, soft tissue sarcoma), angiofibroma, sarcoma of the retina, penis cancer, testicular tumor, pediatric solid tumor (e.g., Wilms' tumor, childhood kidney tumor), Kaposi's sarcoma, Kaposi's sarcoma caused by AIDS, tumor of maxillary sinus, fibrous histiocytoma, leiomyosarcoma, rhabdomyosarcoma, and leukemia (e.g., acute myeloid leukemia, acute lymphoblastic leukemia).

In certain instances, provided herein are methods for the treatment that include, the administration of an effective amount of compounds provided herein, or a pharmaceutically acceptable salt thereof. In certain instances, the methods encompass the step of administering to the subject in need thereof an amount of a compound described herein effective for the treatment of disease, or condition in combination with a second agent effective for the treatment, or prevention of the disease, or condition. In certain instances, the compound is in the form of a pharmaceutical composition, or dosage form, as described elsewhere herein.

In certain instances, the subject is a treatment naive subject. In further instances, the subject has previously received therapy. For instance, in certain instances, the subject has not responded to a single agent treatment regimen.

In certain instances, the subject is a subject that discontinued some other therapy because of one or more adverse events associated with the other therapy. In certain instances, the subject has received some other therapy and discontinued that therapy prior to administration of a method provided herein. In further instances, the subject has received therapy and continues to receive that therapy along with administration of a compound provided herein. The compounds described herein can be co-administered with other therapy for treatment of the disease or condition according to the judgment of one of skill in the art. In certain instances, the methods or compositions provided herein can be co-administered with a reduced dose of the other therapy for the treatment of the disease or condition.

### Diagnostic Applications

In some instances, the compounds provided herein are used in diagnostic applications. These applications may be useful, for example, in making a diagnosis, and/or prognosis for a disease, or condition, such as a metabolic disease, or condition.

In some diagnostic and prognostic applications or instances, the compound may be labeled with a detectable moiety. Suitable detectable moieties include, but are not limited to, radioisotopes, fluorescent labels, and enzyme-substrate labels. In another instance, the compound need not be labeled, and the presence of the compound can be detected using a labeled antibody, or antigen binding fragment thereof which specifically binds to the compound.

### Kits

In some instances, a compound provided herein is provided in the form of a kit (i.e., a packaged combination of reagents in predetermined amounts with instructions for performing a procedure). In some instances, the procedure is a diagnostic assay. In certain instances, the procedure is a therapeutic procedure.

In some instances, the kit further comprises a solvent for the reconstitution of the compound. In some instances, the compound is provided in the form of a pharmaceutical composition.

In some instances, the kits can include a compound, or composition provided herein, an optional second agent, or composition, and instructions providing information to a health care provider regarding usage for treating the disorder. Instructions may be provided in printed form, or in the form of an electronic medium such as a floppy disc, CD, or DVD, or in the form of a website address where such instructions may be obtained. A unit dose of a compound, or a composition provided herein, or a second agent, or composition, can include a dosage such that when administered to a subject, a therapeutically, or prophylactically effective plasma level of the compound, or composition can be maintained in the subject for at least one day. In some instances, a compound, or composition can be included as a sterile aqueous pharmaceutical composition, or dry powder (e.g., lyophilized) composition.

In some instances, suitable packaging is provided. As used herein, "packaging" includes a solid matrix, or material customarily used in a system, and capable of holding within fixed limits a compound provided herein, and/or a second agent suitable for administration to a subject. Such materials include, glass, and plastic (e.g., polyethylene, polypropylene, and polycarbonate) bottles, vials, paper, plastic, plastic-foil laminated envelopes, and the like. If e-beam sterilization techniques are employed, the packaging should have sufficiently low density to permit sterilization of the contents.

### Preparation and Synthetic Procedures

In some instances, the compounds described herein are prepared as outlined in **Schemes 1-7.** The synthesis of the compounds in this disclosure is not limited to these general reaction schemes illustrated here. For detailed synthesis of each individual compound, please check the Examples section.

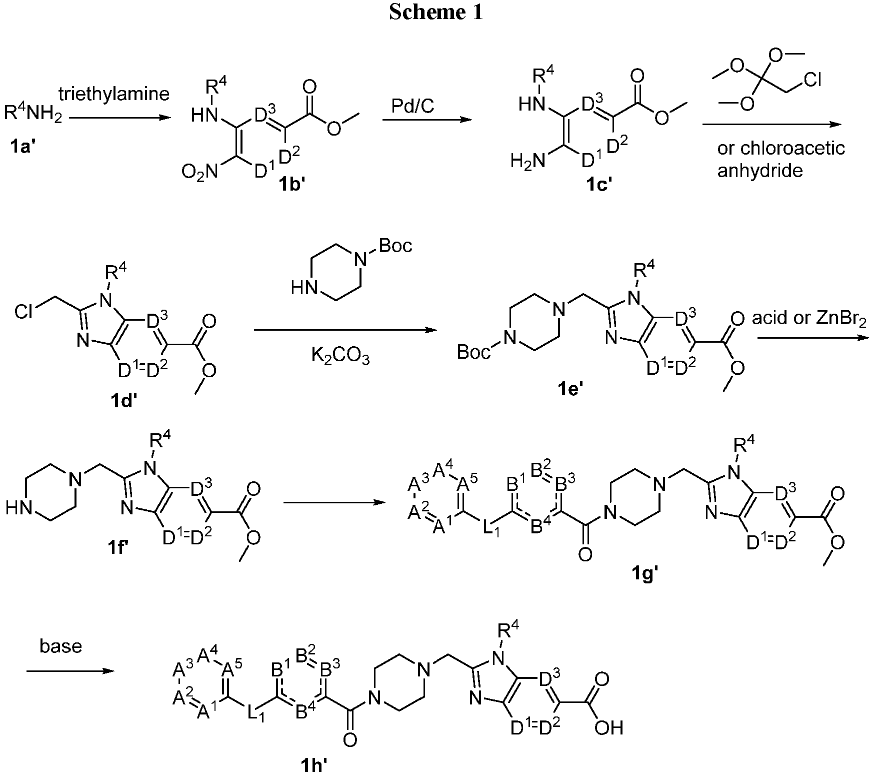

**Scheme** 1. Compound with a formula **1h'** can be made as shown in **Scheme 1.** An amine can be reacted with fluoro, nitro-substituted aryl or heteroaryl compound through a SNAr reaction to provide **1b'.** The nitro group in 1b' will be reduced to an amino group through hydrogenation. Reaction of **1c**' with 2-chloro-1,1,1-trimethoxyethane or chloroacetic anhydride will form **1d'.** Alkylation of **1d**' with *tert-*butyl piperazine-1-carboxylate will provide **1e**', in which the Boc protecting group can be removed by TFA or HCl. The resulting piperazine **1f**' can be coupled with a carboxylic acid to form **1g',** which can be deprotected to give **1h**'.

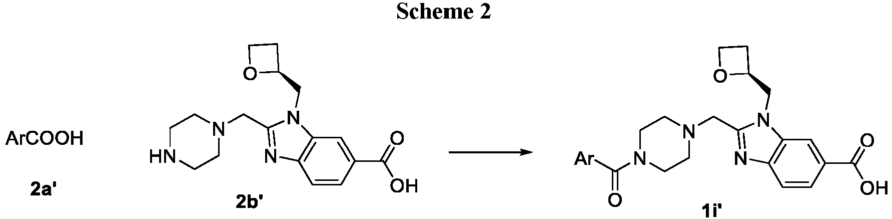

**Scheme 2.** Alternatively, compound **1i**' (analog of **1h**') can be synthesized though a direct coupling reaction of acid-containing piperazine **2b'** with a carboxylic acid **2a'.**

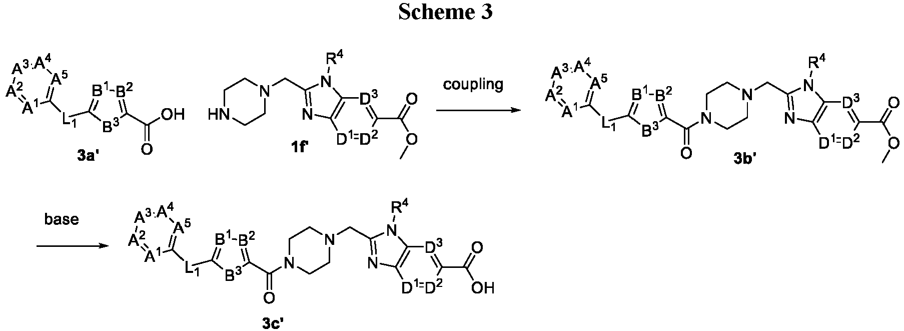

**Scheme 3.** Some of the alternative carboxylic acids **3a'** contain 5-membered heteroaryl ring. They can be coupled with **1f**' to provide compound **3b',** which can be hydrolyzed to provide **3c'.**

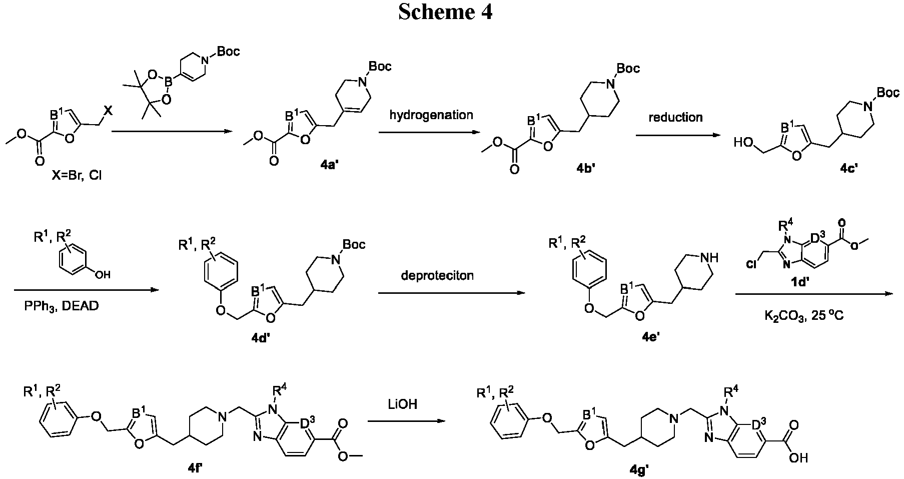

**Scheme 4.** Compound **4a'** can be prepared from a halide and tert-butyl 4-(4,4,5,5-tetramethyl-1,3,2-dioxaborolan-2-yl)-3,6-dihydropyridine-1(2*H*)-carboxylate through a Suzuki reaction, which after hydrogenation would result in compound **4b'.** Reduction of the ester group would form an alcohol **4c',** which can be used to prepare compound **4d'** through a Mitsunobu reaction. Alternatively, **4c'** can be converted to a bromide and can be reacted with a phenol through an alkylation reaction to form **4d'.** After deprotection of the Boc group, **4e'** can be alkylated to give **4f',** and hydrolyzed to provide **4g'.**

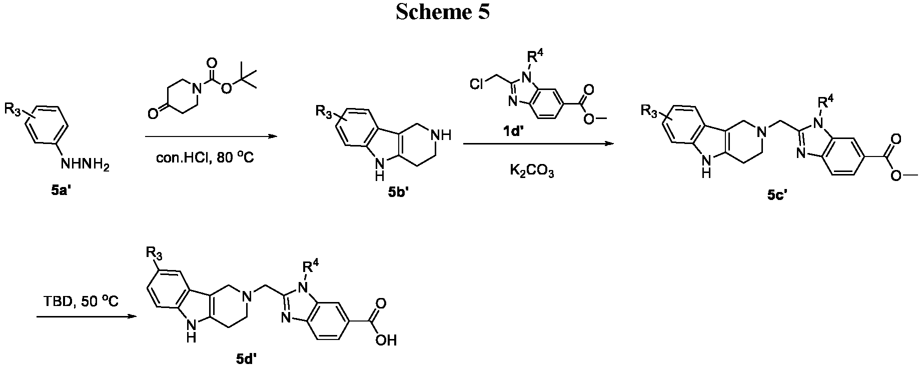

**Scheme 5.** Hydrazine **5a'** can be reacted with *tert-*butyl 4-oxopiperidine-1-carboxylate through Fischer indole synthesis to form carboline **5b',** which can be alkylated with **1d'** to give **5c'.** Compound **5d'** can be prepared through hydrolysis of **5c'.**

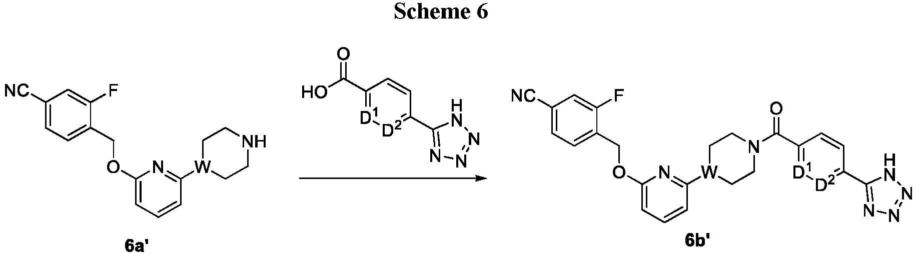

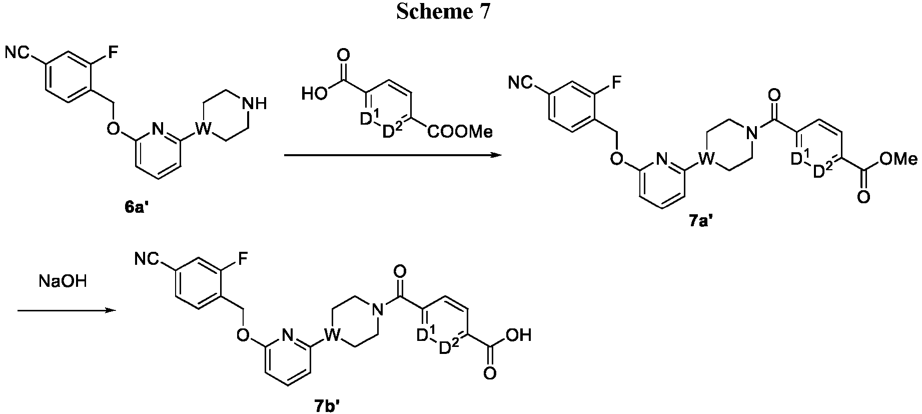

**Schemes 6** and **7.** Coupling of piperidine or piperazine 6a' with various carboxylic acid will lead to compound **6b',** or **7b'.**

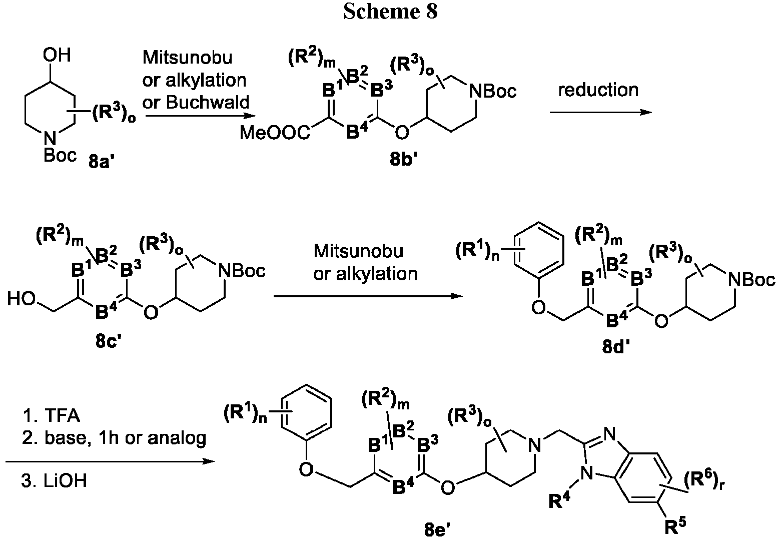

**Scheme 8.** (zero, one, or two of **B¹, B²**, **B³**, and **B⁴** are N, and the rest are CH). Alcohol **8a'** can be used to synthesize **8b'** through Mitsunobu reaction with an alcohol (e.g., phenol), or converted to a mesylate followed by alkylation with a phenol, or coupling with aryl halide through Buchwald reaction to form **8b'.** Reduction of the ester group in **8b'** can afford alcohol **8c'.** Then through Mitsunobu reaction with a phenol or mesylate formation of **8c'** followed by alkylation with a phenol can provide **8d'.** Deprotection of the Boc group with TFA, followed by coupling with **1h** and hydrolysis can afford **8e'.**

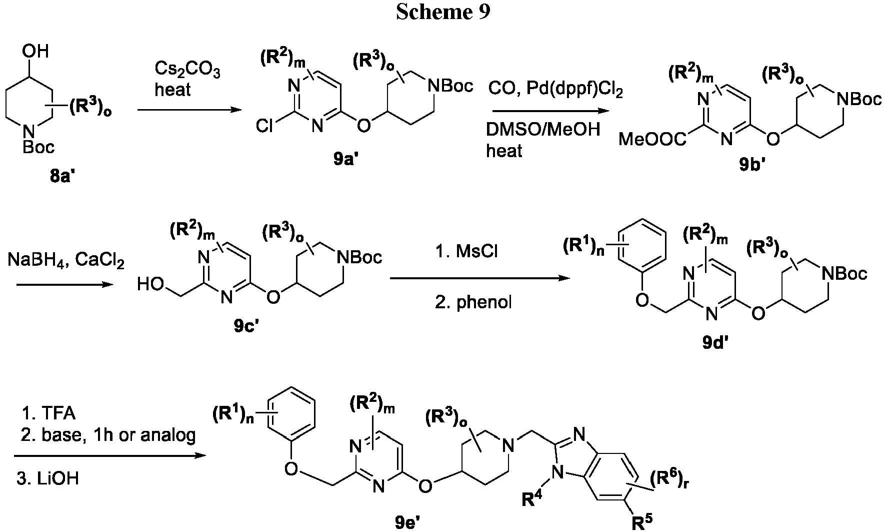

**Scheme** 9. **9a'** can be synthesized from **8a' through** alkylation. Palladium-catalyzed carbonylation would afford ester **9b',** which can be reduced to provide alcohol **9c'.** Mesylation formation of alcohol **9c'** followed by alkylation with a phenol can provide **9d'.** Deprotection of the Boc group with TFA, followed by coupling with **1h** and hydrolysis can afford **9e'.**

### EXAMPLES

### Preparation of the Compounds

The compounds used in the reactions described herein are made according to organic synthesis techniques known to those skilled in this art, starting from commercially available chemicals, and/or from compounds described in the chemical literature. "Commercially available chemicals" are obtained from standard commercial sources such as Acros Organics (Pittsburgh, PA), Advanced ChemBlocks, Inc (Burlingame, CA), Aldrich Chemical (Milwaukee, WI, including Sigma Chemical and Fluka), AK Scientific (Union City, CA), AstaTech, Inc. (Bristol, PA), Aurum Pharmatech LLC (Franklin Park, NJ), Combi-Blocks, Inc. (San Diego, CA), Enamine (Monmouth Jct., NJ), Fisher Scientific Co. (Pittsburgh, PA), Frontier Scientific (Logan, UT), TCI America (Portland, OR), and VWR (Radnor, PA). Specific and analogous reactants are optionally identified through the indices of known chemicals prepared by the Chemical Abstract Service of the American Chemical Society, which are available in most public and university libraries, as well as through on-line databases.

Suitable reference books that detail the synthesis of reactants useful in the preparation of compounds described herein, or provide references to articles that describe their preparation, include for example, "Synthetic Organic Chemistry", John Wiley & Sons, Inc., New York; S. R. Sandler et al., "Organic Functional Group Preparations," 2nd Ed., Academic Press, New York, 1983; "T. L. Gilchrist, "Heterocyclic Chemistry", 2nd Ed., John Wiley & Sons, New York, 1992; and J. March, "Advanced Organic Chemistry: Reactions, Mechanisms and Structure", 4th Ed., Wiley-Interscience, New York, 1992; R. C. Larock "Comprehensive Organic Transformations: A Guide to Functional Group Preparations" 2nd Edition (1999) Wiley-VCH, ISBN: 0-471-19031-4; "Organic Reactions" (1942-2000) John Wiley & Sons, in over 55 volumes; and "Chemistry of Functional Groups" John Wiley & Sons, in 73 volumes. Some compounds require the application of protecting groups. The need for such protection is within the skill in the art. For a general description of protecting groups and their use, see T.W. Greene, P.G.M. Nuts, and Protective Groups in Organic Synthesis, John Wiley & Sons, New York, 1999.

### Analytical Methods and Instrumentation

Proton nuclear magnetic resonance (NMR) spectra were obtained on either Bruker, or Varian spectrometers at 400, or 600 MHz. NMR spectra are reported relative to residual solvent signals as follows: chemical shift δ (ppm), multiplicity, coupling constant *J* (Hz), and integration. Tetramethylsilane (TMS) was used as an internal standard in some of the cases. Mass spectral data were measured using one of the two following systems: System A: Waters Acquity i-class ultra-performance liquid chromatography (UPLC) system with Acquity Photo Diode Array Detector, Acquity Evaporative Light Scattering Detector (ELSD) and Waters ZQ Mass Spectrometer. Data was acquired using Waters MassLynx 4.1 software and purity characterized by UV wavelength 220 nm, evaporative light scattering detection (ELSD) and electrospray positive ion (ESI) (column: Acquity UPLC BEH C18 1.7 µ 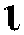 2.1 x 50 mm). System B: Agilent LC/MS consisting of a 1200 series LC and 6140 Quadrupole MS detector (column: Agilent USGYL01131, HPH-C18 2.7 µM, 2.1 x 50 mm). Solvents used: acetonitrile/water, containing 0.1% formic acid; flow rate 0.7 mL/min. Preparatory HPLC purifications were conducted with a flow rate of 15 mL/min and detection by UV wavelength 214 nm and 254 nm (Column: Jupiter^{©} 10 µM Proteo 90 Å, 250 x 21.2 mm A, solvent: acetonitrile/water, containing modifier such as 0.1% trifluoroacetic acid, formic acid or acetic acid). Compound purity was checked on an analytical HPLC (Waters Acquity UPLC H-Class instrument), with a flow rate of 0.5 mL/min (Acquity BEH C18, 50x2.1 mm column).

*Abbreviations used in the examples include:*

| **Abbreviation** | **Name** |
|---|---|
| CH₃CN | acetonitrile |
| aq. | aqueous |
| atm | atmospheres |
| BINAP | (1,1'-binaphthalene-2,2'-diyl)bis(diphenylphosphine) |
| Boc | *t*-butoxycarbonyl |
| CCl₄ | carbon tetrachloride |
| CDCl₃ | deuterated chloroform |
| CO | carbon monoxide gas |
| CO₂ | carbon dioxide |
| Cs₂CO₃ | cesium carbonate |
| CuBr | copper(I) bromide |
| Cu(OAc)₂ | copper(II) acetate |
| DCM | dichloromethane |
| DEAD | diethyl azodicarboxylate |
| DIPEA | diisopropylethylamine |
| DMF | *N,N-*dimethylformamide |
| DMSO | dimethylsulfoxide |
| ESI | electrospray ionization |
| Et₃N | triethylamine |
| EtOAc | ethyl acetate |
| EtOH | ethanol |
| h | hours |
| H₂O | water |
| HATU | 1-[bis(dimethylamino)methylene]-1H-1,2,3-triazolo[4,5-b]pyridinium 3-oxid hexafluorophosphate |
| HCl | hydrochloric acid |
| HOAc | acetic acid |
| K₂CO₃ | potassium carbonate |
| KI | potassium iodide |
| KOH | potassium hydroxide |
| KOtBu | potassium *tert*-butoxide |
| LiAlH₄ | lithium aluminum hydride |
| LiOH | lithium hydroxide |
| MeOH | methanol |
| min | minutes |
| N2 | nitrogen |
| Na₂CO₃ | sodium carbonate |
| Na₂SO₄ | sodium sulfate |
| NaH | sodium hydride |
| NaHCO₃ | sodium bicarbonate |
| NaN₃ | sodium azide |
| NaOH | sodium hydroxide |
| NBS | N-bromosuccinimide |
| NH₄Cl | ammonium chloride |
| NH₄HCO₃ | ammonium bicarbonate |
| NMR | nuclear magnetic resonance |
| Pd₂(dba)₃ | tris(dibenzylideneacetone)dipalladium(0) |
| Pd(dppf)Cl₂ | [1,1'-bis(diphenylphosphino)ferrocene]dichloropalladium(II) |
| Pd/C | palladium on carbon |
| Pd(OAc)₂ | palladium(II) acetate |
| Prep-TLC | preparatory thin layer chromatography |
| RuPhos-Pd-G2 | chloro(2-dicyclohexylphosphino-2',6'-diisopropoxy-1,1'-biphenyl)[2-(2'-amino-1,1'-biphenyl)]palladium(II) |
| sat. | saturated |
| t-BuOH | *tert*-butanol |
| T₃P | propylphosphonic anhydride |
| TBD | 1,5,7-triazabicyclo[4.4.0]dec-5-ene |
| TFA | trifluoracetic acid |
| THF | tetrahydrofuran |

Unless otherwise noted, reagents, and solvents were used as received from commercial suppliers. Anhydrous solvents and oven-dried glassware were used for synthetic transformations sensitive to moisture, and/or oxygen. Reaction times, and yields were not optimized. Example numbers and compound numbers are the same. The compound recited in appended claim 1 is Compound 485 or a pharmaceutically acceptable salt or solvate thereof. Other compounds are disclosed for illustrative or comparative purposes.

### Example 1. (S)-2-((4-(6-(Dimethylamino)pyrimidine-4-carbonyl)piperazin-1-yl)methyl)-1-(oxetan-2-ylmethyl)-1H-benzo[d]imidazole-6-carboxylic acid (1)

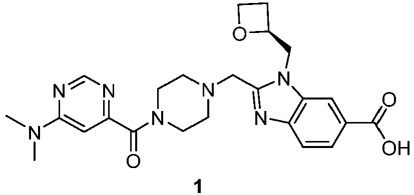

### Step A. (S)-2-((Benzyloxy)methyl)oxetane (1a)

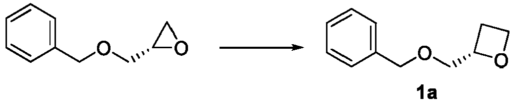

To a solution of KO*t*Bu (2.97 g, 30.4 mmol) in *t*BuOH (50 mL) was added trimethylsulfoxonium (6.68 g, 30.4 mmol). The reaction mixture was stirred at 60 °C for 30 min, then (2S)-2-[(benzyloxy)methyl]oxirane (5 g, 30.4 mmol) was added. The mixture was heated at 80 °C for 2 h. After completion, the mixture was cooled to 25 °C and filtered through celite. The filtered cake was washed with petroleum ether (10 mL). The filtrate was added water (20 mL), and extracted with petroleum ether (10 mL x 2). The combined organic layers were washed with saturated NH₄Cl (10 mL), dried over magnesium sulfate, filtered, and concentrated *in vacuo* to provide a crude residue, which was purified by silica gel column chromatography (petroleum ether:EtOAc = 1:1) to afford the title product **(1a)** (2.5 g, 47%) as a yellow oil. *m*/*z* (ESI, +ve ion) = 201.1 [M+Na] ⁺.

### Step B. (S)-Oxetan-2-ylmethanol (1b)

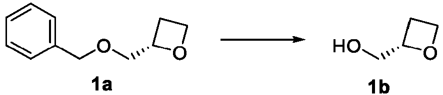

To a solution of **1a** (7 g, 39.3 mmol) in THF (90 mL) was added Pd(OH)₂ (700 mg, 3.93 mmol). The reaction mixture was stirred at 45 °C under 0.4 Mpa H₂ for 48 h. After completion, the reaction was filtered through celite and the resulting crude mixture **(1b)** in THF was carried to the next step without further purification. *m*/*z* (ESI, +ve ion) = 111.1 [M+Na] ⁺.

### Step C. (S)-Oxetan-2-ylmethyl methanesulfonate (1c)

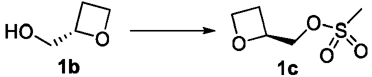

To a solution of **1b** (6 g, 68 mmol) in THF (100 mL) was added methanesulfonic anhydride (17.1 g, 102 mmol), and triethylamine (TEA) (13.6 g, 136.5 mmol) at 0 °C. The reaction mixture was stirred at 20 °C for 2 h. The reaction was quenched with water (30 mL), and extracted with EtOAc (30 mL x 3). The combined organic layers were dried over anhydrous Na₂SO₄, filtered, and concentrated under reduced pressure. The residue was purified by silica gel column chromatography (petroleum:EtOAc = 1:1) to afford the title product (1c) (6 g, 53.1%) as a yellow oil. *m*/*z* (ESI, +ve ion) = 167.1 [M+H]⁺.

### Step D. (S)-2-(Azidomethyl)oxetane (1d)

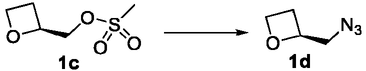

To a solution of **1c** (6 g, 36.1 mmol) in DMF (100 mL) was added NaN₃ (7.02 g, 108 mmol). The reaction mixture was stirred at 80 °C for 16 h. The reaction was added water (20 mL), and extracted with EtOAc (20 mL x 2). The organic layer was washed with H₂O (10 mL), dried, and concentrated to provide a crude title product **(1d)** which was carried to the next step without further purification. *m*/*z* (ESI, +ve ion) = 114.1 [M+H]⁺.

### Step E. (S)-Oxetan-2-ylmethanamine (1e)

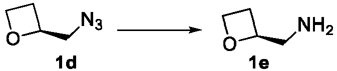

To a solution of **1d** (900 mg, 7.96 mmol) in THF (30 mL) was added 10% Pd/C (10 mg). The reaction mixture was stirred at 20 °C for 2 h under hydrogen. After completion, the crude reaction mixture was filtered through celite. The filtrate (**1e**) was used in the next step without further purification. *m*/*z* (ESI, +ve ion) = 88.2 [M+H]⁺.

### Step F. Methyl (S)-4-nitro-3-((oxetan-2-ylmethyl)amino)benzoate (1f)

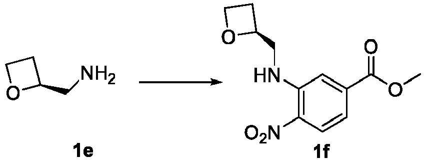

To a solution of **1e** (700 mg, 10.5 mmol) in THF (3 mL) was added methyl 3-fluoro-4-nitrobenzoate (2.09 g, 10.5 mmol), and TEA (3.18 g, 31.5 mmol). The reaction mixture was stirred at 25 °C for 16 h, then added water (15 mL). The solution was extracted with EtOAc (10 mL x 3). The combined organic layers were dried over anhydrous Na₂SO₄, filtered, and concentrated under reduced pressure. The crude residue was purified by silica gel column chromatography (petroleum ether:EtOAc = 1:1) to afford the title product **(1f)** (1.2 g, 43.9%) as a yellow solid. *m*/*z* (ESI, +ve ion) = 267.1 [M+H]⁺.

### Step G. Methyl (S)-4-amino-3-((oxetan-2-ylmethyl)amino)benzoate (1g)

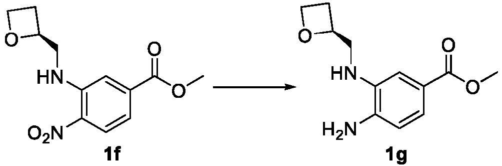

To a solution of **1f** (800 mg, 7.9 mmol) in THF (8 mL) was added 10% Pd/C (100 mg). The reaction mixture was stirred at 20 °C for 3 h under 50 psi H₂. After completion, the crude mixture was filtered through celite, and the filtrate was concentrated to give the title product **(1g)** (0.6 g, 43.9%) as a yellow solid. *m*/*z* (ESI, +ve ion) = 237.2 [M+H]⁺.

### Step H. Methyl (S)-2-(chloromethyl)-1-(oxetan-2-ylmethyl)-1H-benzo[d]imidazole-6-carboxylate (1h)

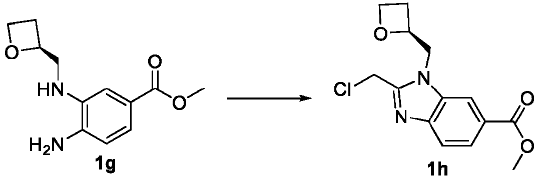

To a solution of **1g** (700 mg, 2.96 mmol) in CH₃CN (10 mL) was added 2-chloro-1,1,1-trimethoxyethane (456 mg, 2.96 mmol), and 4-methyl-benzenesulfonic acid (25 mg, 0.14 mmol). The reaction mixture was stirred at 60 °C for 2 h. The residue was purified by silica gel column chromatography (petroleum ether:EtOAc = 1:1) to afford the title product **(1h)** (0.6 g, 68.9%) as a yellow solid. *m*/*z* (ESI, +ve ion) = 295.1 [M+H]⁺.

### Step I. Methyl (S)-2-((4-(tert-butoxycarbonyl)piperazin-1-yl)methyl)-1-(oxetan-2-ylmethyl)-1H-benzo[d]imidazole-6-carboxylate (1i)

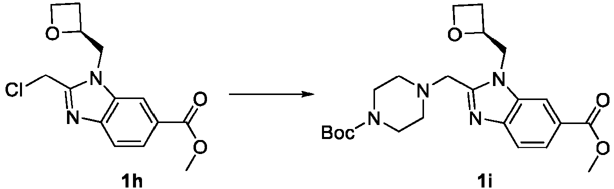

To a solution of **1h** (30 mg, 0.10 mmol) in DMF (2 mL) was added *tert-*butyl piperazine-1-carboxylate (18.9 mg, 0.10 mmol), and K₂CO₃ (22 mg, 0.15 mmol). The reaction mixture was stirred at 25 °C for 16 h. After completion, the mixture was quenched with water (20 mL), extracted with EtOAc (20 mL x 3), and washed with water (10 mL). The organic phase was dried with anhydrous sodium sulfate, concentrated, and purified by silica gel column chromatography (gradient elution, 0-50% EtOAc in petroleum ether) to afford the title product (**1i**) (20 mg, 44.4%) as a yellow solid. *m*/*z* (ESI, +ve ion) = 445.1 [M+H]⁺.

### Step J. Methyl (S)-1-(oxetan-2-ylmethyl)-2-(piperazin-1-ylmethyl)-1H-benzo[d]imidazole-6-carboxylate (1j)

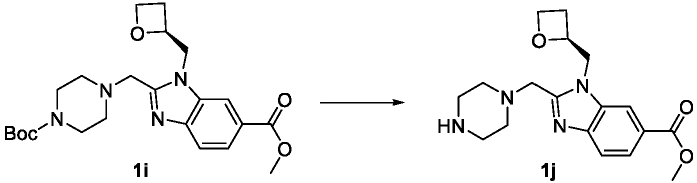

To a solution of **1i** (30 mg, 0.067 mmol) in DCM (2 mL) was added ZnBr₂ (60.8 mg, 0.27 mmol). The reaction mixture was stirred at 25 °C for 12 h. After completion, the residue was purified by reverse phase HPLC (gradient elution, 0-30% CH₃CN in water with 0.1% TFA as a modifier) to afford the title product **(1j)** (15 mg, 65%) as a white solid. *m*/*z* (ESI, +ve ion) = 345.3 [M+H]⁺.

### Step K. Methyl 6-(dimethylamino)pyrimidine-4-carboxylate (1k)

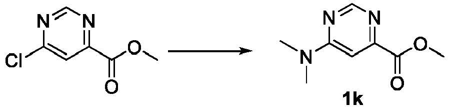

A mixture of methyl 6-chloropyrimidine-4-carboxylate (500 mg, 2.28 mmol), dimethylamine (392 mg, 8.69 mmol), and *N*,*N*-diisopropylethylamine (1123 mg, 8.69 mmol) in THF (30 mL) was stirred at 25 °C for 2 h. The reaction was quenched with H₂O (10 mL), saturated NaHCO₃ (10 mL) solution, and was then extracted with DCM (150 mL x 3). The organic layers were combined, dried, and concentrated to afford the crude title product **(1k)** (1.06 g) as a yellow solid, which was used directly in the next step without further purification. *m*/*z* (ESI, +ve ion) = 182.0 [M+H]⁺.

### Step L. 6-(Dimethylamino)pyrimidine-4-carboxylic acid (1l)

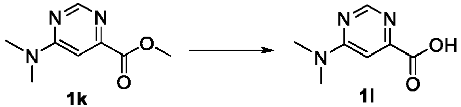

A mixture of **1k** (1.0 g, 5.51 mmol) and sodium hydroxide (1.1 g, 27.6 mmol) in MeOH:H₂O = 10:1 (20 mL) was stirred at 25 °C for 2 h. The reaction was then extracted with EtOAc (150 mL x 3). The organic layers were combined, dried over Na₂SO₄, and concentrated to afford a crude residue, which was purified by reverse phase HPLC to give the title product (**1l**) (346 mg, 37.5%) as a white solid. *m*/*z* (ESI, +ve ion) = 168.0 [M+H]⁺.

### Step M. Methyl (S)-2-((4-(6-(dimethylamino)pyrimidine-4-carbonyl)piperazin-1-yl)methyl)-1-(oxetan-2-ylmethyl)-1H-benzo[d]imidazole-6-carboxylate (1m)

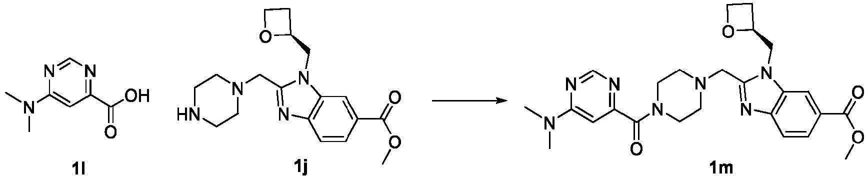

A mixture of **1l** (58 mg, 0.35 mmol), **1j** (60 mg, 0.17 mmol), 1-hydroxybenzotriazole (30 mg, 0.23 mmol), *N*-(3-dimethylaminopropyl)-N'-ethylcarbodiimide hydrochloride (40 mg, 0.21 mmol), and *N,N*-diisopropylethylamine (112 mg, 0.87 mmol) were dissolved in DCM (10 mL) and stirred at 25 °C for 18 h. The reaction mixture was then diluted with DCM (50 mL), and washed with aqueous NaHCO₃ solution (100 mL x 3). The organic layer was dried, filtered, and concentrated to give a crude residue, which was purified by Prep-TLC (silica gel, EtOAc:petroleum ether = 1:4) to afford the title product **(1m)** (40 mg, 66.7%) as a yellow solid. *m*/*z* (ESI, +ve ion) = 494.3 [M+H]⁺.

### Step N. (S)-2-((4-(6-(Dimethylamino)pyrimidine-4-carbonyl)piperazin-1-yl)methyl)-1-(oxetan-2-ylmethyl)-1H-benzo[d]imidazole-6-carboxylic acid (1)

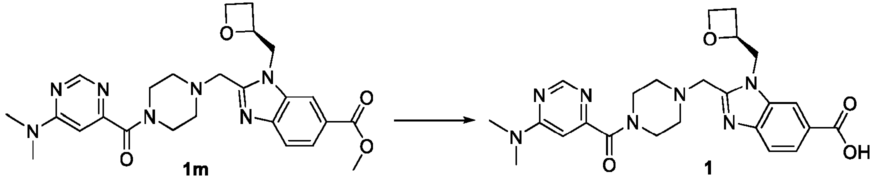

A mixture of **1m** (35 mg, 0.07 mmol), and 1,5,7-triazabicyclo[4.4.0]dec-5-ene (TBD) (77 mg, 0.24 mmol) in CH₃CN:H₂O = 1:1 (20 mL) was stirred at 50 °C for 2 h. The reaction mixture was then extracted with ethyl acetate (150 mL x 3). The combined organic layers were dried over Na₂SO₄, filtered, and concentrated. The crude residue was purified by reverse phase HPLC to give the title product **(1)** (15 mg, 34.2%) as a white solid. ¹H NMR (400 MHz, CDCl₃) δ ppm 8.57 (s, 1H), 8.20 (br s, 1H), 7.97 - 8.10 (m, 1H), 7.71 - 7.89 (m, 1H), 6.71 (s, 1H), 5.21 (br s, 1H), 4.47 - 4.74 (m, 3H), 4.24 - 4.46 (m, 1H), 4.03 (br d, *J =* 6.14 Hz, 2H), 3.79 (br s, 2H), 3.58 (br s, 2H), 3.14 (br s, 6H), 2.59 - 2.73 (m, 2H), 2.57 (br s, 2H), 2.34 - 2.50 (m, 2H). *m*/*z* (ESI, +ve ion) = 480.2 [M+H]⁺.

### Example 2. (S)-2-((4-(3-(2-Chloro-4-(trifluoromethyl)phenoxy)benzoyl)piperazin-1-yl)methyl)-1-(oxetan-2-ylmethyl)-1H-benzo[d]imidazole-6-carboxylic acid (2)

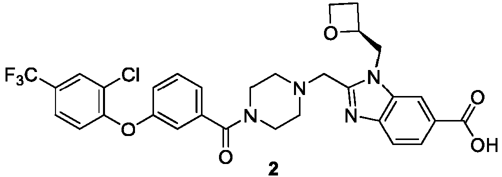

### Step A. (S)-1-(Oxetan-2-ylmethyl)-2-(piperazin-1-ylmethyl)-1H-benzo[d]imidazole-6-carboxylic acid (2a)

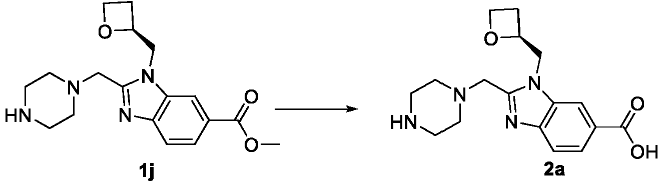

To a solution of **1j** (30 mg, 0.087 mmol) in CH₃CN (1 mL) and H₂O (1 mL) was added 1,5,7-triazabicyclo[4.4.0]dec-5-ene (24.4 mg, 0.17 mmol). The reaction mixture was stirred at 45 °C for 2 h. After completion, the reaction was adjusted to pH = 2 by 10% citric acid. The mixture was concentrated and purified by reverse phase HPLC (CH₃CN and H₂O, 0.5% formic acid as a modifier) to afford the title product **(2a)** (15 mg, 52%) as a white solid. *m*/*z* (ESI, +ve ion) = 331.3 [M+H]⁺.

### Step B. (S)-2-((4-(3-(2-Chloro-4-(trifluoromethyl)phenoxy)benzoyl)piperazin-1-yl)methyl)-1-(oxetan-2-ylmethyl)-1H-benzo|[d]imidazole-6-carboxylic acid (2)

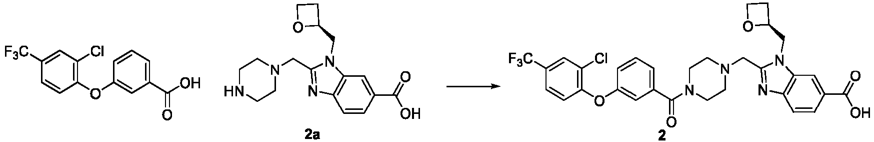

3-(2-Chloro-4-trifluoromethylphenoxy)benzoic acid (4.7 mg, 0.015 mmol) was mixed with HATU (4.6 mg, 0.012 mmol), and DIPEA (2.0 mg, 0.02 mmol) in DMF (0.3 mL) for 10 min at room temperature. Then **2a** (3.3 mg, 0.01 mmol) was added into the mixture and the reaction was stirred for 5 min. The crude mixture was purified by reverse phase HPLC (CH₃CN and water with 0.1% HOAc as a modifier) to give the title product **(2)** (1.2 mg, 17.4 %) as a white powder. ¹H NMR (600 MHz, CD₃OD) δ ppm 8.31 (s, 1H), 7.96 (dd, *J =* 8.44, 1.47 Hz, 1H), 7.87 (br d, *J* = 2.20 Hz, 1H), 7.66 (d, *J =* 8.80 Hz, 1H), 7.62 (br dd, *J =* 8.44, 1.83 Hz, 1H), 7.52 (br t, *J* = 7.89 Hz, 1H), 7.26 (br d, *J* = 7.70 Hz, 1H), 7.18 (d, *J =* 8.44 Hz, 1H), 7.14 (dd, *J =* 8.07, 1.83 Hz, 1H), 7.06 - 7.09 (m, 1H), 5.25 (qd, *J =* 7.34, 2.57 Hz, 1H), 4.86 - 4.87 (m, 1H), 4.68 - 4.73 (m, 1H), 4.63 (td, *J =* 7.89, 5.87 Hz, 1H), 4.44 (dt, *J =* 9.17, 5.87 Hz, 1H), 4.03 (br d, *J =* 13.57 Hz, 1H), 3.94 (br d, *J =* 13.94 Hz, 1H), 3.78 (br s, 2H), 3.44 - 3.53 (m, 2H), 2.77 - 2.83 (m, 1H), 2.58 - 2.70 (m, 2H), 2.46 - 2.55 (m, 3H). *m*/*z* (ESI, +ve ion) = 629.3 [M+H]⁺.

Example compounds **3-7, 13, 21,** 22, and **70** were synthesized as described in Example **2.**

### Example 10. (S)-2-((4-(3-(4-Cyano-2-fluorophenoxy)benzoyl)piperazin-1-yl)methyl)-1-(oxetan-2-ylmethyl)-1H-benzo[d]imidazole-6-carboxylic acid (10)

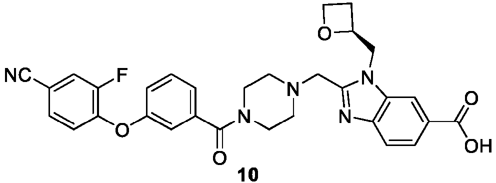

### Step A. Methyl 3-(4-cyano-2-fluorophenoxy)benzoate (10a)

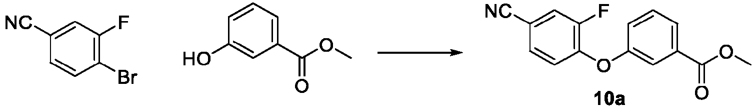

A flask was charged with methyl 3-hydroxybenzoate (17 mg, 0.11 mmol), and DMF (1 mL) was added followed by sodium hydride (60% in mineral oil, 4.4 mg, 0.11 mmol). The reaction was stirred for 30 min at room temperature. Then 4-bromo-3-fluorobenzonitrile (20 mg, 0.1 mmol), and CuBr (2.2 mg, 0.01 mmol) were added and the resulting mixture was heated up to 100 °C overnight. The crude mixture was purified by reverse phase HPLC (CH₃CN and water with 0.1% TFA as a modifier) to give the title product **(10a)** (1.6 mg, 5.9 %) as a white solid. *m*/*z* (ESI, +ve ion) = 272.1 [M+H]⁺.

### Step B. 3-(4-Cyano-2-fluorophenoxy)benzoic acid (10b)

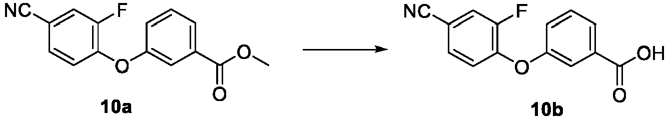

LiOH (2.9 mg, 0.12 mmol) was added to a THF:water (5:1) solution (1.2 mL) of **10a** (1.6 mg, 0.006 mmol), and stirred at room temperature for 5.5 h. The crude mixture was purified by reverse phase HPLC (CH₃CN and water with 0.1% TFA as a modifier) to give the title product (10b) (0.8 mg, 53.3 %) as a white solid. *m*/*z* (ESI, +ve ion) = 258.2 [M+H]⁺.

### Step C. (S)-2-((4-(3-(4-Cyano-2-fluorophenoxy)benzoyl)piperazin-1-yl)methyl)-1-(oxetan-2-ylmethyl)-1H-benzo[d]imidazole-6-carboxylic acid (10)

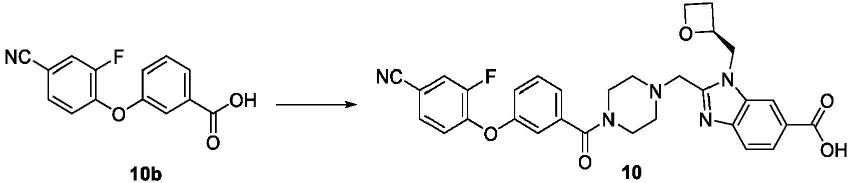

The title compound was prepared from **10b** by procedures similar to those described in Example **2,** Step B. *m*/*z* (ESI, +ve ion) = 570.2 [M+H]⁺.

Example compounds **11,** and **12** were synthesized as described in Example **10.**

### Example 14. (S)-1-(Oxetan-2-ylmethyl)-2-((4-(3-(o-tolyloxy)benzoyl)piperazin-1-yl)methyl)-1H-benzo[d]imidazole-6-carboxylic acid (14)

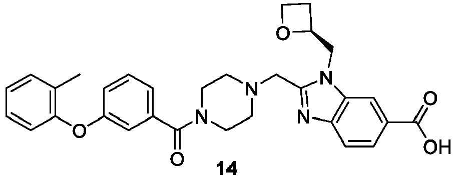

### Step A. Methyl 3-(o-tolyloxy)benzoate (14a)

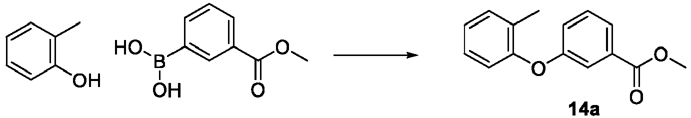

o-Cresol (10.8 mg, 0.1 mmol), (3-(methoxycarbonyl)phenyl)boronic acid (36 mg, 0.2 mmol), Cu(OAc)₂ (36.3 mg, 0.2 mmol), and molecular sieves were mixed in dry DCM (4 mL). Et₃N (20.2 mg, 0.2 mmol) was added, and the mixture was stirred overnight. Then the solvent was removed *in vacuo,* and the residue was purified by reverse phase HPLC (CH₃CN and water with 0.1% TFA as a modifier) to provide the title product **(14a).** *m*/*z* (ESI, +ve ion) = 243.1 [M+H]⁺.

### Step B. 3-(o-Tolyloxy)benzoic acid (14b)

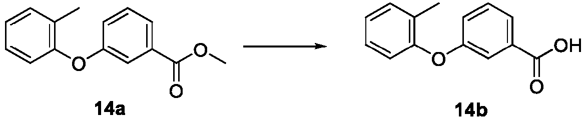

The title compound **14b** was prepared from **14a** by procedures similar to those described in Example **10,** Step B.

### Step C. (S)-1-(Oxetan-2-ylmethyl)-2-((4-(3-(o-tolyloxy)benzoyl)piperazin-1-yl)methyl)-1H-benzo[d]imidazole-6-carboxylic acid (14)

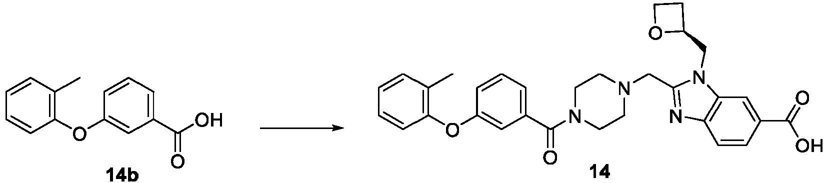

The title compound was prepared from **14b** by procedures similar to those described in Example **2,** Step B. ¹H NMR (600 MHz, CD₃OD) δ ppm 8.30 (s, 1H), 7.96 (br d, *J =* 8.44 Hz, 1H), 7.65 (d, *J =* 8.80 Hz, 1H), 7.40 (t, *J =* 7.89 Hz, 1H), 7.30 (br d, *J =* 7.34 Hz, 1H), 7.21 (br t, *J =* 7.52 Hz, 1H), 7.10 - 7.16 (m, 1H), 7.08 (d, *J =* 7.34 Hz, 1H), 6.95 - 7.00 (m, 1H), 6.93 (d, *J =* 8.07 Hz, 1H), 6.81 - 6.88 (m, 1H), 5.24 (qd, *J =* 7.15, 2.75 Hz, 1H), 4.86 - 4.87 (m, 1H), 4.67 - 4.73 (m, 1H), 4.59 - 4.67 (m, 1H), 4.44 (dt, *J =* 8.99, 6.14 Hz, 1H), 4.02 (d, *J=* 13.94 Hz, 1H), 3.94 (d, *J =* 13.57 Hz, 1H), 3.76 (br s, 2H), 3.38 - 3.49 (m, 2H), 2.76 - 2.83 (m, 1H), 2.57 - 2.65 (m, 2H), 2.44 - 2.57 (m, 3H), 2.18 (s, 3H). *m*/*z* (ESI, +ve ion) = 541.4 [M+H]⁺.

Example compounds **15, 16, 17, 18, 23,** and **57** were synthesized as described in Example **14.**

### Example 19. (S)-2-((4-(5-((3-Chloro-2-methylphenoxy)methyl)furan-2-carbonyl)piperazin-1-yl)methyl)-1-(oxetan-2-ylmethyl)-1H-benzo[d]imidazole-6-carboxylic acid (19)

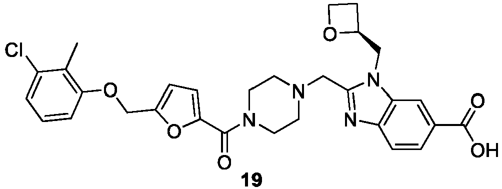

### Step A. Ethyl 5-((3-chloro-2-methylphenoxy)methyl)furan-2-carboxylate (19a)

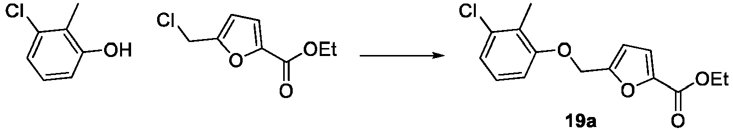

A flask was charged with ethyl 5-(chloromethyl)furan-2-carboxylate (18 mg, 0.1 mmol), ethyl 5-(chloromethyl)furan-2-carboxylate (16 mg, 0.12 mmol), K₂CO₃ (32 mg, 0.24 mmol), and DMF (1 mL). The suspension was stirred overnight and purified by reverse phase HPLC (CH₃CN and water with 0.1% HOAc as a modifier) to provide the title product **(19a)** (28 mg, 98%) as a white solid. *m*/*z* (ESI, +ve ion) = 295.0 [M+H]⁺.

### Step B. 5-((3-Chloro-2-methylphenoxy)methyl)furan-2-carboxylic acid (19b)

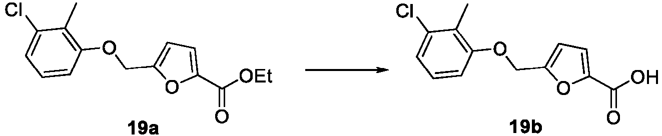

The title compound was prepared from **19a** by procedures similar to those described in Example **59,** Step C.

### Step C. (S)-2-((4-(5-((3-Chloro-2-methylphenoxy)methyl)furan-2-carbonyl)piperazin-1-yl)methyl)-1-(oxetan-2-ylmethyl)-1H-benzo[d]imidazole-6-carboxylic acid (19)

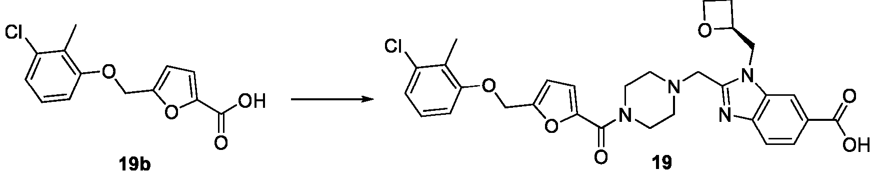

The title compound was prepared from **19b** by procedures similar to those described in Example **2,** Step B. ¹H NMR (600 MHz, CD₃OD) δ ppm 8.26 (s, 1H), 7.97 (dd, *J* = 8.44, 1.10 Hz, 1H), 7.64 (d, *J* = 8.44 Hz, 1H), 7.08 - 7.13 (m, 1H), 6.97 - 7.04 (m, 2H), 6.94 (d, *J =* 8.07 Hz, 1H), 6.62 (d, *J =* 3.30 Hz, 1H), 5.26 (qd, *J =* 7.15, 2.75 Hz, 1H), 5.14 (s, 2H), 4.86 - 4.89 (m, 1H), 4.69 - 4.75 (m, 1H), 4.63 (td, *J =* 7.89, 5.87 Hz, 1H), 4.45 (dt, *J =* 9.17, 5.87 Hz, 1H), 4.03 (d, *J =* 13.57 Hz, 1H), 3.95 (d, *J =* 13.57 Hz, 1H), 3.76 (br s, 4H), 2.77 - 2.83 (m, 1H), 2.48 - 2.64 (m, 5H), 2.20 (s, 3H). *m*/*z* (ESI, +ve ion) = 579.2 [M+H]⁺.

Example compounds **24, 26, 30, 31, 41, 42, 43, 47, 49, 55, 64-69, 71,** and **81** were synthesized similar to as described in Example **19.**

### Example 20. (S)-1-(Oxetan-2-ylmethyl)-2-((4-((5-(phenoxymethyl)furan-2-yl)methyl)piperazin-1-yl)methyl)-1H-benzo[d]imidazole-6-carboxylic acid (20)

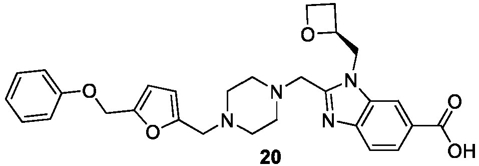

### Step A. 5-(Phenoxymethyl)furan-2-carbaldehyde (20a)

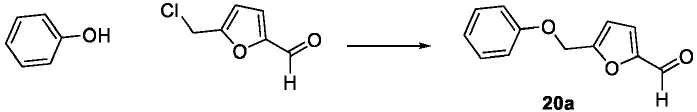

The title compound was made from phenol and 5-(chloromethyl)furan-2-carbaldehyde by procedures similar to those described in Example **19,** Step A.

### Step B. (S)-1-(Oxetan-2-ylmethyl)-2-((4-((5-(phenoxymethyl)furan-2-yl)methyl)piperazin-1-yl)methyl)-1H-benzo[d]imidazole-6-carboxylic acid (20)

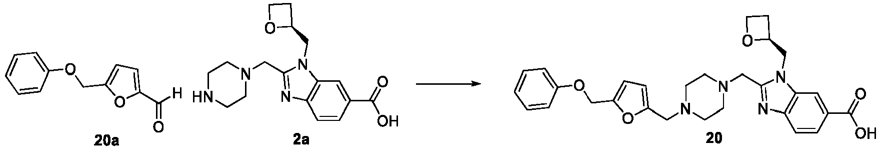

To a methoxyethanol (1 mL) solution of 5-(phenoxymethyl)furan-2-carbaldehyde (2.0 mg, 0.01 mmol), and **2a** (1.7 mg, 0.005 mmol) was added 5-ethyl-2-methylpyridine borane (1.1 µL, 0.0075 mmol), and HOAc (0.4 µL, 0.0075 mmol). The mixture was stirred at 60 °C overnight, and subsequently cooled down. The crude reaction mixture was purified by reverse phase HPLC (CH₃CN and water with 0.1% HOAc as a modifier) to provide the title product **(20)** (2.4 mg, 75%) as a white solid. ¹H NMR (600 MHz, CD₃OD) δ ppm 8.27 - 8.35 (m, 1H), 7.96 (dd, *J =* 8.44, 1.47 Hz, 1H), 7.66 (d, *J =* 8.44 Hz, 1H), 7.20 - 7.26 (m, 2H), 6.93 - 6.98 (m, 2H), 6.87 - 6.91 (m, 1H), 6.45 (br d, *J =* 2.93 Hz, 1H), 6.36 (br s, 1H), 5.21 (qd, *J =* 7.21, 2.57 Hz, 1H), 5.01 (s, 1H), 4.78 - 4.82 (m, 2H), 4.66 (br dd, *J =* 15.41, 2.57 Hz, 1H), 4.61 (td, *J* = 7.89, 5.87 Hz, 1H), 4.43 (dt, *J* = 9.17, 6.05 Hz, 1H), 4.01 (d, *J =* 13.94 Hz, 1H), 3.91 (br d, *J =* 13.94 Hz, 1H), 3.72 - 3.78 (m, 2H), 2.76 (dtd, *J =* 11.32, 8.09, 8.09, 6.24 Hz, 1H), 2.54 - 2.72 (m, 8H), 2.44 - 2.52 (m, 1H). *m*/*z* (ESI, +ve ion) = 517.2 [M+H]⁺.

Example compounds **8, 9, 35,** and **63** were synthesized as described in Example **20.**

### Example 27. (S)-2-((4-((6-(2,4-Dichlorophenyl)benzofuran-2-yl)methyl)piperazin-1-yl)methyl)-1-(oxetan-2-ylmethyl)-1H-benzo[d]imidazole-6-carboxylic acid (27)

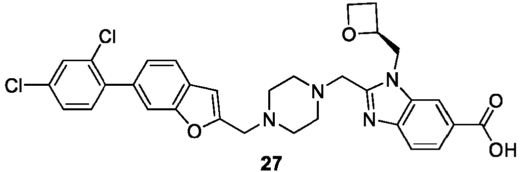

### Step A. Ethyl 6-(2,4-dichlorophenyl)benzofuran-2-carboxylate (27a)

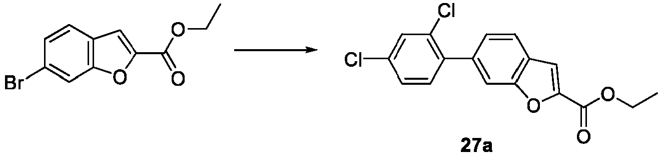

Under a nitrogen atmosphere, [1,1'-Bis(diphenylphosphino)ferrocene]palladium(II) chloride complex with dichloromethane (1:1) (1.52 g, 1.8 mmol), (2,4-dichlorophenyl)boronic acid (2.13 g, 11.1 mmol), and ethyl 6-bromo-1-benzofuran-2-carboxylate (2.5 g, 9.3 mmol) were added to a mixture of 1,4-dioxane (24 mL), K₂CO₃ (3.86 g, 27.8 mmol), and water (12 mL). The reaction was heated to 80 °C, stirred for one hour, and quenched with water (50 mL). The mixture was extracted with dichloromethane (50 mL, x2). The combined organic layers were dried over Na₂SO₄, filtered, and concentrated. The crude residue was purified by silica gel column chromatography (n-pentane:EtOAc = 95:5) to afford the title product **(27a)** as a white solid (1.5 g, 43%). *m*/*z* (ESI, +ve ion) = 357.0 [M+Na]⁺.

### Step B. (6-(2,4-Dichlorophenyl)benzofuran-2-yl)methanol (27b)

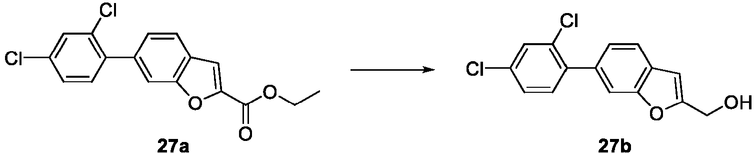

A flask was charged with LiAlH₄ (0.2 g, 5.1 mmol), and THF (30 mL). Then **27a** (1.5 g, 4.7 mmol) in 10 mL THF was added dropwise at 20 °C. The reaction mixture was stirred at 70 °C for 3 h, then quenched by adding ethyl acetate (5 mL) slowly at 0 °C. After removing the solvents, the crude residue was diluted with water (40 mL), and extracted with ethyl acetate (40 mL x 3). The organic phase was washed with water (20 mL), brine (20 mL), dried with anhydrous sodium sulfate, filtered, concentrated, and purified by silica gel column chromatography (gradient elution, EtOAc in petroleum ether 0-20%) to afford the title product **(27b)** as a white solid (386.1 mg, 27.8%). *m*/*z* (ESI, +ve ion) = 275.0 [M-OH]⁺.

### Step C. 2-(Bromomethyl)-6-(2,4-dichlorophenyl)benzofuran (27c)

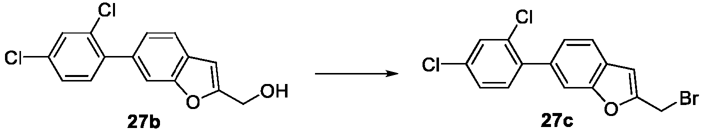

To a stirred solution of **27b** (50 mg, 0.15 mmol) in dichloromethane (1.5 mL) was added phosphorus tribromide (73 mg, 0.27 mmol). The mixture was stirred at 0 °C for one hour and diluted with sat. aq. NaHCO₃ (2 mL). The reaction mixture was then extracted with DCM (3 mL x 3), and washed with brine (3 mL). The organic phase was dried with anhydrous sodium sulfate, filtered, and concentrated. The crude residue was purified by silica gel column chromatography (gradient elution, EtOAc in petroleum ether 0-10%) to afford the title product **(27c)** (53 mg, 81.6 %) as a yellow oil.

### Step D. Methyl (S)-2-((4-((6-(2,4-dichlorophenyl)benzofuran-2-yl)methyl)piperazin-1-yl) methyl)-1-(oxetan-2-ylmethyl)-1H-benzo[d]imidazole-6-carboxylate (27d)

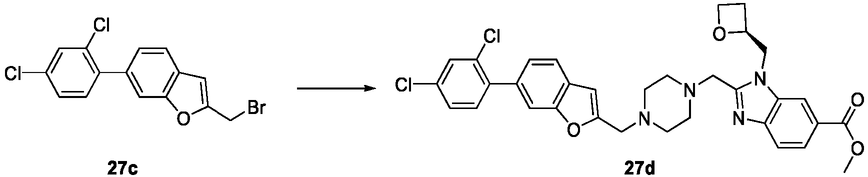

**27c** (20 mg, 0.056 mmol) was added to a solution of **1j** (19 mg, 0.056 mmol), and potassium carbonate (31 mg, 0.225 mmol,) in DMF (0.5 ml), and stirred for 24 h. The reaction was then diluted with water (2 mL), and extracted with EtOAc (2 mL x 2). The organic phase was washed with saturated sodium bicarbonate solution (2 mL), dried over magnesium sulfate, filtered, and concentrated under reduced pressure. The crude mixture was purified by silica gel column chromatography (gradient elution, MeOH in dichloromethane 0-20%) to afford the title product **(27d)** (17 mg, 44.0%) as a yellow oil. *m*/*z* (ESI, +ve ion) = 619.2 [M+H]⁺.

### Step E. (S)-2-((4-((6-(2,4-Dichlorophenyl)benzofuran-2-yl)methyl)piperazin-1-yl)methyl)-1-(oxetan-2-ylmethyl)-1H-benzo[d]imidazole-6-carboxylic acid (27)

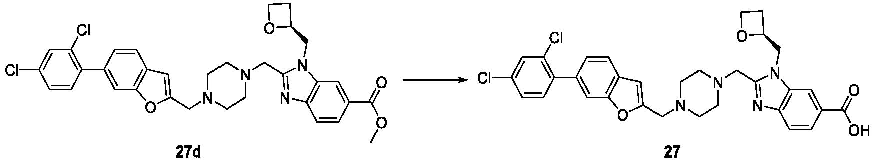

To a mixture of **27d** (17 mg, 0.028 mmol) in acetonitrile (0.5 mL) and water (0.5 mL) was added 1,3,4,6,7,8-hexahydro-2H-pyrimido[1,2-a]pyrimidine (8 mg, 0.056 mmol). The reaction was stirred at 20 °C for 16 h. The mixture was concentrated and purified by reverse phase HPLC (CH₃CN and H₂O with 0.05% NH₄HCO₃ as modifier, gradient elution 0-80%) to afford the title product **27** (3.3 mg, 18.6%) as a white solid. ¹H NMR (400 MHz, CDCl₃) δ ppm 8.18 (s, 1H), 8.01 (d, *J =* 8.5 Hz, 1H), 7.78 (d, *J =* 8.4 Hz, 1H), 7.56 (d, *J =* 8.0 Hz, 1H), 7.51 (d, *J =* 5.5 Hz, 2H), 7.31 (s, 2H), 7.25 (s, 1H), 6.64 (s, 1H), 5.23 - 5.16 (m, 1H), 4.72 - 4.57 (m, 3H), 4.39 - 4.34 (m, 1H), 3.98 (s, 2H), 3.75 (s, 2H), 2.78 - 2.58 (m, 8H), 2.48 - 2.37 (m, 2H). *m*/*z* (ESI, +ve ion) = 605.1 [M+H]⁺.

Example compound **28** was synthesized as described in Example **27.**

### Example 32. (S)-2-((4-(3-(2-Chloro-4-methylbenzyl)benzoyl)piperazin-1-yl)methyl)-1-(oxetan-2-ylmethyl)-1H-benzo[d]imidazole-6-carboxylic acid (32)

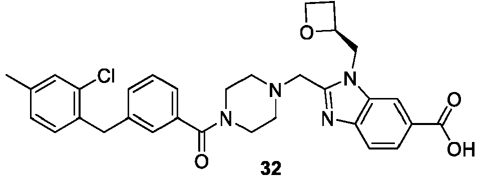

The title compound was made from methyl 3-(bromomethyl)benzoate and (2-chloro-4-methylphenyl)boronic acid by procedures similar to those described in Example **59,** Steps B, and C, followed by procedures similar to those described in Example **2,** Step B. ¹H NMR (600 MHz, CD₃OD) δ ppm 8.29 - 8.33 (m, 1H), 7.94 - 8.00 (m, 1H), 7.63 - 7.69 (m, 1H), 7.34 - 7.40 (m, 1H), 7.28 - 7.33 (m, 1H), 7.20 - 7.26 (m, 2H), 7.14 - 7.20 (m, 2H), 7.05 - 7.10 (m, 1H), 5.24 (qd, *J =* 7.34, 2.57 Hz, 1H), 4.86 - 4.87 (m, 1H), 4.67 - 4.74 (m, 1H), 4.63 (td, *J* = 7.89, 5.87 Hz, 1H), 4.44 (dt, *J =* 9.26, 6.01 Hz, 1H), 4.10 (s, 2H), 4.03 (br d, *J =* 13.94 Hz, 1H), 3.94 (br d, *J =* 13.94 Hz, 1H), 3.70 - 3.82 (m, 2H), 3.36 - 3.45 (m, 2H), 2.80 (dtd, *J =* 11.23, 8.14, 8.14, 6.24 Hz, 1H), 2.57 - 2.68 (m, 2H), 2.41 - 2.57 (m, 3H), 2.26 - 2.30 (s, 3H). *m*/*z* (ESI, +ve ion) = 573.2 [M+H]⁺.

Example compounds **33, 48,** and **56** were synthesized as described in Example **32.**

### Example 34. (S)-1-(Oxetan-2-ylmethyl)-2-((4-(6-(p-tolyloxy)pyrimidine-4-carbonyl)piperazin-1-yl)methyl)-1H-benzo[d]imidazole-6-carboxylic acid (34)

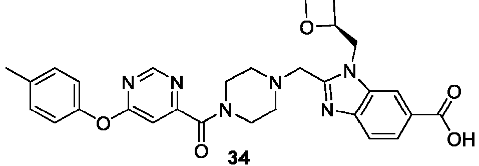

### Step A. Methyl 6-(p-tolyloxy)pyrimidine-4-carboxylate (34a)

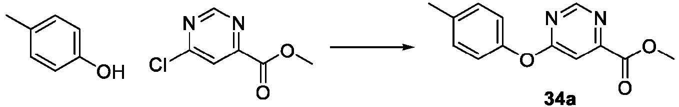

Methyl 6-chloropyrimidine-4-carboxylate (1 g, 5.81 mmol), *p*-cresol (0.94 g, 8.71 mmol), and Cs₂CO₃ (1.94 g, 8.71 mmol) were added successively to DMSO (20 mL). The reaction mixture was stirred at 25 °C for 3 h. After completion, the mixture was quenched by water (20 mL), and extracted with EtOAc (20 mL x 3). The organic layers were combined, dried, filtered, and concentrated to afford a crude residue, which was purified by silica gel column chromatography (petroleum ether:EtOAc = 1:1) to give the title product **(34a)** (0.76 g, 53%) as a white solid. *m*/*z* (ESI, +ve ion) = 245.1 [M+H]⁺.

### Step B. 6-(p-Tolyloxy)pyrimidine-4-carboxylic acid (34b)

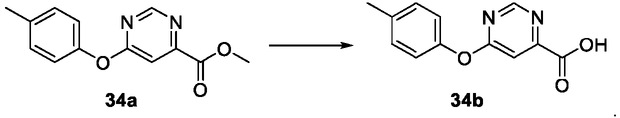

**34a** (1.1 g, 4.5 mmol) was dissolved in a mixed solvent of MeOH:H₂O (20 mL:7 mL). Sodium hydroxide (1.8 g, 45 mmol) was added, and the reaction was stirred at 25 °C for 6 h. After completion, the reaction was quenched by water (20 mL), and the mixture was adjusted to pH = 6.0 by 10% citric acid, and extracted with EtOAc (20 mL x 3). The organic layer was dried, filtered, and concentrated to afford a crude residue, which was purified by silica gel column chromatography (petroleum:EtOAc = 1:2) to give the title product **34b** (830 mg, 80%) as a white solid. *m*/*z* (ESI, +ve ion) = 231.1 [M+H]⁺.

### Step C. (S)-1-(Oxetan-2-ylmethyl)-2-((4-(6-(p-tolyloxy)pyrimidine-4-carbonyl)piperazin-1-yl)methyl)-1H-benzo[d]imidazole-6-carboxylic acid (34)

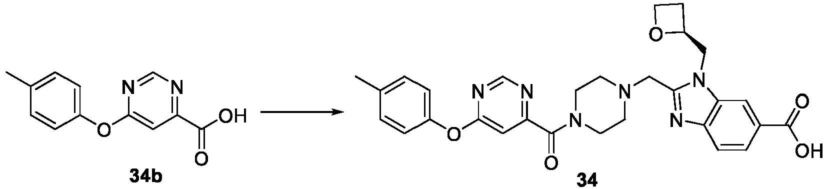

The title compound was prepared from **34b** by procedures similar to those described in Example **2,** Step B. ¹H NMR (600 MHz, CD₃OD) δ ppm 8.71 (d, *J =* 0.73 Hz, 1H), 8.30 - 8.34 (m, 1H), 7.96 (dd, *J* = 8.44, 1.47 Hz, 1H), 7.66 (d, *J =* 8.44 Hz, 1H), 7.27 (d, *J =* 8.44 Hz, 2H), 7.09 - 7.14 (m, 1H), 7.03 - 7.09 (m, 2H), 5.25 (qd, *J =* 7.21, 2.57 Hz, 1H), 4.85 - 4.89 (m, 1H), 4.68 - 4.75 (m, 1H), 4.63 (td, *J =* 7.89, 5.87 Hz, 1H), 4.44 (dt, *J =* 9.17, 5.87 Hz, 1H), 4.04 (br d, *J =* 13.57 Hz, 1H), 3.96 (br d, *J =* 13.94 Hz, 1H), 3.75 - 3.83 (m, 2H), 3.50 (br dd, *J =* 6.05, 3.85 Hz, 2H), 2.76 - 2.84 (m, 1H), 2.61 - 2.71 (m, 2H), 2.53 - 2.60 (m, 2H), 2.45 - 2.53 (m, 1H), 2.37 (s, 3H). *m*/*z* (ESI, +ve ion) = 543.3 [M+H]⁺.

### Example 36. (S)-2-((4-(5-((4-Chloro-2-fluorophenoxy)methyl)furan-2-carbonyl)-piperazin-1-yl)methyl)-1-(oxetan-2-ylmethyl)-1H-benzo[d]imidazole-6-carboxylic acid (36)

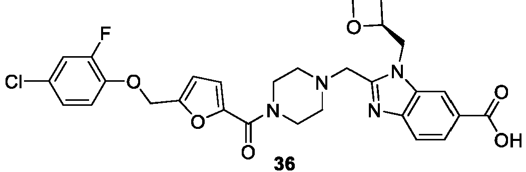

### Step A. (S)-2-((4-(5-(Chloromethyl)furan-2-carbonyl)piperazin-1-yl)methyl)-1-(oxetan-2-ylmethyl)-1H-benzo[d]imidazole-6-carboxylic acid (36a)

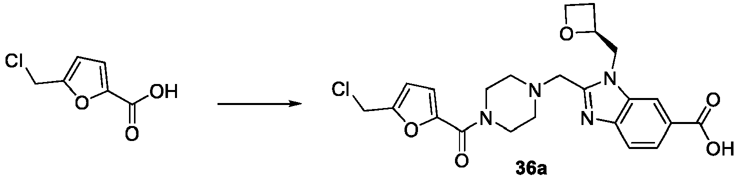

The title compound was prepared from 5-(chloromethyl)furan-2-carboxylic acid and **2a** by procedures similar to those described in Example 2, Step B.

### Step B. (S)-2-((4-(5-((4-chloro-2-fluorophenoxy)methyl)furan-2-carbonyl)piperazin-1-yl)methyl)-1-(oxetan-2-ylmethyl)-1H-benzo[d]imidazole-6-carboxylic acid (36)

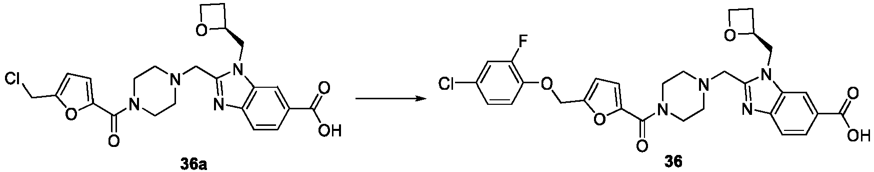

4-Chloro-2-fluorophenol (1.1 mg, 0.008 mmol), and **36a** (3.2 mg, 0.007 mmol) were treated with K₂CO₃ (2.2 mg, 0.016 mmol) in DMF (0.5 mL). The reaction was stirred for 4 h at room temperature. The crude mixture was purified by reverse phase HPLC (CH₃CN and water with 0.1% HOAc as a modifier) to provide the title product **(36)** (0.4 mg, 8.9 %) as a white solid. ¹H NMR (600 MHz, CD₃OD) δ ppm 8.35 (s, 1H), 7.98 (br d, *J =* 8.44 Hz, 1H), 7.69 (d, *J=* 8.44 Hz, 1H), 7.13 - 7.22 (m, 2H), 7.09 (ddd, *J =* 8.80, 2.57, 1.47 Hz, 1H), 6.98 - 7.02 (m, 1H), 6.64 (d, *J =* 3.30 Hz, 1H), 5.26 (qd, *J =* 7.21, 2.57 Hz, 1H), 5.17 (s, 2H), 4.86 - 4.90 (m, 1H), 4.73 (br dd, *J =* 15.41, 2.57 Hz, 1H), 4.60 - 4.69 (m, 1H), 4.46 (dt, *J =* 9.17*,* 5.87 Hz, 1H), 4.06 (br d, *J =* 13.94 Hz, 1H), 3.97 (br d, *J =* 13.94 Hz, 1H), 3.76 (br s, 4H), 2.81 (m, 1H), 2.53 - 2.68 (br s, 4H), 2.52 (m, 1H). *m*/*z* (ESI, +ve ion) = 583.2 [M+H]⁺.

Example compounds **37, 38, 39,** and **40** were synthesized as described in Example **36.**

### Example 44. (S)-2-((4-(7-Methyl-9-oxo-9H-xanthene-3-carbonyl)piperazin-1-yl)methyl)-1-(oxetan-2-ylmethyl)-1H-benzo[d]imidazole-6-carboxylic acid (44)

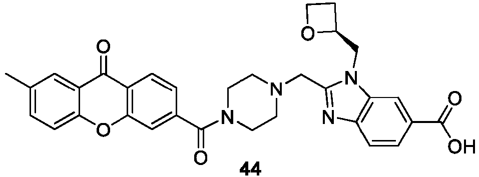

### Step A. Dimethyl 2-fluoroterephthalate (44a)

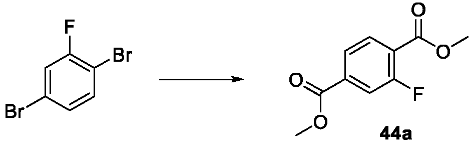

To a mixture of 1,4-dibromo-2-fluorobenzene (1.0 g, 3.9 mmol) in MeOH:DMSO (20 mL, 1:1) was added Pd(OAc)₂ (253 mg, 0.78mmol), DPPP (321 mg, 0.78 mmol), and TEA (5.4 mL, 39 mmol). The mixture was stirred at 85 °C for 16 h under a CO balloon. After completion, the reaction was quenched with water (100 mL), and extracted with EtOAc (50 mL x 3). The organic layers were combined, dried, filtered, and concentrated to afford a crude residue, which was purified by silica gel column chromatography (petroleum ether:EtOAc = 5:1) to give the title product **(44a)** (500 mg, 58 %) as a brown solid. ¹H NMR (400 MHz, CDCl₃) δ ppm 8.00 - 7.96 (m, 1H), 7.85 (dd, *J =* 8.1, 1.5 Hz, 1H), 7.78 (dd, *J=* 11.0, 1.5 Hz, 1H), 3.94 (s, 3H), 3.93 (s, 3H).

### Step B. Dimethyl 2-(p-tolyloxy)terephthalate (44b)

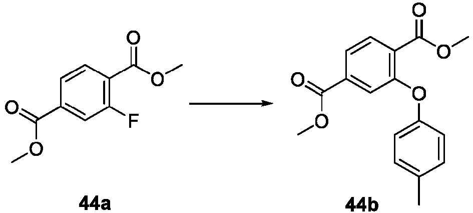

To a mixture of **44a** (600 mg, 2.8 mmol) in DMF (30 mL) was added *p*-cresol (458 mg, 4.2 mmol), and K₂CO₃ (1.1 g, 8.4 mmol). The mixture was stirred at 85 °C for 2 h. After completion, the reaction was quenched with water (50 mL), and extracted with EtOAc (50 mL x 3). The organic layers were combined, dried, filtered, and concentrated to afford a crude residue, which was purified by silica gel column chromatography (petroleum ether:EtOAc = 5:1) to provide the title product **(44b)** (400 mg, 47 %) as a white solid. *m*/*z* (ESI, +ve ion) = 301.1 [M+H]⁺.

### Step C. Methyl 7-methyl-9-oxo-9H-xanthene-3-carboxylate (44c)

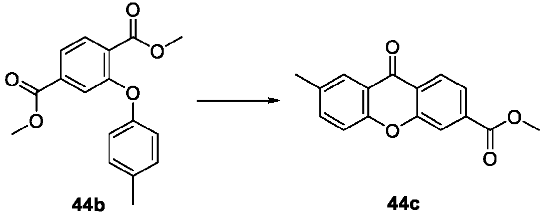

A mixture of **44b** ( 0.5 g, 1.665 mmol) in PPA (30 mL) was stirred at 125 °C for 2 h. After completion, the reaction was quenched with water (30 mL), washed with sat. aq. Na₂CO₃ solution, and extracted with EtOAc (20 mL x 3). The organic layers were combined, dried, filtered, and concentrated to afford the crude title product **(44c)** (205 mg) as a white solid. *m*/*z* (ESI, +ve ion) = 269.1 [M+H]⁺.

### Step D. 7-Methyl-9-oxo-9H-xanthene-3-carboxylic acid (44d)

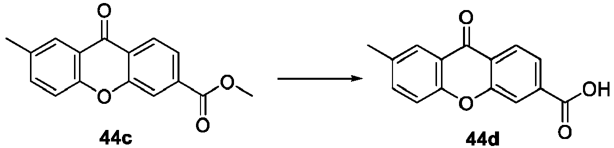

To a mixture of **44c** (250 mg, 0.93 mmol) in MeOH:H₂O (6 mL:2 mL) was added NaOH (373 mg, 9.31 mmol). The mixture was stirred at 20 °C for 5 h. After completion, the reaction was quenched with water (30 mL). Then the mixture was adjusted to pH = 6 with 10% citric acid, and extracted with EtOAc (30 mL x 3). The organic layers were combined, dried, filtered, and concentrated to afford a crude residue, which was purified by silica gel column chromatography (petroleum ether: EtOAc =1:2) to afford the title product **(44d)** (180 mg, 76%) as a white solid. *m*/*z* (ESI, +ve ion) = 255.0 [M+H]⁺.

### Step E. (S)-2-((4-(7-Methyl-9-oxo-9H-xanthene-3-carbonyl)piperazin-1-yl)methyl)-1-(oxetan-2-ylmethyl)-1H-benzo[d]imidazole-6-carboxylic acid (44)

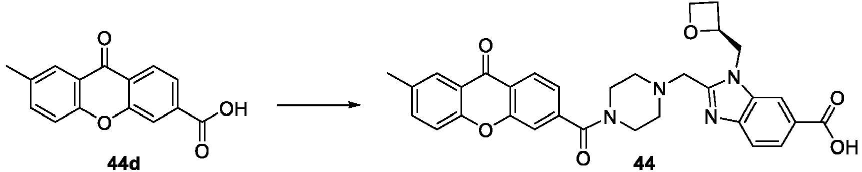

The title compound was prepared from **44d,** and **2a** by procedures similar to those described in Example **2,** Step B. ¹H NMR (600 MHz, CD₃OD) δ ppm 8.35 (d, *J =* 8.07 Hz, 1H), 8.30 (s, 1H), 8.06 - 8.09 (m, 1H), 7.93 - 7.97 (m, 1H), 7.70 (br dd, *J =* 8.44, 2.20 Hz, 1H), 7.64 (br d, *J =* 8.44 Hz, 2H), 7.54 (d, *J =* 8.44 Hz, 1H), 7.44 - 7.47 (m, 1H), 5.25 (qd, *J =* 7.34, 2.57 Hz, 1H), 4.84-4.90 (m, 1H), 4.68 - 4.76 (m, 1H), 4.63 (td, *J =* 7.89, 5.87 Hz, 1H), 4.45 (dt, *J =* 9.17*,* 5.87 Hz, 1H), 4.06 (br d, *J =* 13.57 Hz, 1H), 3.98 (br d, *J =* 13.94 Hz, 1H), 3.85 (br s, 2H), 3.48 (br s, 2H), 2.77 - 2.84 (m, 1H), 2.62 - 2.74 (m, 2H), 2.51 - 2.57 (m, 3H), 2.49 (s, 3H). *m*/*z* (ESI, +ve ion) = 567.2 [M+H]⁺.

### Example 46. 1-((1-Ethyl-1H-imidazol-5-yl)methyl)-2-((4-(3-(methyl(p-tolyl)amino)benzoyl)piperazin-1-yl)methyl)-1H-benzo[d]imidazole-6-carboxylic acid (46)

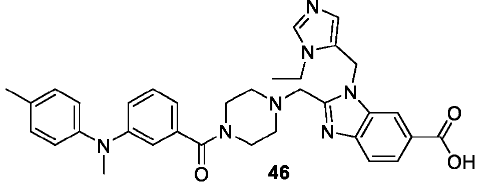

### Step A. Methyl 3-(p-tolylamino)benzoate (46a)

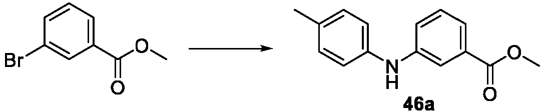

To a mixture of methyl 3-bromobenzoate (3 g, 13.9 mmol), 4-methylaniline (2.2 g, 20.85 mmol), palladium diacetate (0.62 g, 2.78 mmol), BINAP (1.73 g, 2.78 mmol), and Cs₂CO₃ (9 g, 27.8 mmol) was added toluene (50 mL). The reaction mixture was stirred at 100 °C for 3 h. After completion, the mixture was quenched with water (50 mL), and extracted with EtOAc (50 mL x 3). The organic layer was dried, filtered, and concentrated to afford a crude residue, which was purified by silica gel column chromatography (petroleum ether: EtOAc = 20:1) to provide the title product **(46a)** (2.5 g, 80%) as a yellow solid. *m*/*z* (ESI, +ve ion) = 242.1 [M+H]⁺.

### Step B. Methyl 3-(methyl(p-tolyl)amino)benzoate (46b)

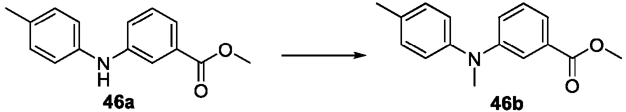

**46a** (180 mg, 0.75 mmol) was dissolved in THF (3 mL), and cooled down in an ice bath. NaH (60% in mineral oil, 60 mg, 1.49 mmol) was added to the reaction mixture, and was stirred at 0 °C for 0.5 h. Then MeI (210 mg, 1.49 mmol) was added. The reaction temperature was raised to room temperature and stirred for 3 h. After completion, the mixture was quenched with water (3 mL), and extracted with EtOAc (3 mL x 3). The organic layer was dried, filtered, and concentrated to afford a crude residue, which was purified by silica gel column chromatography (petroleum ether) to give the title product **(46b)** (158 mg, 85%) as a white solid. *m*/*z* (ESI, +ve ion) = 256.0 [M+H]⁺.

### Step C. 3-(Methyl(p-tolyl)amino)benzoic acid (46c)

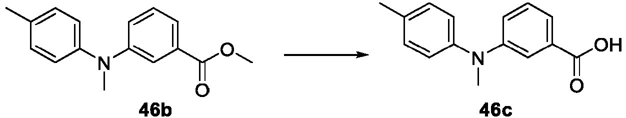

To a mixture of **46b** (500 mg, 1.9 mmol), and sodium hydroxide (2 g, 50 mmol) were added MeOH:H₂O(10 mL:5 mL). The reaction mixture was stirred at room temperature for 5 h. After completion, the mixture was quenched with water (5 mL), and extracted with EtOAc (5 mL x 3). The organic layers were combined, dried, filtered, and concentrated to afford a crude product, which was purified by silica gel column chromatography (petroleum ether:EtOAc = 2:1) to provide the title product **(46c)** (366 mg, 80%) as a white solid. *m*/*z* (ESI, +ve ion) = 242.2 [M+H]⁺.

### Step D. 1-((1-Ethyl-1H-imidazol-5-yl)methyl)-2-((4-(3-(methyl(p-tolyl)amino)benzoyl)piperazin-1-yl)methyl)-1H-benzo[d]imidazole-6-carboxylic acid (46)

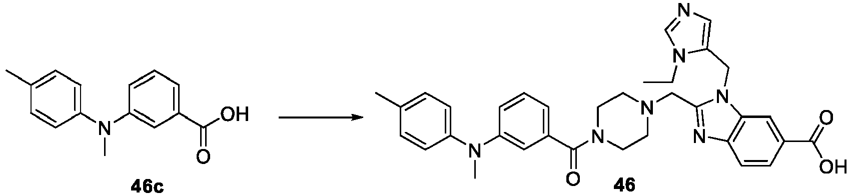

The title compound was prepared from **46c,** and **57i** by procedures similar to those described in Example **2,** Step B. ¹H NMR (600 MHz, CD₃OD) δ ppm 8.16 (d, *J =* 0.73 Hz, 1H), 8.01 (br dd, *J =* 8.44, 1.47 Hz, 1H), 7.79 (s, 1H), 7.72 (br d, *J =* 8.44 Hz, 1H), 7.25 (br t, *J =* 8.07 Hz, 1H), 7.20 (br d, *J =* 8.07 Hz, 2H), 7.03 - 7.07 (m, 2H), 6.88 - 6.93 (m, 1H), 6.73 - 6.77 (m, 2H), 6.57 (s, 1H), 5.78 (s, 2H), 4.09 (q, *J =* 7.34 Hz, 2H), 3.87 - 3.89 (m, 2H), 3.60 (br s, 2H), 3.31 - 3.35 (m, 2H), 3.28 (s, 3H), 2.56 (br s, 2H), 2.39 (br s, 2H), 2.28 - 2.33 (m, 3H), 1.27 - 1.34 (m, 3H). *m*/*z* (ESI, +ve ion) = 592.4 [M+H]⁺.

Example compounds **25, 29, 45, 50*,* 51*,* 52, 54, 58, 75, 76, 85,** and **87** (last 2 steps follow Example 102, Step D, and C), and **88** were synthesized similar to as described in Example **46.**

### Example 57. 2-((4-(3-(2-Chloro-4-methylphenoxy)benzoyl)piperazin-1-yl)methyl)-1-((1-ethyl-1H-imidazol-5-yl)methyl)-1H-benzo[d]imidazole-6-carboxylic acid (57)

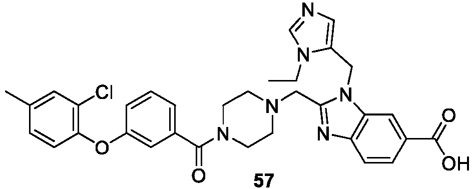

### Step A. Ethyl 1-ethyl-1H-imidazole-5-carboxylate (57a)

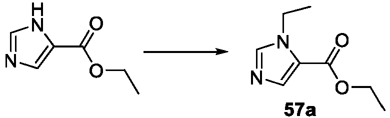

To a solution of ethyl 1*H*-imidazole-5-carboxylate (2.8 g, 20.0 mmol) in DMF (10 mL) was added NaH (60% in mineral oil, 1.2 g, 30.0 mmol) at 0 °C. After stirring at the same temperature for 0.5 h, iodoethane was added (3.12 g, 20.0 mmol), and the resulting mixture was stirred at 0 °C for another 0.5 h. After completion, the mixture was quenched with H₂O (30 mL), and extracted with EtOAc (30 mL x 3). The organic layer was washed with H₂O (30 mL), brine (30 mL), dried over anhydrous Na₂SO₄, filtered, and concentrated *in vacuo* to give a crude residue. The residue was purified by silica gel column chromatography (petroleum ether:EtOAc = 5:1) to afford the title product **(57a)** (850 mg, 25%) as a white solid. *m*/*z* (ESI, +ve ion) = 169.1 [M+H]⁺.

### Step B. 1-Ethyl-1H-imidazole-5-carboxamide (57b)

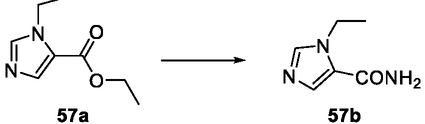

A solution of **57a** (3.6 g, 21.4 mmol) in 7 N NH₃ solution in MeOH (50 mL) was stirred at 80 °C for 36 h in a sealed reactor. After completion, the mixture was concentrated *in vacuo* to give a crude residue. The crude residue was purified by silica gel column chromatography (DCM:MeOH = 10:1) to afford the title product **(57b)** (1.9 g, 64%) as a white solid. *m*/*z* (ESI, +ve ion) = 140.1 [M+H]⁺.

### Step C. (1-Ethyl-1H-imidazol-5-yl)methanamine (57c)

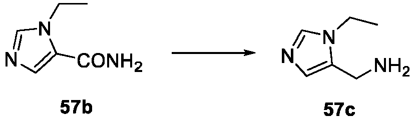

To a solution of **57b** (1.9 g, 13.6 mmol) in THF (20 mL) was added LiAlH₄ (1.1 g, 27.2 mmol) at 0 °C. The mixture was stirred at 60 °C for 16 h under N₂. After completion, the mixture was quenched with Na₂SO₄·10H₂O, and filtered. The filtrate was extracted by DCM (100 mL). The organic layer was concentrated *in vacuo* to afford the crude title product **(57c)** (1.4 g) as a colorless oil, which was used in the next step without further purification. *m*/*z* (ESI, +ve ion) = 126.2 [M+H]⁺.

### Step D. Methyl 3-(((1-ethyl-1H-imidazol-5-yl)methyl)amino)-4-nitrobenzoate (57d)

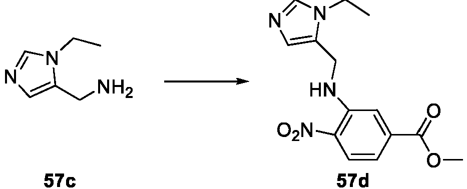

A mixture of **57c** (1.4 g, 11.1 mmol), methyl 3-fluoro-4-nitrobenzoate (2.4 g, 12.2 mmol), and TEA (3.3 g, 33.3 mmol) in THF (20 mL) was stirred at 25 °C for 16 h. After completion, the mixture was diluted with H₂O (50 mL), and extracted with DCM (50 mL x 3). The organic layer was dried over anhydrous Na₂SO₄, filtered, and concentrated *in vacuo* to give a crude residue. The residue was purified by silica gel column chromatography (DCM:MeOH = 10:1) to afford the title product **(57d)** (1.6 g, 47%) as a white solid. *m*/*z* (ESI, +ve ion) = 305.2 [M+H]⁺.

### Step E. Methyl 4-amino-3-(((1-ethyl-1H-imidazol-5-yl)methyl)amino)benzoate (57e)

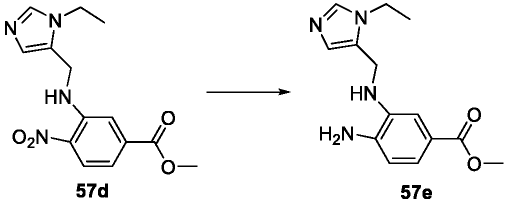

A mixture of **57d** (1.6 g, 5.26 mmol), and 10% Pd/C (200 mg) in MeOH (10 mL) was stirred at 25 °C for 2 h under H₂. After completion, the mixture was filtered through a pad of celite, which was rinsed with additional MeOH (100 mL). The organic layers were combined, and concentrated *in vacuo* to give a crude residue. The residue was purified by silica gel column chromatography (DCM:MeOH = 10:1) to afford the title product **(57e)** (1.3 g, 90%) as a brown solid. *m*/*z* (ESI, +ve ion) = 275.2 [M+H]⁺.

### Step F. Methyl 2-(chloromethyl)-1-((1-ethyl-1H-imidazol-5-yl)methyl)-1H-benzo[d]imidazole-6-carboxylate (57f)

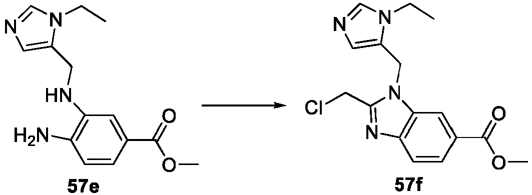

To a solution of **57e** (100 mg, 0.36 mmol) in CH₃CN (3 mL) was added 2-chloro-1,1,1-trimethoxyethane (67.7 mg, 0.44 mmol), and p-toluenesulfonic acid monohydrate (3.4 mg, 0.018 mmol) at room temperature. The mixture was heated to 60 °C, and stirred overnight. The crude reaction mixture was purified by reverse phase HPLC (CH₃CN and water with 0.1% HOAc as a modifier) to give the title product **(57f)** (46.6 mg, 39.0 %) as a white solid. *m*/*z* (ESI, +ve ion) = 333.1 [M+H]⁺.

### Step G. Methyl 2-((4-(tert-butoxycarbonyl)piperazin-1-yl)methyl)-1-((1-ethyl-1H-imidazol-5-yl)methyl)-1H-benzo[d]imidazole-6-carboxylate (57g)

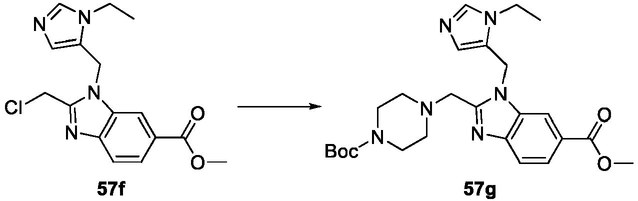

A mixture of *tert-*butyl piperazine-1-carboxylate (11 mg, 0.06 mmol), **57f** (20 mg, 0.06 mmol), and K₂CO₃ (18 mg, 0.13 mmol) was stirred in DMF (1 mL) overnight. The crude mixture was purified by reverse phase HPLC (CH₃CN and water with 0.1% HOAc as a modifier) to provide the title product **(57g)** (23 mg, 79%). *m*/*z* (ESI, +ve ion) = 483.3 [M+H]⁺.

### Step H. Methyl 1-((1-ethyl-1H-imidazol-5-yl)methyl)-2-(piperazin-1-ylmethyl)-1H-benzo[d]imidazole-6-carboxylate (57h)

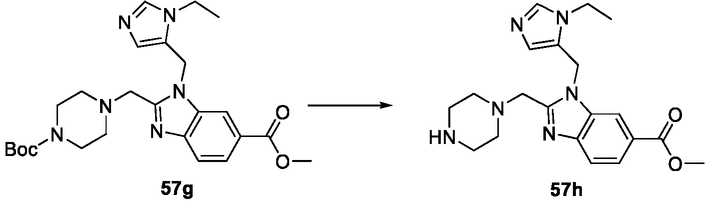

**57g** (23 mg, 0.06 mmol) was dissolved in DCM (2 mL), and treated with trifluoroacetic acid (TFA, 2 mL) at room temperature for 10 min. The solvent and TFA were removed *in vacuo* to provide the title product **(57h)** as a crude residue, which was used in the next step without further purification. *m*/*z* (ESI, +ve ion) = 383.2 [M+H]⁺.

### Step I. 1-((1-Ethyl-1H-imidazol-5-yl)methyl)-2-(piperazin-1-ylmethyl)-1H-benzo[d]imidazole-6-carboxylic acid (57i)

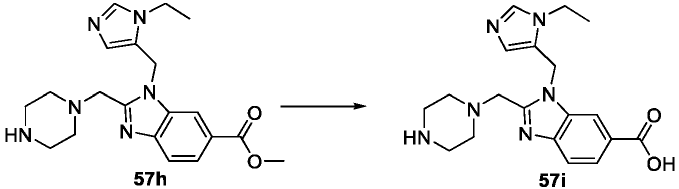

Crude **57h** was dissolved in CH₃CN (1 mL), and LiOH (15 mg, 0.6 mmol) in H₂O (0.5 mL) was added at room temperature. The reaction mixture was stirred overnight and purified by reverse phase HPLC (CH₃CN and water with 0.1% HOAc as a modifier) to provide the title product **(57i)** (8.9 mg, 24.9 %). *m*/*z* (ESI, +ve ion) = 369.3 [M+H]⁺.

### Step J. 2-((4-(3-(2-Chloro-4-methylphenoxy)benzoyl)piperazin-1-yl)methyl)-1-((1-ethyl-1H-imidazol-5-yl)methyl)-1H-benzo[d]imidazole-6-carboxylic acid (57)

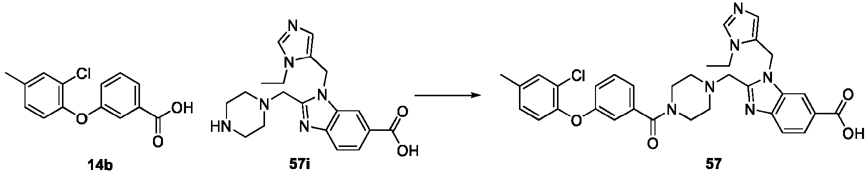

The title compound was prepared from 3-(2-chloro-4-methylphenoxy)benzoic acid (similarly prepared as **14b),** and **57i** by procedures similar to those described in Example **2,** Step B. ¹H NMR (600 MHz, CD₃OD) δ ppm 8.15 (s, 1H), 7.99 (br dd, *J =* 8.62, 1.28 Hz, 1H), 7.77 (s, 1H), 7.70 (d, *J =* 8.44 Hz, 1H), 7.40 (t, *J =* 8.07 Hz, 1H), 7.35 (d, *J =* 1.83 Hz, 1H), 7.16 (br dd, *J =* 8.25, 2.02 Hz, 1H), 7.07 (br d, *J =* 7.34 Hz, 1H), 7.02 (d, *J =* 8.44 Hz, 1H), 6.97 (br dd, *J =* 8.07, 2.57 Hz, 1H), 6.80 - 6.84 (m, 1H), 6.56 (s, 1H), 5.76 (s, 2H), 4.07 (q, *J* = 7.34 Hz, 2H), 3.88 (s, 2H), 3.59 (br s, 2H), 3.24 - 3.30 (m, 2H), 2.55 (br s, 2H), 2.40 (br s, 2H), 2.36 (s, 3H), 1.29 (t, *J =* 7.34 Hz, 3H). *m*/*z* (ESI, +ve ion) = 613.3 [M+H]⁺.

### Example 59. (S)-2-((4-(5-(2,4-Dichlorobenzyl)furan-2-carbonyl)piperazin-1-yl)methyl)-1-(oxetan-2-ylmethyl)-1H-benzo[d]imidazole-6-carboxylic acid (59)

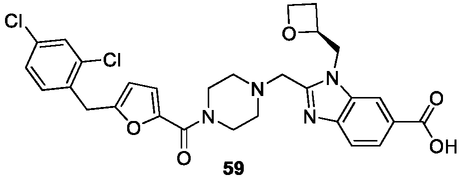

### Step A. 1-(Bromomethyl)-2,4-dichlorobenzene (59a)

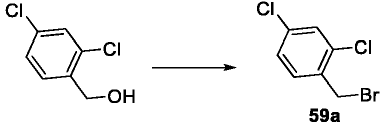

To a solution of (2,4-dichlorophenyl)methanol (1.0 g, 5.6 mmol) in DCM (15 mL) under N₂ was added PBr₃ (760 mg, 2.8 mmol) at 0 °C. After 0.5 h, the mixture was warmed to 25 °C, and stirred for 1.5 h. After completion of the reaction, the mixture was quenched with H₂O (10 mL), and adjusted to pH 7-8 by 7% aqueous NaHCO₃. The reaction mixture was extracted with DCM (20 mL x 2). The organic layer was dried over anhydrous Na₂SO₄, filtered, and concentrated *in vacuo* to provide the title product **(59a)** (1.0 g), which was used in the next step without further purification.

### Step B. Methyl 5-(2,4-dichlorobenzyl)furan-2-carboxylate (59b)

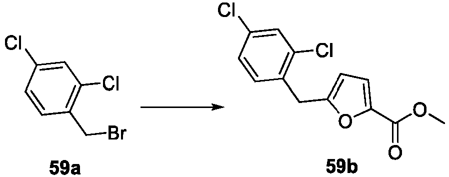

To a mixture of **59a** (0.9 g, 3.8 mmol), and (5-(methoxycarbonyl)furan-2-yl)boronic acid (0.65 g, 3.8 mmol) in dioxane (30 mL) and H₂O (4 mL) were added sodium carbonate (1.21 g, 11.4 mmol) and Pd(dppf)Cl₂ (0.44 g, 0.3 mmol) at 20 °C. The mixture was stirred at 80 °C for 12 h under N₂. After completion, the mixture was quenched with H₂O (50 mL), and extracted with EtOAc (50 mL x 3). The organic layer was washed with H₂O (30 mL), brine (30 mL), dried over anhydrous Na₂SO₄, filtered, and concentrated *in vacuo* to provide a crude residue, which was purified by silica gel column chromatography (petroleum ether:EtOAc = 20:1) to afford the title product **(59b)** (1.0 g, 86.8%) as a colorless oil. *m*/*z* (ESI, +ve ion) = 285.0. [M+H]⁺.

### Step C. 5-[(2,4-Dichlorophenyl)methyl]furan-2-carboxylic acid (59c)

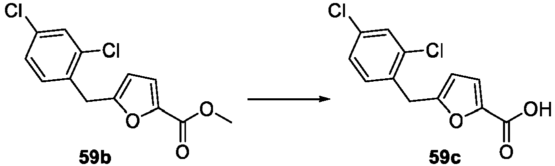

To a mixture of **59b** (1.0 g, 0.0035 mol) in THF (30 mL) and H₂O (10 mL) was added LiOH (0.25 g, 0.0105 mol) at 25 °C. The mixture was stirred for 12 h under N₂. After reaction completion, the mixture was adjusted to pH = 7 with 1 N HCl, and extracted with EtOAc (10 mL x 3). The organic layer was washed with H₂O (30 mL), brine (30 mL), dried over anhydrous Na₂SO₄, filtered, and concentrated *in vacuo* to afford the title product **(59c)** (700 mg, 73.8%) as a white solid, which was used in the next step without further purification. *m*/*z* (ESI, +ve ion) = 270.9 [M+H]⁺.

### Step D. Methyl (S)-2-((4-(5-(2,4-dichlorobenzyl)furan-2-carbonyl)piperazin-1-yl)methyl)-1-(oxetan-2-ylmethyl)-1H-benzo[d]imidazole-6-carboxylate (59d)

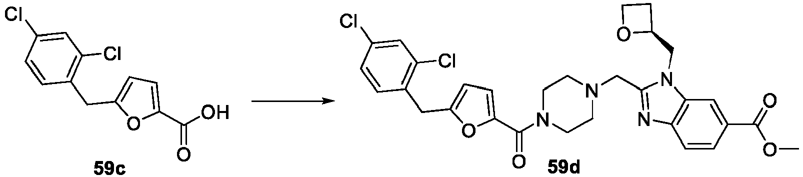

To a solution of **59c** (100 mg, 0.37 mmol) in DCM (10 mL) was added *N,N-*diisopropylethylamine (238 mg, 1.8 mmol), 1-hydroxybenzotriazole (68 mg, 0.48 mmol), *N-*(3-dimethylaminopropyl)-*N*'-ethylcarbodiimide hydrochloride (85 mg, 0.44 mmol) and **1j** (127 mg, 0.37 mmol) at 25 °C. The reaction was stirred at 25 °C for 16 h under N₂. After completion, the reaction was filtered, and concentrated *in vacuo* to give a crude residue, which was purified by silica gel column chromatography (petroleum ether: EtOAc = 5:1) to afford the title product **(59d)** (160 mg, 50.8%) as a white solid. *m*/*z* (ESI, +ve ion) = 597.1 [M+H]⁺.

### Step E. (S)-2-((4-(5-(2,4-Dichlorobenzyl)furan-2-carbonyl)piperazin-1-yl)methyl)-1-(oxetan-2-ylmethyl)-1H-benzo[d]imidazole-6-carboxylic acid (59)

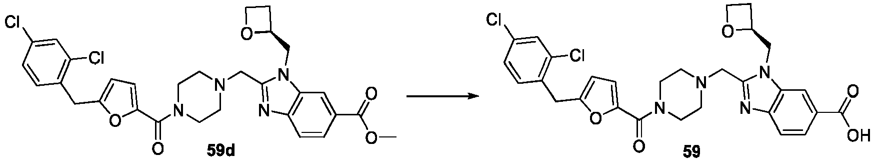

To a mixture of **59d** (40 mg, 0.067 mmol) in CH₃CN:H₂O = 1:1 (10 mL) was added TBD (56 mg, 0.40 mmol) at 25 °C. The mixture was stirred at 50 °C for 2 h under N₂. After completion, the reaction mixture was adjusted to pH = 7 with 1 N HCl, and extracted with EtOAc (10 mL x 3). The organic layer was washed with water (10 mL), brine (10 mL), dried over Na₂SO₄, filtered, and concentrated *in vacuo* to provide a crude residue. The residue was purified by reverse phase HPLC (H₂O:CH₃CN = 1:1) to provide the title compound **(59)** (30 mg, 75.3%) as a white solid. ¹H NMR (400 MHz, CD₃OD) δ ppm 8.35 (s, 1H), 7.98 (dd, *J =* 8.33, 1.32 Hz, 1H), 7.69 (d, *J =* 8.33 Hz, 1H), 7.46 (d, *J =* 1.75 Hz, 1H), 7.21 - 7.40 (m, 2H), 6.96 (d, *J* = 3.51 Hz, 1H), 6.25 (d, *J =* 3.51 Hz, 1H), 5.25 (qd, *J =* 7.16, 2.63 Hz, 1H), 4.90 - 4.84 (m, 1H), 4.72 (dd, *J =* 15.57, 2.41 Hz, 1H), 4.64 (td, *J =* 7.89, 6.14 Hz, 1H), 4.45 (dt, *J =* 9.10, 5.97 Hz, 1H), 4.16 (s, 2H), 4.05 (br d, *J =* 14.03 Hz, 1H), 3.95 (br d, *J =* 14.03 Hz, 1H), 3.75 (br s, 4H), 2.75 - 2.87 (m, 1H), 2.57 (br s, 4H), 2.46 - 2.54 (m, 1H). *m*/*z* (ESI, +ve ion) = 583.2 [M+H]⁺.

### Example 60. (S)-2-((4-((2-((2,4-Dichlorophenoxy)methyl)oxazol-5-yl)methyl)piperidin-1-yl)methyl)-1-(oxetan-2-ylmethyl)-1H-benzo[d]imidazole-6-carboxylic acid (60)

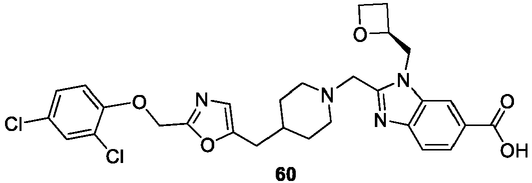

### Step A. Methyl 5-(bromomethyl)oxazole-2-carboxylate (60a)

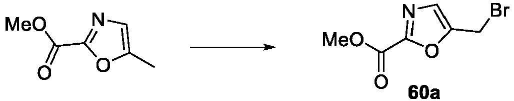

NBS (1034 mg, 5.81 mmol), and AIBN (93 mg, 0.58 mmol) were added to methyl 5-methyl-1,3-oxazole-2-carboxylate (800 mg, 5.81 mmol) in anhydrous CCl₄ (58 mL) at room temperature, and the reaction was heated to 70 °C for one hour. The reaction was quenched with sat. aq. NaHCO₃, and extracted with DCM (20 mL x 3). The organic layer was washed with brine, dried (Na₂SO₄), filtered, concentrated, and purified by silica gel column chromatography (gradient elution, EtOAc in hexanes 0-40%,) to provide the title product **(60a)** (1000 mg, 78%) as a colorless oil. *m*/*z* (ESI, +ve ion) = 222.0 [M+H]⁺

### Step B. Methyl 5-((1-(tert-butoxycarbonyl)-1,2,3,6-tetrahydropyridin-4-yl)methyl)oxazole-2-carboxylate (60b)

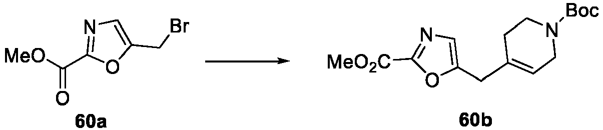

(1,1'-Bis(diphenylphosphino)ferrocene)palladium(II) dichloride (332 mg, 0.45 mmol), K₂CO₃ (1258 mg, 9.1 mmol), and *tert-*butyl 4-(4,4,5,5-tetramethyl-1,3,2-dioxaborolan-2-yl)-1,2,3,6-tetrahydropyridine-1-carboxylate (1406 mg, 4.55 mmol) were added to **60a** (1000 mg, 4.55 mmol) in 1,4-dioxane:H₂O (3:1, 46 mL) at room temperature. The mixture was degassed with argon for 10 min, then heated to 50 °C for 2 h. The reaction mixture was partitioned between EtOAc and sat. aq. NaHCO₃. The organic layer was washed with brine, dried (Na₂SO₄), filtered, concentrated, and purified by silica gel chromatography (gradient elution, EtOAc in hexanes 0-50%) to provide the title product **(60b)** (584 mg, 40%) as an amber oil. *m*/*z* (ESI, +ve ion) = 345.1 [M+Na]⁺

### Step C. tert-Butyl 4-((2-(hydroxymethyl)oxazol-5-yl)methyl)piperidine-1-carboxylate (60c)

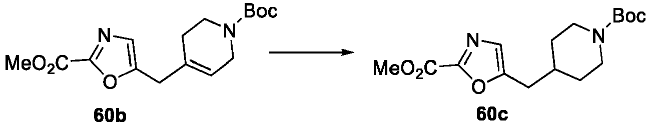

10% Pd/C (96 mg, 0.09 mmol) was added to **60b** (584 mg, 1.81 mmol) in EtOH (18 mL) at room temperature. The reaction was degassed with hydrogen for 10 min, then stirred under a H₂ balloon for 16 h. The mixture was filtered through a pad of celite, rinsed with EtOAc, and concentrated to give the title product **(60c)** (586 mg, 99%) as a light brown oil, which was used in the next step without further purification. *m*/*z* (ESI, +ve ion) = 347.3 [M+Na]⁺.

### Step D. tert-Butyl 4-((2-(hydroxymethyl)oxazol-5-yl)methyl)piperidine-1-carboxylate (60d)

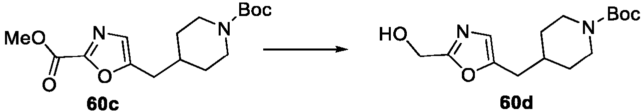

LiAlH₄ (1 M in THF) (3.6 mL, 3.6 mmol) was added to **60c** (586 mg, 1.81 mmol) in anhydrous THF (18 mL) at -78 °C, and stirred at 0 °C for 30 min. The reaction was quenched by adding H₂O dropwise, followed by 15% NaOH, and extraction with EtOAc (20 mL x 3). The organic layer was washed with brine, dried (Na₂SO₄), filtered, concentrated, and purified by silica gel column chromatography (gradient elution, MeOH in CH₂Cl₂ 0-10%) to provide the title product **(60d)** (57 mg, 11%) as a light yellow oil. *m*/*z* (ESI, +ve ion) = 319.2 [M+Na]⁺.

### Step E. tert-Butyl 4-((2-((2,4-dichlorophenoxy)methyl)oxazol-5-yl)methyl)piperidine-1-carboxylate (60e)

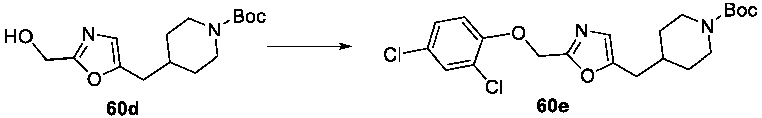

DIAD (58 mg, 0.29 mmol), PPh₃ (76 mg, 0.29 mmol), and 2,4-dichlorophenol (31 mg, 0.19 mmol) were added to **60d** (57 mg, 0.19 mmol) in anhydrous THF (1.9 mL) at 0 °C. After 1.5 h, the reaction was partitioned between EtOAc and H₂O. The organic layer was washed with brine, dried (Na₂SO₄), filtered, concentrated, and purified by silica gel column chromatography (gradient elution, EtOAc in hexanes 0-90%) to provide the title product **(60e)** (65 mg, 76%) as a colorless oil. *m*/*z* (ESI, +ve ion) = 385.0 [M-tBu+H]⁺.

### Step F. Methyl (S)-2-((4-((2-((2,4-dichlorophenoxy)methyl)oxazol-5-yl)methyl)piperidin-1-yl)methyl)-1-(oxetan-2-ylmethyl)-1H-benzo[d]imidazole-6-carboxylate (60f)

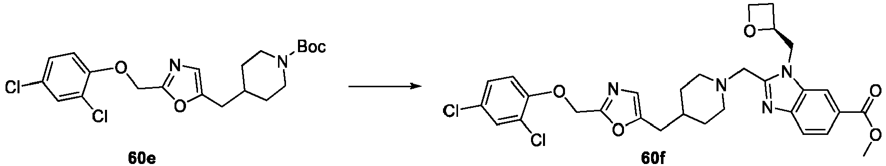

TFA (0.03 mL, 0.41 mmol) was added to **60e** (9 mg, 0.02 mmol) in DCM (0.2 mL) at room temperature. The reaction was stirred for 3 h, concentrated, and co-evaporated with DCM (2 mL x 3) *in vacuo* to give a crude amine (7 mg). *m*/*z* (ESI, +ve ion) = 341.1 [M+H]⁺. The crude amine was redissolved in DCM (0.2 mL). DIPEA (0.07 mL, 0.04 mmol) and **1h** (4 mg, 0.014 mmol) were added consecutively. The reaction was heated at 40 °C for 16 h, concentrated, and purified by silica gel column chromatography (gradient elution, MeOH in DCM 0-10%) to provide the title compound **(60f)** (9 mg, 75%). *m*/*z* (ESI, +ve ion) = 599.3 [M+H]⁺.

### Step G. (S)-2-((4-((2-((2,4-Dichlorophenoxy)methyl)oxazol-5-yl)methyl)piperidin-1-yl)methyl)-1-(oxetan-2-ylmethyl)-1H-benzo[d]imidazole-6-carboxylic acid (60)

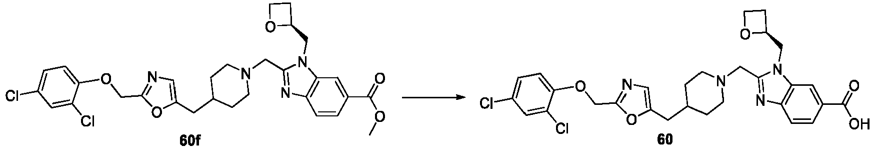

LiOH·H₂O (0.6 mg, 0.015 mmol) was added to **60f** (9 mg, 0.015 mmol) in THF:H₂O (1:1, 0.3 mL), and stirred at room temperature. After 12 h, additional LiOH·H₂O (0.3 mg, 0.007 mmol) was added, and the reaction was stirred for another 3 h. The reaction was acidified with 1 N HCl to pH = 7 and partitioned between EtOAc and sat. aq. NaHCO₃. The organic layer was washed with brine, dried (Na₂SO₄), filtered, concentrated, and purified by silica gel column chromatography (gradient elution, MeOH in DCM 0-15%) to give the title compound **(60)** (8 mg, 91%) as a white solid. *m*/*z* (ESI, +ve ion) = 585.3 [M+H]⁺. ¹H NMR (600 MHz, CD₃OD) δ ppm 8.31 (s, 1H), 7.97 (br dd, *J =* 8.44, 1.47 Hz, 1H), 7.65 (d, *J =* 8.44 Hz, 1H), 7.40 (d, *J =* 2.57 Hz, 1H), 7.25 (br dd, *J =* 8.80, 2.57 Hz, 1H), 7.22 - 7.16 (m, 1H), 6.87 (s, 1H), 5.21 - 5.25 (m, 3H), 4.81 - 4.85 (m, 1H), 4.70 - 4.61 (m, 2 H), 4.45 (dt, *J =* 9.17, 5.87 Hz, 1 H), 4.01 (d, *J =* 13.9 Hz, 1H), 3.90 (d, *J =* 13.9 Hz, 1H), 3.00 - 2.83 (m, 2H), 2.82 - 2.73 (m, 1H), 2.66 (br d, *J* = 6.60 Hz, 1H), 2.50 (ddt, *J* = 11.23, 9.03, 7.29, 7.29 Hz, 1H), 2.11 - 2.30 (m, 2H), 1.59 - 1.72 (m, 2H), 1.21 - 1.40 (m, 2H), 0.81 - 0.96 (m, 2H).

### Example 61. (S)-2-((4-(6-(Cyclohexyl(methyl)amino)pyrimidine-4-carbonyl)piperazin-1-yl)methyl)-1-(oxetan-2-ylmethyl)-1H-benzo[d]imidazole-6-carboxylic acid (61)

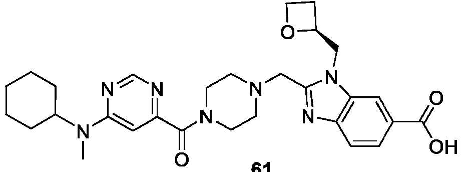

### Step A. Methyl 6-(cyclohexyl(methyl)amino)pyrimidine-4-carboxylate (61a)

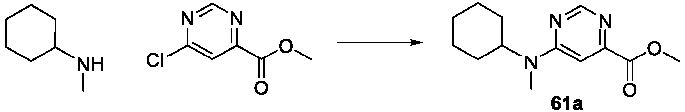

A mixture of methyl 6-chloropyrimidine-4-carboxylate (500 mg, 2.89 mmol), N-methylcyclohexanamine (655 mg, 5.8 mmol), and *N,N*-diisopropylethylamine (748 mg, 5.79 mmol) in THF (50 mL) was heated to 65 °C for 24 h. The reaction was quenched with H₂O (10 ml), NaHCO₃ (10 mL), and extracted with DCM (15 mL x 3). The organic layers were combined, dried, filtered, and concentrated to afford the crude title product **(61a)** (600 mg), which was used in the next step without further purification. *m*/*z* (ESI, +ve ion) = 250.2 [M+H]⁺.

### Step B. 6-(Cyclohexyl(methyl)amino)pyrimidine-4-carboxylic acid (61b)

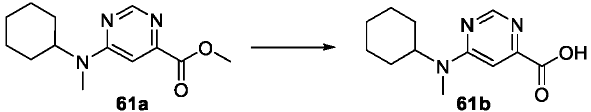

A mixture of **61a** (500 mg, 2.00 mmol), and sodium hydroxide (401 mg, 10 mmol) in MeOH:H₂O = 10:1 (20 mL) was stirred at 25 °C for 2 h. The reaction mixture was then extracted with EtOAc (50 mL x 3). The combined organic layers were dried over anhydrous Na₂SO₄, filtered, and concentrated *in vacuo.* The crude residue was purified by reverse phase HPLC (CH₃CN:H₂O = 1:3) to afford the title product **(61b)** (430 mg, 82.0%) as a white solid. *m*/*z* (ESI, +ve ion) = 236.0 [M+H]⁺.

### Steps C and D. (S)-2-((4-(6-(Cyclohexyl(methyl)amino)pyrimidine-4-carbonyl)piperazin-1-yl)methyl)-1-(oxetan-2-ylmethyl)-1H-benzo[d]imidazole-6-carboxylic acid (61)

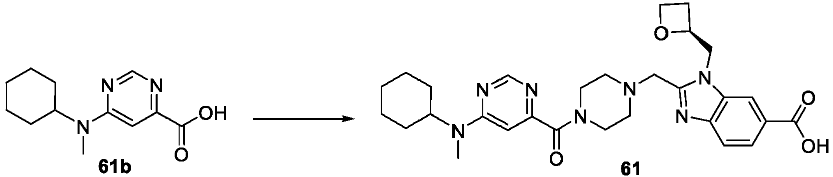

The title compound was prepared from **61b** by procedures similar to those described in Example **61,** Steps B, and C. ¹H NMR (400 MHz, CDCl₃) δ ppm 8.55 (s, 1H), 8.20 (br s, 1H), 8.04 (d, *J* = 7.89 Hz, 1H), 7.79 (d, *J =* 8.33 Hz, 1H), 6.68 (br s, 1H), 5.14 - 5.28 (m, 1H), 4.53 - 4.68 (m, 4H), 4.35 (br d, *J =* 8.33 Hz, 1H), 3.96 - 4.12 (m, 2H), 3.80 (br s, 2H), 3.59 (br s, 2H), 2.93 (br s, 3H), 2.62 - 2.75 (m, 2H), 2.57 (br s, 2H), 2.35 - 2.52 (m, 2H), 1.86 (br d, *J =* 10.52 Hz, 2H), 1.71 (br d, *J =* 10.96 Hz, 3H), 1.10 - 1.58 (m, 5H). *m*/*z* (ESI, +ve ion) = 274.7 [M+H]⁺.

### Example 62. (S)-2-((4-(6-(Methyl(phenyl)amino)pyrimidine-4-carbonyl)piperazin-1-yl)methyl)-1-(oxetan-2-ylmethyl)-1H-benzo[d]imidazole-6-carboxylic acid (62)

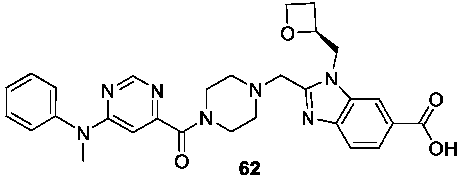

### Step A. 6-(Methyl(phenyl)amino)pyrimidine-4-carboxylic acid (62a)

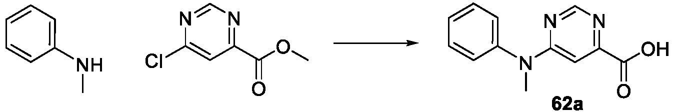

A flask was charged with *N*-methylaniline (0.5 g, 2.9 mmol), and dioxane (15 mL). Methyl 6-chloropyrimidine-4-carboxylate (466 mg, 4.3 mmol), 4-methylbenzenesulfonic acid (1.47 g, 8.7 mmol), and H₂O (62 mg, 3.5 mmol) were added. The resulting mixture was heated to 120 °C and stirred for 8 h. After completion, the mixture was quenched with H₂O (10 mL), and extracted with DCM (20 mL x 2). The aqueous phase was separated, lyophilized, and purified by silica gel column chromatography to provide the title product **(62a)** (0.41 g, 61.7 %). ¹H NMR (400 MHz, DMSO*-d₆*) δ 8.72 (s, 1H), 7.55 (t, *J =* 7.7 Hz, 2H), 7.44-7.39 (m, 3H), 6.83 (s, 1H), 3.47 (s, 3H).

### Step B. Methyl (S)-2-((4-(6-(methyl(phenyl)amino)pyrimidine-4-carbonyl)piperazin-1-yl)methyl)-1-(oxetan-2-ylmethyl)-1H-benzo[d]imidazole-6-carboxylate (62b)

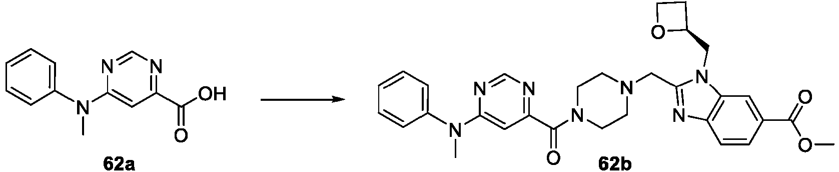

The title compound was prepared from **62a** and **1j** by procedures similar to those described in Example **1,** Step M. *m*/*z* (ESI, +ve ion) = 556.3 [M+H]⁺.

### Step C. (S)-2-((4-(6-(Methyl(phenyl)amino)pyrimidine-4-carbonyl)piperazin-1-yl)methyl)-1-(oxetan-2-ylmethyl)-1H-benzo[d]imidazole-6-carboxylic acid (62)

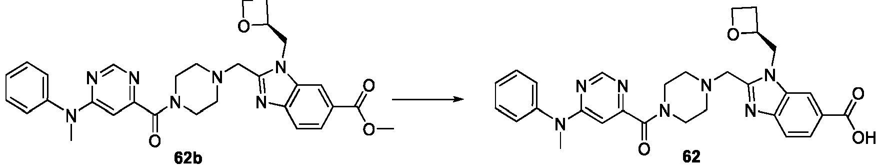

The title compound was prepared from **62b** by procedures similar to those described in Example **1,** Step N. ¹H NMR (400 MHz, DMSO-*d₆*) δ ppm 8.60 (d, *J =* 0.98 Hz, 1H), 8.47 (br s, 1H), 8.18 (s, 1H), 7.83 (br d, *J =* 8.31 Hz, 1H), 7.45 - 7.58 (m, 2H), 7.30 - 7.45 (m, 3H), 6.33 - 6.46 (m, 1H), 5.07 (m, 1H), 4.70 (br dd, *J =* 14.92, 6.85 Hz, 1H), 4.57 (br d, *J* = 13.45 Hz, 1H), 4.41 - 4.52 (m, 1H), 4.34 (dt, *J =* 8.86, 5.84 Hz, 1H), 3.91 (br d, *J =* 13.69 Hz, 1H), 3.77 (br d, *J =* 13.45 Hz, 1H), 3.47 - 3.62 (m, 2H), 3.43 (s, 3H), 3.12 - 3.26 (m, 2H), 2.60 - 2.76 (m, 1H), 2.34 - 2.49 (m, 4H), 2.30 - 2.33 (m, 1H). *m*/*z* (ESI, +ve ion) = 542.3 [M+H]⁺.

### Example 72. (S)-2-((4-((5-((2,4-Dichlorophenoxy)methyl)furan-2-yl)methyl)piperidin-1-yl)methyl)-1-(oxetan-2-ylmethyl)-1H-benzo[d]imidazole-6-carboxylic acid (72)

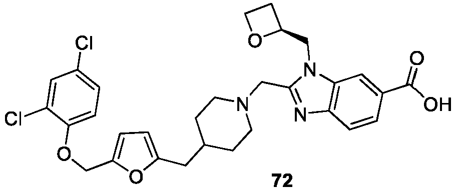

### Step A. tert-Butyl 4-((5-(methoxycarbonyl)furan-2-yl)methyl)-3,6-dihydropyridine-1(2H)-carboxylate (72a)

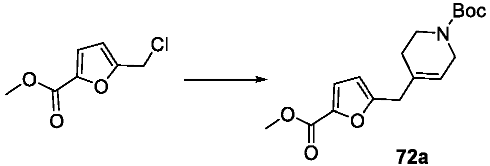

To a mixture of methyl 5-(chloromethyl)furan-2-carboxylate (3.0 g, 0.017 mol), *tert*-butyl-4-(4,4,5,5-tetramethyl-1,3,2-dioxaborolan-2-yl)-3,6-dihydropyridine-1(2*H*)-carboxylate (6.4 g, 0.021 mmol), and sodium carbonate (3.7 g, 0.034 mol) in dioxane:H₂O = 8:1 (80 mL) was added Pd(dppf)Cl₂·DCM (1.4 g, 0.0017 mol) at 25 °C. The mixture was stirred at 90 °C for 18 h under N₂. After completion, the mixture was quenched with H₂O (80 mL), and extracted with EtOAc (80 mL x 3). The organic layer was washed with H₂O (80 mL), brine (80 mL), dried over anhydrous Na₂SO₄, filtered, and concentrated *in vacuo* to give a crude residue, which was purified by silica gel column chromatography (petroleum ether:EtOAc = 20:1) to give the title product **(72a)** (4.2 g, 68% ) as a yellow oil. *m*/*z* (ESI, +ve ion) = 344.2 [M+Na]⁺.

### Step B. tert-Butyl 4-((5-(methoxycarbonyl)furan-2-yl)methyl)piperidine-1-carboxylate (72b)

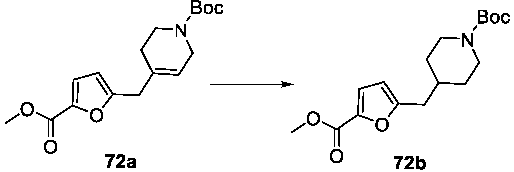

A flask was charged with **72a** (2.0 g, 0.0062 mol). MeOH (30 mL) and Raney-Ni (1.0 g) were added at 25 °C. The mixture was stirred at 25 °C for 36 h under H₂. After reaction completion, the mixture was filtered through a pad of celite, and the filtrate was concentrated *in vacuo* to afford crude **72b** (2.0 g) as a colorless oil, which was used in the next step without purification. *m*/*z* (ESI, +ve ion) = 346.2 [M+Na]⁺.

### Step C. tert-Butyl 4-((5-(hydroxymethyl)furan-2-yl)methyl)piperidine-1-carboxylate (72c)

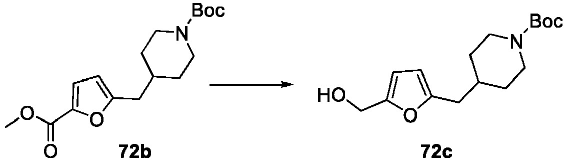

To a mixture of **72b** (1.5 g, 0.0046 mol) in THF (30 mL) was added diisobutylaluminum hydride (1.0 M solution in hexanes, 14 mL, 0.014 mol) at -40 °C. The mixture was stirred at -40 °C for 3 h under N₂. After completion, the reaction was quenched with ice-cold aq. NaOH (2 N) and extracted with EtOAc. The organic layer was washed with water, brine, dried over Na₂SO₄, filtered, and concentrated *in vacuo* to give a crude residue, which was purified by silica gel column chromatography (petroleum ether:EtOAc = 10:1) to afford the title product **(72c)** (1.3 g, 87%) as a yellow oil. *m*/*z* (ESI, +ve ion) = 318.2 [M+Na]⁺.

### Step D. tert-Butyl 4-((5-((2,4-dichlorophenoxy)methyl)furan-2-yl)methyl)piperidine-1-carboxylate (72d)

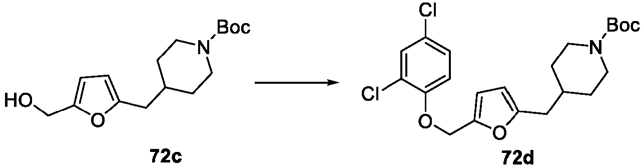

To a mixture of **72c** (1.3 g, 0.0044 mol), 2,4-dichlorophenol (0.79 g, 0.0048 mol), and triphenylphosphine (1.27 g, 0.0048 mol) in THF (30 mL) was added DEAD (0.84 g, 0.0048 mol) at 0 °C. The mixture was warmed to room temperature and stirred for 16 h under N₂. After completion, the mixture was concentrated *in vacuo* to give a crude residue, which was purified by silica gel column chromatography (petroleum ether:EtOAc = 10:1) to afford the title product **(72d)** (800 mg, 37%) as a colorless oil. *m*/*z* (ESI, +ve ion) = 462.1 [M+Na]⁺.

### Step E. 4-((5-((2,4-Dichlorophenoxy)methyl)furan-2-yl)methyl)piperidine (72e)

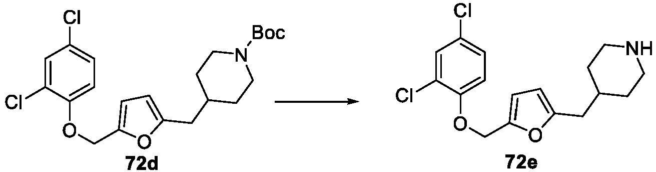

To a mixture of **72d** (500 mg, 1.1 mmol) in DCM (20 mL) was added 2,6-lutidine (3.7 g, 34 mmol), and TMSOTf (3.8 g, 17 mmol) at 0 °C. The reaction was stirred at 0 °C for one hour under N₂. After completion, the mixture was quenched with NH₄Cl (10 mL), and extracted with EtOAc (20 mL x 3). The organic layer was washed with H₂O (20 mL), brine (20 mL), dried over anhydrous Na₂SO₄, filtered, and concentrated *in vacuo* to afford a crude title product (**72e**) (400 mg) as a colorless oil, which was used in next step without further purification. *m*/*z* (ESI, +ve ion) = 340.1 [M+H]⁺.

### Step F. Methyl (S)-2-((4-((5-((2,4-dichlorophenoxy)methyl)furan-2-yl)methyl)piperidin-1-yl)methyl)-1-(oxetan-2-ylmethyl)-1H-benzo[d]imidazole-6-carboxylate (72f)

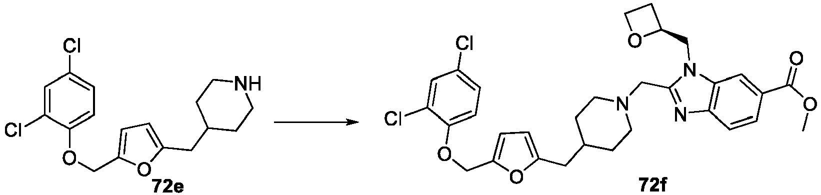

To a mixture of **72e** (400 mg, 1.2 mmol), and K₂CO₃ (325 mg, 2.4 mmol) in DMF (30 mL) was added **1h** (346 mg, 1.2 mmol) at 25 °C. The mixture was stirred at 25 °C for 16 h under N₂. After completion, the mixture was quenched with H₂O (30 mL), and extracted with DCM (30 mL x 3). The organic layer was washed with H₂O (30 mL x 3), brine (30 mL), dried over anhydrous Na₂SO₄, filtered, and concentrated *in vacuo* to give a crude residue, which was purified by silica gel column chromatography (petroleum ether:EtOAc = 1:1) to afford the title product (**72f**) (520 mg, 67%) as a yellow oil. *m*/*z* (ESI, +ve ion) = 598.2 [M+H]⁺.

### Step G. (S)-2-((4-((5-((2,4-Dichlorophenoxy)methyl)furan-2-yl)methyl)piperidin-1-yl)methyl)-1-(oxetan-2-ylmethyl)-1H-benzo[d]imidazole-6-carboxylic acid (72)

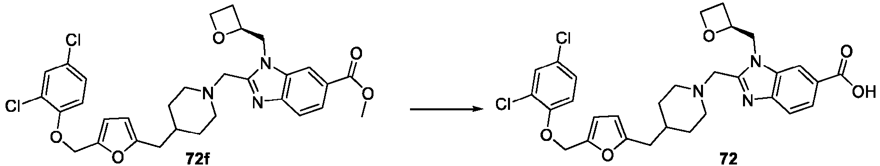

To a mixture of **72f** (50 mg, 0.084 mmol) in THF (6 mL) and H₂O (2 mL) was added lithium hydroxide (20 mg, 0.84 mmol), and the reaction was heated to 50 °C. After stirring at 50 °C for 24 h under N₂, the mixture was adjusted to pH = 7 with 1 N HCl, diluted with H₂O (5 mL), and extracted with EtOAc (10 mL x 3). The organic layer was washed with H₂O (10 mL), brine (10 mL), dried over anhydrous Na₂SO₄, filtered, and concentrated *in vacuo* to give a crude residue, which was purified by reverse phase HPLC (55% CH₃CN in H₂O with formic acid as a modifier) to afford the title compound (**72**) (30 mg, 58%) as a white solid. ¹H NMR (400 MHz, CD₃OD) δ ppm 8.27 (d, *J=* 0.88 Hz, 1H), 7.96 (dd, *J=* 8.33, 1.32 Hz, 1H), 7.63 (d, *J* = 8.33 Hz, 1H), 7.35 (d, *J =* 2.63 Hz, 1H), 7.20 - 7.24 (m, 1H), 7.15 - 7.19 (m, 1H), 6.37 (d, *J* = 3.07 Hz, 1H), 6.02 (d, *J =* 3.07 Hz, 1H), 5.22 (qd, *J =* 7.16, 2.63 Hz, 1H), 5.05 (s, 2H), 4.81 - 4.87 (m, 1H), 4.55 - 4.74 (m, 2H), 4.44 (dt, *J =* 8.99, 6.03 Hz, 1H), 4.00 (d, *J =* 14.03 Hz, 1H), 3.89 (d, *J =* 14.03 Hz, 1H), 2.96 (br d, *J =* 10.96 Hz, 1H), 2.86 (br d, *J =* 10.96 Hz, 1H), 2.71 - 2.82 (m, 1H), 2.56 (d, *J =* 6.58 Hz, 2H), 2.42 - 2.55 (m, 1H), 2.12 - 2.27 (m, 2H), 1.58 - 1.72 (m, 2H), 1.18 - 1.37 (m, 3H). *m*/*z* (ESI, +ve ion) = 584.2 [M+H]⁺.

Example compounds **84, 103, 104** (*tert*-butyl ester was used in the case of **104** and was deprotected using TFA in DCM), **108, and 109** were synthesized as described in Example **72.**

### Example 73. (S)-2-((4-(5-((2,4-Dichlorobenzyl)oxy)furan-2-carbonyl)piperazin-1-yl)methyl)-1-(oxetan-2-ylmethyl)-1H-benzo[d]imidazole-6-carboxylic acid (73)

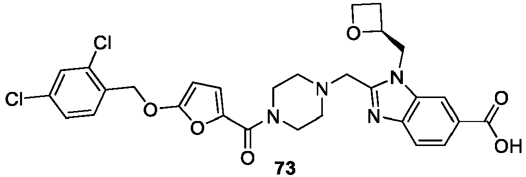

### Step A. Methyl 5-((2,4-dichlorobenzyl)oxy)furan-2-carboxylate (73a)

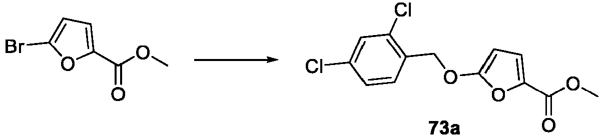

To a mixture of methyl 5-bromofuran-2-carboxylate (1.0 g, 0.0049 mol), (2,4-dichlorophenyl)methanol (0.87 g, 0.0049 mol), 3,4,7,8-tetramethyl-1,10-phenanthroline (0.23 g, 0.00098 mol), and CuI (0.09 g, 0.0004 mol) in toluene (15 mL) was added Cs₂CO₃ (2.4 g, 0.0074 mol), and the reaction mixture was heated to 80 °C under N₂ for 18 h. After completion, the mixture was quenched with H₂O (20 mL), and extracted with EtOAc (20 mL x 3). The organic layer was washed with H₂O (20 mL), brine (20 mL), dried over anhydrous Na₂SO₄, filtered, and concentrated *in vacuo* to give a crude residue, which was purified by silica gel column chromatography (petroleum ether:EtOAc = 50:1) to afford the title product (73a) (220 mg, 13%) as a white solid. *m*/*z* (ESI, +ve ion) = 301.0 [M+H]⁺.

### Step B. 5-((2,4-Dichlorobenzyl)oxy)furan-2-carboxylic acid (73b)

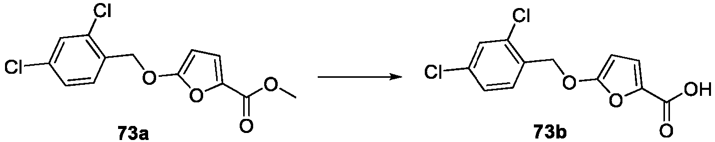

The title compound was prepared from **73a** by procedures similar to those described in Example **59,** Step C. *m*/*z* (ESI, +ve ion) = 287.0 [M+H]⁺.

### Step C. Methyl (S)-2-((4-(5-((2,4-dichlorobenzyl)oxy)furan-2-carbonyl)piperazin-1-yl)methyl)-1-(oxetan-2-ylmethyl)-1H-benzo[d]imidazole-6-carboxylate (73c)

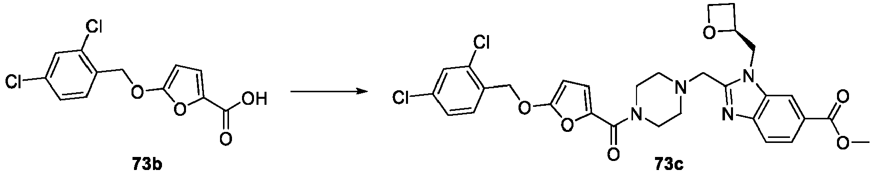

The title compound was prepared from **73b,** and **1j** by procedures similar to those described in Example **59,** Step D. *m*/*z* (ESI, +ve ion) = 613.2 [M+H]⁺.

### Step D. (S)-2-((4-(5-((2,4-Dichlorobenzyl)oxy)furan-2-carbonyl)piperazin-1-yl)methyl)-1-(oxetan-2-ylmethyl)-1H-benzo[d]imidazole-6-carboxylic acid (73)

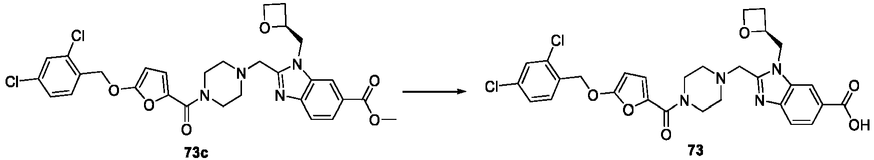

To a mixture of **73c** (50 mg, 0.082 mmol) in THF:H₂O = 3:1 (15 mL) was added lithium hydroxide (20 mg, 0.82 mmol), and the reaction mixture was heated to 50 °C. After stirring at 50 °C for 16 h under N₂, the reaction was cooled down, and adjusted to pH = 7 with 1 N HCl. Water (10 mL) was added, and the mixture was extracted with EtOAc (10 mL x 3). The organic layer was washed with H₂O (10 mL), brine (10 mL), dried over anhydrous Na₂SO₄, filtered, and concentrated *in vacuo* to give a crude residue, which was purified by reverse phase HPLC (45% CH₃CN in H₂O with 0.05% NH₄HCO₄ as a modifier) to afford the title compound (73) (30 mg, 60%) as a white solid. ¹H NMR (400 MHz, CD₃OD) δ ppm 8.31 (d, *J =* 0.9 Hz, 1H), 7.96 (dd, *J =* 8.5, 1.4 Hz, 1H), 7.65 (d, *J =* 8.5 Hz, 1H), 7.53 (dd, *J =* 6.6, 5.3 Hz, 2H), 7.37 (dd, *J =* 8.3, 2.1 Hz, 1H), 7.01 (d, *J* = 3.6 Hz, 1H), 5.59 (d, *J* = 3.6 Hz, 1H), 5.30 - 5.21 (m, 3H), 4.88 - 4.84 (m, 1H), 4.71 (dd, *J =* 15.4, 2.6 Hz, 1H), 4.63 (dt, *J=* 14.0, 7.1 Hz, 1H), 4.45 (dt, *J* = 9.1, 5.9 Hz, 1H), 4.03 (d, *J* = 13.8 Hz, 1H), 3.93 (d, *J* = 13.8 Hz, 1H), 3.78 (s, 4H), 2.79 (ddd, *J =* 16.2, 8.7, 5.7 Hz, 1H), 2.63 - 2.47 (m, 5H). *m*/*z* (ESI, +ve ion) = 599.1 [M+H]⁺.

### Example 74. (S)-2-((4-((5-((2,4-Dichlorobenzyl)oxy)furan-2-yl)methyl)piperazin-1-yl)methyl)-1-(oxetan-2-ylmethyl)-1H-benzo[d]imidazole-6-carboxylic acid (74)

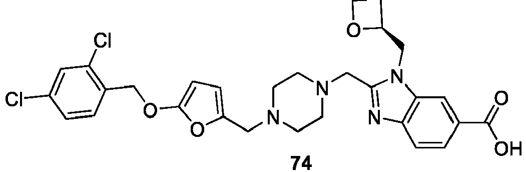

### Step A. Methyl 5-((2,4-dichlorobenzyl)oxy)furan-2-carboxylate (74a)

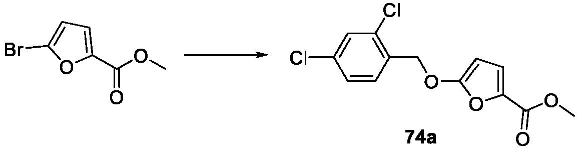

To a mixture of methyl 5-bromofuran-2-carboxylate (1g, 4.9 mmol), (2,4-dichlorophenyl)methanol (0.95 g, 5.3 mmol), 3,4,7,8-tetramethyl-1,10-phenanthroline (0.23 g, 0.9 mmol), and CuI (0.09 g, 0.4 mmol) in toluene (12 mL) was added Cs₂CO₃ (2.39 g, 7.3 mmol), and the reaction was heated to 80 °C for 18 h under N₂. The mixture was then filtered and concentrated *in vacuo* to give a crude residue, which was purified by silica gel column chromatography (EtOAc in petroleum ether, 20-30%) to provide the title product (**74a**) (0.35 g, 23.9%) as a yellow solid. *m*/*z* (ESI, +ve ion) = 301.0 [M+H]⁺.

### Step B. (5-((2,4-Dichlorobenzyl)oxy)furan-2-yl)methanol (74b)

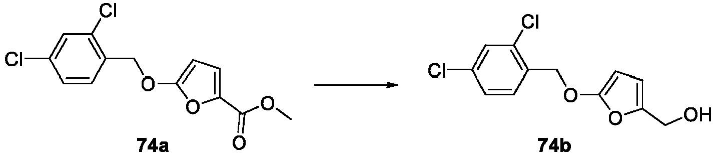

A mixture of **74a** (350 mg, 1.16 mmol) in anhydrous THF (15 mL) was added DIBAL-H (3.5 mL, 3.5 mmol) at -45 °C under N₂ and the reaction was stirred for one hour at -45 °C. The reaction was quenched with ice-cold aq. NaOH (2 N) and extracted with EtOAc (30 mL x 3). The organic layer was washed with water (20 mL), brine (20 mL), dried over Na₂SO₄, filtered, and concentrated *in vacuo.* The crude residue was purified by Prep-TLC (40% EtOAc in petroleum ether) to afford the title product (**74b**) (181 mg, 51%) as a colorless oil. *m*/*z* (ESI, +ve ion) = 294.9 [M+Na]⁺.

### Step C. 5-((2,4-Dichlorobenzyl)oxy)furan-2-carbaldehyde (74c)

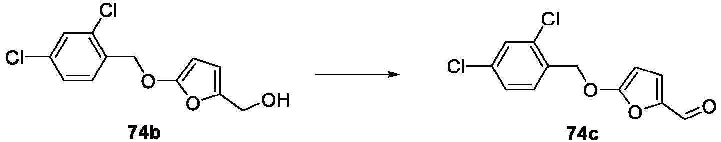

To a mixture of **74b** (30 mg, 0.11 mmol) in CCl₄ (3 mL) was added MnO₂ (47 mg, 0.55 mmol), and the reaction was stirred at 25 °C for 18 h. The mixture was then filtered and concentrated *in vacuo* to afford the title product (**74c**) (15 mg, 25%) as a colorless oil, which was used in the next step without further purification. *m*/*z* (ESI, +ve ion) = 293.0 [M+Na]⁺.

### Step D. Methyl (S)-2-((4-((5-((2,4-dichlorobenzyl)oxy)furan-2-yl)methyl)piperazin-1-yl)methyl)-1-(oxetan-2-ylmethyl)-1H-benzo[d]imidazole-6-carboxylate (74d)

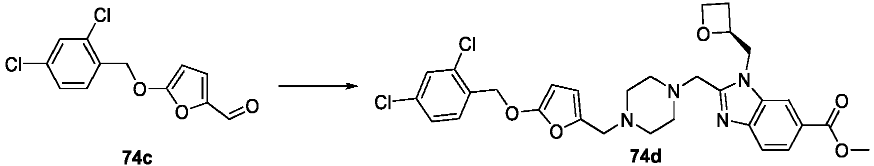

To a mixture of **74c** (118 mg, 0.435 mmol), and **1j** (25 mg, 0.07 mmol) in CH₃OH (5 mL) was added NaBH₃CN (9 mg, 0.145 mmol). The reaction was stirred at 25 °C for 36 h. The mixture was quenched with ice-water (5 mL), and extracted with EtOAc (15 mL x 3). The organic layer was washed with water (5 mL), brine (5 mL), dried, filtered, and concentrated to give a crude residue, which was purified by reverse phase HPLC (35% CH₃CN in H₂O with formic acid as a modifier) to afford the title product (**74d**) (13 mg, 3.6%) as a white solid. *m*/*z* (ESI, +ve ion) = 599.2 [M+H]⁺.

### Step E. (S)-2-((4-((5-((2,4-Dichlorobenzyl)oxy)furan-2-yl)methyl)piperazin-1-yl)methyl)-1-(oxetan-2-ylmethyl)-1H-benzo[d]imidazole-6-carboxylic acid (74)

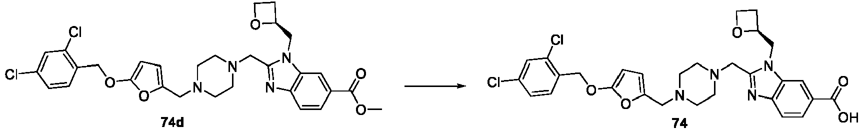

To a mixture **of 74d** (13 mg, 0.0217 mmol) in CH₃OH:H₂O (6:1, 3.5 mL) was added LiOH (8 mg, 0.33 mmol), and the reaction was stirred at 25 °C for 18 h. The mixture was adjusted to pH = 7 with 1 N HCl, and extracted with DCM:CH₃OH (10:1) (10 mL x 2). The organic layer was concentrated *in vacuo* to give a crude residue which was purified by reverse phase HPLC (56% CH₃CN in H₂O with NH₄HCO₃ as a modifier) to afford the title product (**74**) (4.4 mg, 31%) as a white solid. ¹H NMR (400 MHz, CD₃OD) δ ppm 8.16 (s, 1H), 7.93 (dd, *J* = 8.33, 1.32 Hz, 1H), 7.49 - 7.59 (m, 3H), 7.36 (br dd, *J* = 8.11, 1.97 Hz, 1H), 6.16 (d, *J* = 3.07 Hz, 1H), 5.29 (d, *J* = 3.07 Hz, 1H), 5.24 (m, 1H), 5.16 (s, 2H), 4.76 - 4.86 (m, 1H), 4.54 - 4.72 (m, 2H), 4.41 - 4.48 (m, 1H), 3.98 (br d, *J =* 13.59 Hz, 1H), 3.89 (br d, *J =* 13.59 Hz, 1H), 3.46 (s, 2H), 2.70 - 2.81 (m, 1H), 2.39 - 2.67 (m, 9H). *m*/*z* (ESI, +ve ion) = 585.2 [M+H]⁺.

### Example 78. (S)-2-((4-(3-(((2-Chloro-4-methylphenyl)(methyl)amino)methyl)benzoyl)piperazin-1-yl)methyl)-1-(oxetan-2-ylmethyl)-1H-benzo[d]imidazole-6-carboxylic acid (78)

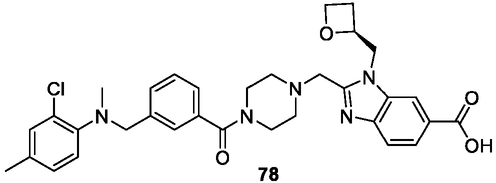

### Step A. Methyl 3-(((2-chloro-4-methylphenyl)amino)methyl)benzoate (78a)

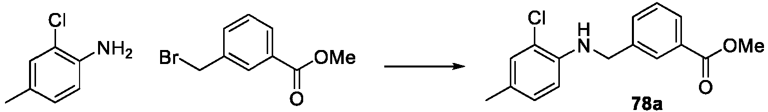

2-Chloro-4-methylaniline (14.2 mg, 0.1 mmol), methyl 3-(bromomethyl)benzoate (27.5 mg, 0.12 mmol), and K₂CO₃ (17.3 mg, 0.125 mmol) were suspended in DMF (1 mL). The reaction mixture was stirred at 100 °C for 1.5 hours. After being cooled to room temperature, the crude residue was purified by reverse phase HPLC (CH₃CN and water with 0.1% TFA as a modifier) to provide the title product (**78a**) (21 mg, 72.7 %) as a light brown solid. *m*/*z* (ESI, +ve ion) = 290.1 [M+H]⁺.

### Step B. (S)-2-((4-(3-(((2-Chloro-4-methylphenyl)(methyl)amino)methyl)benzoyl)piperazin-1-yl)methyl)-1-(oxetan-2-ylmethyl)-1H-benzo[d]imidazole-6-carboxylic acid (78)

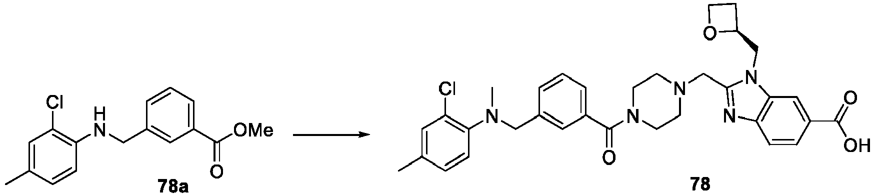

The title compound was prepared from **78a,** and **2a** by procedures similar to those described in Example **46,** Steps B to D. ¹H NMR (600 MHz, CD₃OD) δ ppm 8.33 (s, 1H), 7.97 (dd, *J* = 8.44, 1.47 Hz, 1H), 7.66 (d, *J =* 8.44 Hz, 1H), 7.47 (br d, *J* = 7.70 Hz, 1H), 7.34 - 7.43 (m, 2H), 7.28 (br d, *J* = 7.70 Hz, 1H), 7.16 (s, 1H), 6.94 - 6.99 (m, 2H), 5.25 (qd, *J* = 7.15, 2.75 Hz, 1H), 4.85 - 4.92 (m, 1H), 4.69 - 4.75 (m, 1H), 4.61 - 4.68 (m, 1H), 4.46 (dt, *J* = 9.17, 5.87 Hz 1H), 4.21 (s, 2H), 4.04 (d, *J =* 13.57 Hz 1H), 3.94 (d, *J =* 13.94 Hz, 1H), 3.78 - 3.85 (m, 1H), 3.71 - 3.78 (m, 1H), 3.27 - 3.29 (m, 2H), 2.73 - 2.89 (m, 1H), 2.68 (s, 3H), 2.60 - 2.67 (m, 2H), 2.46 - 2.58 (m, 1H), 2.40 (br s, 2H), 1.88-2.01 (s, 3H). *m*/*z* (ESI, +ve ion) = 602.4 [M+H]⁺.

Example compounds **77, 79,** and **80** were synthesized as described in Example **78.**

### Example 83. 4-(((6-(1-(6-(1H-Tetrazol-5-yl)nicotinoyl)piperidin-4-yl)pyridin-2-yl)oxy)methyl)-3-fluorobenzonitrile (83)

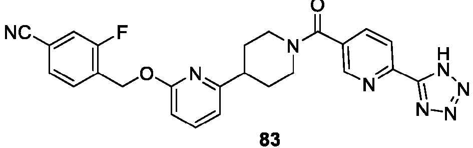

### Step A. tert-Butyl 6-fluoro-3',6'-dihydro-[2,4'-bipyridine]-1'(2'H)-carboxylate (83a)

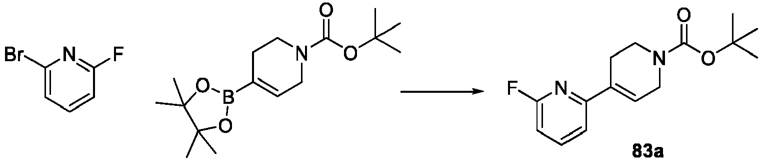

A solution of 2-bromo-6-fluoropyridine (2 g, 11.36 mmol), *tert*-butyl 4-(4,4,5,5-tetramethyl-1,3,2-dioxaborolan-2-yl)-3,6-dihydro-2*H*-pyridine-1-carboxylate (3.86 g, 12.5 mmol), sodium carbonate (4.71 g, 34.08 mmol), and Pd(dppf)Cl₂ (831 mg, 1.14 mmol) in a mixture of DMF (50 mL), and water (10 mL) was stirred at 80 °C for 3 h under nitrogen. After completion, the reaction mixture was extracted with EtOAc (50 mL x 2). The organic layer was washed with brine (50 mL), dried (sodium sulfate), filtered, and concentrated to give a crude residue, which was purified by silica gel column chromatography (gradient elution, EtOAc in hexanes 0-10%) to afford the title product (**83a**) (3.1 g, 97%) as a yellow oil. *m*/*z* (ESI, +ve ion) = 301.2 [M+Na]⁺.

### Step B. tert-Butyl 4-(6-fluoropyridin-2-yl) piperidine-1-carboxylate (83b)

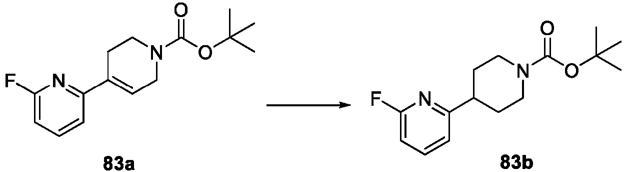

To a solution of **83a** (5 g, 17.96 mmol) in methanol (70 mL) was added 10% Pd/C (0.5 g). The mixture was stirred under a hydrogen atmosphere (1 atm) at 20 °C for one hour. The reaction mixture was filtered through a pad of celite, and the filtrate was concentrated under reduced pressure to afford the title product (**83b**) (3.9 g) as a yellow oil, which was used in the next step without further purification. *m*/*z* (ESI, +ve ion) = 303.2 [M+Na]⁺.

### Step C. tert-Butyl 4-(6-((4-cyano-2-fluorobenzyl)oxy)pyridin-2-yl)piperidine-1-carboxylate (83c)

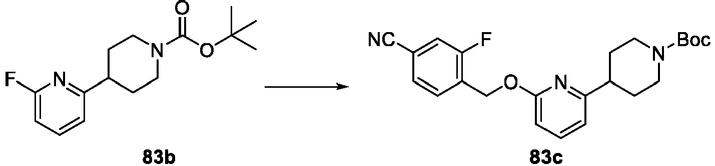

To a solution of **83b** (391 mg, 1.39 mmol), and 3-fluoro-4-(hydroxymethyl)benzonitrile (210 mg, 1.39 mmol) in dry 1,4-dioxane (2.5 mL) under nitrogen at 0 °C was added potassium *tert*-butoxide (156 mg, 1.39 mmol) in portions over 5 min. The mixture was heated to 110 °C for 4 h. After completion, the organic solvent was removed under reduced pressure. The crude residue was partitioned between EtOAc (5 mL) and water (5 mL). The organic phase was washed with water (5 mL), dried over anhydrous sodium sulfate, filtered, concentrated, and purified by reverse phase HPLC (gradient elution, CH₃CN in H₂O 0-90% with 0.1% NH₃·H₂O as a modifier) to afford the title product (**83c**) (305 mg, 74.2%) as a yellow oil. *m*/*z* (ESI, +ve ion) = 434.2 [M+Na]⁺.

### Step D. 3-Fluoro-4-(((6-(piperidin-4-yl)pyridin-2-yl)oxy)methyl)benzonitrile (83d)

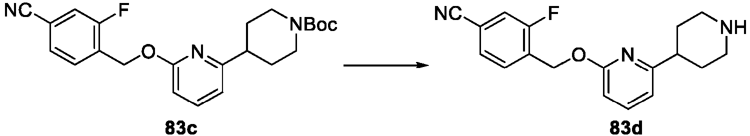

A solution of **83c** (305 mg, 0.74 mmol) in 2 N HCl:EtOAc (4 mL) was stirred at 20 °C for 0.5 h. After completion, the reaction mixture was concentrated and purified by silica gel column chromatography (gradient elution, MeOH in DCM 5-10%) to afford the title product (**83d**) (179 mg, 74.2%) as a yellow solid. *m*/*z* (ESI, +ve ion) = 312.2 [M+H]⁺.

### Step E. 4-(((6-(1-(6-(1H-Tetrazol-5-yl)nicotinoyl)piperidin-4-yl)pyridin-2-yl)oxy)methyl)-3-fluorobenzonitrile (83)

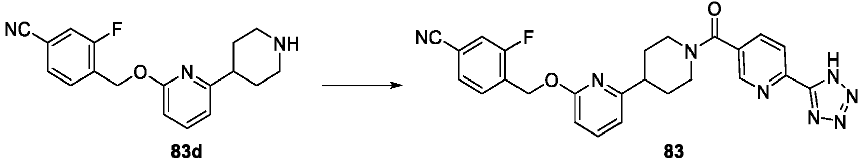

A flask was charged with **83d** (6.8 mg, 0.022 mmol), 6-(1*H*-tetrazol-5-yl)nicotinic acid (3.8 mg, 0.02 mmol), and HATU (11.4 mg, 0.03 mmol). DMF (1 mL) was added, followed by DIPEA (0.014 mL, 0.08 mmol). The reaction was stirred for 10 min and then directly purified by reverse phase HPLC (CH₃CN and water with 0.1% HOAc as a modifier) to give the title compound (**83**) (2.5 mg, 25.8%) as a white solid. ¹H NMR (600 MHz, CD₃OD) δ ppm 8.83 (d, *J =* 2.20 Hz, 1H), 8.35 (d, *J* = 8.07 Hz, 1H), 8.10 (dd, *J =* 8.07, 1.83 Hz, 1H), 7.57 - 7.68 (m, 3H), 7.51 - 7.57 (m, 1H), 6.88 (d, *J* = 7.34 Hz, 1H), 6.72 (d, *J* = 8.44 Hz, 1H), 5.46 - 5.59 (m, 2H), 4.69 - 4.76 (m, 1H), 3.80 (br d, *J* = 12.84 Hz, 1H), 3.35 (br d, *J* = 12.84 Hz, 1H), 3.05 (br d, *J* = 12.47 Hz, 1H), 2.93 - 2.99 (m, 1H), 1.95 (br d, *J =* 10.64 Hz, 1H), 1.71 - 1.87 (m, 3H). *m*/*z* (ESI, +ve ion) = 485.2 [M+H]⁺.

Example compounds **82, 97** (starting from **74c**), and **107** (from **72e**) were synthesized as described in Example **83.**

### Example 89. (S)-2-((4-(3-(4-Isopropyl-2-methylphenoxy)benzoyl)piperazin-1-yl)methyl)-1-(oxetan-2-ylmethyl)-1H-benzo[d]imidazole-6-carboxylic acid (89)

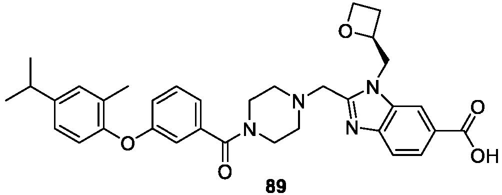

### Step A. Methyl 3-(4-isopropyl-2-methylphenoxy)-4-nitrobenzoate (89a)

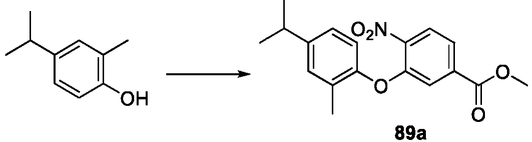

To a mixture of methyl 3-fluoro-4-nitrobenzoate (2.65 g, 13.3 mmol) in DMF (50 mL) was added 4-isopropyl-2-methylphenol (2 g, 13.3 mmol), and K₂CO₃ (5.5 g, 39.9 mmol). The mixture was stirred at 25 °C for one hour. After reaction completion, the reaction was quenched with water (50 mL), and extracted with EtOAc (50 mL x 3). The combined organic layers were dried over anhydrous Na₂SO₄, filtered, and concentrated to afford a crude residue, which was purified by silica gel column chromatography (petroleum:EtOAc = 5:1) to afford the title product (**89a**) (1.45 g, 56%) as a white solid. *m*/*z* (ESI, +ve ion) = 330.0 [M+H]⁺.

### Step B. Methyl 4-amino-3-(4-isopropyl-2-methylphenoxy)benzoate (89b)

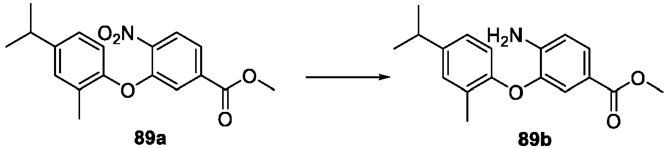

To a mixture of **89a** (3 g, 9.1 mmol) in MeOH (50 mL) was added 10% Pd/C (200 mg, 1.9 mmol). The mixture was stirred at 20 °C for 12 h under a H₂ atmosphere. After reaction completion, the mixture was filtered and concentrated under reduced pressure. The residue was purified by silica gel column chromatography (petroleum ether:EtOAc = 3:1) to afford the title product (**89b**) (2.52 g, 92 %) as a white solid. *m*/*z* (ESI, +ve ion) = 300.1 [M+H]⁺.

### Step C. Methyl 3-(4-isopropyl-2-methylphenoxy)benzoate (89c)

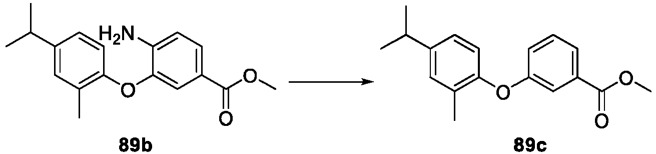

A mixture of isobutyl nitrite (1.4 g, 13.2 mmol) in DMF (20 mL) was stirred at 70 °C for 10 min. Then, **89b** (1.6 g, 5.3 mmol) was added to the above mixture. The reaction mixture was stirred at 70 °C for one hour. After completion, the reaction was quenched with H₂O (20 mL), and extracted with EtOAc (20 mL x 3). The organic layer was dried, filtered, and concentrated to afford a crude residue, which was purified by silica gel column chromatography (petroleum ether:EtOAc = 20:1) to afford the title product (**89c**) (0.7 g, 45 %) as a white solid.

### Step D. 3-(4-Isopropyl-2-methylphenoxy)benzoic acid (89d)

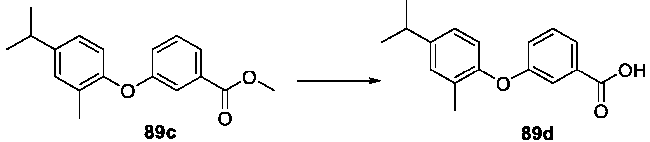

The title compound was prepared from **89c** by procedures similar to those described in Example **78,** Step D. *m*/*z* (ESI, +ve ion) = 271.1 [M+H]⁺.

### Step E. Methyl (S)-2-((4-(3-(4-isopropyl-2-methylphenoxy)benzoyl)piperazin-1-yl)methyl)-1-(oxetan-2-ylmethyl)-1H-benzo[d]imidazole-6-carboxylate (89e)

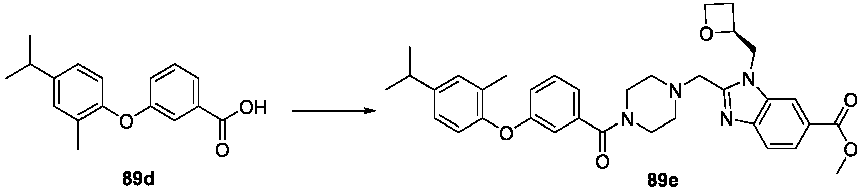

The title compound was prepared from **89d** by procedures similar to those described in Example **59,** Step D. *m*/*z* (ESI, +ve ion) = 597.3 [M+H]⁺.

### Step F. (S)-2-((4-(3-(4-Isopropyl-2-methylphenoxy)benzoyl)piperazin-1-yl)methyl)-1-(oxetan-2-ylmethyl)-1H-benzo[d]imidazole-6-carboxylic acid (89)

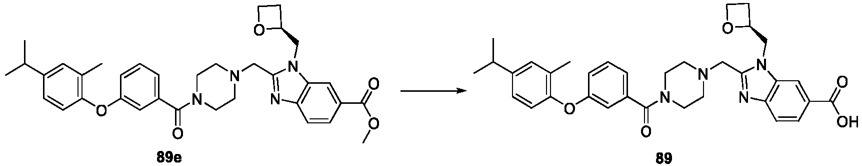

To a mixture of **89e** (80 mg, 0.13 mmol) in MeOH:H₂O (6 mL:2 mL) was added LiOH (64 mg, 2.68 mmol). The mixture was stirred at 20 °C for 5 h. After completion, the reaction was quenched with water (10 mL), adjusted to pH = 6 by 10% citric acid, and extracted with EtOAc (10 mL x 3). The organic layers were combined, dried, filtered, and concentrated to afford a crude residue, which was purified by reverse phase HPLC (CH₃CN:water with formic acid as a modifier) to give the title product (**89**) (20 mg, 26%) as a white solid. ¹H NMR (400 MHz, CD₃OD) δ ppm 8.46 (d, *J =* 0.9 Hz, 1H), 8.10 - 8.14 (m, 1H), 7.79 (d, *J =* 8.3 Hz, 1H), 7.36 - 743 (m, 1H), 7.17 (d, *J* = 1.8 Hz, 1H), 7.04 - 7.12 (m, 2H), 6.97 (ddd, *J =* 8.3, 2.6, 0.9 Hz, 1H), 6.84 - 6.89 (m, 2H), 5.28 - 5.17 (m, 1H), 4.92 - 4.87 (m, 1H), 4.73 (dd, *J* = 15.3, 2.6 Hz, 1H), 4.65 (td, *J* = 7.9, 5.7 Hz, 1H), 4.46 (dt, *J* = 9.2, 5.9 Hz, 1H), 4.30 - 4.39 (m, 2H), 3.85 (br s, 2H), 3.62 (br s, 2H), 2.97 - 2.79 (m, 6H), 2.48 - 2.55 (m, 1H), 2.15 (s, 3H), 1.25 (d, *J* = 8.0 Hz, 6H). *m*/*z* (ESI, +ve ion) = 583.3 [M+H]⁺.

### Example 90. (S)-2-((6-((4-Chloro-2-fluorobenzyl)oxy)-1,3,4,5-tetrahydro-2H-pyrido[4,3-b]indol-2-yl)methyl)-1-(oxetan-2-ylmethyl)-1H-benzo[d]imidazole-6-carboxylic acid (90)

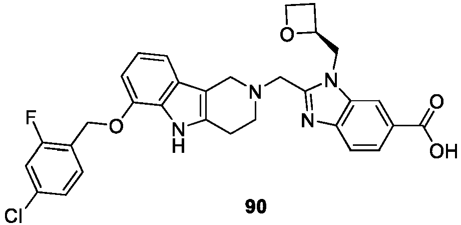

### Step A. 1-(Bromomethyl)-4-chloro-2-fluorobenzene (90a)

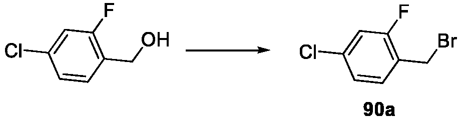

To a solution of (4-chloro-2-fluorophenyl)methanol (1 g, 6.2 mmol) in DCM (10 mL) was added PBr₃ (0.84 g, 3.1 mmol) at 0 °C, and the reaction was stirred at the same temperature for one hour. The mixture was diluted with DCM (50 mL), washed with sat. aq. NaHCO₃ (10 mL), H₂O (10 mL), dried over Na₂SO₄, filtered, and concentrated *in vacuo* to give the title product (**90a**) (1.05 g, 73%) as a yellow oil, which was used in the next step without further purification.

### Step B. 4-Chloro-2-fluoro-1-((2-iodophenoxy)methyl)benzene (90b)

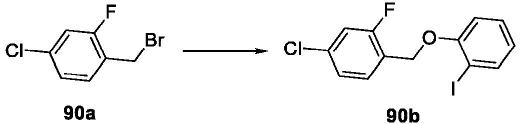

A mixture of **90a** (1.0 g, 4.5 mmol), 2-iodophenol (1.0 g, 4.5 mmol), and potassium carbonate (1.87 g, 13.4 mmol) in CH₃CN (20 mL) was stirred at 70 °C for 2 h. The mixture was diluted with EtOAc (100 mL), and washed with water (30 mL x 2). The organic layer was dried over Na₂SO₄, filtered, and concentrated *in vacuo* to give a crude residue. The residue was purified by silica gel column chromatography (EtOAc in petroleum ether 5-10%) to afford the title product (**90b**) (1.5 g, 82%) as a white solid. ¹H NMR (400 MHz, DMSO-*d*₆) δ ppm 7.79 (dd, *J* = 7.7, 1.2 Hz, 1H), 7.66 (t, *J=* 8.1 Hz, 1H), 7.52 (dd, *J =* 10.0, 1.8 Hz, 1H), 7.42 - 7.34 (m, 2H), 7.15 (d, *J=* 8.2 Hz, 1H), 6.79 (t, *J =* 7.5 Hz, 1H), 5.21 (s, 2H).

### Step C. tert-Butyl 2-(2-((4-chloro-2-fluorobenzyl)oxy)phenyl)hydrazine-1-carboxylate (90c)

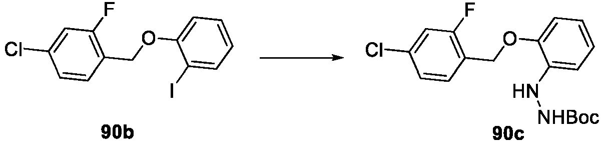

A mixture of **90b** (1.5 g, 4.1 mmol), *tert*-butyl carbazate (700 mg, 5.3 mmol), Cs₂CO₃ (2.0 g, 6.1 mmol), copper(I) iodide (80 mg, 0.4 mmol), and 3,4,7,8-tetramethyl-1,10-phenanthroline (0.1 g, 0.4 mmol) in DMF (30 mL) was stirred at 80 °C under N₂ for 28 h. The mixture was diluted with EtOAc (250 mL), washed with H₂O (100 mL), and then brine (100 mL). The organic layer was dried over Na₂SO₄, filtered, and concentrated *in vacuo* to give a crude residue, which was purified by silica gel column chromatography (EtOAc in petroleum ether 20-30%) to afford the title product (**90c**) (0.32 g, 17%) as a light-yellow oil. *m*/*z* (ESI, +ve ion) = 389.2 [M+Na]⁺.

### Step D. (2-((4-Chloro-2-fluorobenzyl)oxy)phenyl)hydrazine (90d)

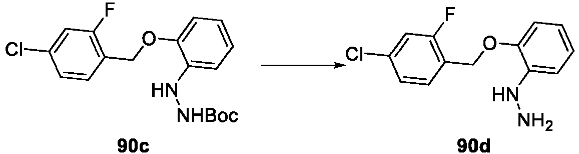

To a solution of 90c (330 mg, 0.9 mmol) in DCM (3 mL) was added 4 N HCl in dioxane (5 mL). The mixture was stirred at 20 °C for 6 h and then concentrated to give the title product (**90d**) (220 mg) as a brown solid, which was used in next step without further purification. *m*/*z* (ESI, +ve ion) = 250.1 [M-NH₂]⁺.

### Step E. 6-((4-Chloro-2-fluorobenzyl)oxy)-2,3,4,5-tetrahydro-1H-pyrido[4,3-b]indole (90e)

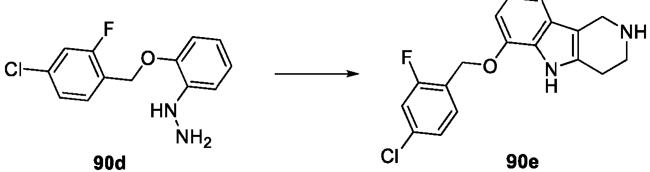

A mixture of **90d** (200 mg, 0.66 mmol) and *tert-butyl* 4-oxopiperidine-1-carboxylate (145 mg, 0.73 mmol) in EtOH (15 mL) and conc. HCl (3 mL) was stirred at 80 °C for 4 h. The mixture was concentrated *in vacuo* to give a crude residue, which was purified by reverse phase HPLC (37% CH₃CN in H₂O, 0.5% formic acid as a modifier) to give the title product (**90e**) (160 mg, 66%) as a brown solid. *m*/*z* (ESI, +ve ion) = 331.1 [M+H]⁺.

### Step F. Methyl (S)-2-((6-((4-chloro-2-fluorobenzyl)oxy)-1,3,4,5-tetrahydro-2H-pyrido[4,3-b]indol-2-yl)methyl)-1-(oxetan-2-ylmethyl)-1H-benzo[d]imidazole-6-carboxylate (90f)

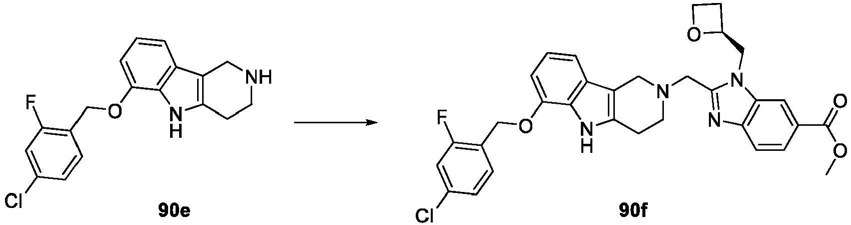

To a mixture of **90e** (61 mg, 0.186 mmol), and K₂CO₃ (70 mg, 0.51 mmol) in DMF (5 mL) was added **1h** (50 mg, 0.17 mmol), and KI (14 mg, 0.08 mmol) in DMF (5 mL). The resulting mixture was stirred at 22 °C for 5 h. The mixture was poured into water (20 mL), and extracted with EtOAc (20 mL x 2). The organic layer was washed with H₂O (10 mL), brine (10 mL), dried over Na₂SO₄, filtered, and concentrated *in vacuo* to give a crude residue, which was purified by Prep-TLC (silica gel, DCM:CH₃OH = 10:1) to give the title product (**90f**) (32 mg, 20%) as a yellow oil. *m*/*z* (ESI, +ve ion) = 589.2 [M+H]⁺.

### Step G. (S)-2-((6-((4-Chloro-2-fluorobenzyl)oxy)-1,3,4,5-tetrahydro-2H-pyrido[4,3-b]indol-2-yl)methyl)-1-(oxetan-2-ylmethyl)-1H-benzo[d]imidazole-6-carboxylic acid (90)

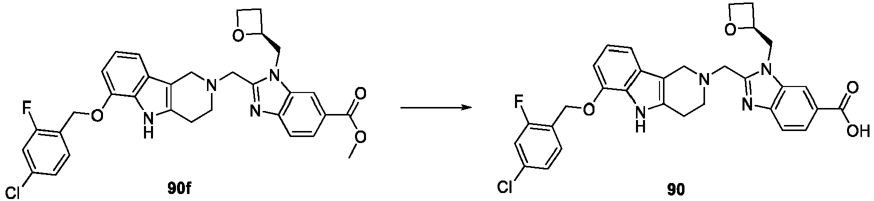

A mixture of **90f** (32 mg, 0.056 mmol), and lithium hydroxide (27 mg, 1.12 mmol) in CH₃OH:H₂O = 10:1 (6 mL) was stirred at 20 °C for 5 h. The mixture was adjusted to pH = 7 with 1 N HCl. The solvent was removed, and the residue was purified by reverse phase HPLC (27% CH₃CN in H₂O with 0.5% TFA as a modifier) to afford the title compound (**90**) (9.7 mg, 23.9%) as a white solid. ¹H NMR (400 MHz, DMSO-*d*₆) δ ppm 10.87 (s, 1H), 8.08 (s, 1H), 7.80 (dd, *J* = 8.6, 1.1 Hz, 1H), 7.76 (t, *J* = 8.1 Hz, 1H), 7.56 - 7.41 (m, 2H), 7.35 (dd, *J* = 8.1, 2.0 Hz, 1H), 6.91 (d, *J =* 7.9 Hz, 1H), 6.83 (t, *J =* 7.8 Hz, 1H), 6.70 (d, *J =* 7.7 Hz, 1H), 5.25 (s, 2H), 5.01 - 5.13 (m, 1H), 4.73 (dd, *J* = 14.9, 6.9 Hz, 1H), 4.56 - 4.65 (m, 1H), 4.45 (dd,*J* = 13.6, 8.3 Hz, 1H), 4.38 - 4.30 (m, 1H), 4.15 (d, *J* = 13.6 Hz, 1H), 4.03 (d, *J =* 13.2 Hz, 1H), 3.62 - 3.74 (m, 2H), 2.83 - 2.90 (m, 2H), 2.73 - 2.80 (m, 1H), 2.65 - 269 (m, 1H), 2.53 - 2.58 (m, 1H), 2.30 - 2.35 (m, 1H). *m*/*z* (ESI, +ve ion) = 575.1 [M+H]⁺.

Example compound **53** was synthesized as described in Example 90.

### Example 91. 5-(4-(6-((4-Cyano-2-fluorobenzyl)oxy)pyridin-2-yl)piperidine-1-carbonyl)picolinic acid (91)

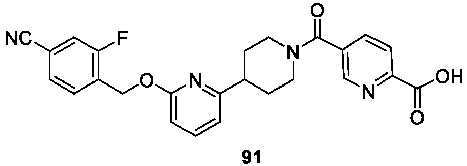

### Step A. Methyl 5-(4-(6-((4-cyano-2-fluorobenzyl)oxy)pyridin-2-yl)piperidine-1-carbonyl)picolinate (91a)

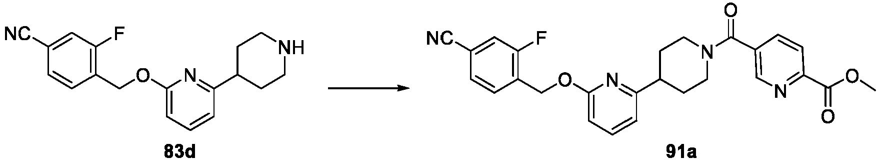

To a flask charged with **83d** (15 mg, 0.05 mmol), 6-(methoxycarbonyl)pyridine-3-carboxylic acid (14.5 mg, 0.08 mmol), and HATU (36.6 mg, 0.096 mmol) was added DMF (1 mL), followed by DIPEA (0.028 mL, 0.16 mmol). The reaction was stirred for 10 min and then was directly purified by reverse phase HPLC (CH₃CN and water with 0.1% TFA as a modifier) to give the title product (**91a**) (23 mg, 60.1%) as a white solid. *m*/*z* (ESI, +ve ion) = 475.2 [M+H]⁺.

### Step B. 5-(4-(6-((4-Cyano-2-fluorobenzyl)oxy)pyridin-2-yl)piperidine-1-carbonyl)picolinic acid (91)

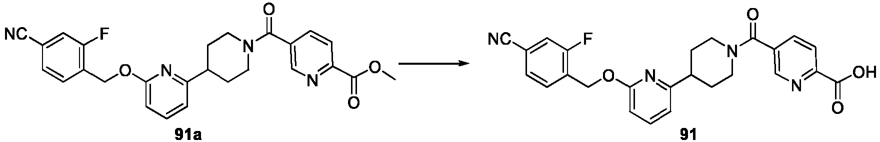

**91a** (23.0 mg, 0.049 mmol) was dissolved in CH₃CN (0.5 mL), followed by the addition of 2 N NaOH aqueous solution (0.5 mL). The mixture was stirred at room temperature overnight and purified by reverse phase HPLC (CH₃CN and water with 0.1% HOAc as a modifier) to give the title compound (**91**) as a white solid (13.6 mg, 60.9%). ¹H NMR (600 MHz, CD₃OD) δ ppm 8.74 (s, 1H), 8.25 (d, *J =* 8.07 Hz, 1H), 8.03 - 8.09 (m, 1H), 7.67 (t, *J =* 7.52 Hz, 1H), 7.57 - 7.64 (m, 2H), 7.51 - 7.57 (m, 1H), 6.87 (d, *J* = 7.34 Hz, 1H), 6.71 (d, *J =* 8.07 Hz, 1H), 5.46 - 5.59 (m, 2H), 4.66 - 4.77 (m, 1H), 3.72 (br d, *J =* 12.10 Hz, 1H), 3.30 - 3.39 (m, 1H), 2.98 - 3.06 (m, 1H), 2.94 (ddd, *J* = 11.55, 7.70, 3.85 Hz, 1H), 1.91 - 1.97 (m, 1H), 1.72 - 1.88 (m, 3H). *m*/*z* (ESI, +ve ion) = 461.2 [M+H]⁺.

### Example 92. 4-(((6-(4-(6-(1H-Tetrazol-5-yl)nicotinoyl)piperazin-1-yl)pyridin-2-yl)oxy)methyl)-3-fluorobenzonitrile (92)

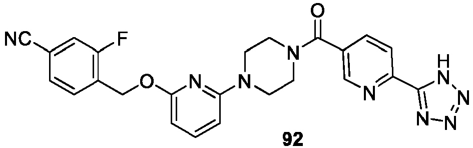

### Step A. 4-(((6-Chloropyridin-2-yl)oxy)methyl)-3-fluorobenzonitrile (92a)

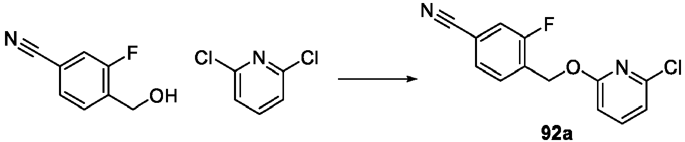

A solution of 3-fluoro-4-(hydroxymethyl)benzonitrile (151 mg, 1.0 mmol) in THF (2 mL) was cooled to 0 °C. KOtBu (168 mg, 1.5 mmol) was added portion wise. The resulting mixture was stirred at 0 °C for 5 min, followed by addition of 2,6-dichloropyridine (123 mg, 0.83 mmol). The reaction was slowly warmed up to room temperature, and stirred overnight. After completion, the reaction was quenched with sat. aq. NH₄Cl solution (5 mL), and extracted with ethyl acetate (5 mL x 3). The combined organic phase was washed with brine, dried with Na₂SO₄, filtered, then purified by silica gel column chromatography (hexanes:EtOAc = 5:1). Fractions containing the desired product was concentrated to give the title product (**92a**) (79.4 mg, 36.4%) *m*/*z* (ESI, +ve ion) = 263.2 [M+H]⁺.

### Step B. tert-Butyl 4-(6-((4-cyano-2-fluorobenzyl)oxy)pyridin-2-yl)piperazine-1-carboxylate (92b)

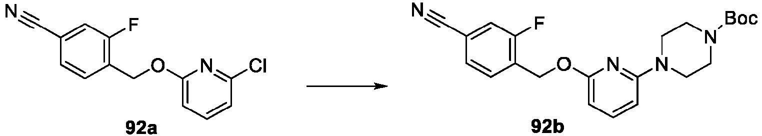

To a mixture of **92a** (79.4 mg, 0.3 mmol), *tert*-butyl piperazine-1-carboxylate (61.5 mg, 0.33 mmol), Pd₂(dba)₃ (13.7 mg, 0.015 mmol), BINAP (18.7 mg, 0.3 mmol), and Cs₂CO₃ (195 mg, 0.6 mmol) was added toluene (5 mL). The reaction mixture was stirred at 110 °C overnight. After completion, the reaction mixture was quenched by water (50 mL), and extracted with EtOAc (50 mL x 3). The combined organic layers were washed with brine, dried with Na₂SO₄, filtered, and concentrated. The crude residue was purified by reverse phase HPLC (CH₃CN and water with 0.1% TFA as a modifier) to give the title product (**92b**) (20.7 mg, 17 %) as a light brown solid. ¹H NMR (400 MHz, DMSO-*d6*) δ ppm 7.24 (d, *J =* 3.5 Hz, 1H), 6.46 (dd, *J =* 3.5, 0.5 Hz, 1H), 5.54 (d, *J =* 5.4 Hz, 1H), 4.78 - 4.68 (m, 1H), 3.79 (s, 3H), 1.38 (d, *J =* 6.6 Hz, 3H).

### Step 3. 3-Fluoro-4-(((6-(piperazin-1-yl)pyridin-2-yl)oxy)methyl)benzonitrile (92c)

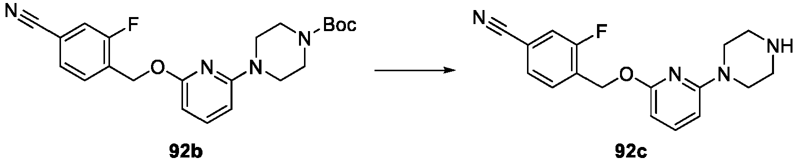

To a mixture of **92b** (20.7 mg, 0.05 mmol) in ethyl acetate (0.2 mL) was added 2 N HCl (1.2 mL). The reaction was stirred for 3 h, and the crude product was lyophilized to give the title product (**92c**) as a white solid (14.2 mg, 80 %). *m*/*z* (ESI, +ve ion) = 313.4 [M+H]⁺.

### Step D. 4-(((6-(4-(6-(1H-tetrazol-5-yl)nicotinoyl)piperazin-1-yl)pyridin-2-yl)oxy)methyl)-3-fluorobenzonitrile (92)

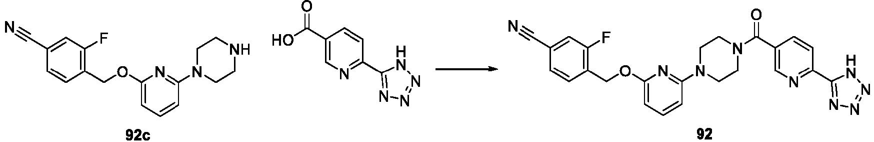

To a flask charged with **92c** (4 mg, 0.009 mmol), 6-(1*H*-tetrazol-5-yl)nicotinic acid (2.0 mg, 0.01 mmol), and HATU (4.3 mg, 0.011 mmol) was added DMF (1 mL), followed by DIPEA (0.005 mL, 0.028 mmol). The reaction was stirred for 10 min, and directly purified by reverse phase HPLC (CH₃CN and water with 0.1% HOAc as a modifier) to give the title compound (**92**) (1.1 mg, 24.1%) as a white solid. ¹H NMR (600 MHz, CD₃OD) δ ppm 8.22 - 8.41 (m, 2H), 8.08 (br d, *J =* 7.70 Hz, 1H), 7.65 (br t, *J =* 7.52 Hz, 1H), 7.56 (dd, *J =* 12.10, 8.80 Hz, 2H), 7.50 (t, *J =* 8.07 Hz, 1H), 6.36 (d, *J =* 8.07 Hz, 1H), 6.20 (d, *J=* 7.70 Hz, 1H), 5.46 (s, 2H), 3.84 (br s, 2H), 3.65 (br s, 2H), 3.56 (br s, 4H). *m*/*z* (ESI, +ve ion) = 486.3 [M+H]⁺.

### Examples 94 and 96. 2-((4-(5-((R)-1-(2,4-dichlorophenoxy)ethyl)furan-2-carbonyl)piperazin-1-yl)methyl)-1-(((S)-oxetan-2-yl)methyl)-1H-benzo[d]imidazole-6-carboxylic acid (94) and 2-((4-(5-((S)-1-(2,4-dichlorophenoxy)ethyl)furan-2-carbonyl)piperazin-1-yl)methyl)-1-(((S)-oxetan-2-yl)methyl)-1H-benzo[d]imidazole-6-carboxylic acid (96)

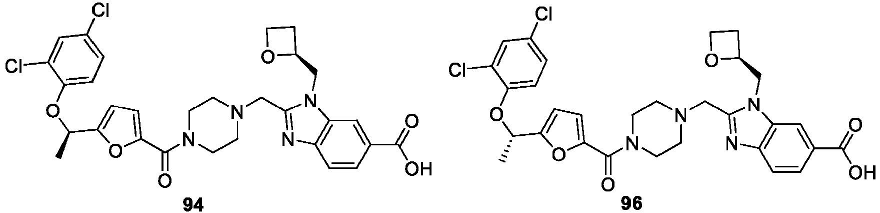

### Step A. Methyl 5-acetylfuran-2-carboxylate (94a)

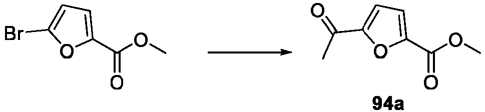

A solution of methyl 5-bromofuran-2-carboxylate (50 mg, 0.24 mmol) in anhydrous DMF (1.2 mL) was treated with tributyl-(1-ethoxyvinyl)stannane (106 mg, 0.29 mmol), and bis(triphenylphosphine)palladium(II) chloride (17 mg, 0.024 mmol) under nitrogen. The reaction was heated to 115 °C, and stirred for 18 h, cooled down, treated with 1 N HCl (5 mL), and stirred at 20 °C for 30 min. The reaction was then diluted with water (2 mL), and extracted with EtOAc (2 mL x 3). The organic layer was washed with water (2 mL), brine (2 mL), dried with anhydrous sodium sulfate, filtered, concentrated, and purified by silica gel column chromatography (EtOAc in petroleum ether 0-11%) to afford the title product (**94a**) (33 mg, 74.1%) as a yellow oil. *m*/*z* (ESI, +ve ion) = 169.1 [M+H]⁺.

### Step B. Methyl 5-(1-hydroxyethyl)furan-2-carboxylate (94b)

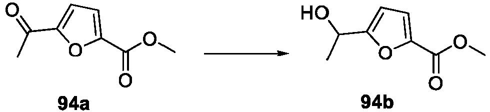

To a solution of **94a** (1.3 g, 7.7 mmol) in anhydrous MeOH (20 mL) at 0 °C was added NaBH₄ (0.15 g, 3.85 mmol) in portions. The reaction mixture was stirred for 2 h at 25 °C, and quenched with H₂O (3 mL). The organic solvent was removed under reduced pressure, and the residue was extracted with EtOAc (3 mL x 3). The combined organic extracts were washed with brine (5 mL), dried with Na₂SO₄, filtered, and concentrated. The resulting crude residue was purified by silica gel column chromatography (EtOAc in petroleum ether 0-20%) to afford the title product (**94b**) (1.2 g, 67.0%) as a yellow oil. *m*/*z* (ESI, +ve ion) = 240.1 [M+H]⁺.

### Step C. Methyl 5-(1-(2,4-dichlorophenoxy)ethyl)furan-2-carboxylate (94c)

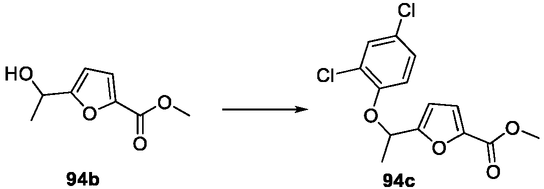

To a mixture of **94b** (50 mg, 0.29 mmol), 2,4-dichlorophenol (53 mg, 0.32 mmol), and triphenylphosphine (85 mg, 0.32 mmol) in THF (2.5 mL) was added diethyl azodicarboxylate (56 mg, 0.32 mmol) at 0 °C. The mixture was stirred at 25 °C for 16 h under N₂, and concentrated. The crude residue was purified by silica gel column chromatography (EtOAc in petroleum ether 0-10%) to afford the title product (**94c**) (31 mg, 30.1%) as a yellow oil. *m*/*z* (ESI, +ve ion) = 337.0 [M+Na]⁺.

### Step D. 5-(1-(2,4-Dichlorophenoxy)ethyl)furan-2-carboxylic acid (94d)

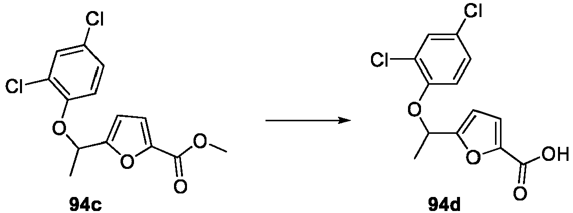

A solution of **94c** (15 mg, 0.05 mmol) in acetonitrile (0.5 mL) was treated with lithium hydroxide (6 mg, 0.24 mmol) in water (0.5 mL) at 0 °C, and stirred at 25 °C for 2 h. The mixture was concentrated, acidified to pH = 2 by 1 N HCl, and extracted with EtOAc (2 mL x 3). The organic phase was washed with brine (2 mL), dried with anhydrous sodium sulfate, and concentrated to afford the title product (**94d**) (11 mg) as a yellow oil, which was used directly in the next step without further purification. *m*/*z* (ESI, -ve ion) = 299.0 [M-H]⁻.

### Step E. Methyl 2-((4-(5-(1-(2,4-dichlorophenoxy)ethyl)furan-2-carbonyl)piperazin-1-yl)methyl)-1-(((S)-oxetan-2-yl)methyl)-1H-benzo[d]imidazole-6-carboxylate (94e)

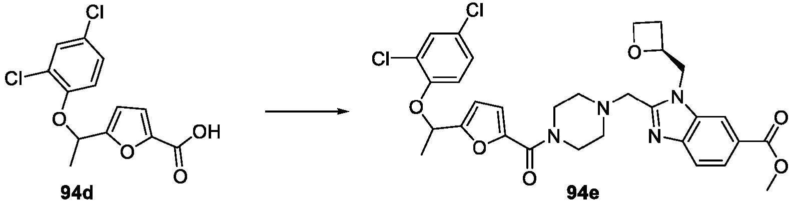

**1j** (213 mg, 0.62 mmol), and **94d** (186 mg, 0.62 mmol) were dissolved in DMF (3 mL). Diisopropylethylamine (319 mg, 2.47 mmol), and HATU (352 mg, 0.93 mmol) were added under a N₂ atmosphere, and the mixture was stirred at 20 °C for 2 h. The reaction was quenched with water (10 mL), and extracted with EtOAc (10 mL x 3). The combined organic phase was washed with water (5 mL), brine (5 mL), dried with anhydrous sodium sulfate, filtered, and concentrated. The crude residue was purified by silica gel column chromatography (MeOH in DCM 5-10% with 0.5% formic acid) to afford the title product (**94e**) (180 mg, 42.5%) as a yellow oil. *m*/*z* (ESI, +ve ion) = 627.2 [M+H]⁺.

### Step F. 2-((4-(5-((R)-1-(2,4-Dichlorophenoxy)ethyl)furan-2-carbonyl)piperazin-1-yl)methyl)-1-(((S)-oxetan-2-yl)methyl)-1H-benzo[d]imidazole-6-carboxylic acid (94) and 2-((4-(5-((S)-1-(2,4-dichlorophenoxy)ethyl)furan-2-carbonyl)piperazin-1-yl)methyl)-1-(((S)-oxetan-2-yl)methyl)-1H-benzo[d]imidazole-6-carboxylic acid (96)

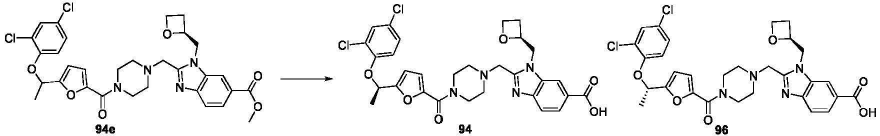

A solution of **94e** (50 mg, 0.08 mmol) in MeOH (1 mL) was treated with sodium hydroxide (13 mg, 0.21 mmol) in water (1 mL) at 0 °C, and the reaction mixture was stirred at 20 °C for 4 h. The mixture was concentrated, and then diluted with water (5 mL), adjusted to pH = 5 with 0.1 N HCl, and extracted with EtOAc (5 mL x 3). The organic phase was washed with water (2 mL), brine (2 mL), dried with anhydrous sodium sulfate, filtered, and concentrated. The mixture was purified by silica gel column chromatography (MeOH in DCM 5-10%, 0.5% formic acid), and further separated using a chiral column (ChiralPak OJ, 4.6*150 mm, 3 µm) with CO₂/MeOH 75:25 as the mobile phase, and a flow rate of 2.0 mL/min to afford the title compounds (**94**, 1^{st} eluting peak) (4.1 mg, 8.03%) and (**96**, 2^{nd} eluting peak) (5 mg, 9.3%) as white solids. Data for **94:** ¹H NMR (400 MHz, CD₃OD) δ ppm 8.34 (s, 1H), 7.98 (br d, *J* = *8.56* Hz, 1H), 7.68 (br d, *J* = 8.56 Hz, 1H), 7.34 (br d, *J =* 2.45 Hz, 1H), 7.18 (br dd, *J =* 8.80, 2.45 Hz, 1H), 7.12 (br d, *J =* 8.80 Hz, 1H), 6.97 (d, *J =* 3.42 Hz, 1H), 6.54 (d, *J=* 3.67 Hz, 1H), 5.54 (q, *J =* 6.36 Hz, 1H), 5.21 - 5.29 (m, 1H), 4.72 (br dd, *J =* 15.28, 2.57 Hz, 1H), 4.60 - 4.68 (m, 2H), 4.46 (dt, *J* = 9.11, 5.96 Hz, 1H), 4.04 (br d, *J =* 13.94 Hz, 1H), 3.94 (br d, *J =* 13.94 Hz, 1H), 3.67 - 3.75 (m, 4H), 2.75 - 2.85 (m, 1H), 2.44 - 2.63 (m, 5H), 1.73 (d, *J =* 6.36 Hz, 3H). *m*/*z* (ESI, +ve ion) = 613.2 [M+H]⁺. Data for **96:** ¹H NMR (400 MHz, CD₃OD) δ ppm 8.31 (s, 1H), 7.97 (br d, *J=* 8.56 Hz, 1H), 7.67 (br d, *J =* 8.31 Hz, 1H), 7.27 - 7.35 (m, 1H), 7.19 (dd, *J =* 8.80, 2.69 Hz, 1H), 7.12 (d, *J =* 9.05 Hz, 1H), 6.97 (d, *J =* 3.42 Hz, 1H), 6.54 (d, *J* = 3.42 Hz, 1H), 5.54 (q, *J* = 6.36 Hz, 1H), 5.26 (qd, *J =* 7.17, 2.69 Hz, 1H), 4.72 (br dd, *J =* 15.41, 2.69 Hz, 1H), 4.56 - 4.68 (m, 2H), 4.45 (dt, *J =* 9.23, 5.90 Hz, 1H), 4.03 (br d, *J* = 13.69 Hz, 1H), 3.94 (br d, *J =* 13.69 Hz, 1H), 3.71 (br s, 4H), 2.80 (ddd, *J =* 10.76, 5.75, 2.57 Hz, 1H), 2.40 - 2.67 (m, 5H), 1.73 (d, *J =* 6.60 Hz, 3H). *m*/*z* (ESI, +ve ion) = 613.2 [M+H]⁺. The stereochemistries of **94,** and **96** are assigned arbitrarily.

### Example 95. (S)-2-((4-(3-((2-Chloro-4-methylphenyl)(methyl)amino)-4-fluorobenzoyl)piperazin-1-yl)methyl)-1-(oxetan-2-ylmethyl)-1H-benzo[d]imidazole-6-carboxylic acid (95)

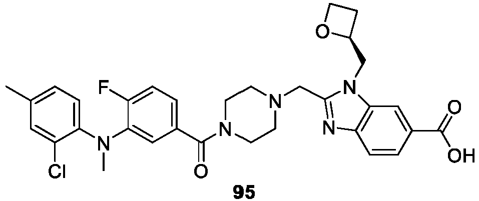

### Step A. Methyl (S)-2-((4-(3-((2-chloro-4-methylphenyl)(methyl)amino)-4-fluorobenzoyl)piperazin-1-yl)methyl)-1-(oxetan-2-ylmethyl)-1H-benzo[d]imidazole-6-carboxylate (95a)

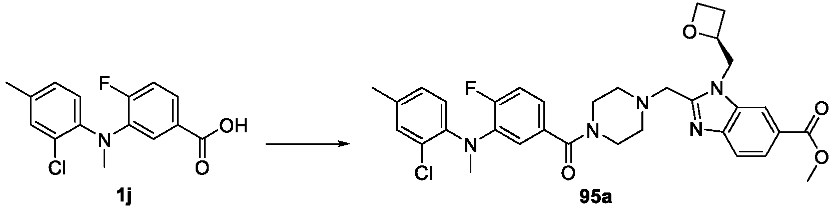

**1j** (80 mg, 0.23 mmol), and 3-((2-chloro-4-methylphenyl) (methyl)amino)-4-fluorobenzoic acid (68 mg, 0.23 mmol, prepared by procedures similar to those described in Example **46,** Steps A to C) were dissolved in DCM (2 mL). DIPEA (0.16 mL, 0.93 mmol), and propylphosphonic anhydride solution (50 wt.% in ethyl acetate, 442 mg, 0.69 mmol) was added. The solution was stirred at 20 °C for one hour and concentrated. The mixture was purified by silica gel column chromatography (MeOH in DCM 5-10%) to afford the title product (**95a**) (61 mg, 43.9 %) as a yellow solid. *m*/*z* (ESI, +ve ion) = 620.2 [M+H]⁺.

### Step B. (S)-2-((4-(3-((2-Chloro-4-methylphenyl)(methyl)amino)-4-fluorobenzoyl)piperazin-1-yl)methyl)-1-(oxetan-2-ylmethyl)-1H-benzo[d]imidazole-6-carboxylic acid (95)

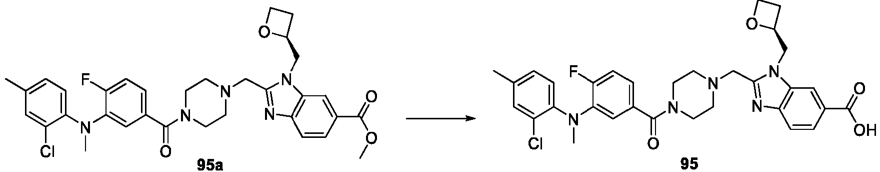

To a solution of **95a** (65 mg, 0.1 mmol) in MeOH:H₂O = 5:1 (1.2 mL) was added sodium hydroxide (21 mg, 0.5 mmol) at 0 °C. The reaction mixture was then warmed to room temperature and stirred for 5 h. The mixture then was concentrated, diluted with water (5 mL), adjusted to pH = 4 with 0.1 N HCl, and extracted with EtOAc (5 mL x 3). The organic phase was washed with water (5 mL), brine (5 mL), dried with anhydrous sodium sulfate, filtered, and concentrated. The crude mixture was purified by silica gel column chromatography (MeOH in DCM 5-10%, gradient elution) to afford the title product (**95**) (10.4 mg, 16.0%) as a white solid. ¹H NMR (400 MHz, CD₃OD) δ ppm 8.34 (d, *J =* 0.98 Hz, 1H), 7.97 (dd, *J =* 8.56, 1.47 Hz, 1H), 7.68 (d, *J* = 8.56 Hz, 1H), 7.25 (s, 1H), 7.02 - 7.18 (m, 3H), 6.96 (ddd, *J* = 8.31, 4.16, 1.96 Hz, 1H), 6.85 (dd, *J =* 8.44, 2.08 Hz, 1H), 5.24 (qd, *J =* 7.21, 2.32 Hz, 1H), 4.78 - 4.85 (m, 1H), 4.71 (br dd, *J =* 15.41, 2.69 Hz, 1H), 4.63 (td, *J* = 7.82, 6.11 Hz, 1H), 4.44 (dt, *J=* 9.05, 5.87 Hz, 1H), 3.99 - 4.06 (m, 1H), 3.90 - 3.96 (m, 1H), 3.72 (br s, 2H), 3.48 (br s, 2H), 3.25 (s, 3H), 2.74 - 2.86 (m, 1H), 2.39 - 2.69 (m, 5H), 2.32 (s, 3H). *m*/*z* (ESI, +ve ion) = 606.2 [M+H]⁺.

Example compounds **98,** and **100** were synthesized as described in Example **95.**

### Example 99. (S)-2-((4-(2-((2-Chloro-4-methylphenyl)(methyl)amino)isonicotinoyl)piperazin-1-yl)methyl)-1-(oxetan-2-ylmethyl)-1H-benzo[d]imidazole-6-carboxylic acid (99)

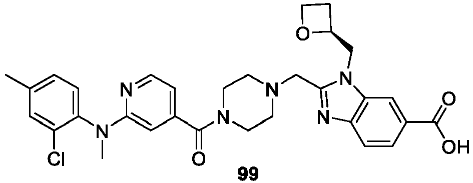

### Step A. tert-Butyl 4-(2-bromoisonicotinoyl)piperazine-1-carboxylate (99a)

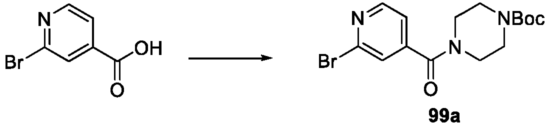

A solution of 2-bromopyridine-4-carboxylic acid (1.0 g, 0.005 mol), *tert*-butyl piperazine-1-carboxylate (0.93 g, 0.005 mol), DIPEA (3.5 mL, 0.02 mol), and propylphosphonic anhydride (T₃P) (6.36 g, 0.02 mol) in DCM (10 mL) was stirred at 20 °C for 3 h. After completion, the reaction was diluted with H₂O (50 mL), and extracted with EtOAc (50 mL x 3). The organic layer was dried, concentrated and purified by silica gel column chromatography (petroleum ether:EtOAc = 1:2) to afford the title product (**99a**) (1.85 g, 94 %) as yellow solid. *m*/*z* (ESI, +ve ion) = 370.1 [M+H]⁺.

### Step B. tert-Butyl 4-(2-((2-chloro-4-methylphenyl)amino)isonicotinoyl)piperazine-1-carboxylate (99b)

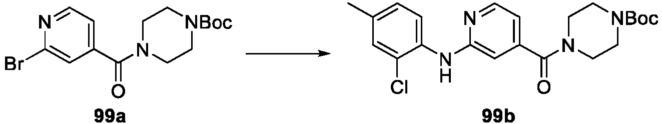

A solution of **99a** (1.27 g, 3.4 mmol), 2-chloro-4-methylaniline (0.48 g, 3.4 mmol), chloro(2-dicyclohexylphosphino-2',6'-diisopropoxy-1,1'-biphenyl) [2-(2'-amino-1,1'-biphenyl)]palladium(II) (0.53 g, 0.6 mmol), and Cs₂CO₃ (2.82 g, 10.0 mmol) in dioxane (12 mL) was stirred at 95 °C for 12 h. After completion, the reaction was diluted with H₂O (50 mL), and extracted with EtOAc (50 mL x 3). The organic layer was dried, filtered, concentrated, and purified by silica gel column chromatography (CH₂Cl₂:MeOH = 50:1) to afford the title product (**99b**) (0.85 g, 56%) as a yellow oil. *m*/*z* (ESI, +ve ion) = 431.2 [M+H]⁺.

### Step C. tert-Butyl 4-(2-((2-chloro-4-methylphenyl)(methyl)amino)isonicotinoyl)piperazine-1-carboxylate (99c)

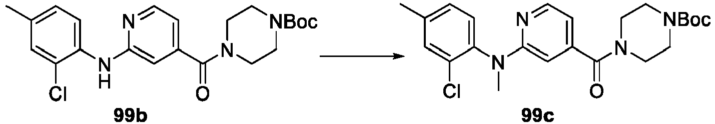

A solution of **99b** (652 mg, 1.51 mmol), and NaH (60% in mineral oil, 72 mg, 3.03 mmol) in DMF (8 mL) was stirred at 0 °C for 0.5 h. Then MeI (258 mg, 1.81 mmol) was added. The resulting mixture was stirred at 0 °C for 1.5 h. After completion, the reaction was quenched with H₂O (1 mL), and extracted with EtOAc (10 mL x 3). The organic layer was dried, filtered, concentrated, and purified by silica gel column chromatography (DCM:MeOH = 50:1) to afford the title product (**99c**) (575 mg, 81%) as a yellow solid. *m*/*z* (ESI, +ve ion) = 445.2 [M+H]⁺.

### Step D. (2-((2-Chloro-4-methylphenyl)(methyl)amino)pyridin-4-yl)(piperazin-1-yl)methanone (99d)

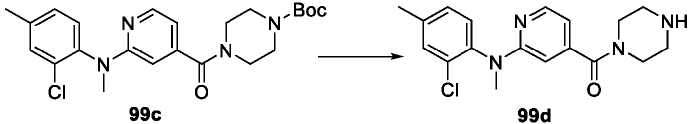

A solution of **99c** (295 mg, 0.66 mmol), and 2 N HCl in EtOAc solution (5 mL) was stirred at 20 °C for 3 h. After completion, the reaction was cooled down in an ice bath. Sat. aq. NaHCO₃ solution was added to the mixture to adjust to pH = 7. The reaction was diluted with H₂O (10 mL), and extracted with EtOAc (20 mL x 3). The organic layer was dried, concentrated and purified by reversed phase HPLC (30% CH₃CN in H₂O with 0.05% formic acid as a modifier) to afford the title product (**99d**) (129 mg, 52%) as a yellow solid. *m*/*z* (ESI, +ve ion) = 345.1 [M+H]⁺.

### Step E. Methyl (S)-2-((4-(2-((2-chloro-4-methylphenyl)(methyl)amino)isonicotinoyl)piperazin-1-yl)methyl)-1-(oxetan-2-ylmethyl)-1H-benzo[d]imidazole-6-carboxylate (99e)

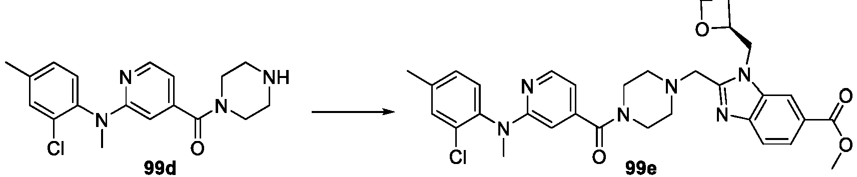

A solution of **99d** (84 mg, 0.24 mmol), **1h** (72 mg, 0.24 mmol), and K₂CO₃ (67 mg, 0.49 mmol) in DMF (2 mL) was stirred at 20 °C for 5 h. After completion, the reaction was diluted with H₂O (20 mL), and extracted with EtOAc (20 mL x 3). The organic layer was dried, filtered, concentrated, and purified by silica gel column chromatography (DCM:MeOH = 50:1) to afford the title product (**99e**) (45 mg, 29%) as a white solid. *m*/*z* (ESI, +ve ion) = 603.2 [M+H]⁺.

### Step F. (S)-2-((4-(2-((2-Chloro-4-methylphenyl)(methyl)amino)isonicotinoyl)piperazin-1-yl)methyl)-1-(oxetan-2-ylmethyl)-1H-benzo[d]imidazole-6-carboxylic acid (99)

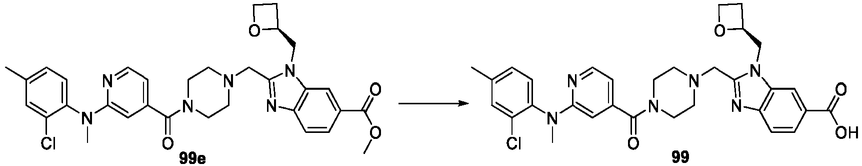

A solution of **99e** (46 mg, 0.076 mmol), and LiOH (9 mg, 0.38 mmol) in THF:H₂O = 1:1 (1 mL) was stirred at 20 °C for 3 h. After completion, the reaction was cooled down in an ice bath, and 1 N HCl was added to the mixture to adjust to pH = 5, and the reaction was extracted with EtOAc (50 mL x 3). The organic layer was dried, filtered, concentrated, and purified by reverse phase HPLC (25% CH₃CN in H₂O with 0.05% formic acid as a modifier) to afford the title product (**99**) (9.8 mg, 21.4%) as a white solid. ¹H NMR (400 MHz, CD₃OD) δ ppm 8.32 (s, 1H), 8.18 (d, *J =* 5.14 Hz, 1H), 7.97 (br d, *J =* 8.31 Hz, 1H), 7.67 (d, *J =* 8.31 Hz, 1H), 7.42 (s, 1H), 7.26 (d, *J =* 0.98 Hz, 2H), 6.63 (dd, *J* = 5.14, 1.22 Hz, 1H), 6.07 (s, 1H), 5.23 (qd, *J =* 6.89, 2.08 Hz, 1H), 4.81 - 4.85 (m, 1H), 4.69 (br dd, *J =* 15.41, 2.45 Hz, 1H), 4.56 - 4.66 (m, 1H), 4.43 (dt, *J =* 9.05, 5.87 Hz, 1H), 4.01 (d, *J =* 13.69 Hz, 1H), 3.91 (d, *J =* 13.94 Hz, 1H), 3.69 (br s, 2H), 3.33 - 3.44 (m, 5H), 2.68 - 2.89 (m, 1H), 2.41 - 2.64 (m, 5H), 2.40 (s, 3H). *m*/*z* (ESI, +ve ion) = 589.2 [M+H]⁺.

Example compounds **86, 93,** and **101** were synthesized as described in Example **99.**

### Example 102. (S)-2-((4-(2-((2,4-Dichlorophenoxy)methyl)oxazole-5-carbonyl)piperazin-1-yl)methyl)-1-(oxetan-2-ylmethyl)-1H-benzo[d]imidazole-6-carboxylic acid (102)

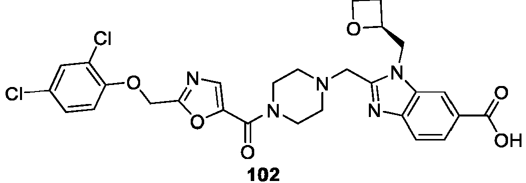

### Step A. Methyl 2-(bromomethyl)oxazole-5-carboxylate (102a)

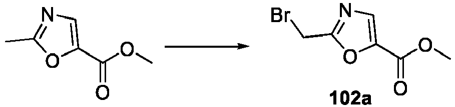

To a stirred solution of NBS (1 g, 7.1 mmol), and AIBN (0.47 g, 2.8 mmol) in CCl₄ (20 mL) at 80 °C was added methyl 2-methyl-1,3-oxazole-5-carboxylate (1.9 g, 10.6 mmol). The reaction mixture was stirred at 80 °C for 16 h. After completion, the reaction was diluted with H₂O (15 mL), and extracted with EtOAc (20 mL x 3). The combined organic layers were dried, filtered, and concentrated to give a crude residue, which was purified by silica gel column chromatography (EtOAc:petroleum ether = 1:10) to afford the title product (**102a**) (0.8 g, 49%) as a yellow oil. *m*/*z* (ESI, +ve ion) = 220.1 [M+H]⁺.

### Step B. Methyl 2-((2,4-dichlorophenoxy)methyl)oxazole-5-carboxylate (102b)

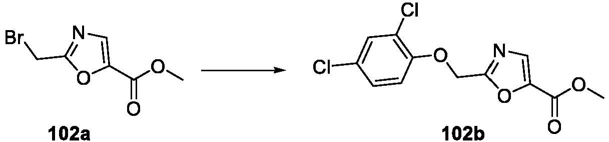

To a solution of **102a** (300 mg, 1.36 mmol), and 2,4-dichlorophenol (333 mg, 2.0 mmol) in CH₃CN (10 mL) was added K₂CO₃ (565 mg, 4.1 mmol). The reaction mixture was stirred at 20 °C for 2 h. After completion, the reaction solution was diluted with H₂O (10 mL), and extracted with EtOAc (20 mL x 3). The combined organic layers were dried, filtered, and concentrated to give a crude residue, which was purified by silica gel column chromatography (EtOAc:petroleum ether = 1:10) to afford the title product (**102b**) (267 mg, 62%) as a white solid. *m*/*z* (ESI, +ve ion) = 302.2 [M+H]⁺.

### Step C. 2-((2,4-Dichlorophenoxy)methyl)oxazole-5-carboxylic acid (102c)

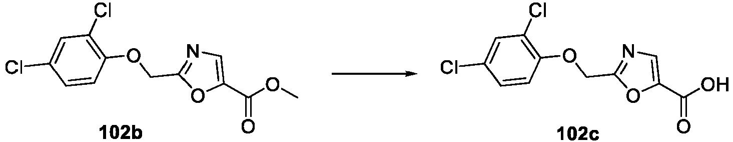

To a stirred solution of **102b** (300 mg, 0.99 mmol) in MeOH (5 mL) was added a solution of NaOH (198 mg, 4.97 mmol) in H₂O (1 mL). The reaction mixture was stirred at 20 °C for 2 h, and cooled down in an ice bath. 1 N HCl (2.0 mL) was added to adjust to pH = 5. The reaction mixture was then extracted with EtOAc (5 mL x 3). The combined organic layers were dried, filtered, and concentrated to give a crude residue, which was purified by silica gel column chromatography (EtOAc in petroleum ether 20-30%) to afford the title product (**102c**) (210 mg, 70%) as a white solid. *m*/*z* (ESI, +ve ion) = 288.0 [M+H]⁺.

### Step D. Methyl (S)-2-((4-(2-((2,4-dichlorophenoxy)methyl)oxazole-5-carbonyl)piperazin-1-yl)methyl)-1-(oxetan-2-ylmethyl)-1H-benzo[d]imidazole-6-carboxylate (102d)

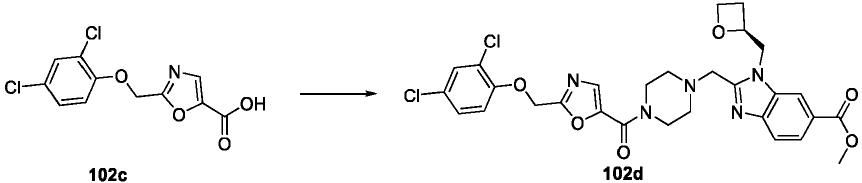

To a stirred solution of **102c** (100 mg, 0.35 mmol), **1j** (143 mg, 0.42 mmol), and DIPEA (0.18 mL, 1.04 mmol) in DCM (3 mL) was added T₃P (221 mg, 0.694 mmol, 2 equiv) at 20 °C. The reaction mixture was stirred at 20 °C for 2 h. After completion, the reaction solution was diluted with H₂O (5 mL), and extracted with EtOAc (20 mL x 3). The combined organic layers were dried, filtered, and concentrated to give a crude residue which was purified by silica gel column chromatography (EtOAc:petroleum ether = 1:5) to afford the title product **(102d)** (90 mg, 40%) as a white solid. *m*/*z* (ESI, +ve ion) = 614.1 [M+H]⁺.

### Step E. (S)-2-((4-(2-((2,4-Dichlorophenoxy)methyl)oxazole-5-carbonyl)piperazin-1-yl)methyl)-1-(oxetan-2-ylmethyl)-1H-benzo[d]imidazole-6-carboxylic acid (102)

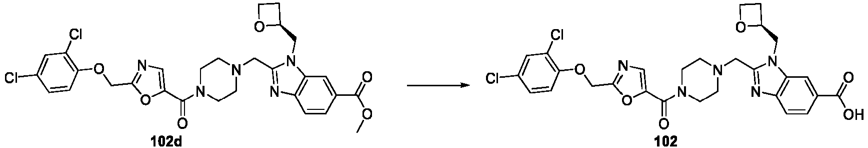

To a stirred solution of **102d** (10 mg, 0.016 mmol) in CH₃CN:H₂O = 1:1 (2 mL) was added TBD (5 mg, 0.033 mmol) at 20 °C. The reaction mixture was stirred for 2 h, and cooled down in an ice bath. 1 N HCl (0.1 mL) was added to adjust to pH = 5. The reaction mixture was then extracted with EtOAc (5 mL x 3). The combined organic layers were dried, filtered, and concentrated to give a crude residue, which was purified by silica gel column chromatography (EtOAc in petroleum ether 20-30%) to afford the title product (**102**) (3.2 mg, 30%) as a white solid. ¹H NMR (400 MHz, CD₃OD) δ ppm 8.30 (s, 1H), 7.97 (br d, *J =* 8.77 Hz, 1H), 7.62 - 7.68 (m, 2H), 7.41 (d, *J =* 2.63 Hz, 1H), 7.24 - 7.30 (m, 1H), 7.21 (d, *J =* 8.77 Hz, 1H), 5.34 (s, 2H), 5.26 (qd, *J =* 7.24, 2.85 Hz, 1H), 4.89 - 4.92 (m, 1H), 4.71 (br dd, *J =* 15.57, 2.85 Hz, 1H), 4.60 - 4.67 (m, 1H), 4.41 - 4.49 (m, 1H), 4.00 - 4.07 (m, 1H), 3.92 - 3.99 (m, 1H), 3.69 - 3.80 (m, 4H), 2.79 (br d, *J =* 5.70 Hz, 1H), 2.46 - 2.64 (m, 5H). *m*/*z* (ESI, +ve ion) = 600.1 [M+H]⁺.

Example compound **113** was synthesized as described in Example **102.**

### Example 105. (S)-2-((4-(1-(3-((2-chloro-4-methylphenyl)(methyl)amino)phenyl)cyclopropyl)piperazin-1-yl)methyl)-1-(oxetan-2-ylmethyl)-1H-benzo[d]imidazole-6-carboxylic acid (105)

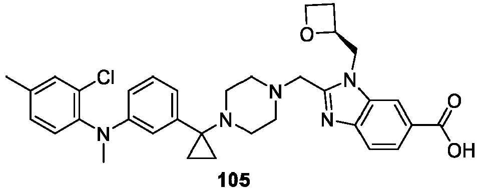

### Step A. 1-(1-(3-Bromophenyl)cyclopropyl)-4-tosylpiperazine (105a)

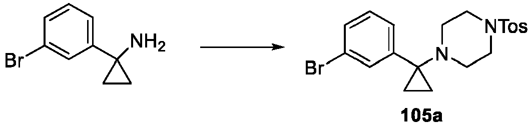

A flask was charged with 1-(3-bromophenyl)cyclopropan-1-amine (50 mg, 0.24 mmol). DIPEA (1 mL), and *N,N*-bis(2-chloroethyl)-4-methylbenzenesulfonamide (77 mg, 0.26 mmol) were added. The reaction mixture was stirred at 120 °C for 40 h. The reaction was diluted with water (10 mL) and extracted with EtOAc (10 mL x 3). The organic phase was washed with water (5 mL), brine (5 mL), dried with anhydrous sodium sulfate, filtered, and concentrated. The crude residue was purified by silica gel column chromatography (EtOAc in petroleum ether 0-20%) to afford the title product (**105a**) (53 mg, 46%) as a yellow solid. *m*/*z* (ESI, +ve ion) = 435.1 [M+H]⁺.

### Step B. 2-Chloro-4-methyl-N-(3-(1-(4-tosylpiperazin-1-yl)cyclopropyl)phenyl)aniline (105b)

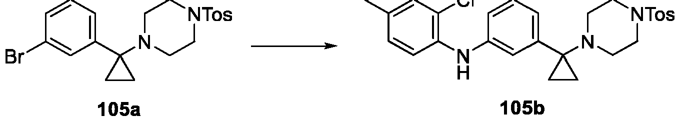

To a solution of **105a** (18 mg, 0.04 mmol), cesium carbonate (27 mg, 0.08 mmol), palladium diacetate (1 mg, 0.004 mmol), and racemic-2,2'-bis(diphenylphosphino)-1,1'-binaphthyl (3 mg, 0.04 mmol) in toluene (0.5 mL) was added 2-chloro-4-methylaniline (6 mg, 0.04 mmol). The reaction was stirred at 100 °C for 2 h. After completion, the mixture was concentrated. The reaction was diluted with water (5 mL), and extracted with EtOAc (5 mL x 3). The organic phase was washed with water (5 mL), brine (5 mL), dried with anhydrous sodium sulfate, filtered, and concentrated to give a crude residue, which was purified by silica gel column chromatography (EtOAc in petroleum ether 0-20%) to afford the title product (**105b**) (12 mg, 53%) as a yellow solid. *m*/*z* (ESI, +ve ion) = 496.2 [M+H]⁺.

### Step C. 2-Chloro-N,4-dimethyl-N-(3-(1-(4-tosylpiperazin-1-yl)cyclopropyl)phenyl)aniline (105c)

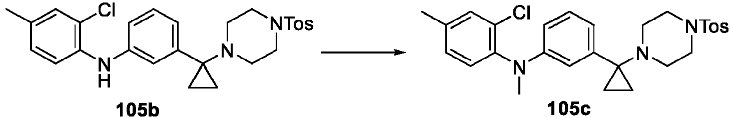

NaH (60% in mineral oil, 72 mg, 1.8 mmol) was added in successive portions to a solution of **105b** (446 mg, 0.9 mmol) in anhydrous DMF (5 mL) under a nitrogen atmosphere at 0 °C, followed by dropwise addition of iodomethane (255 mg, 1.8 mmol). The reaction mixture was stirred at 20 °C for one hour and quenched with water (20 mL) at 0 °C. The mixture was extracted with EtOAc (20 mL x 3), and the organic phase was washed with water (10 mL), brine (10 mL), dried with anhydrous sodium sulfate, filtered, and concentrated. The crude residue was purified by silica gel column chromatography (EtOAc in petroleum ether 0-20%) to afford the title product (**105c**) (12 mg, 58%) as a yellow oil. *m*/*z* (ESI, +ve ion) = 510.2 [M+H]⁺.

### Step D. 2-Chloro-N,4-dimethyl-N-(3-(1-(piperazin-1-yl)cyclopropyl)phenyl)aniline (105d)

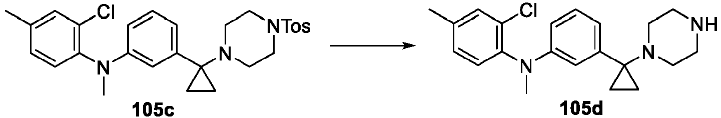

To a solution of **105c** (240 mg, 0.47 mmol) in trifluoroacetic acid (4 mL) was added conc. H₂SO₄ (455 mg, 3.29 mmol). The mixture was heated at 75 °C, and stirred for 3 h. After completion, the reaction was diluted with water (10 mL), and adjusted to pH = 11 with NH₃·H₂O. The mixture was extracted with EtOAc (20 mL x 3), and the organic phase was washed with water (10 mL), brine (10 mL), dried with anhydrous sodium sulfate, filtered, and concentrated. The crude was purified by silica gel column chromatography (MeOH in dichloromethane 5-10%, 0.5%NH₃·H₂O as a modifier) to afford the title product (**105d**) (139 mg) as a white solid that was impure. *m*/*z* (ESI, +ve ion) = 356.2 [M+H]⁺.

### Step E. Methyl (S)-2-((4-(1-(3-((2-chloro-4-methylphenyl)(methyl)amino)phenyl)cyclopropyl)piperazin-1-yl)methyl)-1-(oxetan-2-ylmethyl)-1H-benzo[d]imidazole-6-carboxylate (105e)

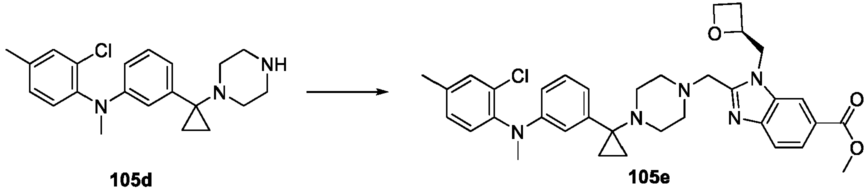

To a solution of **105d** (139 mg, 0.39 mmol) in DMF (3 mL) was added **1h** (127 mg, 0.43 mmol), and potassium carbonate (162 mg, 1.17 mmol). The reaction was stirred at 20 °C for 3 h. The reaction was diluted with water (10 mL), and extracted with EtOAc (20 mL x 3). The organic phase was washed with water (10 mL), brine (10 mL), dried with anhydrous sodium sulfate, filtered, and concentrated. The crude residue was purified by silica gel column chromatography (MeOH in DCM 0-5%, gradient elution) to afford the title product (**105e**) (147 mg) as a white solid with 81% purity. *m*/*z* (ESI, +ve ion) = 614.2 [M+H]⁺.

### Step F. (S)-2-((4-(1-(3-((2-chloro-4-methylphenyl)(methyl)amino)phenyl)cyclopropyl)piperazin-1-yl)methyl)-1-(oxetan-2-ylmethyl)-1H-benzo[d]imidazole-6-carboxylic acid (105)

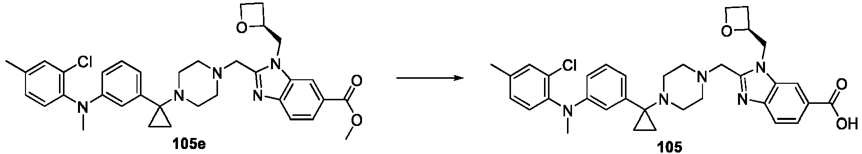

To a solution of **105e** (80 mg, 0.13 mmol) in THF:H₂O (1:1, 2 mL) was added lithium hydroxide (6 mg, 0.26 mmol) at 0 °C. The reaction mixture was stirred at 20 °C for 16 h. After completion, THF was removed *in vacuo.* The resulting mixture was adjusted to pH = 7 with 1 N HCl, and extracted with DCM (5 mL x 2). The combined organic phase was washed with water (2 mL), brine (2 mL), dried over magnesium sulfate, filtered, and concentrated to give a crude residue, which was purified by reverse phase HPLC (gradient 35-45% CH₃CN in H₂O, 0.1% TFA as a modifier) to afford the title compound (**105**) (14.5 mg, 18%) as a white solid. ¹H NMR (400 MHz, CD₃OD) δ ppm 8.39 (br s, 1H), 8.04 - 8.12 (m, 1H), 7.75 (br d, *J =* 8.56 Hz, 1H), 7.35 (s, 1H), 7.15 - 7.29 (m, 3H), 6.86 (br d, *J =* 7.58 Hz, 1H), 6.59 - 6.67 (m, 2H), 5.13 (qd, *J =* 7.16, 2.63 Hz, 1H), 4.68 - 4.78 (m, 1H), 4.51 - 4.65 (m, 2H), 4.23 - 4.40 (m, 3H), 3.25 (s, 3H), 2.85 - 3.60 (m, 8H), 2.65 - 2.73 (m, 1H), 2.38 - 2.48 (m, 1H), 2.35 (s, 3H), 1.32 (br dd, *J =* 8.68, 7.46 Hz, 2H), 1.07 - 1.22 (m, 2H). *m*/*z* (ESI, +ve ion) = 600.3 [M+H]⁺.

### Example 106. (S)-2-((4-(3-((2-chloro-4-methylphenyl)(methyl)amino)benzylidene)piperidin-1-yl)methyl)-1-(oxetan-2-ylmethyl)-1H-benzo[d]imidazole-6-carboxylic acid (106)

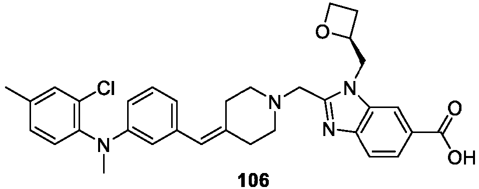

### Step A. tert-Butyl 4-(3-bromobenzylidene)piperidine-1-carboxylate (106a)

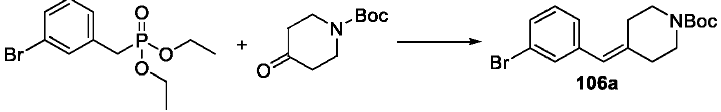

A solution of diethyl (3-bromobenzyl)phosphonate (2 g, 6.5 mmol) in THF (15 mL) was added NaH (60% in mineral oil, 260 mg, 13.0 mmol) at 0 °C. After stirring for 30 min at 0 °C, a solution of *tert*-butyl 4-oxopiperidine-1-carboxylate (2.59 g, 13.0 mmol) in THF (5 mL) was added. The resulting mixture was stirred for 0.5 h at the same temperature, and then stirred another 8 h at 20 °C under a N₂ atmosphere. The mixture was quenched with H₂O (10 mL), and extracted with EtOAc (10 mL x 2). The combined organic layer were dried over Na₂SO₄, filtered, concentrated, and purified by silica gel column chromatography (EtOAc:petroleum ether = 1:10) to give the title product (**106a**) (2.2 g, 96%) as a white solid. *m*/*z*(ESI, +ve ion) = 374.1 [M+Na]⁺.

### Step B. tert-Butyl 4-(3-((2-chloro-4-methylphenyl)amino)benzylidene)piperidine-1-carboxylate (106b)

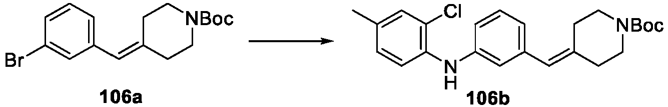

A solution of **106a** (1.0 g, 2.84 mmol) in 1,4-dioxane (5 mL) was added 2-chloro-4-methylaniline (482 mg, 3.41 mmol), Ruphos-Pd-G2 (241 mg, 0.31 mmol), Cs₂CO₃ (1.85 g, 5.68 mmol) under N₂. The mixture was stirred for 7 h at 95 °C and then concentrated to give a crude residue, which was purified by silica gel column chromatography (EtOAc:petroleum ether = 1:10) to give the title product (**106b**) (760 mg, 65%) as a light brown oil. *m*/*z* (ESI, +ve ion) = 435.1 [M+Na]⁺.

### Step C. tert-Butyl 4-(3-((2-chloro-4-methylphenyl)(methyl)amino)benzylidene)piperidine-1-carboxylate (106c)

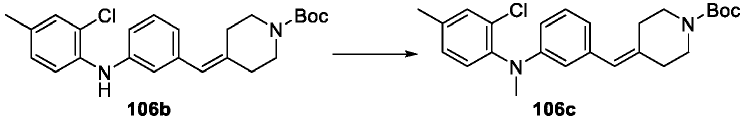

A solution of **106b** (45 mg, 0.11 mol) in DMF (1 mL) was added KOH (78 mg, 0.54 mol) and iodomethane (232 mg, 1.6 mol). The mixture was stirred for 16 h at 20 °C. The reaction was diluted with water (10 mL) and extracted with EtOAc (5mL x 3). The combined organic layers were dried over Na₂SO₄, filtered, concentrated, and purified by prep-TLC (EtOAc:petroleum ether = 1:5) to give the title product (**106c**) (32 mg, 68%) as a colorless oil. *m*/*z* (ESI, +ve ion) = 449.2 [M+Na]⁺.

### Step D. 2-Chloro-N,4-dimethyl-N-(3-(piperidin-4-ylidenemethyl)phenyl)aniline (106d)

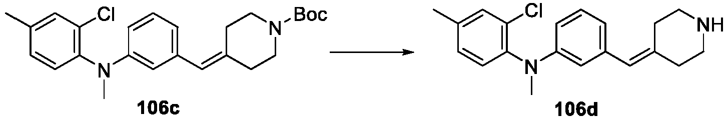

A mixture of **106c** (45 mg, 0.105 mmol) in DCM (3 mL) was added 4 N HCl in dioxane (0.3 mL). The mixture was stirred at 14 °C for 2 h. After completion, the reaction mixture was concentrated *in vacuo* to give the title product (**106d**) (46 mg, HCl salt) as a white solid, which was used in the next step without further purification. *m*/*z* (ESI, +ve ion) = 327.1 [M+H]⁺.

### Step E. Methyl (S)-2-((4-(3-((2-chloro-4-methylphenyl)(methyl)amino)benzylidene)piperidin-1-yl)methyl)-1-(oxetan-2-ylmethyl)-1H-benzo[d]imidazole-6-carboxylate (106e)

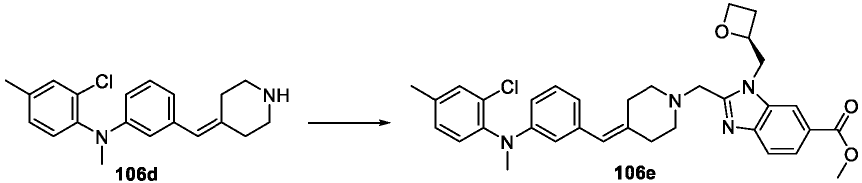

A mixture of **106d** (41 mg, 0.10 mmol), and potassium carbonate (56 mg, 0.41 mmol) in DMF (3 mL) was stirred at 16 °C for 0.5 h. Then potassium iodide (2 mg, 0.01 mmol) and **1h** (30 mg, 0.10 mmol) in DMF (0.5 mL) were added to the above mixture. After stirring for 2.5 h, the mixture was poured into water (10 mL), and extracted with EtOAc (10 mL x 2). The combined organic layers were dried over Na₂SO₄, filtered, concentrated, and purified by reverse phase HPLC (53% CH₃CN in H₂O with 0.05% TFA as a modifier) to give the title product (106e) (29 mg, 39%) as a white solid. *m*/*z* (ESI, +ve ion) = 585.3 [M+H]⁺.

### Step F. (S)-2-((4-(3-((2-Chloro-4-methylphenyl)(methyl)amino)benzylidene)piperidin-1-yl)methyl)-1-(oxetan-2-ylmethyl)-1H-benzo[d]imidazole-6-carboxylic acid (106)

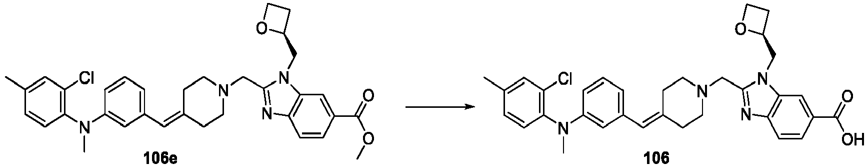

To a solution of **106e** (29 mg, 0.05 mmol) in a mixed solvent of CH₃OH:H₂O = 5:1 (3.5 mL) was added LiOH·H₂O (40 mg, 0.95 mmol). The reaction was stirred at 16 °C for 18 h, adjusted to pH = 7 with 1 N HCl, and extracted with EtOAc (10 mL x 2). The combined organic layers were dried over Na₂SO₄, filtered, concentrated, and purified by reverse phase HPLC (42% CH₃CN in H₂O with 0.5% TFA as a modifier) to afford the title compound (**106**) (16.8 mg, 48.4%) as a white solid. ¹H NMR (400 MHz, CD₃OD) δ ppm 8.35 (d, *J =* 0.88 Hz, 1H), 8.02 - 8.09 (m, 1H), 7.82 (d, *J* = 8.77 Hz, 1H), 7.36 (s, 1H), 7.11 - 7.23 (m, 3H), 6.59 (d, *J* = 7.45 Hz, 1H), 6.46 - 6.55 (m, 2H), 6.29 (s, 1H), 5.22 (qd, *J =* 7.16, 2.63 Hz, 1H), 4.61 - 4.83 (m, 5H), 4.42 (dt, *J =* 9.43, 5.81 Hz, 1H), 3.55 (br s, 2H), 3.42 (br s, 2H), 3.21 (s, 3H), 2.72 - 2.89 (m, 3H), 2.67 (br t, *J =* 5.92 Hz, 2H), 2.44 - 2.55 (m, 1H), 2.36 (s, 3H). *m*/*z* (ESI, +ve ion) = 571.1 [M+H]⁺.

### Example 110. 1'-((6-(2H-Tetrazol-5-yl)pyridin-3-yl)methyl)-6-((2,4-dichlorobenzyl)oxy)-1',2',3',6'-tetrahydro-2,4'-bipyridine (110)

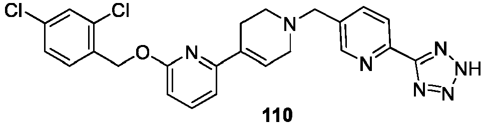

### Step A. tert-Butyl 6-((2,4-dichlorobenzyl)oxy)-3',6'-dihydro-[2,4'-bipyridine]-1'(2'H)-carboxylate (110a)

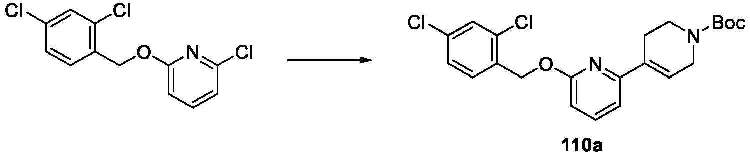

(1,1'-Bis(diphenylphosphino)ferrocene)palladium(II) dichloride (12.7 mg, 0.017 mmol), Cs₂CO₃ (113 mg, 0.35 mmol), and *tert*-butyl 4-(4,4,5,5-tetramethyl-1,3,2-dioxaborolan-2-yl)-1,2,3,6-tetrahydropyridine-1-carboxylate (53 mg 0.17 mmol) were added to 2-chloro-6-[(2,4-dichlorophenyl)methoxy]pyridine (50 mg, 0.17 mmol) in anhydrous 1,4-dioxane (0.9 mL) at room temperature. The mixture was flushed with argon for 10 min, then heated to 90 °C for 2 h. The reaction was partitioned between EtOAc and sat. aq. NaHCO₃. The organic layer was washed with brine, dried (Na₂SO₄), filtered, concentrated, and purified by silica gel column chromatography (gradient elution, EtOAc in hexanes 0-50%) to provide the title product (**110a**) (48 mg, 64%) as a white oil. *m*/*z* (ESI, +ve ion) = 435.2 [M+H]⁺.

### Step B. 5-((6-((2,4-Dichlorobenzyl)oxy)-3',6'-dihydro-[2,4'-bipyridin]-1'(2'H)-yl)methyl)picolinonitrile (110b)

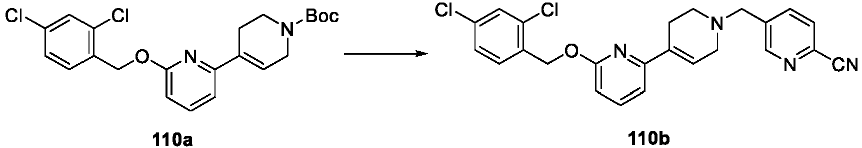

TFA (0.15 mL, 2.0 mmol) was added to **110a** (44 mg, 0.1 mmol) in DCM (1 mL) at room temperature, and stirred for 30 min. The reaction was concentrated to give a crude TFA salt which was redissolved in anhydrous THF (0.1 M, 1.0 mL). 5-Formylpyridine-2-carbonitrile (45 mg, 0.15 mmol) was added. After stirring for 10 min, sodium triacetoxyborohydride (42 mg, 0.2 mmol) was added, and the reaction was stirred for 3 h. The reaction was partitioned between EtOAc and sat. aq. NaHCO₃. The organic layer was washed with brine, dried (Na₂SO₄), concentrated, and purified by silica gel column chromatography (gradient elution, MeOH in DCM 0-5%) to provide the title product (**110b**) (15 mg, 21%) as a white oil. *m*/*z* (ESI, +ve ion) = 451.2 [M+H]⁺.

### Step C. 1'-((6-(2H-Tetrazol-5-yl)pyridin-3-yl)methyl)-6-((2,4-dichlorobenzyl)oxy)-1',2',3',6'-tetrahydro-2,4'-bipyridine (110)

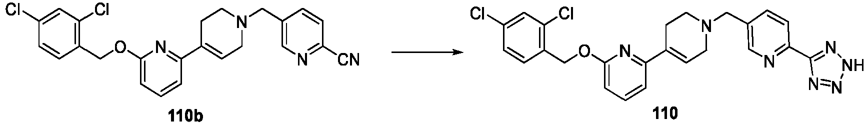

NaN₃ (2 mg, 0.032 mmol), and NH₄Cl (2 mg, 0.032 mmol) were added to **110b** (13 mg, 0.029 mmol) in anhydrous DMF (0.3 mL) at room temperature. After stirring for 10 min, the reaction was heated to 100 °C for 40 h, and then cooled down. The crude mixture was directly purified by reverse phase HPLC (gradient elution, 0-90% CH₃CN in water with 0.1% TFA as a modifier) to afford the title compound (**110**) (2.6 mg, 15%) as a white solid. ¹H NMR (600 MHz, CD₃OD) δ ppm 8.94 (br s, 1H), 8.38 (d, *J =* 8.07 Hz, 1H), 8.23 (dd, *J =* 7.89, 1.28 Hz, 1H), 7.72 (t, *J =* 7.89 Hz, 1H), 7.45 - 7.50 (m, 2H), 7.31 (dd, *J =* 8.25, 2.02 Hz, 1H), 7.18 (d, *J = 7.70* Hz, 1H), 6.84 (d, *J =* 8.44 Hz, 1H), 6.70 (br s, 1H), 5.48 (s, 2H), 4.62 (s, 2 H), 4.01 (br s, 2H), 3.65 (br s, 2H), 2.97 (br s, 2H). *m*/*z* (ESI, +ve ion) = 494.2 [M+H]⁺.

### Example 111. (S)-2-((4-(3-((2-Chloro-4-methylphenyl)(methyl)amino)phenoxy)piperidin-1-yl)methyl)-1-(oxetan-2-ylmethyl)-1H-benzo[d]imidazole-6-carboxylic acid (111)

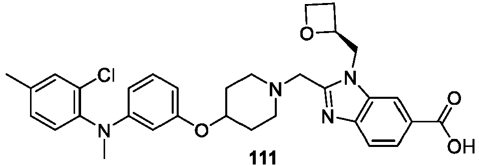

### Step A. tert-Butyl 4-{3-[(2-chloro-4-methylphenyl)(methyl)amino]phenoxylpiperidine-1-carboxylate (111a)

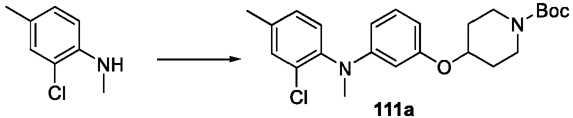

*tert*-Butyl 4-(3-bromophenoxy)piperidine-1-carboxylate (137 mg, 0.39 mmol), Pd(OAc)₂ (1.4 mg, 0.006 mmol), BINAP (4 mg, 0.006 mmol), and Cs₂CO₃ (157 mg, 0.48 mmol) were added to a flask charged with 2-chloro-N,4-dimethylaniline (50 mg, 0.32 mmol) in anhydrous toluene (0.2 M, 1.6 mL) at room temperature. The mixture was degassed with argon for 10 min, then heated to 120 °C for 2 h. The reaction was partitioned between EtOAc and sat. aq. NaHCO₃. The organic layer was washed with brine, dried (Na₂SO₄), filtered, concentrated, and purified by silica gel column chromatography (gradient elution, EtOAc in hexanes 0-20%) to provide an impure title product (**111a**) as a white oil (122 mg). *m*/*z* (ESI, +ve ion) = 432.2 [M+H]⁺.

### Step B. 2-Chloro-N,4-dimethyl-N-(3-(piperidin-4-yloxy)phenyl)aniline (111b)

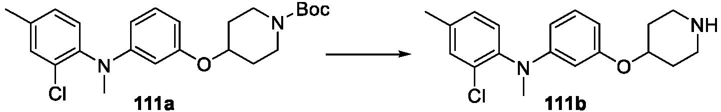

A flask was charged with **111a** (30 mg, impure) in DCM (0.7 mL) at room temperature. TFA (0.1 mL, 1.4 mmol) was added. The reaction was stirred for 30 min, and then concentrated. The crude residue was partitioned between EtOAc and sat. aq. NaHCO₃. The organic layer was washed with brine, dried (Na₂SO₄), and concentrated to provide the crude title product (**111b**) (23 mg) as a colorless oil, which was used in the next step without further purification. *m*/*z* (ESI, +ve ion) = 331.2 [M+H]⁺.

### Step C. (S)-2-((4-(3-((2-Chloro-4-methylphenyl)(methyl)amino)phenoxy)piperidin-1-yl)methyl)-1-(oxetan-2-ylmethyl)-1H-benzo[d]imidazole-6-carboxylic acid (111)

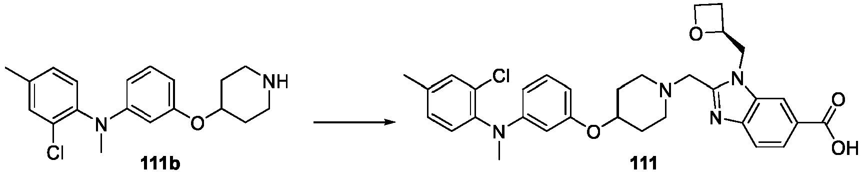

DIPEA (0.012 mL, 0.07 mmol), and **1h** (12 mg, 0.04 mmol) were added to **111b** (23 mg, 0.07 mmol) in DCM (1.4 mL) at room temperature, and heated to 40 °C for 16 h. The reaction was concentrated to provide a crude ester, which was re-dissolved in THF:H₂O (1:1, 0.4 mL). LiOH·H₂O (2 mg, 0.045 mmol) was added. After stirring for 12 h at room temperature, the reaction was acidified with 1 N HCl. The mixture was partitioned between EtOAc and sat. aq. NaHCO₃. The organic layer was washed with brine, dried (Na₂SO₄), filtered, concentrated, and purified by reverse phase HPLC (0-70% CH₃CN and water with 0.1% HOAc as a modifier, gradient elution) to provide the title product (**111**) as a white solid (2.5 mg, 6% over 3 steps). ¹H NMR (600 MHz, CD₃OD) δ ppm 8.50 (s, 1H), 8.27 (s, 1H), 7.95 (dd, *J =* 8.44, 1.47 Hz, 1H), 7.62 (d, *J =* 8.44 Hz, 1H), 7.34 (s, 1H), 7.12 - 7.21 (m, 1H), 7.01 (t, *J =* 8.25 Hz, 1H), 6.31 (dd, *J* = 8.07, 1.83 Hz, 1H), 6.14 (dd, *J =* 8.07, 2.20 Hz, 1H), 6.03 (t, *J* = 2.38 Hz, 1H), 5.25 (qd, *J* = 7.15, 2.38 Hz, 1H), 4.85-4.90 (m, 1H), 4.70 (dd, *J* = 15.41, 2.93 Hz, 1H), 4.63 (td, *J* = 7.89, 5.87 Hz, 1H), 4.46 (dt, *J* = 9.17, 5.87 Hz, 1H), 4.23 - 4.31 (m, 1H), 3.85 - 4.03 (m, 2H), 3.17 (s, 3H), 2.72 - 2.84 (m, 3H), 2.47 - 2.57 (m, 1H), 2.39 - 2.46 (m, 2H), 2.37 (s, 3H), 1.91 - 2.01 (m, 2H), 1.74 (br s, 2H). *m*/*z* (ESI, +ve ion) = 575.3 [M+H]⁺.

### Example 112. 2-((4-(3-(1-(2-Chloro-4-methylphenyl)ethyl)benzoyl)piperazin-1-yl)methyl)-1-(((S)-oxetan-2-yl)methyl)-1H-benzo[d]imidazole-6-carboxylic acid (112)

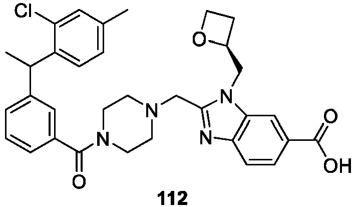

### Step A. Methyl 3-(2-chloro-4-methylbenzoyl)benzoate (112a)

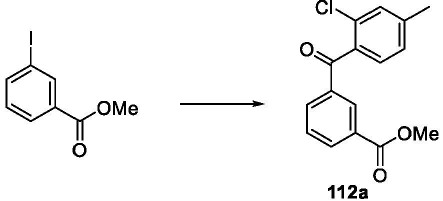

To a mixture of methyl 3-iodobenzoate (400 mg, 1.53 mmol), (2-chloro-4-methylphenyl)boronic acid (260 mg, 1.53 mmol), K₂CO₃ (527 mg, 3.82 mmol), and Pd(dppf)Cl₂·DCM (125 mg, 0.15 mmol) was added anisole (12 mL) under a carbon monoxide (CO) atmosphere. The mixture was stirred at 80 °C for 12 h. After completion, the mixture was filtered, concentrated, and purified by silica gel column chromatography (EtOAc:petroleum ether = 1:10) to afford the title product (**112a**) (291 mg, 59%) as a yellow solid. *m*/*z* (ESI, +ve ion) = 289.0 [M+H]⁺.

### Step B. 3-(2-Chloro-4-methylbenzoyl)benzoic acid (112b)

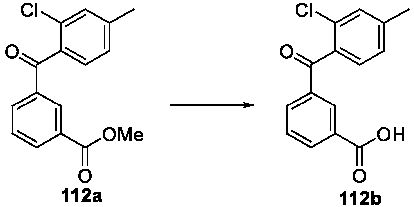

A flask was charged with **112a** (150 mg, 0.52 mmol), and CH₃OH:H₂O = 10:1 (8 mL) followed by the addition of sodium hydroxide (104 mg, 2.6 mmol). The mixture was stirred at 16 °C for 3 h. The mixture was then adjusted to pH = 7 with 1N HCl, and extracted with EtOAc (30 mL x 2). The combined organic layers were washed with H₂O (5 mL), brine (5 mL), dried over Na₂SO₄, filtered, and concentrated *in vacuo* to give the title product (**112b**) (130 mg) as a gray solid, which was used in the next step without further purification. *m*/*z* (ESI, +ve ion) = 274.9 [M+H]⁺.

### Step C. 3-(1-(2-Chloro-4-methylphenyl)-1-hydroxyethyl)benzoic acid (112c)

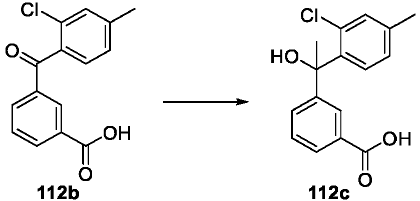

To a mixture of **112b** (130 mg, 0.47 mmol) in THF (10 mL) was added CH₃MgBr (3 M in hexanes, 0.6 mL, 1.66 mmol) at 0 °C. The mixture was stirred at 0 °C for one hour. After completion, the mixture was quenched with sat. aq. NH₄Cl (10 mL), diluted with H₂O (10 mL), and extracted with EtOAc (20 mL x 2). The combined organic layers were dried over Na₂SO₄, filtered, and concentrated *in vacuo* to give the title product (**112c**) (116 mg, crude) as a yellow oil, which was used in next step without further purification. *m*/*z* (ESI, +ve ion) = 273.1 [M-OH]⁺.

### Step D. 3-(1-(2-Chloro-4-methylphenyl)ethyl)benzoic acid (112d)

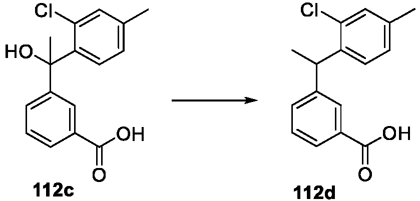

To a flask charged with **112c** (116 mg, 0.4 mmol) was added triethylsilane (2 mL), followed by TFA (1 mL). The mixture was stirred at 16 °C for 3 h. After completion, the reaction mixture was poured into water (10 mL), and extracted with EtOAc (20 mL x 2). The combined organic layers were dried over Na₂SO₄, filtered, and concentrated *in vacuo.* The crude residue was purified by reverse phase HPLC (61% CH₃CN in H₂O with 0.5% TFA as a modifier) to give the title product (**112d**) (69 mg, 60%) as a white solid. *m*/*z* (ESI, +ve ion) = 275.1 [M+H]⁺.

### Step E. Methyl 2-((4-(3-(1-(2-chloro-4-methylphenyl)ethyl)benzoyl)piperazin-1-yl)methyl)-1-(((S)-oxetan-2-yl)methyl)-1H-benzo[d]imidazole-6-carboxylate (112e)

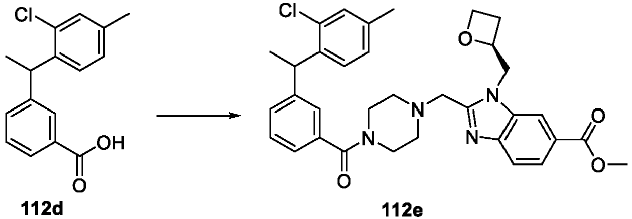

The title compound was prepared from **112d,** and **1j** by procedures similar to those described in Example **102,** Step D. *m*/*z* (ESI, +ve ion) = 601.1 [M+H]⁺.

### Step F. 2-((4-(3-(1-(2-Chloro-4-methylphenyl)ethyl)benzoyl)piperazin-1-yl)methyl)-1-(((S)-oxetan-2-yl)methyl)-1H-benzo[d]imidazole-6-carboxylic acid (112)

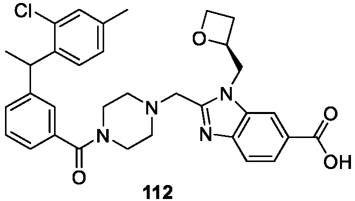

The title compound was prepared from **112e** by procedures similar to those described in Example **106,** Step F. ¹H NMR (400 MHz, CD₃OD) δ ppm 8.47 (s, 1H), 8.14 (dd, *J =* 8.33, 1.32 Hz, 1H), 7.81 (d, *J =* 8.33 Hz, 1H), 7.34 - 7.45 (m, 2H), 7.22 - 7.31 (m, 3H), 7.19 (s, 1H), 7.12 (br d, *J* = 7.89 Hz, 1H), 5.23 (qd, *J =* 7.16, 2.19 Hz, 1H), 4.92 (br d, *J* = 3.51 Hz, 1H), 4.74 (br dd, *J =* 15.35, 2.63 Hz, 1H), 4.58 - 4.70 (m, 2H), 4.32 - 4.51 (m, 3H), 3.87 (br s, 2H), 3.57 (br s, 2H), 2.77 - 3.02 (m, 5H), 2.47 - 2.58 (m, 1H), 2.28 (s, 3H), 1.62 (d, *J=* 7.45 Hz, 3H). *m*/*z* (ESI, +ve ion) = 587.2 [M+H]⁺.

### Example 114. (S)-2-((4-((6-((2-Cyano-4-methylphenoxy)methyl)pyridin-2-yl)methyl)piperidin-1-yl)methyl)-1-(oxetan-2-ylmethyl)-1H-benzo[d]imidazole-6-carboxylic acid (114)

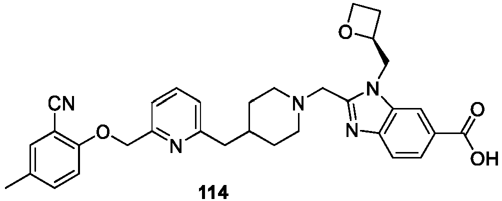

### Step A. (E)-2-Hydroxy-5-methylbenzaldehyde oxime (114a)

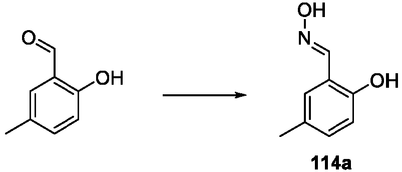

A solution of 2-hydroxy-5-methylbenzaldehyde (5 g, 36.7 mmol), hydroxylamine hydrochloride (12.75 g, 183.5 mmol) and pyridine (5.81 g, 73.4 mmol) in EtOH (50 mL) was stirred at 60 °C for 3 h. After completion, the reaction mixture was concentrated to about 10 mL. EtOAc (150 mL) and 1 N HCl (50 mL) were added. The organic phase was washed with water (50 mL x 2), brine (50 mL), dried with Na₂SO₄, filtered and concentrated to afford the title product (**114a**) (5.1 g, 87%) as a white solid. *m*/*z* (ESI, +ve ion) = 152.2 [M+H]⁺.

### Step B. 2-Hydroxy-5-methylbenzonitrile (114b)

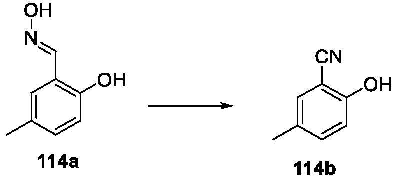

To a solution of **114a** (100 mg, 0.66 mmol) and PPh₃ (433 mg, 1.65 mmol) in CH₂Cl₂ (2 mL) was added diethyl azodicarboxylate (334 mg, 1.65 mmol) dropwise at 17 °C. The resulting mixture was stirred at 17 °C for 3 h. After completion, the reaction mixture was partitioned between CH₂Cl₂ (10 mL) and 0.1 N NaOH(10 mL). The aqueous layer was washed with CH₂Cl₂ (50 mL x 3) and acidified with 1 N HCl (10 mL). The acidic aqueous layer was extracted with CH₂Cl₂ (50 mL x 2). The combined organic layer was dried, filtered, and concentrated to afford the title product (**114b**) (51 mg, 52%) as a white solid. *m*/*z* (ESI, +ve ion) = 134.2 [M+H]⁺.

### Step C. (6-(Chloromethyl)pyridin-2-yl)methanol (114c)

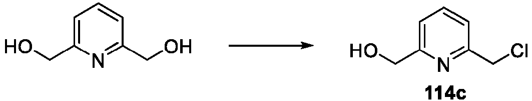

To a solution of pyridine-2,6-diyldimethanol (20 g, 143 mmol) in DMF (100 mL) was added NaH (60% in mineral oil, 5.75 g, 143 mmol) at 0 °C. The mixture was stirred at 0 °C for 0.5 h. Then *tert*-butyl(chloro)dimethylsilane (26 g, 172 mmol) was added dropwise. The mixture was stirred at 17 °C for 16 h. The reaction was diluted with EtOAc (200 ml) and washed with sat. NaHCO₃ (50 mL), and brine (50 mL). The EtOAc layer was dried, filtered, concentrated and the resulting crude residue was purified by silica gel column chromatography (5% MeOH in DCM) to afford the title product (**114c**) (3.5 g, 14.7%) as a colorless oil. *m*/*z* (ESI, +ve ion) = 158.1 [M+H]⁺.

### Step D. tert-Butyl 4-((6-(hydroxymethyl)pyridin-2-yl)methyl)-3,6-dihydropyridine-1(2H)-carboxylate (114d)

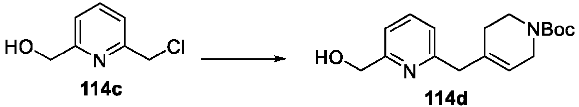

A flask was charged with **114c** (3.3 g, 20.9 mmol), *tert*-butyl 4-(4,4,5,5-tetramethyl-1,3,2-dioxaborolan-2-yl)-3,6-dihydropyridine-1(2*H*)-carboxylate (7.13 g, 22.9 mmol), K₂CO₃ (8.67 g, 62.6 mmol) and Pd(dppf)Cl₂.DCM (0.85 g, 1.0 mmol) and added 3 mL of a mixed solvent of 1,4-dioxane/H₂O (4:1). The mixture was degassed with nitrogen and stirred at 90 °C for 3 h under nitrogen. After completion, the reaction was diluted with H₂O (100 mL) and extracted with EtOAc (100 mL x 3). The organic layer was dried, filtered and concentrated. The crude residue was purified by silica gel column chromatography (petroleum ether:EtOAc = 3:2) to afford the title product (**114d**) (3.8 g, 57%) as a yellow oil. *m*/*z* (ESI, +ve ion) = 305.2 [M+H]⁺.

### Step E. tert-Butyl 4-((6-(hydroxymethyl)pyridin-2-yl)methyl)piperidine-1-carboxylate (114e)

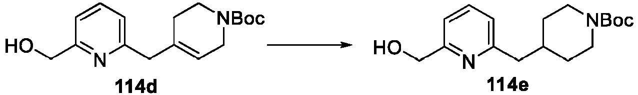

A solution of **114d** (3.8 g, 12.5 mmol) and 10% Pd/C (380 mg) in MeOH (30 mL) was stirred under hydrogen at 17 °C for 12 h. After completion, the reaction was filtered through a pad of celite and rinsed with MeOH (50 mL x 3). The organic layer was concentrated to afford the title product (**114e**) (3.5 g, 87%) as a yellow oil, which was used in the next step without further purification. *m*/*z* (ESI, +ve ion) = 307.3 [M+H]⁺.

### Step F. tert-Butyl 4-((6-((2-cyano-4-methylphenoxy)methyl)pyridin-2-yl)methyl)piperidine-1-carboxylate (114f)

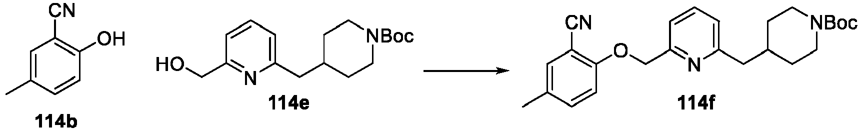

A flask was charged with **114b** (332 mg, 2.50 mmol), **114e** (510 mg, 1.66 mmol), and PPh₃ (655 mg, 2.50 mmol). Anhydrous THF (10 mL) was added and the reaction was cooled down in an ice bath. Diethyl azodicarboxylate (435 mg, 2.50 mmol) was added dropwise and the resulting mixture was stirred at 0 °C for 1 h, then at 17°C for 11 h under nitrogen. After completion, the organic layer was concentrated and purified by silica gel column chromatography (petroleum ether:EtOAc = 4:1) to afford the title product (**114f**) (600 mg, 79%) as a yellow oil. *m*/*z* (ESI, +ve ion) = 422.2 [M+H]⁺.

### Step G. 2-((2-Chloro-4-methylphenoxy)methyl)-6-(piperidin-4-ylmethyl)pyridine (114g)

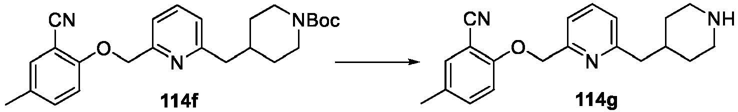

A solution of **114f** (523 mg, 1.24 mmol) and 2,6-lutidine (2.66 g, 24.8 mmol) in anhydrous DCM (10 mL) was stirred at 0 °C for 0.5 h. TMSOTf (2.76 g, 12.4 mmol) was added and the resulting mixture was stirred at 0 °C for 2 h. After completion, the reaction was quenched with saturated ammonium chloride solution (1 mL). The reaction mixture was diluted with H₂O (10 mL) and extracted with EtOAc (10 mL x 3). The organic layer was dried, filtered and concentrated to afford the title product (**114g**) (400 mg, 95%) as a yellow oil, which was used in the next step without further purification. *m*/*z* (ESI, +ve ion) = 322.2 [M+H]⁺.

### Step H. Methyl (S)-2-((4-((6-((2-cyano-4-methylphenoxy)methyl)pyridin-2-yl)methyl)piperidin-1-yl)methyl)-1-(oxetan-2-ylmethyl)-1H-benzo[d]imidazole-6-carboxylate (114h)

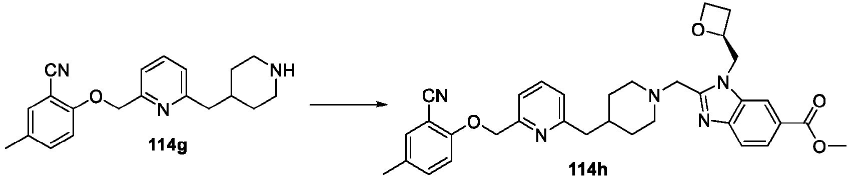

A solution of **114g** (98 mg, 0.30 mmol), **1h** (90 mg, 0.30 mmol) and K₂CO₃ (126 mg, 0.91 mmol) in DMF (2 mL) was stirred at 17 °C for 6 h. After completion, the reaction was diluted with H₂O (10 mL) and extracted with EtOAc (10 mL x 3). The organic layer was dried, filtered and concentrated to give a crude residue, which was purified by silica gel column chromatography (DCM:MeOH = 50:1) to afford the title product (**114h**) (158 mg) as a white solid with some impurity. *m*/*z* (ESI, +ve ion) = 580.3 [M+H]⁺.

### Step I. (S)-2-((4-((6-((2-Cyano-4-methylphenoxy)methyl)pyridin-2-yl)methyl)piperidin-1-yl)methyl)-1-(oxetan-2-ylmethyl)-1H-benzo[d]imidazole-6-carboxylic acid (114)

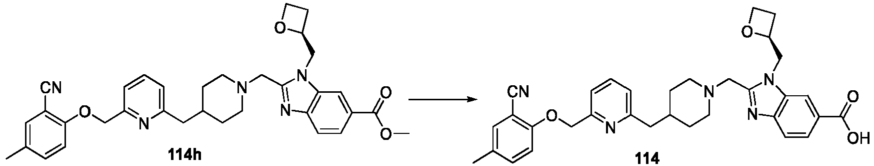

A solution of **114h** (79 mg, 0.136 mmol) and LiOH (10 mg, 0.41 mmol) in THF:H₂O = 1:1 (2 mL) was stirred at 30 °C for 6 h. After completion, 1 N HCl was added to the mixture to adjust pH = 5 in an ice bath. The reaction mixture was then extracted with EtOAc (50 mL x 3). The organic layer was dried, filtered and concentrated and the crude residue was purified by reverse phase HPLC (25% CH₃CN in H₂O, with 0.05% TFA as a modifier) to afford the TFA salt of the title product (**114**) (16.4 mg, 18%) as a white solid. ¹H NMR (400 MHz, CD₃OD) δ ppm 8.33 (d, *J =* 0.88 Hz, 1H), 8.04 (dd, *J=* 8.55, 1.53 Hz, 1H), 7.88 - 7.94 (m, 1H), 7.80 (d, *J =* 9.21 Hz, 1H), 7.57 (d, *J =* 7.45 Hz, 1H), 7.42 - 7.48 (m, 2 H), 7.38 (d, *J* = 7.02 Hz, 1H), 7.13 - 7.17 (m, 1H), 5.29 (s, 2H), 5.20 (qd, *J*=7.09, 2.41 Hz, 1H), 4.76 - 4.82 (m, 2H), 4.71 - 4.75 (m, 1H), 4.60 - 4.69 (m, 2H), 4.41 (dt, *J*=9.43, 5.81 Hz, 1H), 3.73 - 3.84 (m, 2H), 3.15 - 3.28 (m, 2H), 2.89 (d, *J*=7.02 Hz, 2H), 2.79 (dtd, *J*=11.46, 8.19, 8.19, 5.92 Hz, 1H), 2.41 - 2.54 (m, 1H), 2.14 - 2.26 (m., 1H), 1.90 - 2.01 (m, 2H) 1.57 - 1.74 (m, 2H). *m*/*z* (ESI, +ve ion) = 566.2 [M+H]⁺.

Example compounds **118, 120, 140** were synthesized in similar procedures as described in Example **114.** Example **123** was synthesized from methyl 5-fluoro-6-methylpicolinate as described in Example **60**, Steps A, B, followed by as described in Example **72,** Step C and Example **114,** Steps E to I. Example **126, 133** were synthesized following the same synthetic sequence as Example **123.** Example **135** was synthesized from as described in Example **60,** Steps A to C, followed by as described in Example **72,** Step C and Example **114,** Steps F to I. Example **162** was synthesized in Example **60,** Steps A to C, followed by as described in Example **72,** Step C to D, Example **116,** Step D and Example **114,** Steps H to I.

### Example 115. (S)-2-((4-(3-((4-Cyclopropyl-2-methylphenyl)(methyl)amino)benzoyl)piperazin-1-yl)methyl)-1-(oxetan-2-ylmethyl)-1H-benzo[d]imidazole-6-carboxylic acid (115)

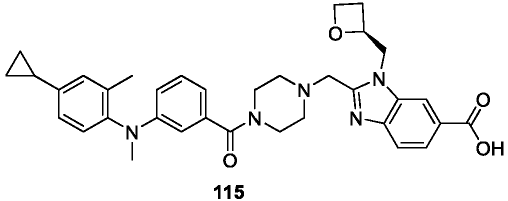

### Step A. 4-Cyclopropyl-2-methylaniline (115a)

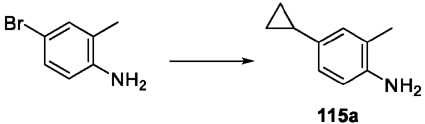

1,1'-Bis(diphenylphosphino)ferrocene-palladium(II)dichloridedichloromethane complex (44 mg, 0.05 mmol) was added to a solution of 4-bromo-2-methylaniline (50 mg, 0.27 mmol), cesium carbonate (350 mg, 1.07 mmol), cyclopropylboronic acid (46 mg, 0.54 mmol) in 1,4-dioxane (2 mL) and H₂O (0.1 mL). The resulting mixture was stirred at 100 °C for 5 h and was concentrated. Then the reaction was diluted with water (5 mL) and the mixture was extracted with EtOAc (5 mL x 3). The organic layer was washed with water (5 mL), brine (5 mL), dried with anhydrous sodium sulfate, filtered and concentrated. The crude residue was purified by silica gel column chromatography (gradient elution, EtOAc in petroleum ether 0-10%) to afford the title product (**115a**) (30 mg, 68.3%) as a yellow oil. *m*/*z* (ESI, +ve ion) = 148.2 [M+H]⁺.

### Step B. Methyl 3-((4-cyclopropyl-2-methylphenyl)amino)benzoate (115b)

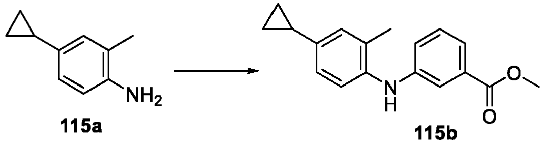

Chloro(2-dicyclohexylphosphino-2',6'-di-i-propoxy-1,1'-biphenyl)(2-amino-1,1'-biphenyl-2-yl)palladiuM(II) (RuPhos Palladacycle) (16 mg, 0.02 mmol) was added to a solution of **115a** (30 mg, 0.2 mmol), cesium carbonate (332 mg, 1 mmol), methyl 3-bromobenzoate (44 mg, 0.2 mmol) in 1,4-dioxane (3 mL). The resulting reaction mixture was stirred at 90 °C for 16 h and concentrated. The reaction was diluted with water (5 mL) and extracted with EtOAc (5 mL x 3). The organic layer was washed with water (5 mL), brine (5 mL), dried with anhydrous sodium sulfate, filtered and concentrated. The crude residue was purified by silica gel column chromatography (gradient elution, EtOAc in petroleum ether 0-10%) to afford an impure title product (**115b**) (32 mg, 80% purity) as a yellow oil. *m*/*z* (ESI, +ve ion) = 282.1 [M+H]⁺.

### Step C. 3-((4-Cyclopropyl-2-methylphenyl)(methyl)amino)benzoic acid (115c)

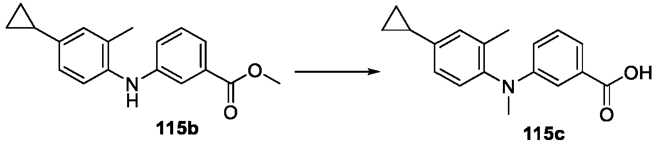

The title product (**115c**) was prepared from **115b** by procedures similar to those described in Example **46,** Steps B and C.

### Step D. Methyl (S)-2-((4-(3-((4-cyclopropyl-2-methylphenyl)(methyl)amino)benzoyl)piperazin-1-yl)methyl)-1-(oxetan-2-ylmethyl)-1H-benzo[d]imidazole-6-carboxylate (115d)

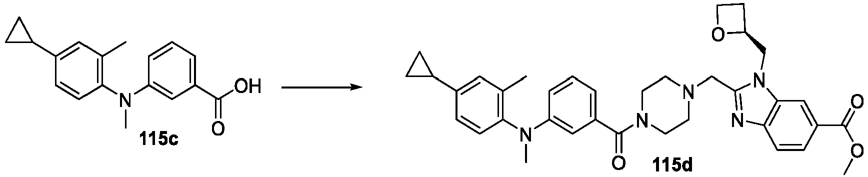

Intermediate **1j** (122 mg, 0.36 mmol) and **115c** (100 mg, 0.36 mmol) were dissolved in DCM (3 mL). Diisopropylethylamine (184 mg, 1.42 mmol) and HATU (203 mg, 0.53 mmol) were added and the reaction was stirred at 20 °C for 1 h. After removal of the volatiles, the crude residue was purified with prep-TLC (MeOH in DCM 5-10%, with 0.5% NH₃.H₂O as a modifier) to afford an impure title product (**115d**) (107 mg, 83% purity) as a yellow oil. *m*/*z* (ESI, +ve ion) = 608.2 [M+H]⁺.

### Step E. (S)-2-((4-(3-((4-Cyclopropyl-2-methylphenyl)(methyl)amino)benzoyl)piperazin-1-yl)methyl)-1-(oxetan-2-ylmethyl)-1H-benzo[d]imidazole-6-carboxylic acid(115)

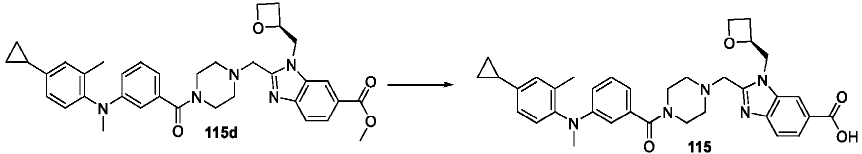

To a solution of the impure **115d** (65 mg, 0.1 mmol) in MeOH:H₂O = 5:1 (2.4 mL) was added sodium hydroxide (39 mg, 0.97 mmol) at 0 °C. The reaction mixture was raised to room temperature and stirred at 20 °C for 16 h. After concentration, the mixture was diluted with water (5 mL), adjusted to pH = 4 with 0.1 N HCl, and extracted with EtOAc (5 mL x 3). The organic layer was washed with water (5 mL), brine (5 mL), dried with anhydrous sodium sulfate, filtered and concentrated. The crude residue was purified by reverse phase HPLC (gradient elution, 40-50% CH₃CN in H₂O, with 0.1% formic acid as a modifier) to afford the title product (**115**) (19.2 mg) as a white solid. ¹H NMR (400 MHz, CD₃OD) δ ppm 8.46 (s, 1H), 8.12 (dd, *J =* 8.56, 1.47 Hz, 1H), 7.80 (d, *J =* 8.56 Hz, 1H), 7.17 - 7.26 (m, 1H), 7.05 (s, 1H), 6.91 - 7.02 (m, 2H), 6.68 (d, *J=* 7.34 Hz, 1H), 6.62 (dd, *J =* 8.31, 2.20 Hz, 1H), 6.44 (s, 1H), 5.16 - 5.29 (m, 1H), 4.87 - 4.91 (m, 1 H), 4.60 - 4.78 (m, 2H), 4.46 (dt, *J* = 9.11, 6.08 Hz, 1H), 4.27 - 4.43 (m, 2H), 3.50 - 3.89 (m, 4H), 3.21 (s, 3H), 2.74 - 3.01 (m, 5H), 2.52 (ddd, *J=* 16.38, 11.62, 7.21 Hz, 1H), 2.07 (s, 3H), 1.84 - 1.96 (m, 1H), 0.92 - 1.03 (m, 2 H), 0.61 - 0.75 (m, 2 H).*m*/*z* (ESI, +ve ion) = 594.3 [M+H]⁺.

Example **122** was synthesized in similar procedures as described in Example **115.**

### Example 116. (S)-2-((4-(3-((2-Chloro-4-methylphenyl)(methyl)amino)benzyl)piperidin-1-yl)methyl)-1-(oxetan-2-ylmethyl)-1H-benzo[d]imidazole-6-carboxylic acid (116)

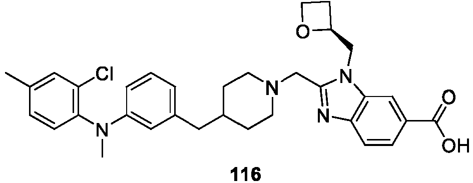

### Step A. (3-((2-Chloro-4-methylphenyl)(methyl)amino)phenyl)methanol (116a)

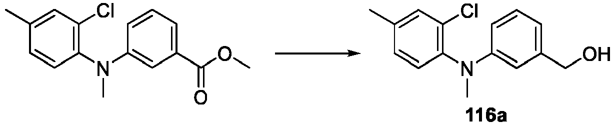

To a solution of methyl 3-((2-chloro-4-methylphenyl)(methyl)amino)benzoate (650 mg, 2.2 mmol, prepared by a similar procedure as described in Example 115, Step B) in 10 mL anhydrous THF was added LiAlH₄ (256 mg, 6.7 mmol) at 0 °C. The reaction was stirred at 0 °C for 1 h under N₂ and quenched with Na₂SO₄.10H₂O. The mixture was filtered and the filtrate cake was rinsed with DCM (100 mL). The organic layer was concentrated to afford the title product (**116a**) (487 mg, 85%) as a colorless oil, which was used in the next step without further purification. *m*/*z* (ESI, +ve ion) = 262.2 [M+H]⁺.

### Step B. N-(3-(Bromomethyl)phenyl)-2-chloro-N,4-dimethylaniline (116b)

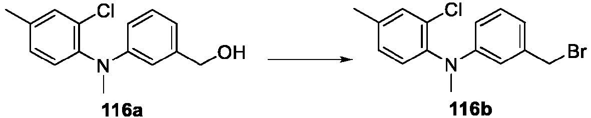

To a solution of **116a** (487 mg, 1.9 mmol) in DCM (20 mL) was added PBr₃ (506 mg, 1.9 mmol) at 0 °C. The mixture was stirred at 17 °C for 2 h. After completion, the mixture was diluted with H₂O (30 mL), and extracted with EtOAc (30 mL x 3). The organic layer was washed with H₂O (30 mL), brine (30 mL), dried over anhydrous Na₂SO₄, filtered and concentrated *in vacuo* to give the crude title product (**116b**) (591 mg) as a colorless oil, which was used in the next step without further purification. *m*/*z* (ESI, +ve ion) = 324.0 [M+H]⁺.

### Step C. tert-Butyl 4-(3-((2-chloro-4-methylphenyl)(methyl)amino)benzyl)piperidine-1-carboxylate (116c)

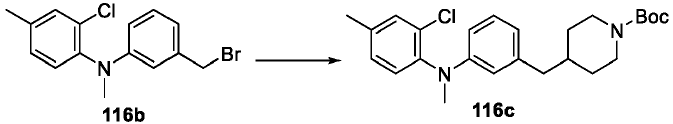

The title product (**116c**) was prepared by procedures similar to those described in Example **60,** Steps B and C.

### Step D. 2-Chloro-N,4-dimethyl-N-(3-(piperidin-4-ylmethyl)phenyl)aniline (116d)

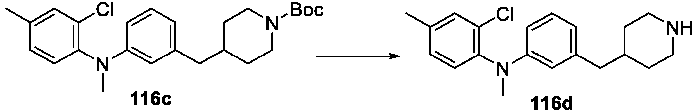

To a solution of **116c** (56 mg, 0.13 mmol) in DCM (8 mL) was added TFA (2 mL). The mixture was stirred at 17 °C for 1 h. After completion, the mixture was concentrated *in vacuo* to afford the crude title product (**116d**) (62 mg) as a colorless oil, which was used in the next step without further purification. *m*/*z* (ESI, +ve ion) = 329.2 [M+H]⁺.

### Step E. Methyl (S)-2-((4-(3-((2-chloro-4-methylphenyl)(methyl)amino)benzyl)piperidin-1-yl)methyl)-1-(oxetan-2-ylmethyl)-1H-benzo[d]imidazole-6-carboxylate (116e)

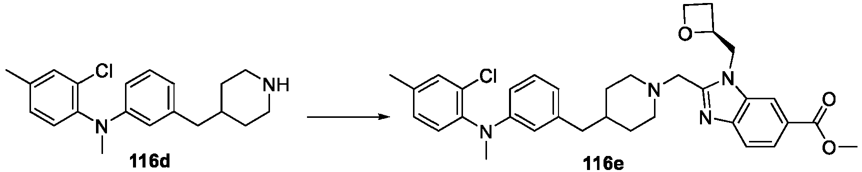

A mixture of 116d (62 mg, 0.19 mmol) and K₂CO₃ (78 mg, 0.57 mmol) in DMF (5 mL) was added **1h** (56 mg, 0.19 mmol) at 17 °C. The mixture was stirred at 17 °C for 16 h. After completion, the reaction was quenched with H₂O (5 mL) and extracted with DCM (20 mL x 3). The organic layer was washed with brine (10 mL), dried over anhydrous Na₂SO₄, filtered and concentrated and the resulting crude residue was purified by silica gel column chromatography (2% MeOH in DCM) to afford the title product (116e) (45 mg, 40%) as a white solid. *m*/*z* (ESI, +ve ion) = 587.3 [M+H]⁺.

### Step F. (S)-2-((4-(3-((2-Chloro-4-methylphenyl)(methyl)amino)benzyl)piperidin-1-yl)methyl)-1-(oxetan-2-ylmethyl)-1H-benzo[d]imidazole-6-carboxylic acid (116)

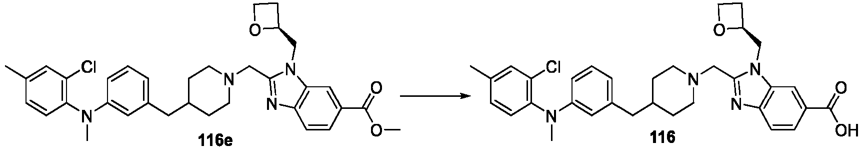

A mixture of **116e** (45 mg, 0.07 mmol) in THF (3 ml) and H₂O (1 mL) was added lithium hydroxide (18 mg, 0.70 mmol) at room temperature and heated at 30 °C for 2 h. After completion, the mixture was adjusted to pH = 7 with 1 N HCl. The residue was purified by reverse phase HPLC (CH₃CN in water, with 0.1% TFA as a modifier) to afford the TFA salt of the title product (116) (17.1 mg, 32%) as a white solid. ¹H NMR (400 MHz, CD₃OD) δ ppm 8.32 (s, 1H), 8.03 (dd, *J =* 8.56, 1.47 Hz, 1H), 7.80 (d, *J =* 8.31 Hz, 1H), 7.34 (s, 1H), 7.12 - 7.20 (m, 2H), 7.08 (t, *J=* 7.83 Hz, 1H), 6.55 (d, *J=* 7.09 Hz, 1 H), 6.43 (dd, *J=* 8.31, 2.20 Hz, 1H), 6.33 (s, 1H), 5.15 - 5.27 (m, 1H), 4.74 - 4.80 (m, 2H), 4.72 (d, *J=* 6.85 Hz, 1H), 4.58 - 4.69 (m, 2H), 4.39 (dt, *J =* 9.29, 5.87 Hz, 1H), 3.76 (br s, 2H), 3.12 - 3.27 (m, 5H), 2.72 - 2.87 (m, 1H), 2.43 - 2.57 (m, 3H), 2.36 (s, 3H), 1.62 - 1.98 (m, 2H), 1.42 - 1.60 (m, 2H). *m*/*z* (ESI, +ve ion) = 573.3 [M+H]⁺.

Example **117** was synthesized in similar procedures as described in Example **116.**

### Example 121. (S)-2-((8-((4-Chloro-2-fluorobenzyl)oxy)-1,3,4,9-tetrahydro-2H-pyrido[3,4-b]indol-2-yl)methyl)-1-(oxetan-2-ylmethyl)-1H-benzo[d]imidazole-6-carboxylic acid (121)

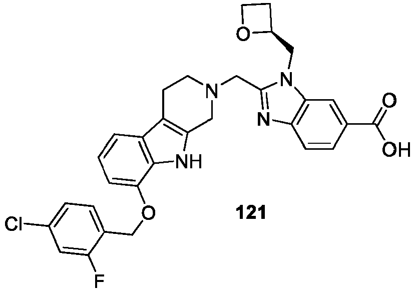

### Step A. 7-Methoxy-1H-indole-3-carbaldehyde (121a)

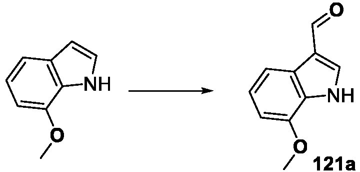

Phosphoryl chloride (2.9 g, 19 mmol) was added dropwise to a stirred mixture of 7-methoxy-1H-indole (2.5 g, 17 mmol) in DMF (25 ml) at 0 °C. The reaction was raised to room temperature and stirred at 25 °C for 16 h under N₂. After completion, the reaction mixture was poured into crushed ice and neutralized with 2 N NaOH solution. The mixture was extracted with EtOAc (20 ml x 6). The organic layer was washed with H₂O (20 mL), brine (20 mL), dried over anhydrous Na₂SO₄, filtered and concentrated to give a crude residue, which was purified by silica gel column chromatography (petroleum ether: EtOAc = 3:1) to afford the title product **(121a)** (2.2 g, 70%) as a colorless oil. *m*/*z* (ESI, +ve ion) = 176.1 [M+H]⁺.

### Step B. (Z)-7-Methoxy-3-(2-nitrovinyl)-1H-indole (121b)

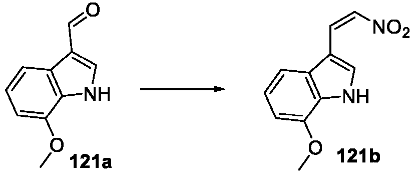

A mixture of **121a** (2.2 g, 0.013 mol) in nitromethane (23 g, 0.38 mol) was added ammonium acetate (2.9 g, 0.038 mol) at 25 °C. The reaction was stirred at 100 °C for 90 min under N₂. After completion, the reaction was quenched with H₂O (20 mL) and extracted with EtOAc (20 mL x 3). The organic layer was washed with H₂O (20 mL), brine (20 mL), dried over anhydrous Na₂SO₄, filtered and concentrated to give a crude residue, which was purified by silica gel chromatography (petroleum ether: EtOAc = 3:1) to afford the title product **(121b)** (2.5 g, 87%) as a yellow solid. *m*/*z* (ESI, +ve ion) = 219.1 [M+H]⁺.

### Step C. 2-(7-Methoxy-1H-indol-3-yl)ethan-1-amine (121c)

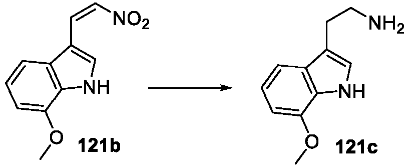

To a mixture of **121b** (3.0 g, 14 mmol) in anhydrous THF (50 mL) was added LiAlH₄ (3.3 g, 82 mmol) in THF (20 mL) at 0 °C under N₂. The reaction was heated to reflux for 1 h, and then allowed to stir at 25 °C for 16 h under N₂. After completion, the reaction was quenched with H₂O (50 mL), followed by addition of 15% NaOH solution. The mixture was extracted with EtOAc (50 mL x 4). The organic layer was washed with H₂O (50 mL), brine (50 mL), dried over anhydrous Na₂SO₄, filtered and concentrated to give a crude residue, which was purified by silica gel column chromatography (1% MeOH in DCM) to afford the title product **(121c)** (2.6 g, 89%) as a black oil. *m*/*z* (ESI, +ve ion) = 191.1 [M+H]⁺.

### Step D. 8-Methoxy-2,3,4,9-tetrahydro-1H-pyrido[3,4-b]indole (121d)

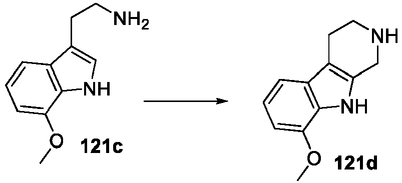

To a solution of **121c** (2.0 g, 11 mmol) in MeOH (2 mL) was added HCHO (975 mg, 13 mmol, 40% in water) and HOAc (20 mL) at 0 °C. The reaction was stirred at 0 °C for 1 h under N₂. After completion, the mixture was adjusted to pH = 8 with 2 N NaOH, and extracted with EtOAc (20 mL x 3). The organic layer was washed with H₂O (20 mL), brine (20 mL), dried over anhydrous Na₂SO₄, filtered and concentrated to give a crude residue, which was purified by silica gel column chromatography (5% MeOH in DCM) to afford the title product **(121d)** (2.0 g, 85%) as a brown solid. *m*/*z* (ESI, +ve ion) = 203.1 [M+H]⁺.

### Step E. 2,3,4,9-Tetrahydro-1H-pyrido[3,4-b]indol-8-ol (121e)

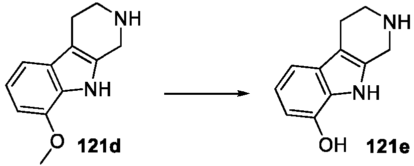

A flask was charged with 121d (2.0 g, 9.9 mmol) and added 40% HBr in H₂O (5 mL) at 25 °C. The reaction was stirred at 100 °C for 2 h under N₂. After completion, the reaction mixture was filtered and concentrated *in vacuo* to give a crude residue, which was purified by reverse phase HPLC (100% H₂O) to afford the title product (121e) (1.0 g, 46%) as a black solid. *m*/*z* (ESI, +ve ion) = 189.1 [M+H]⁺.

### Step F. tert-Butyl 8-hydroxy-1,3,4,9-tetrahydro-2H-pyrido[3,4-b]indole-2-carboxylate (121f)

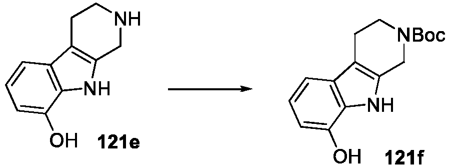

To a solution of **121e** (400 mg, 2.1 mmol) in THF/H₂O (30 mL) was added (Boc)₂O (232 mg, 1.1 mmol) and NaHCO₃ (179 mg, 2.1 mmol) at 0 °C. The mixture was stirred at 0 °C for 0.5 h under N₂. After completion, the reaction was diluted with H₂O (20 mL) and extracted with EtOAc (30 mL x 3). The organic layer was washed with H₂O (20 mL), brine (20 mL), dried over anhydrous Na₂SO₄, filtered and concentrated to give a crude residue, which was purified by silica gel column chromatography (petroleum ether: EtOAc = 1:1) to afford the title product **(121f)** (90 mg, 13%) as a white solid. *m*/*z* (ESI, +ve ion) = 289.1 [M+H]⁺.

### Step G. tert-Butyl 8-((4-Chloro-2-fluorobenzyl)oxy)-1,3,4,9-tetrahydro-2H-pyrido[3,4-b]indole-2-carboxylate (121g)

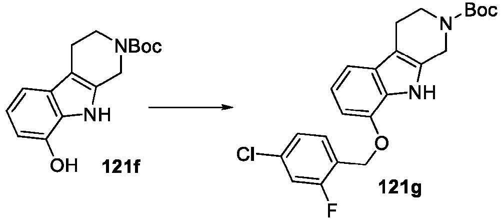

To a solution of **121f** (50 mg, 0.17 mmol) and 1-(bromomethyl)-4-chloro-2-fluorobenzene (43 mg, 0.19 mmol) in acetone (8 mL) was added K₂CO₃ (24 mg, 0.17 mmol) at 25 °C. The reaction was then heated at 30 °C for 48 h under N₂. After completion, the reaction was quenched with H₂O (8 mL) and extracted with EtOAc (10 mL x 3). The organic layer was washed with H₂O (10 mL), brine (10 mL), dried over anhydrous Na₂SO₄, filtered and concentrated to give a crude residue, which was purified by silica gel column chromatography (petroleum ether:EtOAc = 10:1) to afford the title product (121g) (35 mg, 44%) as a colorless oil. *m*/*z* (ESI, +ve ion) = 431.0 [M+H]⁺.

### Step H. 8-((4-Chloro-2-fluorobenzyl)oxy)-2,3,4,9-tetrahydro-1H-pyrido[3,4-b]indole (121h)

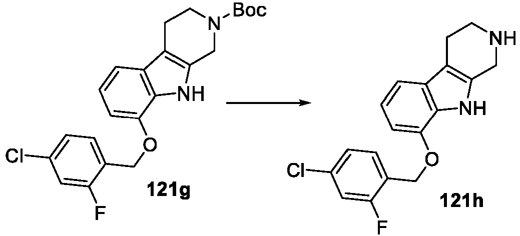

To a solution of **121g** (35 mg, 0.081 mmol) in DCM (3 mL) was added TFA (0.3 ml) at 0 °C. The mixture was stirred at 0 °C for 1 h under N₂. After completion, the reaction was concentrated to afford the title product **(121h)** (20 mg, 71%) as a white solid, which was used in the next step without further purification. *m*/*z* (ESI, +ve ion) = 331.1 [M+H]⁺.

### Step I. tert-Butyl (S)-2-((8-((4-chloro-2-fluorobenzyl)oxy)-1,3,4,9-tetrahydro-2H-pyrido[3,4-b]indol-2-yl)methyl)-1-(oxetan-2-ylmethyl)-1H-benzo[d]imidazole-6-carboxylate (121i)

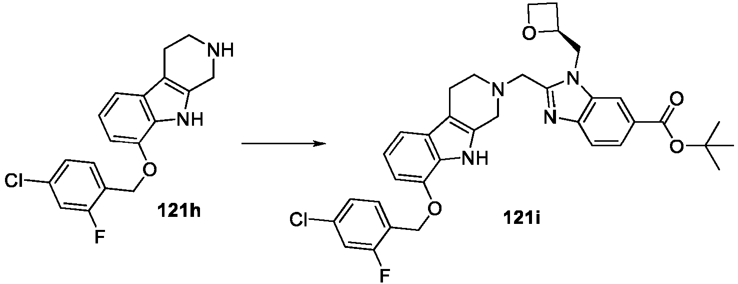

To a solution of **121h** (30 mg, 0.091 mmol) and *tert-butyl* 2-(chloromethyl)-3-[(2*S*)-oxetan-2-ylmethyl]-1,3-benzodiazole-5-carboxylate (34 mg, 0.10 mmol, prepared as described for **1h**) in DMF (5 mL) was added K₂CO₃ (25 mg, 0.18 mmol) and KI (15 mg, 0.091 mmol) at 25 °C. The reaction was stirred at 25 °C for 1 h under N₂. After completion, the reaction was quenched with H₂O (5 mL) and extracted with EtOAc (5mL x 3). The organic layer was washed with H₂O (5 mL), brine (5 mL), dried over anhydrous Na₂SO₄, filtered and concentrated to give a crude residue, which was purified by silica gel column chromatography (petroleum ether:EtOAc = 3:1) to afford the title product **(121i)** (15 mg, 24%) as a yellow oil. *m*/*z* (ESI, +ve ion) = 631.2 [M+H]⁺.

### Step J. (S)-2-((8-((4-Chloro-2-fluorobenzyl)oxy)-1,3,4,9-tetrahydro-2H-pyrido[3,4-b]indol-2-yl)methyl)-1-(oxetan-2-ylmethyl)-1H-benzo[d]imidazole-6-carboxylic acid (121)

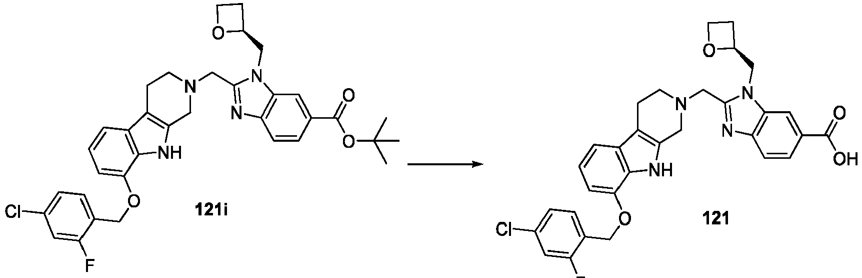

To a solution of **121i** (10 mg, 0.016 mmol) in DCM (4 mL) was added TFA (0.8 ml) at 0 °C. The reaction was raised to room temperature and stirred at 25 °C for 16 h under N₂. After completion, the reaction was concentrated to give a crude residue, which was purified by reverse phase HPLC (40% CH₃CN in H₂O, with 0.05%TFA as modifier) to afford the TFA salt of the title compound (121) (6 mg, 55%) as a yellow solid. ¹H NMR (400 MHz, CD₃OD) δ ppm 8.35 (d, *J =* 0.88 Hz, 1H), 8.01 - 8.09 (m, 1H), 7.82 (d, *J= 8.77* Hz, 1H), 7.62 (t, J=8.11 Hz, 1H), 7.19 - 7.30 (m, 2H), 7.14 (d, *J=* 7.89 Hz, 1H), 7.00 (t, J= 7.67 Hz, 1H), 6.81 (d, *J=* 7.89 Hz, 1H), 5.26 (s, 2H), 5.17 (qd, *J=* 7.16, 2.19 Hz, 1H), 4.97 (s, 2H), 4.73 - 4.81 (m, 1H), 4.62 - 4.69 (m, 3H), 4.56 (dd, *J =* 16.01, 5.92 Hz, 1H), 4.27 - 4.33 (m, 1H), 3.81 - 3.89 (m, 2H), 3.17 (t, *J =* 5.92 Hz, 2H), 2.70 - 2.81 (m, 1H), 2.39 - 2.51 (m, 1H). *m*/*z* (ESI, +ve ion) = 575.1 [M+H]⁺.

Example **124, 125, 129, 141** were synthesized in similar procedures as described in Example **60.**

### Example 128. 2-((4-((2-((2,4-Dichlorophenoxy)methyl)pyridin-4-yl)methyl)piperidin-1-yl)methyl)-1-((1-ethyl-1H-imidazol-5-yl)methyl)-1H-benzo[d]imidazole-6-carboxylic acid (128)

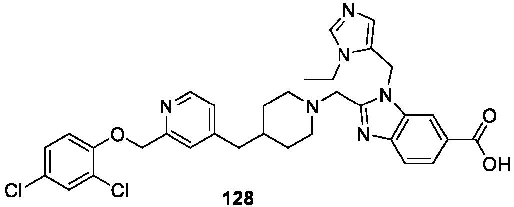

### Step A. Methyl 4-((1-(tert-butoxycarbonyl)-1,2,3,6-tetrahydropyridin-4-yl)methyl)picolinate (128a)

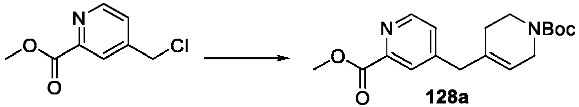

To a mixture of methyl 4-(chloromethyl)pyridine-2-carboxylate (116 mg, 0.63 mmol), *tert*-butyl 4-(4,4,5,5-tetramethyl-1,3,2-dioxaborolan-2-yl)-1,2,3,6-tetrahydropyridine-1-carboxylate (194 mg, 0.63 mmol), Pd(dppf)Cl₂ (46 mg, 0.063 mmol) and K₃PO₄ (266 mg, 1.25 mmol) was added THF (6 mL) and water (6 mL). The resulting suspension was heated at reflux overnight. After being cooled to room temperature, the mixture was extracted with ethyl acetate (10 mL x 3) and washed with brine. The combined organic phase was dried with anhydrous Na₂SO₄, filtered and concentrated. The resulting crude residue was purified by reverse phase HPLC (gradient elution, 20-100% CH₃CN in water, with 0.1% TFA as modifier) to afford the title product **(128a)** (42 mg, 20%) as a white solid. *m*/*z* (ESI, +ve ion) = 333.1 [M+H]⁺.

### Step B. Methyl 4-((1-(tert-butoxycarbonyl)piperidin-4-yl)methyl)picolinate (128b)

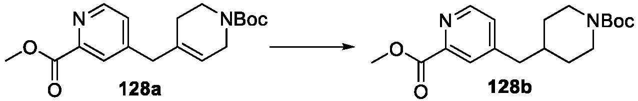

A solution of **128a** (42 mg, 0.12 mmol) and 10% Pd/C (8.4 mg) in MeOH (6 mL) was stirred under hydrogen at room temperature for 22 h. After completion, the reaction was filtered through a pad of celite and washed with MeOH (6 mL x 3). The organic layer was concentrated to afford the title product **(128b)** (34 mg) as a colorless oil, which was used in the next step without further purification. *m*/*z* (ESI, +ve ion) = 335.3 [M+H]⁺.

### Step C. tert-Butyl 4-((2-(hydroxymethyl)pyridin-4-yl)methyl)piperidine-1-carboxylate (128c)

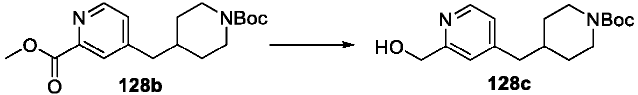

To a mixture of **128b** (34 mg, 0.1 mmol), CaCl₂ (23 mg, 0.2 mmol) and NaBH₄ (15 mg, 0.4 mmol) was added THF (10 mL). The resulting suspension was stirred at 60 °C overnight and cooled down. The reaction was quenched with water (10 mL), extracted with ethyl acetate (10 mL x 3) and washed with brine. The combined organic phase was dried with anhydrous Na₂SO₄, filtered and concentrated to give a crude residue, which was purified by silica gel column chromatography (EtOAc in hexanes) to afford the title product **(128c)** (7 mg, 19% for 2 steps) as a colorless oil. *m*/*z* (ESI, +ve ion) = 307.2 [M+H]⁺.

### Step D. tert-Butyl 4-((2-((2,4-dichlorophenoxy)methyl)pyridin-4-yl)methyl)piperidine-1-carboxylate (128d)

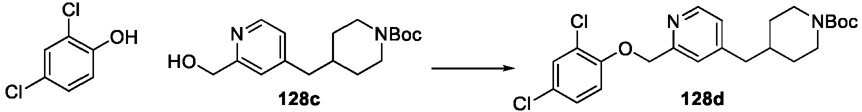

A flask was charged with 2,4-dichlorophenol (7 mg, 0.043 mmol), **128c** (12 mg, 0.039 mmol), and PPh₃ (12 mg, 0.047 mmol). Anhydrous THF (5 mL) was added and the reaction was cooled down in an ice bath. Diethyl azodicarboxylate (9.5 mg, 0.047 mmol) was added dropwise and the resulting mixture was stirred at 0 °C for 0.5 h, then at room temperature overnight. After completion, the reaction was quenched with water (20 mL), extracted with EtOAc (20 mL x 3) and washed with brine. The combined organic layer was dried with anhydrous Na₂SO₄, filtered and concentrated to provide a crude residue, which was purified by silica gel column chromatography (EtOAc in hexanes) to afford the title product **(128d)** (12.6 mg) as a colorless oil with some impurity, which was used in next step without further purification. *m*/*z* (ESI, +ve ion) = 451.3 [M+H]⁺.

### Step E. 2-((2,4-Dichlorophenoxy)methyl)-4-(piperidin-4-ylmethyl)pyridine (128e)

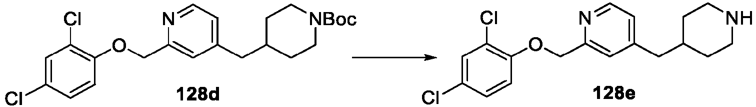

A solution of **128d** (12.6 mg, 0.02 mmol) in DCM (0.5 mL) was treated with TFA (0.5 mL) at room temperature for 10 min. After completion, the solvent was removed *in vacuo,* and the resulting crude residue was purified by reverse phase HPLC (gradient elution, 20-100% CH₃CN in water, with 0.1% formic acid as a modifier) to afford the title product (128e) (5.9 mg, 44%) as a white solid. *m*/*z* (ESI, +ve ion) = 351.3 [M+H]⁺.

### Step F. Methyl 2-((4-((2-((2,4-dichlorophenoxy)methyl)pyridin-4-yl)methyl)piperidin-1-yl)methyl)-1-((1-ethyl-1H-imidazol-5-yl)methyl)-1H-benzo[d]imidazole-6-carboxylate (128f)

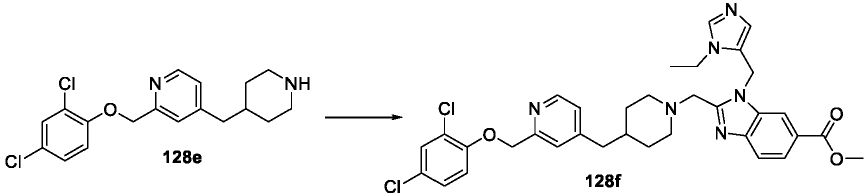

A solution of **128e** (4.8 mg, 0.014 mmol), 57f (5.5 mg, 0.016 mmol) and Et₃N (14 mg, 0.14 mmol) in DMF (1 mL) was stirred at room temperature for 24 h. After completion, the reaction was purified by reverse phase HPLC (gradient elution, 0-90% CH₃CN in water, with 0.1% TFA as a modifier) to provide the title product **(128f)** (14 mg) as a white solid with some impurity. *m*/*z* (ESI, +ve ion) = 647.3 [M+H]⁺.

### Step G. 2-((4-((2-((2,4-Dichlorophenoxy)methyl)pyridin-4-yl)methyl)piperidin-1-yl)methyl)-1-((1-ethyl-1H-imidazol-5-yl)methyl)-1H-benzo[d]imidazole-6-carboxylic acid (128)

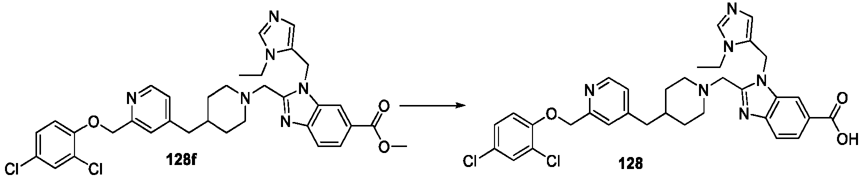

A solution of **128f** (14 mg, impure) in THF (0.5 mL) was treated with 2 N NaOH (0.5 mL) and MeOH (2 drops). The resulting mixture was stirred at room temperature for 6 h. After completion, the reaction was purified by reverse phase HPLC (gradient elution, 0-90% CH₃CN in water with 0.1% formic acid as a modifier) to provide the title product (128) (4.5 mg, 51%) as a white solid. ¹H NMR (600 MHz, CD₃OD) δ ppm 8.41 (d, J= 5.14 Hz, 1H), 8.13 (br d, *J =* 1.10 Hz, 1H), 7.99 (dd, *J =* 8.62, 1.65 Hz, 1H), 7.87 (s, 1H), 7.68 - 7.72 (m, 1H), 7.41 - 7.44 (m, 1H), 7.40 (s, 1H), 7.22 (dd, *J=* 8.80, 2.57 Hz, 1H), 7.18 (br dd, *J=* 5.14, 1.47 Hz, 1H), 7.07 (d, *J=* 8.80 Hz, 1H), 6.60 (s, 1H), 5.76 (s, 2H), 5.24 (s, 2H), 4.08 (q, *J=* 7.34 Hz, 2H), 3.84 (s, 2H), 2.81 (br d, *J=* 11.37 Hz, 2H), 2.57 (br d, *J=* 6.97 Hz, 2H), 2.04 - 2.12 (m, 2H), 1.57 - 1.64 (m, 1H), 1.51 (br d, *J=* 12.47 Hz, 2H), 1.29 (t, *J=* 7.34 Hz, 3H), 1.02 - 1.09 (m, 2H). *m*/*z* (ESI, +ve ion) = 633.2 [M+H]⁺.

Example compounds 137, 139, 148, 149, 151, 158, 159, 165, 166, 165, 166, 187, and **188** were synthesized in similar procedures as described in Example 128.

### Example 130. 2-((4-((3-((2-Chloro-4-methylphenoxy)methyl)phenyl)(hydroxy)methyl)piperidin-1-yl)methyl)-1-((1-ethyl-1H-imidazol-5-yl)methyl)-1H-benzo[d]imidazole-6-carboxylic acid (130)

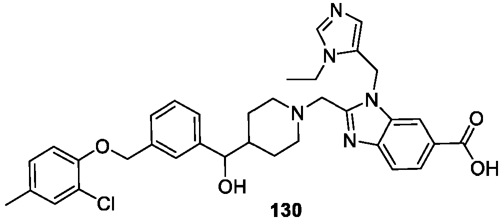

### Step A. 1-[(3-Bromophenyl)methoxy]-2-chloro-4-methylbenzene (130a)

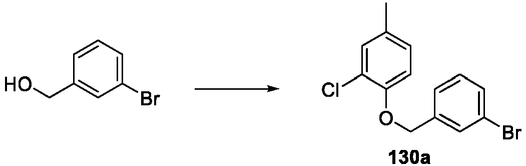

A mixture of (3-bromophenyl)methanol (3.0 g, 0.016 mol), 2-chloro-4-methylphenol (2.7 g, 0.019 mol) and PPh₃ (4.6 g, 0.018 mol) in THF (50 mL) was added DEAD (3.1 g, 0.018 mol) at 0 °C. The mixture was stirred at 25 °C for 8 h under N₂. After completion, the mixture was concentrated *in vacuo* to give a crude residue, which was purified by silica gel column chromatography (petroleum ether: EtOAc = 10:1) to afford the title product (130a) (4.5 g, 86%) as a yellow oil. ¹H NMR (400 MHz, DMSO-d6) δ 7.67 (s, 1H), 7.50 - 7.56 (m, 1H), 7.46 (d, *J= 7.7* Hz, 1H), 7.36 (t, *J =* 7.8 Hz, 1H), 7.26 (s, 1H), 7.09 (d, *J =* 0.9 Hz, 2H), 5.18 (s, 2H), 2.23 (s, 3H).

### Step B. tert-Butyl 4-((3-((2-chloro-4-methylphenoxy)methyl)phenyl)(hydroxy)methyl)piperidine-1-carboxylate (130b)

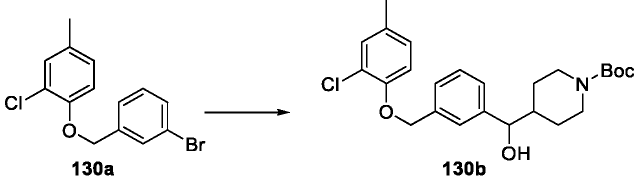

To a mixture of **130a** (3.0 g, 9.6 mmol, 1.0 eq) in THF (50 mL) was added nBuLi (2.6 M in hexanes) (5 ml, 13 mmol) at -65 °C. After 30 min, tert-butyl 4-formylpiperidine-1-carboxylate (2.1 g, 9.6 mmol) was added dropwise and the reaction was stirred at -65 °C for 1 h under N₂. After completion, the mixture was quenched with NH₄Cl (40 mL), and extracted with EtOAc (30 mL x 3). The organic layer was washed with H₂O (20 mL), brine (20 mL), dried over anhydrous Na₂SO₄, filtered and concentrated *in vacuo* to give a crude residue, which was purified by silica gel column chromatography (petroleum ether:EtOAc = 10:1) to afford the title product **(130b)** (3.6 g, 75%) as a yellow oil. *m*/*z* (ESI, +ve ion) = 468.2 [M+Na]⁺.

### Step C. Methyl 2-((4-((3-((2-chloro-4-methylphenoxy)methyl)phenyl)(hydroxy)methyl)piperidin-1-yl)methyl)-1-((1-ethyl-1H-imidazol-5-yl)methyl)-1H-benzo[d]imidazole-6-carboxylate (130c)

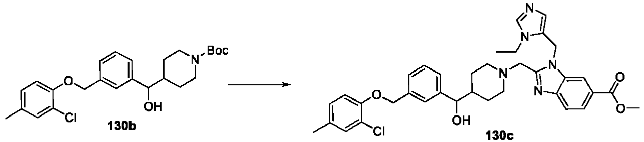

To a solution of **130b** (13 mg, 0.03 mmol) in anhydrous CH₂Cl₂ (0.3 mL) was added TFA (0.12 mL) at room temperature. After 1 h, the mixture was concentrated, and sat. NaHCO₃ (0.5 mL) was added. The reaction was extracted with EtOAc (2 mL x 3). The organic layer was dried over Na₂SO₄, filtered, and concentrated to afford a crude amine (13 mg). To this crude amine (13 mg) in dichloroethane (0.3 mL) was added **57f** (12 mg, 0.036 mmol) and DIPEA (0.01 mL) at room temperature. The reaction was then heated at 60 °C for 20 h. After cooling down to room temperature, the reaction was concentrated and purified by silica gel column chromatography (gradient elution, 0-10% MeOH in DCM) to afford the title product **(130c)** (16 mg, impure) as a colorless film. *m*/*z* (ESI, +ve ion) = 642.3 [M + H]⁺.

### Step D. 2-((4-((3-((2-Chloro-4-methylphenoxy)methyl)phenyl)(hydroxy)methyl)piperidin-1-yl)methyl)-1-((1-ethyl-1H-imidazol-5-yl)methyl)-1H-benzo[d]imidazole-6-carboxylic acid (130)

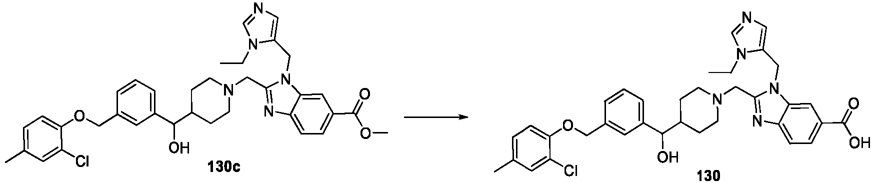

130c (16 mg, impure) was dissolved in a mixed solvent of THF and H₂O (2:1, 0.25 mL) at room temperature. LiOH (2.1 mg, 0.05 mmol) was added. After 16 h of stirring, the reaction was acidified with 1 N HCl to pH = 7 and concentrated. The crude residue was purified by reverse phase HPLC (gradient elution, 0-70% CH₃CN in water, with 0.1% TFA as a modifier) to provide the TFA salt of the title product (130) as a white solid (12.5 mg, 58% over 2 steps, racemic). ¹H NMR (600 MHz, CD₃OD) δ ppm 8.92 (s, 1H), 8.22 (d, *J =* 0.92 Hz, 1H), 8.05 (dd, *J=* 8.54, 1.53 Hz, 1H), 7.82 (d, *J=* 8.54 Hz, 1H), 7.46 (s, 1H), 7.35 - 7.41 (m, 2H), 7.30 (br d, *J =* 6.41 Hz, 1H), 7.18 (d, *J =* 1.53 Hz, 1H), 6.96 - 7.04 (m, 3H), 5.80 (s, 2H), 5.14 (s, 2H), 4.57 (br s, 2H), 4.47 (br d, *J=* 6.71 Hz, 1H), 4.29 (q, J= 7.22 Hz, 2H), 3.56 - 3.75 (m, 2H), 2.86 - 3.02 (m, 2H), 2.22 (s, 3H), 2.02 - 2.11 (m, 1H), 1.83 - 1.95 (m, 1H), 1.55 - 1.62 (m, 3H), 1.52 (t, *J=* 7.32 Hz, 3H). *m*/*z* (ESI, +ve ion) = 628.3 [M+H]⁺.

### Example 131. 2-((4-((3-((2-Chloro-4-methylphenoxy)methyl)phenyl)fluoromethyl)piperidin-1-yl)methyl)-1-((1-ethyl-1H-imidazol-5-yl)methyl)-1H-benzo[d]imidazole-6-carboxylic acid (131)

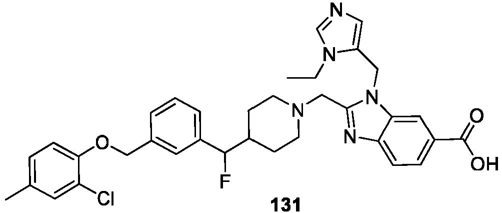

### Step A. tert-Butyl 4-((3-((2-chloro-4-methylphenoxy)methyl)phenyl)fluoromethyl)piperidine-1-carboxylate (131a)

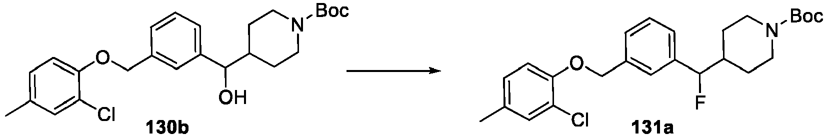

To a solution of **130b** (53 mg, 0.12 mmol) in anhydrous CH₂Cl₂ (1.2 mL) was added Deoxo-Fluor (70 mg, 0.14 mmol, 45% in THF) at 0 °C. After 0.5 h, the reaction was warmed to room temperature and stirred for 16 h. Then sat. NaHCO₃ was added. The mixture was extracted with EtOAc (5 mL x 3) and the organic layer was dried over Na₂SO₄, filtered, and concentrated. The resulting crude residue was purified by silica gel column chromatography (gradient elution, 0-40% EtOAc in hexanes) to afford the title product **(131a)** (19 mg, 35%) as a colorless film. *m*/*z* (ESI, +ve ion) = 470.1 [M + H]⁺.

### Step B. 2-((4-((3-((2-Chloro-4-methylphenoxy)methyl)phenyl)fluoromethyl)piperidin-1-yl)methyl)-1-((1-ethyl-1H-imidazol-5-yl)methyl)-1H-benzo[d]imidazole-6-carboxylic acid (131)

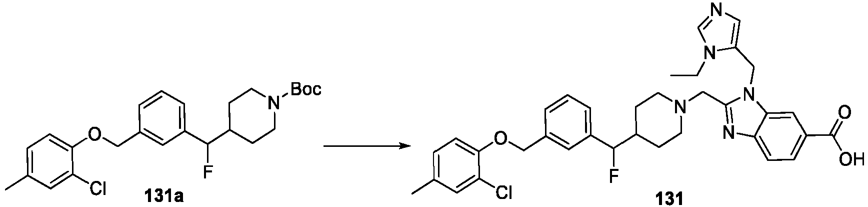

The title product (131) was synthesized in similar procedures from **131a** as described in Example **130,** Steps C and D as a racemic mixture. ¹H NMR (600 MHz, CD₃OD) δ ppm 9.04 (d, *J =* 1.10 Hz, 1H), 8.20 - 8.26 (m, 1H), 8.06 (dd, J=8.62, 1.65 Hz, 1H), 7.80 - 7.90 (m, 1H), 7.35 - 7.51 (m, 3H), 7.25 - 7.35 (m, 1H), 7.19 (d, J=1.83 Hz, 1H), 6.92 - 7.08 (m, 3H), 5.79 - 5.90 (m, 2H), 5.37 (dd, *J =* 46.8, 6.24 Hz, 1H), 5.13 - 5.17 (m, 2H), 4.59 - 4.69 (m, 2 H) 4.33 (q, *J=* 7.34 Hz, 2H) 3.65 - 3.85 (m, 2 H), 3.06 (br s, 2H), 2.12 - 2.25 (m, 4H), 2.00 - 2.10 (m, 1H), 1.67 - 1.76 (m, 3H), 1.56 (t, *J=* 7.3 Hz, 3H). *m*/*z* (ESI, +ve ion) = 630.3 [M+H]⁺.

### Example 132. 2-((4-((3-((2-Chloro-4-methylphenoxy)methyl)phenyl)difluoromethyl)piperidin-1-yl)methyl)-1-((1-ethyl-1H-imidazol-5-yl)methyl)-1H-benzo|[d]imidazole-6-carboxylic acid (132)

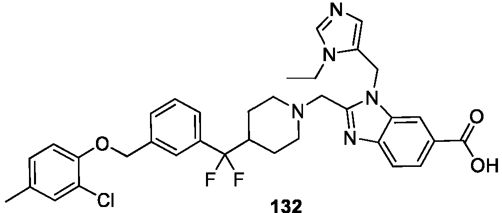

### Step A. tert-Butyl 4-(3-((2-chloro-4-methylphenoxy)methyl)benzoyl)piperidine-1-carboxylate (132a)

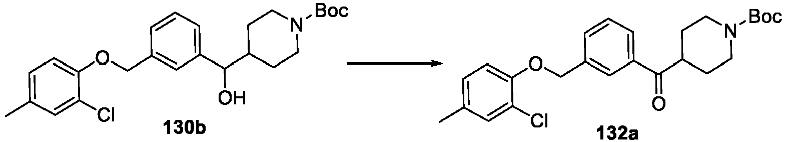

To a solution of **130b** (2.0 g, 4.5 mmol) in DCM (50 mL) was added Dess-Martin periodinane (3.8 g, 8.9 mmol) at 0 °C. The reaction was heated at 30 °C for 16 h under N₂. After completion, the mixture was quenched with aq. NaHCO₃ (40 mL), and extracted with DCM (50 mL x 3). The organic layer was washed with H₂O (50 mL), brine (50 mL), dried over anhydrous Na₂SO₄, filtered and concentrated *in vacuo* to give a crude residue, which was purified by silica gel column chromatography (petroleum ether: EtOAc = 3:1) to afford the title product **(132a)** (1.2 g, 58%) as a white solid. *m*/*z* (ESI, +ve ion) = 466.2 [M+Na]⁺.

### Step B. tert-Butyl 4-((3-((2-chloro-4-methylphenoxy)methyl)phenyl)difluoromethyl)piperidine-1-carboxylate (132b)

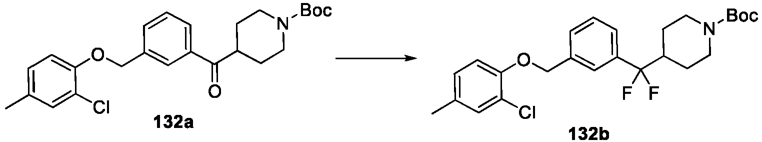

Deoxo-Fluor (112 mg, 0.50 mmol, 45% in THF) was added to **132a** (150 mg, 0.34 mmol) and heated to 80 °C. After 40 h, the reaction was concentrated and the crude residue was purified by silica gel column chromatography (gradient elution, 0-40% EtOAc in hexanes) to afford the title compound (132b) (34 mg, 21%) as a colorless film. *m*/*z* (ESI, +ve ion) = 466.2 [M + H]⁺.

### Step C. 2-((4-((3-((2-Chloro-4-methylphenoxy)methyl)phenyl)difluoromethyl)piperidin-1-yl)methyl)-1-((1-ethyl-1H-imidazol-5-yl)methyl)-1H-benzo[d]imidazole-6-carboxylic acid (132)

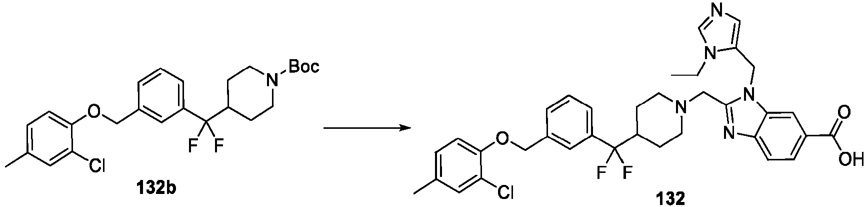

The title product (132) was synthesized in similar procedures from **132b** as described in Example 130, Steps C and D.

¹H NMR (600 MHz, MeOD) δ ppm 9.01 (d, J= 1.10 Hz, 1H), 8.21 (d, J= 1.10 Hz, 1H), 8.05 (dd, J= 8.80, 1.47 Hz, 1H), 7.82 (d, *J=* 8.80 Hz, 1H), 7.58 - 7.62 (m, 2H), 7.51 (t, *J* = 7.70 Hz, 1H), 7.42 (br d, *J=* 7.70 Hz, 1H), 7.20 (d, *J=* 1.47 Hz, 1H), 6.99 - 7.07 (m, 3H), 5.82 (d, *J=* 0.73 Hz, 2H), 5.19 (s, 2H), 4.46 (br s, 2H), 4.32 (q, *J =* 7.34 Hz, 2H), 3.48 - 3.58 (m, 2H), 2.76 - 2.87 (m, 2H), 2.31 - 2.44 (m, 1H), 2.24 (s, 3H), 1.86 (br d, *J =* 13.20 Hz, 2H), 1.51 - 1.64 (m, 5H). *m*/*z* (ESI, +ve ion) = 648.3 [M+H]⁺.

Example **161** was prepared in similar procedures as described in Example **132.**

### Example 136. (S)-2-((4-((2-(((3-Chloro-5-cyclopropylpyridin-2-yl)oxy)methyl)oxazol-5-yl)methyl)piperidin-1-yl)methyl)-1-(oxetan-2-ylmethyl)-1H-benzo[d]imidazole-6-carboxylic acid (136)

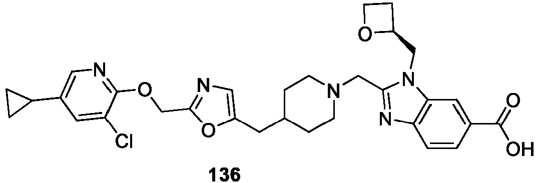

### Step A. 2,3-Dichloro-5-cyclopropylpyridine (136a)

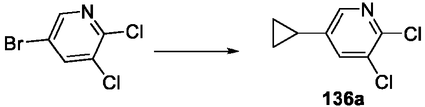

A mixture of 5-bromo-2,3-dichloropyridine (2.2 g, 10.0 mmol), cyclopropylboronic acid (1.0 g, 12.0 mmol), Pd(OAc)₂ (112 mg, 0.5 mmol), tri(o-tolyl)phosphine (304 mg, 1.0 mmol) and K₂CO₃ (4.1 g, 30.0 mmol) in 20 mL toluene and 5 mL H₂O was stirred at 80 °C for 4 h under N₂. After completion, the reaction was diluted with H₂O (20 mL) and extracted with EtOAc (30 mL x 3). The organic layer was dried over anhydrous Na₂SO₄, filtered, and concentrated to give a crude residue, which was purified by silica gel column chromatography (5% EtOAc in petroleum ether) to afford the title product **(136a)** (1.7 g, 91%) as a colorless oil. *m*/*z* (ESI, +ve ion) = 188.1 [M+H]⁺.

### Step B. tert-Butyl 4-((2-(((3-chloro-5-cyclopropylpyridin-2-yl)oxy)methyl)oxazol-5-yl)methyl)piperidine-1-carboxylate (136b)

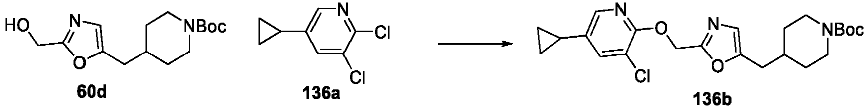

A mixture of **136a** (260 mg, 1.4 mmol), **60d** (411 mg, 1.4 mmol) and Cs₂CO₃ (1.4 g, 4.2 mmol) in DMF (5 mL) was stirred at 80 °C for 16 h. After completion, the reaction was diluted with H₂O (30 mL) and extracted with EtOAc (30 mL x 3). The combined organic layer was washed with brine (30 mL x 3), dried over anhydrous Na₂SO₄, filtered and concentrated to give a crude residue, which was purified by silica gel column chromatography (5% MeOH in DCM) to afford the title product (136b) (340 mg, 54%) as colorless oil. *m*/*z* (ESI, +ve ion) = 448.2 [M+H]⁺.

### Step C. 2-(((3-Chloro-5-cyclopropylpyridin-2-yl)oxy)methyl)-5-(piperidin-4-ylmethyl)oxazole (136c)

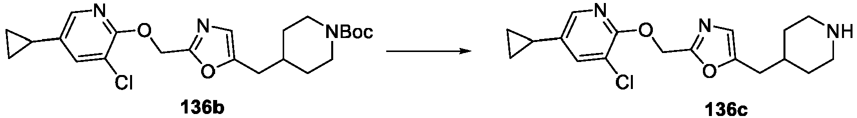

To a mixture **of 136b** (100 mg, 0.22 mmol) in DCM (10 mL) was added 2,6-lutidine (523 mg, 4.4 mmol) and TMSOTf (488 mg, 2.2 mmol) at 0 °C. The mixture was stirred at 0 °C for 1 h. After completion, the reaction was quenched with NH₄Cl (10 mL) and extracted with EtOAc (20 mL x 3). The combined organic layer was washed with H₂O (20 mL), brine (20 mL), dried over anhydrous Na₂SO₄, filtered and concentrated to afford the crude title product **(136c)** (230 mg) as a yellow oil, which was used in the next step without further purification. *m*/*z* (ESI, +ve ion) = 348.2 [M+H]⁺.

### Step D. Methyl (S)-2-((4-((2-(((3-chloro-5-cyclopropylpyridin-2-yl)oxy)methyl)oxazol-5-yl)methyl)piperidin-1-yl)methyl)-1-(oxetan-2-ylmethyl)-1H-benzo[d]imidazole-6-carboxylate (136d)

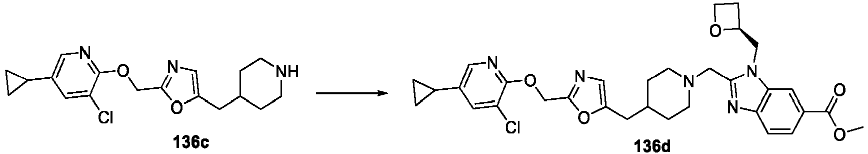

To a stirred solution of **136c** (230 mg, 0.22 mmol) and K₂CO₃ (93 mg, 0.66 mmol) in DMF (3 mL) was added **1h** (65 mg, 0.22 mmol) at 15 °C. The mixture was stirred at 15 °C for 16 h. After completion, the reaction was quenched with H₂O (5 mL) and extracted with DCM (20 mL x 3). The combined organic layer was washed with brine (10 mL), dried over anhydrous Na₂SO₄, filtered and concentrated to give a crude residue, which was purified by silica gel column chromatography (2% MeOH in DCM) to afford the title product (136d) (78 mg, 59%) as a white solid. *m*/*z* (ESI, +ve ion) = 606.3 [M+H]⁺.

### Step E. (S)-2-((4-((2-(((3-Chloro-5-cyclopropylpyridin-2-yl)oxy)methyl)oxazol-5-yl)methyl)piperidin-1-yl)methyl)-1-(oxetan-2-ylmethyl)-1H-benzo[d]imidazole-6-carboxylic acid (136)

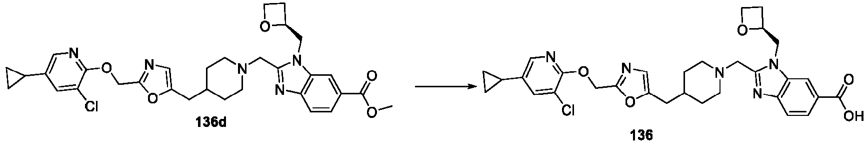

A mixture of **136d** (78 mg, 0.1 mmol) in THF (3 mL) and H₂O (1 mL) was added lithium hydroxide (24 mg, 1.0 mmol). The reaction was stirred at 17 °C for 16 h. After completion, the reaction was adjusted to pH = 7 with 1 N HCl and extracted with EtOAc (20 mL x 3). The organic layer was dried, filtered and concentrated and the crude residue was purified by reverse phase HPLC (CH₃CN in H₂O, with 0.1%TFA as a modifier) to afford the TFA salt of the title product (136) (26.0 mg, 29%) as a white solid. ¹H NMR (400 MHz, CD₃OD) δ ppm 8.33 (d, *J* = 0.73 Hz, 1H), 8.04 (dd, *J =* 8.56, 1.47 Hz, 1H), 7.86 (d, *J =* 1.96 Hz, 1H), 7.80 (d, *J* = 8.56 Hz, 1H), 7.45 (d, *J* = 1.96 Hz, 1H), 6.92 (s, 1H), 5.44 (s, 2H), 5.14 - 5.25 (m, 1H), 4.80 (d, *J* = 3.42 Hz, 2H), 4.71 - 4.78 (m, 1 H), 4.56 - 4.71 (m, 2H), 4.40 (dt, *J* = 9.23, 5.78 Hz, 1H), 3.78 (br s, 2H), 3.15 - 3.32 (m, 2H), 2.68 - 2.86 (m, 3H), 2.42 - 2.55 (m, 1H), 1.90 - 2.12 (m, 3H), 1.86 (tt, *J =* 8.47, 5.10 Hz, 1H), 1.51 - 1.70 (m, 2H), 0.89 - 1.01 (m, 2H), 0.55 - 0.70 (m, 2H). *m*/*z* (ESI, +ve ion) = 592.3 [M+H]⁺.

Example **144** was synthesized in similar procedures as described in Example **136.**

Example **138** was synthesized in similar procedures as described in Example **60.**

Example 142 and Example **143** were synthesized from 4-iodophenol and (2,4-dichlorophenyl)methanol as described in Example **114,** Step F, followed by similar procedures in Example 90, Steps C to E and Example **134,** Steps A to B.

### Example 145. 2-((4-((2-((2,4-Dichlorophenoxy)methyl)pyridin-4-yl)oxy)piperidin-1-yl)methyl)-1-((1-ethyl-1H-imidazol-5-yl)methyl)-1H-benzo[d]imidazole-6-carboxylic acid (145)

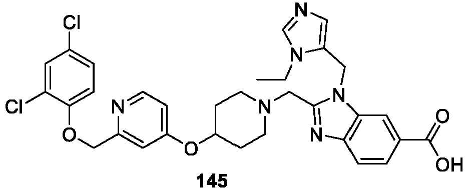

### Step A. Methyl 4-((1-(tert-butoxycarbonyl)piperidin-4-yl)oxy)picolinate (145a)

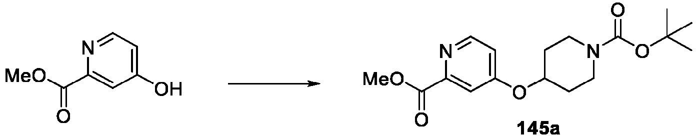

To a stirred solution of methyl 4-hydroxypicolinate (400 mg, 2.61 mmol), 1-Boc-4-hydroxypiperidine (526 mg, 2.61 mmol) and triphenylphosphine (856 mg, 3.27 mmol) in THF (15 mL) was added diisopropyl azodicarboxylate (660 mg, 3.27 mmol) dropwise at room temperature. The mixture was heated at 55 °C for 23 h. After cooling down to room teperature, it was concentrated under reduced pressure. The crude residue was purified by silica gel column chromatography (gradient elution, 10% to 60% acetone in hexanes) to afford the title product **(145a)** (454 mg, 52%) as a yellow gum. *m*/*z* (ESI, +ve ion) = 337.4 [M+H]⁺.

### Step B. tert-Butyl 4-((2-(hydroxymethyl)pyridin-4-yl)oxy)piperidine-1-carboxylate (145b)

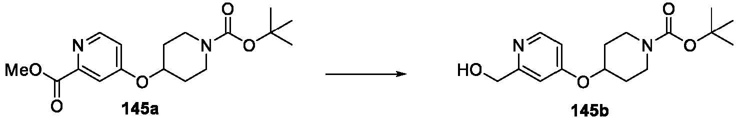

To a stirred solution of **145a** (420 mg, 1.25 mmol) in EtOH (6.2 mL) was added CaCl₂ (416 mg, 3.75 mmol) at 0 °C, followed by the addition of NaBH₄ (134 mg, 3.75 mmol). The resulting mixture was allowed to warm to room temperature and stirred for 2.5 h. It was cooled back down to 0 °C and the reaction was quenched by adding saturated ammonium chloride. The reaction mixture was diluted with H₂O and extracted with EtOAc (x3). The combined organic layer was washed with brine, dried over Na₂SO₄, filtered and concentrated under reduced pressure. The crude residue was purified by silica gel column chromatography (gradient elution, 0-10% MeOH in DCM) to afford the title product **(145b)** (313 mg, 81%) as a colorless gum. *m*/*z* (ESI, +ve ion) = 309.5 [M+H]⁺.

### Step C. tert-Butyl 4-((2-((2,4-dichlorophenoxy)methyl)pyridin-4-yl)oxy)piperidine-1-carboxylate (145c)

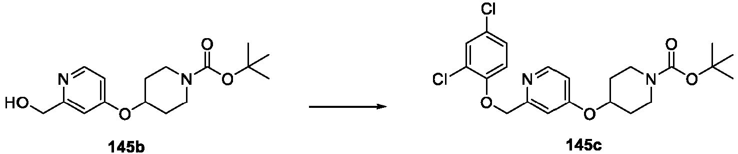

To a stirred solution of **145b** (50.0 mg, 0.162 mmol), 2,4-dichlorophenol (27.8 mg, 0.170 mmol) and triphenylphosphine (63.8 mg, 0.243 mmol) in THF (1.0 mL) at room temperature was added diisopropyl azodicarboxylate (49.2 mg, 0.243 mmol) dropwise. The mixture was stirred at room teperature for 6 h. The reaction mixture was concentrated under reduced pressure. The crude residue was purified by silica gel column chromatography (gradient elution, 20-40% acetone in hexanes) to afford the title product **(145c)** (62.2 mg, 85%) as a white solid. *m*/*z* (ESI, +ve ion) = 453.3 [M+H]⁺.

### Step D. 2-((2,4-Dichlorophenoxy)methyl)-4-(piperidin-4-yloxy)pyridine (145d)

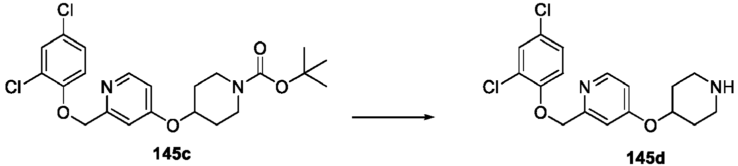

A solution of **145c** (60 mg, 0.132 mmol) in DCM/TFA (2:1, 2.0 mL) was stirred at room temperature for 2 h. The mixture was concentrated under reduced pressure and the residue was diluted with DCM, and washed with saturated sodium bicarbonate and brine. The organic layer was dried over Na₂SO₄, filtered and concentrated under reduced pressure to provide the title product (145d) (51.1 mg) as a white solid, which was used in the next step without further purification. *m*/*z* (ESI, +ve ion) = 353.3 [M+H]⁺.

### Step E. Methyl 1-((1-ethyl-1H-imidazol-5-yl)methyl)-2-(hydroxymethyl)-1H-benzo[d]imidazole-6-carboxylate (145e)

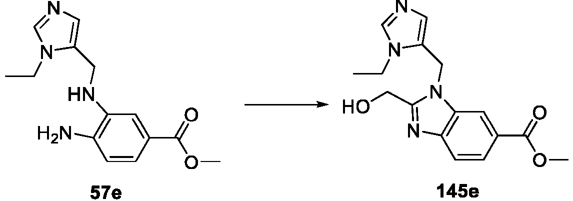

A mixture of **57e** (150 mg, 0.547 mmol) and glycolic acid (62.4 mg, 0.82 mmol) in 1,3,5-trimethylbenzene (1.5 mL) was heated at 140 °C for 19 h. It was cooled to room temperature and stirred at the same temperature for 3 h. The yellow solution was decanted off to give an orange residue that was dissolved in MeOH (5 mL) and concentrated under reduced pressure. The crude residue was purified by silica gel column chromatography (gradient elution, 0-15% MeOH in DCM) to afford the title product **(145e)** (32.4 mg, 19%) as a white foamy solid. *m*/*z* (ESI, +ve ion) = 315.1 [M+H]⁺.

### Step F. Methyl 1-((1-ethyl-1H-imidazol-5-yl)methyl)-2-formyl-1H-benzo[d]imidazole-6-carboxylate (145f)

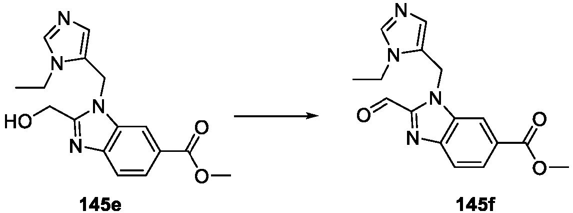

To s stirred suspension of **145e** (32.0 mg, 0.102 mmol) in DCM (2 mL) was added Dess-Martin periodinane (47.5 mg, 0.112 mmol) at room temperature. The mixture was stirred at the same temperature for 1 h. The reaction was quenched by the addtion of 10% Na₂S₂O₃ aqueous solution (1 mL) and saturated sodium bicarbonate (0.6 mL). The resulting mixture was stirred at room temperature for 5 min, diluted with water and extracted with DCM (x3). The combined organic layer was washed with brine, dried over Na₂SO₄, filtered and concentrated under reduced pressure to provide the title product **(145f)** (40 mg) as a white foamy solid, which was used in the next step without further purification. *m*/*z* (ESI, +ve ion) = 313.2 [M+H]⁺.

### Step G. Methyl 2-((4-((2-((2,4-dichlorophenoxy)methyl)pyridin-4-yl)oxy)piperidin-1-yl)methyl)-1-((1-ethyl-1H-imidazol-5-yl)methyl)-1H-benzo[d]imidazole-6-carboxylate (145g)

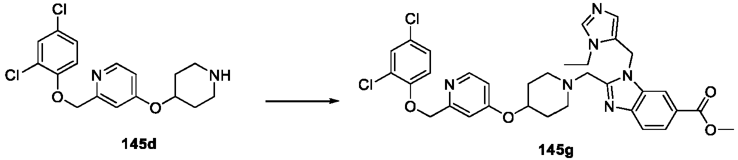

To a stirred solution of **145d** (10.0 mg, 0.032 mmol) and **145f** (13.6 mg, 0.038 mmol) in 1,2-dichloroethane (0.3 mL) at room temperature was added AcOH (5.8 mg, 0.096 mmol), followed by addition of sodium triacetoxyborohydride (10.1 mg, 0.048 mmol). After stirring at the same temperature for 1 h, the reaction was quenched with saturated sodium bicarbonate and extracted with DCM (x3). The combined organic layer was washed with brine, dried over Na₂SO₄, filtered and concentrated under reduced pressure. The crude residue was purified by silica gel column chromatography (0-20% MeOH in DCM, gradient elution) to afford the title product (145g) (15.4 mg, 74%) as a off-white foamy solid. *m*/*z* (ESI, +ve ion) = 651.3 [M+H]⁺.

### Step H. 2-((4-((2-((2,4-Dichlorophenoxy)methyl)pyridin-4-yl)oxy)piperidin-1-yl)methyl)-1-((1-ethyl-1H-imidazol-5-yl)methyl)-1H-benzo[d]imidazole-6-carboxylic acid (145)

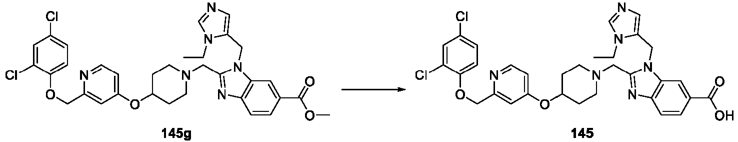

To a stirred solution of **145g** (12.3 mg, 0.0189 mmol) in THF/MeOH/H₂O (2:1:1, 0.4 mL) was added lithium hydroxide monohydrate (2.4 mg, 0.57 mmol) at room temperature. It was stirred for 4 h, then 1 N HCl was added to the mixture to adjust to pH = 3. The solution was purified by reverse phase HPLC (gradient elution, 20-60% CH₃CN in H₂O, with 0.1% TFA as a modifier) to give the TFA salt of the title compound (145) (8 mg, 56% yield) as a white solid. ¹H NMR (600 MHz, CD₃OD) δ 9.07 (d, *J* =1.3 Hz, 1H), 8.56 (d, *J =* 6.6 Hz, 1H), 8.22 (d, *J=* 0.7 Hz, 1H), 8.06 (dd, *J=* 8.4, 1.5 Hz, 1H), 7.81 (d, *J =* 8.6 Hz, 1H), 7.50 - 7.53 (m, 2H), 7.31 - 7.36 (m, 2H), 7.19 (d, *J* = 9.0 Hz, 1H), 7.08 (d, *J=* 1.1 Hz, 1H), 5.91 (s, 2H), 5.35 (s, 2H), 4.93 (br s, 1H), 4.31 - 4.40 (m, 4H), 3.15 - 3.20 (m, 2H), 2.91 - 3.04 (m, 2H), 2.15 - 2.20 (m, 2H), 1.94 (br s, 2H), 1.56 (t, *J=* 7.3 Hz, 3H). *m*/*z* (ESI, +ve ion) = 635.0 [M+H]⁺.

### Example 146. (S)-2-((4-((6-((2-(Hydroxymethyl)-4-methylphenoxy)methyl)pyridin-2-yl)methyl)piperidin-1-yl)methyl)-1-(oxetan-2-ylmethyl)-1H-benzo[d]imidazole-6-carboxylic acid (146)

### Step A. tert-Butyl 4-((6-((2-(methoxycarbonyl)-4-methylphenoxy)methyl)pyridin-2-yl)methyl)piperidine-1-carboxylate (146a)

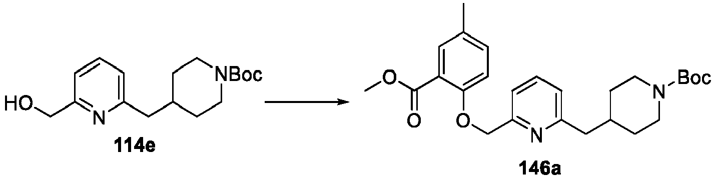

To a solution of 114e (800 mg, 2.61 mmol), methyl 2-hydroxy-5-methylbenzoate (434 mg, 2.61 mmol) and PPh₃ (1.02 g, 3.92 mmol) in THF (20 mL) was added DEAD (682 mg, 3.92 mmol) dropwise at 0 °C. The resulting mixture was stirred at 15 °C for 16 h. The reaction was diluted with EtOAc (50 mL) and washed with brine (50 mL). The EtOAc layer was dried over Na₂SO₄, filtered and concentrated. The crude residue was purified by silica gel column chromatography (20% EtOAc in petroleum ether) to afford the title product (146a) (260 mg, 21.8%) as a colorless oil. *m*/*z* (ESI, +ve ion) = 455.2 [M+H]⁺.

### Step B. tert-Butyl 4-((6-((2-(hydroxymethyl)-4-methylphenoxy)methyl)pyridin-2-yl)methyl)piperidine-1-carboxylate (146b)

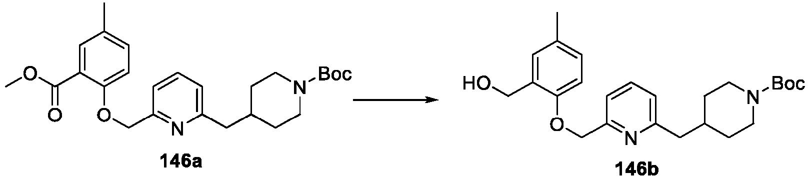

To a solution of **146a** (180 mg, 0.40 mmol) in THF (10 ml) was added LiBH₄ (43 mg, 1.98 mmol). The reaction was stirred at 15 °C for 16 h, quenched with saturated NH₄Cl (30 ml), and extracted with EtOAc (50 ml x 2). The combined EtOAc layer was dried, filtered and concentrated to give a crude residue, which was purified by silica gel column chromatography (15% MeOH in DCM) to afford the title product **(146b)** (150 mg, 91.7%) as a white solid. *m*/*z* (ESI, +ve ion) = 427.3 [M+H]⁺.

### Step C. (S)-2-((4-((6-((2-(Hydroxymethyl)-4-methylphenoxy)methyl)pyridin-2-yl)methyl)piperidin-1-yl)methyl)-1-(oxetan-2-ylmethyl)-1H-benzo[d]imidazole-6-carboxylic acid (146)

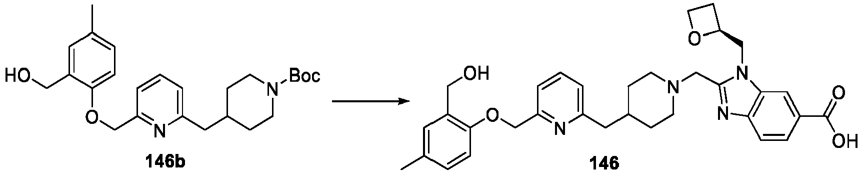

The title compound (146) was prepared from **146b** as described in Example **114,** Steps G-I. ¹H NMR (400 MHz, CD₃OD) δ ppm 8.18 (s, 1H), 7.93 (dd, *J =* 8.33, 1.32 Hz, 1H), 7.73 (t, *J* = 7.89 Hz, 1H), 7.55 (d, *J=* 8.77 Hz, 1H), 7.40 (d, *J* = 7.45 Hz, 1H), 7.13 - 7.24 (m, 2H), 6.99 (dd, *J =* 8.55, 1.97 Hz, 1H), 6.83 (d, *J =* 7.89 Hz, 1H), 5.24 (qd, *J =* 7.09, 2.85 Hz, 1H), 5.16 (s, 2H), 4.80 - 4.87 (m, 1 H), 4.55 - 4.74 (m, 4H), 4.45 (dt, *J =* 9.10, 5.97 Hz, 1H), 3.94 (d, *J =* 13.59 Hz, 1H), 3.82 (d, *J =* 13.59 Hz, 1H), 2.91 (d, *J =* 11.40 Hz, 1 H), 2.68 - 2.84 (m, 4H), 2.45 - 2.59 (m, 1H), 2.24 (s, 3H), 1.97 - 2.19 (m, 2H), 1.71 - 1.83 (m, 1H), 1.51 - 1.66 (m, 2H), 1.23 - 1.46 (m, 2H). *m*/*z* (ESI, +ve ion) = 571.3 [M+H]⁺.

### Example 150. 2-((4-((2-((2,4-Dichlorophenoxy)methyl)pyridin-4-yl)methyl)-4-(hydroxymethyl)piperidin-1-yl)methyl)-1-((1-ethyl-1H-imidazol-5-yl)methyl)-1H-benzo[d]imidazole-6-carboxylic acid (150)

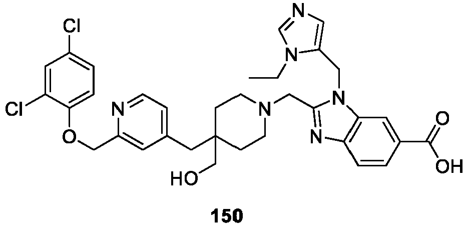

### Step A. Methyl 2-((2,4-dichlorophenoxy)methyl)isonicotinate (150a)

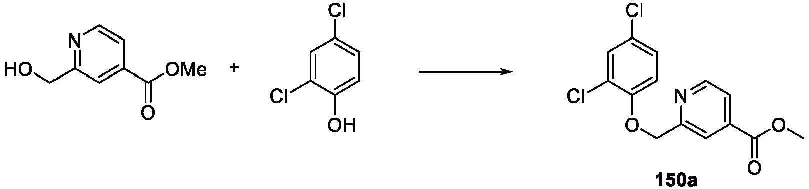

To a solution of methyl 2-(hydroxymethyl)pyridine-4-carboxylate (0.5 g, 2.99 mmol), 2,4-dichlorophenol (0.51 g, 2.99 mmol) and PPh₃ (1.18 g, 4.49 mmol) in THF (15 mL) was added DIAD (0.88 mL, 4.49 mmol) dropwise. The resulting mixture was stirred at room temperature for 4 h. After completion, reacation was concentrated and the resulting crude residue was purified by silica gel column chromatography (gradient elution, 40-70% EtOAc in hexanes) to provide the title product (150a) (0.84 g, 90%) as a white solid. *m*/*z* (ESI, +ve ion) = 312.1 [M+H]⁺

### Step B. (2-((2,4-Dichlorophenoxy)methyl)pyridin-4-yl)methanol (150b)

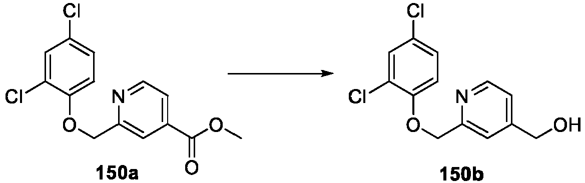

To an ice cold suspension of **150a** (1.9 g, 6.09 mmol) in EtOH (25 mL) and THF (25 mL) was added CaCl₂ (2.03 g, 18.3 mmol) and NaBH₄ (0.65 g, 18.3 mmol). The resulting reaction mixture was warmed to room temperature and stirred for 2 h. After completion, reaction was cooled to 0 °C and quenched with sat. NH₄Cl (20 mL). EtOH and THF were removed under reduced pressure. The resulting aqueous phase was extracted with EtOAc (20 mL x 3). The combined organic layer was washed with brine (20 mL), dried over Na₂SO₄, concentrated and purified by silica gel column chromatography (gradient elution, 70-100% EtOAc in hexanes) to provide the title product **(150b)** (1.02 g, 59%) as a white solid. *m*/*z* (ESI, +ve ion) = 284.3 [M+H]⁺

### Step C. 4-(Bromomethyl)-2-((2,4-dichlorophenoxy)methyl)pyridine (150c)

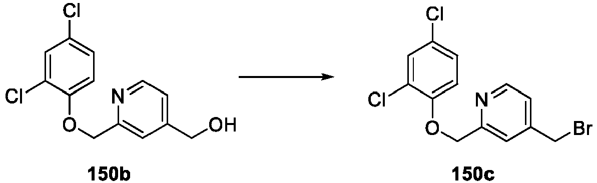

PBr₃ (0.18 mL, 1.94 mmol) was added dropwise to an ice cold solution of **150b** (0.5 g, 1.76 mmol) in THF (8.8 mL). The resulting white suspension was stirred at room temperature for 1 h. After completion, the reaction was cooled to 0 °C, and added ice water (10 mL). The reaction mixture was extracted with EtOAc (10 mL x 3). The combined organic layer was washed with sat. NaHCO₃ solution (50 mL), brine (10 mL), dried over Na₂SO₄, filtered, and concentrated to provide the title product **(150c)** (0.63 g) as a white solid, which was used in the next step without further purification. *m*/*z* (ESI, +ve ion) = 346.0 [M+H]⁺

### Step D. 1-(tert-Butyl) 4-methyl 4-((2-((2,4-dichlorophenoxy)methyl)pyridin-4-yl)methyl)piperidine-1,4-dicarboxylate (150d)

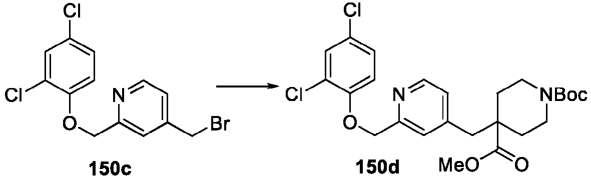

To a solution of diisopropylamine (0.44 mL, 3.12 mmol) in THF (4 mL) at -78 °C, n-butyllithium in hexanes (1.37 mL, 3.42 mmol) was added dropwise. The resulting solution was warmed to room temperature and stirred for 30 min before cooling down to -78 °C again. A solution of 1-tert-butyl 4-methyl piperidine-1,4-dicarboxylate (0.54 g, 2.23 mmol) in THF (3 mL) was added dropwise, and the reaction was stirred for an additional 30 min, followed by dropwise addition of **150c** (0.52 g, 1.49 mmol) in THF. The reaction was warmed to room temperature slowly and stirred overnight. Then the reaction was quenched with sat. NH₄Cl solution (10 mL) and extracted with EtOAc (10 mL x 3). The combined organic layer was washed with brine (10 mL), dried over Na₂SO₄, filtered, concentrated and purified by silica gel column chromatography (gradient elution, 60-100% EtOAc in hexanes) to provide the title product (150d) (0.47 g, 62%) as a colorless oil. *m*/*z* (ESI, +ve ion) = 509.3 [M+H]⁺

### Step E. tert-Butyl 4-((2-((2,4-dichlorophenoxy)methyl)pyridin-4-yl)methyl)-4-(hydroxymethyl)piperidine-1-carboxylate (150e)

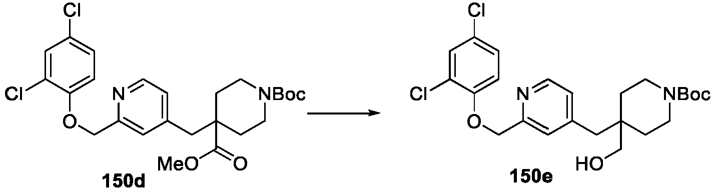

Lithium aluminum hydride solution in THF (0.77 mL, 1.85 mmol) was added dropwise to an ice cold solution of **150d** (0.47 g, 0.92 mmol) in THF (4.6 mL). The reaction was stirred at 0 °C for 10 min. and quenched with water (5 mL). The resulting mixture was filtered through a pad of celite. The filtrate was extracted with EtOAc (x3). The combined organic layer was washed with brine (5 mL), dried over Na₂SO₄, filtered, concentrated and purified by silica gel column chormatography (gradient elution, 80-100% EtOAc in hexanes) to provide the title product (150e) (0.26 g, 59%) as a colorless oil. *m*/*z* (ESI, +ve ion) = 481.3 [M+H]⁺

### Step F. (4-((2-((2,4-Dichlorophenoxy)methyl)pyridin-4-yl)methyl)piperidin-4-yl)methanol (150f)

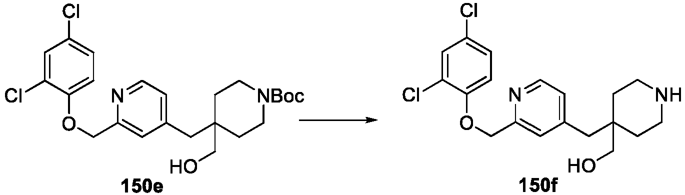

A solution of **150e** (15 mg, 0.031 mmol) in DCM (1 mL) and TFA (0.5 mL) was stirred at room temperature for 10 min. After completion, the reaction solvent was removed under reduced pressure and the resulting residue was diluted with EtOAc (3 mL). The organic layer was washed with sat. NaHCO₃ (5 mL), brine (3 mL), dried over Na₂SO₄, filtered and concentrated to afford the title product (150f) (12 mg) as a colorless oil, which was used in the next step without further purification. *m*/*z* (ESI, +ve ion) = 381.1 [M+H]⁺

### Step G. Methyl 2-((4-((2-((2,4-dichlorophenoxy)methyl)pyridin-4-yl)methyl)-4-(hydroxymethyl)piperidin-1-yl)methyl)-1-((1-ethyl-1H-imidazol-5-yl)methyl)-1H-benzo[d]imidazole-6-carboxylate (150g)

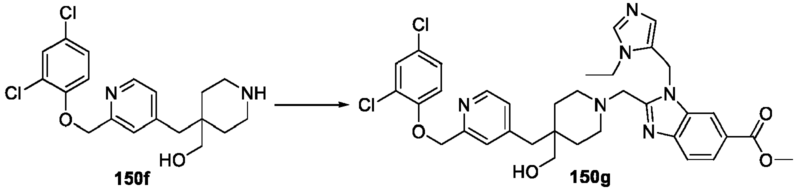

A solution of **150f** (11.5 mg, 0.03 mmol), 57f (16.7 mg, 0.045 mmol) and DIPEA (0.026 mL, 0.15 mmol) in DMF (0.5 mL) was stirred at room temperature overnight. After completion, the reaction mixture was diluted with EtOAc (5 mL). The organic layer was washed with water (3 mL x 2), brine (3 mL), dried over Na₂SO₄, filtered and concentrated and the resulting crude residue was purified by silica gel column chromatography (gradient elution, 0-20% MeOH in DCM) to afford the title product (150g) (6.4 mg, 31%) as a pale-yellow oil. *m*/*z* (ESI, +ve ion) = 677.2 [M+H]⁺.

### Step H. 2-((4-((2-((2,4-Dichlorophenoxy)methyl)pyridin-4-yl)methyl)-4-(hydroxymethyl)piperidin-1-yl)methyl)-1-((1-ethyl-1H-imidazol-5-yl)methyl)-1H-benzo[d]imidazole-6-carboxylic acid (150)

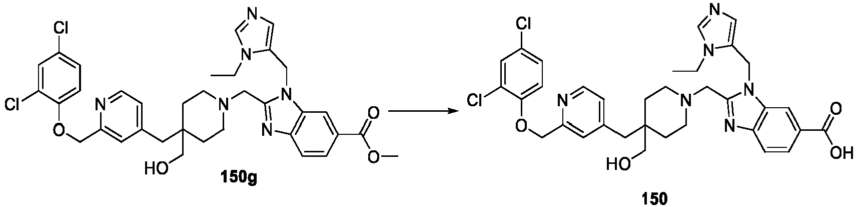

A solution of **150g** (6.4 mg, 0.009 mmol) and LiOH (1.2 mg, 0.028 mmol) in THF:MeOH:H₂O = 2:1:1 (0.4 mL) was stirred at 30 °C overnight. After completion, the reaction mixture was diluted with water to 1 mL and purified by reverse phase HPLC (gradient elution, 20-100% CH₃CN in H₂O, with 0.1% formic acid as a modifier) to afford the title product **(150)** (3.1 mg, 49%) as a white solid. ¹H NMR (600 MHz, CD₃OD) δ ppm 8.42 (d, *J* = 5.1 Hz, 1H), 8.13 (s, 1H), 8.00 (dd, *J* = 8.6, 1.7 Hz, 1H), 7.80 (s, 1H), 7.73 (d, *J =* 8.4 Hz, 1H), 7.41 (s, 1H), 7.35 (d, *J =* 2.6 Hz, 1H), 7.22 (dd, *J =* 5.1, 1.5 Hz, 1H), 7.19 (dd, *J* = 9.2, 2.6 Hz, 1H), 7.05 (d, *J =* 9.2 Hz, 1H), 6.57 (s, 1H), 5.73 (s, 2H), 5.25 (s, 2H), 4.04 (q, *J =* 7.2 Hz, 2H), 3.87 (s, 2H), 3.21 (s, 2H), 2.72 (s, 2H), 2.49 - 2.56 (m, 2H), 2.39 - 2.46 (m, 2H), 1.19 - 1.34 (m, 7H). *m*/*z* (ESI, +ve ion) = 663.3 [M+H]⁺.

Examples **168, 169,** and **170** were synthesized from **145d** in similar procedures as described in Example **150,** Steps G and H. Example **171, 174, 182, 183** were synthesized in similar procedures as described in Example **168.**

### Example 152. (S)-2-((4-((6-((2-Chloro-4-cyclopropylphenoxy)methyl)pyridin-2-yl)methyl)piperidin-1-yl)methyl)-1-(oxetan-2-ylmethyl)-1H-benzo[d]imidazole-6-carboxylic acid (152)

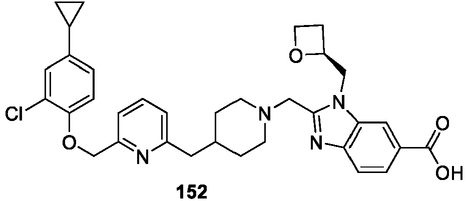

### Step A. tert-Butyl 4-((6-((2-chloro-4-cyclopropylphenoxy)methyl)pyridin-2-yl)methyl)piperidine-1-carboxylate (152b)

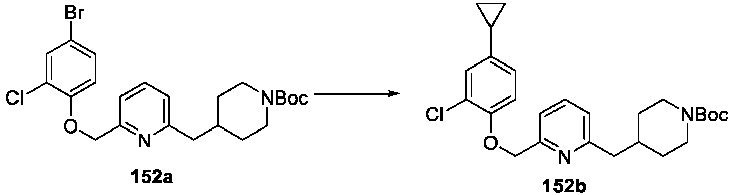

A mixture of **152a** (800 mg, 1.6 mmol, prepared in similar procedures as described in the synthesis of **114f),** cyclopropylboronic acid (690 mg, 8 mmol), Pd(dppf)Cl₂ (130 mg, 0.1 mmol) in toluene (26 mL) was degassed with nitrogen and stirred at 10 °C. A solution of Cs₂CO₃ (1.04 g, 3.2 mmol) in H₂O (4 mL) was added and the resulting mixture was heated at 100 °C for 2 h under N₂. After cooling down, the reaction was filtered and diluted with EtOAc (50 mL), washed with H₂O (15 mL), and brine (15 mL). The organic layer was concentrated and purified by silica gel column chromatography (EtOAc:petroleum ether = 1:4) to provide the title product **(152b)** (0.52 g, 62.5%) as a yellow oil. *m*/*z* (ESI, +ve ion) = 457.3 [M+H]⁺.

### Step B. 2-((2-Chloro-4-cyclopropylphenoxy)methyl)-6-(piperidin-4-ylmethyl)pyridine (152c)

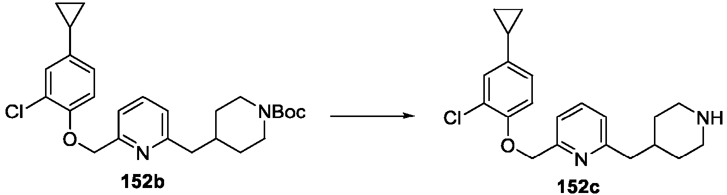

A mixture of 152b (40 mg, 0.09 mmol) in DCM (3 mL) was added TFA (0.5 mL) at 0 °C. The reaction was stirred at 0 °C for 1 h, and then diluted with DCM (15 mL), washed with aq. NaHCO₃ (8 mL x 2), H₂O (10 mL), and brine (10 mL). The organic layer was dried over Na₂SO₄, filtered and concentrated to afford the title product (152c) (25 mg, 72%) as a yellow oil. *m*/*z* (ESI, +ve ion) = 357.1 [M+H]⁺.

### Step C. Methyl (S)-2-((4-((6-((2-chloro-4-cyclopropylphenoxy)methyl)pyridin-2-yl)methyl)piperidin-1-yl)methyl)-1-(oxetan-2-ylmethyl)-1H-benzo[d]imidazole-6-carboxylate (152d)

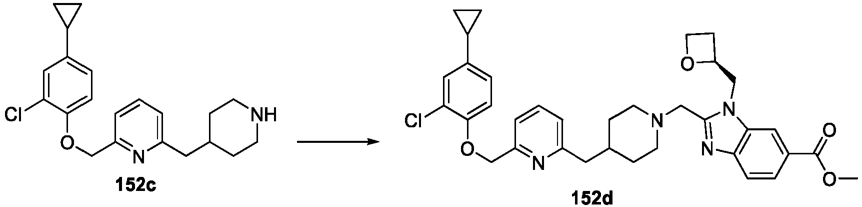

To a mixture of **152c** (50 mg, 0.14 mmol), **1h** (41 mg, 0.14 mmol) in DMF (5 mL) was added K₂CO₃ (58 mg, 0.42 mg), and potassium iodide (12 mg, 0.7 mmol) at 0 °C. The reaction was raised to room temperature and stirred for 1 h. The reaction was quenched with H₂O (50 ml), and extracted with ethyl acetate (50 mL x 3). The organic layer was dried over Na₂SO₄, filtered and concentrated to afford a crude residue, which was purified by reverse phase HPLC (65% CH₃CN in H₂O, with 0.05% TFA as a modifier) to afford the title product **(152d)** (60 mg, 62.7%) as a white solid. *m*/*z* (ESI, +ve ion) = 615.3 [M+H]⁺.

### Step D. (S)-2-((4-((6-((2-Chloro-4-cyclopropylphenoxy)methyl)pyridin-2-yl)methyl)piperidin-1-yl)methyl)-1-(oxetan-2-ylmethyl)-1H-benzo[d]imidazole-6-carboxylic acid (152)

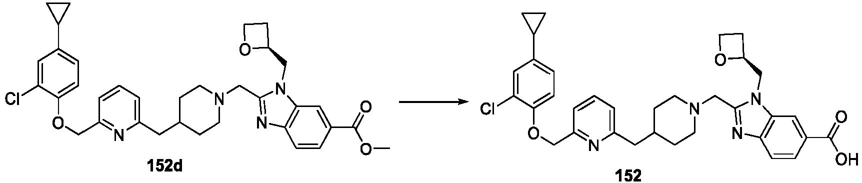

To a solution of **152d** (30 mg, 0.05 mmol) in MeOH/H₂O = 10:1 (5 ml) was added lithium hydroxide (23 mg, 0.98 mmol). The mixture was stirred for 48 h at 30 °C. The reaction was quenched with H₂O (50 ml), and then extracted with EtOAc (50 mL x 3). The organic layer was dried over Na₂SO₄ and concentrated to afford a crude residue, which was purified by reverse HPLC (65% CH₃CN in H₂O, with 0.05% TFA as a modifier) to afford the TFA salt of the title compound (152) (15mg, 51%) as a white solid. ¹H NMR (400 MHz, CD₃OD) δ 8.32 (d, *J= 0.91* Hz, 1H), 8.03 (dd, *J=* 8.55, 1.51 Hz, 1H), 7.85 (t, *J=* 7.75 Hz, 1H), 7.79 (d, *J =* 8.56 Hz, 1H), 7.55 (d, *J =* 7.56 Hz, 1H), 7.30 (d, *J =* 7.66 Hz, 1H), 7.09 (d, *J =* 2.06 Hz, 1H), 6.96 (dt, *J=* 8.50, 5.25 Hz, 2H), 5.19 (s, 2H), 4.60 - 4.80 (m, 6H), 4.39 (dt, *J=* 9.3, 5.9 Hz, 1H), 3.69 - 3.84 (m, 2H), 3.19 - 3.25 (m, 2H), 2.85 (d, J = 7.04 Hz, 2H), 2.73 - 2.82 (m, 1H), 2.42 - 2.52 (m, 1H), 2.17 (br s, 1H), 1.88 - 1.98 (m, 2H), 1.82 (td, *J* = 8.41, 4.20 Hz, 1H), 1.54 - 1.72 (m, 2H), 0.90 (ddd, *J =* 8.41, 6.29, 4.38 Hz, 2H), 0.58 (dt, *J =* 6.39, 4.51 Hz, 2H). *m*/*z* (ESI, +ve ion) = 601.2 [M+H]⁺.

Example **153** was synthesized in similar procedures as described in Example **152.**

### Example 154. (S)-2-((4-((2-(((4-Chloro-6-cyclopropylpyridin-3-yl)oxy)methyl)oxazol-5-yl)methyl)piperidin-1-yl)methyl)-1-(oxetan-2-ylmethyl)-1H-benzo[d]imidazole-6-carboxylic acid (154)

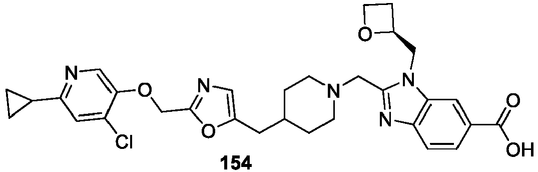

### Step A. 2-Bromo-5-methoxypyridine 1-oxide (154a)

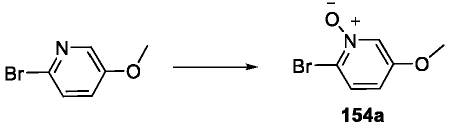

To a solution of 2-bromo-5-methoxypyridine (15 g, 79.8 mmol) in DCE (200 ml) was added m-CPBA (20.6 g,119.7 mmol). The reaction was stirred at 60 °C for 16 h. Then the reaction mixture was cooled down and washed with sat. Na₂CO₃ (200 mL x 3). The DCE layer was dried, filtered, and concentrated to afford the title product **(154a)** (15.8 g, 96.9%) as a white solid. *m*/*z* (ESI, +ve ion) = 206.0 [M+H]⁺.

### Step B. 2-Bromo-5-methoxy-4-nitropyridine 1-oxide (154b)

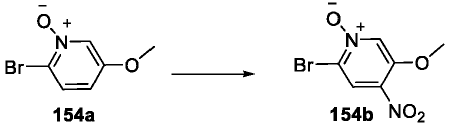

A flask was charged with sulfuric acid (40 mL) and added 154a (8 g, 39.2 mmol) followed by a dropwise addition of nitric acid (4.8 mL, 108 mmol). Then the reaction was cooled to 60 °C and the rest of nitric acid (19.2 mL, 432 mmol) was added. The resulting mixture was stirred at 60 °C for 30 min, then cooled and poured into ice/water mixture (800 mL). The solid was collected by filtration, washed with EtOAc (50 mL) and dried in an oven to afford the title compound **(154b)** (5.0 g, 49%) as a yellow solid, which was used in the next step without further purification. *m*/*z* (ESI, +ve ion) = 248.9 [M+H]⁺.

### Step C. 2-Bromo-5-methoxypyridin-4-amine (154c)

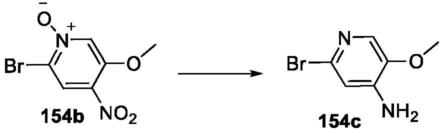

To a solution of 154b (4.5 g, 18.07 mmol) and iron powder (10.1 g, 180.72 mmol) in 75% EtOH/H2O (60 mL) was added NH₄Cl (9.7, 180.72 mmol). The mixture was stirred at 70 °C for 3 h. Then the reaction mixture was filtered and the residue was washed with EtOH (50 mL x 3). The combined filtrate was concentrated and redissolved in EtOAc (100 mL). The EtOAc layer was washed with water (20 mL), brine (20 mL), dried over Na₂SO₄, filtered and concentrated to afford the title compound **(154c)** (2.9 g, 75.3%) as a yellow solid, which was used in the next step without further purification. *m*/*z* (ESI, +ve ion) = 205.1 [M+H]⁺.

### Step D. 2-Bromo-4-chloro-5-methoxypyridine (154d)

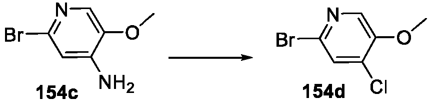

To a solution of **154c** (3.3 g, 16.3 mmol) and 37% HCl (30 mL) at 0 °C was added a solution of sodium nitrite (1.2 g, 17.1 mmol) in water (5 mL) dropwise. The resulting mixture was stirred at 0 °C for 15 min, then a solution of copper(I) chloride (1.96 g, 19.51 mmol) in 37% HCl (60 mL) was added dropwise at the same temperature. The resulting mixture was raised to room temperature and stirred for 2 h. Then the reaction was diluted with water (500 ml) and solid precipitates crashed out. The solids were collected by filtration, washed with water (50 mL), and redissolved in EtOAc (100 mL). The EtOAc layer was washed with sat. NaHCO₃ (100 mL x 2), brine (200 mL), dried over Na₂SO₄, and filtered. The filtrate was concentrated and the crude residue was purified by silica gel column chromatography (15% EtOAc in petroleum ether) to afford the title compound **(154d)** (1.9 g, 52.3%) as a yellow solid. ¹H NMR (400 MHz, CD₃OD) δ 8.15 (s, 1H), 7.67 (s, 1H), 4.00 (s, 3H).

### Step E. 6-Bromo-4-chloropyridin-3-ol (154e)

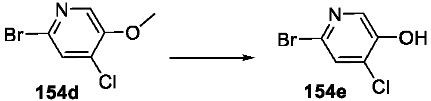

To a solution of **154d** (550 mg, 2.47 mmol) in CHCl₃ (15 mL) was added BBr₃ (1.25 g, 4.95 mmol). The reaction was stirred at 50 °C for 16 h, cooled down, washed with sat. NaHCO₃ (20 ml) and brine (20 ml). The organic layer was dried over Na₂SO₄, filtered, and concentrated to provide a crude residue, which was purified by silica gel column chromatography (5% MeOH in DCM) to afford the title product **(154e)** (350 mg, 67.8%) as a white solid. *m*/*z* (ESI, +ve ion) = 210.0 [M+H]⁺.

### Step F. tert-Butyl 4-((2-(((6-bromo-4-chloropyridin-3-yl)oxy)methyl)oxazol-5-yl)methyl)piperidine-1-carboxylate (154f)

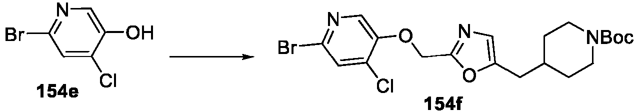

To a solution of **60d** (498 mg,1.69 mmol) and DIPEA (524 mg, 4.06 mmol) in DCM (10 mL) was added MsCl (232 mg, 2.03 mmol) at 0 °C. The reaction was stirred at 0 °C for 0.5 h and then washed with water (10 mL). The DCM layer was dried over Na₂SO₄, filtered and concentrated to provide a residue A. A separate flask was charged with **154e** (350 mg,1.69 mmol), K₂CO₃ (466 mg,3.38 mmol). DMF (10 mL) was added. To this mixture was added a solution of residue A in DMF. The reaction was stirred at 50 °C for 2 h. After cooling down, the reaction mixture was diluted with EtOAc (50 ml), and washed with brine (50 mL x 2). The EtOAc layer was dried, filtered, and concentrated to provide a crude residue, which was purified by silica gel column chromatography (30% EtOAc in petroleum ether) to afford the title product (154f) (540 mg, 65.7%) as a white solid. *m*/*z* (ESI, +ve ion) = 510.1 [M+Na]⁺.

### Step G. (S)-2-((4-((2-(((4-Chloro-6-cyclopropylpyridin-3-yl)oxy)methyl)oxazol-5-yl)methyl)piperidin-1-yl)methyl)-1-(oxetan-2-ylmethyl)-1H-benzo[d]imidazole-6-carboxylic acid (154)

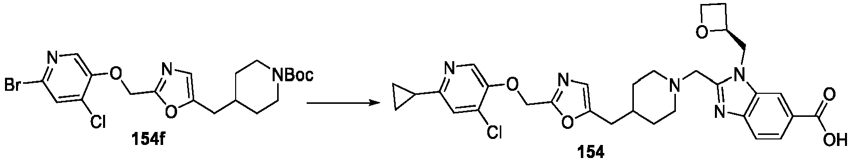

The title product **(154)** was synthesized in similar procedures as described in Example **114,** Steps G to I. ¹H NMR (400 MHz, CD₃OD) δ ppm 8.34 (d, *J =* 0.88 Hz, 1H), 8.28 (s, 1H), 7.98 - 8.09 (m, 1H), 7.80 (d, *J=* 8.77 Hz, 1H), 7.38 (s, 1H), 6.96 (s, 1H), 5.30 (s, 2H), 5.14 - 5.25 (m, 1H), 4.61 - 4.83 (m, 5H), 4.40 (dt, *J =* 9.43, 5.81 Hz, 1H), 3.72 - 3.88 (m, 2H), 3.18 - 3.29 (m, 2H), 2.73 - 2.86 (m, 3H), 2.41 - 2.56 (m, 1H), 1.89 - 2.13 (m, 4H), 1.61 (br s, 2H), 0.97 - 1.06 (m, 2H), 0.85 - 0.97 (m, 2H). *m*/*z* (ESI, +ve ion) = 592.2 [M+Na]⁺.

### Example 155. 2-((4-((2-((2,4-Dichlorophenoxy)methyl)pyridin-4-yl)methylene)piperidin-1-yl)methyl)-1-((1-ethyl-1H-imidazol-5-yl)methyl)-1H-benzo[d]imidazole-6-carboxylic acid (155)

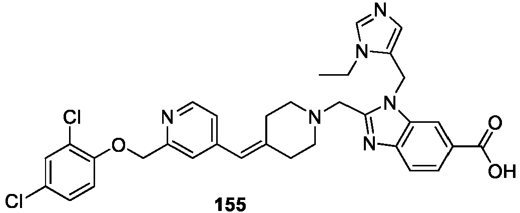

### Step A. Methyl 4-((1-(tert-butoxycarbonyl)piperidin-4-ylidene)methyl)picolinate (155a)

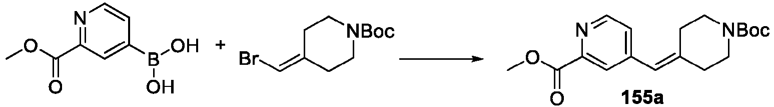

A flask was charged with (2-(methoxycarbonyl)pyridin-4-yl)boronic acid (66 mg, 0.37 mmol), *tert-butyl* 4-(bromomethylidene)piperidine-1-carboxylate (101 mg, 0.37 mmol), Cs₂CO₃ (238 mg, 0.73 mmol) and Pd(dppf)Cl₂ (27 mg, 0.037 mmol) and added a mixed solvent of THF:H₂O = 2:1 (18 mL). The mixture was degassed with nitrogen and stirred at 50 °C for 18 h under argon. After completion, the reaction was extracted with EtOAc (10 mL x 3). The organic layer was dried, filtered and concentrated. The crude residue was purified by silica gel column chromatography (EtOAc in hexanes) to afford the title product **(155a)** (16.5 mg, 14%) as a colorless oil. *m*/*z* (ESI, +ve ion) = 333.2 [M+H]⁺.

### Step B. tert-Butyl 4-((2-(hydroxymethyl)pyridin-4-yl)methylene)piperidine-1-carboxylate (155b)

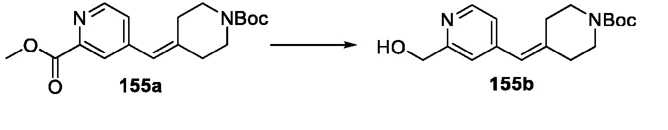

To a mixture of **155a** (17 mg, 0.05 mmol), CaCl₂ (11 mg, 0.1 mmol) and NaBH₄ (7 mg, 0.2 mmol) was added THF (10 mL). The resulting suspension was stirred at 60 °C overnight. After cooling down, the reaction was quenched with water (10 mL), extracted with ethyl acetate (10 mL x 3) and washed with brine. The combined organic phase was dried with anhydrous Na₂SO₄, filtered and concentrated to give a crude product which was purified by silica gel column chromatography (EtOAc in hexanes) to afford the title product **(155b)** (9.5 mg, 63%) as a colorless oil. *m*/*z* (ESI, +ve ion) = 305.1 [M+H]⁺.

### Step C. tert-Butyl 4-((2-((2,4-dichlorophenoxy)methyl)pyridin-4-yl)methylene)piperidine-1-carboxylate (155c)

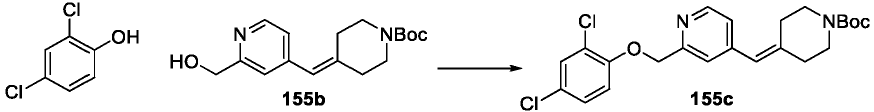

A flask was charged with 2,4-dichlorophenol (5.6 mg, 0.034 mmol), **155b** (9.5 mg, 0.031 mmol), and PPh₃ (9.8 mg, 0.038 mmol). Anhydrous THF (5 mL) was added and the reaction was cooled down in an ice bath. Diethyl azodicarboxylate (7.6 mg, 0.038 mmol) was added dropwise and the resulting mixture was stirred at 0 °C for 0.5 h, then at room temperature overnight. After completion, the reaction was quenched with water (5 mL), extracted with ethyl acetate (5 mL x 3) and washed with brine. The combined organic phase was dried with anhydrous Na₂SO₄, filtered and concentrated to provide a crude residue, which was purified by silica gel column chromatography (EtOAc in hexanes) to afford the title product **(155c)** as a colorless oil with some impurity, which was used in next step without further purification. *m*/*z* (ESI, +ve ion) = 451.1 [M+H]⁺.

### Step D. 2-((2,4-Dichlorophenoxy)methyl)-4-(piperidin-4-ylidenemethyl)pyridine (155d)

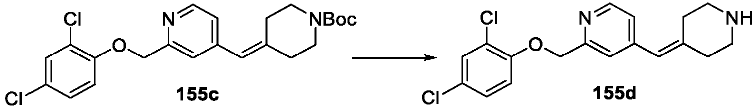

A solution of **155c** (impure) in DCM (2 mL) was treated with TFA (1 mL) at room temperature for 10 min. After completion, the solvent was removed *in vacuo,* and the resulting crude residue was purified by reverse phase HPLC (gradient elution, 0-90% CH₃CN in water with 0.1% TFA as a modifier) to provide the title product **(155d)** (6.3 mg, 44% for 2 steps) as a white solid. *m*/*z* (ESI, +ve ion) = 351.2 [M+H]⁺.

### Step E. Methyl 2-((4-((2-((2,4-dichlorophenoxy)methyl)pyridin-4-yl)methylene)piperidin-1-yl)methyl)-1-((1-ethyl-1H-imidazol-5-yl)methyl)-1H-benzo[d]imidazole-6-carboxylate (155e)

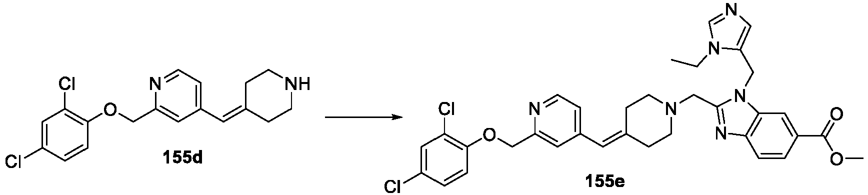

A solution of 155d (6.2 mg, 0.018 mmol), 57f (5.9 mg, 0.018 mmol) and Et₃N (18 mg, 0.18 mmol) in DMF (0.8 mL) was stirred at room temperature for 18 h. After completion, the reaction was purified by reverse phase HPLC (gradient elution, 0-90% CH₃CN in water, with 0.1% TFA as a modifier) to provide the title product (155e) (4.4 mg, 39%) as a white solid. m/z (ESI, +ve ion) = 645.3 [M+H]⁺.

### Step F. 2-((4-((2-((2,4-Dichlorophenoxy)methyl)pyridin-4-yl)methylene)piperidin-1-yl)methyl)-1-((1-ethyl-1H-imidazol-5-yl)methyl)-1H-benzo[d]imidazole-6-carboxylic acid (155)

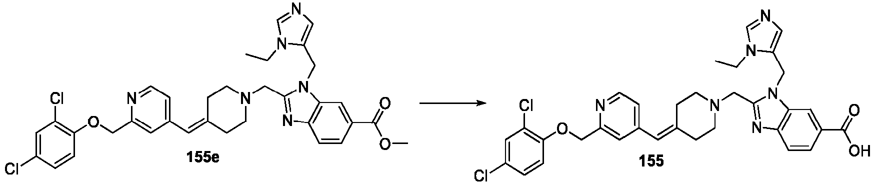

A solution of **155e** (4.4 mg, 0.007 mmol) in CH₃CN (0.4 mL) was treated with 2 N NaOH (0.4 mL). The resulting mixture was stirred at room temperature for 24 h. After completion, the reaction was purified by reverse phase HPLC (gradient elution, 0-75% CH₃CN in water, with 0.1% formic acid as a modifier) to provide the title product (155) (2.7 mg, 63%) as a white solid. ¹H NMR (600 MHz, CD₃OD) δ ppm 8.44 (d, *J =* 5.14 Hz, 1H), 8.16 (br d, *J* = 0.73 Hz, 1H), 8.00 (br dd, J= 8.62, 1.65 Hz, 1H), 7.79 (s, 1 H), 7.69 - 7.73 (m, 1H), 7.42 (br d, *J = 2.57* Hz, 1H), 7.38 (s, 1H), 7.23 (br dd, *J =* 8.80, 2.57 Hz, 1H), 7.16 (br dd, *J =* 5.14, 1.10 Hz, 1H), 7.07 (br d, *J =* 9.17 Hz, 1H), 6.58 (br d, *J =* 0.73 Hz, 1H), 6.30 (s, 1H), 5.80 (s, 2H), 5.25 (s, 2H), 4.11 (q, *J =* 7.34 Hz, 2H), 3.86 (s, 2H), 2.55 - 2.60 (m, 2H), 2.40 - 2.44 (m, 2H), 2.29 (dt, *J =* 9.81, 5.18 Hz, 4H), 1.32 (t, *J =* 7.34 Hz, 3H) *m*/*z* (ESI, +ve ion) = 631.3 [M+H]⁺.

Example 156 was synthesized from 102c and 57i as described in Example 57, Step J. Example 160 was synthesized in similar procedures as described in Example 156.

### Example 157. 2-((4-(3-((2,4-Dichlorophenoxy)methyl)phenoxy)piperidin-1-yl)methyl)-1-((1-ethyl-1H-imidazol-5-yl)methyl)-1H-benzo[d]imidazole-6-carboxylic acid (157)

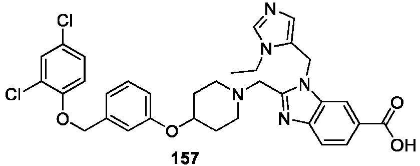

### Step A. Methyl 2-((4-(3-((2,4-dichlorophenoxy)methyl)phenoxy)piperidin-1-yl)methyl)-1-((1-ethyl-1H-imidazol-5-yl)methyl)-1H-benzo[d]imidazole-6-carboxylate (157b)

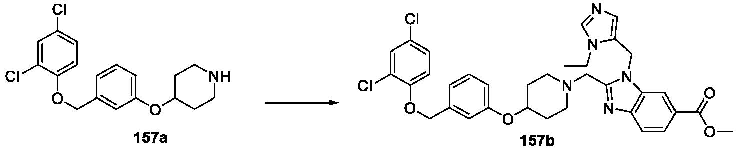

To a stirred solution of 157a (13.6 mg, 0.0386 mmol, prepared as described for the synthesis of 145d) and 145f (14.5 mg, 0.0463 mmol) in 1,2-dichloroethane (0.3 mL) was added HOAc (7.0 mg, 0.116 mmol) at room temperature, followed by addition of sodium triacetoxyborohydride (12.2 mg, 0.058 mmol). The mixture was stirred for 1 h at the same temperature. The reaction was quenched by addition of saturated sodium bicarbonate, and extracted with DCM (x3). The combined organic layer was washed with brine, dried over Na₂SO₄, filtered and concentrated under reduced pressure. The crude residue was purified by silica gel column chromatography (gradient elution, 1-8% MeOH in DCM) to afford the title product **(157b)** (11.5 mg, 46%) as a colorless gum. *m*/*z* (ESI, +ve ion) = 648.3 [M+H]⁺.

### Step B. 2-((4-(3-((2,4-Dichlorophenoxy)methyl)phenoxy)piperidin-1-yl)methyl)-1-((1-ethyl-1H-imidazol-5-yl)methyl)-1H-benzo[d]imidazole-6-carboxylic acid (157)

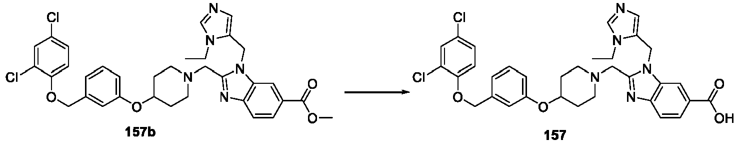

To a stirred solution of **157b** (11.5 mg, 0.0177 mmol) in THF/MeOH/H₂O (2:1:11, 0.4 mL) was added lithium hydroxide monohydrate (2.23 mg, 0.053 mmol) at room temperature. After the mixture was stirred at the same temperature for 2 h, additional lithium hydroxide monohydrate (2.23 mg, 0.053 mmol) was added. After an additional 1.5 h, 1 N HCl was added to the mixture to adjust to pH = 3. The solution was purified by reverse preparative HPLC (gradient elution, 20-60% MeCN in H₂O, with 0.1% TFA as a modifier) to provide the TFA salt of the title compound 157 (11.4 mg, 86%) as a white solid. ¹H NMR (600 MHz, CD₃OD) δ 9.04 (s, 1H), 8.23 (d, *J =* 0.9 Hz, 1H), 8.06 (dd, *J =* 8.4, 1.5 Hz, 1H), 7.83 (d, *J =* 8.4 Hz, 1H), 7.42 (d, *J* = 2.6 Hz, 1H), 7.31 (t, *J =* 7.9 Hz, 1H), 7.23 (dd, *J =* 8.8, 2.6 Hz, 1H), 7.02 - 7.12 (m, 4H), 6.94 (dd, *J =* 8.1, 2.0 Hz, 1H), 5.87 (s, 2H), 5.15 (s, 2H), 4.64 (br s, 1H), 4.54 (br s, 2H), 4.35 (q, *J =* 7.3 Hz, 2H), 3.19 - 3.41 (m, 4H), 2.13 - 2.20 (m, 2H), 1.88 - 2.01 (m, 2H), 1.56 (t, *J =* 7.2 Hz, 3H). *m*/*z* (ESI, +ve ion) = 634.3 [M+H]⁺.

### Example 163. 2-((4-((2-((2,4-Dichlorophenoxy)methyl)pyridin-4-yl)methyl)-4-methylpiperidin-1-yl)methyl)-1-((1-ethyl-1H-imidazol-5-yl)methyl)-1H-benzo[d]imidazole-6-carboxylic acid (163)

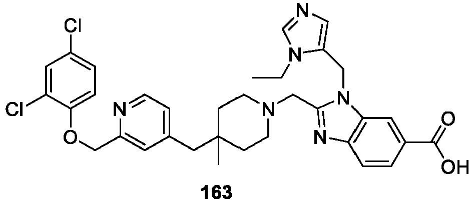

### Step A. tert-Butyl 4-(((1H-imidazole-1-carbonothioyl)oxy)methyl)-4-((2-((2,4-dichlorophenoxy)methyl)pyridin-4-yl)methyl)piperidine-1-carboxylate (163a)

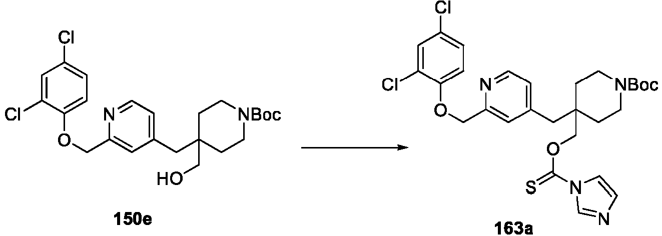

Thiocarbonyldiimidazole (42.6 mg, 0.24 mmol) was added to a solution of 150e (23 mg, 0.048 mmol) in THF (0.24 mL) at room temperature. The resulting solution was heated at 60 °C for 2h. After completion, the reaction was cooled down, diluted with water (1 mL) and EtOAc (1 mL), and was extracted with EtOAc (1 mL x 3). The combined organic layer was washed with brine (1 mL), dried over Na₂SO₄, filtered, and concentrated to afford the crude title product (163a) (28 mg) as a yellow solid, which was used in the next step without further purification. *m*/*z* (ESI, +ve ion) = 591.3 [M+H]⁺

### Step B. tert-Butyl 4-((2-((2,4-dichlorophenoxy)methyl)pyridin-4-yl)methyl)-4-methylpiperidine-1-carboxylate (163b)

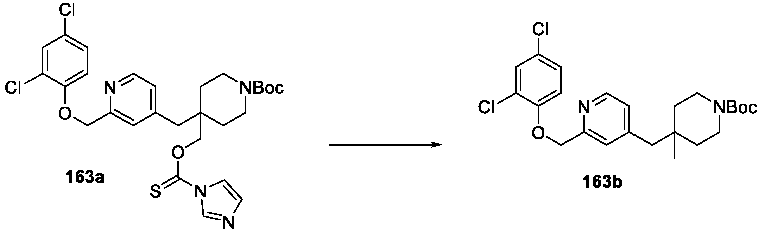

AIBN (2.33 mg, 0.0142 mmol) and tributyltin hydride (0.064 mL, 0.24 mmol) were added to a solution of **163a** (0.028 g, 0.047 mmol) in toluene (2.4 mL) under Ar at room temperature. The resulting solution was heated at 100 °C overnight. After completion, the reaction solvent was removed under reduced pressure to provide a crude title product (163b) (7.5 mg), which was used in the next step without further purification. *m*/*z* (ESI, +ve ion) = 465.3 [M+H]⁺

### Step C. 2-((4-((2-((2,4-Dichlorophenoxy)methyl)pyridin-4-yl)methyl)-4-methylpiperidin-1-yl)methyl)-1-((1-ethyl-1H-imidazol-5-yl)methyl)-1H-benzo[d]imidazole-6-carboxylic acid (163)

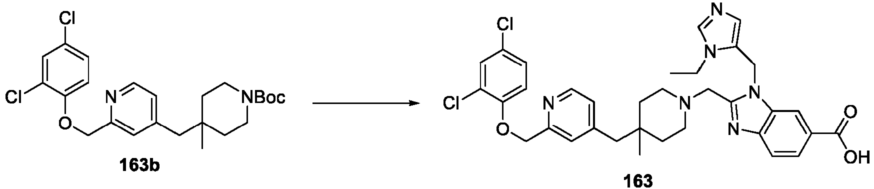

The title product (163) was synthesized in similar procedures as described in Example 150, Steps F-H. ¹H NMR (600 MHz, CD₃OD) δ ppm 8.42 (d, *J =* 5.1 Hz, 1H), 8.13 (d, *J* = 0.7 Hz, 1H), 8.00 (dd, *J* = 8.4, 1.5 Hz, 1H), 7.81 (s, 1H), 7.72 (d, *J* = 8.4 Hz, 1H), 7.36 - 7.38 (m, 2H), 7.20 (dd, *J =* 8.8, 2.6 Hz, 1H), 7.15 (dd, *J =* 5.1, 1.5 Hz, 1H), 7.06 (d, *J =* 8.8 Hz, 1H), 6.57 (s, 1H), 5.73 (s, 2H), 5.26 (s, 2H), 4.05 (q, *J=* 7.3 Hz, 2H), 3.88 (s, 2H), 2.54 - 2.66 (m, 4H), 2.35 - 2.42 (m, 2H), 1.24 - 1.35 (m, 5H), 1.16 - 1.22 (m, 2H), 0.83 (s, 3H). *m*/*z* (ESI, +ve ion) = 647.3 [M+H]⁺

### Example 164. 2-((4-((2-((2,4-Dichlorophenoxy)methyl)pyridin-4-yl)methyl)-4-(fluoromethyl)piperidin-1-yl)methyl)-1-((1-ethyl-1H-imidazol-5-yl)methyl)-1H-benzo[d]imidazole-6-carboxylic acid (164)

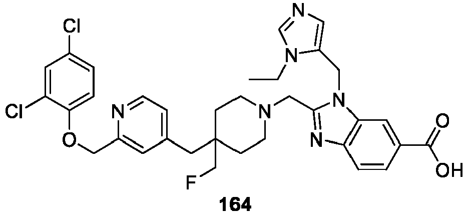

### Step A. tert-Butyl 4-((2-((2,4-dichlorophenoxy)methyl)pyridin-4-yl)methyl)-4-(fluoromethyl)piperidine-1-carboxylate (164a)

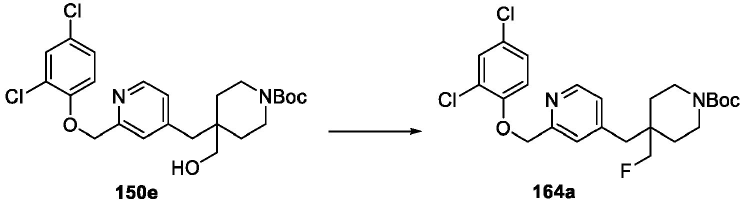

Deoxo-fluor (0.104 mL, 0.254 mmol) was added dropwise to a solution of 150e (0.102 g, 0.212 mmol) in DCM (1.1 mL) at room temperature. Then the reaction was heated to 45 °C overnight. After completion, the reaction was quenched with sat. NaHCO₃ solution (5 mL) and extracted with EtOAc (5 mL x 3). The combined organic layer was washed with brine (5 mL), dried over Na₂SO₄, filtered, and concentrated to give a crude residue, which was purified by silica gel column chromatography (gradient elution, 40-80% EtOAc in hexanes) to afford the title product (164a) (45.0 mg, 44%) as a yellow oil. *m*/*z* (ESI, +ve ion) = 483.3 [M+H]⁺

### Step B. 2-((4-((2-((2,4-Dichlorophenoxy)methyl)pyridin-4-yl)methyl)-4-(fluoromethyl)piperidin-1-yl)methyl)-1-((1-ethyl-1H-imidazol-5-yl)methyl)-1H-benzo[d]imidazole-6-carboxylic acid (164)

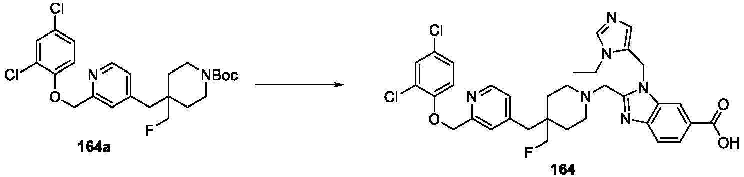

The title product (164) was synthesized in similar procedures from 164a as described in Example 150, Steps F-H. *m*/*z* (ESI, +ve ion) = 665.3 [M+H]⁺.

### Example 178. (S)-2-((4-((4-((2,4-Dichlorophenoxy)methyl)pyridin-2-yl)oxy)piperidin-1-yl)methyl)-1-(oxetan-2-ylmethyl)-1H-benzo[d]imidazole-6-carboxylic acid (178)

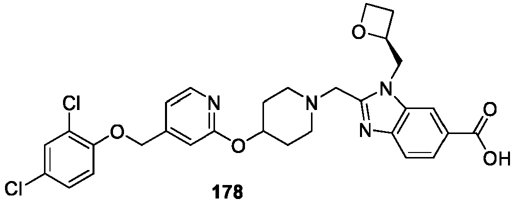

### Step A. Methyl 4-((1-(tert-butoxycarbonyl)piperidin-4-yl)oxy)picolinate (178a)

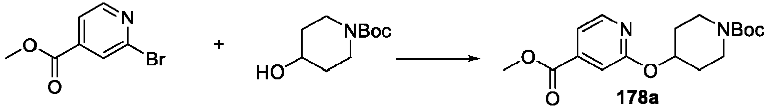

A solution of Pd₂(dba)₃ (46 mg, 0.05 mmol) and BINAP (62 mg, 0.1 mmol) in toluene (2 mL) was heated at 120 °C for 5 min before transferred into a mixture of methyl 2-bromopyridine-4-carboxylate (216 mg, 1.0 mmol), *tert-butyl* 4-hydroxypiperidine-1-carboxylate (241 mg, 1.2 mmol) and Cs₂CO₃ (652 mg, 2.0 mmol) in 18 mL of toluene. The mixture was degassed with argon and stirred at 120 °C for 16 h under argon. After completion, the solvent was removed *in vacuo* and the residue was purified by silica gel column chromatography (EtOAc in hexanes) to afford the title product **(178a)** with impurities as a colorless oil, which was used in the next step without further purification. *m*/*z* (ESI, +ve ion) = 337.3 [M+H]⁺.

### Step B. tert-Butyl 4-((2-(hydroxymethyl)pyridin-4-yl)oxy)piperidine-1-carboxylate (178b)

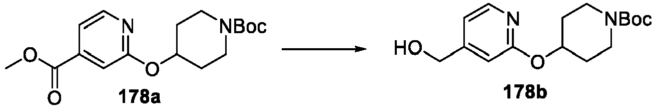

To a mixture of **178a** (77 mg, 0.23 mmol), CaCl₂ (51 mg, 0.46 mmol) and NaBH₄ (35 mg, 0.91 mmol) was added THF (10 mL). The resulting suspension was stirred at 60 °C overnight. After completion, the reaction was quenched with water (10 mL), extracted with ethyl acetate (10 mL x 3). The combined organic phase was washed with brine, dried with anhydrous Na₂SO₄, filtered, and concentrated to give a crude residue which was purified by silica gel column chromatography (EtOAc in hexanes) to afford the title product (178b) (35 mg, 23% for 2 steps) as a colorless oil. *m*/*z* (ESI, +ve ion) = 309.3 [M+H]⁺.

### Step C. tert-Butyl 4-((2-((2,4-dichlorophenoxy)methyl)pyridin-4-yl)oxy)piperidine-1-carboxylate (178c)

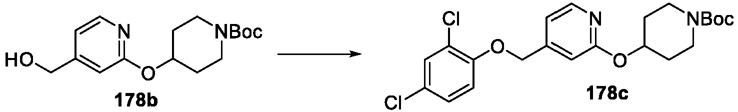

A flask was charged with 2,4-dichlorophenol (27 mg, 0.17 mmol), 178b (17 mg, 0.06 mmol), and PPh₃ (44 mg, 0.17 mmol). Anhydrous THF (5 mL) was added and the reaction was cooled down in an ice bath. Diethyl azodicarboxylate (34 mg, 0.17 mmol) was added dropwise and the resulting mixture was stirred at 0 °C for 0.5 h, then at room temperature overnight. After completion, the reaction was quenched with water (5 mL), and extracted with ethyl acetate (5 mL x 3). The combined organic phase was washed with brine, dried with anhydrous Na₂SO₄, filtered, and concentrated to give a crude residue, which was purified by silica gel column chromatography (EtOAc in hexanes) to afford the title product (178c) as a colorless oil with some impurities, which was used in the next step without further purification. *m*/*z* (ESI, +ve ion) = 453.3 [M+H]⁺.

### Step D. 2-((2,4-Dichlorophenoxy)methyl)-4-(piperidin-4-yloxy)pyridine (178d)

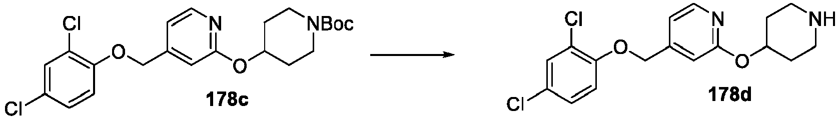

A solution of 178c (15 mg, impure) in DCM (2 mL) was treated with TFA (1 mL) at room temperature for 10 min. After completion, the solvent was removed *in vacuo,* and the resulting crude was purified by reverse phase HPLC (gradient elution, 0-75% CH₃CN in water, with 0.1% TFA as a modifier) to provide the title product (178d) (17 mg, 62% for 2 steps) as a white solid. *m*/*z* (ESI, +ve ion) = 353.1 [M+H]⁺.

### Step E. Methyl (S)-2-((4-((2-((2,4-dichlorophenoxy)methyl)pyridin-4-yl)oxy)piperidin-1-yl)methyl)-1-(oxetan-2-ylmethyl)-1H-benzo[d]imidazole-6-carboxylate (178e)

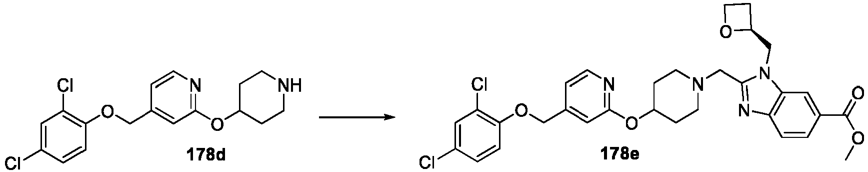

A solution of 178d (4.6 mg, 0.01 mmol), **1h** (3.2 mg, 0.011 mmol) and DIPEA (25.5 mg, 0.2 mmol) in DMF (1 mL) was stirred at room temperature for 22 h. After completion, the reaction was purified by reverse phase HPLC (gradient elution, 20-100% CH₃CN in water with 0.1% TFA as a modifier) to provide the title product (178e) (5.6 mg, impure) as a white solid, which was used in the next step without further purification. m/z (ESI, +ve ion) = 611.3 [M+H]⁺.

### Step F. (S)-2-((4-((2-((2,4-Dichlorophenoxy)methyl)pyridin-4-yl)oxy)piperidin-1-yl)methyl)-1-(oxetan-2-ylmethyl)-1H-benzo[d]imidazole-6-carboxylic acid (178)

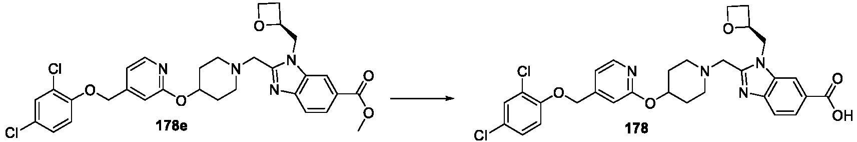

A solution of **178e** (5.6 mg, 0.008 mmol, impure) in CH₃CN (0.5 mL) was treated with 2 N NaOH (0.5 mL). The resulting mixture was stirred at room temperature for 26 h. After completion, the reaction was purified by reverse phase HPLC (gradient elution, 0-90% CH₃CN in water, with 0.1% formic acid as a modifier) to provide the title product (178) (3.5 mg, 59% for 2 steps) as a white solid. ¹H NMR (600 MHz, CD₃OD) δ ppm 8.32 - 8.37 (m, 1H), 8.10 (d, *J=* 5.13 Hz, 1H), 7.97 (br dd, *J =* 8.44, 1.47 Hz, 1H), 7.68 (d, *J =* 8.44 Hz, 1H), 7.46 (d, *J=* 2.57 Hz, 1H), 7.26 (br dd, *J =* 8.80, 2.57 Hz, 1H), 7.08 (d, *J =* 8.80 Hz, 1H), 6.98 - 7.03 (m, 1H), 6.88 (s, 1H), 5.26 (qd, *J =* 7.34, 2.57 Hz, 1H), 5.18 (s, 2H), 5.09 (m, 1H), 4.85 - 4.90 (m, 1H), 4.72 (br dd, *J=* 15.59, 2.75 Hz, 1H), 4.65 (td, *J* = 7.89, 5.87 Hz, 1H), 4.47 (dt, *J* = 9.17, 6.05 Hz, 1H), 4.10 (br d, *J =* 13.94 Hz, 1H), 4.00 (br d, *J=* 13.94 Hz, 1H), 2.91 - 2.96 (m, 1H), 2.88 (br d, *J =* 3.67 Hz, 1H), 2.81 (m, 1H), 2.49 - 2.61 (m, 3H), 2.05 - 2.11 (m, 2H), 1.80 - 1.90 (m, 2H). *m*/*z* (ESI, +ve ion) = 597.3 [M+H]⁺.

Example 179 was synthesized in similar procedures as described in Example 178.

### Example 186. 2-(((S)-4-(2-((2,4-Dichlorophenoxy)methyl)oxazole-5-carbonyl)-2-methylpiperazin-1-yl)methyl)-1-(((S)-oxetan-2-yl)methyl)-1H-benzo[d]imidazole-6-carboxylic acid (186)

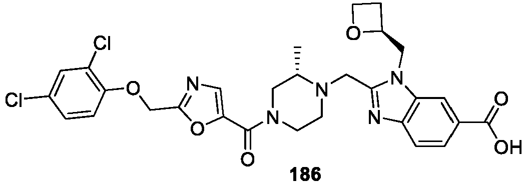

### Step A. tert-Butyl (S)-4-(2-((2,4-dichlorophenoxy)methyl)oxazole-5-carbonyl)-2-methylpiperazine-1-carboxylate (186a)

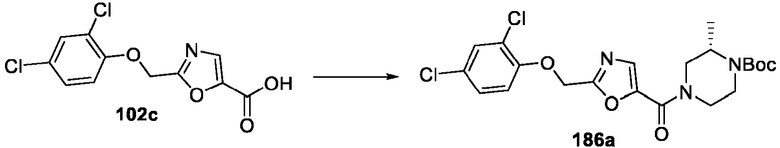

To a solution of 102c (49 mg, 0.17 mmol), *tert*-butyl (2*S*)-2-methylpiperazin-1-yl formate (34 mg, 0.17 mmol) and T₃P (178 mg, 0.26 mmol) in DMF (2 ml) was added triethylamine (51mg, 0.51 mmol). The mixture was stirred at 15 °C for 16 h, diluted with EtOAc (20 ml) and washed with brine (20 mL x 3). The organic layer was dried, filtered and evaporated to dryness to afford the title product (186a) (80 mg) as a colorless oil, which was used in the next step without further purification. *m*/*z* (ESI, +ve ion) = 470.1 [M+H]⁺.

### Step B. (S)-(2-((2,4-Dichlorophenoxy)methyl)oxazol-5-yl)(3-methylpiperazin-1-yl)methanone (186b)

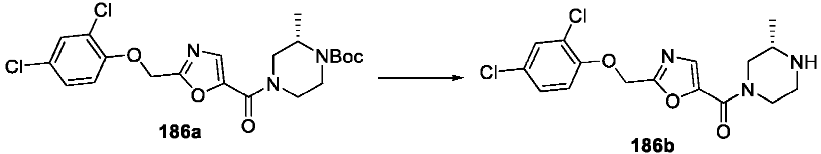

To a solution of 186a (100 mg, 0.21 mmol) in DCM (5 ml) was added TFA (1 mL). The reaction was stirred at 20 °C for 2 h and concentrated. The crude was redissolved in DCM and washed with sat. NaHCO₃ (5 mL x 2). The DCM layer was dried, filtered, and concentrated to afford the title product (186b) (78 mg) as a colorless oil, which was used in the next step without further purification. *m*/*z* (ESI, +ve ion) = 370.1 [M+H]⁺.

### Step C. (5)-2-(Chloromethyl)-1-(oxetan-2-ylmethyl)-1H-benzo[d]imidazole-6-carboxylic acid (186c)

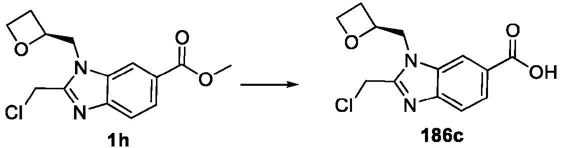

To a solution of **1h** (390 mg, 1.33 mmol) in 2 mL of THF/H₂O (1:1) was added LiOH (64 mg, 2.66 mmol). The mixture was stirred at 25 °C for 2 h. After completion, the mixture was adjusted to pH = 5-6 with 1 N HCl and extracted with EtOAc (20 mL x 3). The combined organic layer was dried, filtered and concentrated. The crude residue was purified by Prep-TLC (10% MeOH in DCM) to provide the title product (186c) (290 mg, 74%) as a white solid. *m*/*z* (ESI, +ve ion) = 281.1 [M+H]⁺.

### Step D. 2-(((S)-4-(2-((2,4-Dichlorophenoxy)methyl)oxazole-5-carbonyl)-2-methylpiperazin-1-yl)methyl)-1-(((S)-oxetan-2-yl)methyl)-1H-benzo[d]imidazole-6-carboxylic acid (186)

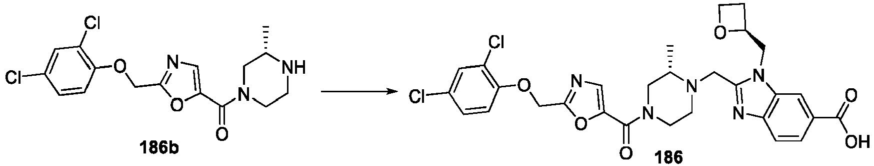

To a solution of 186b (78 mg, 0.21 mmol) and 186c (61 mg, 0.22 mmol) in DMF (3 mL) was added DIPEA (85 mg, 0.66 mmol). The mixture was stirred at 50 °C for 16 h. The mixture was purified by reversed phase HPLC (45 % CH₃CN in H2O, with 0.05 %TFA as a modifier) to afford the TFA salt of the title product (186) (6.5 mg, 4.2%) as a white solid. ¹H NMR (400 MHz, CD₃OD) δ 8.49 (s, 1H), 8.17 (dd, *J =* 8.6, 1.3 Hz, 1H), 7.83 (d, *J =* 8.6 Hz, 1H), 7.70 (s, 1H), 7.42 (s, 1H), 7.26 (dt, *J =* 22.0, 5.6 Hz, 2H), 5.36 (s, 2H), 5.31 - 5.17 (m, 1H), 4.77 - 4.85 (m, 2H), 4.61 - 4.70 (m, 2H), 4.45 (dt, *J* = 9.2*,* 5.9 Hz, 1H), 4.22 (d, *J =* 16.4 Hz, 1H), 3.98 - 4.17 (m, 2H), 3.65 (br s, 1H), 3.48 (br s, 1H), 2.91 - 3.16 (m, 2H), 2.77 - 2.90 (m, 1H), 2.65 - 2.81 (m, 1H), 2.52 (ddd, *J =* 16.4, 11.5, 7.3 Hz, 1H), 1.20 (s, 3H). *m*/*z* (ESI, +ve ion) = 614.1 [M+H]⁺.

### Example 189. 2-((4-Cyano-4-((2-((2,4-dichlorophenoxy)methyl)pyridin-4-yl)methyl)piperidin-1-yl)methyl)-1-((1-ethyl-1H-imidazol-5-yl)methyl)-1H-benzo[d]imidazole-6-carboxylic acid (189)

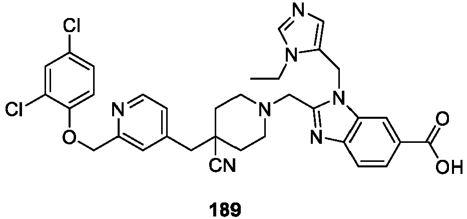

### Step A. tert-Butyl 4-cyano-4-((2-((2,4-dichlorophenoxy)methyl)pyridin-4-yl)methyl)piperidine-1-carboxylate (189a)

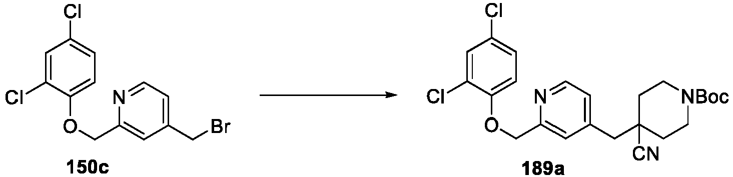

To a solution of diisopropylamine (0.20 mL, 1.39 mmol) in THF (2 mL) at -78 °C, n-butyllithium in hexanes (0.61 mL, 1.52 mmol) was added dropwise. The resulting solution was warmed to room temperature and stirred for 30 min before cooling to -78 °C again. A solution of *tert-butyl* 4-cyanopiperidine-1-carboxylate (0.21 g, 0.99 mmol) in THF (1.5 mL) was added dropwise. The reaction was stirred for 30 min, followed by the careful addition of **150c** (0.23 g, 0.66 mmol). The reaction was allowed to slowly warm to room temperature and stirred overnight. Saturated NH₄Cl solution (10 mL) was added and the reaction was extracted with EtOAc (10 mL x 3). The combined organic layer was washed with brine (10 mL), dried over Na₂SO₄, concentrated and purified by silica gel column chromatography (gradient elution, 60-100% EtOAc in hexanes) to afford the title product **(150d)** (0.027 g, 9%) as a colorless oil. *m*/*z* (ESI, +ve ion) = 476.3 [M+H]⁺.

### Step B. 4-((2-((2,4-Dichlorophenoxy)methyl)pyridin-4-yl)methyl)piperidine-4-carbonitrile (189b)

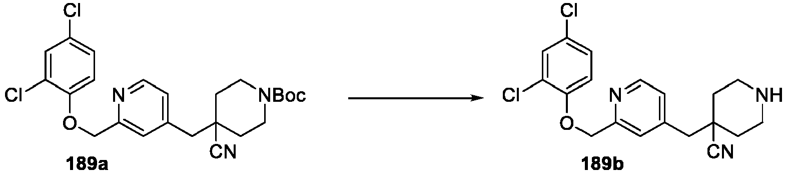

A solution of 189a (9.3 mg, 0.020 mmol) in DCM (1 mL) and TFA (0.5 mL) was stirred at room temperature for 10 min. After completion, the solvent was removed under reduced pressure. The resulting residue was diluted with EtOAc (3 mL) and washed with sat. NaHCO₃ (5 mL), brine (3 mL), dried with Na₂SO₄, filtered, and concentrated to afford the crude title product (189b) (6.8 mg) as a yellow oil, which was used in the next step without further purification. *m*/*z* (ESI, +ve ion) = 376.3 [M+H]⁺ .

### Step C. 2-((4-Cyano-4-((2-((2,4-dichlorophenoxy)methyl)pyridin-4-yl)methyl)piperidin-1-yl)methyl)-1-((1-ethyl-1H-imidazol-5-yl)methyl)-1H-benzo[d]imidazole-6-carboxylic acid (189)

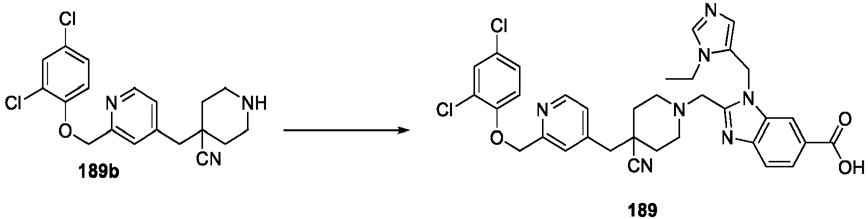

The title product (189) was prepared in similar procedures as described in Example 150, Steps G and H. ¹H NMR (600 MHz, CD₃OD) δ ppm 9.06 (d, *J =* 1.5 Hz, 1H), 8.56 (d, *J* = 5.1 Hz, 1H), 8.22 (d, *J =* 1.1 Hz, 1H), 8.06 (dd, *J =* 8.6, 1.7 Hz,1H), 7.80 (d, *J =* 8.1 Hz, 1H), 7.63 (s, 1H), 7.42 (d, *J =* 2.6 Hz, 1H), 7.41 (dd, *J* = 5.3, 1.7 Hz, 1H), 7.25 (dd, *J* = 8.8, 2.6 Hz, 1H), 7.11 (d, *J* = 8.8 Hz, 1H), 7.10 (d, *J =* 1.5 Hz, 1H), 5.90 (s, 2H), 5.30 (s, 2H), 4.34 (q, *J =* 7.3 Hz, 2H), 4.17 (s, 2H), 3.13 (br d, *J =* 11.7 Hz, 2H), 3.03 (s, 2H), 2.64 (t, *J =* 11.9 Hz, 2H), 1.88 (br d, *J =* 12.8 Hz, 2H), 1.67 (dd, *J =* 1.0, 1.0 Hz, 2H), 1.53 (t, *J =* 7.3 Hz, 3H). *m*/*z* (ESI, +ve ion) = 658.3 [M+H]⁺.

### Example 198. (S)-2-((4-((6-((2,4-Dichlorophenoxy)methyl)pyridin-2-yl)oxy)piperidin-1-yl)methyl)-1-(oxetan-2-ylmethyl)-1H-benzo[d]imidazole-6-carboxylic acid (198)

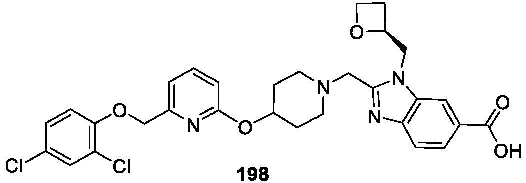

### Step A. 2-Bromo-6-((2,4-dichlorophenoxy)methyl)pyridine (198a)

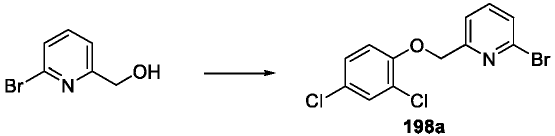

To a solution of (6-bromopyridin-2-yl)methanol (1.57 g, 8.35 mmol) in anhydrous THF (16.7 mL) at 0 °C was added DIAD (2.53 g, 12.5 mmol), PPh₃ (3.29 g, 12.5 mmol) and 2,4-dichlorophenol (1.36 g, 8.35 mmol). The resulting mixture was stirred at room temperature for 1.5 h and sat. NaHCO₃ (10 mL) was added. The mixture was extracted with EtOAc (10 mL x 3), washed with brine (10 mL), dried over Na₂SO₄, filtered, concentrated and the crude residue was purified by silica gel column chromatography (gradient elution, 0-20% EtOAc in hexanes) to provide the title product **(198a)** (738 mg, 27%). *m*/*z* (ESI, +ve ion) = 334.0 [M + H]⁺.

### Step B. tert-Butyl 4-((6-((2,4-dichlorophenoxy)methyl)pyridin-2-yl)oxy)piperidine-1-carboxylate (198b)

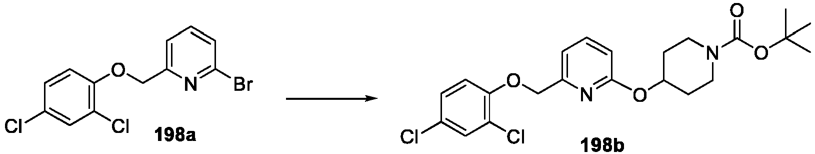

To a solution of *tert-butyl* 4-hydroxypiperidine-1-carboxylate (44 mg, 0.22 mmol) in anhydrous DMF (1.1 mL) was added NaH (13 mg, 0.33 mmol, 60% dispersion in mineral oil) at 0 °C. After stirring for 10 min, the reaction was heated to 50 °C and a solution of 198a (80 mg, 0.24 mmol) in DMF (0.6 mL) was added dropwise. After stirring for 8 h, the reaction mixture was cooled down to room temperature and quenched with H₂O (1 mL). The crude mixture was extracted with EtOAc (3 mL x 2), washed with brine (3 mL), dried over Na₂SO₄, filtered, concentrated and the crude residue was purified by silica gel column chromatography (gradient elution, 0-50% EtOAc in hexanes) to afford the title product (198b) (39 mg, 40%). *m*/*z* (ESI, +ve ion) = 453.3 [M + H]⁺.

### Step C. 2-((2,4-Dichlorophenoxy)methyl)-6-(piperidin-4-yloxy)pyridine (198c)

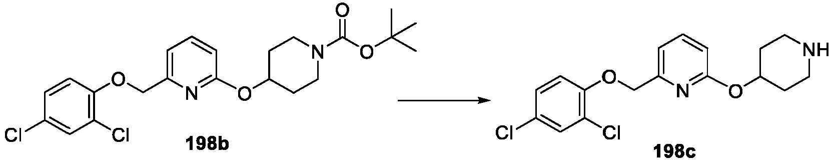

To a solution of 198b (37 mg, 0.082 mmol) in anhydrous CH₂Cl₂ (0.8 mL) was added TFA (0.3 mL, 4.1 mmol) at room temperature. After stirring for 1 h, the reaction was concentrated and sat. NaHCO₃ (0.5 mL) was added to neutralize the TFA. The mixture was extracted with EtOAc (2 mL x 3) and the comined organic layer was dried over Na₂SO₄, filtered, and concentrated to afford the crude title product (198c) (28 mg), which was used in the next step without further purification. *m*/*z* (ESI, +ve ion) = 353.3 [M + H]⁺.

### Step D. Methyl (S)-2-((4-((6-((2,4-dichlorophenoxy)methyl)pyridin-2-yl)oxy)piperidin-1-yl)methyl)-1-(oxetan-2-ylmethyl)-1H-benzo[d]imidazole-6-carboxylate (198d)

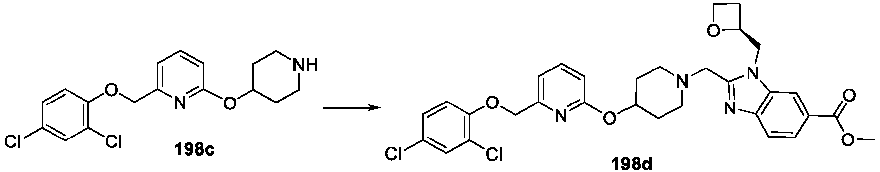

To a solution of crude 198c (8 mg, 0.023 mmol) in dichloroethane (0.2 mL) was added **1h** (8 mg, 0.027 mmol) and DIPEA (0.01 mL, 8.8 mg, 0.068 mmol) at room temperature. The reaction was heated to 60 °C. After 20 h, the reaction was cooled down to room temperature and concentrated to provide the crude title product (198d), which was used in the next step without further purification. *m*/*z* (ESI, +ve ion) = 611.3 [M + H]⁺.

### Step E. (S)-2-((4-((6-((2,4-Dichlorophenoxy)methyl)pyridin-2-yl)oxy)piperidin-1-yl)methyl)-1-(oxetan-2-ylmethyl)-1H-benzo[d]imidazole-6-carboxylic acid (198)

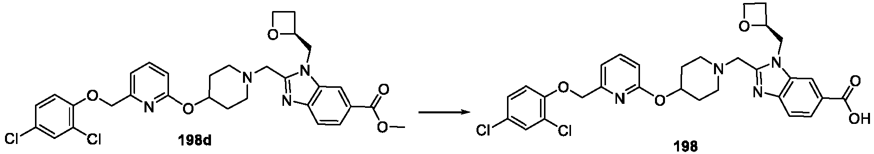

The crude **198d** was dissolved in THF/H₂O (1:1, 0.4 mL) at room temperature. LiOH.H₂O (5 mg, 0.11 mmol) and a few drops of MeOH were added to the mixture and heated to 50 °C. After stirring for 1 h, the reaction was acidified with 1 N HCl to adjust to pH = 5. The mixture was concentrated and purified by reverse phase HPLC (gradient elution, 0-70% CH₃CN in water with 0.1% TFA as a modifier) to provide the title product (198) (13.8 mg, 86% over 3 steps) as a white solid. *m*/*z* (ESI, +ve ion) = 597.3 [M+H]⁺. ¹H NMR (600 MHz, CD₃OD) δ ppm 8.35 (s, 1H), 8.05 (dd, *J=* 8.62, 1.28 Hz, 1H), 7.81 (d, *J =* 8.44 Hz, 1H), 7.74 (t, *J* = 7.89 Hz, 1H), 7.44 (d, *J* = 2.57 Hz, 1H), 7.25 (dd, *J =* 8.99, 2.38 Hz, 1H), 7.17 (d, *J =* 6.97 Hz, 1H), 7.11 (d, *J =* 8.80 Hz, 1H), 6.79 (br d, *J =* 8.07 Hz, 1H), 5.36 (br s, 1H), 5.22 (ddd, *J =* 14.49, 7.15, 2.20 Hz, 1H), 5.17 (s, 2H), 4.87 (s, 2H), 4.63 - 4.71 (m, 3H), 4.43 (dt, *J=* 9.17, 5.87 Hz, 1H), 3.57 - 3.78 (m, 4H), 2.77 - 2.84 (m, 1H), 2.50 (ddd, *J =* 16.69, 11.55, 7.34 Hz, 1H), 2.16 - 2.36 (m, 4H).

Examples **199, 214, 246, 247, and 264** were synthesized in similar procedures as described in Example 198.

Examples **248, 249** were synthesized in similar procedures as described in Example 198, with NH₄OH used as the modifier for the last step purification.

Example 200 was synthesized in similar procedures as described in Example 114.

Examples **202 and 203** (stereochemistry arbitrarily assigned) were synthesized from (3-bromophenyl)methanol and 2,4-dichlorophenol in similar procedures as described in Example 131, Step A, then Example 152, step B to step C, then chiral separation by UPCC (Daicel CHIRALPAK AD_3, 3*150mm, CO₂/MeOH (0.1%DEA) = 50/50), followed by Example 152, Step D.

### Examples 204 and 205. (R)-2-((4-((3-((2,4-dichlorophenoxy)methyl)phenyl)(methoxy)methyl)piperidin-1-yl)methyl)-1-((1-ethyl-1H-imidazol-5-yl)methyl)-1H-benzo[d]imidazole-6-carboxylic acid (204) and (S)-2-((4-((3-((2,4-dichlorophenoxy)methyl)phenyl)(methoxy)methyl)piperidin-1-yl)methyl)-1-((1-ethyl-1H-imidazol-5-yl)methyl)-1H-benzo[d]imidazole-6-carboxylic acid (205)

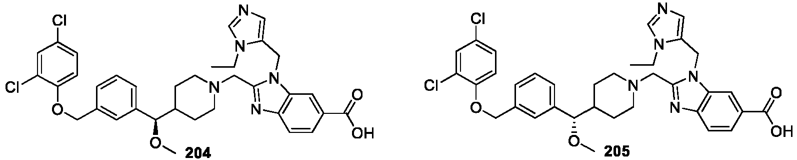

### Step A. tert-Butyl 4-((3-((2,4-dichlorophenoxy)methyl)phenyl)(methoxy)methyl)piperidine-1-carboxylate (204a)

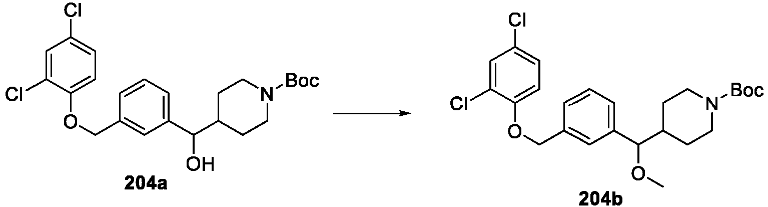

To a mixture of NaH (86 mg, 2.1 mmol, 60% dispersion in mineral oil) in THF (15 mL) was added a THF (20 mL) solution of **204a** (made by following similar procedures as **130b)** (500 mg, 1.1 mmol). The reaction was stirred at 0 °C for 30 min. Then CH₃I (759 mg, 5.3 mmol) was added. The reaction was allowed to warm to room temperature and stirred for 48 h under N₂. After completion, the reaction was quenched with H₂O (20 mL), extracted with EtOAc (20 mL x 3). The combined organic layer was washed with H₂O (10 mL), brine (10 mL), dried over anhydrous Na₂SO₄, filtered and concentrated to give a crude residue, which was purified by silica gel column chromatography (petroleum ether:EtOAc = 10:1) to afford the title product **(204b)** (400 mg, 74%) as a yellow oil. *m*/*z* (ESI, +ve ion) = 502.2 [M+Na]⁺.

### Step B. tert-Butyl (R)-4-((3-((2,4-dichlorophenoxy)methyl)phenyl)(methoxy)methyl)piperidine-1-carboxylate (204b-P1) and tert-butyl (R)-4-((3-((2,4-dichlorophenoxy)methyl)phenyl)(methoxy)methyl)piperidine-1-carboxylate (204b-P2)

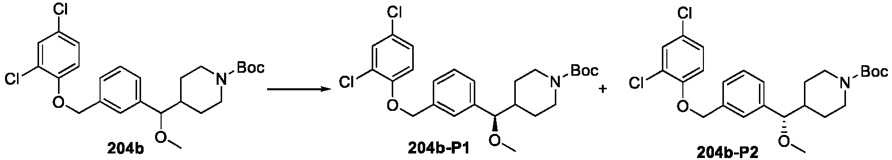

The title products **(204b-P1** and **204b-P2)** were obtained through chiral separation of **204b** by UPCC (Daicel CHIRALPAK IA 4.6*250mm, CO₂/MeOH (0.1% DEA) = 80:20). **204b-P1:** 130 mg, RT=1.186 min, white solid; **204b-P2:** 140 mg, RT=1.441min, white solid. The stereochemistry is arbitrarily assigned.

### Step C. (R)-2-((4-((3-((2,4-Dichlorophenoxy)methyl)phenyl)(methoxy)methyl)piperidin-1-yl)methyl)-1-((1-ethyl-1H-imidazol-5-yl)methyl)-1H-benzo[d]imidazole-6-carboxylic acid (204) and (S)-2-((4-((3-((2,4-dichlorophenoxy)methyl)phenyl)(methoxy)methyl)piperidin-1-yl)methyl)-1-((1-ethyl-1H-imidazol-5-yl)methyl)-1H-benzo[d]imidazole-6-carboxylic acid (205)

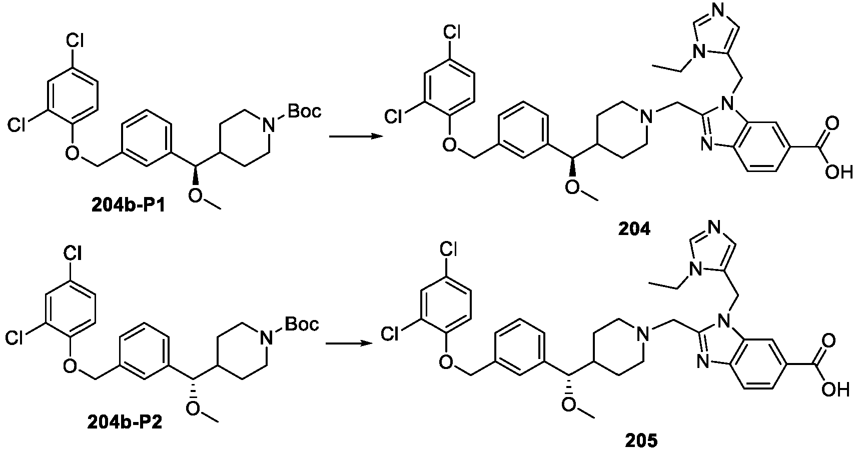

The title compound **204** was synthesized in similar procedures from **204b-P1** as described in Example **152,** Step B to D. *m*/*z* (ESI, +ve ion) = 662.3 [M+H]⁺. ¹H NMR (400 MHz, CD₃OD) δ ppm 8.89 - 8.97 (m, 2H), 8.23 (s, 1H), 8.05 (dd, *J* = 8.5, 1.5 Hz, 1H), 7.83 (d, *J* = 8.3 Hz, 1H), 7.37 - 7.46 (m, 3H), 7.20 - 7.29 (m, 2H), 7.12 (d, *J* = 8.7 Hz, 1H), 6.99 (br s, 1H), 5.82 (s, 2H), 5.21 (s, 2H), 4.45 - 4.65 (m, 2H), 4.30 (q, *J* = 7.3 Hz, 2H), 4.02 (br d, *J* = 7.0 Hz, 1H), 3.50 - 3.74 (m, 2H), 3.20 (s, 3H), 2.78 - 3.05 (m, 2H), 2.10 (br s, 1H), 1.88 (br s, 1H), 1.45 - 1.67 (m, 6H). *m*/*z* (ESI, +ve ion) = 662.3.

The title compound **205** was synthesized in similar procedures from **204b-P2** as described in Example **152,** Step B to D. *m*/*z* (ESI, +ve ion) = 662.3 [M+H]⁺. ¹H NMR (400 MHz, CD₃OD) δ ppm 8.12 (s, 1H), 7.97 (dd, *J* = 8.5, 1.1 Hz, 1H), 7.74 (s, 1H), 7.64 (d, *J* = 8.7 Hz, 1H), 7.41 (d, *J* = 2.2 Hz, 1H), 7.18 - 7.29 (m, 2H), 7.08 (d, *J* = 8.7 Hz, 1H), 6.82 - 6.88 (m, 2H), 6.85 (dd, *J* = 8.2 Hz, 1H), 6.52 (s, 1H), 5.76 (s, 2H), 5.14 (s, 2H), 4.31 - 4.39 (m, 1H), 4.09 (q, *J* = 7.4 Hz, 2H), 4.83 (s, 2H), 3.30 (s, 3H), 2.66 - 2.78 (m, 2H), 2.35 (br t, *J* = 8.9 Hz, 2H), 1.81 - 1.93 (m, 2H), 1.52 - 1.77 (m, 2H), 1.22 - 1.38 (m, 4H).

### Example 206. (S)-2-((4-(3-((2-Cyano-4-fluorophenoxy)methyl)phenoxy)piperidin-1-yl)methyl)-1-(oxetan-2-ylmethyl)-1H-benzo[d]imidazole-6-carboxylic acid (206)

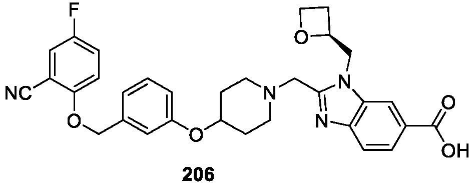

### Step A. tert-Butyl 4-(3-(methoxycarbonyl)phenoxy)piperidine-1-carboxylate (206a)

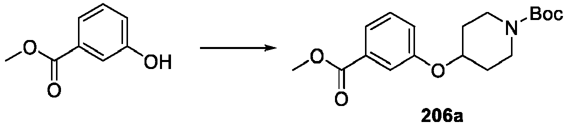

A mixture of methyl 3-hydroxybenzoate (4.88 g, 32.1 mmol), *tert-*butyl (4-hydroxypiperidin-1-yl) formate (6.5 g, 32.1 mmol), PPh₃ (12.63 g, 48.1 mmol) in THF (30 mL) was added DEAD (8.39 g, 48.1 mmol) at 0 °C under N₂. The mixture was stirred at 20 °C for 18 h and concentrated *in vacuo.* The crude residue was purified by silica gel column chromatography (petroleum ether:EtOAc = 10:1) to afford the title product **(206a)** (4.3 g) with some impurity as a white solid. *m*/*z* (ESI, +ve ion) = 358.1 [M+Na]⁺.

### Step B. tert-Butyl 4-(3-(hydroxymethyl)phenoxy)piperidine-1-carboxylate (206b)

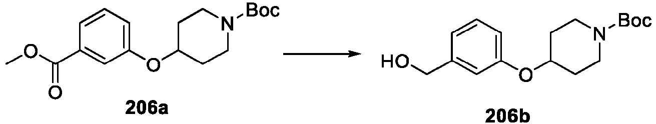

A solution of **206a** (4.3 g, 12.8 mmol) in THF (40 mL) was cooled down to 0 °C. Lithium aluminum hydride (15.4 mL, 15.3 mmol, 1 N in THF) was added and the reaction was stirred at 0 °C for 1.5 h. The reaction was quenched with Na₂SO₄.10H₂0 (15 g) at 0 °C. After stirring at the same temperature for 20 min, the mixture was filtered and concentrated *in vacuo* to give a crude residue, which was purified by silica gel column chromatography (petroleum ether:EtOAc = 3:1) to provide the title product **(206b)** (3.2 g, 77%) as a colorless oil. *m*/*z* (ESI, +ve ion) = 330.1 [M+Na]⁺.

### Step C. tert-Butyl 4-(3-((2-cyano-4-fluorophenoxy)methyl)phenoxy)piperidine-1-carboxylate (206c)

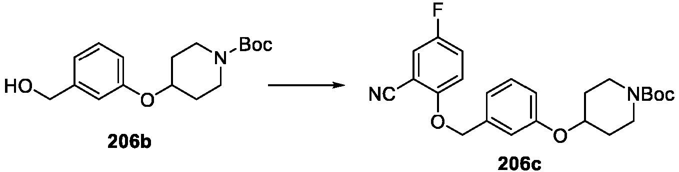

To a solution of **206b** (50 mg, 0.16 mmol, 1.0 eq) in DMF (1.5 mL) was added NaH (10 mg, 0.24 mmol, 60% dispersion in mineral oil) at 0 °C and stirred for 30 min at the same temperature. A solution of 2,5-difluorobenzonitrile (23 mg, 0.17 mmol) in DMF (0.5 mL) was added. After stirring at 20 °C for 2 h, the mixture was poured into ice water (8 mL), and extracted with EtOAc (8 mL x 2). The combined organic layer was washed with H₂O (3 mL), brine (3 mL), dried over Na₂SO₄, filtered and concentrated *in vacuo* to give the title product **(206c)** (65 mg) with some impurity as a white solid. *m*/*z* (ESI, +ve ion) = 449.1 [M+Na]⁺.

### Step D. 5-Fluoro-2-((3-(piperidin-4-yloxy)benzyl)oxy)benzonitrile (206d)

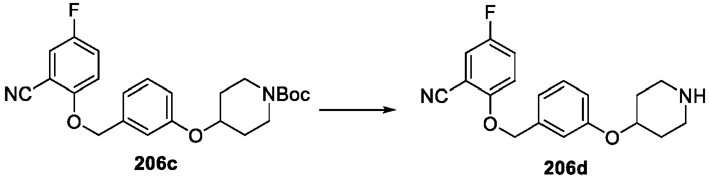

To a solution of **206c** (65 mg, 0.13 mmol) in DCM (1 mL) was added TFA (0.5 mL). The reaction was stirred at 18 °C for 1.5 h and was concentrated to give the crude title product **(206d)** (45 mg) as a yellow oil, which was used in the next step without further purification. *m*/*z* (ESI, +ve ion) = 327.1 [M+H]⁺.

### Step E. Methyl (S)-2-((4-(3-((2-cyano-4-fluorophenoxy)methyl)phenoxy)piperidin-1-yl)methyl)-1-(oxetan-2-ylmethyl)-1H-benzo[d]imidazole-6-carboxylate (206e)

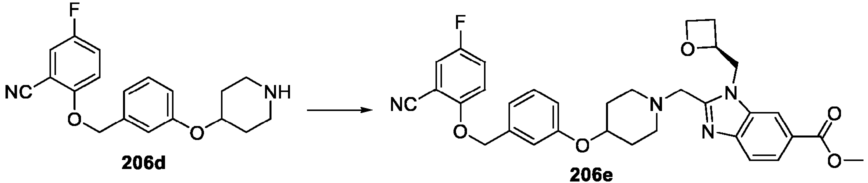

A mixture of **206d** (45 mg, 0.14 mmol), K₂CO₃ (56 mg, 0.41 mmol) in CH₃CN (4 mL) was stirred at 20 °C for 20 min. KI (3 mg, 0.01 mmol) and **1h** (40 mg, 0.14 mmol) were added and the reaction was stirred at 18 °C for another 1.5 h. The reaction was then diluted with EtOAc (15 mL), washed with H₂O (5 mL), and brine (5 mL). The organic layer was dried over Na₂SO₄, filtered and concentrated *in vacuo* to provide the title product **(206e)** (20 mg, 20%) as a white solid which was used in the next step without further purification. *m*/*z* (ESI, +ve ion) = 585.2 [M+H]⁺.

### Step F. (S)-2-((4-(3-((2-Cyano-4-fluorophenoxy)methyl)phenoxy)piperidin-1-yl)methyl)-1-(oxetan-2-ylmethyl)-1H-benzo[d]imidazole-6-carboxylic acid (206)

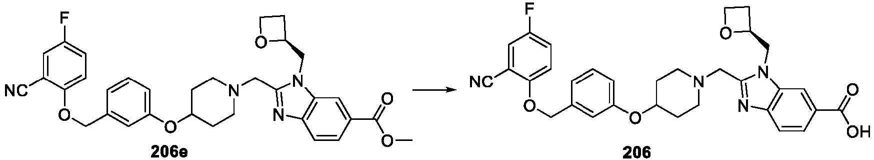

To a solution of **206e** (20 mg, 0.03 mmol) in CH₃OH/H₂O (3 mL, 1:1) was added LiOH (10 mg, 0.3 mmol). The reaction was stirred at 30 °C for 18 h, adjusted to pH = 7 with 1 N HCl, and extracted with DCM/CH₃OH (10:1) (5 mL x 2). The organic layer was concentrated *in vacuo* to give a crude residue, which was purified by prep-TLC (silica gel, DCM/CH₃OH = 10:1) to provide the title product **(206)** (7.2 mg, 35.1%) as a white solid. *m*/*z* (ESI, +ve ion) = 571.2 [M+H]⁺. ¹H NMR (400 MHz, CD₃OD) δ ppm 8.33 (d, *J* = 0.9 Hz, 1H), 7.97 (dd, *J* = 8.5, 1.5 Hz, 1H), 7.67 (d, *J* = 8.3 Hz, 1H), 7.46 (dd, *J* = 8.1, 3.3 Hz, 1H), 7.37 (ddd, *J* = 9.3, 8.0, 3.3 Hz, 1H), 7.21 - 7.31 (m, 2H), 7.09 (s, 1H), 7.01 (d, *J* = 7.4 Hz, 1H), 6.90 (dd, *J* = 8.1, 2.0 Hz, 1H), 5.20 - 5.29 (m, 3H), 4.86 - 4.93 (m, 1H), 4.72 (dd, *J* = 15.7, 2.6 Hz, 1H), 4.65 (td, *J* = 7.6, 6.1 Hz, 1H), 4.46 (dt, *J* = 9.2, 5.9 Hz, 2H), 3.92 - 4.09 (m, 2H), 2.75 - 2.93 (m, 3H), 2.47 - 2.58 (m, 3H), 1.98 - 2.09 (m, 2H), 1.81 (ddd, *J* = 12.5, 8.4, 4.4 Hz, 2H).

Examples **317** were synthesized in similar procedures as described in Example **206,** with THF used as the solvent in the last step.

Examples **207, 221** were synthesized in similar procedures as described in the synthesis of **157a,** followed by Example **152,** Steps C to D.

### Example 208. 2-((4-(3-((2,4-Dichlorophenoxy)methyl)phenoxy)piperidin-1-yl)methyl)-1-(isoxazol-5-ylmethyl)-1H-benzo[d]imidazole-6-carboxylic acid (208)

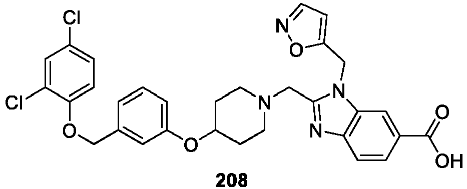

### Step A. tert-Butyl 2-((4-(3-((2,4-dichlorophenoxy)methyl)phenoxy)piperidin-1-yl)methyl)-1-(isoxazol-5-ylmethyl)-1H-benzo[d]imidazole-6-carboxylate (208a)

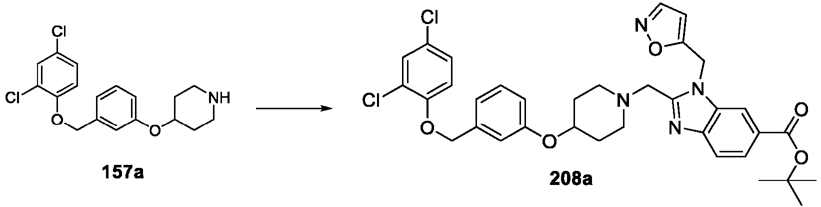

To a mixture of *tert-*butyl 2-(chloromethyl)-3-(1,2-oxazol-5-ylmethyl)-1,3-benzodiazole-5-carboxylate (30 mg, 0.086 mmol, synthesized in similar procedures as **57f** ), **157a** (33 mg, 0.095 mmol) and potassium carbonate (36 mg, 0.259 mmol) in DMF (1 mL) was added potassium iodide (29 mg, 0.173 mmol). The reaction was stirred at 20 °C for 3 h. After completion, the reaction was diluted with H₂O (5 mL) and extracted with EtOAc (10 mL x 3). The combined organic layer was dried, filtered, concentrated and purified by silica gel column chromatography (petroleum ether: EtOAc = 3:1) to afford the title product **(208a)** (35 mg, 58%) as a white solid. *m*/*z* (ESI, +ve ion) = 663.2 [M+H]⁺.

### Step B. 2-((4-(3-((2,4-Dichlorophenoxy)methyl)phenoxy)piperidin-1-yl)methyl)-1-(isoxazol-5-ylmethyl)-1H-benzo[d]imidazole-6-carboxylic acid (208)

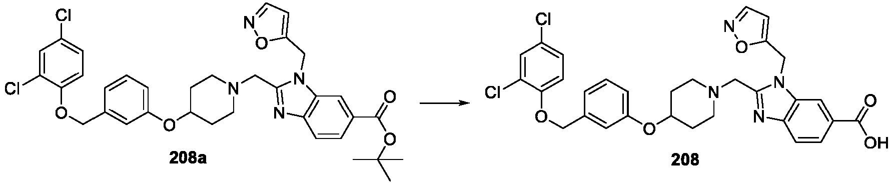

A solution of **208a** (20 mg, 0.03 mmol) in 4 N HCl in dioxane (1 mL) was stirred at 18 °C for 2 h. After completion, the reacion was cooled down in an ice bath. 1 N NaOH was added to the mixture to adjust to pH = 5. The reaction mixture was then extracted with EtOAc (5 mL x 3). The combined organic layer was dried, filtered, concentrated and purified by silica gel column chromatography (petroleum ether:EtOAc = 2:1) to afford the title product **(208)** (9.4 mg, 51%) as a white solid. *m*/*z* (ESI, +ve ion) = 607.2 [M+H]⁺. ¹H NMR (400 MHz, CD₃OD) δ 8.35 (d, *J* = 1.80 Hz, 1H), 8.20 (s, 1H), 7.99 (d, *J* = 8.44 Hz, 1H), 7.67 (d, *J* = 8.44 Hz, 1H), 7.41 (d, *J* = 2.52 Hz, 1H), 7.18 - 7.28 (m, 2H), 7.07 (d, *J* = 8.88 Hz, 1H), 6.96 - 7.03 (m, 2H), 6.86 (dd, *J* = 8.12, 2.1 Hz, 1H), 6.30 (d, *J* = 1.72 Hz, 1H), 5.95 (s, 2H), 5.13 (s, 2H), 4.36 (s, 1H), 3.94 (s, 2H), 2.76 (s, 2H), 2.41 (t, *J* = 8.64 Hz, 2H), 1.88 (s, 2H), 1.65 (d, *J* = 8.9 Hz, 2H)

Examples **257, 280** were synthesized from **145d** in similar procedures as described in Example **208.** *tert-*Butyl 1-((1-(*tert*-butoxycarbonyl)azetidin-3-yl)methyl)-2-(chloromethyl)-*1H*-benzo[*d*]imidazole-6-carboxylate was used for Example **280** at the last step.

Example **215** was synthesized in similar procedures as described in Example **145.**

### Example 216. 2-((4-(3-((2,4-Dichlorophenoxy)methyl)phenoxy)piperidin-1-yl)methyl)-1-(oxazol-5-ylmethyl)-1H-benzo[d]imidazole-6-carboxylic acid (216)

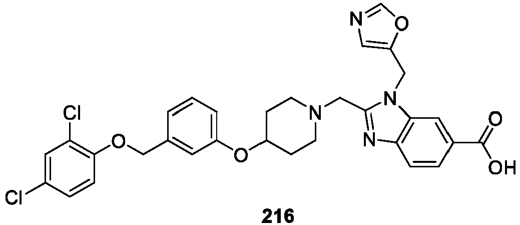

### Step A. tert-Butyl 2-((4-(3-((2,4-dichlorophenoxy)methyl)phenoxy)piperidin-1-yl)methyl)-1-(oxazol-5-ylmethyl)-1H-benzo[d]imidazole-6-carboxylate (216a)

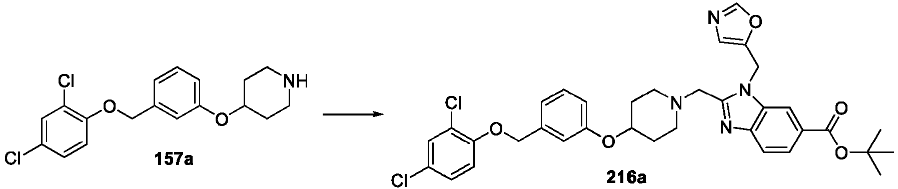

To a solution of **157a** (10 mg, 0.028 mmol) and *tert-*butyl 2-(chloromethyl)-1-(oxazol-5-ylmethyl)-*1H*-benzo[*d*]imidazole-6-carboxylate (9.9 mg, 0.028 mmol) in DMF (1 mL) was added Et₃N (57.5 mg, 0.57 mmol). The resulting mixture was stirred at room temperature overnight. The crude mixture was directly purified by reverse phase HPLC (gradient elution, CH₃CN in H₂O containing 0.1% TFA as a modifier, 20-100%) to afford the title product **(216a)** (17 mg, 90%) as a white solid. *m*/*z* (ESI, +ve ion) = 663.3 [M+H]⁺.

### Step B. 2-((4-(3-((2,4-Dichlorophenoxy)methyl)phenoxy)piperidin-1-yl)methyl)-1-(oxazol-5-ylmethyl)-1H-benzo[d]imidazole-6-carboxylic acid (216)

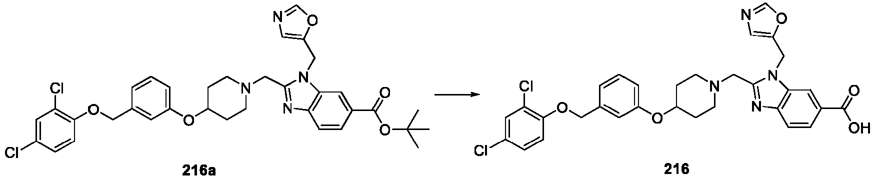

**216a** (17 mg, 0.026 mmol) was dissolved in a mixture containing TFA:triisopropylsilane:H₂O (3.7 mL:0.1 mL:0.2 mL) in DCM (4 mL). The reaction was stirred at room temperature for 1 h. After completion, the reaction was concentrated *in vacuo* and redissolved in DMSO (1 mL) before reverse phase HPLC purification (gradient elution, CH₃CN in H₂O containing 0.1% TFA as a modifier, 0-90%) to afford the title product **(216)** as a white solid (13.4 mg, 72.5%). ¹H NMR (600 MHz,) δ ppm 8.40 (d, *J* = 1.10 Hz, 1H), 8.16 (s, 1H), 8.03 (dd, *J* = 8.62, 1.65 Hz, 1H), 7.79 (d, *J* = 8.44 Hz, 1H), 7.42 (d, *J* = 2.57 Hz, 1H), 7.31 - 7.39 (m, 2H), 7.24 (dd, *J* = 8.80, 2.57 Hz, 1H), 7.15 (s, 1H), 7.07 - 7.13 (m, 2H), 7.01 (dd, *J* = 8.25, 2.38 Hz, 1H), 5.76 (s, 2H), 5.17 (s, 2H), 4.93 (s, 2H), 4.80 (br s, 1H), 3.72 (br s, 2H), 3.67 (br s, 2H), 2.32 (m, 2H), 2.24 (, 2H). *m*/*z* (ESI, +ve ion) = 607.2 [M+H]⁺.

Examples **217** and **232** were synthesized in similar procedures as described in Example **216.**

### Example 220. (S)-2-((4-((2-((2-Chloro-4-fluorophenoxy)methyl)pyridin-4-yl)oxy)piperidin-1-yl)methyl)-1-(oxetan-2-ylmethyl)-1H-benzo[d]imidazole-6-carboxylic acid (220)

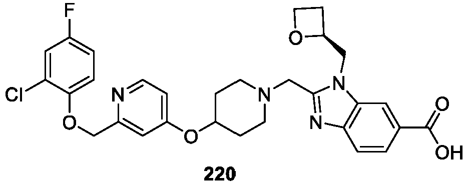

### Step A. tert-Butyl 4-((2-(((methylsulfonyl)oxy)methyl)pyridin-4-yl)oxy)piperidine-1-carboxylate (220a)

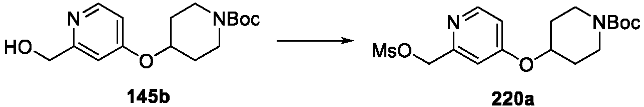

To a solution of **145b** (50 mg, 0.16 mmol) in DCM (3 mL) was added Ms₂O (157 mg, 0.48 mmol) and Et₃N (80 mg, 0.8 mmol) at 0 °C. The mixture was stirred at 5 °C for 1 h. After completion, the mixture was quenched with H₂O (2 ml) and extracted with EtOAc (5 mL x 3). The combined organic layer was dried over anhydrous Na₂SO₄, filtered and concentrated to afford the crude title product **(220a)** (80 mg), which was used in the next step without further purification.

### Step B. tert-Butyl 4-((2-((2-chloro-4-fluorophenoxy)methyl)pyridin-4-yl)oxy)piperidine-1-carboxylate (220b)

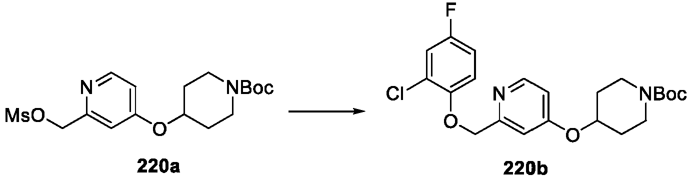

To a solution of crude **220a** (80 mg, 0.21 mmol) in DMF (5 mL) was added 2-chloro-4-fluorophenol (30 mg, 0.21 mmol) and Cs₂CO₃ (105 mg, 0.32 mmol) at 20 °C. The mixture was heated at 50 °C for 5 h. After completion, the reaction was quenched by H₂O (10 mL) and extracted with EtOAc (5 mL x 3). The combined organic layer was dried over anhydrous Na₂SO₄, filtered, and concentrated to afford a crude residue, which was purified by silica gel column chromatography (petroleum ether:EtOAc = 1:1) to afford the title product **220b** (80 mg, 87%). *m*/*z* (ESI, +ve ion) = 437.1 [M+H]⁺.

### Step C. 2-((2-Chloro-4-fluorophenoxy)methyl)-4-(piperidin-4-yloxy)pyridine (220c)

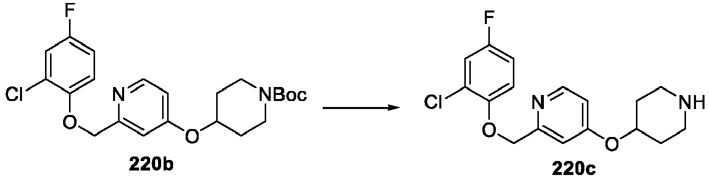

To a solution of **220b** (80 mg, 0.18 mmol) in DCM (3 mL) was added TFA (0.6 mL) at 0 °C. The resulting mixture was stirred at 20 °C for 1 h. After completion, the reaction was concentrated to afford the crude title product **(220c)** (100 mg), which was used in the next step without further purification. *m*/*z* (ESI, +ve ion) = 337.1 [M+H]⁺.

### Step D. Methyl (S)-2-((4-((2-((2-chloro-4-fluorophenoxy)methyl)pyridin-4-yl)oxy)piperidin-1-yl)methyl)-1-(oxetan-2-ylmethyl)-1H-benzo[d]imidazole-6-carboxylate (220d)

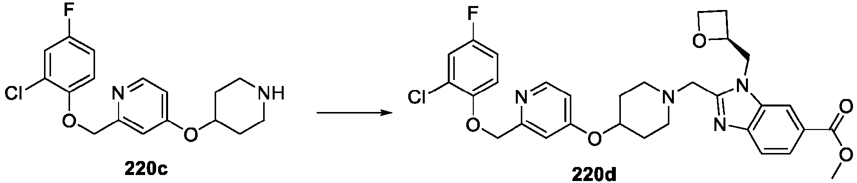

To a solution of **220c** (50 mg, 0.15 mmol) in CH₃CN (3 mL) was added **1h** (44 mg, 0.15 mmol) and K₂CO₃ (124 mg, 0.9 mmol) at 0 °C. The resulting mixture was stirred at 20 °C for 4 h. After completion, the reaction was concentrated and purified by prep-TLC (EtOAc:MeOH = 10:1) to afford the title product **(220d)** (40 mg, 38%). *m*/*z* (ESI, +ve ion) = 595.2 [M+H]⁺.

### Step E. (S)-2-((4-((2-((2-Chloro-4-fluorophenoxy)methyl)pyridin-4-yl)oxy)piperidin-1-yl)methyl)-1-(oxetan-2-ylmethyl)-1H-benzo[d]imidazole-6-carboxylic acid (220)

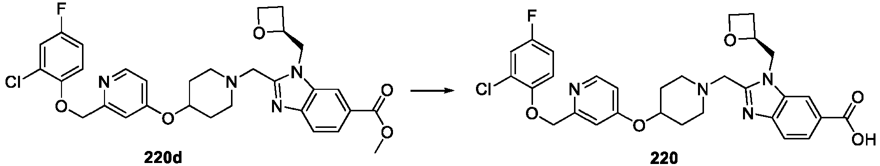

To a solution of **220d** (15 mg, 0.03 mmol) in MeOH/THF/H₂O (1:1:1, 3 mL) was added LiOH (7.3 mg, 0.3 mmol). The resulting mixture was stirred at 20 °C for 3 h. After completion, the mixture was quenched with 1 N HCl aq. (0.5 mL) to adjust to pH = 6, extracted with EtOAc (5 mL x 3). The combined organic layer was dried over anhydrous Na₂SO₄, filtered and concentrated to provide a crude product, which was purified by reverse phase HPLC (CH₃CN in H₂O, with 0.05% TFA as a modifier) to afford the title product **(220)** (7.4 mg, 48%). *m*/*z* (ESI, +ve ion) = 581.2 [M+H]⁺. ¹H NMR (400 MHz, CD₃OD) δ ppm 8.56 (d, *J* = 6.54 Hz, 1H), 8.36 (d, *J* = 0.87 Hz, 1H) 8.05 (dd, *J* = 8.50, 1.53 Hz, 1H), 7.80 (d, *J* = 8.72 Hz, 1H), 7.53 (d, *J* = 2.18 Hz, 1H), 7.35 (dd, *J* = 6.32, 2.40 Hz, 1H), 7.29 (dd, *J* = 7.85, 3.05 Hz, 1H), 7.21 (dd, *J* = 9.16, 4.80 Hz, 1H), 7.08 (ddd, *J* = 9.05, 7.96, 3.05 Hz, 1H), 5.32 (s, 2H), 5.18 - 5.26 (m, 1H), 5.07 (br s, 1H), 4.78 - 4.83 (m, 3H), 4.63 - 4.72 (m, 2H), 4.45 (dt, *J* = 9.37, 5.78 Hz, 1H), 3.46 - 3.70 (m, 4H), 2.76 - 2.87 (m, 1H), 2.45 - 2.56 (m, 1H), 2.14 - 2.42 (m, 4H).

Examples **308, 352, 356, 357, 358, 359** were synthesized from **220a** in similar procedures as described in Example **220.** Examples **309, 318** were synthesized from **206b** in similar procedures as described in Example **220.** Example **316, 321, 322, 333, 354** were synthesized in similar procedures as described in Example **220.** For all these compounds following Example **220** synthesis, DMF could be used as the solvent at the second to last step and THF used as the solvent in last step. Last step purification can have the option of no modifier in the reverse phase purification.

Example **378, 379** were synthesized in similar procedures as described in Example **220,** except DIPEA/DMF was used in the second to last step instead of K₂CO₃. NH₄OH was used as the modifier for the last step purification.

Example **288** was synthesized from **206b** in similar procedures as described in Example **220.** 4 N HCl in dioxane was used to remove the Boc group (Step C) instead of TFA. Example **294** was synthesized in similar procedures as described in Example **288.** The mesylate analog of **57f** derived from **145e** was used in the second to last step.

Example **290** was synthesized from methyl 6-hydroxypicolinate and *tert-*butyl (2*S*,*4S*)-4-hydroxy-2-methylpiperidine-1-carboxylate as described in Example **271,** Steps A to B, followed by Example **220,** Steps A to E. Example **291** was synthesized in similar procedures as described in Example **290.**

Example **224** was synthesized from **157a** in similar procedures as described in Example **155,** Steps E and F.

Example **227** is the side product of the last step hydrolysis of Example **168.**

### Example 230. 2-((4-((2-((4-Chloro-2-fluorophenoxy)methyl)pyridin-4-yl)oxy)piperidin-1-yl)methyl)-1-((1-ethyl-1H-imidazol-5-yl)methyl)-1H-benzo[d]imidazole-6-carboxylic acid (230)

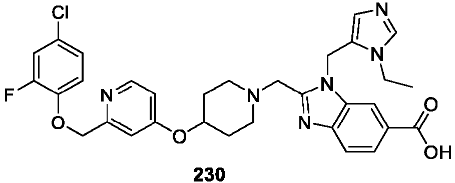

### Step A. Methyl 2-((4-((2-((4-chloro-2-fluorophenoxy)methyl)pyridin-4-yl)oxy)piperidin-1-yl)methyl)-1-((1-ethyl-1H-imidazol-5-yl)methyl)-1H-benzo[d]imidazole-6-carboxylate (230b)

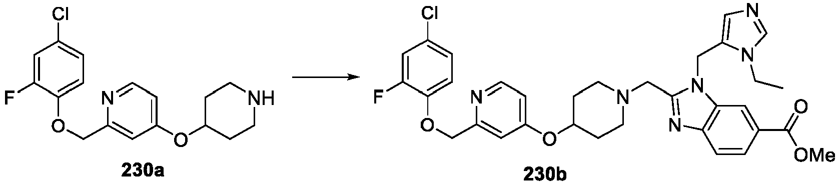

A solution of **230a** (prepared in similar procedures as **145d,** 19.0 mg, 0.056 mmol), **57f** (25.2 mg, 0.056 mmol) and DIPEA (0.02 mL, 1.13 mmol) in DMF (0.3 mL) was stirred at room temperature overnight. After completion, the reaction mixture was diluted with EtOAc (5 mL). The organic layer was washed with water (3 mL x 2), brine (3 mL), dried over Na₂SO₄, filtered and concentrated and the resulting crude residue was purified by silica gel column chromatography (gradient elution, 0-20% MeOH in DCM) to afford the title product **(230b)** (7.6 mg, 21%) as a pale-yellow oil. *m*/*z* (ESI, +ve ion) = 633.3 [M+H]⁺.

### Step B. 2-((4-((2-((4-Chloro-2-fluorophenoxy)methyl)pyridin-4-yl)oxy)piperidin-1-yl)methyl)-1-((1-ethyl-1H-imidazol-5-yl)methyl)-1H-benzo[d]imidazole-6-carboxylic acid (230)

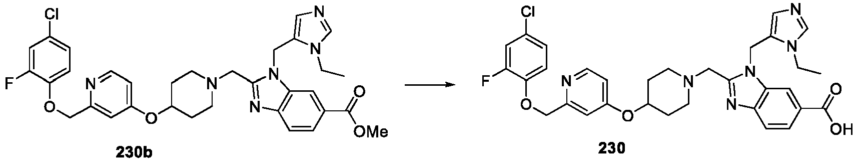

A solution of **230b** (7.6 mg, 0.012 mmol) and LiOH (2.5 mg, 0.060 mmol) in THF:MeOH:H₂O = 2:1:1 (0.4 mL) was stirred at 30 °C overnight. After completion, the reaction mixture was diluted with water to 1 mL and purified by reverse phase HPLC (gradient elution, 20-50% CH₃CN in H₂O, with 0.1% TFA as a modifier) to afford the title product **(230)** (8.7 mg, 99%) as a white solid. *m*/*z* (ESI, +ve ion) = 619.3 [M+H]⁺. ¹H NMR (600 MHz, CD₃OD) δ ppm 9.11 (d, *J* = 1.1 Hz, 1H), 8.63 (d, *J* = 7.0 Hz, 1H), 8.26 (s, 1H), 8.09 (dd, *J* = 8.4, 1.5 Hz, 1H), 7.85 (d, *J* = 8.8 Hz, 1H), 7.60 (d, *J* = 2.6 Hz, 1H), 7.48 (dd, *J* = 6.8, 2.8 Hz, 1H), 7.32 (dd, *J* = 11.0, 2.2 Hz, 1H), 7.23 - 7.27 (m, 2H), 7.11 (d, *J* = 1.1 Hz, 1H), 5.93 (s, 2H), 5.42 (s, 2H), 5.06 (br s, 1H), 4.50 (br s, 2H), 4.39 (q, *J* = 7.2 Hz, 2H), 3.30 - 3.37 (m, 2H), 3.15 - 3.25 (m, 2H), 2.22 - 2.30 (m, 2H), 2.06 (br s, 2H), 1.59 (t, *J* = 7.3 Hz, 3H).

Examples **259, 267** were synthesized in similar procedures as described in Example **230.**

Example **250** was synthesized in similar procedures as described in Example **230,** with the last step reverse phase HPLC purification using 0.05% NH₄OH as a modifier.

Examples **336, 344, 345, 362, 365** were synthesized in similar procedures as described in Example **250.** Example **346** is the side product isolated in the last step synthesis of Example **345.** Example **364** was synthesized from **274c** in similar procedures as described in Example **250.**

Example **277** was synthesized in similar procedures as described in Example **230,** with the last step product purified through normal phase silica gel column chromatography.

Example **302, 335, 337,** and **363** was synthesized in similar procedures as described in Example **277.**

Example **231** was synthesized from 4-(3-((4-chloro-2-fluorophenoxy)methyl)phenoxy)piperidine (following similar procedures of **145d)** and **57f** as described in Example **155,** Steps E and F.

Examples **242** and **243** were synthesized in similar procedures as described in Example **231.**

### Example 239. 2-((4-((2-((4-Chloro-2-isocyanophenoxy)methyl)pyridin-4-yl)oxy)piperidin-1-yl)methyl)-1-(oxazol-2-ylmethyl)-1H-benzo[d]imidazole-6-carboxylic acid (239)

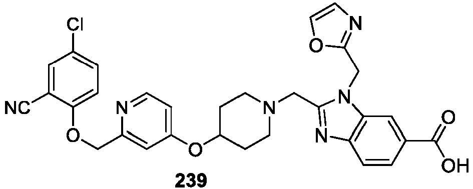

### Step A. tert-Butyl 2-((4-((2-((4-chloro-2-isocyanophenoxy)methyl)pyridin-4-yl)oxy)piperidin-1-yl)methyl)-1-(oxazol-2-ylmethyl)-1H-benzo[d]imidazole-6-carboxylate (239b)

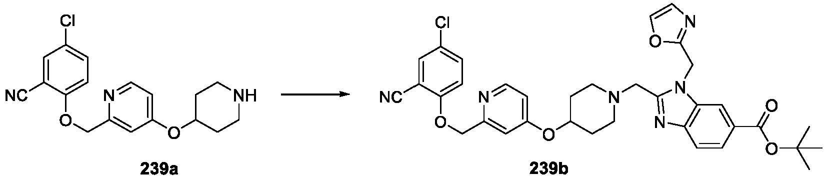

A solution of **239a** (prepared in similar procedures as **145d,** 13.0 mg, 0.038 mmol), *tert*-butyl 2-(chloromethyl)-1-(oxazol-2-ylmethyl)-*1H*-benzo[*d*]imidazole-6-carboxylate (13.2 mg, 0.038 mmol) and DIPEA (132.0 µL, 0.76 mmol) in DMF (0.4 mL) was stirred at room temperature for 2 h. After completion, the reaction mixture was diluted with EtOAc (5 mL). The organic layer was washed with water (3 mL x 2), brine (3 mL), dried over Na₂SO₄, filtered and concentrated and the resulting crude residue was purified by silica gel column chromatography (gradient elution, 80-100% EtOAc/hexanes then 0-20% MeOH in DCM) to afford the title product **(239b)** (20.9 mg, 84%) as a colorless oil. *m*/*z* (ESI, +ve ion) = 655.1 [M+H]⁺.

### Step B. 2-((4-((2-((4-Chloro-2-isocyanophenoxy)methyl)pyridin-4-yl)oxy)piperidin-1-yl)methyl)-1-(oxazol-2-ylmethyl)-1H-benzo[d]imidazole-6-carboxylic acid (239)

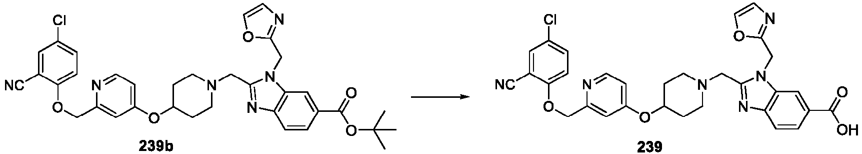

A solution of **239b** (20.9 mg, 0.032 mmol) in DCM (1.0 mL) and TFA (1.0 mL) was stirred at room temperature overnight. After completion, the reaction solvent was removed under reduced pressure and the resulting residue was diluted with water to 1 mL and purified by reverse phase HPLC (gradient elution, 20-60% CH₃CN in H₂O, with 0.1% TFA as a modifier) to afford the title product **(239)** (22.7 mg, 100%) as a white solid. *m*/*z* (ESI, +ve ion) = 599.3 [M+H]⁺. ¹H NMR (600 MHz, CD₃OD) δ ppm 8.57 (d, *J =* 8.2 Hz, 1H), 8.35 (d, *J* = 1.1 Hz, 1H), 8.05 (dd, *J* = 8.6, 1.7 Hz, 1H), 7.96 (d, *J* = 1.1 Hz, 1H), 7.80 (d, *J* = 8.1 Hz, 1H), 7.78 (d, *J* = 2.6 Hz, 1H), 7.70 (dd, *J* = 8.8, 2.6 Hz, 1H), 7.61 (d, *J* = 2.6 Hz, 1H), 7.31 - 7.34 (m, 2H), 7.19 (d, *J* = 1.1 Hz, 1H), 5.86 (s, 2H), 5.42 (s, 2H), 5.06 (br s, 1H), 4.75 (s, 2H), 3.55 (br s, 2H), 3.37 - 3.48 (m, 2H), 2.28 - 2.39 (m, 2H), 2.12 - 2.24 (m, 2H).

**Examples 240, 241, 258, 265, 266** were synthesized in similar procedures as described in Example **239.** Examples **268, 269, 270** were synthesized from the corresponding analogs of **198c** in similar procedures as described in Example **239.**

Examples **251, 252, 253** were synthesized in similar procedures as described in Example **198,** Steps A to C, followed by Example **219,** Steps A to B.

Example **254** was synthesized from methyl 3-hydroxybenzoate and *tert-*butyl (2*S*,*4S*)-4-hydroxy-2-methylpiperidine-1-carboxylate in similar procedures as described in Example **145,** Steps A to D, followed by Example **155,** Steps E and F.

Example **255** was synthesized from **157a** in similar procedures as described in Example **152,** Steps C and D.

Example **256** was synthesized from **186b** and *t*-butyl ester analog of **57f** in similar procedures as described in Example **208,** Steps A and B.

### Examples 260 and 263. (R)-2-((4-((3-((4-Chloro-2-cyanophenoxy)methyl)phenyl)fluoromethyl)piperidin-1-yl)methyl)-1-((1-ethyl-1H-imidazol-5-yl)methyl)-1H-benzo[d]imidazole-6-carboxylic acid (260) and (S)-2-((4-((3-((4-chloro-2-cyanophenoxy)methyl)phenyl)fluoromethyl)piperidin-1-yl)methyl)-1-((1-ethyl-1H-imidazol-5-yl)methyl)-1H-benzo[d]imidazole-6-carboxylic acid (263)

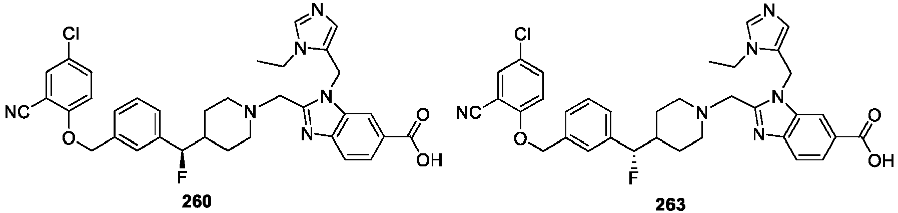

### Step A. tert-Butyl 4-(hydroxy(3-(methoxycarbonyl)phenyl)methyl)piperidine-1-carboxylate (260a)

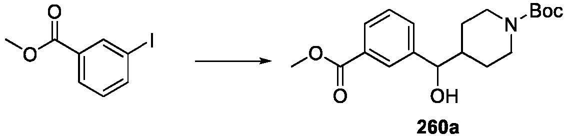

A solution of methyl 3-iodobenzoate (3.5 g, 13.4 mmol) and isopropyl magnesium chloride (2 M in THF) (7.35 mL, 14.7 mmol) in anhydrous THF (30 mL) was stirred at -65 °C for 0.5 h. Then *tert-*butyl (4-formylpiperidin-1-yl) formate (3.16 g, 14.7 mmol) was added to the mixture. After stirring at 17 °C for 1 h, the reaction was quenched with saturated NH₄Cl solution (100 mL) and extracted with ethyl acetate (100 mL x 3). The organic layer was dried over Na₂SO₄, filtered, concentrated and purified by silica gel column chromatography (petroleum ether:EtOAc = 7:3) to afford the title product **(260a)** (4.2 g, 85%) as a colorless oil. *m*/*z* (ESI, +ve ion) = 372.2 [M+Na]⁺.

### Step B. tert-Butyl 4-(fluoro(3-(methoxycarbonyl)phenyl)methyl)piperidine-1-carboxylate (260b)

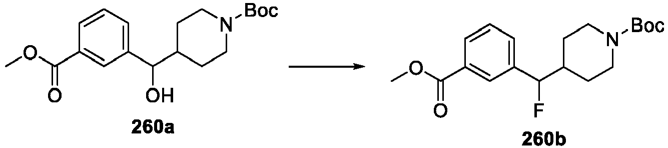

To a solution of **260a** (4.2 g, 12 mmol, 1.0 eq) in THF (45 mL) was added Deoxo-Fluor (3.18 g, 14.4 mmol) at 0 °C. The mixture was stirred at 0 °C for 2 h under N₂. After completion, saturated aqueous sodium bicarbonate solution was added to the mixture to adjust to pH = 7 in ice bath. Then the reaction was diluted with H₂O (100 mL) and extracted with EtOAc (100 mL x 3). The combined organic layer was dried over Na₂SO₄, filtered, concentrated and purified by silica gel column chromatography (petroleum:EtOAc = 8:2) to afford the title product **(260b)** (2.8 g, 62%) as a colorless oil. *m*/*z* (ESI, +ve ion) = 374.2 [M+Na]⁺.

### Step C. tert-Butyl 4-(fluoro(3-(hydroxymethyl)phenyl)methyl)piperidine-1-carboxylate (260c)

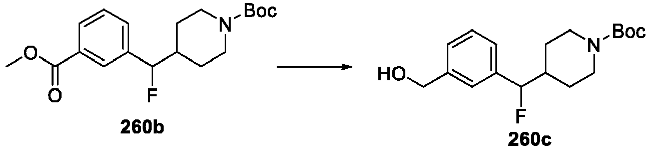

A solution of **260b** (2.7 g, 7.7 mmol) and LiAlH₄ (440 mg, 11.5 mmol) in THF (30 mL) was stirred at 0 °C for 1 h. After completion, the reaction was quenched with sodium sulfate decahydrate and the precipitates were rinsed with THF (50 mL x 3). The combined organic layer was dried over Na₂SO₄, filtered, concentrated and purified by silica gel column chromatography (petroleum ether:EtOAc = 7:3) to afford the title product **(260c)** (2.1 g, 79%) as colorless oil. *m*/*z* (ESI, +ve ion) = 346.1 [M+Na].

### Step D. tert-Butyl 4-((3-((4-chloro-2-cyanophenoxy)methyl)phenyl)fluoromethyl)piperidine-1-carboxylate (260d)

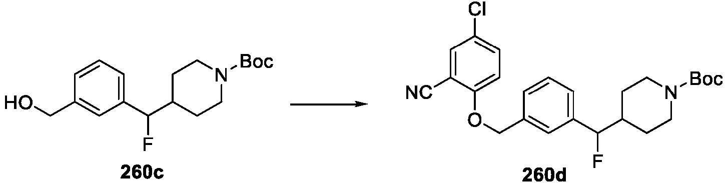

A solution of **260c** (2.17 g, 6.7 mmol), 5-chloro-2-hydroxybenzonitrile (1.03 g, 6.7 mmol), PPh₃ (2.64 g, 10.0 mmol) and DEAD (1.75 g, 10.0 mmol) in THF (20 mL) was stirred at 0 °C for 1 h and 25 °C for 12 h. After completion, the reaction was diluted with H₂O (50 mL) and extracted with EtOAc (50 mL x 3). The organic layer was dried over anhydrous Na₂SO₄, filtered, concentrated and purified by silica gel column chromatography (petroleum ether: EtOAc = 9:1) to afford the title product **(260d)** (1.5 g, 46%) as a colorless oil. *m*/*z* (ESI, +ve ion) = 481.2 [M+Na]⁺.

### Step E. tert-Butyl (R)-4-((3-((4-chloro-2-cyanophenoxy)methyl)phenyl)fluoromethyl)piperidine-1-carboxylate (260d-P1) and tert-butyl (S)-4-((3-((4-chloro-2-cyanophenoxy)methyl)phenyl)fluoromethyl)piperidine-1-carboxylate (260d-P2)

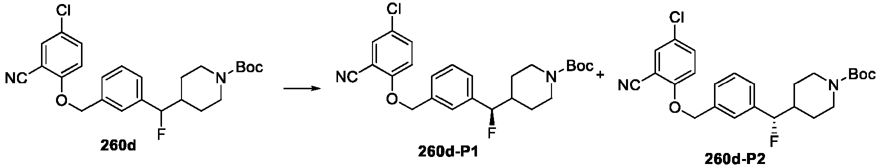

The title products **(260d-P1** and **260d-P2)** were obtained through chiral separation of **260d** by UPCC (Daicel CHIRALPAK OJ-3 3*150mm, CO₂/MeOH (0.1%DEA) = 85/15) to afford **260d-P1** (130 mg, RT = 2.911m) and **260d-P2** (140 mg, RT = 3.321m) as a colorless oil. The stereochemistry of **260d-P1** and **260d-P2** are arbitrarily assigned.

### Step F. (R)-2-((4-((3-((4-Chloro-2-cyanophenoxy)methyl)phenyl)fluoromethyl)piperidin-1-yl)methyl)-1-((1-ethyl-1H-imidazol-5-yl)methyl)-1H-benzo[d]imidazole-6-carboxylic acid (260) and (S)-2-((4-((3-((4-chloro-2-cyanophenoxy)methyl)phenyl)fluoromethyl)piperidin-1-yl)methyl)-1-((1-ethyl-1H-imidazol-5-yl)methyl)-1H-benzo[d]imidazole-6-carboxylic acid (263)

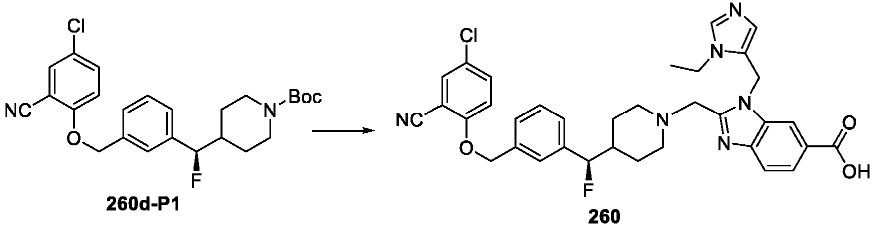

The title compound **(260)** was synthesized in similar procedures from **260d-P1** as described in Example **152,** Step B to D. A 1:1 THF:H₂O mixed solvent was used for the last step. *m*/*z* (ESI, +ve ion) = 641.2 [M+H]⁺. ¹H NMR (400 MHz, CD₃OD) δ ppm 8.98 (d, *J* = 1.0 Hz, 1H), 8.23 (d, *J* = 0.7 Hz, 1H), 8.05 (dd, *J* = 8.6, 1.7 Hz, 1H), 7.82 (d, *J* = 8.3 Hz, 1H), 7.69 (d, *J* = 2.7 Hz, 1H), 7.62 (dd, *J* =9.1, 2.7 Hz, 1H), 7.42 - 7.54 (m, 3H), 7.31 - 7.37 (m, 1H), 7.28 (d, *J* = 9.1 Hz, 1H), 7.00 (d, *J* = 1.2 Hz, 1H), 5.83 (s, 2H), 5.36 (dd, *J* = 48.0, 8.0 Hz, 1H), 5.28 - 5.31 (m, 2H), 4.48 (br s, 2H), 4.32 (q, *J* = 7.3 Hz, 2H), 3.45 - 3.63 (m, 2H), 2.73 - 2.92 (m, 2H), 2.02 - 2.20 (m, 2H), 1.97 (br d, *J* = 15.4 Hz, 1H), 1.44 - 1.72 (m, 5H).

The title compound **(263)** was synthesized in similar procedures from **260d-P2** as described in Example **152,** Step B to D. A 1:1 THF:H₂O mixed solvent was used for the last step. *m*/*z* (ESI, +ve ion) = 641.3 [M+H]⁺. ¹H NMR (400 MHz, CD₃OD) δ = 8.23 (d, *J* = 0.73 Hz, 1H), 8.05 (dd, *J* = 8.4, 1.6 Hz, 1H), 7.83 (d, *J* = 8.6 Hz, 1H), 7.69 (d, *J* = 2.7 Hz, 1H), 7.62 (dd, *J* = 9.1, 2.7 Hz, 1H), 7.51 (s, 1H), 7.43 - 7.49 (m, 3H), 7.22 - 7.38 (m, 1H), 7.28 (d, *J* = 9.1 Hz, 1H), 7.01 (s, 1H), 5.83 (s, 2H), 5.37 (dd, *J* = 48.0, 8.0 Hz, 1H), 5.28 (s, 2H), 4.53 (br s, 2H), 4.33 (q, *J* = 7.3 Hz, 2H), 3.50 - 3.68 (m, 2H), 2.79 - 2.99 (m, 2H), 2.05 - 2.22 (m, 1H), 1.99 (br d, *J* = 13.5 Hz, 1H), 1.45 - 1.73 (m, 6H).

### Example 261. 2-((4-((2-((4-Cyano-2-fluorophenoxy)methyl)pyrimidin-4-yl)oxy)piperidin-1-yl)methyl)-1-((1-ethyl-1H-imidazol-5-yl)methyl)-1H-benzo[d]imidazole-6-carboxylic acid (261)

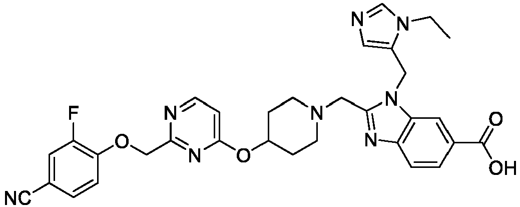

### Step A. 4-((4-Chloropyrimidin-2-yl)methoxy)-3-fluorobenzonitrile (261a)

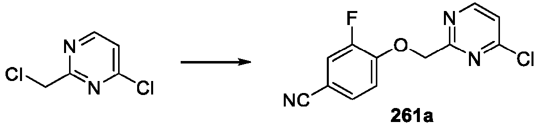

A mixture of 3-fluoro-4-hydroxybenzonitrile (81.8 mg, 0.6 mmol) and 4-chloro-2-(chloromethyl)pyrimidine (81 mg, 0.5 mmol) in DMF (4 mL) was treated with Cs₂CO₃ (324 mg, 1 mmol) at room temperature. The reaction mixture was stirred for 2 h before quenched with water (50 mL) and extracted with EtOAc (20 mL x 3). The combined organic layer was washed with brine, dried with Na₂SO₄, filtered and concentrated *in vacuo.* The crude residue was purified by silica gel column chromatography (EtOAc in hexanes) to provide the title product **(261a)** (93 mg, 71%) as a white solid. *m*/*z* (ESI, +ve ion) = 264.1 [M+H]⁺.

### Step B. tert-Butyl 4-((2-((4-cyano-2-fluorophenoxy)methyl)pyrimidin-4-yl)oxy)piperidine-1-carboxylate (261b)

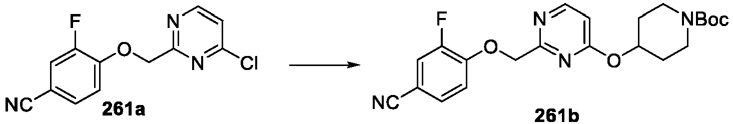

A reaction vial was charged with **261a** (18.7 mg, 0.071 mmol), *tert-*butyl 4-hydroxypiperidine-1-carboxylate (17.1 mg, 0.085 mmol), Cs₂CO₃ (46.2 mg, 0.14 mmol) and degassed with Ar, followed by addition of dry toluene (4 mL) under an Ar balloon. The same preparation was applied to a separate vial containing a solution of Pd₂(dba)₃ (2.9 mg, 0.004 mmol) and BINAP (4.4 mg, 0.0071 mmol) in dry toluene (2 mL), heated up to 110 °C for 5 min, which was then transferred to the first reaction vial using syringe. The resulting reaction mixture was heated at 110 °C overnight. Upon completion, the reaction solvent was removed *in vacuo,* and the residue was diluted with EtOAc/H₂O and extracted with EtOAc. The organic layer was dried over Na₂SO₄, filtered, concentrated and purified by silica gel column chromatography to provide the title product **(261b)** (25 mg, 82%). *m*/*z* (ESI, +ve ion) = 429.3 [M+H]⁺.

### Step C. 3-Fluoro-4-((4-(piperidin-4-ylmethyl)pyrimidin-2-yl)methoxy)benzonitrile (261c)

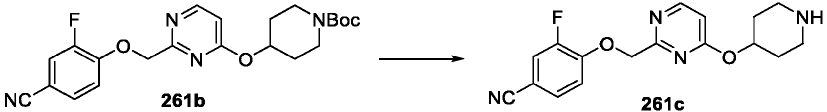

**261b** (149 mg, 0.35 mmol) was dissolved in a solution of TFA (2 mL) in DCM (5 mL). After stirring for 10 min, the reaction was concentrated *in vacuo.* The resulting residue was re-dissolved in DMSO (1 mL) and purified by reverse phase HPLC (gradient elution, 0-60% CH₃CN in H₂O containing 0.1% TFA as a modifier) to afford the title product **(261c)** (117 mg, 76%) as a white solid. *m*/*z* (ESI, +ve ion) = 329.3 [M+H]⁺.

### Step D. Methyl 2-((4-((2-((4-cyano-2-fluorophenoxy)methyl)pyrimidin-4-yl)oxy)piperidin-1-yl)methyl)-1-((1-ethyl-1H-imidazol-5-yl)methyl)-1H-benzo[d]imidazole-6-carboxylate (261d)

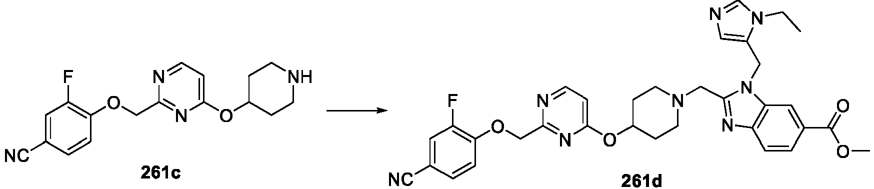

To a solution of **261c** (TFA salt, 7.3 mg, 0.017 mmol) and **57f** (6.6 mg, 0.02 mmol) in DMF (1 mL) was added Et₃N (33.4 mg, 0.33 mmol). The resulting mixture was stirred at room temperature overnight then purified by reverse phase HPLC (gradient elution, 0-75% CH₃CN in H₂O containing 0.1% TFA as a modifier) to provide the title product **(261d)** (9.2 mg, 75%) as a white solid. *m*/*z* (ESI, +ve ion) = 625.3 [M+H]⁺.

### Step E. 2-((4-((2-((4-Cyano-2-fluorophenoxy)methyl)pyrimidin-4-yl)oxy)piperidin-1-yl)methyl)-1-((1-ethyl-1H-imidazol-5-yl)methyl)-1H-benzo[d]imidazole-6-carboxylic acid (261)

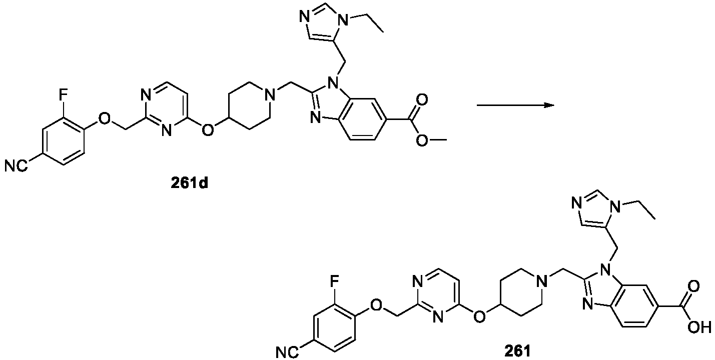

**261d** (9.2 mg, 0.012 mmol) was dissolved in CH₃CN (0.5 mL) and treated with 2 N NaOH (0.5 mL). The mixture was stirred at room temperature for 24 h, then purified by reverse phase HPLC (gradient elution, 0-75% CH₃CN in H₂O containing 0.1% TFA as a modifier) to provide the title product (261) as a white solid (7.8 mg, 86%). *m*/*z* (ESI, +ve ion) = 611.4 [M+H]⁺. ¹H NMR (600 MHz, CD₃OD) δ ppm 9.08 (s, 1H), 8.50 (br d, *J* = 5.87 Hz, 1H), 8.24 (br s, 1H), 8.07 (dd, *J* = 8.80, 1.47 Hz, 1H), 7.85 (br d, *J* = 8.44, 1H), 7.56 - 7.61 (m, 1H), 7.45-7.48 (m, 1H), 7.25 (br t, *J* = 8.44 Hz, 1H), 7.07 (s, 1H), 6.82 (m, 1H), 5.87 (s, 2H), 5.40 (s, 2H), 4.65 - 4.74 (m, 2H), 4.57 - 4.64 (m, 1H), 4.35 (q, *J* = 7.34 Hz, 2H), 3.47 - 3.58 (m, 2H), 3.33 - 3.45 (m, 2H), 2.10 - 2.22 (m, 2H), 1.94 - 2.10 (m, 2H), 1.57 (t, *J* = 7.34 Hz, 3H).

### Example 262. (S)-2-((4-((2-((4-Cyano-2-fluorophenoxy)methyl)pyrimidin-4-yl)oxy)piperidin-1-yl)methyl)-1-(oxetan-2-ylmethyl)-1H-benzo[d]imidazole-6-carboxylic acid (262)

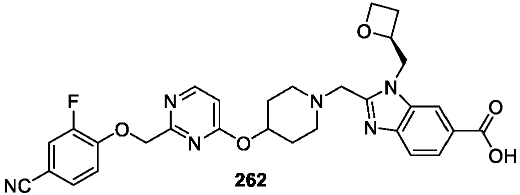

### Step A. Methyl (S)-2-((4-((2-((4-cyano-2-fluorophenoxy)methyl)pyrimidin-4-yl)oxy)piperidin-1-yl)methyl)-1-(oxetan-2-ylmethyl)-1H-benzo[d]imidazole-6-carboxylate (262a)

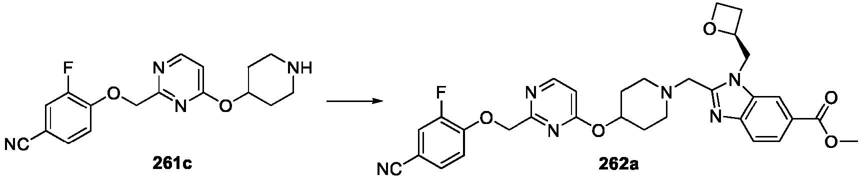

To a solution of **261c** (7.3 mg, 0.017 mmol) and **1h** (5.8 mg, 0.02 mmol) in DMF (1 mL) was added Et₃N (33.4 mg, 0.33 mmol). The resulting mixture was stirred at room temperature overnight then purified by reverse phase HPLC (gradient elution, 0-75% CH₃CN in H₂O containing 0.1% TFA as a modifier) to provide the title product **(262a)** (11.9 mg, with impurities) as a white solid. *m*/*z* (ESI, +ve ion) = 587.4 [M+H]⁺.

### Step B. (S)-2-((4-((2-((4-Cyano-2-fluorophenoxy)methyl)pyrimidin-4-yl)oxy)piperidin-1-yl)methyl)-1-(oxetan-2-ylmethyl)-1H-benzo[d]imidazole-6-carboxylic acid (262)

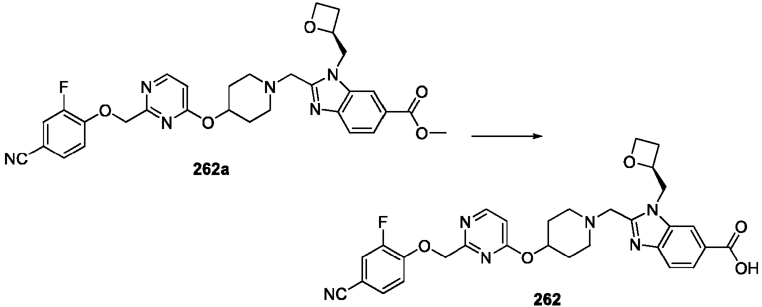

**262a** (12 mg, 0.017 mmol) was dissolved in CH₃CN (0.5 mL) and treated with 2 N NaOH (0.5 mL). The mixture was stirred at room temperature for 24 h, then directly purified by reverse phase HPLC (gradient elution, 0-75% CH₃CN in H₂O containing 0.05% NH₄OH as a modifier) to afford the title product **(262)** as a white solid (5.5 mg, 55%). *m*/*z* (ESI, +ve ion) = 573.3 [M+H]⁺. ¹H NMR (600 MHz, CD₃OD) δ ppm 8.43 (d, *J* = 5.87 Hz, 1H), 8.31 (d, *J* = 1.10 Hz, 1H), 7.97 (dd, *J* = 8.44, 1.47 Hz, 1H), 7.67 (d, *J* = 8.44 Hz, 1H), 7.59 (dd, *J* = 11.00, 1.83 Hz, 1H), 7.43 - 7.50 (m, 1H), 7.22 (t, *J* = 8.44 Hz, 1H), 6.75 (d, *J* = 5.87 Hz, 1H), 5.39 (s, 2H), 5.25 (qd, *J* = 7.07, 2.89 Hz, 1H), 4.96 - 5.03 (m, 1H), 4.85 - 4.90 (m, 1H), 4.70 (br dd, *J* =15.41, 2.93 Hz, 1H), 4.64 (td, *J* = 7.89, 5.87 Hz, 1H), 4.46 (dt, *J* = 9.08, 5.91 Hz, 1H), 4.01 (br d, *J* = 13.57 Hz, 1H), 3.90 (br d, *J* = 13.57 Hz, 1H), 2.77 - 2.84 (m, 2H), 2.71 - 2.77 (m, 1H), 2.49 - 2.55 (m, 1H), 2.30 - 2.38 (m, 2H), 1.87 - 1.94 (m, 2H), 1.68 - 1.77 (m, 2H).

Example **374** was synthesized from 4-chloro-2-(chloromethyl)-5-fluoropyrimidine in similar procedures as described in Example **262.**

Examples **296, 315, 328, 331, 339, 343** were synthesized in similar procedures as described in Example **262.**

### Example 271. 2-((4-((2-((2,4-Dichlorophenoxy)methyl)pyridin-3-yl)oxy)piperidin-1-yl)methyl)-1-((1-ethyl-1H-imidazol-5-yl)methyl)-1H-benzo[d]imidazole-6-carboxylic acid (271)

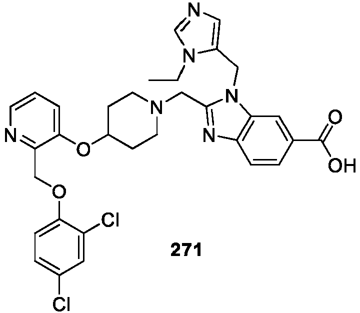

### Step A. Methyl 3-((1-(tert-butoxycarbonyl)piperidin-4-yl)oxy)picolinate (271a)

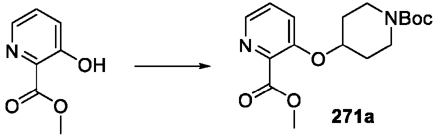

A solution of methyl 3-hydroxypyridine-2-carboxylate (1.5 g, 9.8 mmol), *tert-*butyl 4-hydroxypiperidine-1-carboxylate (1.97 g, 9.8 mmol), PPh₃ (3.86 g, 14.7 mmol) and DEAD (2.56 g, 14.7 mmol) in anhydrous THF (15 mL) was stirred at 0 °C for 1 h under N₂. The resulting mixture was stirred at 17 °C for 11 h. After completion, the reaction was diluted with H₂O (100 mL) and extracted with EtOAc (100 mL x 3). The organic layer was dried over Na₂SO₄, concentrated and purified by silica gel column chromatography (petroleum ether: EtOAc = 7:3) to afford the crude product **(271a)** (5.1 g) which contained some PPh₃O as a colorless oil. *m*/*z* (ESI, +ve ion) = 337.1 [M+H]⁺.

### Step B. tert-Butyl 4-((2-(hydroxymethyl)pyridin-3-yl)oxy)piperidine-1-carboxylate (271b)

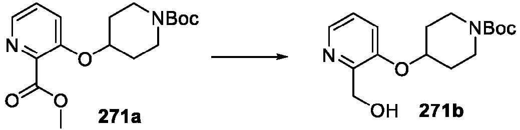

A solution of crude **271a** (5.1 g) and LiBH₄ (330 mg, 1.5 mmol) in THF/MeOH (50 mL/10 mL) was stirred at 0 °C for 1 h. After completion, the reaction was quenched with sodium sulfate decahydrate and filtered. The filter cake was further rinsed with THF (100 mL x 3). The combined organic layer was dried over Na₂SO₄, filtered, concentrated and purified by reverse phase HPLC (27% CH₃CN in H₂O, with 0.05% TFA as a modifier) to afford the title product **(271b)** (1.3 g, 26.3%) as a colorless oil. *m*/*z* (ESI, +ve ion) = 309.2 [M+H]⁺.

### Step C. tert-Butyl 4-((2-((2,4-dichlorophenoxy)methyl)pyridin-3-yl)oxy)piperidine-1-carboxylate (271c)

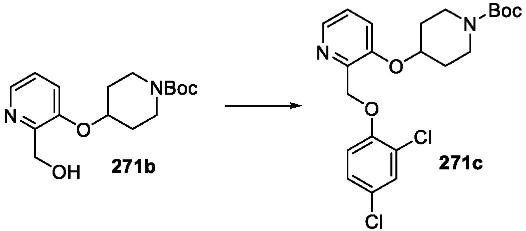

A solution of **271b** (303 mg, 1.02 mmol), 2,4-dichlorophenol (167 mg, 1.02 mmol), PPh₃ (403 mg, 1.54 mmol) and DEAD (268 mg, 1.54 mmol) in anhydrous THF (6 mL) was stirred at 0 °C for 1 h under N₂. Then the resulting mixture was stirred at 17 °C overnight. After completion, the reaction solution was diluted with H₂O (20 mL) and extracted with EtOAc (20 mL x 3). The combined organic layer was dried over Na₂SO₄, filtered, concentrated and purified by silica gel column chromatography (petroleum ether: EtOAc = 7:3) to afford the title product **(271c)** (180 mg, 38%) as a colorless oil. *m*/*z* (ESI, +ve ion) = 453.1 [M+H]⁺.

### Step D. 2-((4-((2-((2,4-Dichlorophenoxy)methyl)pyridin-3-yl)oxy)piperidin-1-yl)methyl)-1-((1-ethyl-1H-imidazol-5-yl)methyl)-1H-benzo[d]imidazole-6-carboxylic acid (271)

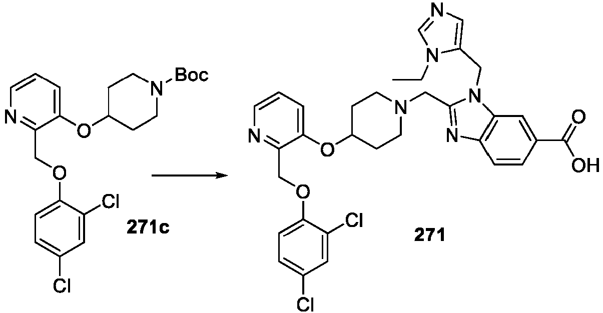

The title compound 271 was synthesized in similar procedures from 271c as described in Example 152, Step B to D. A 1:1 THF:H₂O mixed solvent was used for the last step. *m*/*z* (ESI, +ve ion) = 635.1 [M+H]⁺. ¹H NMR (400 MHz, CD₃OD) δ ppm 8.23 (d, *J* = 1.7 Hz, 1H), 8.05 (dd, *J* = 8.6, 1.5 Hz, 1H), 7.83 (d, *J* = 8.6 Hz, 1H), 7.79 (d, *J* = 7.8 Hz, 1H), 7.56 (dd, *J* = 8.6, 4.9 Hz, 1H), 7.42 (d, *J* = 2.2 Hz, 1H), 7.22 - 7.30 (m, 2H), 7.04 (d, *J* = 1.2 Hz, 1H), 5.86 (s, 2H), 5.37 (s, 2H), 4.73 (s, 2H), 4.35 (q, *J* = 7.3 Hz, 2H), 3.50 - 3.70 (m, 5H), 2.28 - 2.40 (m, 2H), 2.12 - 2.27 (m, 2H), 1.57 (t, *J* = 7.3 Hz, 3H).

Example **286** was synthesized in similar procedures as described in Example **271.**

Example **272** was synthesized in similar procedures from methyl 2-hydroxybenzoate as described in Example **271,** Step A, then Example **60,** Steps D and E, followed by Example **106,** Steps D to F.

Example **284** was synthesized in similar procedures as described in Example **272.**

### Example 274. 2-(((2S,4S)-4-((2-((2,4-Dichlorophenoxy)methyl)pyridin-4-yl)oxy)-2-methylpiperidin-1-yl)methyl)-1-((1-ethyl-1H-imidazol-5-yl)methyl)-1H-benzo[d]imidazole-6-carboxylic acid (274)

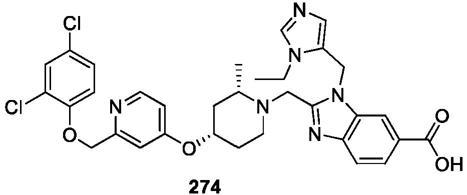

### Step A. tert-Butyl (2S,4R)-2-methyl-4-((methylsulfonyl)oxy)piperidine-1-carboxylate (274a)

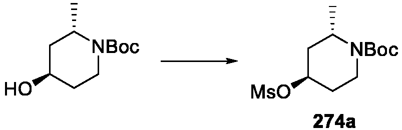

To a mixture of *tert-*butyl (*2S*,*4R*)-4-hydroxy-2-methylpiperidine-1-carboxylate (400 mg, 1.86 mmol), DIPEA (960 mg, 7.43 mmol) in DCM (10 mL) was added a solution of methanesulfonic anhydride (809 mg, 4.64 mmol) in DCM (2.5 mL). After stirring at 0 °C for 2 h, the reaction was diluted with EtOAc (100 mL) and washed with H₂O (40 mL x 2). The organic layer was dried over Na₂SO₄, filtered and concentrated *in vacuo* to afford the title product **(274a)** (550 mg) as a colorless oil, which was used in the next step without further purification. *m*/*z* (ESI, +ve ion) = 316.1 [M+Na]⁺.

### Step B. Methyl 4-(((2S,4S)-1-(tert-butoxycarbonyl)-2-methylpiperidin-4-yl)oxy)picolinate (274b)

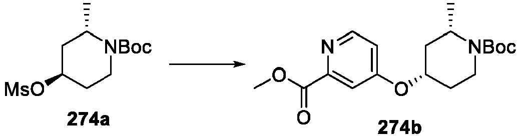

A mixture of **274a** (460 mg, 1.55 mmol), methyl 4-hydroxypyridine-2-carboxylate (238 mg, 1.55 mmol), Cs₂CO₃ (608 mg, 1.86 mmol) in DMF (4 mL) was stirred at 90 °C for 3 h. The mixture was poured into water (20 mL), and extracted with EtOAc (20 mL x 3). The organic layer was washed with H₂O (10 mL), brine (10 mL), dried over Na₂SO₄, filtered and concentrated *in vacuo* to give a crude residue, which was purified by prep-TLC (EtOAc/petroleum ether = 2:1) to afford the title product **(274b)** (317 mg, 52.4%) as a colorless oil. *m*/*z* (ESI, +ve ion) = 351.1 [M+H]⁺.

### Step C. tert-Butyl (2S,4S)-4-((2-(hydroxymethyl)pyridin-4-yl)oxy)-2-methylpiperidine-1-carboxylate (274c)

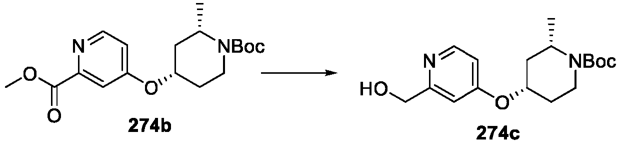

A mixture of **274b** (300 mg, 0.86 mmol) in THF (4 mL) was added LiAlH₄ (1 mL, 1.03 mmol) at 0 °C. The mixture was stirred at 0 °C for 1 h and quenched with Na₂SO₄.10H₂O. After stirring at 20 °C for 30 min, the reaction was filtered and concentrated *in vacuo* to afford the title product **(274c)** (229 mg, 75%) as a colorless oil, which was used in the next step without further purification. *m*/*z* (ESI, +ve ion) = 323.1 [M+H]⁺.

### Step D. 2-(((2S,4S)-4-((2-((2,4-Dichlorophenoxy)methyl)pyridin-4-yl)oxy)-2-methylpiperidin-1-yl)methyl)-1-((1-ethyl-1H-imidazol-5-yl)methyl)-1H-benzo[d]imidazole-6-carboxylic acid (274)

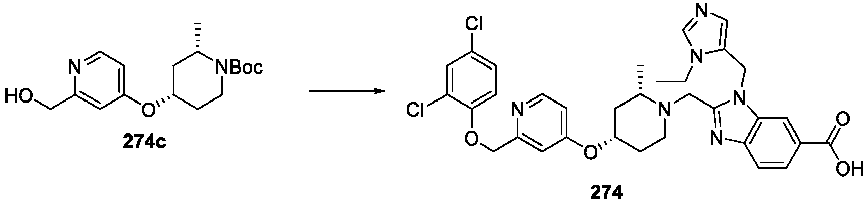

The title product **(274)** was synthesized in similar procedures from **274c** as described in Example **220,** Steps A to E. DMF was used as the solvent at the second to last step. *m*/*z* (ESI, +ve ion) = 649.1 [M+H]⁺. ¹H NMR (400 MHz, MeOD) δ ppm ¹H NMR (400 MHz, CD₃OD) 9.10 (d, *J* = 1.47 Hz, 1 H) 8.57 (d, *J* = 6.60 Hz, 1H) 8.22 (d, *J* = 0.73 Hz, 1H), 8.08 (dd, *J* = 8.56, 1.47 Hz, 1H), 7.83 (d, *J* = 8.31 Hz, 1H), 7.53 (d, *J* = 2.45 Hz, 2H), 7.32 - 7.41 (m, 2H), 7.20 (d, *J* = 8.80 Hz, 1H), 7.08 (d, *J* = 1.22 Hz, 1H), 5.95 (br d, *J* = 3.42 Hz, 2H), 5.37 (s, 2H), 4.76 (br d, *J* = 15.16 Hz, 1H), 4.41 (q, *J* = 7.34 Hz, 2H), 4.17 - 4.08 (m, 1H), 3.23 -3.31 (m, 2H), 3.23 - 3.13 (m, 1H), 2.90 - 2.78 (m, 1H), 2.17 - 2.41 (m, 2H), 1.66 - 1.82 (m, 2H), 1.62 (t, *J* = 7.34 Hz, 3H), 1.40 (d, *J* = 6.11 Hz, 3H).

Examples **283** was synthesized in similar procedures as described in Example **274.** Example **295** was synthesized from *tert-*butyl (*2S*,*4S*)-4-hydroxy-2-methylpiperidine-1-carboxylate in similar procedures as described in Example **274.** LiBH₄ was the reducing agent at Step C. These compounds all used silica gel column or prep-TLC for purification at the last step.

Example **366** was synthesized in similar procedures as described in Example **274,** with the last step purification using reverse phase HPLC (NH₄OH as modifier).

Example **275** was synthesized in similar procedures as described in Example **145.**

### Example 276. 2-((4-((2-((4-Chloro-2-cyanophenoxy)methyl)pyridin-4-yl)oxy)-2,2-dimethylpiperidin-1-yl)methyl)-1-((1-ethyl-1H-imidazol-5-yl)methyl)-1H-benzo[d]imidazole-6-carboxylic acid (276)

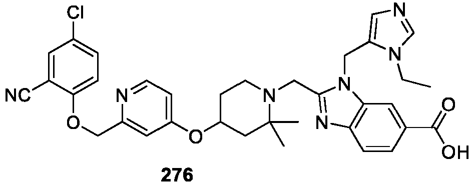

### Step A. tert-Butyl 4-((2-(bromomethyl)pyridin-4-yl)oxy)-2,2-dimethylpiperidine-1-carboxylate (276b)

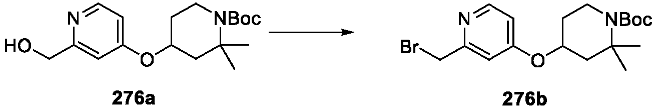

To a solution of **276a** (prepared in similar procedures as **145a,** 70.7 mg, 0.21 mmol), PPh₃ (82.7 mg, 0.32 mmol) in DCM (2.1 mL) was added CBr₄ (105 mg, 0.32 mmol). The reaction was stirred at room temperature for 35 min. Upon completion, the reaction was concentrated *in vacuo* and the resulting crude residue was purified by silica gel column chromatography (gradient elution, 5-40% acetone in hexanes) to afford the title product **(276b)** (57.7 mg, 69%) as a pale-yellow oil. *m*/*z* (ESI, +ve ion) = 399.3 [M+H]⁺.

### Step B. tert-Butyl 4-((2-((4-chloro-2-cyanophenoxy)methyl)pyridin-4-yl)oxy)-2,2-dimethylpiperidine-1-carboxylate (276c)

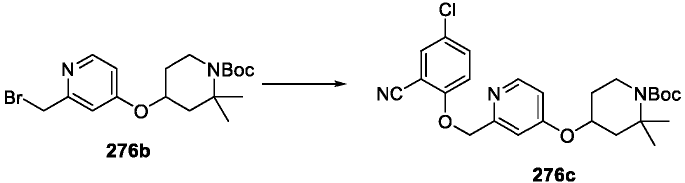

To a solution of **276b** (57.7 mg, 0.14 mmol) and 5-chloro-2-hydroxybenzonitrile (44.4 mg, 0.29 mmol) in DMF (0.72 mL) was added Cs₂CO₃ (118 mg, 0.36 mmol). It was stirred at room temperature for 1 h. Upon completion, the reaction was quenched by water (5 mL). The aqueous layer was extracted with EtOAc (5 mL x 3). The combined organic layer was washed with water (5 mL), brine (5 mL x 3), dried over Na₂SO₄, filtered, concentrated and the resulting crude residue was purified by silica gel column chromatography (gradient elution, 20-100% EtOAc in hexanes) to afford the title product **(276c)** (53.8 mg, 79%) as a white solid. *m*/*z* (ESI, +ve ion) = 472.4 [M+H]⁺.

### Step C. 5-Chloro-2-((4-((2,2-dimethylpiperidin-4-yl)oxy)pyridin-2-yl)methoxy)benzonitrile (276d)

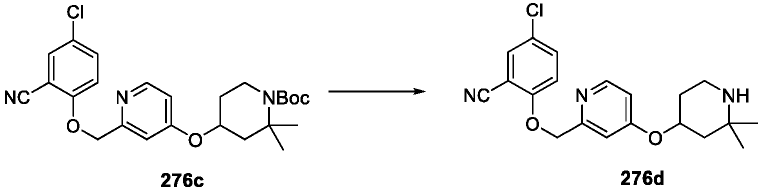

A solution of **276c** (53.8 mg, 0.114 mmol) in DCM (1.14 mL) and TFA (0.57 mL) was stirred at room temperature for 30 min. After completion, the reaction solvent was removed under reduced pressure and the resulting residue was diluted with EtOAc (3 mL). The organic layer was washed with sat. NaHCO₃ (5 mL), brine (3 mL), dried over Na₂SO₄, filtered and concentrated to afford the title product **(276d)** (20 mg) as a colorless oil, which was used in the next step without further purification. *m*/*z* (ESI, +ve ion) = 372.3 [M+H]⁺.

### Step D. Methyl 2-((4-((2-((4-chloro-2-cyanophenoxy)methyl)pyridin-4-yl)oxy)-2,2-dimethylpiperidin-1-yl)methyl)-1-((1-ethyl-1H-imidazol-5-yl)methyl)-1H-benzo[d]imidazole-6-carboxylate (276e)

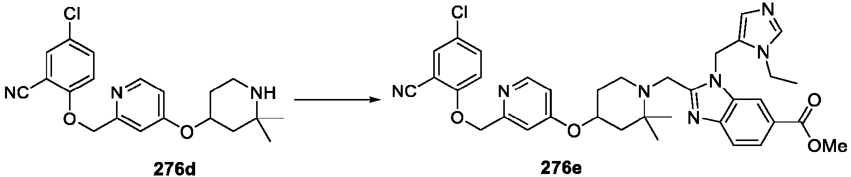

A solution of **276d** (20.0 mg, 0.054 mmol), **57f** (46.2 mg, 0.10 mmol) and Et₃N (75 µL, 0.54 mmol) in DMF (0.3 mL) was stirred at 80 °C. After completion, the reaction mixture was diluted with EtOAc (5 mL). The organic layer was washed with water (3 mL x 2), brine (3 mL), dried over Na₂SO₄, filtered and concentrated and the resulting crude residue was purified by silica gel column chromatography (gradient elution, 0-10% MeOH in DCM) to afford the title product **(276e)** (9.2 mg, 26%) as a white solid. *m*/*z* (ESI, +ve ion) = 668.4 [M+H]⁺.

### Step E. 2-((4-((2-((4-Chloro-2-cyanophenoxy)methyl)pyridin-4-yl)oxy)-2,2-dimethylpiperidin-1-yl)methyl)-1-((1-ethyl-1H-imidazol-5-yl)methyl)-1H-benzo[d]imidazole-6-carboxylic acid (276)

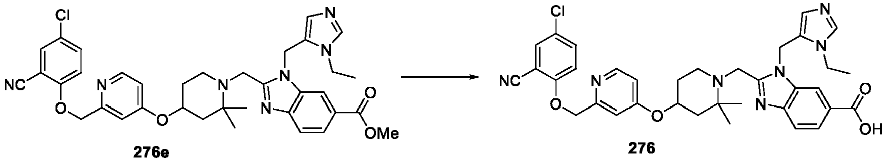

A solution of **276e** (9.2 mg, 0.014 mmol) and LiOH (1.7 mg, 0.041 mmol) in THF:MeOH:H₂O = 2:1:1 (0.4 mL) was stirred at 30 °C overnight. After completion, the reaction mixture was diluted with water to 1 mL and purified by reverse phase HPLC (gradient elution, 15-40% CH₃CN in H₂O, with 0.1% TFA as a modifier) to afford the title product **(276)** (5.8 mg, 55%) as a white solid. ¹H NMR (600 MHz, CD₃OD) δ ppm 9.07 (s, 1H), 8.49 (d, *J* = 6.2 Hz, 1H), 8.17 (s, 1H), 8.07 (d, *J* = 8.4 Hz, 1H), 7.82 (d, *J* = 8.4 Hz, 1H), 7.76 (d, *J* = 2.6 Hz, 1H), 7.69 (dd, *J* = 9.2, 2.6 Hz, 1H), 7.49 (d, *J* = 2.2 Hz, 1H), 7.31 (d, *J* = 9.2 Hz, 1H), 7.18 (dd, *J* = 6.4, 2.4 Hz, 1H), 7.04 (s, 1H), 5.87 - 5.98 (m, 2H), 5.37 (s, 2H), 4.96 - 5.03 (m, 1H), 4.61 (d, *J* = 15.4 Hz, 1H), 4.40 (q, *J* = 7.1 Hz, 2H), 4.17 - 4.28 (m, 1H), 3.20 - 3.27 (m, 1H), 3.05 - 3.17 (m, 1H), 2.25 (dd, *J* = 8.8, 1.5 Hz, 1H), 2.15 - 2.21 (m, 1H), 1.92 - 2.00 (m, 1H), 1.70 - 1.80 (m, 1H), 1.62 (t, *J* = 7.3 Hz, 3H), 1.46 (s, 3H), 1.42 (s, 3H). *m*/*z* (ESI, +ve ion) = 654.5 [M+H]⁺.

Examples **278** and **279** were synthesized from methyl 5-((diethoxyphosphoryl)methyl)oxazole-2-carboxylate and *tert-*butyl (*S*)-2-methyl-4-oxopiperidine-1-carboxylate in similar procedures as described in Example **106,** Step A, then Example **128,** Step B to C (LiBH₄ was used in Step C), followed by Example **220,** Steps A to E. Chiral separation was performed after Step B of Example **220.**

### Example 285. (S)-2-((4-((6-((4-Cyano-2-fluorophenoxy)methyl)pyridin-2-yl)oxy)piperidin-1-yl)methyl)-1-(oxetan-2-ylmethyl)-1H-benzo[d]imidazole-6-carboxylic acid (285)

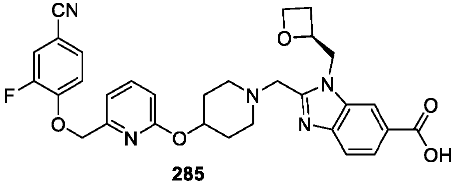

### Step A. tert-Butyl 4-((methylsulfonyl)oxy)piperidine-1-carboxylate (285a)

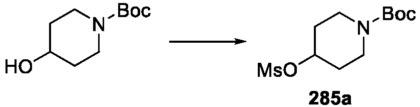

To a solution of *tert-*butyl 4-hydroxypiperidine-1-carboxylate (8 g, 0.0396 mol), methanesulfonic anhydride (17.25 g, 0.099 mol) in DCM (100 mL) was added DIPEA (20.47 g, 0.1584 mol). The reaction was stirred at 23 °C for 2 h. After completion, the reaction was diluted with H₂O (30 mL) and extracted with EtOAc (60 mL x 3). The organic layer was dried, filtered and concentrated to afford the crude title product **(285a)** (10 g) as a colorless oil. *m*/*z* (ESI, +ve ion) = 302.1 [M+Na]⁺.

### Step B. Methyl 6-((1-(tert-butoxycarbonyl)piperidin-4-yl)oxy)picolinate (285b)

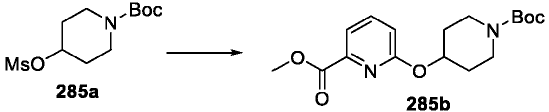

To a solution of **285a** (5.03 g, 17.9 mmol), methyl 6-hydroxypicolinate (2.5 g, 16.3 mmol) in DMF (50 mL) was added Cs₂CO₃ (15.93 g, 48.9 mmol). The reaction was stirred at 90 °C for 10 h. After completion, the reaction was diluted with H₂O (15 mL) and extracted with EtOAc (30 mL x 3). The combined organic layer was dried, filtered, concentrated and purified by silica gel column chromatography (petroleum ether: EtOAc = 6:1) to afford the title product **(285b)** (2.1 g, 36%) as a white solid. *m*/*z* (ESI, +ve ion) = 337.2 [M+H]⁺.

### Step C. tert-Butyl 4-((6-(hydroxymethyl)pyridin-2-yl)oxy)piperidine-1-carboxylate (285c)

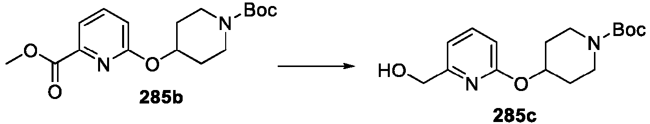

A flask was charged with **285b** (2.1 g, 6.2 mmol) and added LiAlH₄ (1 M in THF, 10 mL, 10 mmol). The reaction was stirred at 0 °C for 2 h. After completion, the reaction was diluted with H₂O (15 mL) and extracted with EtOAc (20 mL x 3). The organic layer was dried, filtered, concentrated and purified by silica gel column chromatography (petroleum ether: EtOAc = 2:1) to afford the title product **(285c)** (1.0 g, 52%) as a white solid. *m*/*z* (ESI, +ve ion) = 309.2 [M+H]⁺.

### Step D. tert-Butyl 4-((6-((4-cyano-2-fluorophenoxy)methyl)pyridin-2-yl)oxy)piperidine-1-carboxylate (285d)

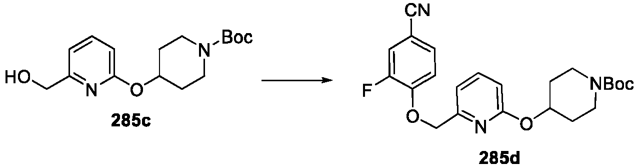

To a solution of **285c** (100 mg, 0.32 mmol), 3-fluoro-4-hydroxybenzonitrile (45 mg, 0.32 mmol), PPh₃ (128 mg, 0.49 mmol)) in anhydrous DCM (1.6 mL) at 0 °C was added slowly N-({[(4-chlorophenyl)methoxy]carbonyl}imino)[(4-chlorophenyl)methoxy]formamide (DCAD) (179 mg, 0.49 mmol) in CH₂Cl₂ (2.5 mL). The reaction was allowed to warm to room temperature and white precipitate was formed. After 1.5 h, reaction mixture was filtered and the filtrate was concentrated to provide a crude residue, which was purified by silica gel column chromatography (gradient elution, 0-40% EtOAc in hexanes) to provide the title product **(285d)** (140 mg, 100%). *m*/*z* (ESI, +ve ion) = 450.3 [M+Na]⁺.

### Step E. (S)-2-((4-((6-((4-Cyano-2-fluorophenoxy)methyl)pyridin-2-yl)oxy)piperidin-1-yl)methyl)-1-(oxetan-2-ylmethyl)-1H-benzo[d]imidazole-6-carboxylic acid (285)

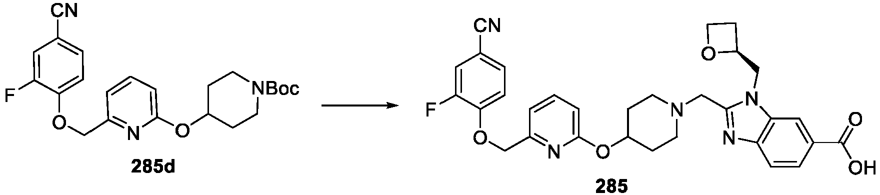

The title product **(285)** was synthesized in similar procedures as described in Example **198,** Steps C to E. *m*/*z* (ESI, +ve ion) = 572.4 [M+H]⁺. ¹H NMR (600 MHz, CD₃OD) δ ppm 8.31 (s, 1H), 7.97 (br dd, *J* = 8.44, 1.10 Hz, 1H), 7.63 - 7.71 (m, 2H), 7.57 (br dd, *J* = 10.82, 2.02 Hz, 1H), 7.50 (br d, *J* = 8.44 Hz, 1H), 7.33 (br t, *J*=8.44 Hz, 1H), 7.05 (d, *J* = 7.34 Hz, 1H), 6.69 (d, *J* = 8.07 Hz, 1H), 5.23 - 5.30 (m, 3H), 5.02 - 5.07 (m, 1 H) 4.93 - 4.85 (m, 1 H), 4.72 (br dd, *J* = 15.41, 2.57 Hz, 1H), 4.65 (br dd, *J* = 13.76, 7.89 Hz, 1H), 4.47 (dt, *J*=9.08, 5.91 Hz, 1H), 3.90 - 4.07 (m, 2H), 2.77 - 2.90 (m, 3H), 2.40 - 2.57 (m, 3H), 1.97 - 2.04 (m, 2H), 1.73 - 1.85 (m, 2 H).

### Example 297. (S)-2-((4-((2-((4-(Difluoromethyl)-2-fluorophenoxy)methyl)pyridin-4-yl)oxy)piperidin-1-yl)methyl)-1-(oxetan-2-ylmethyl)-1H-benzo[d]imidazole-6-carboxylic acid (297)

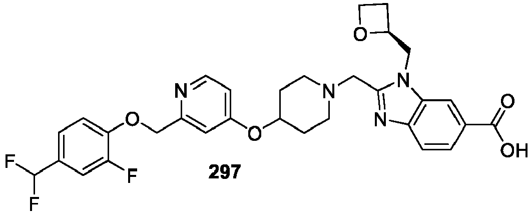

### Step A. 4-(Difluoromethyl)-2-fluorophenol (297a)

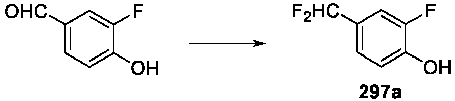

To a solution of 3-fluoro-4-hydroxybenzaldehyde (3 g, 21.4 mmol) in anhydrous CH₂Cl₂ (107 mL) at 0 °C was added Deoxo-Fluor (15.8 g, 32.1 mmol, 45% in THF) dropwise. The reaction was warmed to room temperature and stirred for 24 h. After completion, the reaction was cooled down to 0 °C, quenched with sat. NaHCO₃ dropsise, and extracted with EtOAc. The combined organic layer was washed with water, brine, dried over Na₂SO₄, filtered, concentrated and purified by silica gel column chromatography (gradient elution, 0-30% EtOAc in hexanes) to afford the title product **(297a)** (1.88 g, 54%) as a colorless oil. A stock solution of **297a** in toluene (18.8 mL, 100 mg/mL) was prepared and stored in freezer for further use. *m*/*z* (ESI, -ve ion) = 161.1 [M - H]⁻.

### Step B. (S)-2-((4-((2-((4-(Difluoromethyl)-2-fluorophenoxy)methyl)pyridin-4-yl)oxy)piperidin-1-yl)methyl)-1-(oxetan-2-ylmethyl)-1H-benzo[d]imidazole-6-carboxylic acid (297)

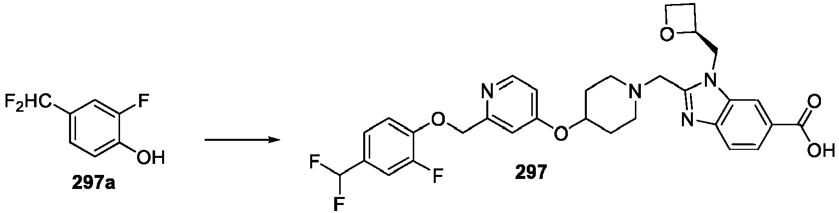

The title product **(297)** was prepared from **297a** in similar procedures as described in Example **277.** *m*/*z* (ESI, +ve ion) = 597.3 [M+H]⁺. ¹H NMR (600 MHz, DMSO-d₆) δ ppm 8.37 (d, *J* = 5.7 Hz, 1H), 8.26 (d, *J* = 1.3 Hz, 1H), 7.79 (dd, = 8.4, 1.5 Hz, 1H), 7.64 (d, *J* = 8.4 Hz, 1H), 7.48 (d, *J* = 11.7 Hz, 1H), 7.34 - 7.38 (m, 2H), 7.07 (d, *J* = 2.6 Hz, 1H), 6.97 (dd, *J* = 5.9, 2.6 Hz, 1H), 6.96 (t, *J* = 40.0 Hz, 1H), 5.24 (s, 2H), 5.08 (qd, *J* = 7.2, 2.8 Hz, 1H), 4.78 (dd, *J* = 15.3, 7.2 Hz, 1H), 4.63 (dd, *J* = 15.2, 2.8 Hz, 1H), 4.54 - 4.60 (m, 1H), 4.46 - 4.52 (m, 1H), 4.37 (dt, *J* = 9.0, 5.9 Hz, 1H), 3.94 (d, *J* = 13.8 Hz, 1H), 3.79 (d, *J* = 13.6 Hz, 1H), 2.74 - 2.81 (m, 1H), 2.66 - 2.74 (m, 2H), 2.34 - 2.45 (m, 3H), 1.91 - 1.98 (m, 2H), 1.58 - 1.68 (m, 2H).

### Example 299. (S)-2-((4-((2-((4-Chloro-2-(fluoromethyl)phenoxy)methyl)pyridin-4-yl)oxy)piperidin-1-yl)methyl)-1-(oxetan-2-ylmethyl)-1H-benzo[d]imidazole-6-carboxylic acid (299)

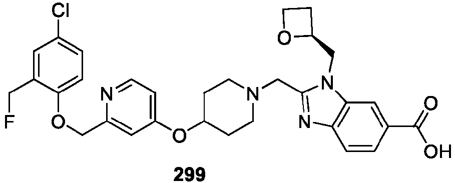

### Step A. tert-Butyl 4-((2-((4-chloro-2-(methoxycarbonyl)phenoxy)methyl)pyridin-4-yl)oxy)piperidine-1-carboxylate (299a)

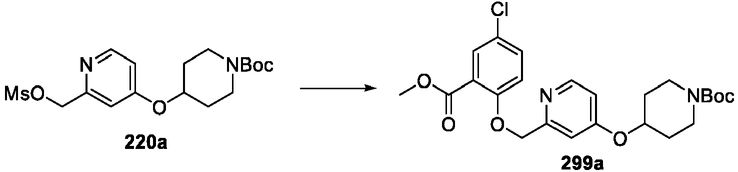

A solution of **220a** (1.0 g, 2.6 mmol), methyl 5-chloro-2-hydroxybenzoate (490 mg, 2.6 mmol) and K₂CO₃ (2.54 g, 7.8 mmol) in DMF (10 mL) was stirred at 50 °C for 5 h. After completion, the reaction was diluted with H₂O (30 mL) and extracted with EtOAc (30 mL x 3). The combined organic layer was dried over Na₂SO₄, filtered, concentrated and purified by silica gel column chromatography (petroleum ether:EtOAc = 7:3) to afford the title product **(299a)** (1.0 g, 77%) as a yellow oil. *m*/*z* (ESI, +ve ion) = 477.1 [M+H]⁺.

### Step B. tert-Butyl 4-((2-((4-chloro-2-(hydroxymethyl)phenoxy)methyl)pyridin-4-yl)oxy)piperidine-1-carboxylate (299b)

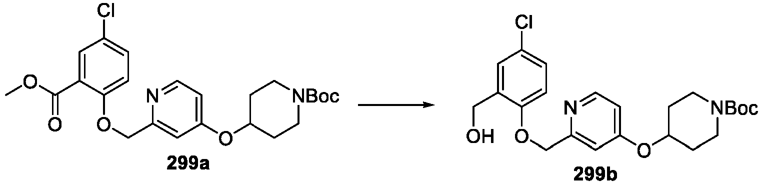

A solution of **299a** (1.0 g, 2.1 mmol) and LiAlH₄ (240 mg, 6.3 mmol) in THF (12 mL) was stirred at 0 °C for 1 h. After completion, the reaction was quenched with sodium sulfate decahydrate, filtered and the filter cake was rinsed by DCM (50 mL x 3). The combined organic layer was dried over Na₂SO₄, filtered, concentrated and purified by silica gel column chromatography (petroleum ether: EA =1:1) to afford the title product **(299b)** (712 mg, 71%) as yellow solid. *m*/*z* (ESI, +ve ion) = 449.1 [M+H]⁺.

### Step C. tert-Butyl 4-((2-((4-chloro-2-(fluoromethyl)phenoxy)methyl)pyridin-4-yl)oxy)piperidine-1-carboxylate (299c)

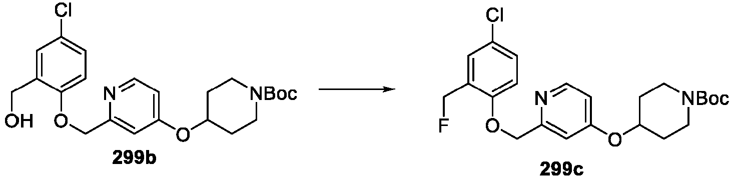

To a mixture of **299b** (200 mg, 0.445 mmol) in DCM (2 mL) was added DAST (144 mg, 0.891 mmol) at 0 °C. The mixture was stirred at 20 °C for 12 h under N₂. After completion, the reaction was diluted with EtOAc (10 mL) and H₂O (5 mL). The organic phase was separated and concentrated. The crude residue was purified with prep-TLC plate (EtOAc:petroleum ether = 1: 5) to give the title product **(299c)** (90 mg, 53.8 %) as an oil. *m*/*z* (ESI, +ve ion) = 451.2 [M+H]⁺.

### Step D. 2-((4-Chloro-2-(fluoromethyl)phenoxy)methyl)-4-(piperidin-4-yloxy)pyridine (299d)

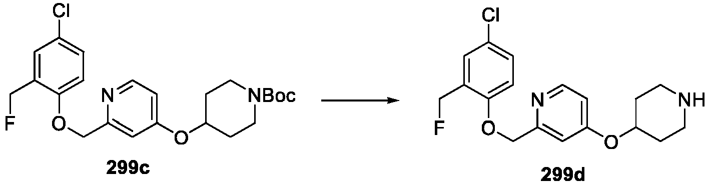

To a mixture of **299c** (120 mg, 0.266 mmol) in dioxane (1 mL) was added 4 N HCl in dioxane (0.33 mL, 1.33 mmol) at 0 °C. The mixture was stirred at 20 °C for 1 h under N₂. After completion, the mixture was concentrated to give the crude title product **(299d)** (90 mg) as a colorless oil. *m*/*z* (ESI, +ve ion) = 351.2 [M+H]⁺.

### Step E. (S)-2-((4-((2-((4-Chloro-2-(fluoromethyl)phenoxy)methyl)pyridin-4-yl)oxy)piperidin-1-yl)methyl)-1-(oxetan-2-ylmethyl)-1H-benzo[d]imidazole-6-carboxylic acid (299)

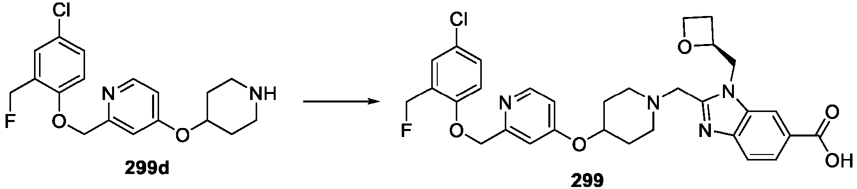

The title product **(299)** was synthesized from **299d** in similar procedures as described in Example **72,** Steps F and G. *m*/*z* (ESI, +ve ion) = 595.2 [M+H]⁺. ¹H NMR (400 MHz, DMSO-d₆) δ ppm 8.35 (d, *J* = 5.9 Hz, 1H), 8.16 (s, 1H), 7.78 (br d, *J* = 8.3 Hz, 1H), 7.53 (br d, *J* = 8.3 Hz, 1H), 7.47 (br s, 1H), 7.41 (br dd, *J* = 8.8, 2.0 Hz, 1H), 7.13 (br d, *J* = 8.8 Hz, 1H), 7.06 (br d, *J* = 2.0 Hz, 1H), 6.94 (br dd, *J* = 5.4, 2.5 Hz, 1H), 5.50 (d, *J* = 48.0 Hz, 2H), 5.18 (s, 2H), 5.03 - 5.12 (m, 1H), 4.73 (br dd, *J* = 15.2, 6.9 Hz, 1H), 4.44 - 4.65 (m, 3H), 4.36 (dt, *J* = 8.8, 5.9 Hz, 1H), 3.73 - 3.97 (m, 2H), 2.63 - 2.83 (m, 3H), 2.30 - 2.47 (m, 3H), 1.87 - 2.03 (m, 2H), 1.55 - 1.69 (m, 2H).

Examples **287** and **292** were synthesized in similar procedures from **299b** as described in Example **299,** Steps D to E. The mesylate analog of **57f** derived from **145e** was used in the second to last step for **287.**

### Example 300. 2-((4-(3-((4-Cyano-2-fluorophenoxy)methyl)phenoxy)piperidin-1-yl)methyl)-4-fluoro-1-((1-(fluoromethyl)cyclopropyl)methyl)-1H-benzo[d]imidazole-6-carboxylic acid (300)

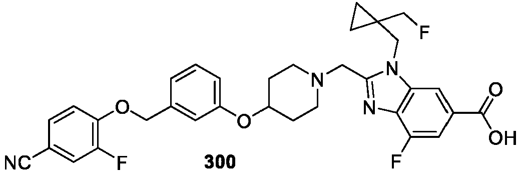

### Step A. Methyl 3-fluoro-5-(((1-(fluoromethyl)cyclopropyl)methyl)amino)-4-nitrobenzoate (300a)

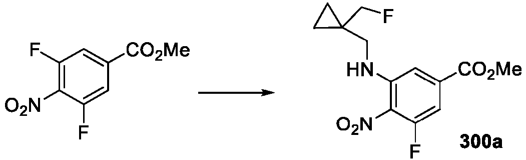

To a solution of methyl 3,5-difluoro-4-nitrobenzoate (500 mg, 2.3 mmol) and [1-(fluoromethyl)cyclopropyl]methanamine trifluoroacetic Acid (500 mg, 2.3 mmol) in anhydrous DMF (7.7 mL) was added DIPEA (1.0 mL, 5.8 mmol) at room temperature. After stirring for 0.5 h, the reaction was diluted with EtOAc (20 mL) and washed with brine (10 mL x 2). The organic layer was dried over Na₂SO₄, filtered, concentrated and purified by silica gel column chromatography (gradient elution, 0-40% EtOAc in hexanes) to afford the title product **(300a)** (481 mg, 70%) as an orange solid. *m*/*z* (ESI, +ve ion) = 301.4 [M + H]⁺.

### Step B. Methyl 4-amino-3-fluoro-5-(((1-(fluoromethyl)cyclopropyl)methyl)amino)benzoate (300b)

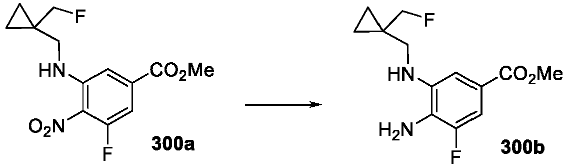

10% Pd/C (33 mg, 0.031 mmol) was added to **300a** (186 mg, 0.62 mmol) in EtOH (6.2 mL) at room temperature. The mixture was purged with hydrogen for 10 min, then stirred under a H₂ balloon for 1 h. The reaction was filtered through a pad of celite, rinsed with EtOAc, and concentrated to provide the crude title product **(300a)** (167 mg). *m*/*z* (ESI, +ve ion) = 271.4 [M + H]⁺.

### Step C. Methyl 2-(chloromethyl)-4-fluoro-1-((1-(fluoromethyl)cyclopropyl)methyl)-1H-benzo[d]imidazole-6-carboxylate (300c)

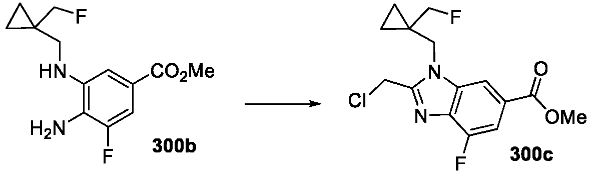

To a solution of crude **300b** (167 mg, 0.62 mmol) and 2-chloro-1,1,1-trimethoxyethane (0.25 mL, 287 mg, 1.86 mmol) in anhydrous CH₃CN (3.1 mL) at room temperature was added PTSA (6 mg, 0.03 mmol) and heated to 60 °C. After stirring for 30 min, the mixture was concentrated and purified by silica gel column chromatography (gradient elution, 0-80% EtOAc in hexanes) to afford the title product **(300c)** (194 mg, 95% over 2 steps) as a light brown oil. *m*/*z* (ESI, +ve ion) = 329.3 [M + H]⁺.

### Step D. tert-Butyl 4-(3-((4-cyano-2-fluorophenoxy)methyl)phenoxy)piperidine-1-carboxylate (300d)

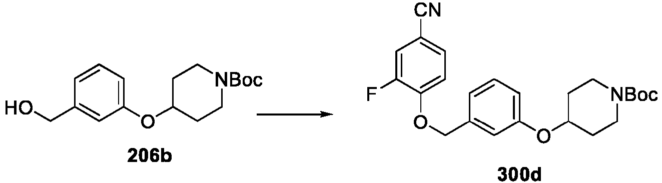

To a solution of **206b** (100 mg, 0.33 mmol) in anhydrous CH₂Cl₂ (1.6 mL) at 0 °C was added PPh₃ (3.29 g, 12.5 mmol), 3-fluoro-4-hydroxybenzonitrile (1.36 g, 8.35 mmol) followed by addition of a solution of DCAD (2.53 g, 12.5 mmol) in anhydrous CH₂Cl₂ (1.6 mL) dropsise. The resulting mixture was warmed to room temperature, stirred for 1 h, filtered and concentrated. The crude residue was purified by silica gel column chromatography (gradient elution, 0-40% EtOAc in hexanes) to provide the title product **(300d)** (130 mg) with some triphenylphosphine oxide impurities (130 mg). *m*/*z* (ESI, +ve ion) = 449.3 [M + Na]⁺.

### Step E. 3-Fluoro-4-((3-(piperidin-4-yloxy)benzyl)oxy)benzonitrile (300e)

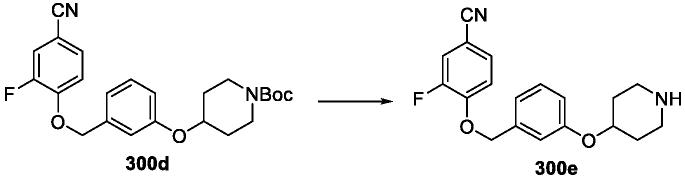

To a solution of **300d** (28 mg, 0.066 mmol) in anhydrous CH₂Cl₂ (0.66 mL) was added TFA (0.25 mL, 3.3 mmol) at room temperature. After stirring for 1 h, the mixture was concentrated and azeotroped with (2 mL x 2) to afford the crude title product **(300e)** (21 mg), which was used in the next step without further purification. *m*/*z* (ESI, +ve ion) = 327.3 [M + H]⁺.

### Step F. Methyl 2-((4-(3-((4-cyano-2-fluorophenoxy)methyl)phenoxy)piperidin-1-yl)methyl)-4-fluoro-1-((1-(fluoromethyl)cyclopropyl)methyl)-1H-benzo[d]imidazole-6-carboxylate (300f)

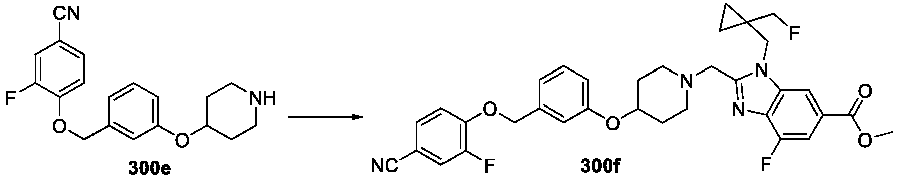

To a solution of crude **300e** (21 mg, 0.066 mmol) in dichloroethane (0.66 mL) was added **1h** (8 mg, 0.059 mmol) and DIPEA (0.034 mL, 0.20 mmol) at room temperature. The reaction was heated to 60 °C. After 20 h, the reaction was cooled down to room temperature, concentrated and purified by silica gel column chromatography (gradient elution, 0-60% EtOAc in hexanes) to afford the title product **(300f)** (24 mg, 59% over 2 steps) as a light brown oil. *m*/*z* (ESI, +ve ion) = 619.3 [M + H]⁺.

### Step G. 2-((4-(3-((4-Cyano-2-fluorophenoxy)methyl)phenoxy)piperidin-1-yl)methyl)-4-fluoro-1-((1-(fluoromethyl)cyclopropyl)methyl)-1H-benzo[d]imidazole-6-carboxylic acid (300)

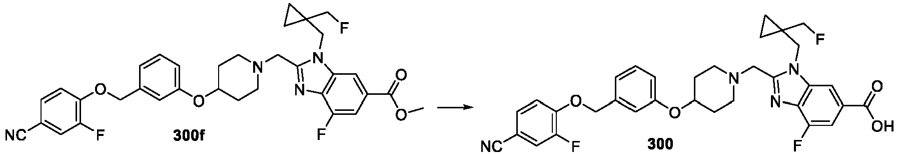

A flask charged with **300f** (24 mg, 0.039 mmol) was dissolved in THF/H₂O (1:1, 0.8 mL) at room temperature. LiOH.H₂O (8 mg, 0.19 mmol) and a few drops of MeOH were added to the mixture and heated to 40 °C. After 2.5 h, the reaction was cooled down and acidified with acetic acid to pH = 5. The solution was diluted with EtOAc (3 mL) and washed with brine (2 mL). The organic layer was dried over Na₂SO₄, filtered, concentrated and purified by silica gel column chromatography (gradient elution, 0-5% MeOH/DCM) to afford the title product **(300)** as a colorless film, which was further lyophilized to provide a white solid (11.6 mg, 49%). *m*/*z* (ESI, +ve ion) = 605.3 [M + H]⁺. ¹H NMR (600 MHz, CD₃OD) δ ppm 8.16 (d, *J* = 0.73 Hz, 1H), 7.64 (d, *J* = 11.00 Hz, 1H), 7.55 (dd, *J* = 10.82, 2.02 Hz, 1H), 7.50 (dd, *J* = 8.44, 1.47 Hz, 1H), 7.29 (td, *J* = 8.16, 1.65 Hz, 2H), 6.99 - 7.06 (m, 2 H), 6.92 (dd, *J*=8.25, 2.38 Hz, 1H), 5.23 (s, 2H), 4.70 (s, 2H), 4.46 (br dd, *J* = 7.15, 3.85 Hz, 1H), 4.08 - 4.22 (m, 2H), 3.99 (s, 2H), 2.81 - 2.87 (m, 2H), 2.47 - 2.54 (m, 2H), 1.98 - 2.07 (m, 2H), 1.77 - 1.84 (m, 2H), 0.89 (q, *J* = 5.26 Hz, 2H), 0.77 - 0.82 (m, 2H). *m*/*z* (ESI, +ve ion) = 605.3 [M + H]⁺.

Examples **301, 303, 310** were synthesized in similar procedures as described in Example **300,** except HCl in dioxane was used in deprotecting the Boc group at Step E.

### Example 304. (S)-2-((4-((2-((2,4-Dichlorophenoxy)methyl)pyrimidin-4-yl)oxy)piperidin-1-yl)methyl)-1-(oxetan-2-ylmethyl)-1H-benzo[d]imidazole-6-carboxylic acid (304)

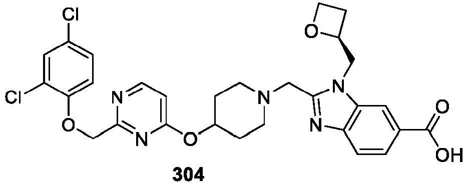

### Step A. Methyl (S)-2-((4-((2-((2,4-dichlorophenoxy)methyl)pyrimidin-4-yl)oxy)piperidin-1-yl)methyl)-1-(oxetan-2-ylmethyl)-1H-benzo[d]imidazole-6-carboxylate (304b)

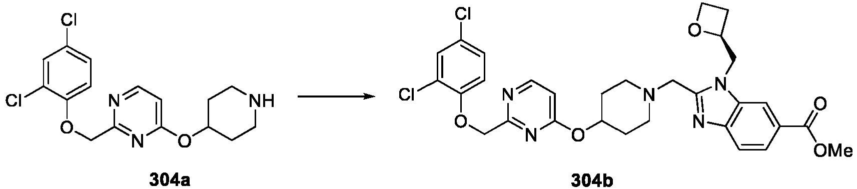

A solution of **304a** (TFA salt, prepared in similar procedures as **261c,** 24.0 mg, 0.051 mmol), **1h** (18.1 mg, 0.062 mmol) and DIPEA (170 µL, 1.03 mmol) in DMF (0.5 mL) was stirred at room temperature overnight. After completion, the reaction mixture was diluted with EtOAc (5 mL). The organic layer was washed with water (3 mL x 2), brine (3 mL), dried over Na₂SO₄, filtered and concentrated and the resulting crude residue was purified by silica gel column chromatography (gradient elution, 80-100% EtOAc/hexanes then 0-20% MeOH in DCM) to afford the title product **(304b)** (11.9 mg, 38%) as a pale-yellow oil. *m*/*z* (ESI, +ve ion) = 612.3 [M+H]⁺.

### Step B. (S)-2-((4-((2-((2,4-Dichlorophenoxy)methyl)pyrimidin-4-yl)oxy)piperidin-1-yl)methyl)-1-(oxetan-2-ylmethyl)-1H-benzo[d]imidazole-6-carboxylic acid (304)

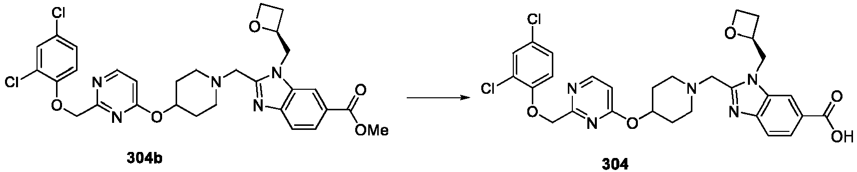

A solution of **304b** (11.9 mg, 0.019 mmol) and LiOH (4.1 mg, 0.097 mmol) in THF:MeOH:H₂O = 2:1:1 (0.4 mL) was stirred at 30 °C overnight. After completion, the reaction mixture was diluted with water to 1 mL and purified by reverse phase HPLC (gradient elution, 20-45% CH₃CN in H₂O, with 0.05% NH₄OH as a modifier) to afford the title product **(304)** (10.5 mg, 88%) as a white solid. ¹H NMR (600 MHz, DMSO-d₆) δ ppm 8.49 (d, *J* = 5.9 Hz, 1H), 8.22 (s, 1H), 7.79 (dd, *J* = 8.4, 1.5 Hz, 1H), 7.60 (d, *J* = 8.4 Hz, 1H), 7.57 (d, *J* = 2.6 Hz, 1H), 7.28 (dd, *J* = 8.8, 2.6 Hz, 1H), 7.11 (d, *J* = 8.8 Hz, 1H), 6.8 (d, *J* = 5.9 Hz, 1H), 5.37 (s, 2H), 5.07 (qd, *J* = 7.2, 3.1 Hz, 1H), 4.85 (dt, *J* = 8.7, 4.6 Hz, 1H), 4.75 (br dd, *J* = 15.2, 7.2 Hz, 1H), 4.61 (br dd, *J* = 15.2, 2.8 Hz, 1H), 4.46 - 4.51 (m, 1H), 4.36 (dt, *J* = 8.8, 5.9 Hz, 1H), 3.91 (d, *J* = 13.6 Hz, 1H), 3.76 (d, *J* = 13.6 Hz, 1H), 2.65 - 2.80 (m, 2H), 2.36 - 2.46 (m, 2H), 2.14 - 2.25 (m, 2H), 1.76 - 1.85 (m, 2H), 1.50 - 1.60 (m, 2H). *m*/*z* (ESI, +ve ion) = 598.2 [M+H]⁺.

Examples **305, 306, 307, 338, 340, 341, 342, 348, 349** were synthesized in similar procedures as described in Example **304.**

Example **369, 370, 371, 373** were synthesized in similar procedures as described in Example **304,** except used a silica gel column to purify at the last step.

### Example 313. 2-(((2S,4S)-4-((2-((4-Cyano-2-fluorophenoxy)methyl)pyrimidin-4-yl)oxy)-2-methylpiperidin-1-yl)methyl)-1-(((S)-oxetan-2-yl)methyl)-1H-benzo[d]imidazole-6-carboxylic acid (313)

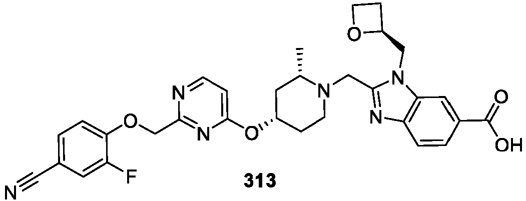

### Step A. tert-Butyl (2S,4S)-4-((2-((4-cyano-2-fluorophenoxy)methyl)pyrimidin-4-yl)oxy)-2-methylpiperidine-1-carboxylate (313a)

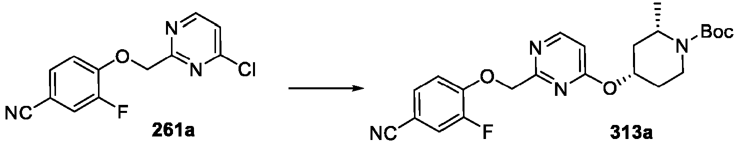

To a solution of **261a** (50 mg, 0.19 mmol), *tert-*butyl (*2S*,*4S*)-4-hydroxy-2-methylpiperidine-1-carboxylate (49 mg, 0.23 mmol) in anhydrous toluene (1.9 mL) was added Pd₂(dba)₃ (7.7 mg, 0.0095 mmol), BINAP (11.8 mg, 0.019 mmol), and Cs₂CO₃ (124 mg, 0.38 mmol) at room temperature. The mixture was flushed with argon for 15 min, then was heated to 90 °C. After 12 h, the reaction was cooled down to room temperature and H₂O (2 mL) was added. The reaction was extracted with EtOAc (3 mL x 3) and the combined organic layer was dried over Na₂SO₄, filtered, concentrated and the crude residue was purified by silica gel column chromatography (gradient elution, 0-50% EtOAc in hexanes) to afford the title product **(313a)** (89 mg, quantitative) *m*/*z* (ESI, +ve ion) = 443.3 [M+H]⁺.

### Step B. 2-(((2S,4S)-4-((2-((4-Cyano-2-fluorophenoxy)methyl)pyrimidin-4-yl)oxy)-2-methylpiperidin-1-yl)methyl)-1-(((S)-oxetan-2-yl)methyl)-1H-benzo[d]imidazole-6-carboxylic acid (313)

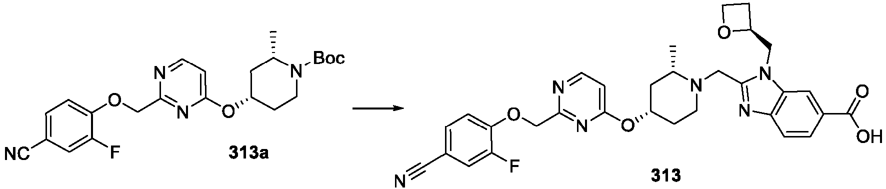

The title product **(313)** was synthesized in similar procedures as described in Example **300,** Steps E to G. *m*/*z* (ESI, +ve ion) = 587.4 [M+H]⁺. ¹H NMR (400 MHz, CD₃OD) δ ppm 8.42 (d, *J* = 5.87 Hz, 1H), 8.31 (d, *J* = 0.98 Hz, 1H), 7.97 (dd, *J* = 8.56, 1.47 Hz, 1H), 7.69 (d, *J* = 8.56 Hz, 1H), 7.60 (dd, *J* = 11.00, 1.96 Hz, 1H), 7.47 (dt, *J*=8.56, 1.59 Hz, 1H), 7.22 (t, *J* = 8.56 Hz, 1H), 6.70 (d, *J* = 5.87 Hz, 1H), 5.40 (s, 2H), 5.24 - 5.32 (m, 1H), 4.91 - 4.96 (m, 1H), 4.73 (dd, *J* = 15.53, 5.75 Hz, 1H), 4.56 - 4.65 (m, 3H), 4.31 (dt, *J* = 9.29, 5.87 Hz, 1H), 3.60 (d, *J* = 13.94 Hz, 1H), 2.70 - 2.83 (m, 2H), 2.35 - 2.51 (m, 2H), 2.19 (td,*J*=12.23, 1.96 Hz, 1H), 1.82 - 2.01 (m, 2H), 1.40 - 1.65 (m, 2H), 1.20 (d, *J*=6.11 Hz, 3H).

Examples **311, 312, 314, 323, 324, 325, 326, 327, 329, 353, 375, 376, 377** were synthesized in similar procedures as described in Example **313.** HCl in dioxane was used to remove Boc group for Examples **311** and **312.**

Example **319** was synthesized from methyl 3-hydroxybenzoate and *tert-*butyl (*2S*,*4R*)-4-hydroxy-2-methylpiperidine-1-carboxylate in similar procedures as described in Example **206,** Steps A and B, followed by Example **300,** Steps D to G.

Example **320** was synthesized in similar procedures as described in Example **319.**

### Example 330. (S)-2-((4-((6-((4-Cyano-2-fluorophenoxy)methyl)-3-fluoropyridin-2-yl)oxy)piperidin-1-yl)methyl)-1-(oxetan-2-ylmethyl)-1H-benzo[d]imidazole-6-carboxylic acid (330)

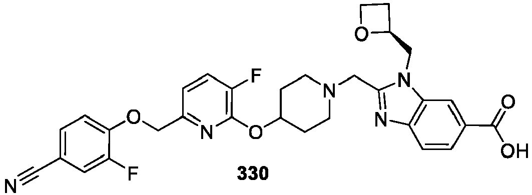

### Step A. Methyl 6-((1-(tert-butoxycarbonyl)piperidin-4-yl)oxy)-5-fluoropicolinate (330a)

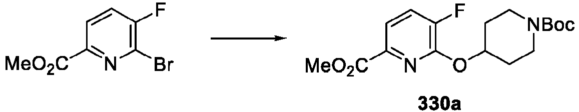

To a solution of methyl 6-bromo-5-fluoropyridine-2-carboxylate (100 mg, 0.43 mmol), *tert-*butyl 4-hydroxypiperidine-1-carboxylate (103 mg, 0.51 mmol) in anhydrous toluene (4.3 mL) was added Pd₂(dba)₃ (17 mg, 0.021 mmol), BINAP (27 mg, 0.043 mmol), and Cs₂CO₃ (278 mg, 0.86 mmol) at room temperature. The reaction was flushed with argon for 15 min, then heated to 90 °C. After 12 h, the reaction was cooled down to room temperature and H₂O (2 mL) was added. The reaction mixture was extracted with EtOAc (3 mL x 3) and the combined organic layer was dried over Na₂SO₄, filtered, concentrated and the crude residue was purified by silica gel column chromatography (gradient elution, 0-50% EtOAc in hexanes) to afford the title product **(330a)** (105 mg, 69%) as a colorless oil. *m*/*z* (ESI, +ve ion) = 377.3 [M + Na]⁺.

### Step B. tert-Butyl 4-((3-fluoro-6-(hydroxymethyl)pyridin-2-yl)oxy)piperidine-1-carboxylate (330b)

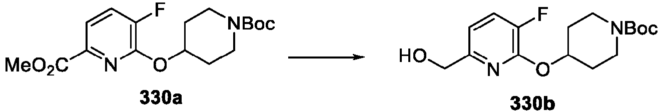

To a solution of **330a** (103 mg, 0.29 mmol) in anhydrous THF (2.9 mL) was added LiAlH₄ (0.44 mL, 0.44 mmol, 1 M in THF) at 0 °C. After 0.5 h, EtOAc (3 mL) was added dropwise and the reaction was diluted with half-saturated Rochelle salt solution (2 mL). The reaction mixture was extracted with EtOAc (5 mL x 3) and the combined organic layer was dried over Na₂SO₄, filtered, concentrated and the crude residue was purified by silica gel column chromatography (gradient elution, 0-60% EtOAc in hexanes) to afford the title product **(330b)** (78 mg, 82%) as a colorless oil. *m*/*z* (ESI, +ve ion) = 349.4 [M + Na]⁺.

### Step C. (S)-2-((4-((6-((4-Cyano-2-fluorophenoxy)methyl)-3-fluoropyridin-2-yl)oxy)piperidin-1-yl)methyl)-1-(oxetan-2-ylmethyl)-1H-benzo[d]imidazole-6-carboxylic acid (330)

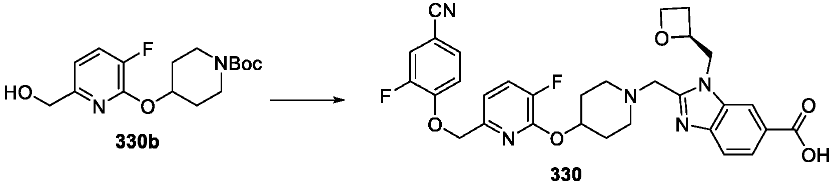

The title product **(330)** was synthesized in similar procedures as described in Example **300,** Steps D to G. *m*/*z* (ESI, +ve ion) = 590.3 [M+H]⁺. ¹H NMR (600 MHz, CD₃OD) δ ppm 8.33 (d, *J* = 1.10 Hz, 1H), 7.97 (dd, *J* = 8.62, 1.65 Hz, 1H), 7.67 (d, *J* = 8.44 Hz, 1H), 7.57 (dd, *J* = 10.82, 2.02 Hz, 1H), 7.45 - 7.53 (m, 2 H), 7.34 (t, *J* = 8.44 Hz, 1H), 7.07 (dd, *J* = 8.07, 2.93 Hz, 1H), 5.26 (qd, *J*=7.21, 2.57 Hz, 1H), 5.23 (s, 2H), 5.14 (dt, *J*=7.79, 3.99 Hz, 1H), 4.88 (dd, *J* = 15.41, 7.34 Hz, 1H), 4.72 (dd, *J* = 15.41, 2.57 Hz, 1H), 4.65 (td, *J* = 7.89, 5.87 Hz, 1H), 4.47 (dt, *J*=9.17, 6.05 Hz, 1H), 3.92 - 4.08 (m, 2H), 2.78 - 2.91 (m, 3H) 2.44 - 2.57 (m, 3H), 2.00 - 2.07 (m, 2H), 1.79 - 1.90 (m, 2H).

Example **347** was synthesized in similar procedures as described in Example **330.**

### Example 355. 2-(((2S,4S)-4-((2-((2-cyano-4-fluorophenoxy)methyl)pyrimidin-4-yl)oxy)-2-methylpiperidin-1-yl)methyl)-1-(((S)-oxetan-2-yl)methyl)-1H-benzo[d]imidazole-6-carboxylic acid (355)

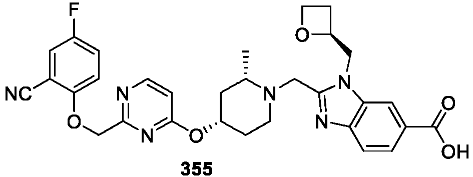

### Step A. tert-Butyl (2S,4S)-4-((2-chloropyrimidin-4-yl)oxy)-2-methylpiperidine-1-carboxylate (355a)

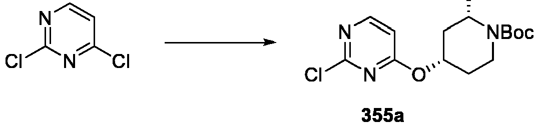

To a solution of 2,4-dichloropyrimidine (5.20 g, 34.90 mmol) and *tert-*butyl (*2S*,*4S*)-4-hydroxy-2-methyl-piperidine-1-carboxylate (7.51 g, 34.90 mmol) in DMF (50 mL) was added Cs₂CO₃ (17.06 g, 52.4 mmol). The reaction was stirred at 80 °C for 3 h. After completion, the reaction was diluted with H₂O (200 mL) and extracted with EtOAc (200 mL x 3). The combined organic layer was dried over Na₂SO₄ and concentrated to afford a crude residue, which was purified by silica gel column chromatography (gradient elution, 2.5 to 5% EtOAc in petroleum ether) to afford the title product **(355a)** (5.65 g, 48%) as a white solid. *m*/*z* (ESI, +ve ion) = 328.1 [M+H]⁺.

### Step B. Methyl 4-(((2S,4S)-1-(tert-butoxycarbonyl)-2-methylpiperidin-4-yl)oxy)pyrimidine-2-carboxylate (355b)

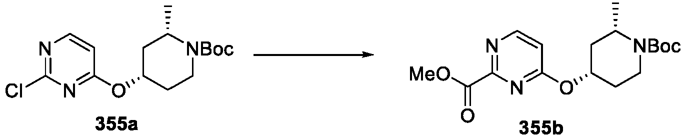

A mixture of **355a** (7.20 g, 21.96 mmol), Pd(dppf)Cl₂ (803.57 mg, 1.10 mmol), Et₃N (3.06 mL, 21.96 mmol) in MeOH (70 mL) and DMSO (35 mL) was degassed and purged with N₂ 3 times. The reaction was heated at 60 °C for 16 h under a CO (50 Psi) atmosphere. After completion, the reaction was concentrated, diluted with H₂O (200 mL) and extracted with EtOAc (200 mL x 3). The combined organic layer was washed with brine (100 mL x 3), dried over Na₂SO₄, filtered, concentrated and purified by silica gel column chromatography (gradient elution, 2-20% EtOAc in petroleum ether) to afford the title product **(355b)** (4.0 g, 52%) as a white solid. *m*/*z* (ESI, +ve ion) = 352.2 [M+H]⁺.

### Step C. tert-Butyl (2S,4S)-4-((2-(hydroxymethyl)pyrimidin-4-yl)oxy)-2-methylpiperidine-1-carboxylate (355c)

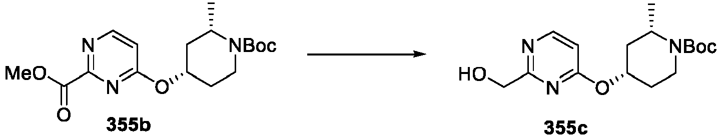

A mixture of **355b** (4.50 g, 12.81 mmol), CaCl₂ (1.42 g, 12.81 mmol) in EtOH (90 mL) was degassed and purged with N₂ 3 times followed by addition of NaBH₄ (484.45 mg, 12.81 mmol) at 0 °C. It was allowed to stir at 20 °C for 1 h under a N₂ atmosphere. After completion, reaction mixture was cooled to 0 °C, followed by addition of MeOH (50 mL), and then concentrated. The resulting residue was diluted with H₂O (100 mL) and the aqueous layer was extracted with EtOAc (100 mL x 3). The combined organic layer was dried over Na₂SO₄, filtered, concentrated and purified by silica gel column chromatography (gradient elution, 5-100% EtOAc in hexanes) to afford the title product **(355c)** (2.7 g, 65% yield) as a white solid, *m*/*z* (ESI, +ve ion) = 324.1 [M+H]⁺.

### Step D. tert-Butyl (2S,4S)-2-methyl-4-((2-(((methylsulfonyl)oxy)methyl)pyrimidin-4-yl)oxy)piperidine-1-carboxylate (355d)

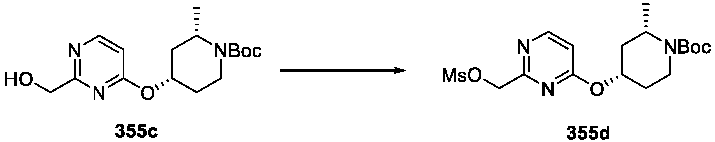

Et₃N (174 µL, 1.24 mmol) and MsCl (57.4 µL, 0.74 mmol) were added to an ice-cold solution of **355c** (200.0 mg, 0.062 mmol) in dichloromethane (3.1 mL). The reaction was stirred at 0 °C for 1 h. After completion, reaction was quenched with saturated aqueous NaHCO₃ (10 mL) and extracted with EtOAc (10 mL x 3). The combined organic layer was washed with brine (10 mL), dried over Na₂SO₄, filtered and concentrated to afford the crude title product **(355d)** (248.0 mg) as a pale-yellow oil, which was used in the next step without further purification. *m*/*z* (ESI, +ve ion) = 402.3 [M+H]⁺.

### Step E. tert-Butyl (2S,4S)-4-((2-((2-cyano-4-fluorophenoxy)methyl)pyrimidin-4-yl)oxy)-2-methylpiperidine-1-carboxylate (355e)

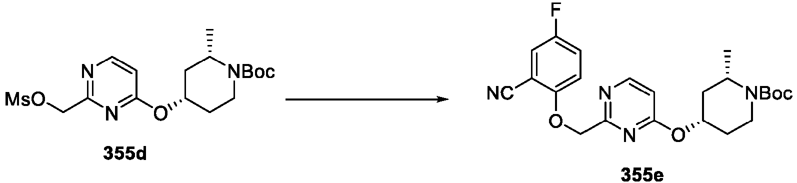

To a solution of **355d** (248.0 mg, 0.62 mmol) and 5-fluoro-2-hydroxybenzonitrile (88.9 mg, 0.65 mmol) in DMF (3.1 mL) was added K₂CO₃ (171.0 mg, 1.24 mmol). The reaction was stirred at room temperature for 2 h. Upon completion, the reaction was quenched by addition of water (5 mL) and extracted with EtOAc (10 mL x 3). The combined organic layer was washed with water (10 mL), brine (10 mL x 3), dried over Na₂SO₄, filtered, concentrated and the resulting crude residue was purified by silica gel column chromatography (gradient elution, 0-40% EtOAc in hexanes) to afford the title product **(355e)** (164.0 mg, 60%) as a yellow. *m*/*z* (ESI, +ve ion) = 443.4 [M+H]⁺.

### Step F. 5-Fluoro-2-((4-(((2S,4S)-2-methylpiperidin-4-yl)oxy)pyrimidin-2-yl)methoxy)benzonitrile (355f)

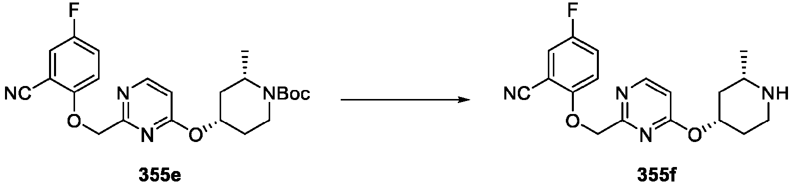

A solution of **355e** (164.0 mg, 0.37 mmol) in DCM (6 mL) and TFA (3 mL) was stirred at room temperature for 10 min. After completion, the reaction solvent was removed under reduced pressure and the resulting residue was diluted with EtOAc (10 mL). The organic layer was washed with sat. NaHCO₃ (5 mL), brine (3 mL), dried over Na₂SO₄, filtered and concentrated to afford the title product **(355f)** (127 mg) as a colorless oil, which was used in the next step without further purification. *m*/*z* (ESI, +ve ion) = 343.4 [M+H]⁺

### Step G. Methyl 2-(((2S,4S)-4-((2-((2-cyano-4-fluorophenoxy)methyl)pyrimidin-4-yl)oxy)-2-methylpiperidin-1-yl)methyl)-1-(((S)-oxetan-2-yl)methyl)-1H-benzo[d]imidazole-6-carboxylate (355g)

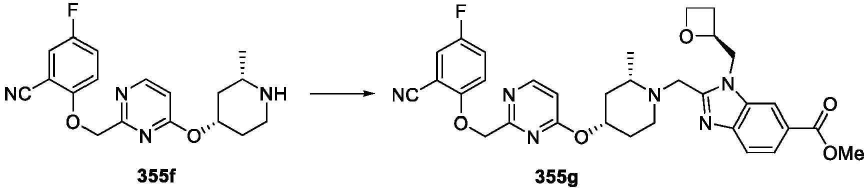

A solution of **355d** (127.0 mg, 0.37 mmol), **1h** (186.0 mg, 0.63 mmol) and DIPEA (323 µL, 1.85 mmol) in DMF (1.2 mL) was stirred at 70 °C overnight. After completion, the reaction mixture was diluted with EtOAc (20 mL). The organic layer was washed with water (10 mL x 2), brine (10 mL x 3), dried over Na₂SO₄, filtered and concentrated and the resulting crude residue was purified by silica gel column chromatography (gradient elution, 0-20% acetone in DCM) to afford the title product **(355g)** (160.0 mg, 72%) as a colorless oil. m/z (ESI, +ve ion) = 601.3 [M+H]⁺.

### Step H. 2-(((2S,4S)-4-((2-((2-Cyano-4-fluorophenoxy)methyl)pyrimidin-4-yl)oxy)-2-methylpiperidin-1-yl)methyl)-1-(((S)-oxetan-2-yl)methyl)-1H-benzo[d]imidazole-6-carboxylic acid (355)

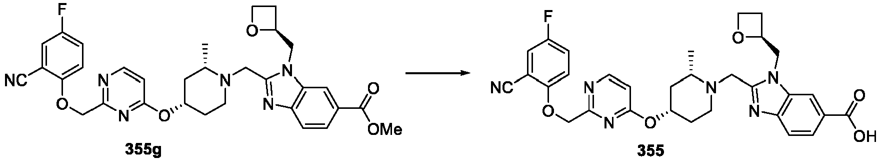

A solution of **355g** (160.0 mg, 0.27 mmol) and LiOH (112.0 mg, 2.66 mmol) in THF:MeOH:H₂O = 2:1:1 (12 mL) was stirred at 30 °C for 2 h. After completion, the reaction mixture was diluted with water (20 mL) and neutralized with glacial acetic acid (0.16 mL) until pH = 7. Then the reaction was extracted with 30% IPA in chloroform (30 mL x 3). The combined organic layer was dried over Na₂SO₄, filtered, concentrated and purified by silica gel column chromatography (gradient elution, 0-10% MeOH in DCM) to afford the title product **(355)** (122.0 mg, 76%) as a colorless oil. *m*/*z* (ESI, +ve ion) = 587.2 [M+H]⁺. ¹H NMR (400 MHz, DMSO-d₆) δ ppm 8.48 (d, *J* = 5.8 Hz, 1H), 8.24 (s, 1H), 7.80 (dd, *J* = 8.3, 1.5 Hz, 1H), 7.76 (dd, *J* = 8.0, 3.3 Hz, 1H), 7.65 (d, *J =* 8.3, Hz, 1H), 7.48 (ddd, *J =* 9.4, 8.3, 3.1 Hz, 1H), 7.22 (dd, *J* = 9.5, 4.0 Hz, 1H), 6.77 (d, *J =* 5.8 Hz, 1H), 5.75 (s, 2H), 5.46 (s, 2H), 5.08 - 5.18 (m, 1H), 4.65 - 4.85 (m, 2H), 4.36 - 4.51 (m, 2H), 4.20 - 4.29 (m, 1H), 3.55 (br d, *J =* 14.1 Hz, 1H), 2.58 - 2.77 (m, 2H), 2.29 - 2.44 (m, 2H), 2.05 - 2.22 (m, 1H), 1.73 - 1.90 (m, 1H), 1.36 - 1.52 (m, 1H), 1.20 - 1.34 (m, 1H), 1.09 (d, *J =* 6.3 Hz, 3H).

### Example 367. (S)-2-((4-((2-((4-Cyano-2-fluorophenoxy)methyl)-5-fluoropyridin-4-yl)oxy)piperidin-1-yl)methyl)-1-(oxetan-2-ylmethyl)-1H-benzo[d]imidazole-6-carboxylic acid (367)

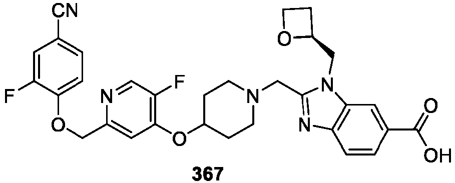

### Step A. Methyl 4-((1-(tert-butoxycarbonyl)piperidin-4-yl)oxy)-5-fluoropicolinate (367b)

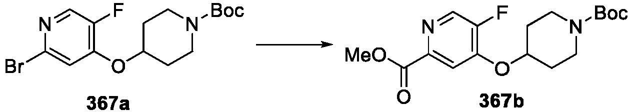

To a solution of **367a** (prepared similar to **145a** from 2-bromo-5-fluoropyridin-4-ol, 360 mg, 0.96 mmol), palladium diacetate (43 mg, 0.19 mmol) and DPPP (79 mg, 0.19 mmol) in DMSO/MeOH (15 mL) was added Et₃N (968 mg, 9.57 mmol). The reaction mixture was purged with CO and stirred at 80 °C for 4 h. After completion, the reaction was diluted with H₂O (25 mL) and extracted with EtOAc (30 mL x 3). The combined organic layer was dried, filtered, concentrated and purified by silica gel column chromatography (DCM:MeOH = 20:1) to afford the title product **(367b)** (230 mg, 64%) as a white solid. *m*/*z* (ESI, +ve ion) = 355.2 [M+H]⁺.

### Step B. tert-Butyl 4-((5-fluoro-2-(hydroxymethyl)pyridin-4-yl)oxy)piperidine-1-carboxylate (367b)

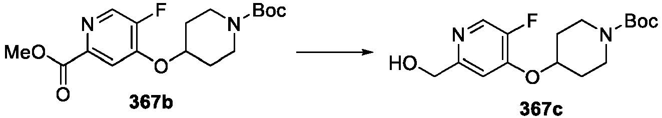

A flask was charged with **367b** (200 mg, 0.56 mmol) and added THF (10 mL). LiAlH₄ (1.1 mL, 1 M in THF) was added at 0 °C under nitrogen. The reaction was stirred at 0 °C for 2 h. After completion, the reaction was quenched with saturated NH₄Cl aqueous solution (15 mL) and extracted with EtOAc (20 mL x 3). The organic layer was dried, filtered and concentrated to afford the crude title product **(367c)** (93 mg, 48%) as a white solid, which was used in the next step without further purification. *m*/*z* (ESI, +ve ion) = 327.1 [M+H]⁺.

### Step C. tert-Butyl 4-((5-fluoro-2-(((methylsulfonyl)oxy)methyl)pyridin-4-yl)oxy)piperidine-1-carboxylate (367d)

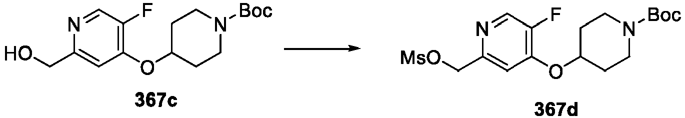

To a solution of **367c** (40 mg, 0.12 mmol), methanesulfonic anhydride (53 mg, 0.31 mmol) in DCM (2 mL) was added DIPEA (47 mg, 0.37 mmol). The reaction mixture was stirred at 25 °C for 2 h. After completion, the reaction solution was diluted with H₂O (10 mL) and extracted with EtOAc (10 mL x 3). The organic layer was dried, filtered and concentrated to afford the crude title product **(367d)** (43 mg, 82%) as a yellow oil. *m*/*z* (ESI, +ve ion) = 405.1 [M+H]⁺.

### Step D. tert-Butyl 4-((2-((4-cyano-2-fluorophenoxy)methyl)-5-fluoropyridin-4-yl)oxy)piperidine-1-carboxylate (367e)

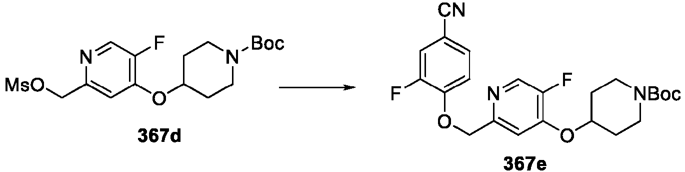

To a solution of **367d** (50 mg, 0.12 mmol), 3-fluoro-4-hydroxybenzonitrile (20 mg, 0.15 mmol) in DMF (2 mL) was added K₂CO₃ (51 mg, 0.37 mmol). The reaction was stirred at 80 °C for 2 h. After completion, the reaction was diluted with H₂O (15 mL) and extracted with EtOAc (20 mL x 3). The combined organic layer was dried, filtered, concentrated and purified by silica gel column chromatography (DCM:MeOH = 30:1) to afford the title product (**367e**) (48 mg, 83%) as a white solid. *m*/*z* (ESI, +ve ion) = 446.1 [M+H]⁺.

### Step E. 3-Fluoro-4-((5-fluoro-4-(piperidin-4-yloxy)pyridin-2-yl)methoxy)benzonitrile (367f)

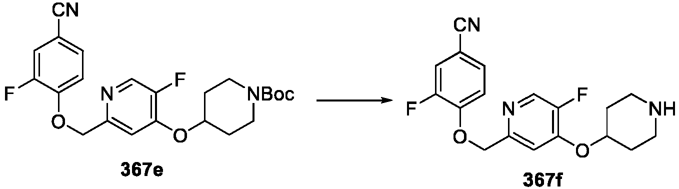

A solution of **367e** (40 mg, 0.09 mmol) in 4 N HCl in dioxane (1 mL) was stirred at 25 °C for 2 h. After completion, 1 N NaOH was added to the mixture to adjust to pH = 8 in an ice bath. The reaction mixture was then extracted with EtOAc (15 mL x 3). The organic layer was dried, filtered, concentrated and purified by silica gel column chromatography (DCM:MeOH = 20:1) to afford the title product **(367f)** (30 mg, 92%) as a white solid, which was used in the next step without further purification. *m*/*z* (ESI, +ve ion) = 346.1 [M+H]⁺.

### Step F. Methyl (S)-2-((4-((2-((4-Cyano-2-fluorophenoxy)methyl)-5-fluoropyridin-4-yl)oxy)piperidin-1-yl)methyl)-1-(oxetan-2-ylmethyl)-1H-benzo[d]imidazole-6-carboxylate (367g)

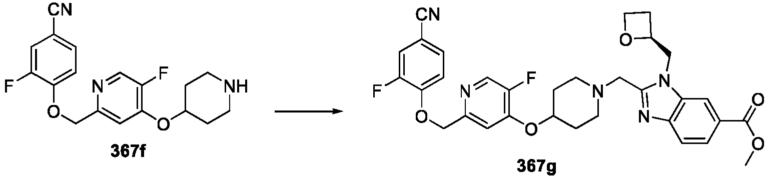

To a solution of **367f** (30 mg, 0.09 mmol), **1h** (31 mg, 0.11 mmol) in DMF (1 mL) was added K₂CO₃ (36 mg, 0.26 mmol). The reaction mixture was stirred at 25 °C for 2 h. After completion, the reaction solution was diluted with H₂O (15 mL) and extracted with EtOAc (20 mL x 3). The combined organic layer was dried, filtered, concentrated and purified by silica gel column chromatography (DCM:MeOH = 20:1) to afford the title product **(367g)** (34 mg, 62%) as a white solid. *m*/*z* (ESI, +ve ion) = 604.3 [M+H]⁺.

### Step G. (S)-2-((4-((2-((4-Cyano-2-fluorophenoxy)methyl)-5-fluoropyridin-4-yl)oxy)piperidin-1-yl)methyl)-1-(oxetan-2-ylmethyl)-1H-benzo[d]imidazole-6-carboxylic acid (367)

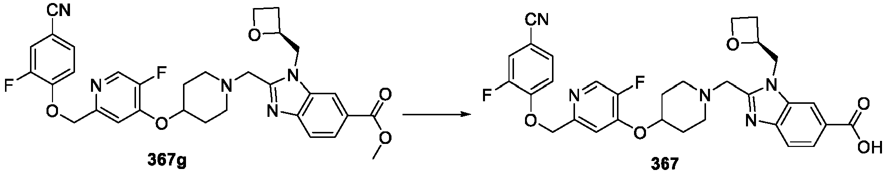

To a solution of **367g** (30 mg, 0.05 mmol) in THF (1.5 mL) was added a solution of lithium hydroxide (12 mg, 0.5 mmol) in H₂O (0.5 mL). The reaction was stirred at 25 °C for 2 h. After completion, 1 N HCl was added to the reaction to adjust to pH= 5 in an ice bath. The reaction mixture was then extracted with EtOAc (15 mL x 3). The organic layer was dried, filtered, concentrated and purified by silica gel column chromatography (EtOAc in petroleum ether, 20-30%) to afford the title product **(367)** (24 mg, 78% yield) as a white solid. *m*/*z* (ESI, +ve ion) = 590.3 [M+H]⁺. ¹H NMR (400 MHz, CD₃OD) δ ppm 8.27 - 8.37 (m, 2H), 7.96 (d, *J =* 8.56 Hz, 1H), 7.66 (d, *J* = 8.31 Hz, 1H), 7.57 (dd, *J =* 10.88, 1.83 Hz, 1H), 7.52 (br d, *J* = 8.56 Hz, 1 H), 7.38 (d, *J* = 6.85 Hz, 1H) 7.33 (t, *J* = 8.44 Hz, 1H), 5.18 - 5.34 (m, 3H), 4.65-4.75 (m, 3H), 4.46 (dt, *J=* 9.05, 5.87 Hz, 1H) 3.89 - 4.09 (m, 2H), 3.68 - 3.78 (m, 1H), 2.72 - 2.91 (m, 3H), 2.24 - 2.58 (m, 3H), 1.98 - 2.13 (m, 2H), 1.79 - 1.94 (m, 2H).

Example **380** was synthesized in similar procedure as described in Example **367.**

### Example 368. 2-((4-((2-((4-Chloro-2-fluorophenoxy)methyl)pyridin-4-yl)oxy)piperidin-1-yl)methyl)-1-((1-methoxycyclopropyl)methyl)-1H-benzo[d]imidazole-6-carboxylic acid (368)

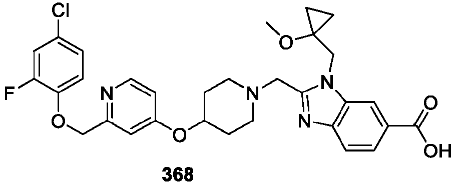

### Step A. Methyl 2-((4-((2-((4-chloro-2-fluorophenoxy)methyl)pyridin-4-yl)oxy)piperidin-1-yl)methyl)-1-((1-methoxycyclopropyl)methyl)-1H-benzo[d]imidazole-6-carboxylate (368b)

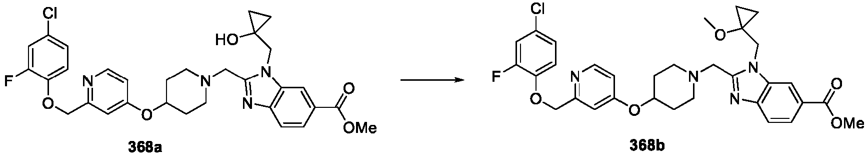

An ice-cold solution of **368a** (an intermediate in the synthesis of **345,** 200 mg, 0.34 mmol) in DMF (1 mL) was degassed and purged with N₂ for 3 times, and NaH (26.89 mg, 0.67 mmol, 60% dispersion in mineral oil) was added. After stirring for 30 min at 0 °C, MeI (25.1 µL, 0.40 mmol) was added. The reaction was warmed to room temperature and stirred for 3.5 h under a N₂ atmosphere. After completion, reaction was quenched with saturated aqueous NH₄Cl (20 mL) and extracted with EtOAc (30 mL x 3). The combined organic layer was dried over Na₂SO₄, filtered, concentrated and purified by reverse phase HPLC (gradient elution, 35-65% CH₃CN in H₂O, with 0.05% NH₄HCO₃ as a modifier) to afford the title product **(368b)** (80.0 mg, 39%) as a white solid. *m*/*z* (ESI, +ve ion) =609.2.

### Step B. 2-((4-((2-((4-Chloro-2-fluorophenoxy)methyl)pyridin-4-yl)oxy)piperidin-1-yl)methyl)-1-((1-methoxycyclopropyl)methyl)-1H-benzo[d]imidazole-6-carboxylic acid (368)

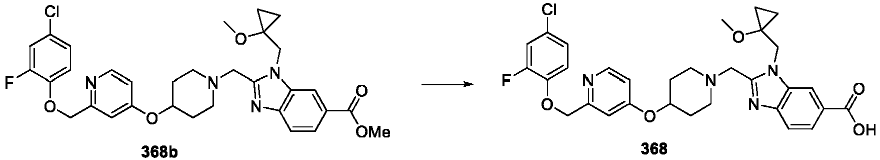

To a solution of **368b** (25 mg, 0.041 mmol) in THF (0.6 mL), MeOH (0.3 mL) and H₂O (0.3 mL) was added LiOH.H₂O (5.17 mg, 0.12 mmol). The reaction was stirred at 20 °C for 16 h. After completion, the reaction solvent was removed under reduced pressure. The resulting residue was diluted with water (2.0 mL) and then neutralized with saturated aqueous citric acid until pH = 7. A white precipitation was formed. It was filtered and dried to afford the title product **(368)** (13.5 mg, 55% yield) as a white solid. *m*/*z* (ESI, +ve ion) =595.2. ¹H NMR (400 MHz, DMSO-d₆) δ ppm 8.36 (d, *J =* 5.5 Hz, 1H), 8.27 (s, 1H), 7.80 (d, *J =* 8.5 Hz, 1H), 7.65 (d, *J* = 8.5 Hz, 1H), 7.46 (dd, *J =* 11.0, 2.5 Hz, 1H), 7.17 - 7.30 (m, 2H), 7.05 (d, *J* = 2.0 Hz, 1H), 6.97 (dd, *J =* 5.6, 2.3 Hz, 1H), 5.18 (s, 2H), 4.73 (s, 2H), 4.59 (br s, 1H), 3.92 (br s, 2H), 3.21 (s, 3H), 2.69 - 2.84 (m, 2H), 2.57 - 2.65 (m, 2H), 1.91 - 2.04 (m, 2H), 1.66 (br d, *J =* 9.5 Hz, 2H), 0.83 (br d, *J =* 10.5 Hz, 4H).

### Example 372. (S)-2-((4-((6-((4-Cyano-2-fluorophenoxy)methyl)pyridin-2-yl)oxy)piperidin-1-yl)methyl)-3-(oxetan-2-ylmethyl)-3H-imidazo[4,5-b]pyridine-5-carboxylic acid (372)

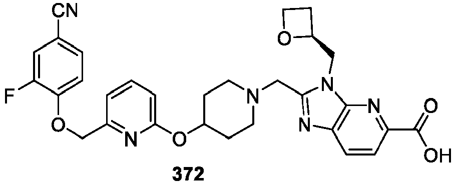

### Step A. 4-((6-Bromopyridin-2-yl)methoxy)-3-fluorobenzonitrile (372a)

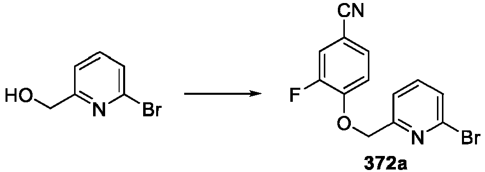

To a solution of (6-bromopyridin-2-yl)methanol (1.53 g, 8.14 mmol), 3-fluoro-4-hydroxybenzonitrile (1 g, 7.32 mmol) and PPh₃ (3.2 g, 12.2 mmol) in THF (33 mL) was added DIAD (2.4 mL, 12.2 mmol) dropwise. The resulting mixture was stirred at room temperature for 2 h. After completion, reacation was concentrated and the resulting crude residue was purified by silica gel column chromatography (gradient elution, 0-50% EtOAc in hexanes) to provide an impure product, which was triturated with MeOH. Product precipitated out as a white solid. After stirring for an additional 10 min at 0 °C, the mixture was filtered and rinsed with MeOH. The solid was dried on high vacuum pump to afford the title product **(372a)** (1.59 g, 63.6%) as a white solid. *m*/*z* (ESI, +ve ion) = 307.1 [M+H]⁺.

### Step B. tert-Butyl 4-((6-((4-cyano-2-fluorophenoxy)methyl)pyridin-2-yl)oxy)piperidine-1-carboxylatetert-butyl 4-((6-((4-cyano-2-fluorophenoxy)methyl)pyridin-2-yl)oxy)piperidine-1-carboxylate (372b)

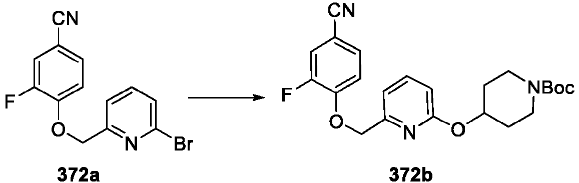

A flask was charged with **372a** (1.59 g, 5.2 mmol), *tert*-butyl 4-hydroxypiperidine-1-carboxylate (1.25 g, 6.2 mmol), Pd₂(dba)₃ (209 mg, 0.26 mmol), BINAP (322 mg, 0.52 mmol) and Cs₂CO₃ (3.37 g, 10.4 mmol) and added toluene (52 mL). The resulting mixture was degassed with Ar for 5 min and then heated at 110 °C overnight under Ar. After completion, the solvent was removed *in vacuo* and the residue was diluted with EtOAc and water. The aqueous layer was extracted with EtOAc (3x) and the combined organic layer was washed with brine, dried, filtered, concentrated and purified by silica gel column chromatography (gradient elution, 0-40% EtOAc in hexanes) to afford the title product **(372b)** (1.65 g, 75%). *m*/*z* (ESI, +ve ion) = 450.3 [M+Na]⁺.

### Step C. (S)-2-((4-((6-((4-Cyano-2-fluorophenoxy)methyl)pyridin-2-yl)oxy)piperidin-1-yl)methyl)-3-(oxetan-2-ylmethyl)-3H-imidazo[4,5-b]pyridine-5-carboxylic acid (372)

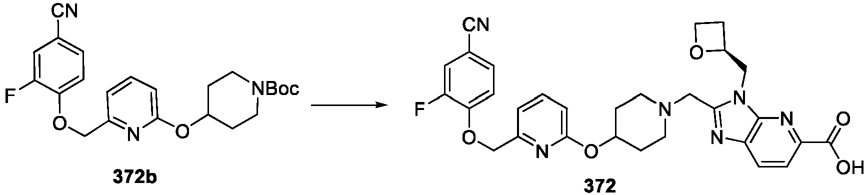

The title product **(372)** was synthesized from **372b** in similar procedures as described in Example **300,** Steps E to G. *m*/*z* (ESI, +ve ion) =573.3. ¹H NMR (400 MHz, DMSO-d₆) δ ppm 13.06 (br s, 1H), 8.14 (d, *J =* 8.3 Hz, 1H), 7.99 (d, *J =* 8.3 Hz, 1H), 7.88 (dd, *J =* 11.3, 2.0 Hz, 1H), 7.73 (dd, *J =* 8.3, 7.3 Hz, 1H), 7.64 - 7.69 (m, 1H), 7.45 (t, *J* = 8.7 Hz, 1H), 7.06 (d, *J=* 7.3 Hz, 1H), 6.74 (d, *J* = 8.0 Hz, 1H), 5.76 (s, 1H), 5.31 (s, 2H), 5.12 - 5.20 (m, 1H), 4.88 - 4.96 (m, 1H), 4.84 (dd, *J=* 14.7, 6.4 Hz, 1H), 4.66 - 4.76 (m, 1H), 4.49 (ddd, *J =* 8.4, 7.2, 5.8 Hz, 1H), 4.37 (dt, *J* = 9.0, 6.1 Hz, 1H), 3.88 - 4.02 (m, 2H), 2.63 - 2.82 (m, 3H), 2.26 - 2.38 (m, 2H), 1.86 - 1.96 (m, 2H), 1.56 - 1.70 (m, 2H).

### Example 381. (S)-2-((4-((2-((4-Chloro-2-fluorophenoxy)methyl)pyrimidin-4-yl)amino)piperidin-1-yl)methyl)-1-(oxetan-2-ylmethyl)-1H-benzo[d]imidazole-6-carboxylic acid (381)

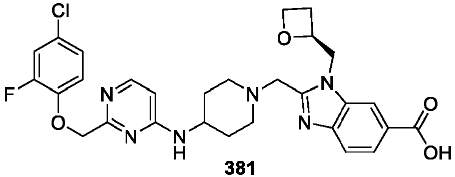

### Step A. tert-Butyl 4-((2-((4-chloro-2-fluorophenoxy)methyl)pyrimidin-4-yl)amino)piperidine-1-carboxylate (381a)

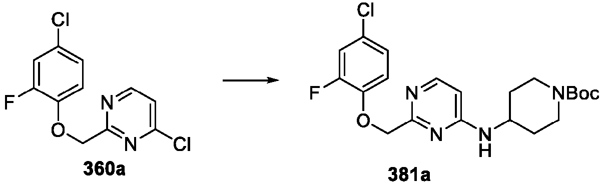

A solution of **360a** (27.3 mg, 0.1 mmol), *tert-*butyl 4-aminopiperidine-1-carboxylate (22 mg, 110 µmol), and DIPEA (34.8 µL, 0.2 mmol) in DMF (0.5 mL) was heated to 40 °C for 24 h. Upon completion, the reaction mixture was diluted with H₂O (5 mL) and stirred 30 min. The aqueous layer was decanted and the residue was rinsed with H₂O (1 mL) and dried to provide the title product **(381a)** (8 mg), which was used directly in the next step without further purification. *m*/*z* (ESI, +ve ion) = 437.4 [M+H]⁺.

### Step B. 2-((4-Chloro-2-fluorophenoxy)methyl)-N-(piperidin-4-yl)pyrimidin-4-amine (381b)

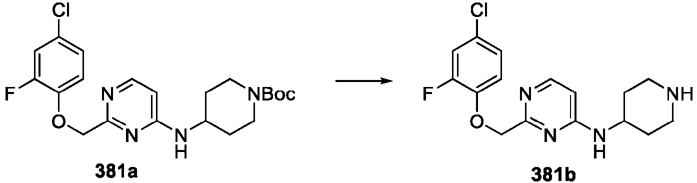

A solution of **381a** (8 mg, 18.3 µmol) and trifluoroacetic acid (0.5 mL) in DCM (1 mL) was stirred at 21 °C for 2 h. Upon completion, the reaction was concentrated and co-evaporated with DCE (3 x 2 mL) to provide the title product **(381b)** (8 mg), which was used directly in the next step without further purification. *m*/*z* (ESI, +ve ion) = 337.3 [M+H]⁺.

### Step C. Methyl (S)-2-((4-((2-((4-chloro-2-fluorophenoxy)methyl)pyrimidin-4-yl)amino)piperidin-1-yl)methyl)-1-(oxetan-2-ylmethyl)-1H-benzo[d]imidazole-6-carboxylate (381c)

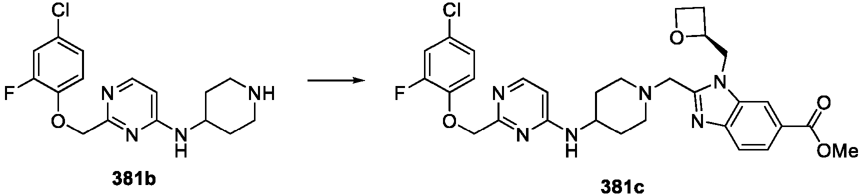

A solution of crude **381b** (8 mg), **1h** (7 mg, 23.8 µmol) and DIPEA (41.4 µL, 238 µmol) in DMF (0.5 mL) was stirred at 21 °C for 24 h. Upon completion, the reaction mixture was diluted with H₂O (10 mL), stirred for 30 min and the aqueous layer was decanted. The residue was dried to provide the title product **(381c)** (14 mg), which was used directly in the next step without further purification. *m*/*z* (ESI, +ve ion) = 595.3 [M+H]⁺.

### Step D. (S)-2-((4-((2-((4-chloro-2-fluorophenoxy)methyl)pyrimidin-4-yl)amino)piperidin-1-yl)methyl)-1-(oxetan-2-ylmethyl)-1H-benzo[d]imidazole-6-carboxylic acid (381)

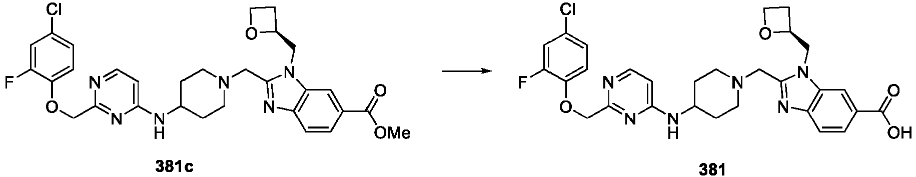

A mixture of **381c** (14 mg, 23.5 µmol) and LiOH (1 M) (118 µL, 118 µmol) in THF (235 µL), and MeOH (235 µL) were stirred at 21 °C for 4 h. Upon completion, the reaction mixture was diluted with H₂O (2 mL) and purified directly by reverse phase HPLC (Gemini 5 µm C18 110 Å, 250 x 21.2 mm AXIA packed) (gradient elution, 10-40% CH₃CN in H₂O, with 0.05% NH₄OH as a modifier) to provide the title product **(381)** (7.1 mg, 52%) as a colorless solid. *m*/*z* (ESI, +ve ion) = 581.3 [M+H]⁺. ¹H NMR (400 MHz, CD₃OD) δ ppm: 8.19 (s, 1H), 7.90 - 8.03 (m, 2H), 7.59 (d, J = 8.44 Hz, 1H), 7.19 - 7.23 (m, 1H), 7.02 - 7.08 (m, 2H), 6.36 (br s, 1H), 5.28 (qd, J = 7.03, 2.87 Hz, 1H), 5.11 (s, 2H), 4.91 - 4.93 (m, 1H), 4.84 - 4.87 (m, 1H), 4.68 - 4.74 (m, 1H), 4.61 - 4.68 (m, 1H), 4.48 (dt, *J* = 9.17, 5.99 Hz, 1H), 4.00 (d, *J* = 13.69 Hz, 1H), 3.85 - 3.97 (m, 1H), 2.88 - 2.95 (m, 1H), 2.74 - 2.86 (m, 2H), 2.48 - 2.60 (m, 1H), 2.15 - 2.31 (m, 2H), 1.85 (br s, 2H), 1.52 (br d, *J =* 12.84 Hz, 2H).

Example **382** was synthesized in similar procedures as described in Example **381.**

**Example 475** was synthesized in similar procedures starting from 4-bromo-2-fluorophenol as described in Example **355,** Steps A to E, then a step of cyclopropanation similar as described in Example **136,** Step A (Pd(OAc)₂, PPh₃, K₃PO₄), followed by Example **480,** Steps F to H, with LiOH used at the last step.

Examples **476, 508, 509,** and **510** were synthesized in similar procedures as described in Example **475.**

Example **477** was synthesized in similar procedures as described in Example **300,** Step D, followed by similar procedures as described in Example **355,** Steps F to H.

Example **478** was synthesized in similar procedures as described in Example **367.**

Example **479** was synthesized from **367f** in similar procedures as described in Example **355.**

### Example 480. 2-((4-((2-(1-(4-Cyano-2-fluorophenoxy)ethyl)pyrimidin-4-yl)oxy)piperidin-1-yl)methyl)-1-(((S)-oxetan-2-yl)methyl)-1H-benzo[d]imidazole-6-carboxylic acid (480)

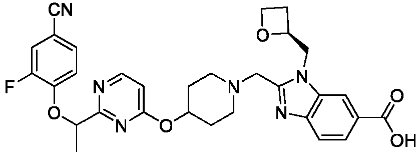

### Step A. tert-Butyl 4-((2-(1-ethoxyvinyl)pyrimidin-4-yl)oxy)piperidine-1-carboxylate (480b)

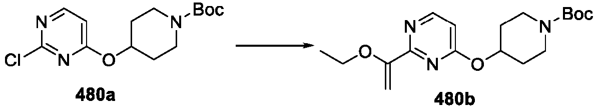

A solution of **480a** (synthesized in similar procedures as **355a)** (1 g, 3.2 mmol) and tributyl(1-ethoxyethenyl)stannane (1.39 g, 3.8 mmol) in NMP (10 mL) was added Pd(PPh₃)₂Cl₂ (0.22 g, 0.3 mmol). After heating at 110 °C for 6 h under N₂, the mixture was filtered, poured into H₂O (50 mL) and extracted with EtOAc (35 mL x 3). The combined organic layer was washed with H₂O (25 mL), dried over Na₂SO₄, filtered and concentrated *in vacuo.* The resulting crude residue was purified by Prep-TLC (EtOAc:petroleum ether = 1:10) to afford the impure title product **(480b)** (0.53 g) as a colorless oil. *m*/*z* (ESI, +ve ion) = 350.1 [M+Na]⁺.

### Step B. tert-Butyl 4-((2-acetylpyrimidin-4-yl)oxy)piperidine-1-carboxylate (480c)

A mixture of **480b** (530 mg, 1.51 mmol) in acetone (10 mL) was added 1 N HCl (6 mL, 6.05 mmol) at 25 °C. After stirring at 25 °C for 18 h, acetone was removed under reduced pressure and the residue was diluted with a mixed solution of THF/H₂O (5 mL/5 mL). Na₂CO₃ (1.6 g, 15.1 mmol) was added to the residue. The resulting mixture was stirred at 25 °C for 1 h and extracted with EtOAc (10 mL x 2). The combined organic layer was dried over Na₂SO₄, filtered and concentrated *in vacuo,* which was purified by Prep-TLC (EtOAc:petroleum ether = 1:4) to afford the title product **(480c)** (240 mg, 44%) as a colorless oil. *m*/*z* (ESI, +ve ion) = 322.1 [M+H]⁺.

### Step C. tert-Butyl 4-((2-(1-hydroxyethyl)pyrimidin-4-yl)oxy)piperidine-1-carboxylate (480d)

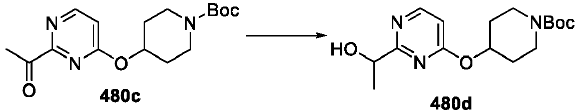

To a mixture of **480c** (260 mg, 0.806 mmol) in CH₃OH (8 mL) was added NaBH₄ (61 mg, 1.6 mmol) at 0 °C. After stirring at 0 °C for 1 h, the mixture was poured into water (20 mL) and extracted with EtOAc (35 mL x 3). The combined organic layer was dried over Na₂SO₄, filtered and concentrated *in vacuo* to afford a crude title product **(480d,** racemic) (210 mg, 72%) as a colorless oil, which was used in the next step without further purification. *m*/*z* (ESI, +ve ion) = 324.3 [M+H]⁺.

### Step D. tert-Butyl 4-((2-(1-((methylsulfonyl)oxy)ethyl)pyrimidin-4-yl)oxy)piperidine-1-carboxylate (480e)

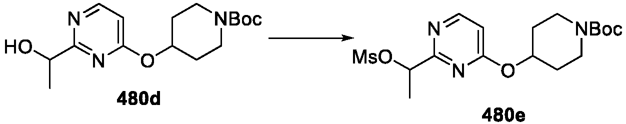

A solution of **480d** (216 mg, 0.0928 mmol) and DIPEA (345 mg, 2.67 mmol) in DCM (6 mL) was added methanesulfonic anhydride (291 mg, 1.67 mmol) at 0 °C. After stirring at 0 °C for 1 h, the reaction was diluted with DCM (20 mL) and washed with H₂0 (10 mL x 2). The combined organic layer was dried over Na₂SO₄, filtered and concentrated *in vacuo* to afford the crude title product **(480e)** (250 mg) as a colorless oil, which was used in the next step without further purification. *m*/*z* (ESI, +ve ion) = 402.2 [M+H]⁺.

### Step E. tert-Butyl 4-((2-(1-(4-cyano-2-fluorophenoxy)ethyl)pyrimidin-4-yl)oxy)piperidine-1-carboxylate (480f)

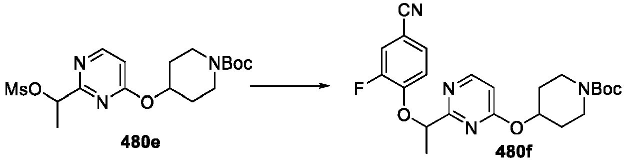

A mixture of **480e** (250 mg, 0.56 mmol), 3-fluoro-4-hydroxybenzonitrile (77 mg, 0.56 mmol) and K₂CO₃ (232 mg, 1.68 mmol) in DMF (5 mL) was heated at 80 °C for 2 h. The mixture was poured into water (30 mL) and extracted with EtOAc (25 mL x 2). The combined organic layer was dried over Na₂SO₄, filtered and concentrated to give a crude residue, which was purified by Prep-TLC (EtOAc:petroleum ether = 2:1) to afford the title product **(480f)** (180 mg, 65%) with about 90% purity as a colorless oil. *m*/*z* (ESI, +ve ion) = 443.1 [M+H]⁺.

### Step F. 3-Fluoro-4-(1-(4-(piperidin-4-yloxy)pyrimidin-2-yl)ethoxy)benzonitrile (480g)

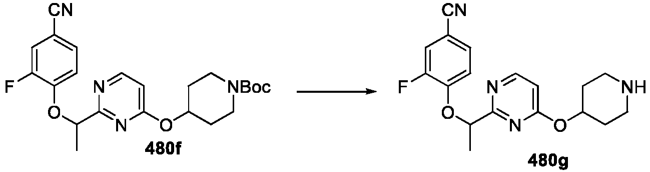

A solution of **480f** (180 mg, 0.41 mmol) in DCM (3 mL) was added TFA (1 mL). After stirring at 25 °C for 1 h, the reaction was concentrated to afford the crude title product (**480g**) (130 mg) as a yellow oil, which was used in the next step without further purification. *m*/*z* (ESI, +ve ion) = 343.1 [M+H]⁺.

### Step G. Methyl 2-((4-((2-(1-(4-cyano-2-fluorophenoxy)ethyl)pyrimidin-4-yl)oxy)piperidin-1-yl)methyl)-1-(((S)-oxetan-2-yl)methyl)-1H-benzo[d]imidazole-6-carboxylate (480h)

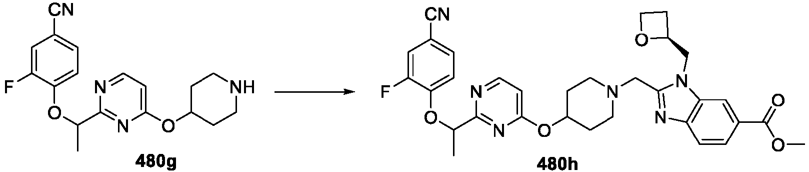

A mixture of **480g** (143 mg, 0.42 mmol), **1h** (123 mg, 0.42 mmol), and K₂CO₃ (173 mg, 1.25 mmol) in DMF (5 mL) was stirred at 25 °C for 4 h. The mixture was poured into water (35 mL) and extracted with EtOAc (25 ml x 2). The combined organic layer was washed with H₂O (20 mL), dried over Na₂SO₄, filtered and concentrated *in vacuo* to give a crude residue, which was purified by Prep-TLC (EtOAc:petroleum ether =2 :1) to afford the title product **(480h)** (180 mg, 64.6%) as a yellow oil. *m*/*z* (ESI, +ve ion) = 601.3 [M+H]⁺.

### Step H. 2-((4-((2-(1-(4-Cyano-2-fluorophenoxy)ethyl)pyrimidin-4-yl)oxy)piperidin-1-yl)methyl)-1-(((S)-oxetan-2-yl)methyl)-1H-benzo[d]imidazole-6-carboxylic acid (480)

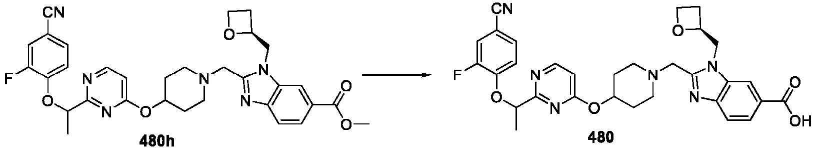

To a mixture of **480h** (20 mg, 0.033 mmol) in THF/H₂O (1mL/1mL) was added NaOH (27 mg, 0.66 mmol). After stirring at 25 °C for 18 h, the reaction was adjusted to pH = 7~8 with 1 N HCl, and extracted with DCM/CH₃OH (10/1) (15 mL x 2). The combined organic layer was dried over Na₂SO₄, filtered, concentrated and purified by Prep-TLC (DCM:CH₃OH = 10:1) to afford the title product **(480)** (5.1 mg, 24%, diastereomer mixture) as a white solid. ¹H NMR (400 MHz, CD₃OD) δ ppm 8.42 (d, *J =* 5.75 Hz, 1H), 8.30 (s, 1H), 7.97 (br d, *J=* 8.50 Hz, 1H), 7.66 (d, *J* = 8.50 Hz, 1H), 7.53 - 7.58 (m, 1H), 7.36 (br t, *J*=7.38 Hz, 1 H), 7.01 (t, *J =* 8.38 Hz, 1H), 6.72 (d, *J =* 6.00 Hz, 1H), 5.58 (q, *J =* 6.50 Hz, 1H), 5.22 - 5.38 (m, 2H), 5.03 - 5.11 (m, 1H), 4.56 - 4.77 (m, 2 H), 4.42 - 4.51 (m, 1H), 4.02 (br dd, *J* = 13.88, 4.38 Hz, 1H), 3.91 (br dd, *J* = 13.76, 4.00 Hz, 1H), 2.71 - 2.89 (m, 3H), 2.41 - 2.60 (m, 2H), 2.30 - 2.39 (m, 1H), 1.95 - 2.10 (m, 2 H), 1.70 - 1.89 (m, 4 H), 1.55 - 1.69 (m, 1H). *m*/*z* (ESI, +ve ion) = 587.2 [M+H]⁺.

Example **481** was synthesized in similar procedures from *tert-*butyl 4-hydroxypiperidine-1-carboxylate as described in Example **355,** Steps A to E, followed by Example **360,** Steps C to E.

Example **482** was synthesized in similar procedures as described in Example **481,** except TFA was used to remove the Boc protecting group.

### Example 483. (R)-2-((4-((2-((2-Chloro-4-fluorophenoxy)methyl)pyrimidin-4-yl)oxy)piperidin-1-yl)methyl)-1-((1-(isoxazole-3-carbonyl)azetidin-2-yl)methyl)-1H-benzo[d]imidazole-6-carboxylic acid (483)

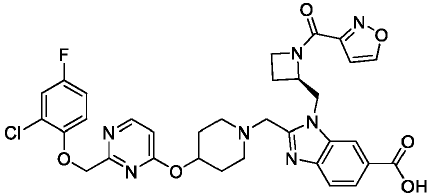

### Step A. (R)-1-(Azetidin-2-ylmethyl)-2-((4-((2-((2-chloro-4-fluorophenoxy)methyl)pyrimidin-4-yl)oxy)piperidin-1-yl)methyl)-1H-benzo[d]imidazole-6-carboxylic acid (483b)

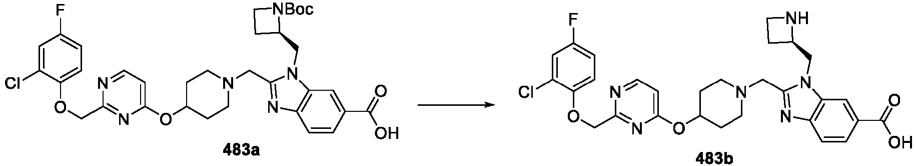

A mixture of **483a** (synthesized in similar procedures as described in Example **355)** (64 mg, 0.094 mmol) in DCM (2 mL) was added TFA (0.5 mL). After stirring at 25 °C for 2 h, the reaction was concentrated to afford the crude title product **(483b),** which was used in the next step without further purification. *m*/*z* (ESI, +ve ion) = 581.2 [M+H]⁺.

### Step B. (R)-2-((4-((2-((2-Chloro-4-fluorophenoxy)methyl)pyrimidin-4-yl)oxy)piperidin-1-yl)methyl)-1-((1-(isoxazole-3-carbonyl)azetidin-2-yl)methyl)-1H-benzo[d]imidazole-6-carboxylic acid (483)

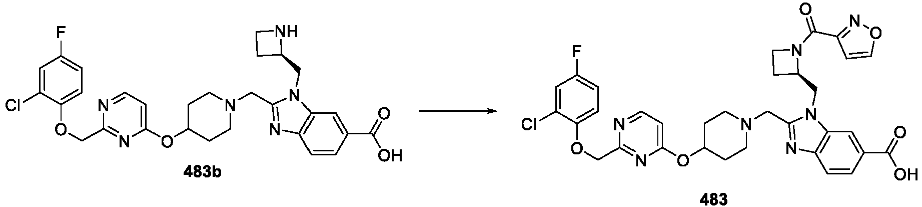

A solution of 1,2-oxazole-3-carboxylic acid (15 mg, 0.133 mmol) and DIPEA (86 mg, 0.664 mmol) in DCM (2.5 mL) was added HATU (55 mg, 0.146 mmol) at 25 °C. After stirring for 30 min, a solution of **483b** (54 mg, 0.093 mmol) in DCM (0.5 mL) was added to the above mixture. After stirring for another 30 min, the reaction was concentrated and purified by reverse phase HPLC (37% CH₃CN in H₂O, with 0.5% NH₄OH as a modifier) to afford the title product **(483)** (4.7 mg, 5.0%) as a white solid. *m*/*z* (ESI, +ve ion) = 676.2 [M+H]⁺.

Example **484** was synthesized from **261c** in similar procedures as described in Example **360,** Steps D and E, with NaI added in Step D.

Example **485** was synthesized from 2-(bromomethyl)-4-chloropyrimidine in similar procedures as described in Example **216,** Steps A to C, followed by Example **304,** Steps A and B.

### Example 486. (S)-2-((4-((2-((4-Cyano-2-fluorophenoxy)methyl)pyrimidin-4-yl)oxy)piperidin-1-yl)methyl)-1-(oxetan-2-ylmethyl)-1H-imidazo[4,5-b]pyridine-6-carboxylic acid (486)

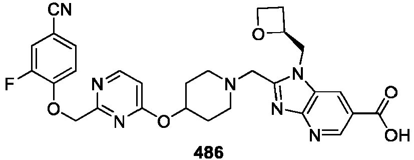

### Step A. (S)-5-Bromo-2-nitro-N-(oxetan-2-ylmethyl)pyridin-3-amine (486a)

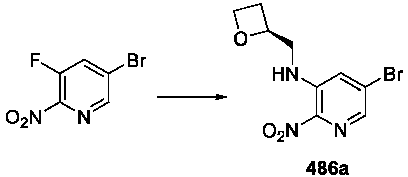

To a mixture of 5-bromo-3-fluoro-2-nitropyridine (750 mg, 3.39 mmol) and K₂CO₃ (935 mg, 6.78 mmol) in DMF (10 mL) was added **1e** (1.48 g, 16.97 mmol). After stirring at 25 °C for 16 h, the reaction was diluted with EtOAc (50 mL) and washed with brine (50 mL x 2). The combined organic layer was dried and evaporated to dryness to afford the crude title product **(486a)** (950 mg) as a yellow solid. *m*/*z* (ESI, +ve ion) = 288.0 [M+H]⁺.

### Step B. (S)-5-Bromo-N³-(oxetan-2-ylmethyl)pyridine-2,3-diamine (486b)

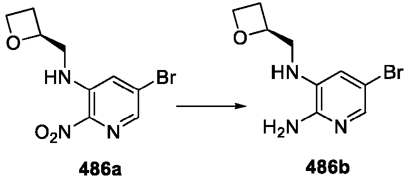

To a solution of **486a** (220 mg, 0.76 mmol) in acetic acid (5 ml) was added Fe (128 mg, 2.3 mmol). After stirring at 25 °C for 2 h, the reaction was filtered and the filtrate was diluted with EtOAc (30 ml) and washed with brine (30 mL x 3). The combined organic layer was dried, evaporated to dryness to afford the crude title product **(486b)** (185 mg) as a brown solid. *m*/*z* (ESI, +ve ion) = 258.0 [M+H]⁺.

### Step C. (S)-6-Bromo-2-(chloromethyl)-1-(oxetan-2-ylmethyl)-1H-imidazo[4,5-b]pyridine (486c)

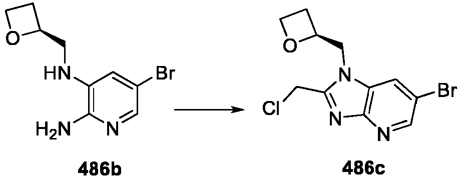

To a solution of **486b** (70 mg, 0.27 mmol) and 2-chloro-1,1,1-trimethoxyethane (125.7 mg, 0.81 mmol) in CH₃CN (3 ml) was added TsOH (17.2 mg, 0.02 mmol). After stirring under N₂ at 60 °C for 16 h, the reaction was diluted with EtOAc (20 mL) and washed with brine (20 mL x 2). The combined organic layer was dried and evaporated to dryness to afford the crude title product **(486c)** (70 mg) as a yellow solid. *m*/*z* (ESI, +ve ion) = 316.0 [M+H]⁺.

### Step D. (S)-4-((4-((1-((6-Bromo-1-(oxetan-2-ylmethyl)-1H-imidazo[4,5-b]pyridin-2-yl)methyl)piperidin-4-yl)oxy)pyrimidin-2-yl)methoxy)-3-fluorobenzonitrile (486d)

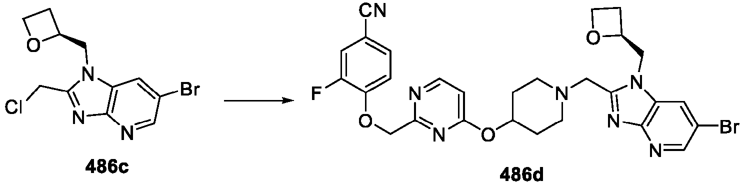

To a solution of **261c** (40 mg, 0.12 mmol) and K₂CO₃ (49.7 mg, 0.36 mmol) in CH₃CN (5 ml) was added **486c** (38.5 mg,0.122 mmol). After stirring at 25 °C for 16 h, the reaction was diluted with EtOAc (20 mL) and washed with brine (20 mL x 2). The combined organic layer was dried and evaporated to dryness and the resulting residue was purified by Prep-TLC (5% MeOH/DCM) to afford the title product **(486d)** (70 mg, 85%) as a yellow solid. *m*/*z* (ESI, +ve ion) = 608.1 [M+H]⁺.

### Step E. Methyl (5)-2-((4-((2-((4-cyano-2-fluorophenoxy)methyl)pyrimidin-4-yl)oxy)piperidin-1-yl)methyl)-1-(oxetan-2-ylmethyl)-1H-imidazo[4,5-b]pyridine-6-carboxylate (486e)

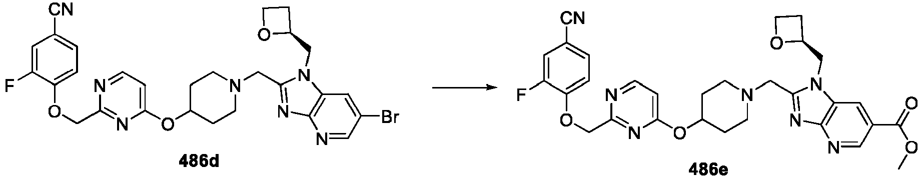

To a solution of **486d** (100 mg, 0.16 mmol) and Et₃N (49.7 mg, 0.49 mmol) in 50% DMSO/MeOH (5 ml) was added Pd(dppf)Cl₂ (12 mg, 0.016 mmol). The reaction was stirred under CO (1 atm) at 75 °C for 16 h. Then the reaction was diluted with EtOAc (50 mL) and washed with brine (50 mL x 3). The combined organic layer was dried, evaporated to dryness and the resulting crude residue was purified by Prep-TLC (5% MeOH/DCM) to afford the title product **(486e)** (50 mg, 47 %) as a yellow solid. *m*/*z* (ESI, +ve ion) = 588.2 [M+H]⁺.

### Step F. (S)-2-((4-((2-((4-Cyano-2-fluorophenoxy)methyl)pyrimidin-4-yl)oxy)piperidin-1-yl)methyl)-1-(oxetan-2-ylmethyl)-1H-imidazo[4,5-b]pyridine-6-carboxylic acid (486)

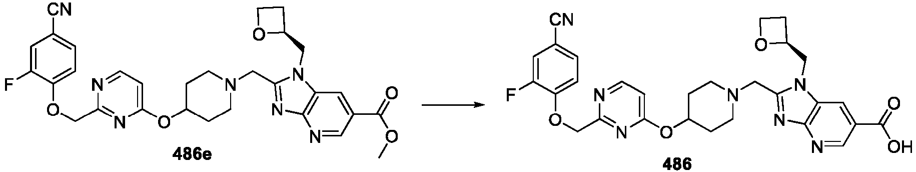

To a solution of **486e** (50 mg, 0.085 mmol) in 50% THF/H₂O (2 mL) was added LiOH (4 mg, 0.17 mmol). After stirring at 20 °C for 2 h, the reaction was adjusted to pH = 6 and purified by reverse phase HPLC (CH₃CN in H₂O) to afford the title product (2.8 mg, 5.6%) as a white solid. ¹H NMR (400 MHz, CD₃OD) δ ppm 9.06 (d, *J* = 1.71 Hz, 1H), 8.68 (d, *J =* 1.71 Hz, 1H), 8.44 (d, *J =* 5.87 Hz, 1H), 7.56 - 7.65 (m, 1H), 7.47 (br d, *J =* 8.56 Hz, 1H), 7.23 (t, *J* = 8.56 Hz, 1H), 6.76 (d, *J*=5.87 Hz, 1H), 5.39 (s, 2H), 5.15 - 5.32 (m, 1H), 5.02 (br d, *J =* 4.16 Hz, 1H), 4.84 - 4.86 (m, 1H), 4.59 - 4.80 (m, 2H), 4.48 (dt, *J* = 9.11, 5.96 Hz, 1H), 4.08 (br d, *J=* 14.18 Hz, 1H), 3.97 (br d, *J* = 13.94 Hz, 1H), 2.69 - 2.95 (m, 3H), 2.37 - 2.57 (m, 3H), 1.85 - 2.04 (m, 2H), 1.68 - 1.82 (dt, *J* = 8.44, 4.10 Hz, 2H). *m*/*z* (ESI, +ve ion) = 574.1 [M+H]⁺.

Examples **512** and **513** were synthesized in similar procedures as described in Example **355,** Steps A to F, followed by similar procedures described in Example **486,** with KI added at Step D of **486** synthesis.

Example **487** and **488** were synthesized from **261a** following similar procedures as described in Example **313.**

Example **489** was synthesized from **355d** in similar procedures as described in Example **355,** Steps E and F, followed by Example **367,** Steps F and G.

### Example 490. 2-(((2R,4S)-4-((2-((4-Cyano-2-fluorophenoxy)methyl)pyrimidin-4-yl)oxy)-2-(fluoromethyl)piperidin-1-yl)methyl)-1-(((S)-oxetan-2-yl)methyl)-1H-benzo[d]imidazole-6-carboxylic acid (490)

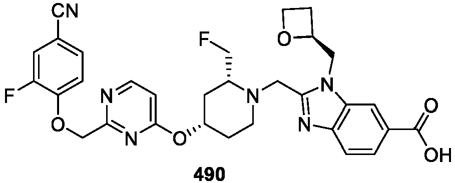

### Step A. tert-Butyl (2R,4S)-4-((2-((4-cyano-2-fluorophenoxy)methyl)pyrimidin-4-yl)oxy)-2-(hydroxymethyl)piperidine-1-carboxylate (490b)

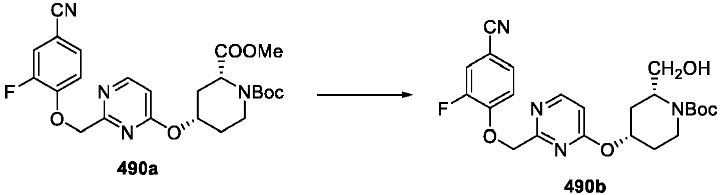

To a solution of **490a** (synthesized in similar procedures as **261b,** 144 mg, 0.30 mmol) in anhydrous EtOH (3.0 mL) at 0 °C was added NaBH₄ (32 mg, 0.89 mmol) and CaCl₂ (99 mg, 0.89 mmol). The reaction was raised to room temperature, stirred for 12 h and quenched with 1 N HCl (1 mL) dropwise at 0 °C. The reaction was extracted with EtOAc (5 mL x 3) and the combined organic layer was dried over Na₂SO₄, filtered, concentrated and purified by silica gel column chromatography (gradient elution, 0-5% MeOH in CH₂Cl₂) to provide the title product **(490b)** (24 mg, 18%). *m*/*z* (ESI, +ve ion) = 459.3 [M+H]⁺.

### Step B. Methyl 2-(((2R,4S)-4-((2-((4-cyano-2-fluorophenoxy)methyl)pyrimidin-4-yl)oxy)-2-(hydroxymethyl)piperidin-1-yl)methyl)-1-(((S)-oxetan-2-yl)methyl)-1H-benzo[d]imidazole-6-carboxylate (490c)

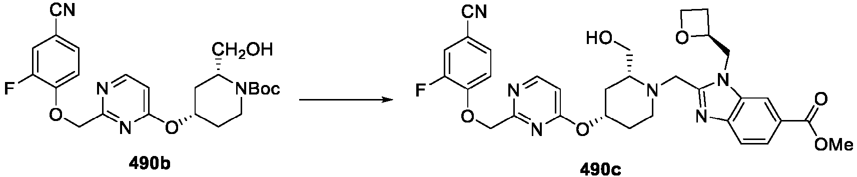

The title product **(490c)** was synthesized from **490b** in similar procedures as described in Example **355,** Step F and G.

### Step C. Methyl 2-(((2R,4S)-4-((2-((4-Cyano-2-fluorophenoxy)methyl)pyrimidin-4-yl)oxy)-2-(fluoromethyl)piperidin-1-yl)methyl)-1-(((S)-oxetan-2-yl)methyl)-1H-benzo[d]imidazole-6-carboxylate (490d)

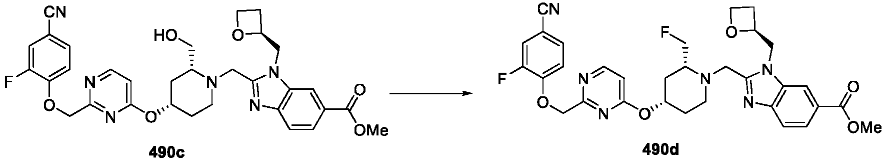

To a solution of **490c** (23 mg, 0.037 mmol) in anhydrous CH₂Cl₂ (0.37 mL) at 0 °C was added Deoxo-Fluor (22 mg, 0.045 mmol, 45% in THF) dropwise. The reaction was allowed to warm to room temperature and stirred for 1 h. Additional Deoxo-Fluor (22 mg, 0.045 mmol, 45% in THF) was added. After completion, the reaction was cooled down to 0 °C and quenched with dropwise addition of sat. NaHCO₃. The reaction was extracted with EtOAc, and washed with water and brine. The organic layer was dried over Na₂SO₄, filtered, concentrated and purified by silica gel column chromatography (gradient elution, 0-5% MeOH in CH₂Cl₂) to afford the title product **(490d)** (8 mg, 35%) as a colorless oil. *m*/*z* (ESI, +ve ion) = 619.3 [M+H]⁺.

### Step D. 2-(((2R,4S)-4-((2-((4-Cyano-2-fluorophenoxy)methyl)pyrimidin-4-yl)oxy)-2-(fluoromethyl)piperidin-1-yl)methyl)-1-(((S)-oxetan-2-yl)methyl)-1H-benzo[d]imidazole-6-carboxylic acid (490)

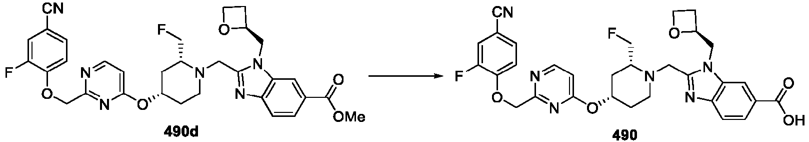

To a solution of **490d** (8 mg, 0.013 mmol) in THF/H₂O (1/1, 0.3 mL) was added LiOH.H₂O (3 mg, 0.065 mmol) and a few drops of MeOH. After heating at 40 °C for 4 h, the reaction was acidified with acetic acid to adjust to pH = 5. The reaction mixture was diluted with EtOAc (3 mL) and washed with brine (2 mL). The organic layer was then dried over Na₂SO₄, filtered, concentrated and purified by silica gel column chromatography (gradient elution, 0-5% MeOH in DCM) to afford the title product **(490)** (3 mg, 38%) as a colorless film. NMR showed a pair of rotamers. ¹H NMR (400 MHz, CD₃OD) δ ppm 8.43 (dd, *J =* 5.87, 1.71 Hz, 1 H), 8.34 - 8.30 (m, 1 H), 8.01 - 7.93 (m, 1 H), 7.71 - 7.65 (m, 1 H), 7.64 - 7.50 (m, 1 H), 7.49 - 7.40 (m, 1 H), 7.27 - 7.11 (m, 1 H), 6.77 - 6.71 (m, 1 H), 5.48 (s, 1 H), 5.42 - 5.34 (m, 1 H), 5.30 - 5.19 (m, 1 H) 5.18 - 4.97 (m, 1 H), 5.30 - 4.91 (m, 1 H), 4.96 - 4.89 (m, 1 H), 4.81 - 4.30 (m, 5 H), 4.21 - 4.06 (m, 1 H), 3.14 - 2.44 (m, 5 H), 2.31 - 1.52 (m, 4 H). *m*/*z* (ESI, +ve ion) = 605.4 [M+H]⁺.

Example **491** was synthesized from 3,5-dichloropyridazine in similar procedures as described in Example **355,** with the first step using NaH as a base and reaction run at 0 to 20 °C for 2 h.

Example **492** and **493, 494,** and **495** were synthesized in similar procedures as described in Example **355.**

Example **496** was synthesized from **220a** in similar procedures as described in Example **367,** Step D, followed by Example **355,** Steps F to H.

Example **497** was synthesized from **274c** in similar procedures as described in Example **367,** Steps C to G.

### Example 498. (S)-2-((4-((2-((4-Cyano-2,5-difluorophenoxy)methyl)pyrimidin-4-yl)oxy)piperidin-1-yl)methyl)-1-(oxetan-2-ylmethyl)-1H-benzo [d]imidazole-6-carboxylic acid (498)

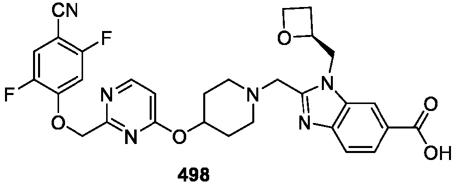

### Step A. 2,5-Difluoro-4-hydroxybenzonitrile (498a)

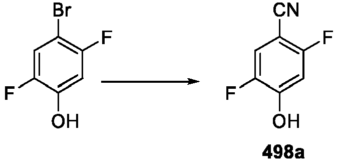

To a solution of 4-bromo-2,5-difluorophenol (300 mg, 1.4 mmol) in DMF (10 mL) was added CuCN (514 mg, 5.7 mmol). After heating at 100 °C for 12 h, the reaction was cooled down, quenched with H₂O (30 mL) and extracted with EtOAc (50 mL x 3). The combined organic layer was dried over anhydrous Na₂SO₄, filtered, concentrated and purified by reverse phase HPLC (gradient elution, 0-70% CH₃CN in H₂O) to afford the title product **(498a)** (200 mg, 85%) as a white solid. *m*/*z* (ESI, -ve ion) = 154.1 [M-H]⁻.

### Step B. tert-Butyl 4-((2-(((methylsulfonyl)oxy)methyl)pyrimidin-4-yl)oxy)piperidine-1-carboxylate (498c)

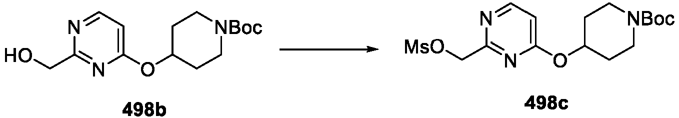

To a solution of **498b** (synthesized in similar procedures as **355c,** 400 mg, 1.2 mmol) in DCM (10 mL) was added DIPEA (1000 mg, 7.7 mmol). After stirring for 3 min at 0 °C, methanesulfonic anhydride (675 mg, 3.8 mmol) was added and the resulting mixture was stirred at 20 °C for 2 h. The reaction was quenched with H₂O (8 mL) and extracted with EtOAc (5 mL x 3). The combined organic layer was dried over anhydrous Na₂SO₄, filtered and concentrated to afford the crude title product **(498c),** which was used in the next step without further purification. *m*/*z* (ESI, +ve ion) = 388.2 [M+H]⁺.

Step C. *tert*-Butyl 4-((2-((4-cyano-2,5-difluorophenoxy)methyl)pyrimidin-4-yl)oxy)piperidine-1-carboxylate (498d)

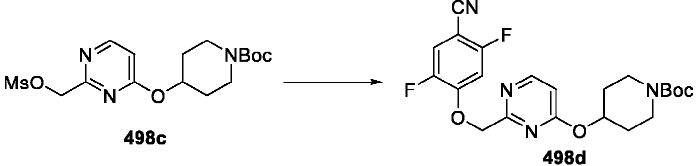

To a solution of **498c** (100 mg, 0.25 mmol) in DMF (10 mL) was added **498a** (44 mg, 0.28 mmol) and K₂CO₃ (178 mg, 1.29 mmol). After heating at 80 °C for 5 h, the reaction was quenched with H₂O (40 mL) and extracted with EtOAc (15 mL x 3). The combined organic layer was dried over anhydrous Na₂SO₄, filtered, concentrated and purified by reverse phase HPLC (gradient elution, 0-80% CH₃CN in H₂O) to afford the title product **(498d)** (100 mg, 82%) as a light-yellow solid. *m*/*z* (ESI, +ve ion) = 447.2 [M+H]⁺.

### Step D. (S)-2-((4-((2-((4-Cyano-2,5-difluorophenoxy)methyl)pyrimidin-4-yl)oxy)piperidin-1-yl)methyl)-1-(oxetan-2-ylmethyl)-1H-benzo[d]imidazole-6-carboxylic acid (498)

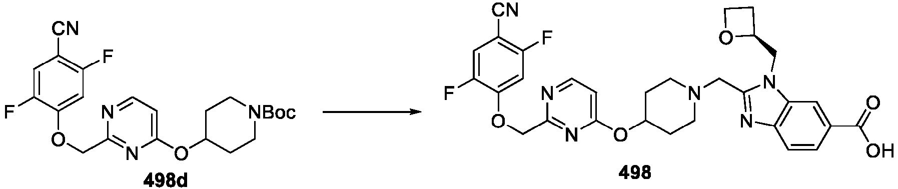

The title compound **(498)** was synthesized from **498d** in similar procedures as described in Example **220,** Steps C to E, with Step D heating to 90 °C for 12 h. ¹H NMR (400 MHz, CD₃OD) δ ppm 8.44 (d, *J =* 5.87 Hz, 1H), 8.21 (s, 1H), 7.95 (dd, *J =* 8.31, 1.47 Hz, 1H), 7.64 (dd, *J =*10.51*,* 6.11 Hz, 1 H), 7.60 (d, *J =* 8.56 Hz, 1H), 7.25 (dd, *J =* 11.00, 6.85 Hz, 1H), 6.77 (d, *J =* 5.87 Hz, 1H), 5.40 (s, 2H), 5.26 (qd, *J* = 7.13, 2.81 Hz, 1H), 5.02 (br d, *J =* 4.16 Hz, 1H), 4.83 - 4.87 (m, 1H), 4.60 - 4.73 (m, 2H), 4.46 (dt, *J*=9.17, 5.93 Hz, 1H), 3.96 - 4.02 (m, 1H), 3.85 - 3.93 (m, 1H), 2.73 - 2.84 (m, 3H), 2.48 - 2.57 (m, 1H), 2.29 - 2.39 (m, 2H), 1.87 - 2.01 (m, 2H), 1.76 (td, *J* = 8.80, 4.40 Hz, 2H). *m*/*z* (ESI, +ve ion) = 589.1 [M+H]⁺.

Example **500** was synthesized in similar procedures as described in Example **498,** with KI added at the second to last step and reaction run at room temperature.

Example **499** was synthesized in similar procedures as described in Example **220.**

Examples **501, 503** were synthesized in similar procedures as described in Example **363.**

Example **502** was synthesized from **498d** in similar procedures as described in Example **206,** Steps D to F, with THF as the solvent for the last step.

Example **504** was synthesized in similar procedures from **498c** as described in Example **498,** Step D, followed by Example **355,** Step F and Example **304,** Steps A and B. Isopropanol was used as solvent to replace MeOH at the last step.

Example **505** was synthesized in similar procedures as described in Example **504.**

Example **506,** Example **507** were synthesized from **261a** in similar procedures as described in Example **313.** DMF was used as solvent for the second to last step and THF/H₂O/isopropanol (2:1:1) was used as solvent for the last step.

Example **511** was synthesized in similar procedures as described in Example **382.**

Example **514** was synthesized in similar procedures as described in Example **261,** Steps A to C, followed by Example **367,** Steps F and G, with KI added at the second to last step.

Example **515** was synthesized in similar procedures as described in Example **355,** Steps A to F, followed by Example **367,** Steps F and G, with KI added at the second to last step.

### Biological Example 1

### GLP-1R cAMP assay

cAMP accumulation was measured in Chinese hamster ovary (CHO) cells stably overexpressing the human GLP-1 receptor using the HitHunter cAMP Assay for Small Molecules Kit (Eurofins). Briefly, cells were grown in Ham's F12 Nutrient Mix with 10% FBS, and lifted with Cell Dissociation Buffer, Enzyme free, PBS based (ThermoFisher). Cells were pelleted, and resuspended in Hank's buffered saline solution with 10 mM HEPES, and 625 µM 3-isobutyl-1-methylxanthine. The anti-cAMP antibody reagent was then added to cells at a 1:2 ratio and 5 µL of mixture was seeded in 384-well small volume white assay plates, at 10,000 cells/well. Cells were then treated with 50 nL compound using an ECHO 550 acoustic dispenser (Labcyte) in a 20-point dose response format in triplicate for 30 min. Cells were then lysed, and detection reagents added according to the manufacturer's protocol. After overnight incubation, luminescence was measured using a Perkin Elmer Envision plate reader. Dose response curves were analyzed using GraphPad Prism 9.0.

Results are reported in Table 1 below. In the Table, EC₅₀ values are +++ ≤ 50 nM< ++ ≤ 500 nM < +. Molecular weights are calculated by standard techniques, and mass spectrometry results are reported according to the Examples above.

| **Example Compound** | **EC50** | **MW Calc.** | **[M+H]⁺ Found** |
|---|---|---|---|
| **1** | ++ | 479.54 | 480.2 |
| **2** | + | 629.03 | 629.3 |
| **3** | + | 542.61 | 543.3 |
| **4** | + | 551.6 | 552.3 |
| **5** | + | 540.62 | 541.3 |
| **6** | + | 556.62 | 557.4 |
| **7** | + | 561.04 | 561.2 |
| **8** | ++ | 565.07 | 565.3 |
| **9** | ++ | 585.48 | 585.3 |
| **10** | + | 569.59 | 570.2 |
| **11** | + | 595.48 | 595.3 |
| **12** | + | 579.03 | 579.5 |
| **13** | + | 544.58 | 545.3 |
| **14** | + | 540.62 | 541.4 |
| **15** | ++ | 575.06 | 575.2 |
| **16** | + | 558.61 | 559.3 |
| **17** | + | 540.62 | 541.2 |
| **18** | ++ | 554.65 | 555.2 |
| **19** | ++ | 579.05 | 579.2 |
| **20** | + | 516.6 | 517.2 |
| **21** | + | 540.62 | 541.4 |
| **22** | + | 540.62 | 541.3 |
| **23** | + | 589.09 | 589.3 |
| **24** | + | 593.08 | 593.2 |
| **25** | + | 539.64 | 540.3 |
| **26** | ++ | 599.47 | 599.1 |
| **27** | + | 605.52 | 605.2 |
| **28** | + | 589.06 | 589.2 |
| **29** | ++ | 553.66 | 554.3 |
| **30** | + | 530.58 | 531.3 |
| **31** | + | 565.02 | 565.2 |
| **32** | ++ | 573.09 | 573.2 |
| **33** | + | 538.65 | 539.3 |
| **34** | +++ | 542.6 | 543.3 |
| **35** | + | 526.64 | 527.3 |
| **36** | + | 583.01 | 583.2 |
| **37** | + | 573.58 | 574.2 |
| **38** | + | 579.05 | 579.2 |
| **39** | + | 565.02 | 565.2 |
| **40** | + | 565.02 | 565.2 |
| **41** | ++ | 558.64 | 559.3 |
| **42** | + | 544.61 | 545.3 |
| **43** | + | 544.61 | 545.3 |
| **44** | ++ | 566.61 | 567.2 |
| **45** | +++ | 567.69 | 568.3 |
| **46** | ++ | 591.72 | 592.4 |
| **47** | +++ | 637.52 | 637.2 |
| **48** | ++ | 593.51 | 593.2 |
| **49** | + | 619.65 | 620.4 |
| **50** | + | 574.08 | 574.3 |
| **51** | +++ | 588.11 | 588.4 |
| **52** | +++ | 602.13 | 602.4 |
| **53** | + | 500.48 | 501.2 |
| **54** | ++ | 626.16 | 626.4 |
| **55** | +++ | 596.69 | 597.3 |
| **56** | ++ | 611.14 | 611.4 |
| **57** | ++ | 613.12 | 613.3 |
| **58** | + | 612.13 | 612.4 |
| **59** | ++ | 583.47 | 583.2 |
| **60** | +++ | 585.48 | 585.3 |
| **61** | + | 547.66 | 548.2 |
| **62** | + | 541.61 | 542.3 |
| **63** | +++ | 623.54 | 625.2 |
| **64** | ++ | 589.09 | 589.3 |
| **65** | ++ | 627.14 | 627.4 |
| **66** | + | 583.62 | 584.3 |
| **67** | + | 621.67 | 622.4 |
| **68** | + | 583.62 | 584.3 |
| **69** | + | 621.67 | 622.3 |
| **70** | + | 603.69 | 604.3 |
| **71** | + | 653.58 | 653.2 |
| **72** | +++ | 584.49 | 584.2 |
| **73** | + | 599.47 | 599.1 |
| **74** | + | 585.48 | 585.2 |
| **75** | +++ | 602.13 | 602.4 |
| **76** | +++ | 640.19 | 640.4 |
| **77** | + | 640.19 | 640.4 |
| **78** | + | 602.13 | 602.4 |
| **79** | + | 626.16 | 626.4 |
| **80** | + | 588.11 | 588.4 |
| **81** | ++ | 605.09 | 605.3 |
| **82** | + | 484.5 | 485.2 |
| **83** | +++ | 484.5 | 485.2 |
| **84** | +++ | 582.48 | 582.1 |
| **85** | ++ | 589.09 | 589.3 |
| **86** | + | 624.15 | 624.2 |
| **87** | ++ | 614.14 | 614.2 |
| **88** | + | 589.09 | 589.2 |
| **89** | ++ | 582.7 | 583.3 |
| **90** | + | 575.04 | 575.1 |
| **91** | + | 460.47 | 461.2 |
| **92** | ++ | 485.48 | 486.1 |
| **93** | ++ | 628.17 | 628.4 |
| **94** | + | 613.49 | 613.2 |
| **95** | ++ | 606.1 | 606.3 |
| **96** | + | 613.49 | 613.1 |
| **97** | + | 514.37 | 514.2 |
| **98** | +++ | 606.1 | 606.3 |
| **99** | ++ | 589.09 | 589.2 |
| **100** | +++ | 606.1 | 606.2 |
| **101** | ++ | 589.09 | 589.2 |
| **102** | ++ | 600.45 | 600.1 |
| **103** | +++ | 594.53 | 594.2 |
| **104** | +++ | 632.59 | 632.2 |
| **105** | + | 600.16 | 600.3 |
| **106** | ++ | 571.12 | 571.3 |
| **107** | + | 513.38 | 513.2 |
| **108** | +++ | 623.54 | 623.2 |
| **109** | +++ | 622.55 | 622.2 |
| **110** | ++ | 494.38 | 494.2 |
| **111** | ++ | 575.11 | 575.3 |
| **112** | ++ | 587.12 | 587.2 |
| **113** | + | 600.45 | 600.1 |
| **114** | +++ | 565.674 | 566.2 |
| **115** | +++ | 593.728 | 594.3 |
| **116** | +++ | 573.13 | 573.3 |
| **117** | ++ | 571.12 | 571.2 |
| **118** | +++ | 575.11 | 575.2 |
| **120** | +++ | 613.16 | 613.3 |
| **121** | + | 575.04 | 575.1 |
| **122** | +++ | 614.14 | 614.3 |
| **123** | +++ | 593.1 | 593.2 |
| **124** | +++ | 623.54 | 623.2 |
| **125** | +++ | 624.52 | N/A |
| **126** | ++ | 612.52 | 612.3 |
| **128** | +++ | 633.57 | 633.2 |
| **129** | +++ | 586.47 | 586.2 |
| **130** | +++ | 628.17 | 628.3 |
| **131** | +++ | 630.16 | 630.3 |
| **132** | +++ | 648.15 | 648.3 |
| **133** | +++ | 650.58 | 650.3 |
| **135** | ++ | 612.52 | 612.1 |
| **136** | +++ | 592.09 | 592.3 |
| **137** | +++ | 650.58 | 650.3 |
| **138** | +++ | 614.1 | 614.4 |
| **139** | +++ | 633.57 | 633.3 |
| **140** | +++ | 633.57 | 633.3 |
| **141** | +++ | 603.12 | 603.3 |
| **142** | + | 591.49 | 591 |
| **143** | ++ | 629.54 | 629 |
| **144** | +++ | 630.15 | 630.2 |
| **145** | +++ | 635.55 | 635 |
| **146** | +++ | 570.69 | 571.3 |
| **148** | +++ | 608.56 | 608.3 |
| **149** | +++ | 605.52 | 605.3 |
| **150** | +++ | 663.6 | 663.3 |
| **151** | +++ | 608.56 | 608.4 |
| **152** | +++ | 601.14 | 601.2 |
| **153** | +++ | 639.2 | 639.2 |
| **154** | ++ | 592.09 | 592.2 |
| **155** | +++ | 631.56 | 631.3 |
| **156** | +++ | 638.51 | 638.3 |
| **157** | +++ | 634.56 | 634.3 |
| **158** | +++ | 599.13 | 599.4 |
| **159** | +++ | 596.08 | 596.3 |
| **160** | +++ | 644.13 | 644.3 |
| **161** | +++ | 668.57 | 668.3 |
| **162** | +++ | 612.52 | 612.3 |
| **163** | +++ | 647.6 | 647.3 |
| **164** | +++ | 665.59 | 665.3 |
| **165** | +++ | 623.15 | 623.3 |
| **166** | +++ | 585.1 | 585.3 |
| **168** | +++ | 597.49 | 597.3 |
| **169** | +++ | 611.52 | 611.3 |
| **170** | +++ | 608.48 | 608.3 |
| **171** | +++ | 626.11 | 626.4 |
| **174** | +++ | 596.51 | 596.3 |
| **178** | ++ | 597.49 | 597.3 |
| **179** | +++ | 635.55 | 635.3 |
| **182** | +++ | 588.06 | 588.3 |
| **183** | +++ | 571.609 | 572.4 |
| **186** | +++ | 614.48 | 614.1 |
| **187** | +++ | 568.649 | 569.4 |
| **188** | +++ | 606.702 | 607.4 |
| **189** | +++ | 658.58 | 658.3 |
| **198** | +++ | 597.49 | 597.3 |
| **199** | +++ | 635.55 | 635.3 |
| **200** | +++ | 595.52 | 595.2 |
| **202** | +++ | 650.58 | 650.2 |
| **203** | +++ | 650.58 | 650.2 |
| **204** | ++ | 662.61 | 662.3 |
| **205** | ++ | 662.61 | 662.3 |
| **206** | +++ | 570.621 | 571.2 |
| **207** | +++ | 580.05 | 580.2 |
| **208** | +++ | 607.49 | 607.2 |
| **214** | +++ | 625.51 | 625.2 |
| **215** | +++ | 625.13 | 625.3 |
| **216** | +++ | 607.49 | 607.2 |
| **217** | +++ | 607.49 | 607.3 |
| **220** | +++ | 581.04 | 581.2 |
| **221** | +++ | 580.05 | 580.1 |
| **224** | +++ | 614.5 | 632.3 (ammonium + H) |
| **227** | +++ | 615.51 | 615.3 |
| **230** | +++ | 619.09 | 619.3 |
| **231** | +++ | 618.11 | 618.3 |
| **232** | +++ | 598.06 | 598.3 |
| **239** | +++ | 599.04 | 599.3 |
| **240** | +++ | 599.04 | 599.3 |
| **241** | +++ | 599.04 | 599.3 |
| **242** | +++ | 596.51 | 596.3 |
| **243** | +++ | 596.51 | 596.3 |
| **246** | +++ | 626.11 | 626.3 |
| **247** | +++ | 619.09 | 619.3 |
| **248** | ++ | 588.06 | 588.3 |
| **249** | +++ | 581.04 | 581.3 |
| **250** | +++ | 581.04 | 581.3 |
| **251** | + | 599.04 | 599.3 |
| **252** | + | 599.04 | 599.3 |
| **253** | + | 599.04 | 599.3 |
| **254** | +++ | 610.53 | 610.2 |
| **255** | +++ | 620.53 | 620.1 |
| **256** | +++ | 652.53 | 652.2 |
| **257** | +++ | 608.48 | 608.1 |
| **258** | +++ | 592.02 | 592.3 |
| **259** | +++ | 612.09 | 612.3 |
| **260** | +++ | 641.14 | 641.2 |
| **261** | +++ | 610.65 | 611.4 |
| **262** | +++ | 572.597 | 573.3 |
| **263** | +++ | 641.14 | 641.3 |
| **264** | +++ | 605.07 | 605.3 |
| **265** | +++ | 592.02 | 592.3 |
| **266** | +++ | 592.02 | 592.3 |
| **267** | +++ | 605.07 | 605.3 |
| **268** | +++ | 592.02 | 592.3 |
| **269** | +++ | 592.02 | 592.3 |
| **270** | +++ | 592.02 | 592.3 |
| **271** | ++ | 635.55 | 635.1 |
| **272** | ++ | 634.56 | 634.2 |
| **274** | +++ | 649.57 | 649.2 |
| **275** | +++ | 609.662 | 610.3 |
| **276** | +++ | 654.17 | 654.3 |
| **277** | +++ | 571.609 | 572.4 |
| **278** | +++ | 637.56 | 637.2 |
| **279** | +++ | 637.56 | 637.2 |
| **280** | +++ | 596.51 | 596.2 |
| **283** | +++ | 611.52 | 611.1 |
| **284** | + | 596.51 | 596.2 |
| **285** | +++ | 571.609 | 572.4 |
| **286** | + | 597.49 | 597.2 |
| **287** | +++ | 631.13 | 631.2 |
| **288** | + | 581.04 | 581.2 |
| **290** | +++ | 595.07 | 595.2 |
| **291** | +++ | 595.07 | 595.2 |
| **292** | +++ | 593.08 | 593.3 |
| **294** | ++ | 619.09 | 619.2 |
| **295** | +++ | 611.52 | 611.1 |
| **296** | +++ | 582.03 | 582.3 |
| **297** | +++ | 596.607 | 597.3 |
| **299** | +++ | 595.07 | 595.2 |
| **300** | +++ | 604.63 | 605.3 |
| **301** | +++ | 595.619 | 596.3 |
| **302** | +++ | 614.598 | 615.3 |
| **303** | +++ | 629.628 | 630.3 |
| **304** | +++ | 598.48 | 598.2 |
| **305** | +++ | 616.47 | 616.2 |
| **306** | +++ | 572.597 | 573.3 |
| **307** | +++ | 590.588 | 591.3 |
| **308** | +++ | 582.03 | 582.2 |
| **309** | +++ | 588.612 | 589.1 |
| **310** | +++ | 613.61 | 614.4 |
| **311** | +++ | 597.595 | 598.3 |
| **312** | +++ | 615.586 | 616.3 |
| **313** | +++ | 586.624 | 587.4 |
| **314** | +++ | 606.606 | 607.3 |
| **315** | +++ | 600.02 | 600.3 |
| **316** | +++ | 620.08 | 620.1 |
| **317** | +++ | 588.612 | 589.2 |
| **318** | +++ | 570.621 | 571.3 |
| **319** | +++ | 584.648 | 585.3 |
| **320** | +++ | 602.639 | 603.3 |
| **321** | +++ | 597.06 | 597.2 |
| **322** | +++ | 614.598 | 615.1 |
| **323** | +++ | 604.615 | 605.3 |
| **324** | +++ | 616.04 | 616.3 |
| **325** | +++ | 605.07 | 605.3 |
| **326** | +++ | 582.03 | 582.3 |
| **327** | +++ | 616.04 | 616.3 |
| **328** | +++ | 590.588 | 591.3 |
| **329** | +++ | 600.02 | 600.3 |
| **330** | +++ | 589.6 | 590.3 |
| **331** | +++ | 595.635 | 596.3 |
| **333** | +++ | 607.08 | 607.2 |
| **335** | +++ | 599.03 | 599.1 |
| **336** | +++ | 599.03 | 599.1 |
| **337** | +++ | 604.08 | 604.1 |
| **338** | +++ | 615.586 | 616.3 |
| **339** | +++ | 613.626 | 614.4 |
| **340** | +++ | 621.52 | 621.3 |
| **341** | +++ | 639.51 | 639.3 |
| **342** | +++ | 615.586 | 616.3 |
| **343** | +++ | 588.616 | 589.3 |
| **344** | +++ | 587.02 | 587.3 |
| **345** | +++ | 581.04 | 581.1 |
| **346** | +++ | 581.04 | 581.1 |
| **347** | +++ | 589.6 | 590.4 |
| **348** | +++ | 595.635 | 596.4 |
| **349** | +++ | 613.626 | 614.4 |
| **352** | +++ | 563.05 | 563.3 |
| **353** | +++ | 609.662 | 610.3 |
| **354** | +++ | 623.12 | 623.2 |
| **355** | +++ | 586.624 | 587.3 |
| **356** | +++ | 614.598 | 615.2 |
| **357** | +++ | 596.607 | 597.2 |
| **358** | +++ | 588.06 | 588.1 |
| **359** | +++ | 606.05 | 606.1 |
| **362** | +++ | 619.645 | 620.3 |
| **363** | +++ | 585.636 | 586.3 |
| **364** | +++ | 610.634 | 611.4 |
| **365** | +++ | 589.6 | 590.4 |
| **366** | +++ | 602.09 | 602.1 |
| **367** | +++ | 589.6 | 590.3 |
| **368** | +++ | 595.07 | 595.1 |
| **369** | +++ | 589.05 | 589.3 |
| **370** | +++ | 583.02 | 583.3 |
| **371** | +++ | 616.574 | 617.3 |
| **372** | +++ | 572.597 | 573.3 |
| **373** | +++ | 573.585 | 574.3 |
| **374** | +++ | 590.588 | 591.3 |
| **375** | +++ | 587.612 | 588.3 |
| **376** | +++ | 629.613 | 630.2 |
| **377** | +++ | 630.601 | 631.1 |
| **378** | +++ | 582.03 | 582.3 |
| **379** | +++ | 572.597 | 573.3 |
| **380** | +++ | 599.03 | 599.2 |
| **381** | +++ | 581.05 | 581.3 |
| **382** | +++ | 595.07 | 595.3 |
| **475** | +++ | 587.652 | 588.2 |
| **476** | +++ | 594.672 | 595.3 |
| **477** | +++ | 603.08 | 603.3 |
| **478** | +++ | 607.59 | 608.2 |
| **479** | +++ | 565.578 | 566.3 |
| **480** | ++ | 586.624 | 587.2 |
| **481** | +++ | 589.05 | 589.1 |
| **482** | +++ | 583.568 | 584.2 |
| **483** | +++ | 676.1 | 676.2 |
| **484** | +++ | 586.624 | 587.3 |
| **485** | +++ | 579.605 | 580.3 |
| **486** | +++ | 573.585 | 574.1 |
| **487** | ++ | 598.635 | 599.4 |
| **488** | ++ | 598.635 | 599.4 |
| **489** | +++ | 603.08 | 603.1 |
| **490** | +++ | 604.615 | 605.4 |
| **491** | +++ | 572.597 | 573.3 |
| **492** | +++ | 604.615 | 605.3 |
| **493** | +++ | 604.615 | 605.3 |
| **494** | +++ | 601.559 | 602.2 |
| **495** | +++ | 583.568 | 584.1 |
| **496** | +++ | 582.58 | 583.1 |
| **497** | +++ | 578.617 | 579.3 |
| **498** | +++ | 590.588 | 591.2 |
| **499** | +++ | 585.636 | 586.3 |
| **500** | +++ | 586.624 | 587.2 |
| **501** | +++ | 603.627 | 604.3 |
| **502** | +++ | 608.578 | 609.2 |
| **503** | +++ | 599.663 | 600.3 |
| **504** | +++ | 590.588 | 591.3 |
| **505** | +++ | 608.578 | 609.3 |
| **506** | +++ | 584.608 | 585.3 |
| **507** | +++ | 584.608 | 585.3 |
| **508** | +++ | 613 | 613.3 |
| **509** | +++ | 627 | 627.3 |
| **510** | +++ | 609 | 609.3 |
| **511** | +++ | 653.13 | 653.1 |
| **512** | + | 580.593 | 581.2 |
| **513** | +++ | 587.612 | 588.2 |
| **514** | +++ | 597.595 | 598.2 |
| **515** | +++ | 600.651 | 601.2 |

### Intravenous glucose tolerance test (IVGTT) in cynomolgus monkeys

Aged (< 8 years old) cynomolgus monkeys (*Macaca fascicularis*; N = 4-5/test article) were fasted overnight and anesthetized with ketamine prior to the intravenous glucose tolerance test (IVGTT). The test articles were formulated at 6 mg/mL in vehicle (5% polyethylene glycol 400: 95% (12% (w/v) sulfobutylether-β-cyclodextrin in deionized water) (v/v)]) and administered by intravenous bolus into a peripheral vein 1-6 minutes prior to the IVGTT. The IVGTT was initiated by administration of an IV bolus of glucose (0.5 g/kg as a 50% glucose solution). Blood samples were collected from a peripheral vein 0, 1, 3, 5, 10, 20, 40 and 60 minutes upon completion of the IV bolus glucose injection. The blood samples were collected directly into potassium ethylene diamine tetra-acetic acid tubes and plasma separated by centrifugation at 2,500 g for 10 minutes within 30 minutes of collection. The plasma from all time points was analyzed for insulin concentrations (µU/mL) using a Cobas e411 Immunology analyzer. Insulin area-under-the-curve (AUC) was calculated using GraphPad Prism software (v 9). As shown in FIG. 1, an increase in insulin secretion was observed, consistent with the substantial increase of insulin AUC following administration of compounds **145, 171, 313, 326, 335, 355,** and **338** respectively.

## Claims

1. A compound which is 2-{[(2S,4S)-4-({2-[(2,4-difluorophenoxy)methyl]pyrimidin-4-yl}oxy)-2-methylpiperidin-1-yl]methyl}-1-{[(2S)-oxetan-2-yl]methyl}-1H-1,3-benzodiazole-6-carboxylic acid: 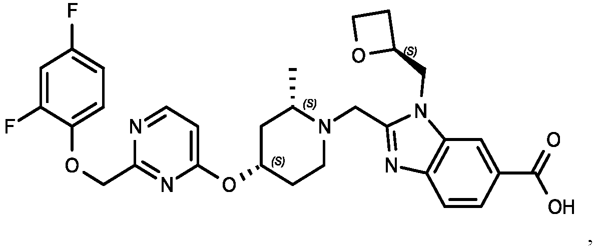 or a pharmaceutically acceptable salt or solvate thereof.

2. The compound of claim 1, which is:

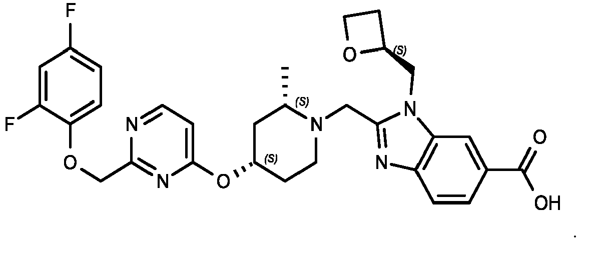

3. The compound of claim 1, which is a pharmaceutically acceptable salt of:

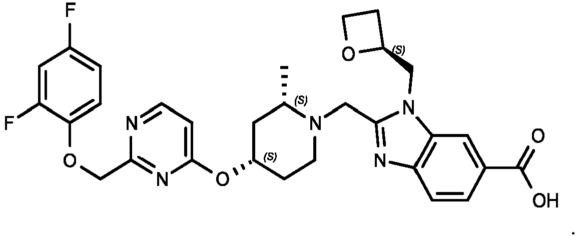

4. A pharmaceutical composition comprising the compound of any one of the preceding claims, and one or more pharmaceutically acceptable carriers, excipients, or diluents.

5. The compound of any one of claims 1 to 3, or the pharmaceutical composition of claim 4, for use in therapy.

6. The compound of any one of claims 1 to 3, or the pharmaceutical composition of claim 4, for use in the treatment of type 2 diabetes.

## Patentansprüche

1. Verbindung, die 2-{[(2S,4S)-4-({2-[(2,4-Difluorphenoxy)methyl]pyrimidin-4-yl}oxy)-2-methylpiperidin-1-yl]methyl}-1-{[(2S)-oxetan-2-yl]methyl}-1H-1,3-benzodiazol-6-carbonsäure ist: 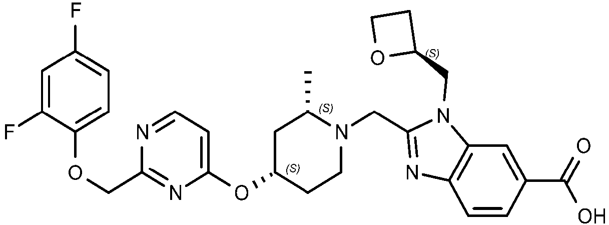 oder ein pharmazeutisch unbedenkliches Salz oder Solvat davon.

2. Verbindung nach Anspruch 1, die:

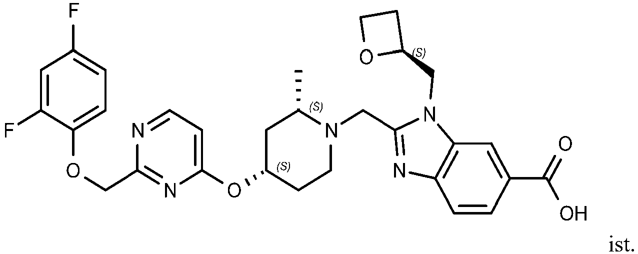

3. Verbindung nach Anspruch 1, die ein pharmazeutisch unbedenkliches Salz von:

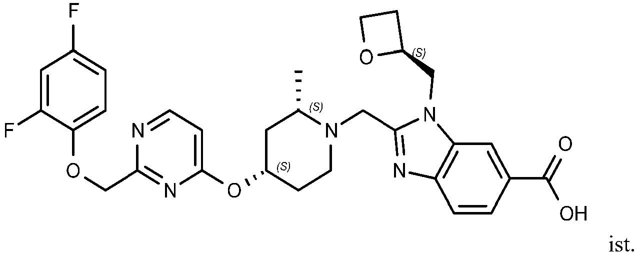

4. Pharmazeutische Zusammensetzung, umfassend die Verbindung nach einem der vorhergehenden Ansprüche und einen bzw. ein oder mehrere pharmazeutisch unbedenkliche Trägerstoffe, Hilfsstoffe oder Verdünnungsmittel.

5. Verbindung nach einem der Ansprüche 1 bis 3 oder pharmazeutische Zusammensetzung nach Anspruch 4 zur Verwendung in einer Therapie.

6. Verbindung nach einem der Ansprüche 1 bis 3 oder pharmazeutische Zusammensetzung nach Anspruch 4 zur Verwendung bei der Behandlung von Typ-2-Diabetes.

## Revendications

1. Composé, lequel est l'acide 2-{[(2S,4S)-4-({2-[(2,4-difluorophénoxy)méthyl]pyrimidin-4-yl}oxy)-2-méthylpipéridin-1-yl]méthyl}-1-{[(2S)-oxétan-2-yl]méthyl}-1H-1,3-benzodiazole-6-carboxylique 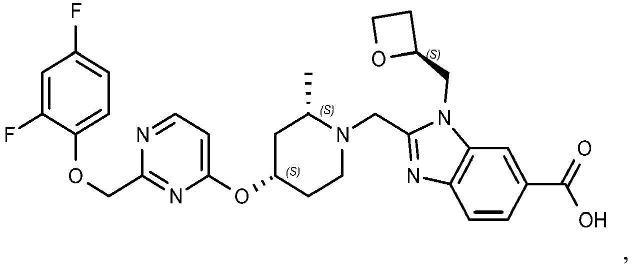 ou un sel ou un solvate pharmaceutiquement acceptable de celui-ci.

2. Composé selon la revendication 1, lequel est :

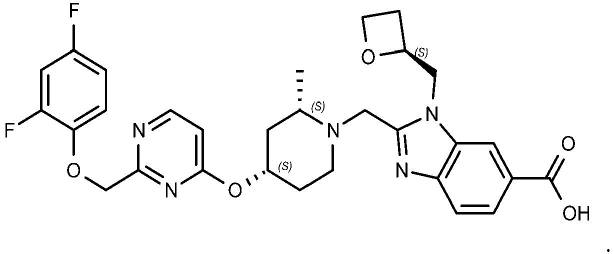

3. Composé selon la revendication 1, lequel est un sel pharmaceutiquement acceptable de :

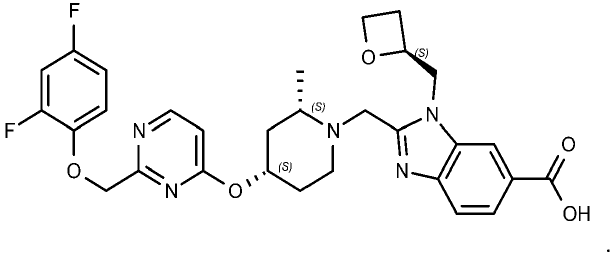

4. Composition pharmaceutique comprenant le composé selon l'une quelconque des revendications précédentes, et un ou plusieurs supports, excipients ou diluants pharmaceutiquement acceptables.

5. Composé selon l'une quelconque des revendications 1 à 3, ou composition pharmaceutique selon la revendication 4, pour utilisation à des fins thérapeutiques.

6. Composé selon l'une quelconque des revendications 1 à 3, ou composition pharmaceutique selon la revendication 4, pour utilisation dans le traitement du diabète de type 2.
